# EUROPEAN PATENT APPLICATION

(11) **EP 3 839 049 A2**
(43) Date of publication of application: **23.06.2021**
(21) Application number: 20188009.3
(22) Date of filing: 18.09.2012
(51) Int. Cl.: C12N 15/85, A01K 67/027, C07K 16/46, C07K 16/18

(54) **ANTIBODIES, VARIABLE DOMAINS & CHAINS TAILORED FOR HUMAN USE**

(30) Priority: 19.09.2011 GB 201116122; 19.09.2011 GB 201116120; 24.02.2012 GB 201203257; 15.03.2012 GB 201204592; 29.03.2012 GB 201205702; 18.05.2012 GB 201208749; 02.07.2012 GB 201211692
(62) Divisional of application: 16189625.3
(71) Applicant: Kymab Limited, Cambridge CB22 3AT (GB)
(72) Inventor: BRADLEY, Allan, Cambridge, Cambridgeshire CB22 3AT (GB); FRIEDRICH, Glenn A., Cambridge, Cambridgeshire CB22 3AT (GB); LEE, E-Chiang, Cambridge, Cambridgeshire CB22 3AT (GB); STRIVENS, Mark, Cambridge, Cambridgeshire CB22 3AT (GB); ENGLAND, Nicholas, Cambridge, Cambridgeshire CB22 3AT (GB)
(74) Representative: CMS Cameron McKenna Nabarro Olswang LLP

(57) **Abstract**

The invention relates to the provision of antibody therapeutics and prophylactics that are tailored specifically for human use. The present invention provides libraries, vertebrates and cells, such as transgenic mice or rats or transgenic mouse or rat cells. Furthermore, the invention relates to methods of using the vertebrates to isolate antibodies or nucleotide sequences encoding antibodies. Antibodies, heavy chains, polypeptides, nucleotide sequences, pharmaceutical compositions and uses are also provided by the invention.

## Description

### FIELD OF THE INVENTION

The present invention relates to the provision of antibody therapeutics and prophylactics that are tailored specifically for human use.

The present invention provides libraries, vertebrates and cells, such as transgenic mice or rats or transgenic mouse or rat cells. Furthermore, the invention relates to methods of using the vertebrates to isolate antibodies or nucleotide sequences encoding antibodies. Antibodies, heavy chains, polypeptides, nucleotide sequences, pharmaceutical compositions and uses are also provided by the invention.

### BACKGROUND

The state of the art provides non-human vertebrates (eg, mice and rats) and cells comprising transgenic immunoglobulin loci, such loci comprising human variable (V), diversity (D) and/or joining (J) segments, and optionally human constant regions. Alternatively, endogenous constant regions of the host vertebrate (eg, mouse or rat constant regions) are provided in the transgenic loci. Methods of constructing such transgenic vertebrates and use of these to generate antibodies and nucleic acids thereof following antigen immunisation are known in the art, eg, see US7501552 (Medarex), US5939598 (Abgenix), US6130364 (Abgenix), WO02/066630 (Regeneron), WO2011004192 (Genome Research Limited), WO2009076464, WO2009143472 and WO2010039900 (Ablexis), the disclosures of which are explicitly incorporated herein. Such transgenic loci in the art include varying amounts of the human V(D) J repertoire. Existing transgenic immunoglobulin loci are based on a single human DNA source. The potential diversity of human antibody variable regions in non-human vertebrates bearing such transgenic loci is thus confined.

The inventors considered that it would be desirable to tailor the genomes of these transgenic non-human vertebrates (and thus antibody and antibody chain products of these) to address the variability - and commonality - in the natural antibody gene usage of humans. The inventors wanted to do this in order to better address human use of antibody-based therapeutic and prophylactic drugs.

It would be desirable also to provide for novel and potentially expanded repertoire and diversity of human variable regions in transgenic immunoglobulin loci and non-human vertebrates harbouring these, as well as in antibodies produced following immunisation of such animals.

### SUMMARY OF THE INVENTION

The present invention has been developed from extensive bioinformatics analysis of natural antibody gene segment distributions across a myriad of different human populations and across more than two thousand samples from human individuals. The inventors have undertaken this huge task to more thoroughly understand and design non-human vertebrate systems and resultant antibodies to better address human medical therapeutics as a whole, as well as to enable rational design to address specific ethnic populations of humans. Using such rational design, the inventors have constructed transgenic non-human vertebrates and isolated antibodies, antibody chains and cells expressing these in a way that yields products that utilise gene segments that have been purposely included on the basis of the human bioinformatics analysis. The examples illustrate worked experiments where the inventors isolated many cells and antibodies to this effect.

The invention also relates to synthetically-extended & ethnically-diverse superhuman immunoglobulin gene repertoires. The present invention thus provides for novel and potentially expanded synthetic immunoglobulin diversities, thus providing a pool of diversity from which human antibody therapeutic leads can be selected. This expanded pool is useful when seeking to find antibodies with desirable characteristics, such as relatively high affinity to target antigen without the need for further affinity maturation (eg, using laborious *in vitro* techniques such as phage or ribosome display), or improved biophysical characteristics, or to address targets and new epitopes that have previously been difficult to address with antibodies are not reached by prior antibody binding sites.

The invention also provides for diversity that is potentially biased towards variable gene usage common to members of a specific human population, which is useful for generating antibodies for treating and/or preventing diseases or conditions within such population. This ability to bias the antibody repertoire allows one to tailor antibody therapeutics with the aim of more effectively treating and/or preventing disease or medical conditions in specific human populations.

The present inventors realised the possibility of providing immunoglobulin gene segments from disparate sources in transgenic loci, in order to provide for novel and potentially-expanded antibody diversities from which antibody therapeutics (and antibody tool reagents) could be generated. This-opens up the potential of transgenic human-mouse/rat technologies to the possibility of interrogating different and possibly larger antibody sequence-spaces than has hitherto been possible.

In rationally designing transgenic antibody loci, as well as antibodies and antibody chains, the inventors also realised that a relatively long HCDR3 length (at least 20 amino acids) is often desirable to address epitopes. For example, naturally-occurring antibodies have been isolated from humans infected with infectious disease pathogens, such antibodies having a long HCDR3 length. Neutralising antibodies have been found in this respect. A long HCDR3 length would be desirable to address other antigens (eg, receptor clefts or enzyme active sites), not just limited to infectious disease pathogens, and thus the inventors realised the general desirability of the possibility of engineering transgenic loci to be able to produce long HCDR3 antibodies and heavy chains. The inventors, through laborious execution of bioinformatics on in excess of 2000 human DNA samples *via* the 1000 Genomes project together with rational sequence choices, identified that the inclusion of the specific human gene segment variant JH6*02 is desirable for producing long HCDR3 antibodies and chains.

Additional rational design and bioinformatics has led the inventors to realise that specific human constant region variants are conserved across many diverse human populations. The inventors realised that this opens up the possibility of making a choice to humanise antibodies, chains and variable domains by using such specific constant regions in products, rather than arbitrarily choosing the human constant region (or a synthetic version of a human constant region). This aspect of the invention also enables one to tailor antibody-based drugs to specific human ethnic populations, thereby more closely matching drug to patient (and thus disease setting) than has hitherto been performed. It can be a problem in the state of the art that antibodies are humanised with an arbitrary choice of human constant region (presumably derived from one (often unknown) ethnic population or non-naturally occurring) that does not function as well in patients of a different human ethnic population. This is important, since the constant region has the major role in providing antibody effector functions, eg, for antibody recycling, cellular and complement recruitment and for cell killing.

To this end, in a first configuration of the invention, there is provided

### First Configuration

A non-human vertebrate or vertebrate cell (optionally an ES cell or antibody-producing cell) comprising a genome having a superhuman immunoglobulin heavy chain human VH and/or D and/or J gene repertoire.

A non-human vertebrate or vertebrate cell (optionally an ES cell or antibody-producing cell) comprising a genome having a superhuman immunoglobulin light chain human VL gene repertoire; optionally wherein the vertebrate or cell is according to the first configuration.

A non-human vertebrate or vertebrate cell (optionally an ES cell or antibody-producing cell) whose genome comprises a transgenic immunoglobulin locus (eg, a heavy chain locus or a light chain locus), said locus comprising immunoglobulin gene segments according to the first and second human immunoglobulin gene segments (optionally V segments) as mentioned below operably connected upstream of an immunoglobulin constant region; optionally wherein the genome is homozygous for said transgenic immunoglobulin locus;
optionally wherein the immunoglobulin locus comprises more than the natural human complement of functional V gene segments; and/or
optionally wherein the immunoglobulin locus comprises more than the natural human complement of functional D gene segments; and/or
optionally wherein the immunoglobulin locus comprises more than the natural human complement of functional J gene segments.

A transgenic non-human vertebrate (eg, a mouse or rat) or vertebrate cell (optionally an ES cell or antibody-producing cell) whose genome comprises a transgenic immunoglobulin locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region; wherein the transgenic locus comprises one or more human immunoglobulin V gene segments, one or more human J gene segments and optionally one or more human D gene segments, a first (optionally a V segment) of said gene segments and a second (optionally a V segment) of said gene segments being different and derived from the genomes of first and second human individuals respectively, wherein the individuals are different; and optionally not related;
optionally wherein the immunoglobulin locus comprises more than the natural human complement of functional V gene segments; and/or
optionally wherein the immunoglobulin locus comprises more than the natural human complement of functional D gene segments; and/or
optionally wherein the immunoglobulin locus comprises more than the natural human complement of functional J gene segments.

A transgenic non-human vertebrate (eg, a mouse or rat) or vertebrate cell (optionally an ES cell or antibody-producing cell) whose genome comprises first and second transgenic immunoglobulin loci, each locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region;
wherein (i) the first transgenic locus comprises one or more human immunoglobulin V gene segments, one or more human J gene segments and optionally one or more human D gene segments, (ii) the second transgenic locus comprises one or more human immunoglobulin V gene segments, one or more human J gene segments and optionally one or more human D gene segments; and (iii) wherein a first (optionally a V) gene segment of said first locus and a second (optionally a V) gene segment of said second gene locus are different and derived from the genomes of first and second human individuals respectively, wherein the individuals are different; and optionally not related;
optionally wherein the first and second loci are on different chromosomes (optionally chromosomes with the same chromosome number) in said genome;
optionally wherein each immunoglobulin locus comprises more than the natural human complement of functional V gene segments; and/or
optionally wherein each immunoglobulin locus comprises more than the natural human complement of functional D gene segments; and/or
optionally wherein each immunoglobulin locus comprises more than the natural human complement of functional J gene segments.

A method of constructing a cell (eg, an ES cell) according to the invention, the method comprising
(a) identifying functional V and J (and optionally D) gene segments of the genome sequence of a (or said) first human individual;
(b) identifying one or more functional V and/or D and/or J gene segments of the genome sequence of a (or said) second human individual, wherein these additional gene segments are not found in the genome sequence of the first individual;
(c) and constructing a transgenic immunoglobulin locus in the cell, wherein the gene segments of (a) and (b) are provided in the locus operably connected upstream of a constant region.

In one embodiment, the gene segment(s) in step (b) are identified from an immunoglobulin gene database selected from the 1000 Genomes, Ensembl, Genbank and IMGT databases.

Throughout this text, Genbank is a reference to Genbank release number 185.0 or 191.0; the 1000 Genomes database is Phase 1, release v3, 16^{th} March 2012; the Ensembl database is assembly GRCh37.p8 (10/04/2012); the IMGT database is available at www.imgt.org.

In one embodiment, the first and second human individuals are members of first and second ethnic populations respectively, wherein the populations are different, optionally wherein the human immunoglobulin gene segment derived from the genome sequence of the second individual is low-frequency (optionally rare) within the second ethnic population.

This configuration of the invention also provides a method of making a transgenic non-human vertebrate (eg, a mouse or rat), the method comprising
(a) constructing an ES cell (eg, a mouse C57BL/6N, C57BL/6J, 129S5 or 129Sv strain ES cell) by carrying out the method above;
(b) injecting the ES cell into a donor non-human vertebrate blastocyst (eg, a mouse C57BL/6N, C57BL/6J, 129S5 or 129Sv strain blastocyst);
(c) implanting the blastocyst into a foster non-human vertebrate mother (eg, a C57BL/6N, C57BL/6J, 129S5 or 129Sv strain mouse); and
(d) obtaining a child from said mother, wherein the child genome comprises a transgenic immunoglobulin locus.

In one embodiment, the invention provides a method of isolating an antibody that binds a predetermined antigen (eg, a bacterial or viral pathogen antigen), the method comprising immunising a non-human vertebrate according to the invention.

### Second Configuration

A library of antibody-producing transgenic cells whose genomes collectively encode a repertoire of antibodies, wherein
(a) a first transgenic cell expresses a first antibody having a chain encoded by a first immunoglobulin gene, the gene comprising a first variable domain nucleotide sequence produced following recombination of a first human unrearranged immunoglobulin gene segment;
(b) a second transgenic cell expresses a second antibody having a chain encoded by a second immunoglobulin gene, the second gene comprising a second variable domain nucleotide sequence produced following recombination of a second human unrearranged immunoglobulin gene segment, the first and second antibodies being non-identical;
(c) the first and second gene segments are different and derived from the genome sequences of first and second human individuals respectively, wherein the individuals are different; and optionally not related;
(d) wherein the cells are non-human vertebrate (eg, mouse or rat) cells.

In one embodiment, the first and second human individuals are members of first and second ethnic populations respectively, wherein the populations are different; optionally wherein the ethnic populations are selected from those identified in the 1000 Genomes database.

In another embodiment, the second human immunoglobulin gene segment is a polymorphic variant of the first human immunoglobulin gene segment; optionally wherein the second gene segment is selected from the group consisting of a gene segment in any of Tables 1 to 7 and 9 to 14 below (eg, selected from Table 13 or Table 14), eg, the second gene segment is a polymorphic variant of VH1-69.

### Third Configuration

An isolated antibody having
(a) a heavy chain encoded by a nucleotide sequence produced following recombination in a transgenic non-human vertebrate cell of an unrearranged human immunoglobulin V gene segment with a human D and human J segment, optionally with affinity maturation in said cell, wherein one of the gene segments is derived from the genome of an individual from a first human ethnic population; and the other two gene segments are derived from the genome of an individual from a second, different, human ethnic population, and wherein the antibody comprises heavy chain constant regions of said non-human vertebrate (eg, rodent, mouse or rat heavy chain constant regions); and/or
(b) a light chain encoded by a nucleotide sequence produced following recombination in a transgenic non-human vertebrate cell of an unrearranged human immunoglobulin V gene segment with a human J segment, optionally with affinity maturation in said cell, wherein one of the gene segments is derived from the genome of an individual from a first human ethnic population (optionally the same as the first population in (a)); and the other gene segment is derived from the genome of an individual from a second, different, human ethnic population (optionally the same as the second population in (a)), and wherein the antibody comprises light chain constant regions of said non-human vertebrate (eg, rodent, mouse or rat heavy light constant regions);
(c) Optionally wherein each variable domain of the antibody is a human variable domain.
(d) Optionally wherein the heavy chain constant regions are gamma-type constant regions.

The invention also provides an isolated nucleotide sequence encoding the antibody, optionally wherein the sequence is provided in an antibody expression vector, optionally in a host cell.

The invention also provides a method of producing a human antibody, the method comprising replacing the non-human vertebrate constant regions of the antibody of the third configuration with human antibody constant regions.

The invention also provides a pharmaceutical composition comprising an antibody according to the third configuration, or an antibody produced according to the method above and a diluent, excipient or carrier; optionally wherein the composition is provided in a container connected to an IV needle or syringe or in an IV bag.

The invention also provides an antibody-producing cell that expresses the second antibody recited in any one of the configurations.

In an alternative configuration, the invention contemplates the combination of nucleotide sequences of first and second immunoglobulin gene segments (eg, two or more polymorphic variants of a particular human germline VH or VL gene segment) to provide a synthetic gene segment. Such synthetic gene segment is used, in one embodiment, to build a transgenic immunoglobulin locus, wherein the synthetic gene segment is provided in combination with one or more human variable and J regions (and optionally one or more human D regions) operably connected upstream of a constant region. When provided in the genome of a non-human vertebrate or cell (eg, mouse or rat cell, eg, ES cell), the invention provides for superhuman gene segment diversity. The sequences to be combined can be selected from gene segments that have been observed to be commonly used in human antibodies raised against a particular antigen (eg, a flu antigen, such as haemaglutinin). By combining the sequences, the synthetic gene segment may recombine *in vivo* to produce an antibody that is well suited to the treatment and/or prevention of a disease or condition (eg, influenza) mediated by said antigen.

### Fourth Configuration

A non-human vertebrate (optionally a mouse or a rat) or vertebrate cell whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof so that the mouse is capable of producing an antibody heavy chain produced by recombination of the human JH6*02 with a D segment and a VH segment.

A non-human vertebrate cell (optionally a mouse cell or a rat cell) whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof for producing (eg, in a subsequent progeny cell) an antibody heavy chain produced by recombination of the human JH6*02 with a D segment and a VH segment.

A heavy chain (eg, comprised by an antibody) isolated from a vertebrate of the invention wherein the heavy chain comprises a HCDR3 of at least 20 amino acids.

A method for producing a heavy chain, VH domain or an antibody specific to a target antigen, the method comprising immunizing a non-human vertebrate according to the invention with the antigen and isolating the heavy chain, VH domain or an antibody specific to a target antigen or a cell producing the heavy chain, VH domain or an antibody, wherein the heavy chain, VH domain or an antibody comprises a HCDR3 that is derived from the recombination of human JH6*02 with a VH gene segment and a D gene segment.

A heavy chain, VH domain or an antibody produced by the method.

A B-cell or hybridoma expressing a heavy chain VH domain that is identical to the VH domain of the heavy chain.

A nucleic acid encoding the VH domain of the heavy chain of claim 22, 23 or 28, or encoding the heavy chain.

A vector (eg, a CHO cell or HEK293 cell vector) comprising the nucleic acid; optionally wherein the vector is in a host cell (eg, a CHO cell or HEK293 cell).

A pharmaceutical composition comprising the antibody, heavy chain or VH domain (eg, comprised by an antibody), together with a pharmaceutically-acceptable excipient, diluent or a medicament (eg, a further antigen-specific variable domain, heavy chain or antibody).

The antibody, heavy chain or VH domain (eg, comprised by an antibody) as above for use in medicine.

The use of an antibody, heavy chain or VH domain (eg, comprised by an antibody) as above in the manufacture of a medicament for treating and/or preventing a medical condition in a human.

### Fifth Configuration

A method of producing an antibody heavy chain, the method comprising
(a) providing an antigen-specific heavy chain variable domain; and
(b) combining the variable domain with a human heavy chain constant region to produce an antibody heavy chain comprising (in N- to C-terminal direction) the variable domain and the constant region;
wherein
the human heavy chain constant region is an IGHG1ref, IGHG2ref, IGHG2a, IGHG3ref, IGHG3a, IGHG3b, IGHG4ref or IGHG4a constant region.

An antibody comprising a human heavy chain, the heavy chain comprising a variable domain that is specific for an antigen and a constant region that is an IGHG1ref, IGHG2ref, IGHG2a, IGHG3ref, IGHG3a, IGHG3b, IGHG4ref or IGHG4a constant region . Optionally, the variable domain comprises mouse-pattern AID somatic mutations.

A polypeptide comprising (in N- to C- terminal direction) a leader sequence, a human variable domain that is specific for an antigen and a human constant region that is an IGHG1ref, IGHG2ref, IGHG2a, IGHG3ref, IGHG3a, IGHG3b, IGHG4ref or IGHG4a constant region wherein (i) the leader sequence is not the native human variable domain leader sequence; and/or (ii) the variable domain comprises mouse AID-pattern somatic mutations and/or mouse Terminal deoxynucleotidyl transferase (TdT)- pattern junctional mutations.

A nucleotide sequence encoding (in 5' to 3' direction) a leader sequence and a human antibody heavy chain, the heavy chain comprising a variable domain that is specific for an antigen and a constant region that is an IGHG1ref, IGHG2ref, IGHG2a, IGHG3ref, IGHG3a, IGHG3b, IGHG4ref or IGHG4a constant region; and the leader sequence being operable for expression of the heavy chain and wherein the leader sequence is not the native human variable domain leader sequence.

A nucleotide sequence encoding (in 5' to 3' direction) a promoter and a human antibody heavy chain, the heavy chain comprising a variable domain that is specific for an antigen and a constant region that is an IGHG1ref, IGHG2ref, IGHG2a, IGHG3ref, IGHG3a, IGHG3b, IGHG4ref or IGHG4a constant region ; and the promoter being operable for expression of the heavy chain and wherein the promoter is not the native human promoter.

A vector (eg, a CHO cell or HEK293 cell vector) comprising a IGHG1ref, IGHG2ref, IGHG2a, IGHG3ref, IGHG3a, IGHG3b, IGHG4ref or IGHG4a constant region nucleotide sequence that is 3' of a cloning site for the insertion of a human antibody heavy chain variable domain nucleotide sequence, such that upon insertion of such a variable domain sequence the vector comprises (in 5' to 3' direction) a promoter, a leader sequence, the variable domain sequence and the constant region sequence so that the vector is capable of expressing a human antibody heavy chain when present in a host cell.

### Sixth Configuration

A non-human vertebrate (eg, a mouse or rat) or a non-human vertebrate cell (eg, an ES cell or a B-cell) having a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein at least two of the human gene segments are variants that are not identical to each other.

A non-human vertebrate (eg, a mouse or rat) or a non-human vertebrate cell (eg, an ES cell or a B-cell) having a genome comprising at least 2 different non-endogenous variable region gene segments of the same type (eg, at least 2 human VH6-1 gene segments, at least 3 human JH6 gene segments, at least 2 human Vκ1-39 gene segments, at least 2 human D2-2 gene segments or at least 2 human Jκ1 gene segments) cis at the same Ig locus.

A non-human vertebrate (eg, a mouse or rat) or a non-human vertebrate cell (eg, an ES cell or a B-cell) having a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) *trans* at the same Ig locus; and optionally a third human gene segment of the same type, wherein the third gene segment is *cis* with one of said 2 different gene segments.

A population of non-human vertebrates (eg, mice or rats) comprising a repertoire of human variable region gene segments, wherein the plurality comprises at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), a first of said different gene segments is provided in the genome of a first vertebrate of the population, and a second of said different gene segments being provided in the genome of a second vertebrate of the population, wherein the genome of the first vertebrate does not comprise the second gene segment.

A non-human vertebrate (eg, a mouse or rat) or a non-human vertebrate cell (eg, an ES cell or a B-cell) having a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.

A method of enhancing the human immunoglobulin gene diversity of a non-human vertebrate (eg, a mouse or rat), the method comprising providing the vertebrate with a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein at least two of the human gene segments are variants that are not identical to each other.

A method of enhancing the immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) cis at the same Ig locus.

A method of enhancing the immunoglobulin gene diversity of a non-human vertebrate (eg, a mouse or rat), the method comprising providing the vertebrate with a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) *trans* at the same Ig locus; and optionally a third human gene segment of the same type, wherein the third gene segment is *cis* with one of said 2 different gene segments.

A method of providing an enhanced human immunoglobulin variable region gene segment repertoire, the method comprising providing a population of non-human vertebrates (eg, a mouse or rat) comprising a repertoire of human variable region gene segments, wherein the method comprises providing at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein a first of said different gene segments is provided in the genome of a first vertebrate of the population, and a second of said different gene segments is provided in the genome of a second vertebrate of the population, wherein the genome of the first vertebrate does not comprise the second gene segment.

A method of enhancing the human immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.

A method of enhancing the human immunoglobulin gene diversity of a non-human vertebrate (eg, a mouse or rat), the method comprising providing the vertebrate with a genome comprising at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations; optionally wherein at least 2 or 3 of said different gene segments are provided at the same Ig locus in said genome.

A non-human vertebrate (eg, a mouse or rat) or a non-human vertebrate cell (eg, an ES cell or a B-cell) having a genome comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 and 9 to 14 (eg, selected from Table 13 or Table 14) (eg, IGHJ6-a) and the second gene segment is the corresponding reference sequence.

A population of non-human vertebrates (eg, mice or rats) comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 and 9 to 14 (eg, selected from Table 13 or Table 14) (eg, IGHJ6-a) and the second gene segment is the corresponding reference sequence, wherein the first gene segment is provided in the genome of a first vertebrate of the population, and the second gene segment is provided in the genome of a second vertebrate of the population.

A method of enhancing the human immunoglobulin gene diversity of a non-human vertebrate (eg, a mouse or rat), the method comprising providing the vertebrate with a genome comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 and 9 to 14 (eg, selected from Table 13 or Table 14) (eg, IGHJ6-a) and the second gene segment is the corresponding reference sequence.

In one aspect of this configuration, the invention relates to human D gene segment variants as described further below.

In one aspect of this configuration, the invention relates to human V gene segment variants as described further below.

In one aspect of this configuration, the invention relates to human J gene segment variants as described further below.

### BRIEF DESCRIPTION OF THE FIGURES

Figures 1 to 3: Schematic illustrating a protocol for producing recombineered BAC vectors to add V gene segments into a mouse genome;
Figure 4: Schematic illustrating a protocol for adding V gene segments to a mouse genome using sequential recombinase mediated cassette exchange (sRMCE); and
Figure 5 (in 4 parts): Alignment of 13 IGHV1-69 variants showing the variable (V) coding region only. Nucleotides that differ from VH1-69 variant *01 are indicated at the appropriate position whereas identical nucleotides are marked with a dash. Where nucleotide changes result in amino acid differences, the encoded amino acid is shown above the corresponding triplet. Boxed regions correspond to CDR1, CDR2 and CDR3 as indicated.
Figure 6 is a schematic illustrating gene segment diversity and the effect of including variant variants in cis according to the invention:-
   (a) Situation in a normal person: Recombination on the same chromosome limits combinations of variants, for instance the antibody gene V4-4 can only be recombined within variant 1 to form for instance for instance V4-4-D-J6 or V4-4-D-J2^{A}. Similarly the variant V4-4^{A} can't be recombined with either J6 or J2^{A} from variant 1 and can only be joined with J-genes from variant 2 to form V4-4^{A}-D-J6^{A} and V4-4^{A}-D-J2. V4-4-J2/J6 complexity = 4.
   (b) Situation in a transgenic mouse: Only one variant is provided so the genome is limited. V4-4-J6/J2 complexity = 2.
   (c) Supra mouse of the invention: The variants are added in *cis* and thus can be recombined in every combination, expanding the repertoire. For instance V4-4 can be combined with J6A, J6, J2A or J2 and similarly V4-4A can be recombined with these same J-genes. The V4-4-J6/J2 complexity = 8, which in this simple example is double that of a person and 4X that of a mouse with a single variant.

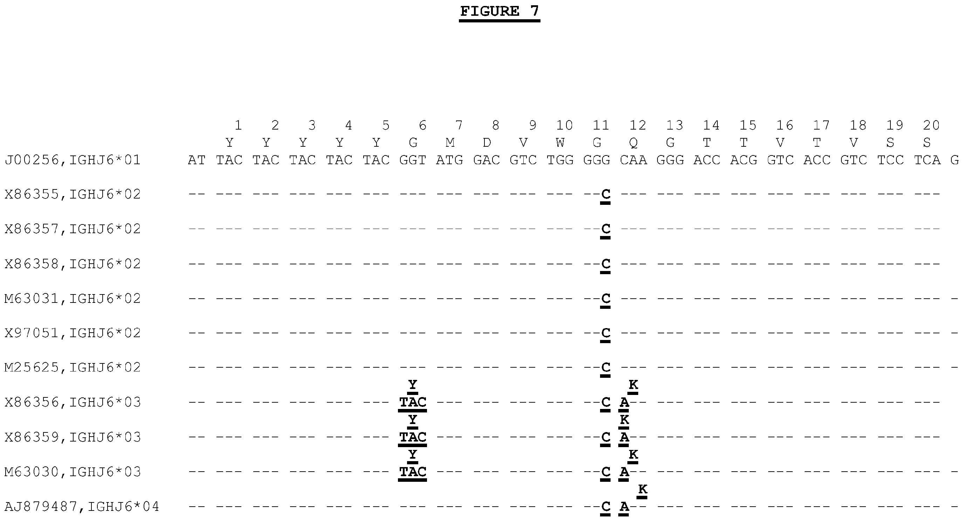

Figure 7: Alignment of human JH6*02 variants. Nucleotides that differ from JH6 *01 are indicated at the appropriate position whereas identical nucleotides are marked with a dash. Where nucleotide changes result in amino acid differences, the encoded amino acid is shown above. Accession numbers (eg, J00256) are shown to the left of the IMGT variant name.

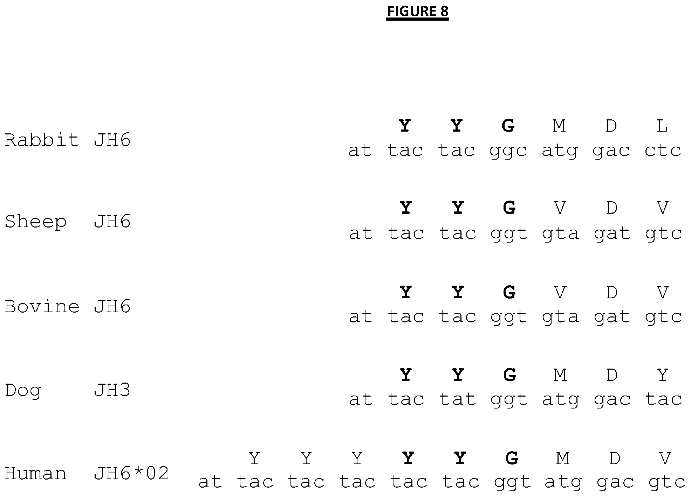

Figure 8: Alignment of JH sequences from various species.

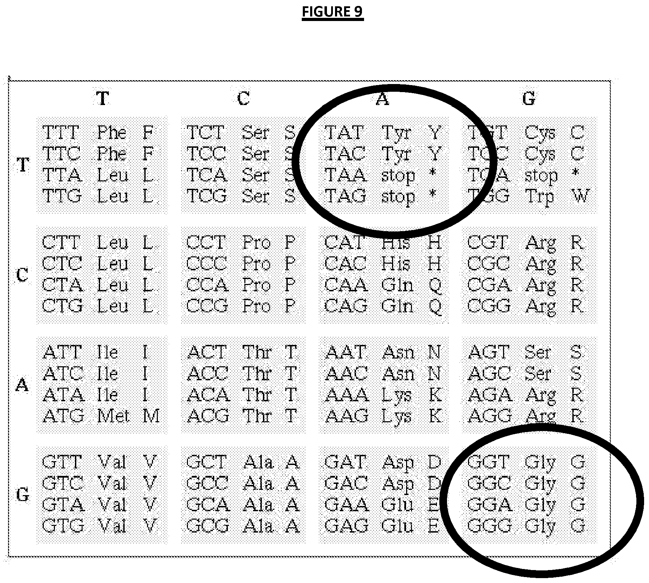

Figure 9: Codon Table

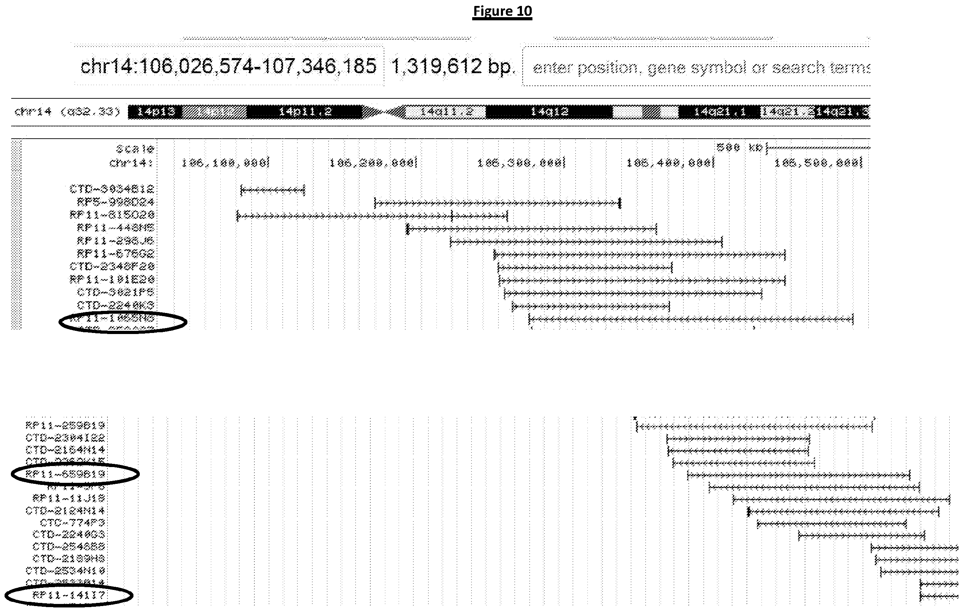

Figure 10: BAC database extract

### BRIEF DESCRIPTION OF THE TABLES

Table 1: Human IgH V Polymorphic Variants
Table 2: Human IgH D Polymorphic Variants
Table 3: Human IgH J Polymorphic Variants
Table 4: Human Ig Vk Polymorphic Variants
Table 5: Human Ig Vλ Polymorphic Variants
Table 6: Human IgH Jk Polymorphic Variants
Table 7: Human IgH Jλ Polymorphic Variants
Table 8: 1000 Genomes Project Human Populations
Table 9: Immunoglobulin Gene Usage in Human Antibody Responses to Infectious Disease Pathogens
Table 10A: Human IgH JH5 Variant Occurrences
Table 10B: Non-Synonymous Human IgH JH5 Variants
Table 11A: Human IgH JH6 Variant Occurrences
Table 11B: Non-Synonymous Human IgH JH6 Variants
Table 12A: Human IgH JH2 Variant Occurrences
Table 12B: Non-Synonymous Human IgH JH2 Variants
Table 13: Variant Frequency Analyses & Human Population Distributions
Table 14: Frequent Human Variant Distributions
Table 15: Human Gene Segment Usage: Heavy Chain Repertoires From Naive Non-Human Vertebrates
Table 16: Human Gene Segment Usage: Heavy Chain Repertoires From Immunised Non-Human Vertebrates
Table 17: Human Gene Segment Usage: Heavy Chain Repertoires From Antigen-Specific Hybridomas
Table 18: Sequence Correlation Table
Table 19: Summary Of Function Correlated With Human Gamma Constant Region Sub-Type
Table 20: Gene Segments Prevalent In Few Human Populations
Table 21: Genomic and sequence information

### DETAILED DESCRIPTION OF THE INVENTION

A suitable source of JH6*02 and other human DNA sequences for use in the invention will be readily apparent to the skilled person. For example, it is possible to collect a DNA sample from a consenting human donor (eg, a cheek swab sample as per the Example herein) from which can be obtained suitable DNA sequences for use in constructing a locus of the invention. Other sources of human DNA are commercially available, as will be known to the skilled person. Alternatively, the skilled person is able to construct gene segment sequence by referring to one or more databases of human Ig gene segment sequences disclosed herein.

An example source for human V, D and J gene segments according to the invention are Bacterial Artificial Chromosomes (RPCI-11 BACs) obtained from Roswell Park Cancer Institute (RPCI)/Invitrogen. See http://bacpac.chori.org/hmale11.htm, which describes the BACs as follows:-

### "RPCI - 11 Human Male BAC Library

*The RPCI-11 Human Male BAC Library (Osoegawa et al., 2001) was constructed using improved cloning techniques (Osoegawa et al., 1998) developed by Kazutoyo Osoegawa. The library was generated by Kazutoyo Osoegawa. Construction was funded by a grant from the National Human Genome Research Institute (NHGRI, NIH) (#1R01RG01165-03). This library was generated according to the new NHGRI*/*DOE "Guidance on Human Subjects in Large-Scale DNA Sequencing...*

*"Male blood was obtained via a double-blind selection protocol. Male blood DNA was isolated from one randomly chosen donor (out of 10 male donors)".*
- Osoegawa K, Mammoser AG, Wu C, Frengen E, Zeng C, Catanese JJ, de Jong PJ; Genome Res. 2001 Mar;11(3):483-96; "A bacterial artificial chromosome library for sequencing the complete human genome";
- Osoegawa, K., Woon, P.Y., Zhao, B., Frengen, E., Tateno, M., Catanese, J.J, and de Jong, P.J. (1998); "An Improved Approach for Construction of Bacterial Artificial Chromosome Libraries"; Genomics 52, 1-8.

### Superhuman Immunoglobulin Gene Repertoires

The invention relates to synthetically-extended & ethnically-diverse superhuman immunoglobulin gene repertoires. The human immunoglobulin repertoires are beyond those found in nature (ie, "Superhuman"), for example, they are more diverse than a natural human repertoire or they comprise combinations of human immunoglobulin gene segments from disparate sources in a way that is non-natural. Thus, the repertoires of the invention are "superhuman" immunoglobulin repertoires, and the invention relates to the application of these in transgenic cells and non-human vertebrates for utility in producing chimaeric antibodies (with the possibility of converting these into fully-human, isolated antibodies using recombinant DNA technology). The present invention thus provides for novel and potentially expanded synthetic immunoglobulin diversities, which provides for a pool of diversity from which antibody therapeutic leads (antibody therapeutics and antibody tool reagents) can be selected. This opens up the potential of transgenic human-mouse/rat technologies to the possibility of interrogating different and possibly larger antibody sequence-spaces than has hitherto been possible. To this end, in one embodiment, the invention provides a SUPERHUMAN MOUSE™ (aka SUPRA-MOUSE™) and a SUPERHUMAN RAT™ (aka SUPRA-RAT™) In developing this thinking, the present inventors have realised the possibility of mining the huge genetics resources now available to the skilled person thanks to efforts such as the HapMap Project, 1000 Genomes Project and sundry other immunoglobulin gene databases (see below for more details). Thus, in some embodiments, the inventors realised the application of these genome sequencing developments in the present invention to generate synthetically-produced and ethnically-diverse artificial immunoglobulin gene repertoires. In one aspect, the inventors realised that such repertoires are useful for the production of antibodies having improved affinity and/or biophysical characteristics, and/or wherein the range of epitope specificities produced by means of such repertoire is novel, provides for antibodies to epitopes that have hitherto been intractable by prior transgenic immunoglobulin loci or difficult to address.

The present invention provides libraries, vertebrates and cells, such as transgenic mice or rats or transgenic mouse or rat cells. Furthermore, the invention relates to methods of using the vertebrates to isolate antibodies or nucleotide sequences encoding antibodies. Antibodies, nucleotide sequences, pharmaceutical compositions and uses are also provided by the invention.

### Variation Analysis

The present inventors have realized methods and antibody loci designs that harness the power of genetic variation analysis. The reference human genome provides a foundation for experimental work and genetic analysis of human samples. The reference human is a compilation of the genomes from a small number of individuals and for any one segment of the genome a high quality single reference genome for one of the two chromosomes is available. Because the reference genome was assembled from a series of very large insert clones, the identity of these clones is known. Accordingly, experimental work with human genomic DNA is usually conducted on the clones from which the reference sequence was derived.

Individual humans differ in their sequence and recently several individuals have had their genomes sequenced, for instance James Watson and Craig Venter. Comparison of the genome sequence of these individuals has revealed differences between their sequences and the reference genome in both coding and non-coding parts of the genome, approximately 1 in 1000 bases are different. Some variants will be significant and contribute to differences between individuals. In extreme cases these will result in genetic disease. Variation can be implicated in differing responses to drugs administered to human patients, eg, yielding an undesirable lowering of patient response to treatment.

The 1000-Genomes Project has the objective of identifying the most frequent variations in the human genome. This public domain project involved sequencing the genomes of more than 1000 individuals from diverse ethnic groups, comparing these sequences to the reference and assembling a catalogue of variants. This has enabled the annotation of variants in coding regions, but because this sequence wasn't derived from large clones of DNA, the analysis of the sequence from diploid individuals can't discriminate the distribution of the variation between the maternal and paternally inherited chromosomes. Where more than one variant is identified in a protein coding gene, it is not possible to illuminate the distribution of the pattern of variants in each version of the protein. For example, if two variants are detected in different positions of the same protein in an individual, this could have resulted from one copy with two variants and none in the other or each copy could have just one variant. To illuminate the sequence of real proteins, the 1000-Genome Project has sequenced mother-father-child trios. This allows one to "phase" the sequence variants, in other words identify blocks of sequence that are inherited from one or other parent and deconvolute the variants.

To further understand the variation within the 1000-genome set a tool has been developed that can identify the significant variants (defined as non-synonymous amino acid changes) from a region of DNA from the phased data in the 1000-genome data set. This tool has been made available online http://www.1000genomes.org/variation-pattern-finder. This tool allows an investigator to download non-synonymous variation delimited between specific coordinates. The downloaded files are configured as individual genotypes, but the data is phased so the haplotype information and the frequencies of specific halotypes in different populations can be extracted.

The inventors' analysis of the 1000-genome data for the individual human coding segments of the C, V D and J genes from the heavy and light chains reveals that there is significant variation in these segments. Individuals will usually have two different heavy chain alleles and also different light chain alleles at both kappa and lambda loci. The repertoire of antibodies that can be generated from each allele will be different. This variation will contribute to a better or differing immune response to certain antigens.

Humanized mice that have hitherto been generated with immunoglobulin heavy and light chain loci contain just one type of immunoglobulin locus. Even if these mice contain a full human heavy chain locus, the variation will be less than contained in a typical human because only one set of C, V, D and J genes are available, while a typical human would have two sets.

The inventors have devised ways to improve on this limitation when constructing transgenic non-human vertebrates and cells for human antibody and variable region production *in vivo.*

Mice can be generated with two different loci, each engineered to have a different repertoire of V, D and J segments. This could be in a single mouse or two or more separate mouse strains and would be analogous to or beyond the repertoire found in a normal human. The engineering of such a mouse would go beyond the repertoire described in humanized mice to date which only have one set of alleles.

However, the inventors also realized that this also has limitations, because the different loci would not normally interact to shuffle V, D and J variants between loci. This same limitation is also inherent in a human, thus this system does not utilize the advantage of recombining variants in all combinations.

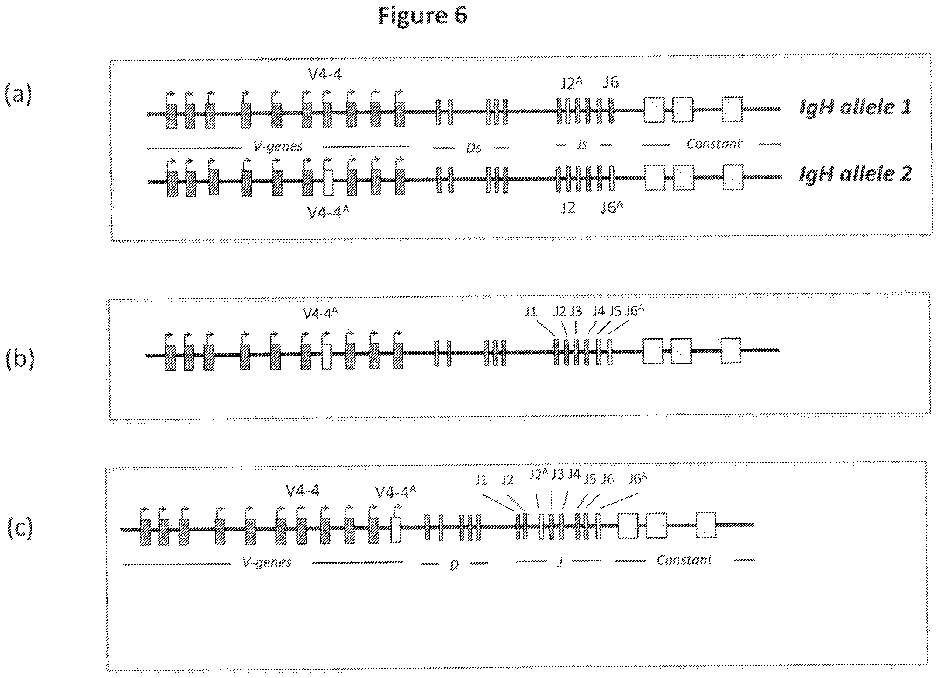

To go beyond the normal repertoire in humans and take advantage of combinations of C, V, D and J variants the inventors decided, in one embodiment, to provide these on the same chromosome in *cis.* See figure 6. These loci would be characterized by having more than the normal number of J, D or V genes. For example n=6 for the J genes, but including one J6 variant and one J2 variant would increase this to n=8. This could be combined with additional variants for the D and V genes, for example. By detailed analysis of the 1000- Genomes database, the inventors have devised a collection of candidate polymorphic human variant gene segments, eg, JH gene segments (eg, see the examples), that can be built into the design of transgenic heavy and light chain loci in mice for expressing increasingly diverse and new, synthetic repertoires of human variable regions. Moreover, by utilizing naturally-occurring human variant gene segments, as per embodiments of the invention, this addresses compatibility with human patients since the inventor's analysis has drawn out candidate variants that are naturally conserved and sometimes very prevalent amongst human ethnic populations. Additionally this enables one to tailor the configurations of the invention to provide for antibody-based drugs that better address specific human ethnic populations.

In an example according to any configuration of the invention, loci (and cells and vertebrates comprising these) are provided in which gene segments from different human populations are used. This is desirable to increase antibody gene diversity to better address more diverse human patients. In an example, the gene segments are from first and second different human populations respectively, and thus the second gene segment is found in the second human population, but not so (or rarely) in the first human population. Rarely means, for example, that the gene segment is found in 5, 4, 3, 2, or 1 or zero individuals in the first population in the 1000 Genomes database. For example, the first gene segment may be shown as present in a first population by reference to Table 13 or 14 herein, the second gene segment may be shown as present in the second population by reference to Table 13 and not in the first population. Optionally, the first gene segment may also be shown as being present in the second population by reference to Table 13 or 14.

In any configuration or aspect of the invention, where a V gene segment is used, this may be used optionally with the native leader sequence. For example, use of genomic DNA (eg, from BACs as in the examples) will mean that the native leader will be used for each V gene segment incorporated into the locus and genomes of the invention. In an alternative, the skilled person may wish to inert a non-native leader sequence together with one or more of the V gene segments. Similarly, in any configuration or aspect of the invention, where a V gene segment is used, this may be used optionally with the native 5' UTR sequence. For example, use of genomic DNA (eg, from BACs as in the examples) will mean that the native 5' UTR sequence will be used for each V gene segment incorporated into the locus and genomes of the invention. In an alternative, the skilled person may wish to exclude the native 5' UTR sequence.

### The present invention provides, in a first configuration

### (a) Superhuman heavy chain gene repertoires

A non-human vertebrate or vertebrate cell (optionally an ES cell or antibody-producing cell) comprising a genome having a superhuman immunoglobulin heavy chain human VH and/or D and/or J gene repertoire.

In one aspect the cell of the invention is an embryonic stem cell. For example, the ES cell is derived from the mouse C57BL/6N, C57BL/6J, 129S5 or 129Sv strain. In one aspect the non-human vertebrate is a rodent, suitably a mouse, and cells of the invention, are rodent cells or ES cells, suitably mouse ES cells. The ES cells of the present invention can be used to generate animals using techniques well known in the art, which comprise injection of the ES cell into a blastocyst followed by implantation of chimaeric blastocystys into females to produce offspring which can be bred and selected for homozygous recombinants having the required insertion. In one aspect the invention relates to a transgenic animal comprised of ES cell-derived tissue and host embryo derived tissue. In one aspect the invention relates to genetically-altered subsequent generation animals, which include animals having a homozygous recombinants for the VDJ and/or VJ regions.

The natural human immunoglobulin gene segment repertoire consists of (see eg, www.imgt.org ):-
VH: total-125 ; functional-41
DH: total-27; functional-23
JH: total-8; functional-6
Vk: total-77; functional-38
Jk: total-5; functional-5
V lambda: total-75; functional-31
J lambda: total-7; functional-5

In one embodiment, the vertebrate or cell genome comprises a transgenic immunoglobulin heavy chain locus comprising a plurality of human immunoglobulin VH gene segments, one or more human D gene segments and one or more human J gene segments, wherein the plurality of VH gene segments consists of more than the natural human repertoire of functional VH gene segments; optionally wherein the genome is homozygous for said transgenic heavy chain locus.

In one embodiment of the vertebrate or cell, the VH gene repertoire consists of a plurality of VH gene segments derived from the genome sequence of a first human individual, supplemented with one or more different VH gene segments derived from the genome sequence of a second, different human individual. Optionally the D and J segments are derived from the genome sequence of the first human individual. Optionally the VH gene segments from the genome sequence of the second individual are selected from the VH gene segments listed in Table 1, 13 or 14. In this way, the locus provides a superhuman repertoire of D gene segments.

Optionally the individuals are not related. Individuals are "not related" in the context of any configuration or aspect of the invention, for example, if one of the individuals does not appear in a family tree of the other individual in the same generation or going back one, two, three or four generations. Alternatively, are not related, for example, if they do not share a common ancestor in the present generation or going back one, two, three or four generations.

In one embodiment of the vertebrate or cell, the transgenic locus comprises more than 41 functional human VH gene segment species, and thus more than the natural human functional repertoire. Optionally the locus comprises at least 42, 43, 44, 45, 46, 47, 48, 49 or 50 functional human VH gene segment species (eg, wherein the locus comprises the full functional VH repertoire of said first individual supplemented with one or more VH gene segments derived from the genome sequence of the second human individual and optionally with one or more VH gene segments derived from the genome sequence of a third human individual). In this way, the locus provides a superhuman repertoire of VH gene segments that is useful for generating a novel gene and antibody diversity for use in therapeutic and tool antibody selection.

In one embodiment of the vertebrate or cell, the transgenic locus comprises a first VH gene segment derived from the genome sequence of the first individual and a second VH gene segment derived from the genome sequence of the second individual, wherein the second VH gene segment is a polymorphic variant of the first VH gene segment. For example, the VH gene segments are polymorphic variants of VH1-69 as illustrated in the examples below. Optionally the locus comprises a further polymorphic variant of the first VH gene segment (eg, a variant derived from the genome sequence of a third human individual). In this way, the locus provides a superhuman repertoire of VH gene segments.

In one embodiment of the vertebrate or cell, the genome (alternatively or additionally to the superhuman VH diversity) comprises a transgenic immunoglobulin heavy chain locus comprising a plurality of human immunoglobulin VH gene segments, a plurality of human D gene segments and one or more human J gene segments, wherein the plurality of D gene segments consists of more than the natural human repertoire of functional D gene segments. Optionally the genome is homozygous for said transgenic heavy chain locus.

In one embodiment of the vertebrate or cell, the D gene repertoire consists of a plurality of D gene segments derived from the genome sequence of a (or said) first human individual, supplemented with one or more different D gene segments derived from the genome sequence of a (or said) second, different human individual. Optionally the individuals are not related. Optionally the J segments are derived from the genome sequence of the first human individual. Optionally the D gene segments from the genome sequence of the second individual are selected from the D gene segments listed in Table 2, 13 or 14. In this way, the locus provides a superhuman repertoire of D gene segments.

In one embodiment of the vertebrate or cell, the transgenic locus comprises more than 23 functional human D gene segment species; optionally wherein the locus comprises at least 24, 25, 26, 27, 28, 29, 30 or 31 functional human D gene segment species (eg, wherein the locus comprises the full functional D repertoire of said first individual supplemented with one or more D gene segments derived from the genome sequence of the second human individual and optionally with one or more D gene segments derived from the genome sequence of a third human individual). In this way, the locus provides a superhuman repertoire of D gene segments.

In one embodiment of the vertebrate or cell, the transgenic locus comprises a first D gene segment derived from the genome sequence of the first individual and a second D gene segment derived from the genome sequence of the second individual, wherein the second D gene segment is a polymorphic variant of the first D gene segment. Optionally the locus comprises a further polymorphic variant of the first D gene segment (eg, a variant derived from the genome sequence of a third human individual). In this way, the locus provides a superhuman repertoire of D gene segments.

In one embodiment of the vertebrate or cell (alternatively or additionally to the superhuman VH and/or JH diversity), the genome comprises a (or said) transgenic immunoglobulin heavy chain locus comprising a plurality of human immunoglobulin VH gene segments, one or more human D gene segments and a plurality of human JH gene segments, wherein the plurality of J gene segments consists of more than the natural human repertoire of functional J gene segments; optionally wherein the genome is homozygous for said transgenic heavy chain locus.

In one embodiment of the vertebrate or cell, the JH gene repertoire consists of a plurality of J gene segments derived from the genome sequence of a (or said) first human individual, supplemented with one or more different J gene segments derived from the genome sequence of a (or said) second, different human individual. Optionally the individuals are not related. Optionally D segments are derived from the genome sequence of the first human individual. Optionally the J gene segments from the genome sequence of the second individual are selected from the J gene segments listed in Table 3 13 or 14. In this way, the locus provides a superhuman repertoire of JH gene segments.

In one embodiment of the vertebrate or cell, the transgenic locus comprises more than 6 functional human JH gene segment segments. Optionally the locus comprises at least 7, 8, 9, 10, 11, 12, 13, 14, 15, or 16 functional human JH gene segments (eg, wherein the locus comprises the full functional JH repertoire of said first individual supplemented with one or more JH gene segments derived from the genome sequence of the second human individual and optionally with one or more JH gene segments derived from the genome sequence of a third human individual). In this way, the locus provides a superhuman repertoire of JH gene segments.

In one embodiment of the vertebrate or cell, the transgenic locus comprises a first JH gene segment derived from the genome sequence of the first individual and a second JH gene segment derived from the genome sequence of the second individual, wherein the second JH gene segment is a polymorphic variant of the first JH gene segment. Optionally the locus comprises a further polymorphic variant of the first JH gene segment (eg, a variant derived from the genome sequence of a third human individual). In this way, the locus provides a superhuman repertoire of JH gene segments.

### (b) Superhuman light chain gene repertoires

The first configuration of the invention also provides:-
A non-human vertebrate or vertebrate cell (optionally an ES cell or antibody-producing cell) comprising a genome having a superhuman immunoglobulin light chain human VL gene repertoire. Optionally the vertebrate or cell comprises a heavy chain transgene according to aspect (a) of the first configuration. Thus, superhuman diversity is provided in both the heavy and light chain immunoglobulin gene segments in the cell and vertebrate. For example, the genome of the cell or vertebrate is homozygous for the heavy and light chain transgenes and endogenous antibody expression is inactivated. Such a vertebrate is useful for immunisation with a predetermined antigen to produce one or more selected antibodies that bind the antigen and have human variable regions resulting from recombination within the superhuman gene segment repertoire. This provides potentially for a novel antibody and gene sequence space from which to select therapeutic, prophylactic and tool antibodies.

In one embodiment of aspect (b) of the first configuration, the vertebrate or cell genome comprises
( i) a transgenic immunoglobulin kappa light chain locus comprising a plurality of human immunoglobulin Vκ gene segments and one or more human J gene segments, wherein the plurality of Vκ gene segments consists of more than the natural human repertoire of functional Vκ gene segments; optionally wherein the genome is homozygous for said transgenic kappa light chain locus; and/or
( ii) a transgenic immunoglobulin lambda light chain locus comprising a plurality of human immunoglobulin Vλ gene segments and one or more human J gene segments, wherein the plurality of Vλ gene segments consists of more than the natural human repertoire of functional Vλ gene segments; optionally wherein the genome is homozygous for said transgenic lambda light chain locus.

In this way, the locus provides a superhuman repertoire of VL gene segments.

In one embodiment of the vertebrate or cell,
(i) the Vκ gene repertoire consists of a plurality of Vκ gene segments derived from the genome sequence of a first human individual, supplemented with one or more Vκ gene segments derived from the genome sequence of a second, different human individual; optionally wherein the individuals are not related; optionally wherein the J segments are derived from the genome sequence of the first human individual; and optionally wherein the Vκ gene segments from the genome sequence of the second individual are selected from the Vκ gene segments listed in Table 4, 13 or 14; and
(i) the Vλ gene repertoire consists of a plurality of Vλ gene segments derived from the genome sequence of a first human individual, supplemented with one or more Vλ gene segments derived from the genome sequence of a second, different human individual; optionally wherein the individuals are not related; optionally wherein the J segments are derived from the genome sequence of the first human individual; and optionally wherein the Vλ gene segments from the genome sequence of the second individual are selected from the Vλ gene segments listed in Table 5, 13 or 14.

In this way, the locus provides a superhuman repertoire of VL gene segments.

In one embodiment of the vertebrate or cell,
- the kappa light transgenic locus comprises more than 38 functional human Vκ gene segment species; optionally wherein the locus comprises at least 39, 40, 41, 42, 43, 44, 45, 46, 47 or 48 functional human Vκ gene segment species (eg, wherein the locus comprises the full functional Vκ repertoire of said first individual supplemented with one or more Vκ gene segments derived from the genome sequence of the second human individual and optionally with one or more Vκ gene segments derived from the genome sequence of a third human individual); and
- the lambda light transgenic locus comprises more than 31 functional human Vλ gene segment species; optionally wherein the locus comprises at least 32, 33, 34, 35, 36, 37, 38, 39, 40 or 41 functional human Vλ gene segment species (eg, wherein the locus comprises the full functional Vλ repertoire of said first individual supplemented with one or more Vλ gene segments derived from the genome sequence of the second human individual and optionally with one or more Vλ gene segments derived from the genome sequence of a third human individual).

In this way, the locus provides a superhuman repertoire of VL gene segments.

In one embodiment of the vertebrate or cell,
- the kappa light transgenic locus comprises a first Vκ gene segment derived from the genome sequence of the first individual and a second Vκ gene segment derived from the genome sequence of the second individual, wherein the second Vκ gene segment is a polymorphic variant of the first Vκ gene segment; optionally wherein the locus comprises a further polymorphic variant of the first Vκ gene segment (eg, a variant derived from the genome sequence of a third human individual); and
- the lambda light transgenic locus comprises a first Vλ gene segment derived from the genome sequence of the first individual and a second Vλ gene segment derived from the genome sequence of the second individual, wherein the second Vλ gene segment is a polymorphic variant of the first Vλ gene segment; optionally wherein the locus comprises a further polymorphic variant of the first Vλ gene segment (eg, a variant derived from the genome sequence of a third human individual).

In this way, the locus provides a superhuman repertoire of VL gene segments.

In one embodiment of the vertebrate or cell, the genome comprises a (or said) transgenic immunoglobulin light chain locus comprising a plurality of human immunoglobulin VL gene segments and a plurality of human JL gene segments, wherein the plurality of J gene segments consists of more than the natural human repertoire of functional J gene segments; optionally wherein the genome is homozygous for said transgenic heavy chain locus.

In one embodiment of the vertebrate or cell,
(i) the Jκ gene repertoire consists of a plurality of Jκ gene segments derived from the genome sequence of a (or said) first human individual, supplemented with one or more Jκ gene segments derived from the genome sequence of a (or said) second, different human individual; optionally wherein the individuals are not related; optionally wherein the Vκ segments are derived from the genome sequence of the first human individual; optionally wherein the Jκ gene segments from the genome sequence of the second individual are selected from the Jκ gene segments listed in Table 6, 13 or 14; and
(ii) the Jκ gene repertoire consists of a plurality of Jλ gene segments derived from the genome sequence of a (or said) first human individual, supplemented with one or more Jλ gene segments derived from the genome sequence of a (or said) second, different human individual; optionally wherein the individuals are not related; optionally wherein the Vλ segments are derived from the genome sequence of the first human individual; optionally wherein the Jλ gene segments from the genome sequence of the second individual are selected from the Jλ gene segments listed in Table 7, 13 or 14.

In this way, the locus provides a superhuman repertoire of JL gene segments.

In one embodiment of the vertebrate or cell,
(i) the transgenic light chain locus comprises more than 5 functional human Jκ gene segment species; optionally wherein the locus comprises at least 6, 7, 8, 9, 10, 11, 12, 13, 14 or 15 functional human Jκ gene segment species (eg, wherein the locus comprises the full functional Jκ repertoire of said first individual supplemented with one or more Jκ gene segments derived from the genome sequence of the second human individual and optionally with one or more Jκ gene segments derived from the genome sequence of a third human individual); and/or
(i) the transgenic light chain locus comprises more than 5 functional human Jλ gene segment species; optionally wherein the locus comprises at least 6, 7, 8, 9, 10, 11, 12, 13, 14 or 15 functional human Jλ gene segment species (eg, wherein the locus comprises the full functional Jλ repertoire of said first individual supplemented with one or more Jλ gene segments derived from the genome sequence of the second human individual and optionally with one or more Jλ gene segments derived from the genome sequence of a third human individual).

In this way, the locus provides a superhuman repertoire of JL gene segments.

In one embodiment of the vertebrate or cell, (i) the kappa light transgenic locus comprises a first Jκ gene segment derived from the genome sequence of the first individual and a second Jκ gene segment derived from the genome sequence of the second individual, wherein the second Jκ gene segment is a polymorphic variant of the first Jκ gene segment; optionally wherein the locus comprises a further polymorphic variant of the first Jκ gene segment (eg, a variant derived from the genome sequence of a third human individual); and (ii) the lambda light transgenic locus comprises a first Jλ gene segment derived from the genome sequence of the first individual and a second Jλ gene segment derived from the genome sequence of the second individual, wherein the second Jκ gene segment is a polymorphic variant of the first Jλ gene segment; optionally wherein the locus comprises a further polymorphic variant of the first Jλ gene segment (eg, a variant derived from the genome sequence of a third human individual).

In this way, the locus provides a superhuman repertoire of JL gene segments.

Further aspects of the first configuration are described below.

### The present invention provides, in a second configuration

A library of antibody-producing transgenic cells whose genomes collectively encode a repertoire of antibodies, wherein
(a) a first transgenic cell expresses a first antibody having a chain (eg, heavy chain) encoded by a first immunoglobulin gene, the gene comprising a first variable domain nucleotide sequence produced following recombination of a first human unrearranged immunoglobulin gene segment (eg, a VH);
(b) a second transgenic cell expresses a second antibody having a chain (eg, a heavy chain) encoded by a second immunoglobulin gene, the second gene comprising a second variable domain nucleotide sequence produced following recombination of a second human unrearranged immunoglobulin gene segment (eg, a VH), the first and second antibodies being non-identical;
(c) the first and second gene segments are different and derived from the genome sequences of first and second human individuals respectively, wherein the individuals are different; and optionally not related;
(d) wherein the cells are non-human vertebrate (eg, mouse or rat) cells (eg, B-cells or hybridomas).

In one embodiment, the library is provided *in vitro.* In another embodiment, the library is provided *in vivo* by one or a plurality of transgenic non-human vertebrates. For example, the or each vertebrate is according to any aspect of the first configuration of the invention.

In one embodiment, the library encodes an antibody repertoire of from 10 to 10⁹ antibodies, for example, 10, 20, 30, 40, 50, 100 or 1000 to 10⁸; or 10, 20, 30, 40, 50, 100 or 1000 to 10⁷; or 10, 20, 30, 40, 50, 100 or 1000 to 10⁶; or 10, 20, 30, 40, 50, 100 or 1000 to 10⁵; or 10, 20, 30, 40, 50, 100 or 1000 to 10⁴ antibodies. In an example, library encodes an antibody repertoire of at least 10³, 10⁴, 10⁵, 10⁶, 10⁷, 10⁸, 10^{9,} or 10¹⁰ antibodies.

The first variable domain nucleotide sequence is produced following recombination of the first human unrearranged immunoglobulin gene segment with one or more other immunoglobulin gene segments (for example, human immunoglobulin gene segments). For example, where the first gene segment is a VH, the first variable domain nucleotide sequence (a VH domain) is produced following recombination of the VH with a human D and JH segments *in vivo,* optionally with somatic hypermutation, in the first transgenic cell or an ancestor thereof. For example, where the first gene segment is a VL, the first variable domain nucleotide sequence (a VL domain) is produced following recombination of the VL with a human JL segment *in vivo,* optionally with somatic hypermutation, in the first transgenic cell or an ancestor thereof.

The second variable domain nucleotide sequence is produced following recombination of the second human unrearranged immunoglobulin gene segment with one or more other immunoglobulin gene segments (for example, human immunoglobulin gene segments). For example, where the second gene segment is a VH, the second variable domain nucleotide sequence (a VH domain) is produced following recombination of the VH with a human D and JH segments *in vivo,* optionally with somatic hypermutation, in the second transgenic cell or an ancestor thereof. For example, where the second gene segment is a VL, the second variable domain nucleotide sequence (a VL domain) is produced following recombination of the VL with a human JL segment *in vivo,* optionally with somatic hypermutation, in the second transgenic cell or an ancestor thereof.

The first and second gene segments are respectively derived from genome sequences of first and second human individuals. In one example, such a gene segment is isolated or cloned from a sample cell taken from said individual using standard molecular biology techniques as know to the skilled person. The sequence of the gene segment may be mutated (eg, by the introduction of up to 5, 6, 7, 8, 9, 10, 11, 12, 13, 14 or 15 nucleotide changes) prior to use in the present invention. In another example, a gene segment is derived by identifying a candidate human immunoglobulin gene segment in a database (see guidance below) and a nucleotide sequence encoding a gene segment for use in the present invention is made by reference (eg, to be identical or a mutant with up to 5, 6, 7, 8, 9, 10, 11, 12, 13, 14 or 15 nucleotide changes to the reference sequence) to the database sequence. The skilled person will be aware of methods of obtaining nucleotide sequences by reference to databases or by obtaining from cellular samples.

In one embodiment of the vertebrate, cell or library of any configuration of the invention, the first and second human individuals are members of first and second ethnic populations respectively, wherein the populations are different. This, therefore, provides for superhuman gene diversity in transgenic loci, cells and vertebrates as per the invention.

### Human Populations

Optionally the ethnic populations are selected from those identified in the 1000 Genomes Project of database. In this respect, see Table 8 which provides details of the ethnic populations on which the 1000 Genomes database is based.

N A Rosenberg et al (Science 20 December 2002: vol. 298 no. 5602 2342-2343) studied the genetic structure of human populations of differing geographical ancestry. In total, 52 populations were sampled, these being populations with:
*African ancestry*
   (Mbuti Pygmies, Biaka Pygmies, San peoples, and speakers of Niger-Kordofanian languages (Bantu, Yoruba or Mandenka populations),
*Eurasian ancestry*
   (European ancestry (Orcadian, Adygel, Basque, French, Russians, Italians, Sardinian, Tuscan), Middle Eastern ancestry (Mozabite, Bedouin, Druze, Palestinians),
   Central/South Asian ancestry (Balochl, Brahul, Makrani, Sindhi, Pathan, Burusho, Hazara, Uygur, Kalash)),
*East Asian ancestry*
   (Han, Dal, Daur, Hezhen, Lahu, Miao, Oroqen, She, Tujia, Tu, Xibo, Yi, Mongola, Naxi, Cambodian, Japanese, Yakut), Oceanic ancestry (Melanesian, Papuan); or
*Americas ancestry*
   (Karitiana, Surui, Colombian, Maya, Pima).

The International HapMap Project, Nature, 2003 Dec 18;426(6968):789-96, discloses that goal of the HapMap Project: to determine the common patterns of DNA sequence variation in the human genome by determining the genotypes of one million or more sequence variants, their frequencies and the degree of association between them in DNA samples from populations with ancestry from parts of Africa, Asia and Europe. The relevant human populations of differing geographical ancestry include Yoruba, Japanese, Chinese, Northern European and Western European populations. More specifically:-
Utah population with Northern or Western European ancestry (samples collected in 1980 by the Centre d'Etude du Polymorphisme Humain (CEPH));
population with ancestry of Yoruba people from Ibadan, Nigeria;
population with Japanese ancestry; and
population with ancestry of Han Chinese from China.

The authors, citing earlier publications, suggest that ancestral geography is a reasonable basis for sampling human populations.

A suitable sample of human populations from which the populations used in the present invention are selected is as follows:-
(a) *European ancestry*
(b) Northern European ancestry; Western European ancestry; Toscani ancestry; British ancestry, Finnish ancestry or Iberian ancestry.
(c) More specifically, population of Utah residents with Northern and/or Western European ancestry; Toscani population in Italia; British population in England and/or Scotland; Finnish population in Finland; or Iberian population in Spain.

(a) *East Asian ancestry*
(b) Japanese ancestry; Chinese ancestry or Vietnamese ancestry.
(c) More specifically, Japanese population in Toyko, Japan; Han Chinese population in Beijing, China; Chinese Dai population in Xishuangbanna; Kinh population in Ho Chi Minh City, Vietnam; or Chinese population in Denver, Colorado, USA.

(a) *West African ancestry*
(b) Yoruba ancestry; Luhya ancestry; Gambian ancestry; or Malawian ancestry.
(c) More specifically, Yoruba population in Ibadan, Nigeria; Luhya population in Webuye, Kenya; Gambian population in Western Division, The Gambia; or Malawian population in Blantyre, Malawi.

*(a) Population of The Americas*
(b) Native American ancestry; Afro-Caribbean ancestry; Mexican ancestry; Puerto Rican ancestry; Columbian ancestry; or Peruvian ancestry.
(c) More specifically, population of African Ancestry in Southwest US; population of African American in Jackson, MS; population of African Caribbean in Barbados; population of Mexican Ancestry in Los Angeles, CA; population of Puerto Rican in Puerto Rico; population of Colombian in Medellin, Colombia; or population of Peruvian in Lima, Peru.

*(a) South Asian ancestry*
(b) Ahom ancestry; Kayadtha ancestry; Reddy ancestry; Maratha; or Punjabi ancestry.
(c) More specifically, Ahom population in the State of Assam, India; Kayadtha population in Calcutta, India; Reddy population in Hyderabad, India; Maratha population in Bombay, India; or Punjabi population in Lahore, Pakistan.

In any configuration of the invention, in one embodiment, each human population is selected from a population marked "(a)" above.

In any configuration of the invention, in another embodiment, each human population is selected from a population marked "(b)" above.

In any configuration of the invention, in another embodiment, each human population is selected from a population marked "(c)" above.

In one embodiment of the library of the vertebrate, cell or library of the invention, the first and second ethnic populations are selected from the group consisting of an ethnic population with European ancestry, an ethnic population with East Asian, an ethnic population with West African ancestry, an ethnic population with Americas ancestry and an ethnic population with South Asian ancestry.

In one embodiment of the library of the vertebrate, cell or library of the invention, the first and second ethnic populations are selected from the group consisting of an ethnic population with Northern European ancestry; or an ethnic population with Western European ancestry; or an ethnic population with Toscani ancestry; or an ethnic population with British ancestry; or an ethnic population with Icelandic ancestry; or an ethnic population with Finnish ancestry; or an ethnic population with Iberian ancestry; or an ethnic population with Japanese ancestry; or an ethnic population with Chinese ancestry; or an ethnic population Vietnamese ancestry; or an ethnic population with Yoruba ancestry; or an ethnic population with Luhya ancestry; or an ethnic population with Gambian ancestry; or an ethnic population with Malawian ancestry; or an ethnic population with Native American ancestry; or an ethnic population with Afro-Caribbean ancestry; or an ethnic population with Mexican ancestry; or an ethnic population with Puerto Rican ancestry; or an ethnic population with Columbian ancestry; or an ethnic population with Peruvian ancestry; or an ethnic population with Ahom ancestry; or an ethnic population with Kayadtha ancestry; or an ethnic population with Reddy ancestry; or an ethnic population with Maratha; or an ethnic population with Punjabi ancestry.

In one embodiment of any configuration of the vertebrate, cell or library of the invention, the human immunoglobulin gene segment derived from the genome sequence of the second individual is low-frequency (optionally rare) within the second ethnic population. Optionally human immunoglobulin gene segment has a Minor Allele Frequency (MAF) (cumulative frequency) of between 0.5% - 5%, optionally less than 0.5%, in the second human population, eg, as in the 1000 Genomes database.

In one embodiment of any configuration of the vertebrate, cell or library of the invention, the first variable region nucleotide sequence is produced by recombination of the first human immunoglobulin gene segment with a first J gene segment and optionally a first D gene segment, wherein the first human immunoglobulin gene segment is a V gene segment and the V, D and J segments are derived from the first human population, optionally from the genome of one individual of the first human population.

In one embodiment of the library of the vertebrate, cell or library of the invention, the second variable region nucleotide sequence is produced by recombination of the second human immunoglobulin gene segment with a second J gene segment and optionally a second D gene segment, wherein the second human immunoglobulin gene segment is a V gene segment derived from the second population and the D and/or J segments are derived from the first human population, optionally the D and J gene segments being from the genome of one individual of the first human population.

In one embodiment of the library of the vertebrate, cell or library of the invention, all of the D and J segments that have been recombined with the first and second V gene segments are D and J segments derived from the first human population, optionally the D and J gene segments being from the genome of one individual of the first human population.

In one embodiment of the library, the second human immunoglobulin gene segment is a polymorphic variant of the first human immunoglobulin gene segment; optionally wherein the second gene segment is selected from the group consisting of a gene segment in any of Tables 1 to 7 and 9 to 14 (eg, selected from Table 13 or 14).

In one embodiment of the library, the first and second human immunoglobulin gene segments are both (i) V_{H} gene segments; (ii) D segments; (iii) J segments (optionally both J_{H} segments, both J_{κ} segments or both J_{λ} segments); (iv) constant regions (optionally both a gamma constant region, optionally both a C gamma-1 constant region); (v) CH1 regions; (vi) CH2 regions; or (vii) CH3 regions.

The library is, for example, a naive and optionally has a library size of from 10 or 10² to 10⁹ cells. For example, from 10, 20, 30, 40, 50, 100 or 1000 to 10⁸; or 10, 20, 30, 40, 50, 100 or 1000 to 10⁷; or 10, 20, 30, 40, 50, 100 or 1000 to 10⁶; or 10, 20, 30, 40, 50, 100 or 1000 to 10⁵; or 10, 20, 30, 40, 50, 100 or 1000 to 10⁴ cells.

The library has, for example, been selected against a predetermined antigen and optionally has a library size of from 10 or 10² to 10⁹ cells. For example, from 10, 20, 30, 40, 50, 100 or 1000 to 10⁸; or 10, 20, 30, 40, 50, 100 or 1000 to 10⁷; or 10, 20, 30, 40, 50, 100 or 1000 to 10⁶; or 10, 20, 30, 40, 50, 100 or 1000 to 10⁵; or 10, 20, 30, 40, 50, 100 or 1000 to 10⁴ cells.

In one embodiment of the library of the invention, said first and second cells are progeny of first and second ancestor non-human vertebrate cells respectively, wherein the first ancestor cell comprises a genome comprising said first human immunoglobulin gene segment; and the second ancestor cell comprises a genome comprising said second human immunoglobulin gene segment.

The invention further provides a library of antibody-producing transgenic cells whose genomes collectively encode a repertoire of antibodies, wherein the library comprises the first and second ancestor cells described above.

The invention further provides a library of hybridoma cells produced by fusion of the library of the invention (eg, a β-cell library) with fusion partner cells and optionally has a library size of from 10 or 10² to 10⁹ cells. For example, from 10, 20, 30, 40, 50, 100 or 1000 to 10⁸; or 10, 20, 30, 40, 50, 100 or 1000 to 10⁷; or 10, 20, 30, 40, 50, 100 or 1000 to 10⁶; or 10, 20, 30, 40, 50, 100 or 1000 to 10⁵; or 10, 20, 30, 40, 50, 100 or 1000 to 10⁴ cells. Production of hybridomas is well known to the skilled person. Examples of fusion partners are SP2/0-g14 (obtainable from ECACC), P3X63-Ag8.653 (obtainable from LGC Standards; CRL-1580), NS1 and NS0 cells. PEG fusion or electrofusion can be carried out, as is conventional.

### The invention provides, in a third configuration:-

An isolated antibody having
(a) a heavy chain encoded by a nucleotide sequence produced following recombination in a transgenic non-human vertebrate cell of an unrearranged human immunoglobulin V gene segment with a human D and human J segment, optionally with affinity maturation in said cell, wherein one of the gene segments (eg, VH) is derived from the genome of an individual from a first human ethnic population; and the other two gene segments (eg, D and JH) are derived from the genome of an individual from a second (eg, a second and third respectively), different, human ethnic population, and wherein the antibody comprises heavy chain constant regions (eg, C gamma) of said non-human vertebrate (eg, rodent, mouse or rat heavy chain constant regions); and/or
(b) a light chain encoded by a nucleotide sequence produced following recombination in a transgenic non-human vertebrate cell of an unrearranged human immunoglobulin V gene segment with a human J segment, optionally with affinity maturation in said cell, wherein one of the gene segments (eg, VL) is derived from the genome of an individual from a first human ethnic population (optionally the same as the first population in (a)); and the other gene segment (eg, JL) is derived from the genome of an individual from a second, different, human ethnic population (optionally the same as the second population in (a)), and wherein the antibody comprises light chain constant regions of said non-human vertebrate (eg, rodent, mouse or rat heavy light constant regions);
(c) Optionally wherein each variable domain of the antibody is a human variable domain.
(d) Optionally wherein the heavy chain constant regions are mu- or gamma-type constant regions.

The invention also provides an isolated nucleotide sequence encoding the antibody of the third configuration, optionally wherein the sequence is provided in an antibody expression vector, optionally in a host cell. Suitable vectors are mammalian expression vectors (eg, CHO cell vectors or HEK293 cell vectors), yeast vectors (eg, a vector for expression in *Picchia pastoris,* or a bacterial expression vector, eg, a vector for *E. coli* expression.

The invention also provides a method of producing a human antibody, the method comprising replacing the non-human vertebrate constant regions of the antibody of the third configuration with human antibody constant regions (eg, a C variant disclosed in table 13 or 18). The skilled person will be aware of standard molecular biology techniques to do this. For example, see Harlow, E. & Lane, D. 1998, 5th edition, Antibodies: A Laboratory Manual, Cold Spring Harbor Lab. Press, Plainview, NY; and Pasqualini and Arap, Proceedings of the National Academy of Sciences (2004) 101:257-259 for standard immunisation. Joining of the variable regions of an antibody to a human constant region can be effected by techniques readily available in the art, such as using conventional recombinant DNA and RNA technology as will be apparent to the skilled person. See e.g. Sambrook, J and Russell, D. (2001, 3'd edition) Molecular Cloning: A Laboratory Manual (Cold Spring Harbor Lab. Press, Plainview, NY).

In one embodiment, the method comprises further making a mutant or derivative of the antibody.

The invention also provides a pharmaceutical composition comprising an antibody according to the third configuration, or a human antibody of the invention and a diluent, excipient or carrier; optionally wherein the composition is provided in a container connected to an IV needle or syringe or in an IV bag.

The invention also provides an antibody-producing cell (eg, a mammalian cell, eg, CHO or HEK293; a yeast cell, eg, *P pastoris;* a bacterial cell, eg, *E coli;* a B-cell; or a hybridoma) that expresses the second antibody of the third configuration or the isolated antibody of the invention.

### The first configuration of the invention also provides:-

A non-human vertebrate or vertebrate cell (optionally an ES cell or antibody-producing cell) whose genome comprises a transgenic immunoglobulin locus (eg, a heavy chain locus or a light chain locus), said locus comprising immunoglobulin gene segments according to the first and second human immunoglobulin gene segments (optionally V segments) described above in connection with the third configuration. The gene segments are operably connected upstream of an immunoglobulin constant region; optionally wherein the genome is homozygous for said transgenic immunoglobulin locus.

Optionally the immunoglobulin locus comprises more than the natural human complement of functional V gene segments; and/or
Optionally wherein the immunoglobulin locus comprises more than the natural human complement of functional D gene segments; and/or
Optionally wherein the immunoglobulin locus comprises more than the natural human complement of functional J gene segments.

In this way, a superhuman immunoglobulin gene repertoire is provided in a transgenic non-human vertebrate or vertebrate cell according to the invention.

### The first configuration also provides:-

A transgenic non-human vertebrate (eg, a mouse or rat) or vertebrate cell (optionally an ES cell or antibody-producing cell) whose genome comprises a transgenic immunoglobulin locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region; wherein the transgenic locus comprises one or more human immunoglobulin V gene segments, one or more human J gene segments and optionally one or more human D gene segments, a first (optionally a V segment) of said gene segments and a second (optionally a V segment) of said gene segments being different and derived from the genomes of first and second human individuals respectively, wherein the individuals are different; and optionally not related;
optionally wherein the immunoglobulin locus comprises more than the natural human complement of functional V gene segments; and/or
optionally wherein the immunoglobulin locus comprises more than the natural human complement of functional D gene segments; and/or
optionally wherein the immunoglobulin locus comprises more than the natural human complement of functional J gene segments.

In this way, a superhuman immunoglobulin gene repertoire is provided in a transgenic non-human vertebrate or vertebrate cell according to the invention.

### The first configuration also provides:-

A transgenic non-human vertebrate (eg, a mouse or rat) or vertebrate cell (optionally an ES cell or antibody-producing cell) whose genome comprises first and second transgenic immunoglobulin loci, each locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region;
wherein (i) the first transgenic locus comprises one or more human immunoglobulin V gene segments, one or more human J gene segments and optionally one or more human D gene segments, (ii) the second transgenic locus comprises one or more human immunoglobulin V gene segments, one or more human J gene segments and optionally one or more human D gene segments; and (iii) wherein a first (optionally a V) gene segment of said first locus and a second (optionally a V) gene segment of said second gene locus are different and derived from the genomes of first and second human individuals respectively, wherein the individuals are different; and optionally not related;
optionally wherein the first and second loci are on different chromosomes (optionally chromosomes with the same chromosome number) in said genome;
optionally wherein each immunoglobulin locus comprises more than the natural human complement of functional V gene segments; and/or
optionally wherein each immunoglobulin locus comprises more than the natural human complement of functional D gene segments; and/or
optionally wherein each immunoglobulin locus comprises more than the natural human complement of functional J gene segments.

In this way, a superhuman immunoglobulin gene repertoire is provided in a transgenic non-human vertebrate or vertebrate cell according to the invention.

In these embodiments of the first configuration, the immunoglobulin gene segments are optionally as described for the third configuration.

In these embodiments of the first configuration, the genome optionally comprises a third immunoglobulin gene segment (optionally a V segment), the third gene segment being derived from a human individual that is different from the individual from which the first (and optionally also the second) gene segment is derived; optionally wherein the first, second and third gene segments are polymorphic variants of a human immunoglobulin gene segment (eg, VH1-69 - see the examples for further description).

In these embodiments of the first configuration, the genome of the vertebrate or cell is optionally homozygous for the first, second and optional third gene segment, wherein a copy of the first, second and optional third gene segments are provided together on the same chromosome operably connected upstream of a common non-human vertebrate constant region.

For example, each first, second and optional third gene segment is a V gene segment.

In one example, the library of the invention is provided by a collection of non-human vertebrates (optionally a collection of rodents, mice or rats); optionally, wherein a first member of said collection produces said first antibody but not said second antibody, and a second member of the collection produces said second antibody (but optionally not said first antibody). It is therefore contemplated to make non-human vertebrates where different human genomes have been used as a source for building the transgenic loci in the vertebrates. For example, a first vertebrate comprises a transgenic heavy chain locus having gene segments only from a first (and optionally a second) human population or individual; a second vertebrate comprises a transgenic heavy chain locus having gene segments only from a third (and optionally a fourth) human population or individual; and optionally third and more vertebrates can be built similarly based on unique or overlapping human population genomes. However, when provided as a mixed population of transgenic vertebrates, the mixed population provides a collective pool of human immunoglobulin genes that is greater than found in a natural human repertoire. This is useful to extend the antibody and gene sequence space beyond those possible with prior transgenic mice and rats bearing human immunoglobulin loci. As explained above, these have been based on a single human genome.

In one embodiment, the collection of non-human vertebrates bear human immunoglobulin genes confined to human populations that are together grouped under the same population genus "(a)" mentioned above. This provides for a gene repertoire that is biased to producing human antibody variable regions prevalent in the population genus (a) and thus useful for generating antibody therapeutics/prophylactics for members of said population. Alternatively, where gene segments from different human populations are provided in a single transgene according to the invention (not necessarily in a collection of vertebrates), the different human populations are for example together grouped under the same population genus "(a)" mentioned above.

The invention also provides a repertoire of antibodies expressed from a library of cells according to the invention.

In the non-human vertebrate or cell of any configuration of the invention, the constant region of the transgenic locus is, in one example, an endogenous constant region of said vertebrate (eg, endogenous mouse or rat constant region, eg, from the same strain of mouse or rat as the non-human vertebrate itself).

The invention also provides a method of constructing a cell (eg, an ES cell) according to the invention, the method comprising
(a) identifying functional V and J (and optionally D) gene segments of the genome sequence of a (or said) first human individual;
(b) identifying one or more functional V and/or D and/or J gene segments of the genome sequence of a (or said) second human individual, wherein these additional gene segments are not found in the genome sequence of the first individual;
(c) and constructing a transgenic immunoglobulin locus in the cell, wherein the gene segments of (a) and (b) are provided in the locus operably connected upstream of a constant region.

Optionally the cell comprises a heavy chain locus constructed according to steps (a) to (c) and/or a light chain locus (kappa and/or lambda loci) constructed according to steps (a) to (c).

Optionally the cell is homozygous for the or each transgenic locus; optionally wherein antibody expression from loci endogenous to said cell has been inactivated. This is useful for confining the functional antibody gene repertoire, and thus antibody production, to antibodies bearing human variable regions.

Optionally the gene segment(s) in step (b) are identified from an immunoglobulin gene database selected from the 1000 Genomes, Ensembl, Genbank and IMGT databases.

Optionally the first and second human individuals are members of first and second ethnic populations respectively, wherein the populations are different, optionally wherein the human immunoglobulin gene segment derived from the genome sequence of the second individual is low-frequency (optionally rare) within the second ethnic population.

The invention also provides a method of making a transgenic non-human vertebrate (eg, a mouse or rat), the method comprising
(a) constructing an ES cell (eg, a mouse C57BL/6N, C57BL/6J, 129S5 or 129Sv strain ES cell) by carrying out the method above;
(b) injecting the ES cell into a donor non-human vertebrate blastocyst (eg, a mouse C57BL/6N, C57BL/6J, 129S5 or 129Sv strain blastocyst);
(c) implanting the blastocyst into a foster non-human vertebrate mother (eg, a C57BL/6N, C57BL/6J, 129S5 or 129Sv strain mouse); and
(d) obtaining a child from said mother, wherein the child genome comprises a transgenic immunoglobulin locus.

The invention provides a transgenic non-human vertebrate (eg, a mouse or rat) made by the method or a progeny thereof. The invention also provides a population of such non-human vertebrates.

Microinjection of ES cells into blastocysts and generation of transgenic mice therafter are conventional practices in the state of the art, and the skilled person is aware of techniques useful to effect this. C57BL/6N, C57BL/6J, 129S5 or 129Sv mouse strains and ES cells are readily and publicly available.

The invention also provides a method of isolating an antibody that binds a predetermined antigen (eg, a bacterial or viral pathogen antigen), the method comprising
(a) providing a vertebrate (optionally a mouse or rat) according to the invention;
(b) immunising (eg, using a standard prime-boost method) said vertebrate with said antigen (optionally wherein the antigen is an antigen of an infectious disease pathogen);
(c) removing B lymphocytes from the vertebrate and selecting one or more B lymphocytes expressing antibodies that bind to the antigen;
(d) optionally immortalising said selected B lymphocytes or progeny thereof, optionally by producing hybridomas therefrom; and
(e) isolating an antibody (eg, and IgG-type antibody) expressed by the B lymphocytes; and
(f) optionally producing a derivative or variant of the antibody.

This method optionally further comprises after step (e) the step of isolating from said B lymphocytes nucleic acid encoding said antibody that binds said antigen; optionally exchanging the heavy chain constant region nucleotide sequence of the antibody with a nucleotide sequence encoding a human or humanised heavy chain constant region and optionally affinity maturing the variable region of said antibody; and optionally inserting said nucleic acid into an expression vector and optionally a host.

### Bioinformatics Analysis & Selection of Immunogobulin Gene Segments

See also the discussion on variation analysis above.

The skilled person will know of sources of human antibody gene sequences, such as IMGT (www.imgt.org), GenBank (www.ncbi.nlm.nih.gov/genbank) Bioinformatics tools for database manipulation are also readily available and known to the skilled person, eg, as publicly available from the 1000 Genomes Project/EBI (www.1000genomes.org)

As a source of antibody gene segment sequences, the skilled person will also be aware of the following available databases and resources (including updates thereof):-
1.1. The Kabat Database (G. Johnson and T. T.Wu, 2002; http://www.kabatdatabase.com) Created by E. A. Kabat and T. T. Wu in 1966, the Kabat database publishes aligned sequences of antibodies, T-cell receptors, major histocompatibility complex (MHC) class I and II molecules, and other proteins of immunological interest. A searchable interface is provided by the Seqhuntll tool, and a range of utilities is available for sequence alignment, sequence subgroup classification, and the generation of variability plots. See also Kabat, E. A.,Wu, T. T., Perry, H., Gottesman, K., and Foeller, C. (1991) Sequences of Proteins of Immunological Interest, 5th ed., NIH Publication No. 91-3242,Bethesda, MD, which is incorporated herein by reference, in particular with reference to human gene segments for use in the present invention.
1.2. KabatMan (A. C. R. Martin, 2002; http://www.bioinf.org.uk/abs/simkab.html). This is a web interface to make simple queries to the Kabat sequence database.
*1.3. IMGT, the International ImMunoGeneTics Information System®; M.-P. Lefranc, 2002;* *http:*//*imgt.cines.fr).* IMGT is an integrated information system that specializes in antibodies, T cell receptors, and MHC molecules of all vertebrate species. It provides a common portal to standardized data that include nucleotide and protein sequences, oligonucleotide primers, gene maps, genetic polymorphisms, specificities, and two-dimensional (2D) and three-dimensional (3D) structures. IMGT includes three sequence databases *(IMGT*/*LIGM-DB, IMGT*/*MHC-DB, IMGT*/*PRIMERDB),* one genome database *(IMGT*/*GENE-DB),* one 3D structure database *(IMGT*/*3Dstructure-DB),* and a range of web resources (*"IMGT* Marie-Paule page") and interactive tools.
*1.4.* V-BASE (I. M. Tomlinson, 2002;http://www.mrc-cpe.cam.ac.uk/vbase*).* V-BASE is a comprehensive directory of all human antibody germline variable region sequences compiled from more than one thousand published sequences. It includes a version of the alignment software DNAPLOT (developed by Hans-Helmar Althaus and Werner Müller) that allows the assignment of rearranged antibody V genes to their closest germline gene segments.
*1.5.* Antibodies-Structure and Sequence(A. C. R. Martin, 2002; htto://www.bioinf.ora.uk/abs*).* This page summarizes useful information on antibody structure and sequence. It provides a query interface to the Kabat antibody sequence data, general information on antibodies, crystal structures, and links to other antibody-related information. It also distributes an automated summary of all antibody structures deposited in the Protein Databank (PDB). Of particular interest is a thorough description and comparison of the various numbering schemes for antibody variable regions.
*1.6.* AAAAA-AHo's Amazing Atlas of Antibody Anatomy (A. Honegger, 2001; http:/lwww.unizh.ch/~antibody*).* This resource includes tools for structural analysis, modeling, and engineering. It adopts a unifying scheme for comprehensive structural alignment of antibody and T-cell-receptor sequences, and includes Excel macros for antibody analysis and graphical representation.
*1.7.* WAM-Web Antibody Modeling (N. Whitelegg and A. R. Rees, 2001; http://antibody.bath.ac.uk*).* Hosted by the Centre for Protein Analysis and Design at the University of Bath, United Kingdom. Based on the AbM package (formerly marketed by Oxford Molecular) to construct 3D models of antibody Fv sequences using a combination of established theoretical methods, this site also includes the latest antibody structural information.
*1.8.* Mike's Immunoglobulin Structure/Function Page (M. R. Clark, 2001; http://www.path.cam.ac.uk/~mrc7/mikeimages.html*)* These pages provide educational materials on immunoglobulin structure and function, and are illustrated by many colour images, models, and animations. Additional information is available on antibody humanization and Mike Clark's Therapeutic Antibody Human Homology Project, which aims to correlate clinical efficacy and anti-immunoglobulin responses with variable region sequences of therapeutic antibodies.
*1.9.* The Antibody Resource Page (The Antibody Resource Page, 2000; http://www.antibodyresource.com*).* This site describes itself as the "complete guide to antibody research and suppliers." Links to amino acid sequencing tools, nucleotide antibody sequencing tools, and hybridoma/cell-culture databases are provided.
*1.9.* Humanization bY Design (J. Saldanha, 2000; http://people.cryst.bbk.ac.uk/~ubcg07s*).* This resource provides an overview on antibody humanization technology. The most useful feature is a searchable database (by sequence and text) of more than 40 published humanized antibodies including information on design issues, framework choice, framework back-mutations, and binding affinity of the humanized constructs.

See also Antibody Engineering Methods and Protocols, Ed. Benny K C Lo, Methods in Molecular Biology™, Human Press. Also at http://www.blogsua.com/pdf/antibody-engineering-methods-and-protocolsantibody-engineering-methods-and-protocols.pdf

As a source of genomic sequence variation data, the skilled person will also be aware of the following available databases and resources (including updates thereof):-
1. HapMap (The International HapMap Consortium. 2003; http://hapmap.ncbi.nlm.nih.gov/index.html.en). The HapMap Project is an international project that aims to compare the genetic sequences of different individuals to identify chromosomal regions containing shared genetic variants. The HapMap www site provides tools to identify chromosomal regions and the variant therein, with options to drill down to population level frequency data.
2. 1000 Genomes (The 1000 Genomes Project Consortium 2010; http://www.1000genomes.org/). This resource provides complete genomic sequence for 2500 unidentified individuals from one of 25 distinct population groups, with the aim of identifying genomic variants of >1%. The site provides the ability to interrogate data utilizing online tools (e.g. 'Variation Pattern Finder') and to download variant data for individual population groups.
3. Japanese SNP Database (H.Haga et al. 2002; http://snp.ims.u-tokyo.ac.ip/index.html). Based on a study identifying 190,562 human genetic variants this site catalogues genomic variants with useful features for searching and summarizing data.

It is possible to identify variants in immunoglobulin genes classed as low-frequency or rare variants that segregate with specific human ethnic populations. For the purpose of this analysis, a low-frequency immunoglobulin gene segment is classed as one with 'Minor Allele Frequency' (MAF) (cumulative frequency) of between 0.5% - 5%, rare variants are those classed as having a MAF of less than 0.5% in a particular human population.

The following bioinformatics protocol is envisaged to indentify human immunoglobulin gene segments for use in the present invention:
(a) Identify one or more genomic regions containing gene segments of interest ('target genomic regions') and calculate the genomic coordinates, using coordinates that match the sequence assembly build used by either the 1000 Genomes project or International HapMap project (or another selected human gene database of choice).
(b) Identify genomic variants mapped to the genomic regions previously identified in (a). Retrieve variant frequencies for variants for each super population and preferably subpopulation where such data is available. Tools readily available on the HapMap WWW site and the VWC tools for the 1000Genomes Project are useful for this step.
(c) Filter list of genomic variants from target genomic regions to contain only variants classed as either 'Non-synonymous' single nucleotide polymorphisms (SNPs) or genomic 'insertions or delections' (indels). Filter further to include those that are present in exonic sequences only.
(d) Correlate population frequency data for each of the identified variants for each of the super populations (for example 'European Ancestry', 'East Asian ancestry', 'West African ancestry', 'Americas', and 'South Asian ancestry') to identify those variants that segregate with less than two super-populations. Further correlate all identified variants with each of the sub-populations (for example, 'European ancestry' super-population might be subdivided into groups such as 'CEU - Utah residents with Northern or Western European ancestry', 'TSI Toscani in Italia' and 'British from England and Scotland') and produce a second score for rarity of variants in within a super-population.
(e) Collect one or more gene segments that show segregation to specific sub-populations for construction of synthetic loci according to the invention.

In one embodiment throughout the present text, "germline" refers to the canonical germline gene segment sequence.

By detailed analysis of the 1000 Genomes database, the inventors have devised a collection of candidate polymorphic antibody gene segment variants, eg, human variant JH gene segments (eg, see Example 4), that can be built into the design of transgenic heavy chain loci in mice for expressing increasingly diverse and new, synthetic repertoires of human variable regions. To this end, the invention provides the following embodiments.

### The present invention provides in a fourth configuration:-

### Selection of Human JH6*02 Variant

### Transgenic IgH Loci. Non-Human Vertebrates. Cells & Antibodies Based on Human JH6*02

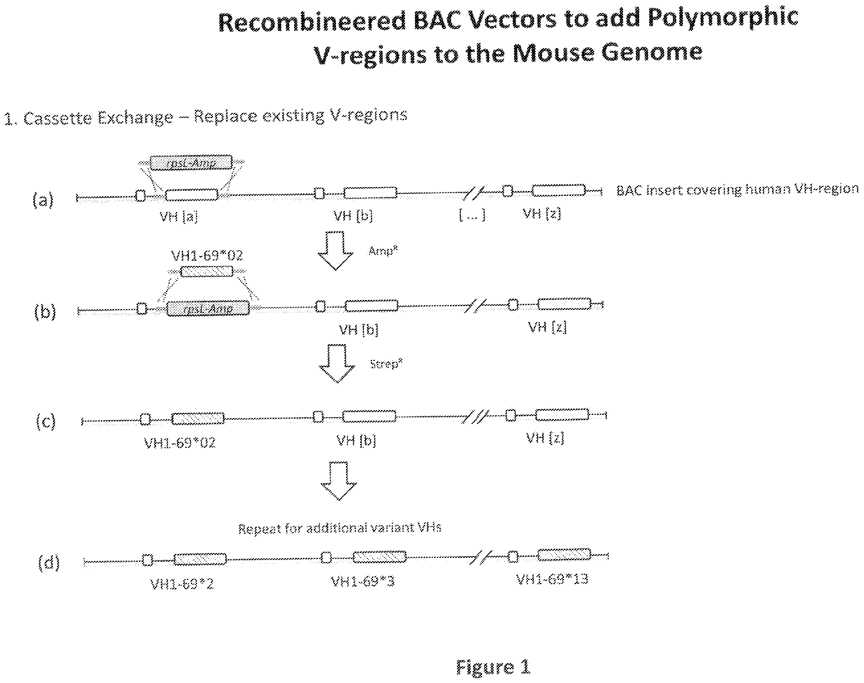

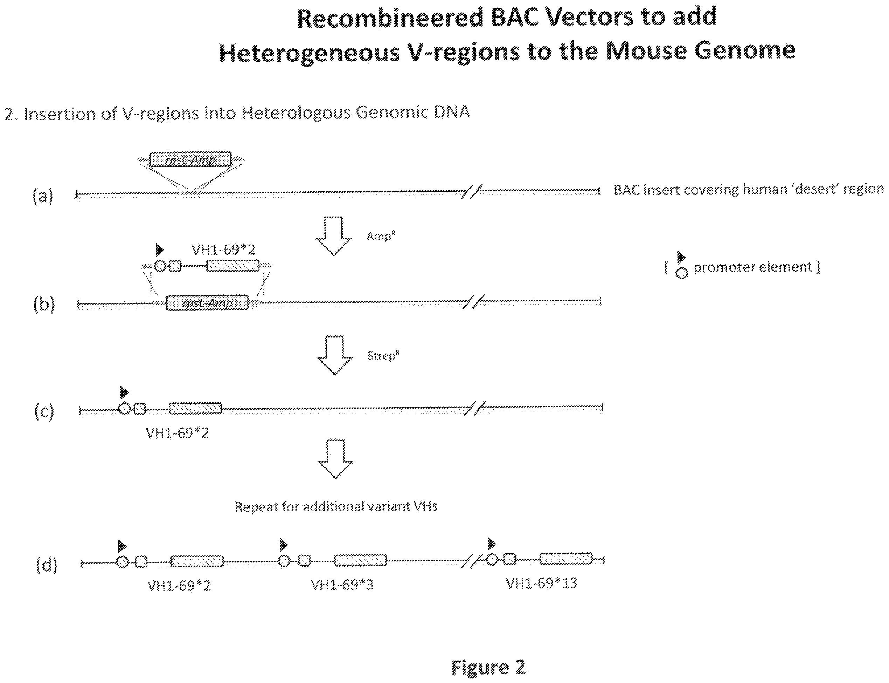

As explained above, in designing transgenic Ig heavy chain loci the present inventors have considered the huge amount of data available from the 1000 Genomes project (see www.1000genomes.org) that analyses gene distributions amongst many human populations, and in particular data on Ig gene segments. The inventors were also aware of human gene segments disclosed in the IMGT database (see www.imgt.org) and in Ensembl (see www.ensembl.org). The inventors needed to make choices about which human gene segments to include amongst the large number of human gene segments presented in these databases and the other sources of human Ig gene segment information known in the art, including those other databases disclosed herein. When choosing human JH gene segments, the inventors were aware that human JH6 encodes a relatively long amino acid sequence, and thus the inventors thought it desirable to include this for increasing the chances of producing IgH chains with relatively long HCDR3 regions. Antibodies with long HCDR3 (at least 20 amino acids according to IMGT nomenclature) have been shown to neutralise a variety of pathogens effectively including HIV, Influenza virus, malaria and Africa trypanosomes. Reference is also made to naturally-occurring Camelid (eg, llama or camel) heavy chain-only antibodies which bear long HCDR3s for reaching relatively inaccessible epitopes (see, eg, EP0937140). Long HCDR3s can form unique stable subdomains with extended loop structure that towers above the antibody surface to confer fine specificity. In some cases, the long HCDR3 itself is sufficient for epitope binding and neutralization (Liu, L et al; Journal of Virology. 2011. 85: 8467-8476, incorporated herein by reference). The unique structure of the long HCDR3 allows it to bind to cognate epitopes within inaccessible structure or extensive glycosylation on a pathogen surface. In human peripheral blood, there is around 3.5% of naive B antibodies or 1.9% of memory B IgG antibodies containing the HCDR3s with lengths of more than 24 amino acids (PLoS One. 2012;7(5):e36750. Epub 2012 May 9; "Human peripheral blood antibodies with long HCDR3s are established primarily at original recombination using a limited subset of germline genes"; Briney BS *e al,* incorporated herein by reference) (Fig. 1). The usage analysis indicates that these antibodies have the preference to use human JH6 with human D2-2, D3-3 or D2-15 (Brinley, BS *et al,* Figs. 2-5). See also PLoS One. 2011 Mar 30;6(3):e16857; Comparison of antibody repertoires produced by HIV-1 infection, other chronic and acute infections, and systemic autoimmune disease"; Breden F *et al,* incorporated herein by reference. Around 20% of all HCDR3 of antibodies use JH6. However, in those antibodies with HCDR3 of more than 24 amino acids, 70% use JH6 (Brinley, BS *et al,* Fig.2).

There is a need in the art for genetically modified non-human vertebrates and cells that can make antibodies and heavy chains that have long human HCDR3s, as well as antibodies, chains and VH domains that can be selected from such vertebrates and cells wherein these can address target epitopes better accessed by long HCDR3s.

The inventors, therefore, chose in this configuration of the invention to include a human JH6 gene segment as a mandatory human gene segment in their IgH locus design. Several different naturally-occurring human JH6 variants are known (eg, JH6*01 to *04 as well as others; IMGT nomenclature). The inventors considered this when deciding upon which human JH6 variant should be included in the transgenic IgH locus design. An alignment of some human JH6 variants is shown in Figure 7 (from www.imgt.org; dashes indicate identical nucleotides; nucleotide changes versus the *01 variant are shown by underlined nucleotides and corresponding amino acid changes are shown by underlined amino acids; Genbank accession numbers (release 185.0) are shown prefixed by J, X, M or A). The inventors used sequencing of human genomic DNA samples, inspection of public IgH DNA databases as well as informed choices on the basis of variant sequences as means to arrive at a rational choice of which JH6 variant to use.

The 1000 Genomes database uses human JH6*03 as the reference sequence, which would be a possible choice for the skilled person wishing to construct a transgenic IgH locus. The inventors noticed (eg, Figure 7 herein) that position 6 in JH6*03 is a tyrosine (Y) encoded by a TAC codon, whereas some other naturally-occurring human variants have a glycine (G) encoded by a GGT codon (the glycine being present as a YYG motif, forming part of a larger YYGXDX motif). To understand the potential significance of this, the inventors carried out analysis of JH sequences from other vertebrate species. The inventors surprisingly noticed that YYG and YYGXDX motifs are conserved across many vertebrate species (see Figures 7 & 8). This suggested to the inventors, therefore, that preservation of this motif might be desirable, which could guide the choice of JH6 variant for use in the present invention.

Another pointer arose when the inventors considered the TAC codon versus the GGT codon encoding Y or G respectively. The inventors considered the impact of these nucleotide sequences on the action of activation-induced cytidine deaminase (AID). The inventors knew that activation-induced cytidine deaminase (AID) is believed to initiate Ig somatic hypermutation (SHM) in a multistep mechanism and they addressed this activity when rationally designing the locus. AID catalyses the deamination of C to U in DNA, generating mutations at C bases. Cytidines located within hotspot motifs are preferentially deaminated. Certain motifs are hotspots for AID acitivity (DGYW, WRC, WRCY, WRCH, RGYW, AGY, TAC, WGCW, wherein W=A or T, Y=C or T, D=A, G or T, H=A or C or T, and R=A or G). The presence of a TAC codon encoding Y at position 6 in JH6*03 creates AID mutation hotspots (the cytidine being the substrate of AID), these hotspots being the underlined motifs in the previous sentence. The inventors considered the impact of this and in doing so they considered possible mutants created by AID activity at the cytidine. Reference is made to Figure 9. The inventors noticed that a mutation at the third base of the TAC codon would yield 3 possible outcomes: Y, stop or stop. Thus, out of the three stop codons possible in the genetic code (the other being encoded by TGA - see Figure 9), two of them would be provided by mutation of the cytidine in the TAC codon encoding position 6 in JH6*03. The inventors, therefore, considered that this might increase the chances of non-productive IgH variable region production in transgenic loci based on JH6*03. Moreover, the inventors noticed that provision of a GGT codon instead (as per the other human JH6 variants) seemed preferable since mutation of the third base would never yield a stop codon (see Figure 9), and furthermore would retain coding, and thus conservation, of glycine at position 6, which the inventors also noticed was is in the YYG and YYGXDX motifs conserved across species.

Having decided against using JH6*03, the inventors needed to make a choice from other possible human variants. The MDV motif is at the C-terminus of HCDR3 based on human JH6, the adjacent framework 4 (FW4) starting with the WGQ motif (with reference to the sequence shown encoded by JH6*01; Figure 7). In making their choices for locus design, the inventors wished to maximise conservation of this HCDR3/FW4 junction in product IgH chains and antibodies including these. The inventors believed this to be desirable for heavy chain variable domain functionality and conformation. The inventors thought that this might in some cases be desirable to minimise immunogenicity (suitable for human pharmaceutical use). Consistent with these considerations, the inventors wanted to make a choice that would minimise mutation around the HCDR3/FW4 junction as a result of SHM *in vivo* to conserve junction configuration. See Rogozin & Diaz; "Cutting Edge: DGYW/WRCH Is a Better Predictor of Mutability at G:C Bases in Ig Hypermutation Than the Widely Accepted RGYW/WRCY Motif and Probably Reflects a Two-Step Activation-Induced Cytidine Deaminase-Triggered Process"; Journal of Immunology; March 15, 2004 vol. 172 no. 6 3382-3384. An example of a DGYW motif is GGCA. The inventors had this in mind when analysing the variant sequences.

With these considerations in mind, the inventors decided specifically to use human JH6*02 as the mandatory human JH6 for their IgH locus design. JH6*01 was rejected as the mandatory JH6 gene segment since the nucleotide sequence GGG CAA (encoding G and Q) contains a GGCA motif which is an AID recognition hotspot. The inventors realised that JH6*04 also contains such a motif due to the presence of the sequence GGC AAA encoding G and K (positions 11 and 12 respectively). The inventors also realised that the *02 variant has a C instead of a G that is in the *01 variant, the C desirably being a synonymous change (ie, not changing the encoded amino acid sequence around the CDR3/FW4 junction) and also this does not provide a GGCA AID hotspot motif. The inventors, therefore, decided that the mandatory JH6 should have this C base and this too pointed them to using the human JH6*02 variant.

In one example of any configuration of the invention herein, the only JH6 species included in the locus or genome is human JH6*02.

The inventors obtained 9 anonymised DNA samples from cheek swabs of 9 consenting human adults. Sequencing was performed on IgH locus DNA to confirm natural JH6 variant usage. It was found that the genome of all 9 humans contained a JH6*02 variant gene segment. In 7 out of the 9 humans, the genome was homozygous for JH6*02 (ie, each chromosome 14 had JH6*02 as its JH6 gene segment in the IgH locus). The inventors also inspected the publicly-available sequence information from the genomes of well-known scientists Craig Venter and Jim Watson. Both of these genomes contain JH6*02 too. This indicated to the inventors that this variant is common in humans.

So, the inventors made a choice of human JH6*02 on the basis of
(i) Containing the YYG and YYGXDX motifs that is conserved across several vertebrate species;
(ii) Provision of one less TAC codon (an AID hotspot that risks stop codons) and a choice instead of a codon that preserves the YYG and YYGXDX motifs;
(iii) Avoidance of a GGCA AID hotspot in the region of the HCDR3/FW4 junction; and
(iv) Common occurrence (and thus conservation and acceptability) in humans of the JH6*02 variant.

This rationale was tested by the inventors in laboratory examples, in order to see if human JH6*02 could desirably participate in antibody gene segment recombination and heavy chain production in a foreign (non-human vertebrate) setting, and moreover to assess if long HCDR3s based on human JH6*02 could be produced *in vivo* (in naive and immunised settings) in such non-human systems. It was noted that in some non-human settings, such as a mouse, the YYG and YYGXDX motifs are not conserved, and thus the inventors decided that it was important to test whether or not JH6*02 (having the YYG and YYGXDX motifs) could function properly in such a foreign setting to participate in VDJ recombination and selection against antigen.

Thus, as explained further in the examples, the inventors constructed transgenic JH6*02-containing IgH loci in ES cells, generated transgenic non-human vertebrates from the ES cells (both naive and immunised with a range of different target antigen types), isolated antibodies and heavy chain sequences based on JH6*02 as well as B-cells expressing these and made hybridomas expressing antigen-specific antibodies that are based on the chosen JH6*02 variant. The inventors found that the JH6*02 variant was extensively used and could contribute to the production of HCDR3 of at least 20 amino acids in many different heavy chains (including antigen-specific heavy chains). The chosen variant was preferably used over other JH gene segments in all settings (naive, immunised and antigen-specific) for the production of HCDR3 of at least 20 amino acids.

Thus, the present invention provides an IgH locus including human JH6*02 (IMGT nomenclature) as a mandatory JH gene segment. In one embodiment, the locus comprises non-human vertebrate (eg, mouse or rat) constant region gene segments downstream (ie, 3' of) the human JH6*02; and one or more VH gene segments (eg, a plurality of human VH gene segments) and one or more D gene segments (eg, a plurality of human D gene segments) upstream of (ie, 5' of) the human JH6*02. For example, the locus is comprised by a vector (eg, a DNA vector, eg, a yeast artificial chromosome (YAC), BAC or PAC). Such a vector (eg, YAC) can be introduced into a non-human vertebrate (eg, mouse or rat) cell using standard techniques (eg, pronuclear injection) so that the locus is integrated into the cell genome for expression of IgH chains comprising at least one chain whose variable domain is a product of the recombination of human JH6*02 with a VH and a D gene segment.

In another example, the locus (eg, with a completely human, rat or mouse constant region, or a human/mouse chimaeric constant region) can be provided in the genome of a non-human vertebrate (eg, mouse or rat) cell. For example, the cell is an ES cell or an antibody-producing cell (eg, an isolated B-cell, an iPS cell or a hybridoma).

In another example, the invention provides a non-human vertebrate (eg, a mouse or a rat) comprising an IgH locus of the invention which comprises a human JH6*02 gene segment, wherein the locus can express an IgH chain whose variable domain is a product of the recombination of human JH6*02 with a VH and a D gene segment. As shown in the examples, the inventors have successfully produced such mice which produce such IgH chains with VH domains based on human JH6*02. The inventors isolated and sequenced IgH chains from the mice before (naive) and after (immunised) exposure to a range of target antigens and confirmed by comparison to IMGT IgH gene segment sequences that the isolated chains (and antibodies containing these) were produced based on JH6*02. Such chains were found in naive mice, as well as in antigen-specific antibodies from immunised mice. B-cells were isolated from immunised mice, wherein the B-cells express antibodies based on JH6*02 and hybridomas were generated from the B-cells, the hybridomas expressing antigen-specific antibodies based on JH6*02. The inventors, therefore, provided the locus, vertebrate, cell and hybridoma of the invention based on the use of human JH6*02 and showed that antibodies based on JH6*02 and B-cells expressing these can be successfully produced and isolated following immunisation of the vertebrates, corresponding hybridomas being a good source of antibodies whose VH domains are based on JH6*02, eg for administration to a patient, eg, for human medicine. Furthermore, it was found possible to produce and isolated antigen-specific antibodies whose VH domains are based on JH6*02 and which had a relatively long HCDR3 (eg, 20 amino acids).

Thus, the present invention provides embodiments as in the following clauses:-
1. A non-human vertebrate (optionally a mouse or a rat) or vertebrate cell whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof so that the mouse is capable of producing an antibody heavy chain produced by recombination of the human JH6*02 with a D segment and a VH segment.
   In another example, the invention provides
   A non-human vertebrate (optionally a mouse or a rat) or vertebrate cell whose genome comprises an immunoglobulin heavy chain locus comprising one, more or all of human IGHV gene segments selected from V3-21, V3-13, V3-7, V6-1, V1-8, V1-2, V7-4-1, V1-3, V1-18, V4-4, V3-9, V3-23, V3-11 and V3-20 (eg, one, more or all of V3-21*03, V3-13*01, V3-7*01, V6-1*01, V1-8*01, V1-2*02, V7-4-1*01, V1-3*01, V1-18*01, V4-4*01, V3-9*01 and V3-23*04). These segments were found in naive repertoires to be productive to produce HCDR3s of at least 20 amino acids in length. In an embodiment, the locus comprises a human JH6, eg, JH6*02.
   The invention also provides a HCDR3, VH domain, antibody heavy chain or antibody having a HCDR3 size of at least 20 amino acids. Optionally, the HCDR3 or VH domain (or VH domain of the heavy chain or antibody) comprises mouse AID-pattern somatic hypermutations and/or mouse dTd-pattern mutations. This can be provided, for example, wherein VH domain is produced in a mouse comprising mouse AID and/or mouse TdT (eg, endogenous AID or TdT). See also Annu. Rev. Biochem. 2007. 76:1-22; Javier M. Di Noia and Michael S. Neuberger, "Molecular Mechanisms of Antibody Somatic Hypermutation" (in particular figure 1 and associated discussion on AID hotspots in mouse); and Curr Opin Immunol. 1995 Apr;7(2):248-54, "Somatic hypermutation", Neuberger MS and Milstein C (in particular, discussion on hotspots in mouse), the disclosures of which are incorporated herein by reference.
   These segments were found in naive repertoires to be productive in recombination with human JH6*02 to produce HCDR3s of at least 20 amino acids in length.
   In an example, the vertebrate is naive. In another embodiment, the vertebrate instead is immunised with a target antigen.
   In an example, the vertebrate or cell mentioned below is capable of so producing an antibody heavy chain upon immunisation with a target antigen. In an example, the vertebrate is an immunised vertebrate that produces antibody heavy chains specific for a target antigen and wherein the variable domains of the heavy chains are the product of recombination between a VH, D and JH6*02. For example, the D is selected from human D3-3, D2-15, D3-9; D4-17; D3-10; D2-2; D5-24; D6-19; D3-22; D6-13; D5-12; D1-26; D1-20; D5-18; D3-16; D2-21; D1-14; D7-27; D1-1; D6-25; D2-14 and D4-23 (eg, selected from D3-9*01; D4-17*01; D3-10*01; D2-2*02; D5-24*01; D6-19*01; D3-22*01; D6-13*01; D5-12*01; D1-26*01; D1-20*01; D5-18*01; D3-16*02; D2-21*02; D1-14*01; D7-27*02; D1-1*01; D6-25*01; D2-15*01; and D4-23*01). For example, the D is human D3-9 or D3-10. In an example, the HCDR3 length is at least 20 amino acids (eg, 20, 21, 23 or 24).
   In an example of the vertebrate or cell, the genome comprises additional human JH gene segments (eg, JH2, 3, 4 and 5 gene segments).
   In an example of the vertebrate or cell, the genome comprises an immunoglobulin light chain locus comprising one or more human V gene segments and one or more human J gene segments upstream of a constant region (eg, a human or a mouse lambda or kappa constant region).
   For rearrangement and expression of heavy chains, the locus comprises control elements, such as an Eµ and Sµ between the J gene segment(s) and the constant region as is known by the skilled person. In one example, a mouse Eµ and Sµ is included in the heavy chain locus between the JH6*02 and the constant region (ie, in 5' to 3' order the locus comprises the JH6*02, Eµ and Sµ and constant region). In an example, the Eµ and Sµ are Eµ and Sµ of a mouse 129-derived genome (eg, a 129Sv-derived genome, eg, 129Sv/EV (such as 129S7Sv/Ev (such as from AB2.1 or AB2.2 cells obtainable from Baylor College of Medicine, Texas, USA) or 129S6Sv/Ev))); in another example, the Eµ and Sµ are Eµ and Sµ of a mouse C57BL/6 -derived genome. In this respect, the locus can be constructed in the IgH locus of the genome of a cell selected from AB2.1, AB2.2, VGF1, CJ7 and FH14. VGF1 cells were established and described in Auerbach W, Dunmore JH, Fairchild-Huntress V, et al; Establishment and chimera analysis of 129/SvEv- and C57BL/6-derived mouse embryonic stem cell lines. Biotechniques 2000; 29:1024-8, 30, 32, incorporated herein by reference.
   Additionally or alternatively, the constant region (or at least a Cµ; or Cµ and gamma constant regions thereof) is a constant region (or Cµ; or Cµ and gamma constant regions thereof) is of a genome described in the paragraph immediately above.
   A suitable source of JH6*02 and other human DNA sequences will be readily apparent to the skilled person. For example, it is possible to collect a DNA sample from a consenting human donor (eg, a cheek swab sample as per the Example herein) from which can be obtained suitable DNA sequences for use in constructing a locus of the invention. Other sources of human DNA are commercially available, as will be known to the skilled person. Alternatively, the skilled person is able to construct gene segment sequence by referring to one or more databases of human Ig gene segment sequences disclosed herein.
2. The vertebrate of clause 1, wherein the vertebrate has been immunised with a target antigen and wherein the variable domain of the heavy chain is the product of recombination between a VH, D and JH6*02 and wherein the HCDR3 length is at least 20 amino acids (eg, 20, 21, 23 or 24).
   Optionally, the immunised vertebrate produces an antibody heavy chain specific for a target antigen and wherein the variable domain of the heavy chain is the product of recombination between a VH, D and JH6*02 and wherein the HCDR3 length is at least 20 amino acids (eg, 20, 21, 23 or 24).
3. A non-human vertebrate cell (optionally a mouse cell or a rat cell) whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof for producing (eg, in a subsequent progeny cell) an antibody heavy chain produced by recombination of the human JH6*02 with a D segment and a VH segment.
4. The cell of clause 3, which is an ES cell capable of differentiation into a progeny antibody-producing cell that expresses said heavy chain.
5. The vertebrate or cell of any preceding clause, wherein the heavy chain locus comprises a human JH6*02 recombination signal sequence (RSS) operably connected 5' to the JH6*02 gene segment.
   For example, the native RSS-JH6*02 sequence can be used to advantageously maintain the natural pairing between RSS and theis JH gene segment. In this respect, the following sequence is used:- 
ggtttttgtggggtgaggatggacattctgccattgtgattactactactacggtatggacgtctggggccaagggaccacggtcaccg
tctcctcag (SEQ ID NO: 238)
 RSSs have a common architecture: 9mer (eg, first underlined sequence above) followed by a 22bp spacer and then a 7mer (eg, second underlined sequence above). Spacers are 23bp +/-1 normally, while the 9 and 7mer are more conserved.
6. The vertebrate or cell of clause 5, wherein the RSS is SEQ ID NO: 238 or a sequence having an identical 9mer and 7mer sequence flanking a sequence that is at least 70% identical to the 22mer sequence of SEQ ID NO: 238.
7. The vertebrate or cell of clause 6, wherein the RSS and JH6*02 are provided as SEQ ID NO: 237.
8. The vertebrate or cell of any preceding clause, wherein the JH6*02 is the only JH6-type gene segment in the genome.
9. The vertebrate or cell of any preceding clause, wherein the JH6*02 is the closest JH gene segment to the constant region in the locus.
10. The vertebrate or cell of any preceding clause, wherein the locus comprises one, more or all human D gene segments D3-9; D4-17; D3-10; D2-2; D5-24; D6-19; D3-22; D6-13; D5-12; D1-26; D1-20; D5-18; D3-16; D2-21; D1-14; D7-27; D1-1; D6-25; D2-14; and D4-23.
   For example, the locus comprises one, more or all of human D gene segments D3-9*01; D4-17*01; D3-10*01; D2-2*02; D5-24*01; D6-19*01; D3-22*01; D6-13*01; D5-12*01; D1-26*01; D1-20*01; D5-18*01; D3-16*02; D2-21*02; D1-14*01; D7-27*02; D1-1*01; D6-25*01; D2-15*01; and D4-23*01.
11. The vertebrate or cell of clause 10, wherein the locus comprises one, more or all human D gene segments D3-9, D3-10, D6-19, D4-17, D6-13, D3-22, D2-2, D2-25 and D3-3.
   These D segments were found to be productive in recombination with human JH6*02 to produce HCDR3s of at least 20 amino acids in length.
   In an example, the locus comprises one, more or all human D gene segments D3-9, D3-10, D6-19, D4-17, D6-13 and D3-22 (for example one, more or all of D3-9*01, D3-10*01, D6-19*01, D4-17*01, D6-13*01 and D3-22*01). These D segments were found in naive repertoires to be productive in recombination with human JH6*02 to produce HCDR3s of at least 20 amino acids in length.
   In an example, the locus comprises one, more or all human D gene segments D3-10, D6-19 and D1-26 (for example, one, more or all of D3-10*01, D6-19*01 and D1-26*01). These D segments were found in immunised repertoires to be productive in recombination with human JH6*02 to produce HCDR3s of at least 20 amino acids in length.
   In an example, the locus comprises one, more or all human D gene segments D3-9 and D3-10 (for example, one, more or all of D3-9*01 and D3-10*01). These D segments were found in antigen-specific repertoires to be productive in recombination with human JH6*02 to produce HCDR3s of at least 20 amino acids in length.
12. The vertebrate or cell of any preceding clause, wherein the locus comprises a plurality of human D gene segments and the JH6*02 is in human germline configuration with respect to the 3'-most human D gene segment (or all of the human D segments comprised by the locus).
   In an example, the 3'-most D gene segment is D7-27. In an example, the locus comprises all of human D gene segments from D1-1 to D7-27 as present in a germline human IgH locus (eg, as shown in the IMGT database).
   Alternatively or additionally, the JH6*02 is in human germline configuration with respect to one, more or all of the Eµ, Sµ and constant region (eg, Cµ).
13. The vertebrate or cell of any preceding clause, wherein the locus comprises one, more or all of IGHV gene segments selected from V3-21, V3-13, V3-7, V6-1, V1-8, V1-2, V7-4-1, V1-3, V1-18, V4-4, V3-9, V3-23, V3-11 and V3-20.
   In an example, the locus comprises one, more or all human IGHV gene segments V3-21, V3-13, V3-7, V6-1, V1-8, V1-2, V7-4-1, V1-3, V1-18, V4-4, V3-9, V3-23 (for example, one, more or all of V3-21*03, V3-13*01, V3-7*01, V6-1*01, V1-8*01, V1-2*02, V7-4-1*01, V1-3*01, V1-18*01, V4-4*01, V3-9*01 and V3-23*04). These segments were found in naive repertoires to be productive in recombination with human JH6*02 to produce HCDR3s of at least 20 amino acids in length.
   In an example, the locus comprises one, more or all human IGHV gene segments V3-7, V3-11 and V4-4 (for example, one, more or all of V3-7*01, V3-11*01 and V4-4*02). These segments were found in immunised repertoires to be productive in recombination with human JH6*02 to produce HCDR3s of at least 20 amino acids in length.
   In an example, the locus comprises one, more or all human IGHV gene segments V4-4, V1-8, V3-9, V3-11 and V3-20 (for example, one, more or all of V4-4*02, V1-8*01, V3-9*01, V3-11*01 and V3-20 (eg, *d01). These segments were found in antigen-specific repertoires to be productive in recombination with human JH6*02 to produce HCDR3s of at least 20 amino acids in length.
14. The vertebrate or cell of any preceding clause, wherein the locus comprises one, more or all of human D3-9*01, D3-10*01, D6-19*01, D6-13*01, D1-26*01, IGHV1-8*01, IGHV4-61*01, IGHV6-1*01, IGHV4-4*02, IGHV1-3*01, IGHV3-66*03, IGHV3-7*01 and IGHV3-9*01.
   These are gene segments that very frequently combine with JH6*02 to produce productive heavy chains and antibodies.
   For example, the locus comprises one, more or all of human IGHV1-8*01, D3-9*01 and D3-10*01. These gene segments were productive with JH6*02 to produce HCDR3s of at least 20 amino acids in more than 10 antibodies.
15. An antibody-producing cell (eg, a B-cell) that is a progeny of the cell of any one of clauses 3 to 14, wherein the antibody-producing cell comprises a heavy chain locus comprising a rearranged variable region produced by recombination of human JH6*02 with a D segment and a VH segment (eg, JH6*02 with human VH3-11 (eg, VH3-11*01) and D3-9; VH3-20 (eg, VH3-20*01) and D3-10; VH4-4 (eg, VH4-4*02) and D3-10; VH3-9 (eg, VH3-9*01) and D3-10; or VH1-8 (eg, VH1-8*01) and D310).
   Such a variable region would be the product of *in vivo* somatic hypermutation in a non-hman vertebrate or cell of the invention.
16. The cell of clause 15, which is a B-cell or hybridoma that expresses a target antigen-specific antibody comprising a heavy chain that comprises a rearranged variable region produced by recombination of human JH6*02 with a D segment and a VH segment (eg, JH6*02 with human VH3-11 (eg, VH3-11*01) and D3-9; VH3-20 (eg, VH3-20*01) and D3-10; VH4-4 (eg, VH4-4*02) and D3-10; VH3-9 (eg, VH3-9*01) and D3-10; or VH1-8 (eg, VH1-8*01) and D310).
   Such a variable region would be the product of *in vivo* somatic hypermutation in a non-hman vertebrate or cell of the invention
17. The vertebrate or cell of any preceding clause, wherein the antibody heavy chain specifically binds a target antigen.
18. The vertebrate or cell of any preceding clause, wherein the antibody heavy chain has a HCDR3 length of at least 20 amino acids.
   Optionally, the HCDR3 length is at least 21, 22, 23, 24, 25, 26, 27, 28, 29 or 30 amino acids. Additionally, in one example the length is no more than 35, 34, 33, 32 or 31 amino acids. For example, the HCDR3 length is 20, 21, 22, 23 or 24 amino acids.
19. The vertebrate or cell of any preceding clause, wherein the antibody heavy chain is a product of the recombination of JH6*02 with a human VH gene segment recited in clause 13 or 14 and/or a D gene segment recited in clause 10, 11 or 14.
20. The vertebrate or cell of any preceding clause, wherein all endogenous non-human vertebrate heavy chain variable region gene segments have been inactivated in the genome (Eeg, by gene segment deletion or inversion).
21. The vertebrate or cell of any preceding clause, wherein the genome is homozygous for said heavy chain locus.
22. A heavy chain (eg, comprised by an antibody) isolated from a vertebrate of any one of clauses 1, 2, 5 to 14 and 17 to 21 wherein the heavy chain comprises a HCDR3 of at least 20 amino acids.
23. The heavy chain of clause 22, wherein the HCDR3 is the product of recombination of human JH6*02 with a human VH gene segment recited in clause 13 or 14 and/or a D gene segment recited in clause 10, 11 or 14.
   In an example, the heavy chain is chimaeric where the C region is non-human. In an example, the heavy chain is human where the C region is human.
24. A heavy chain (eg, comprised by an antibody) whose VH variable domain is identical to the VH variable domain of the heavy chain of clause 22 or 23, and which comprises a human constant region or a human-mouse chimaeric constant region (eg, CH1 is human and the other constant domains are mouse).
25. The heavy chain of clause 22, 23 or 24, whose VH variable domain is specific for a target antigen.
26. A method for producing a heavy chain, VH domain or an antibody specific to a target antigen, the method comprising immunizing a non-human vertebrate according to any one of clauses 1, 2, 5 to 14 and 17 to 21 with the antigen and isolating the heavy chain, VH domain or an antibody specific to a target antigen or a cell producing the heavy chain, VH domain or an antibody, wherein the heavy chain, VH domain or an antibody comprises a HCDR3 that is derived from the recombination of human JH6*02 with a VH gene segment and a D gene segment.
27. A method for producing a human heavy chain or antibody comprising carrying out the method of clause 26, wherein the constant region of the locus is a non-human vertebrate (eg, mouse or rat) constant region, and then replacing the non-human constant region of the isolated heavy chain or antibody with a human constant region (eg, by engineering of the nucleic acid encoding the antibody).
28. A heavy chain, VH domain or an antibody produced by the method of clause 26 or 27.
   Optionally the HCDR3 length is at least 20 amino acids as herein described.
29. A B-cell or hybridoma expressing a heavy chain VH domain that is identical to the VH domain of the heavy chain of clause 22, 23 or 28.
30. A nucleic acid encoding the VH domain of the heavy chain of clause 22, 23 or 28, or encoding the heavy chain of clause 22, 23, 24, 25 or 28.
31. A vector (eg, a CHO cell or HEK293 cell vector) comprising the nucleic acid of clause 30; optionally wherein the vector is in a host cell (eg, a CHO cell or HEK293 cell).
32. A pharmaceutical composition comprising the antibody, heavy chain or VH domain (eg, comprised by an antibody) of any one of clauses 22 to 25 and 28, together with a pharmaceutically-acceptable excipient, diluent or a medicament (eg, a further antigen-specific variable domain, heavy chain or antibody).
33. The antibody, heavy chain or VH domain (eg, comprised by an antibody) of any one of clauses 22 to 25 and 28 for use in medicine (eg, human medicine).

For example, the locus comprises the following human VH gene segments
IGHV6-1
IGHV3-7
IGHV1-8
IGHV3-9
IGHV3-11
IGHV3-13
IGHV1-18
IGHV3-30
IGHV4-31
IGHV4-39
IGHV4-59

Optionally also (i) and/or (ii)
(i)
   IGHV1-2
   IGHV2-5 and
   IGHV3-21
(ii)
   IGHV1-2
   IGHV2-5
   IGHV3-21
   IGHV1-24

For example, the locus comprises the following human VH gene segment variants
IGHV6-1*01
IGHV3-7*01
IGHV1-8*01
IGHV3-9*01
IGHV3-11*01
IGHV3-13*01
IGHV1-18*01
IGHV3-30*18
IGHV4-31*03
IGHV4-39*01 and
IGHV4-59*01;

Optionally also (iii) or (iv)
(ii)
   IGHV1-2*04
   IGHV2-5*10 and
   IGHV3-21*03
(iv)
   IGHV1-2*02
   IGHV2-5*01
   IGHV3-21*01 and
   IGHV1-24*01

For example, the locus comprises the following human JH gene segment variants
IGHJ2*01
IGHJ3*02
IGHJ4*02
IGHJ5*02 and
IGHJ6*02

For example, the locus comprises the following human D gene segments
IGHD1-1
IGHD2-2
IGHD3-9
IGHD3-10
IGHD5-12
IGHD6-13
IGHD1-14
IGHD2-15
IGHD3-16
IGHD4-17
IGHD6-19
IGHD2-21
IGHD5-24
IGHD1-26 and
IGHD7-27
and optionally also (v) or (vi)
(v)
   IGHD3-3
(vi)
   IGHD3-3
   IGHD4-4
   IGHD5-5
   IGHD6-6
   IGHD1-7
   IGHD2-8 and
   IGHD2-8

### The present invention provides in a fifth configuration:-

### Constant Regions Tailored to Human Use & Antibody Humanisation

Additional rational design and bioinformatics has led the inventors to realise that specific human constant region variants are conserved across many diverse human populations. The inventors realised that this opens up the possibility of making a choice to humanise antibodies, chains and variable domains by using such specific constant regions in products, rather than arbitrarily choosing the human constant region (or a synthetic version of a human constant region). This aspect of the invention also enables one to tailor antibody-based drugs to specific human ethnic populations, thereby more closely matching drug to patient (and thus disease setting) than has hitherto been performed. It can be a problem in the state of the art that antibodies are humanised with an arbitrary choice of human constant region (presumably derived from one (often unknown) ethnic population or non-naturally occurring) that does not function as well in patients of a different human ethnic population. This is important, since the constant region has the major role in providing antibody effector functions, eg, for antibody recycling, cellular and complement recruitment and for cell killing.

As discussed further in WO2011066501, human IgG sub-types IgG1, IgG2, gG3 and IgG4 exhibit differential capacity to recruit immune functions, such as antibody-dependent cellular cytotoxicity (ADCC, e.g., IgG1 and IgG3), antibody-dependent cellular phagocytosis (ADCP, e.g., IgG1, IgG2, IgG3 and IgG4), and complement dependent cytotoxicity (CDC, e.g., IgG1, IgG3). Sub-type-specific engagement of such immune functions is based on selectivity for Fc receptors on distinct immune cells and the ability to bind C1q and activate the assembly of a membrane attack complex (MAC). Among the various types, relative affinity for FcY receptors (e.g., FcΥRI, FcΥRIIa/b/c, FcΥRIIIa/b) is high for IgG1 and IgG3, however, there is minimal affinity for IgG2 (restricted to the FcΥRIIa 131H polymorphism), and IgG4 only has measurable affinity for FcYRI. Using comparative sequence analysis and co-crystal structures, the key contact residues for receptor binding have been mapped to the amino acid residues spanning the lower hinge and CH2 region. Using standard protein engineering techniques, some success in enhancing or reducing the affinity of an antibody preparation for Fc receptors and the C1q component of complement has been achieved.

Among the isotypes, IgG2 is least capable of binding the family of Fc receptors. Using IgG2 as the starting point, efforts have been made to find a mutant with diminished effector functions but which retains FcRn binding, prolonged stability, and low immunogenicity. Improved mutants of this nature may provide improved antibody therapeutics with retained safety. Human IgG1 therapeutic antibodies that bind to cell surface targets are able to engage effector cells that may mediate cell lysis of the target cell by antibody-dependent cellular cytotoxicity (ADCC) or complement dependent cytotoxicity (CDC). These mechanisms occur through interaction of the CH2 region of the antibody Fc domain to FcγR receptors on immune effector cells or with C1q, the first component of the complement cascade. Table 19 shows the activities of different human gamma sub-types. The skilled person may choose accordingly to promote or dampen-down activity depending upon the disease setting in humans of interest. For example, use of a human gamma-1 constant region is desirable when one wishes to isolated totally human heavy chains and antibodies that have relatively high complement activation activity by the classical pathway and FcΥR1 recognition in human patients. See also Mol Immunol. 2003 Dec;40(9):585-93; "Differential binding to human Fcgamma Rlla and Fcgamma Rllb receptors by human IgG wild type and mutant antibodies"; Armour KL *et al,* which is incorporated herein by reference.

IgG2 constant regions are well suited to producing antibodies and heavy chains according to the invention for binding to cytokines or soluble targets in humans, since IgG2 is essentially FcΥRI,III-silent, FcΥRIIa-active and has little Complement activity.

IgG1 constant regions have wide utility for human therapeutics, since IgG1 antibodies and heavy chains are FcΥRI,II,III- active and have complement activity. This can be enhanced by using a human gamma-1 constant region that has been activated by engineering as is known in the art.

The work of the inventors has therefore identified a collection of human constant region of different isotypes from which an informed choice can be made when humanising chimaeric antibody chains (or conjugating V domains, such as dAbs or *Camelid* VHH, to constant regions). The collection was identified on the basis of bioinformatics analysis of the 1000 Genomes database, the inventors selecting constant region variants that are frequently occurring across several human ethnic populations, as well as those that appear with relatively high frequency within individual populations (as assessed by the number of individuals whose genomes comprise the variant). By sorting through the myriad possible sequences on this basis, the inventors have provided a collection of human constant region variants that are naturally-occuring and which can be used when rationally designing antibodies, heavy chains and other antibody-based formats that bear a human constant region. In particular, this is useful when humanising chimaeric heavy chains to produce totally human chains in which both the variable and constant regions are human. This is useful for compatibility with human patients receiving antibody-based drugs.

To this end, the invention provides the following aspects:-
1. A method of producing an antibody heavy chain, the method comprising
   (a) providing an antigen-specific heavy chain variable domain (eg, VH (such as a human VH or dAb) or VHH or a humanised heavy chain variable domain); and
   (b) combining the variable domain with a human heavy chain constant region to produce an antibody heavy chain comprising (in N- to C-terminal direction) the variable domain and the constant region;
   wherein
   the human heavy chain constant region is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region.
   Step (b) can be carried out, eg, using recombinant DNA technology using the corresponding nucleotide sequences.
   For the constant region according to any aspect of this configuration, either genomic DNA or equivalent (ie, having introns and exons and optionally also 5' UTR sequences, eg, with native or a non-native leader sequence) can be used for the constant region. For example, any of the "GENOMIC" sequences disclosed as SEQ ID NO: 365 onwards herein. Alternatively, an intronless sequence can be used, for example any of the "CDS" sequences disclosed as SEQ ID NO: 365 onwards herein (eg, with native or a non-native leader sequence).
   Optionally for any aspect of this configuration of the invention, the human heavy chain constant region is an IGHAref constant region.
   Optionally for any aspect of this configuration of the invention, the human heavy chain constant region is an IGHA1a constant region.
   Optionally for any aspect of this configuration of the invention, the human heavy chain constant region is an IGHA2a constant region.
   Optionally for any aspect of this configuration of the invention, the human heavy chain constant region is an IGHA2b constant region.
   Optionally for any aspect of this configuration of the invention, the human heavy chain constant region is IGHG1ref constant region.
   Optionally for any aspect of this configuration of the invention, the human heavy chain constant region is an IGHG2ref constant region.
   Optionally for any aspect of this configuration of the invention, the human heavy chain constant region is an IGHG2a constant region.
   Optionally for any aspect of this configuration of the invention, the human heavy chain constant region is an IGHG3ref constant region.
   Optionally for any aspect of this configuration of the invention, the human heavy chain constant region is an IGHG3a constant region.
   Optionally for any aspect of this configuration of the invention, the human heavy chain constant region is an IGHG3b constant region.
   Optionally for any aspect of this configuration of the invention, the human heavy chain constant region is an IGHG4ref constant region.
   Optionally for any aspect of this configuration of the invention, the human heavy chain constant region is an IGHG4a constant region.
   Optionally for any aspect of this configuration of the invention, the human heavy chain constant region is an IGHDref constant region.
   Optionally for any aspect of this configuration of the invention, the human heavy chain constant region is an IGHEref constant region.
   Optionally for any aspect of this configuration of the invention, the human heavy chain constant region is an IGHMref constant region.
   Optionally for any aspect of this configuration of the invention, the human heavy chain constant region is an IGHMa constant region.
   Optionally for any aspect of this configuration of the invention, the human heavy chain constant region is an IGHMb constant region.
   Optionally, a derivative (eg, a mutant or conjugate) of the heavy chain or an antibody containing the heavy chain is produced. For example, a toxic payload can be conjugated (eg, for oncology applications). For example, one or more mutations can be introduced, as is known in the art, to inactivate or enhance Fc effector function.
2. The method of aspect 1, wherein the variable domain is a human variable domain.
   A human variable domain is, for example, the product of recombination in a transgenic non-human vertebrate of human VH, D and JH gene segments. Alternatively, the variable domain is identified using *in vitro* display technology from a human VH library, eg, using phage display, ribosome display or yeast display, as is known in the art.
   In another embodiment, the variable domain is a humanised variable domain, eg, comprising human frameworks with non-human (eg, mouse or rat) CDRs). Humanisation technology is conventional in the art, and will be readily known to the skilled person.
3. The method of any preceding aspect, wherein the variable domain has previously been selected from a non-human vertebrate that has been immunised with the antigen.
   For example, the vertebrate (such as a mouse or rat) genome comprises a chimaeric heavy chain locus comprising a human variable region (human V, D and JH gene segments) operably connected upstream of a non-human vertebrate constant region so that the locus is able to rearrange for the expression of heavy chains comprising human variable domains and non-human vertebrate constant regions.
   In alternative embodiments, the variable domain is selected using an *in vitro* technology such as phage display, ribosome display or yeast display. In this case the variable domain may be displayed with or without an constant region, provided that it is later combined with a human constant region as per the invention.
4. The method of any preceding aspect, comprising providing an expression vector (Eg, a mammalian expression vector, such as a CHO or HEK293 vector) comprising a nucleotide sequence encoding the constant region; inserting a nucleotide sequence encoding the variable domain into the vector 5' of the constant region sequence; inserting the vector into a host cell and expressing the heavy chain by the host cell; the method further comprising isolating a heavy chain (eg, as part of an antibody) comprising the variable domain and the human constant region.
   The vector comprises regulatory elements sufficient to effect expression of the heavy chain when the vector is harboured by a host cell, eg, a CHO or HEK293 cell.
5. The method of any preceding aspect, further comprising obtaining a nucleotide sequence encoding the heavy chain.
6. An antibody comprising a human heavy chain, the heavy chain comprising a variable domain that is specific for an antigen and a constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region.
7. A polypeptide comprising (in N- to C- terminal direction) a leader sequence, a human variable domain that is specific for an antigen and a human constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region; wherein (i) the leader sequence is not the native human variable domain leader sequence (eg, the leader sequence is another human leader sequence or a non-human leader sequence); and/or (ii) the variable domain comprises mouse AID-pattern somatic mutations or mouse terminal deoxynucleotidyl transferase (TdT)- pattern junctional mutations.
8. A nucleotide sequence encoding (in 5' to 3' direction) a leader sequence and a human antibody heavy chain, the heavy chain comprising a variable domain that is specific for an antigen and a constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region; and the leader sequence being operable for expression (eg, in a mammalian CHO or HEK293 cell) of the heavy chain and wherein the leader sequence is not the native human variable domain leader sequence (eg, the leader sequence is another human leader sequence or a non-human leader sequence).
   In an example, the leader sequence is ATGGGCTGGTCCTGCATCATCCTGTTTCTGGTGGCCACCGCCACCGGCGTGCACAGC
   Which translates to
   MGWSCIILFLVATATGVHS
9. A nucleotide sequence encoding (in 5' to 3' direction) a promoter and a human antibody heavy chain, the heavy chain comprising a variable domain that is specific for an antigen and a constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region; and the promoter being operable for expression (eg, in a mammalian CHO or HEK293 cell) of the heavy chain and wherein the promoter is not the native human promoter.
   In one embodiment, the promoter sequence is a human IGK 3-15 promoter.
10. The antibody, polypeptide or nucleotide sequence of any one of aspects 6 to 9, wherein the variable domain comprises mouse AID-pattern somatic mutations and/or mouse terminal deoxynucleotidyl transferase (TdT)- pattern junctional mutations.
   For example, one way, in any aspect of this configuration of the invention, to provide mouse AID-pattern somatic mutations and/or mouse terminal deoxynucleotidyl transferase (TdT)-pattern junctional mutations is to select a variable domain from a non-human vertebrate or cell. For example, a vertebrate or cell as disclosed herein.
11. A vector (eg, a CHO cell or HEK293 cell vector) comprising the nucleic acid of aspect 8, 9 or 10; optionally wherein the vector is in a host cell (eg, a CHO cell or HEK293 cell).
12. A pharmaceutical composition comprising the antibody or polypeptide of any one of aspects 6, 7 and 10, together with a pharmaceutically-acceptable excipient, diluent or a medicament (eg, a further antigen-specific variable domain, antibody chain or antibody).
13. The antibody or polypeptide of any one of aspects 6, 7 and 10 for use in treating and/or preventing a medical condition in a human patient.
14. Use of the antibody or polypeptide of any one of aspects 6, 7 and 10 for the manufacture of a medicament for treating and/or preventing a medical condition in a human patient.
15. The antibody, polypeptide or use of aspect 13 or 14, wherein the human is a member of a human population selected from population numbers 1-14, wherein the populations are numbered as follows (population labels being according to 1000 Genomes Project nomenclature)
   1= ASW;
   2= CEU;
   3=CHB;
   4=CHS;
   5=CLM;
   6=FIN;
   7=GBR;
   8=IBS;
   9=JPT;
   10=LWK;
   11=MXL;
   12=PUR;
   13=TSI;
   14=YRI.
16. The antibody, polypeptide or use of aspect 15, wherein the constant region is a
   (i) IGHA1a constant region and the human population is selected from any population number 1-14;
   (ii) IGHA2a constant region and the human population is selected from any population number 1-14;
   (ill) IGHA2b constant region and the human population is selected from any population number 1-14;
   (iV) IGHG2a constant region and the human population is selected from any population number 1-9 and 11-13;
   (v) IGHG3a constant region and the human population is selected from any population number 1-14;
   (vi) IGHG3b constant region and the human population is selected from any population number 1-8 and 11-13;
   (vii) IGHG4a constant region and the human population is selected from any population number 1-9 and 11-13;
   (viii)IGHMa constant region and the human population is selected from any population number 1-14; or
   (ix) IGHMb constant region and the human population is selected from any population number 1-14;
   Wherein the populations are numbered as follows (population labels being according to 1000 Genomes Project nomenclature)
   1= ASW;
   2= CEU;
   3=CHB;
   4=CHS;
   5=CLM;
   6=FIN;
   7=GBR;
   8=IBS;
   9=JPT;
   10=LWK;
   11=MXL;
   12=PUR;
   13=TSI;
   14=YRI.
17. A vector (eg, a CHO cell or HEK293 cell vector) comprising a IGHG1ref, IGHG2ref, IGHG2a, IGHG3ref, IGHG3a, IGHG3b, IGHG4ref or IGHG4a constant region nucleotide sequence that is 3' of a cloning site for the insertion of a human antibody heavy chain variable domain nucleotide sequence, such that upon insertion of such a variable domain sequence the vector comprises (in 5' to 3' direction) a promoter, a leader sequence, the variable domain sequence and the constant region sequence so that the vector is capable of expressing a human antibody heavy chain when present in a host cell.

### The present invention provides in a Sixth configuration:-

### Multiple Variants in the Same Genome Cis or Trans

The inventors' analysis has revealed groupings of naturally-occurring human antibody gene segment variants as set out in Table 13 and Table 14. This revealed the possibility of producing transgenic genomes in non-human vertebrates and cells wherein the genomes contain more than the natural human complement of specific human gene segments. In one example, this can be achieved by providing more than the natural human complement of a specific gene segment type on one or both of the respective Ig locus (eg, one or both chromosomes harbouring IgH in a mouse genome or mouse cell genome).

To this end, this configuration of the invention provides the following (as set out in numbered paragraphs):-
1. A non-human vertebrate (eg, a mouse or rat) or a non-human vertebrate cell (eg, an ES cell or a B-cell) having a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein at least two of the human gene segments are variants that are not identical to each other.
   For example, the genome comprises a variable region that comprises V, D and J gene segments (for the variable region of a heavy chain locus) or V and J gene segments (for the variable region of a light chain locus) upstream of a constant region for expression of heavy or light chains respectively.
   In an alternative, the skilled person can choose to provide more than the wild type human complement of a specific gene segment type by providing several copies of one variant type of the human gene segment. Thus, there is provided
   A non-human vertebrate (eg, a mouse or rat) or a non-human vertebrate cell (eg, an ES cell or a B-cell) having a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein the human gene segments are identical variants.
   For example, the genome comprises a variable region that comprises V, D and J gene segments (for the variable region of a heavy chain locus) or V and J gene segments (for the variable region of a light chain locus) upstream of a constant region for expression of heavy or light chains respectively.
2. A non-human vertebrate (eg, a mouse or rat) or a non-human vertebrate cell (eg, an ES cell or a B-cell) having a genome comprising at least 2 different non-endogenous variable region gene segments of the same type (eg, at least 2 human VH6-1 gene segments, at least 3 human JH6 gene segments, at least 2 human Vκ1-39 gene segments, at least 2 human D2-2 gene segments or at least 2 human Jκ1 gene segments) *cis* at the same Ig locus.
   In an alternative, the skilled person can choose to provide more than the wild type human complement of a specific gene segment type by providing several copies of one variant type of the human gene segment. Thus, there is provided
   A non-human vertebrate (eg, a mouse or rat) or a non-human vertebrate cell (eg, an ES cell or a B-cell) having a genome comprising at least 2 non-endogenous variable region gene segments of the same variant type (eg, at least 2 human JH6*02 gene segments) *cis* at the same Ig locus.
3. A non-human vertebrate (eg, a mouse or rat) or a non-human vertebrate cell (eg, an ES cell or a B-cell) having a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) *trans* at the same Ig locus; and optionally a third human gene segment of the same type, wherein the third gene segment is *cis* with one of said 2 different gene segments.
   In an alternative, the skilled person can choose to provide more than the wild type human complement of a specific gene segment type by providing several copies of one variant type of the human gene segment. Thus, there is provided
   A non-human vertebrate (eg, a mouse or rat) or a non-human vertebrate cell (eg, an ES cell or a B-cell) having a genome comprising at least 2 different human variable region gene segments of the same variant type (eg, at least 2 human JH6*02 gene segments) *trans* at the same Ig locus; and optionally a third human gene segment of the same variant type, wherein the third gene segment is *cis* with one of said 2 different gene segments.
4. A population of non-human vertebrates (eg, mice or rats) comprising a repertoire of human variable region gene segments, wherein the plurality comprises at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), a first of said different gene segments is provided in the genome of a first vertebrate of the population, and a second of said different gene segments being provided in the genome of a second vertebrate of the population, wherein the genome of the first vertebrate does not comprise the second gene segment.
5. A non-human vertebrate (eg, a mouse or rat) or a non-human vertebrate cell (eg, an ES cell or a B-cell) having a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.
6. A method of enhancing the human immunoglobulin gene diversity of a non-human vertebrate (eg, a mouse or rat), the method comprising providing the vertebrate with a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein at least two of the human gene segments are variants that are not identical to each other.
7. A method of enhancing the immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) *cis* at the same Ig locus.
8. A method of enhancing the immunoglobulin gene diversity of a non-human vertebrate (eg, a mouse or rat), the method comprising providing the vertebrate with a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) *trans* at the same Ig locus; and optionally a third human gene segment of the same type, wherein the third gene segment is *cis* with one of said 2 different gene segments.
9. A method of providing an enhanced human immunoglobulin variable region gene segment repertoire, the method comprising providing a population of non-human vertebrates (eg, a mouse or rat) comprising a repertoire of human variable region gene segments, wherein the method comprises providing at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein a first of said different gene segments is provided in the genome of a first vertebrate of the population, and a second of said different gene segments is provided in the genome of a second vertebrate of the population, wherein the genome of the first vertebrate does not comprise the second gene segment.
10. A method of enhancing the human immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.
11. The vertebrate, cell or method of any preceding paragraph, wherein at least 2 or 3 of said different gene segments are provided *cis* at the same Ig locus in said genome.
12. The vertebrate, cell or method of any preceding paragraph, wherein the gene segments are derived from the genome sequence of different human individuals that are not genetically related over at least 3 generations.
13. The vertebrate, cell or method of any preceding paragraph, wherein the gene segments are derived from the genome sequence of two or more different human individuals; optionally wherein the different human individuals are from different human populations.
14. The vertebrate, cell or method of paragraph 13, wherein the individuals are not genetically related.
15. A method of enhancing the human immunoglobulin gene diversity of a non-human vertebrate (eg, a mouse or rat), the method comprising providing the vertebrate with a genome comprising at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations; optionally wherein at least 2 or 3 of said different gene segments are provided at the same Ig locus in said genome.
16. The method of paragraph 15, wherein the different human individuals are from different human populations.
17. The method of paragraph 15, wherein the individuals are not genetically related.
18. The vertebrate, cell or method of preceding paragraph, wherein at least one of the different segments is a synthetic mutant of a human germline gene segment.
19. The vertebrate, cell or method of any preceding paragraph, wherein each of said gene segments occurs in 10 or more different human populations.
20. The vertebrate, cell or method of preceding paragraph, wherein each of said gene segments has a human frequency of 5% or greater (eg, 10, 20, 25, 30, 35, 40, 45, 50, 55, 60, 65, 70, 75, 80, 85, 90, or 95% or greater).

In this respect, the skilled person can be guided by the information provided in Table 14.

Frequency can, for example, be cumulative frequency in the 1000 Genomes database.
21. The vertebrate, cell or method of paragraph 20, wherein each of said gene segments occurs in 10 or more different human populations.
22. The vertebrate, cell or method of any preceding paragraph, wherein each of said gene segments occurs in the 1000 Genomes database in more than 50 individuals.
23. The vertebrate, cell or method of preceding paragraph, wherein each of said gene segments (i) has a human frequency of 5% or greater (eg, 10, 20, 25, 30, 35, 40, 45, 50, 55, 60, 65, 70, 75, 80, 85, 90, or 95% or greater); and (ii) occurs in 10 or more different human populations.
   In this respect, the skilled person can be guided by the information provided in Table 14.
   Frequency can, for example, be cumulative frequency in the 1000 Genomes database.
24. A non-human vertebrate (eg, a mouse or rat) or a non-human vertebrate cell (eg, an ES cell or a B-cell) having a genome comprising first and second human Ig locus gene segments of the same type (eg, first and second human JH6 gene segments; or first and second IgG2 gene segments; or first and second human Jλ7 gene segments), wherein the first gene segment is a gene segment selected from Table 14 (eg, IGHJ6-a) and the second gene segment is the corresponding reference sequence (eg, IGHJ6 ref; SEQ ID NO: 244).
   Table 14 lists commonly-occurring natural human variants. It can be seen that these occur across many human populations and thus usefully have wide applicability for human antibody-based drugs.
   For example, the gene segments are provided as targeted insertions into an endogenous non-human vertebrate Ig locus. Alternatively, random integration (eg, using YACs) as is know in the art can be performed.
   For example, the genome comprises a variable region that comprises V, D and J gene segments (for the variable region of a heavy chain locus) or V and J gene segments (for the variable region of a light chain locus) upstream of a constant region for expression of heavy or light chains respectively.
   In another embodiment, the invention enables the skilled person to select two or more different naturally-occurring human gene segment variants for combination into the genome of a non-human vertebrate or cell. A reference sequence need not be included. It may be desirable to use one or more rare gene segments to increase diversity of the repertoire. Additionally or alternatively, it may be desirable to include a mixture of frequent and rare variants of the same type to provide repertoire diversity. The variants may be chosen additionally or alternatively to tailor the gene segment inclusion to one or more specific human populations as indicated by the information provided in Table 13 or Table 14.
   Thus, the invention provides
   A non-human vertebrate (eg, a mouse or rat) or a non-human vertebrate cell (eg, an ES cell or a B-cell) having a genome comprising first and second human Ig locus gene segments of the same type (eg, first and second human JH6 gene segments; or first and second IgG2 gene segments; or first and second human Jλ7 gene segments), wherein the gene segments are gene segments selected from Table 13 or Table 14; and optionally wherein one or more of the gene segments appears in Table 14 (eg, IGHJ6-a) or is a reference sequence (eg, IGHJ6 ref; SEQ ID NO: 244).
25. The vertebrate or cell of paragraph 24, wherein the genome comprises a third human gene segment of said type, the third gene segment being different from the first and second gene segments.
26. The vertebrate or cell of paragraph 24 or 25, wherein the first and second gene segments are *cis* on the same chromosome; and optionally the third gene segment is also *cis* on said chromosome.
27. The vertebrate or cell of paragraph 26, wherein the gene segments are targeted insertions into an endogenous non-human Ig locus.
   For example, the gene segments are heavy chain gene segments and the non-human locus is an IgH locus. For example, the gene segments are light chain (kappa or lambda) gene segments and the non-human locus is an IgL locus.
28. The vertebrate or cell of paragraph 24 or 25, wherein the first and second gene segments are *trans* on different chromosomes.
   Thus, the chromosomes are the same type (eg, both mouse chromosome 6 or rat chromosome 4).
29. The vertebrate or cell of any one of paragraphs 24 to 28, wherein the first gene segment is a gene segment selected from any one of Tables 1 to 7 and 9 to 14 (eg, selected from Table 13 or 14) and the second gene segment is the corresponding reference sequence.
30. A population of non-human vertebrates (eg, mice or rats) comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 to 7 and 9 to 14 (eg, Table 13 or 14) (eg, IGHJ6-a) and the second gene segment is the corresponding reference sequence (eg, SEQ ID NO: 7), wherein the first gene segment is provided in the genome of a first vertebrate of the population, and the second gene segment is provided in the genome of a second vertebrate of the population.
31. The population of paragraph 30, wherein the genome of the first vertebrate does not comprise the second gene segment.
32. The population of paragraph 30 or 31, wherein the population comprises a third human gene segment of said type, the third gene segment being different from the first and second gene segments and optionally wherein the first and third gene segments are present in the genome of the first vertebrate.
33. The population of paragraph 30, 31 or 32, wherein the gene segments are targeted insertions into an endogenous non-human Ig locus in the respective genome.
   For example, the gene segments are heavy chain gene segments and the non-human locus is an IgH locus. For example, the gene segments are light chain (kappa or lambda) gene segments and the non-human locus is an IgL locus.
34. The population of of any one of paragraphs 30 to 33, wherein the first gene segment is a gene segment selected from any one of Tables 1 to 7 and 9 to 14 (eg, Table 13 or 14) and the second gene segment is the corresponding reference sequence.
35. A method of enhancing the human immunoglobulin gene diversity of a non-human vertebrate (eg, a mouse or rat), the method comprising providing the vertebrate with a genome comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 to 7 and 9 to 14 (eg, Table 13 or 14) (eg, IGHJ6-a) and the second gene segment is the corresponding reference sequence (eg, SEQ ID NO: 7).
36. A method of providing an enhanced human immunogolobulin gene segment repertoire, the method comprising providing a population according to any one of paragraphs 30 to 33.

### Variants Prevalent in Few Populations

In another aspect, it is of note that certain human gene segment variants may appear relatively frequently in one or a small number of populations, but is not found prevalently across many different human populations. There is thinking that specific germline gene segment repertoires have evolved in individual human ethnic populations due to iterative exposure to antigens (eg, disease pathogen antigens) to which the population is often exposed. Repeated exposure and mutation may have lead to the evolution of gene segment variants that can provide an effective response to the antigen (pathogen) in the population, and this may explain the conservation of the gene segments in those populations (as opposed to other human ethnic populations that may not have frequently encountered the antigen). With this in mind, the inventors identified gene segment variants from their analysis that are relatively prevalent in a small number of human populations, and not across many populations. The inventors realized that inclusion of one or more of such gene segments in the configurations of the invention (eg, in transgenic Ig loci, vertebrates and cells) would be useful for producing antibodies, Ig chains and variable domains that can address antigens (eg, disease-causing antigens or pathogens) to which the small number of human populations may become exposed. Such products would be useful for treating and/or preventing disease or medical conditions in members of such a population. This aspect could also be useful for addressing infectious disease pathogens that may have been common in the small number of populations, but which in the future or relatively recently in evolution has become a more prevalent disease-causing pathogen in other human populations (ie, those not listed in Table 13 against the gene segment variant(s) in question). To this end, from the 1000 Genomes database the inventors have identified the gene segment variants listed in Table 20.

Thus, according to any configuration or aspect described herein, one, more or all of the gene segments used in the present invention can be a gene segment listed in Table 20A, 20B, 20C or 20D.

### Multiple JH Gene Segment Variants

A specific application of this configuration is the provision of multiple human JH gene segments as follows.

A non-human vertebrate (eg, a mouse or rat) or a non-human vertebrate cell (eg, an ES cell or a B-cell) having a genome comprising at least 3 human JH gene segments of the same type (JH1, JH2, JH3, JH4, JH5 or JH6), wherein at least two of the human JH gene segments are variants that are not identical to each other.

In an example, any cell of the invention is an isolated cell. An "isolated" cell is one that has been identified, separated and/or recovered from a component of its production environment (eg, naturally or recombinantly). Preferably, the isolated cell is free of association with all other components from its production environment, eg, so that the cell can produce an antibody to an FDA-approvable or approved standard. Contaminant components of its production environment, such as that resulting from recombinant transfected cells, are materials that would typically interfere with research, diagnostic or therapeutic uses for the resultant antibody, and may include enzymes, hormones, and other proteinaceous or non-proteinaceous solutes. In preferred embodiments, the polypeptide will be purified: (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of a spinning cup sequenator, or (3) to homogeneity by SDS-PAGE under nonreducing or reducing conditions using Coomassie blue or, preferably, silver stain. Ordinarily, however, an isolated cell will be prepared by at least one purification step.

A non-human vertebrate (eg, a mouse or rat) or a non-human vertebrate cell (eg, an ES cell or a B-cell) having a genome comprising at least 2 different non-endogenous JH gene segments (eg, human gene segments) of the same type (JH1, JH2, JH3, JH4, JH5 or JH6) *cis* at the same Ig (eg, IgH, eg, endogenous IgH, eg, mouse or rat IgH) locus. In an example, the genome comprises a human VH, D and JH repertoire comprising said different JH gene segments. Optionally the non-endogenous JH gene segments are non-mouse or non-rat, eg, human JH gene segments. In an example one or more or all of the non-endogenous gene segments are synthetic.

A non-human vertebrate (eg, a mouse or rat) or a non-human vertebrate cell (eg, an ES cell or a B-cell) having a genome comprising at least 2 different human JH gene segments of the same type (JH1, JH2, JH3, JH4, JH5 or JH6) *trans* at the same Ig (eg, IgH, eg, endogenous IgH, eg, mouse or rat IgH) locus; and optionally a third human JH gene segments of the same type, wherein the third JH is *cis* with one of said 2 different JH gene segments.

A population of non-human vertebrates (eg, mice or rats) comprising a repertoire of human JH gene segments, wherein the plurality comprises at least 2 different human JH gene segments of the same type (JH1, JH2, JH3, JH4, JH5 or JH6), a first of said different JH gene segments is provided in the genome of a first vertebrate of the population, and a second of said different JH gene segments being provided in the genome of a second vertebrate of the population, wherein the genome of the first vertebrate does not comprise the second JH gene segment.

A non-human vertebrate (eg, a mouse or rat) or a non-human vertebrate cell (eg, an ES cell or a B-cell) having a genome comprising at least 2 different non-endogenous (eg, human) JH gene segments of the same type (JH1, JH2, JH3, JH4, JH5 or JH6), wherein the JH gene segments are derived from the genome sequence of different human individuals that are not genetically related over at least 3 generations (eg, 3, 4, 5 or 6 generations). Optionally the non-endogenous JH gene segments are human JH gene segments. In an example one or more or all of the non-endogenous gene segments are synthetic.

A method of enhancing the human immunoglobulin gene diversity of a non-human vertebrate (eg, a mouse or rat), the method comprising providing the vertebrate with a genome comprising at least 3 human JH gene segments of the same type (JH1, JH2, JH3, JH4, JH5 or JH6), wherein at least two of the human JH gene segments are variants that are not identical to each other.

A method of enhancing the immunoglobulin gene diversity of a non-human vertebrate (eg, a mouse or rat), the method comprising providing the vertebrate with a genome comprising at least 2 different non-endogenous (eg, human) JH gene segments of the same type (JH1, JH2, JH3, JH4, JH5 or JH6) *cis* at the same Ig (eg, IgH, eg, endogenous IgH, eg, mouse or rat IgH) locus). Optionally the non-endogenous JH gene segments are non-mouse or non-rat, eg, human JH gene segments. In an example one or more or all of the non-endogenous gene segments are synthetic.

A method of enhancing the immunoglobulin gene diversity of a non-human vertebrate (eg, a mouse or rat), the method comprising providing the vertebrate with a genome comprising at least 2 different human JH gene segments of the same type (JH1, JH2, JH3, JH4, JH5 or JH6) *trans* at the same Ig (eg, IgH, eg, endogenous IgH, eg, mouse or rat IgH) locus; and optionally a third human JH gene segments of the same type, wherein the third JH is *cis* with one of said 2 different JH gene segments.

A method of providing an enhanced human immunoglobulin JH gene segment repertoire, the method comprising providing a population of non-human vertebrates (eg, a mouse or rat) comprising a repertoire of human JH gene segments, wherein the method comprises providing at least 2 different human JH gene segments of the same type (JH1, JH2, JH3, JH4, JH5 or JH6), wherein a first of said different JH gene segments is provided in the genome of a first vertebrate of the population, and a second of said different JH gene segments is provided in the genome of a second vertebrate of the population, wherein the genome of the first vertebrate does not comprise the second JH gene segment.

A method of enhancing the human immunoglobulin gene diversity of a non-human vertebrate (eg, a mouse or rat), the method comprising providing the vertebrate with a genome comprising at least 2 different non-endogenous (eg, human) JH gene segments of the same type (JH1, JH2, JH3, JH4, JH5 or JH6), wherein the JH gene segments are derived from the genome sequence of different human individuals that are not genetically related over at least 3 generations (eg, 3, 4, 5, or 6 generations). Optionally the non-endogenous JH gene segments are human JH gene segments. In an example one or more or all of the non-endogenous gene segments are synthetic.

In an example of the vertebrate or cell or the method of the invention at least 2 or 3 of said different gene segments are provided *cis* at the same Ig locus in said genome.

In an example of the vertebrate or cell or the method of the invention the JH gene segments are derived from the genome sequence of different human individuals that are not genetically related over at least 3 generations (eg, 3, 4, 5, or 6 generations).

In an example of the vertebrate or cell or the method of the invention the JH gene segments are derived from the genome sequence of two or more different human individuals; optionally wherein the different human individuals are from different human populations.

In an example of the vertebrate or cell or the method of the invention the individuals are not genetically related (eg, going back 3, 4, 5, or 6 generations).

In an example of the vertebrate or cell or the method of the invention at least one of the different JH segments is a synthetic mutant of a human germline JH gene segment.

The invention also provides a method of enhancing the human immunoglobulin gene diversity of a non-human vertebrate (eg, a mouse or rat), the method comprising providing the vertebrate with a genome comprising at least 2 human JH gene segments of the same type (JH1, JH2, JH3, JH4, JH5 or JH6), wherein the JH gene segments are derived from the genome sequence of different human individuals that are not genetically related over at least 3 generations (eg, 3, 4, 5, or 6 generations); optionally wherein at least 2 or 3 of said different gene segments are provided at the same IgH locus in said genome.

In an example of the vertebrate or cell or the method of this embodiment of the invention the genome comprises a substantially complete functional repertoire of human JH gene segment types supplemented with one, two or more human JH gene segments, wherein said substantially complete functional repertoire and the supplementary JH gene segments are not found together in the germline genome of a human individual.

In an example of the population of the invention, the population comprises a substantially complete functional repertoire of human JH gene segment types supplemented with one, two or more human JH gene segments, wherein said substantially complete functional repertoire and the supplementary JH gene segments are not found together in the germline genome of a human individual.

A non-human vertebrate (eg, a mouse or rat) or a non-human cell (eg, an ES cell or a B-cell) having a genome comprising a substantially complete functional repertoire of human JH gene segment types supplemented with one, two or more human JH gene segments, wherein said substantially complete functional repertoire and the supplementary JH gene segments are not found together in the germline genome of a human individual.

A population of non-human vertebrates (eg, mice or rats) comprising a substantially complete functional repertoire of human JH gene segment types supplemented with one, two or more human JH gene segments, wherein said substantially complete functional repertoire and the supplementary JH gene segments are not found together in the germline genome of a human individual.

In an example of the vertebrate or the population, at least one of said JH gene segments is SEQ ID NO: 1, 2, 3 or 4. For example, at least one of said JH gene segments is SEQ ID NO: 1 and at least one, two or more of said supplementary JH gene segments is a variant according to any example above. For example, at least one of said JH gene segments is SEQ ID NO: 2 and at least one, two or more of said supplementary JH gene segments is a variant according to any one of the examples above. For example, at least one of said JH gene segments is SEQ ID NO: 2 and at least one, two or more of said supplementary JH gene segments is a variant according to any one of the examples above.

In an embodiment, the non-human vertebrate or vertebrate cell of the invention comprises a genome that comprises VH, D and JH gene repertoires comprising human gene segments, the JH gene repertoire (eg, a human JH gene segment repertoire) comprising
a plurality of JH1 gene segments provided by at least 2 different JH1 gene segments in *cis* at the same Ig locus in said genome;
a plurality of JH2 gene segments provided by at least 2 different JH2 gene segments in *cis* at the same Ig locus in said genome;
a plurality of JH3 gene segments provided by at least 2 different JH3 gene segments in *cis* at the same Ig locus in said genome;
a plurality of JH4 gene segments provided by at least 2 different JH4 gene segments in *cis* at the same Ig locus in said genome;
a plurality of JH5 gene segments provided by at least 2 different JH5 gene segments in *cis* at the same Ig locus in said genome; and/or
a plurality of JH6 gene segments provided by at least 2 different JH6 gene segments in *cis* at the same Ig locus in said genome;
optionally wherein the JH gene segments are derived from the genome sequence of two or more different human individuals.

Optionally said at least 2 different JH gene segments are human gene segments or synthetic gene segments derived from human gene segments.

Optionally, the Ig locus is a IgH locus, eg, an endogenous locus, eg, a mouse or rat IgH locus.

In an embodiment, the non-human vertebrate or vertebrate cell of the invention comprises a genome that comprises VH, D and JH gene repertoires comprising human gene segments, the JH gene repertoire (eg, a human JH gene segment repertoire) comprising
a plurality of JH1 gene segments provided by at least 3 different JH1 gene segments;
a plurality of JH2 gene segments provided by at least 3 different JH2 gene segments;
a plurality of JH3 gene segments provided by at least 3 different JH3 gene segments;
a plurality of JH4 gene segments provided by at least 3 different JH4 gene segments;
a plurality of JH5 gene segments provided by at least 3 different JH5 gene segments; and/or
a plurality of JH6 gene segments provided by at least 3 different JH6 gene segments;
optionally wherein the JH gene segments are derived from the genome sequence of two or three different human individuals;
optionally wherein at least 2 or 3 of said different gene segments are provided in *cis* at the same Ig locus in said genome.

Optionally said at least 3 different JH gene segments are human gene segments or synthetic gene segments derived from human gene segments.

Optionally, the Ig locus is a IgH locus, eg, an endogenous locus, eg, a mouse or rat IgH locus.

Optionally in the vertebrate or cell the different human individuals are from different human populations.

Optionally in the vertebrate or cell the individuals are not genetically related (eg, Going back 3, 4, 5 or 6generations).

Optionally in the vertebrate or cell at least one of the different JH segments is a synthetic mutant of a human germline JH gene segment.

In an embodiment of a non-human vertebrate or vertebrate cell (optionally an ES cell or B-cell) according to the invention, the vertebrate or cell genome comprises human VH, D and JH gene repertoires, the JH gene repertoire (eg, a human JH gene repertoire) comprising a plurality of JH1 gene segments provided by at least 2 different human JH1 gene segments, optionally in *cis* at the same Ig locus in said genome;
a plurality of JH2 gene segments provided by at least 2 different human JH2 gene segments, optionally in *cis* at the same Ig locus in said genome;
a plurality of JH3 gene segments provided by at least 2 different human JH3 gene segments, optionally in *cis* at the same Ig locus in said genome;
a plurality of JH4 gene segments provided by at least 2 different human JH4 gene segments, optionally in *cis* at the same Ig locus in said genome;
a plurality of JH5 gene segments provided by at least 2 different human JH5 gene segments, optionally in *cis* at the same Ig locus in said genome; and/or
a plurality of JH6 gene segments provided by at least 2 different human JH6 gene segments, optionally in *cis* at the same Ig locus in said genome;
wherein the JH gene segments are derived from the genome sequence of different human individuals that are not genetically related over at least 3 generations (eg, 3, 4, 5 or 6 generations).

Optionally said at least 2 different JH gene segments are human gene segments or synthetic gene segments derived from human gene segments.

Optionally, the Ig locus is a IgH locus, eg, an endogenous locus, eg, a mouse or rat IgH locus.

Optionally in the vertebrate or cell the human individuals are from different human populations.

### JH5

An embodiment provides a vertebrate, cell or population of the invention whose genome comprises a plurality of JH5 gene segments, wherein the plurality comprises a human JH5 gene variant of SEQ ID NO: 1, wherein the variant comprises a nucleotide mutation at one or more positions corresponding to positions
106,330,024
106,330,027
106,330,032
106,330,041
106,330,044
106,330,045
106,330,062
106,330,063
106,330,065
106,330,066
106,330,067
106,330,068 and
106,330,071
on human chromosome 14.

In the vertebrate, cell or population optionally the plurality comprises a human JH5 gene variant of SEQ ID NO: 1, wherein the variant comprises a guanine at a position corresponding to position 106,330,067 on human chromosome 14; and optionally no further mutation from the sequence of SEQ ID NO: 1.

Optionally the variant comprises additionally a mutation at a position corresponding to (i) position 106,330,071 on human chromosome 14 (optionally the additional mutation being a guanine); (ii) position 106,330,066 on human chromosome 14 (optionally the additional mutation being a guanine); and/or (iii) position 106,330,068 on human chromosome 14 (optionally the additional mutation being a thymine).

Optionally the plurality comprises a human JH5 gene variant of SEQ ID NO: 1, wherein the variant comprises a guanine at a position corresponding to position 106,330,071 on human chromosome 14; and optionally no further mutation from the sequence of SEQ ID NO: 1.

Optionally the variant comprises additionally a mutation at a position corresponding to (i) position 106,330,063 on human chromosome 14 (optionally the additional mutation being an adenine); and/or (ii) position 106,330,067 on human chromosome 14 (optionally the additional mutation being a guanine).

Optionally the plurality comprises a human JH5 gene variant of SEQ ID NO: 1, wherein the variant comprises a cytosine at a position corresponding to position 106,330,045 on human chromosome 14; and optionally no further mutation from the sequence of SEQ ID NO: 1.

Optionally the plurality comprises a human JH5 gene variant of SEQ ID NO: 1, wherein the variant comprises an adenine at a position corresponding to position 106,330,044 on human chromosome 14; and optionally no further mutation from the sequence of SEQ ID NO: 1.

Optionally the variant comprises additionally a mutation at a position corresponding to (i) position 106,330,066 on human chromosome 14 (optionally the additional mutation being a guanine); and/or (ii) position 106,330,068 on human chromosome 14 (optionally the additional mutation being a thymine).

Optionaly the plurality comprises a human JH5 gene variant of SEQ ID NO: 1, wherein the variant comprises a guanine at a position corresponding to position 106,330,066 on human chromosome 14; and optionally no further mutation from the sequence of SEQ ID NO: 1.

Optionally the variant comprises additionally a mutation at a position corresponding to (i) position 106,330,067 on human chromosome 14 (optionally the additional mutation being a guanine); and/or (ii) position 106,330,068 on human chromosome 14 (optionally the additional mutation being a thymine).

Optionally the plurality comprises a human JH5 gene variant of SEQ ID NO: 1, wherein the variant comprises a thymine at a position corresponding to position 106,330,068 on human chromosome 14; and optionally no further mutation from the sequence of SEQ ID NO: 1.

Optionally the variant comprises additionally a mutation at a position corresponding to (i) position 106,330,067 on human chromosome 14 (optionally the additional mutation being a guanine); and/or (ii) position 106,330,066 on human chromosome 14 (optionally the additional mutation being a guanine).

Optionally the plurality comprises a human JH5 gene variant of SEQ ID NO: 1, wherein the variant comprises a cytosine at a position corresponding to position 106,330,027 on human chromosome 14; and optionally no further mutation from the sequence of SEQ ID NO: 1.

Optionally the plurality comprises a human JH5 gene variant of SEQ ID NO: 1, wherein the variant comprises an adenine at a position corresponding to position 106,330,024 on human chromosome 14; and optionally no further mutation from the sequence of SEQ ID NO: 1.

Optionally the plurality comprises a human JH5 gene variant of SEQ ID NO: 1, wherein the variant comprises a thymine at a position corresponding to position 106,330,032 on human chromosome 14; and optionally no further mutation from the sequence of SEQ ID NO: 1.

Optionally the plurality comprises a human JH5 gene variant of SEQ ID NO: 1, wherein the variant comprises a thymine at a position corresponding to position 106,330,041 on human chromosome 14; and optionally no further mutation from the sequence of SEQ ID NO: 1.

Optionally the plurality comprises a human JH5 gene variant of SEQ ID NO: 1, wherein the variant comprises an adenine or thymine at a position corresponding to position 106,330,063 on human chromosome 14; and optionally no further mutation from the sequence of SEQ ID NO: 1.

Optionally the variant comprises additionally a mutation at a position corresponding to position 106,330,071 on human chromosome 14 (optionally the additional mutation being a guanine).

Optionally the plurality comprises a human JH5 gene variant of SEQ ID NO: 1,wherein the variant comprises a cytosine at a position corresponding to position 106,330,062 on human chromosome 14; and optionally no further mutation from the sequence of SEQ ID NO: 1.

Optionally the genome comprises SEQ ID NO:1; optionally in *cis* at the same Ig locus as one, two or more of the variants.

### JH6

An embodiment provides a vertebrate, cell or population of the invention whose genome comprises a plurality of JH6 gene segments, wherein the plurality comprises a human JH6 gene variant of SEQ ID NO: 2, wherein the variant comprises a nucleotide mutation at one or more positions corresponding to positions
106,329,411
106,329,413
106,329,414
106,329,417
106,329,419
106,329,426
106,329,434
106,329,435, and
106,329,468
on human chromosome 14.

Optionally the genome of the vertebrate, cell or population comprises a plurality of JH6 gene segments, wherein the plurality comprises a human JH6 gene variant of SEQ ID NO: 2, wherein the variant comprises a guanine at a position corresponding to position 106,329,435 on human chromosome 14; and optionally no further mutation from the sequence of SEQ ID NO: 2.

Optionally the variant comprises additionally a mutation at a position corresponding to (i) position 106,329,468 on human chromosome 14 (optionally the additional mutation being a guanine); (ii) position 106,329,419 on human chromosome 14 (optionally the additional mutation being an adenine); (iii) position 106,329,434 on human chromosome 14 (optionally the additional mutation being a cytosine) and/or position 106,329,414 on human chromosome 14 (optionally the additional mutation being a guanine); (iv) position 106,329,426 on human chromosome 14 (optionally the additional mutation being an adenine); (v) position 106,329,413 on human chromosome 14 (optionally the additional mutation being an adenine); (vi) position 106,329,417 on human chromosome 14 (optionally the additional mutation being a thymine); (vii) position 106,329,411 on human chromosome 14 (optionally the additional mutation being a thymine); (viii) position 106,329,451 on human chromosome 14 (optionally the additional mutation being an adenine); (ix) position 106,329,452 on human chromosome 14 (optionally the additional mutation being a cytosine); and/or (x) position 106,329,453 on human chromosome 14 (optionally the additional mutation being a cytosine).

Optionally the variant comprises additionally mutations at positions corresponding to position 106,329,451 on human chromosome 14, the additional mutation being an adenine; position 106,329,452 on human chromosome 14, the additional mutation being a cytosine; and position 106,329,453 on human chromosome 14, the additional mutation being a cytosine.

The vertebrate, cell or population optionally comprises a plurality of JH6 gene segments,, wherein the plurality comprises a human JH6 gene variant of SEQ ID NO: 2, wherein the variant comprises a guanine at a position corresponding to position 106,329,468 on human chromosome 14; and optionally no further mutation from the sequence of SEQ ID NO: 2.

Optionally the variant comprises additionally a mutation at a position corresponding to position 106,329,435 on human chromosome 14 (optionally the additional mutation being a guanine).

Optionally the vertebrate, cell or population comprises a plurality of JH6 gene segments,, wherein the plurality comprises a human JH6 gene variant of SEQ ID NO: 2, wherein the variant comprises a thymine at a position corresponding to position 106,329,417 on human chromosome 14; and optionally no further mutation from the sequence of SEQ ID NO: 2.

Optionally the variant comprises additionally a mutation at a position corresponding to position 106,329,435 on human chromosome 14 (optionally the additional mutation being a guanine).

Optionally the vertebrate, cell or population comprises a plurality of JH6 gene segments,, wherein the plurality comprises a human JH6 gene variant of SEQ ID NO: 2, wherein the variant comprises a cytosine at a position corresponding to position 106,329,434 on human chromosome 14; and optionally no further mutation from the sequence of SEQ ID NO: 2.

Optionally the variant comprises additionally a mutation at a position corresponding to (i) position 106,329,414 on human chromosome 14 (optionally the additional mutation being a guanine); and/or (ii) position 106,329,435 on human chromosome 14 (optionally the additional mutation being a guanine).

Optionally the vertebrate, cell or population comprises a plurality of JH6 gene segments, wherein the plurality comprises a human JH6 gene variant of SEQ ID NO: 2, wherein the variant comprises a thymine at a position corresponding to position 106,329,411 on human chromosome 14; and optionally no further mutation from the sequence of SEQ ID NO: 2.

Optionally the variant comprises additionally a mutation at a position corresponding to position 106,329,435 on human chromosome 14 (optionally the additional mutation being a guanine).

Optionally the vertebrate, cell or population comprises a plurality of JH6 gene segments, wherein the plurality comprises a human JH6 gene variant that is an antisense sequence of a variant described above.

Optionally the genome comprises SEQ ID NO:2; optionally *cis* at the same Ig locus as one, two or more of the JH6 variants.

### JH2

An embodiment provides a vertebrate, cell or population of the invention whose genome comprises a plurality of JH2 gene segments, wherein the plurality comprises a human JH2 gene variant of SEQ ID NO: 3, wherein the variant comprises a nucleotide mutation at one or more positions corresponding to positions
106,331,455
106,331,453, and
106,331,409
on human chromosome 14.

Optionally the vertebrate, cell or population comprises said plurality of JH2 gene segments, wherein the plurality comprises a human JH2 gene variant of SEQ ID NO: 3, wherein the variant comprises a guanine at a position corresponding to position 106,331,455 on human chromosome 14; and optionally no further mutation from the sequence of SEQ ID NO: 3.

Optionally the variant comprises additionally a mutation at a position corresponding to (i) position 106,331,453 on human chromosome 14 (optionally the additional mutation being an adenine); and/or (ii) position 106,331,409 on human chromosome 14 (optionally the additional mutation being an adenine); (iii) position 106,329,434 on human chromosome 14 (optionally the additional mutation being an adenine).

Optionally the vertebrate, cell or population comprises a plurality of JH2 gene segments, wherein the plurality comprises a human JH2 gene variant of SEQ ID NO: 3, wherein the variant comprises an adenine at a position corresponding to position 106,331,453 on human chromosome 14; and optionally no further mutation from the sequence of SEQ ID NO: 3.

Optionally the variant comprises additionally a mutation at a position corresponding to position 106,331,409 on human chromosome 14 (optionally the additional mutation being an adenine).

Optionally the vertebrate, cell or population comprises a plurality of JH2 gene segments, wherein the plurality comprises a human JH2 gene variant of SEQ ID NO: 3, wherein the variant comprises an adenine at a position corresponding to position 106,331,409 on human chromosome 14; and optionally no further mutation from the sequence of SEQ ID NO: 3.

Optionally the vertebrate, cell or population comprises a plurality of JH2 gene segments, wherein the plurality comprises a human JH2 gene variant that is an antisense sequence of a variant described above.

Optionally the genome comprises SEQ ID NO:3; optionally *cis* at the same Ig locus as one, two or more of the JH2 variants.

Optionally the vertebrate, cell or population genome comprises two or more different JH gene segments selected from SEQ ID NOs: 1 to 3 and variants described above; optionally wherein said JH gene segments are *cis* at the same immunoglobulin Ig locus.

### Multiple Human D Gene Segment Variants

A specific application of this configuration is the provision of multiple human D gene segments as follows (as set out in numbered clauses, starting at clause number 154).
154. A non-human vertebrate (eg, a mouse or rat) or a non-human vertebrate cell (eg, an ES cell or a B-cell) having a genome comprising at least 3 human D gene segments of the same type (eg, D2-2 gene segments), wherein at least two of the human D gene segments are variants that are not identical to each other (eg, D2-2ref and D2-2a).
   In an example of any aspect of the sixth configuration of the invention (V, D, J or C), one or more or all of the variants are naturally-occurring human gene segments.
   In an example of any aspect of the sixth configuration of the invention (V, D, J or C), one or more of the variants may be a synthetic variant of a human gene segment.
155. A non-human vertebrate (eg, a mouse or rat) or a non-human vertebrate cell (eg, an ES cell or a B-cell) having a genome comprising at least 2 different non-endogenous D gene segments of the same type type (eg, D2-2ref and D2-2a) *cis* at the same Ig locus.
156. A non-human vertebrate (eg, a mouse or rat) or a non-human vertebrate cell (eg, an ES cell or a B-cell) having a genome comprising at least 2 different human D gene segments of the same type (eg, D2-2ref and D2-2a) *trans* at the same Ig locus; and optionally a third human D gene segment (eg, (eg, D2-2ref, D2-2a or D2-2b) of the same type, wherein the third D is *cis* with one of said 2 different D gene segments.
157. A population of non-human vertebrates (eg, mice or rats) comprising a repertoire of human D gene segments, wherein the plurality comprises at least 2 different human D gene segments of the same type (eg, D2-2 gene segments), a first of said different D gene segments (eg, D2-2ref) is provided in the genome of a first vertebrate of the population, and a second of said different D gene segment (eg, D2-2a) being provided in the genome of a second vertebrate of the population, wherein the genome of the first vertebrate does not comprise the second D gene segment.
158. A non-human vertebrate (eg, a mouse or rat) or a non-human vertebrate cell (eg, an ES cell or a B-cell) having a genome comprising at least 2 different non-endogenous D gene segments of the same type (eg, human D2-2 gene segments), wherein the D gene segments are derived from the genome sequence of different human individuals that are not genetically related over at least 3 generations.
159. A method of enhancing the human immunoglobulin gene diversity of a non-human vertebrate (eg, a mouse or rat), the method comprising providing the vertebrate with a genome comprising at least 3 human D gene segments of the same type (eg, D2-2 gene segments), wherein at least two of the human D gene segments are variants that are not identical to each other (eg, D2-2ref and D2-2a).
160. A method of enhancing the immunoglobulin gene diversity of a non-human vertebrate (eg, a mouse or rat), the method comprising providing the vertebrate with a genome comprising at least 2 different non-endogenous D gene segments of the same type (eg, human D2-2 gene segments) *cis* at the same Ig locus.
161. A method of enhancing the immunoglobulin gene diversity of a non-human vertebrate (eg, a mouse or rat), the method comprising providing the vertebrate with a genome comprising at least 2 different human D gene segments of the same type (eg, D2-2ref and D2-2a) *trans* at the same Ig locus; and optionally a third human D gene segment (eg, D2-2ref, D2-2a or D2-2b) of the same type, wherein the third D is *cis* with one of said 2 different D gene segments.
162. A method of providing an enhanced human immunoglobulin D gene segment repertoire, the method comprising providing a population of non-human vertebrates (eg, a mouse or rat) comprising a repertoire of human D gene segments, wherein the method comprises providing at least 2 different human D gene segments of the same type (eg, D2-2ref and D2-2a), wherein a first of said different D gene segments is provided in the genome of a first vertebrate of the population, and a second of said different D gene segments is provided in the genome of a second vertebrate of the population, wherein the genome of the first vertebrate does not comprise the second D gene segment.
163. A method of enhancing the human immunoglobulin gene diversity of a non-human vertebrate (eg, a mouse or rat), the method comprising providing the vertebrate with a genome comprising at least 2 different non-endogenous D gene segments of the same type (eg, D2-2ref and D2-2a), wherein the D gene segments are derived from the genome sequence of different human individuals that are not genetically related over at least 3 generations.
164. The vertebrate or cell of clause 154, 156 or 158, or the method of clause 159, 161 or 163, wherein at least 2 or 3 of said different gene segments are provided *cis* at the same Ig locus in said genome.
165. The vertebrate or cell of clause 154, 155 or 156, or the method of any one of clauses 159 to 162 and 164, wherein the D gene segments are derived from the genome sequence of different human individuals that are not genetically related over at least 3 generations.
166. The vertebrate or cell of any one of clauses 154 to 157, or the method of any one of clauses 159 to 162 and 165, wherein the D gene segments are derived from the genome sequence of two or more different human individuals; optionally wherein the different human individuals are from different human populations.
167. The vertebrate, cell or method of clause 166, wherein the individuals are not genetically related.
168. The vertebrate, cell or method of any one of clauses 154 to 167, wherein at least one of the different D segments is a synthetic mutant of a human germline D gene segment.
169. A method of enhancing the human immunoglobulin gene diversity of a non-human vertebrate (eg, a mouse or rat), the method comprising providing the vertebrate with a genome comprising at least 2 human D gene segments of the same type (eg, D2-2ref and D2-2a), wherein the D gene segments are derived from the genome sequence of different human individuals that are not genetically related over at least 3 generations; optionally wherein at least 2 or 3 of said different gene segments are provided at the same IgH locus in said genome.
170. The vertebrate or cell of any one of clauses 154 to 158 and 164 to 168, wherein the genome comprises a substantially complete functional repertoire of human D gene segment types supplemented with one, two or more variant human D gene segments, wherein said substantially complete functional repertoire and the supplementary D gene segments are not found together in the germline genome of a human individual.
171. The population of clause 157, wherein the population comprises a substantially complete functional repertoire of human D gene segment types supplemented with one, two or more variant human D gene segments, wherein said substantially complete functional repertoire and the supplementary D gene segments are not found together in the germline genome of a human individual.
172. A non-human vertebrate (eg, a mouse or rat) or a non-human cell (eg, an ES cell or a B-cell) having a genome comprising a substantially complete functional repertoire of human D gene segment types supplemented with one, two or more variant human D gene segments, wherein said substantially complete functional repertoire and the supplementary D gene segments are not found together in the germline genome of a human individual.
173. A population of non-human vertebrates (eg, mice or rats) comprising a substantially complete functional repertoire of human JH gene segment types supplemented with one, two or more variant human D gene segments, wherein said substantially complete functional repertoire and the supplementary D gene segments are not found together in the germline genome of a human individual.
174. The vertebrate or cell of clause 172 or the population of clause 173, comprising first and second D gene segments selected from
   D2-2ref and D2-2a; or
   D2-21ref and D2-21a; or
   D3-10ref and D3-10a; or
   D3-16ref and D3-16a; or
   D2-8ref and D2-8a; or
   D3-3ref and D3-3a; or
   D4-23ref and D4-23a; or
   D6-13ref and D6-13a; or
   D3-9ref and D3-9a; or
   D4-4ref and D4-4a; or
   D7-27ref and D7-27a;
   Optionally wherein the first and/or second D gene segment is present in two or more copies.
   For example, there are provided two or three copies of the first gene segment, optionally with one, two or three copies of the second gene segment. Copies can be arranged in *cis* or *trans.*
175. The vertebrate, cell or population of clause 174, comprising human gene segments D2-2ref and D2-2a; and D3-3ref and D3-3a; and optionally also D2-15.
   In an example, the vertebrate, cell or population comprises one or more D segments selected from human D3-3, D2-15, D3-9; D4-17; D3-10; D2-2; D5-24; D6-19; D3-22; D6-13; D5-12; D1-26; D1-20; D5-18; D3-16; D2-21; D1-14; D7-27; D1-1; D6-25; D2-14 and D4-23 (eg, selected from D3-9*01; D4-17*01; D3-10*01; D2-2*02; D5-24*01; D6-19*01; D3-22*01; D6-13*01; D5-12*01; D1-26*01; DI-20*01; D5-18*01; D3-16*02; D2-21*02; DI-14*01; D7-27*02; D1-1*01; D6-25*01; D2-15*01; and D4-23*01), together with the reference sequence(s) of said selected segment(s). These were found in variable domains having a HCDR3 length of at least 20 amino acids (see examples herein).
176. A non-human vertebrate or vertebrate cell according to clause 155, comprising a genome that comprises VH, D and JH gene repertoires comprising human gene segments, the D gene repertoire comprising one or more of
   a plurality of D2-2 gene segments provided by at least 2 different D2-2 gene segments in *cis* at the same Ig locus in said genome;
   a plurality of D2-21 gene segments provided by at least 2 different D2-21 gene segments in *cis* at the same Ig locus in said genome;
   a plurality of D3-10 gene segments provided by at least 2 different D3-10 gene segments in *cis* at the same Ig locus in said genome;
   a plurality of D3-16 gene segments provided by at least 2 different D3-16 gene segments in *cis* at the same Ig locus in said genome;
   a plurality of D2-8 gene segments provided by at least 2 different D2-8 gene segments in *cis* at the same Ig locus in said genome;
   a plurality of D3-3 gene segments provided by at least 2 different D3-3 gene segments in *cis* at the same Ig locus in said genome;
   a plurality of D4-23 gene segments provided by at least 2 different D4-23 gene segments in *cis* at the same Ig locus in said genome;
   a plurality of D6-13 gene segments provided by at least 2 different D6-13 gene segments in *cis* at the same Ig locus in said genome;
   a plurality of D3-9 gene segments provided by at least 2 different D3-9 gene segments in *cis* at the same Ig locus in said genome;
   a plurality of D4-4 gene segments provided by at least 2 different D4-4 gene segments in *cis* at the same Ig locus in said genome; and
   a plurality of D7-27 gene segments provided by at least 2 different D7-27 gene segments in *cis* at the same Ig locus in said genome;
   optionally wherein the D gene segments are derived from the genome sequence of two or more different human individuals.
177. A non-human vertebrate or vertebrate cell according to clause 155, comprising a genome that comprises VH, D and JH gene repertoires comprising human gene segments, the D gene repertoire comprising one or more of
   a plurality of D2-2 gene segments provided by at least 2 different D2-2 gene segments in *trans* in said genome;
   a plurality of D2-21 gene segments provided by at least 2 different D2-21 gene segments in *trans* in said genome;
   a plurality of D3-10 gene segments provided by at least 2 different D3-10 gene segments in *trans* in said genome;
   a plurality of D3-16 gene segments provided by at least 2 different D3-16 gene segments in *trans* in said genome;
   a plurality of D2-8 gene segments provided by at least 2 different D2-8 gene segments in *trans* in said genome;
   a plurality of D3-3 gene segments provided by at least 2 different D3-3 gene segments in *trans* in said genome;
   a plurality of D4-23 gene segments provided by at least 2 different D4-23 gene segments in *trans* in said genome;
   a plurality of D6-13 gene segments provided by at least 2 different D6-13 gene segments in *trans* in said genome;
   a plurality of D3-9 gene segments provided by at least 2 different D3-9 gene segments in *trans* in said genome;
   a plurality of D4-4 gene segments provided by at least 2 different D4-4 gene segments in *trans* in said genome; and
   a plurality of D7-27 gene segments provided by at least 2 different D7-27 gene segments in *trans* in said genome;
   optionally wherein the D gene segments are derived from the genome sequence of two or more different human individuals.
178. A non-human vertebrate or vertebrate cell (optionally an ES cell or B-cell), according to clause 154, comprising a genome that comprises VH, D and JH gene repertoires comprising human gene segments, the D gene repertoire comprising one or more of
   a plurality of D2-2 gene segments provided by at least 3 different D2-2 gene segments;
   a plurality of D2-21 gene segments provided by at least 3 different D2-21 gene segments;
   a plurality of D3-10 gene segments provided by at least 3 different D3-10 gene segments;
   a plurality of D3-16 gene segments provided by at least 3 different D3-16 gene segments;
   a plurality of D2-8 gene segments provided by at least 3 different D2-8 gene segments;
   a plurality of D3-3 gene segments provided by at least 3 different D3-3 gene segments;
   a plurality of D4-23 gene segments provided by at least 3 different D4-23 gene segments;
   a plurality of D6-13 gene segments provided by at least 3 different D6-13 gene segments;
   a plurality of D3-9 gene segments provided by at least 3 different D3-9 gene segments;
   a plurality of D4-4 gene segments provided by at least 3 different D4-4 gene segments; and
   a plurality of D7-27 gene segments provided by at least 3 different D7-27 gene segments;
   optionally wherein the D gene segments are derived from the genome sequence of two or three different human individuals;
   optionally wherein at least 2 or 3 of said different gene segments are provided in *cis* at the same Ig locus in said genome.
179. The vertebrate or cell of clause 176, 177 or 178, wherein the different human individuals are from different human populations.
180. The vertebrate or cell of any one of clauses 176 to 179, wherein the individuals are not genetically related.
181. The vertebrate or cell of any one of clauses 176 to 180, wherein at least one of the different D segments is a synthetic mutant of a human germline D gene segment.
182. A non-human vertebrate or vertebrate cell (optionally an ES cell or B-cell) according to clause 158, comprising a genome comprising human VH, D and JH gene repertoires, the D gene repertoire comprising of one or more of
   a plurality of D2-2 gene segments provided by at least 2 different D2-2 gene ; optionally in *cis* in said genome;
   a plurality of D2-21 gene segments provided by at least 2 different D2-21 gene ; optionally in *cis* in said genome;
   a plurality of D3-10 gene segments provided by at least 2 different D3-10 gene ; optionally in *cis* in said genome;
   a plurality of D3-16 gene segments provided by at least 2 different D3-16 gene ; optionally in *cis* in said genome;
   a plurality of D2-8 gene segments provided by at least 2 different D2-8 gene ; optionally in *cis* in said genome;
   a plurality of D3-3 gene segments provided by at least 2 different D3-3 gene ; optionally in *cis* in said genome;
   a plurality of D4-23 gene segments provided by at least 2 different D4-23 gene ; optionally in *cis* in said genome;
   a plurality of D6-13 gene segments provided by at least 2 different D6-13 gene ; optionally in *cis* in said genome;
   a plurality of D3-9 gene segments provided by at least 2 different D3-9 gene ; optionally in *cis* in said genome;
   a plurality of D4-4 gene segments provided by at least 2 different D4-4 gene ; optionally in *cis* in said genome; and
   a plurality of D7-27 gene segments provided by at least 2 different D7-27 gene ; optionally in *cis* in said genome;
   wherein the D gene segments are derived from the genome sequence of different human individuals that are not genetically related over at least 3 generations.
183. The vertebrate or cell of clause 182, wherein the human individuals are from different human populations.
184. The vertebrate, cell or population of any one of clauses 154 to 183, wherein one or more of the D gene segments is a variant of a human germline D gene segment, wherein the variant gene segment encodes an amino acid sequence that differs by 1, 2 or 3 amino acids from the corresponding amino acid sequence encoded by the human germline D gene segment, provided in that said amino acid sequence encoded by the variant does not include a stop codon when said corresponding amino acid sequence does not include a stop codon.
   Optionally, the variant and germline D gene segments encode the respective amino acid sequences in reading frame 2 (IMGT numbering). See Briney et *al* 2012*.*
185. The vertebrate, cell or population of clause 184, wherein said corresponding amino acid sequence encoded by the human germline D gene segment is a hydrophilic or hydrophobic sequence (according to J Mol Biol. 1997 Jul 25;270(4):587-97; Corbett SJ *et al;* Table 2).
186. The vertebrate, cell or population of clause 184 or 185, comprising said variant and said germline human D gene segments; optionally wherein the variant and germline human D gene segments are *cis* on the same chromosome.
187. The vertebrate, cell or population of any one of clauses 184 to 186, wherein germline human D gene segment is a D2, D3, D5 or D6 family gene segment; optionally a D2-2, D2-15, D3-3, D3-9, D3-10, D3-22, D5-5, D5-18, D6-6, D6-13, D6-19 gene segment.
   These D segments are usable in all three reading frames.
   Optionally a variant of 1, 2, 3, 4, 5, 6, 7, 8, 9, 10 or all of these human germline D gene segments is used.
188. The vertebrate, cell or population of any one of clauses 154 to 187, comprising a plurality of D2-2 gene segments, wherein the plurality comprises D2-2 gene segments that vary from each other at one or more nucleotide positions corresponding to positions
   106,382,687 and
   106,382,711
   on human chromosome 14.
189. The vertebrate, cell or population of clause 188, wherein the plurality comprises a human D2-2 gene segment ((optionally two copies and/or in homozygous state) comprising a thymine at a position corresponding to position 106,382,687 on human chromosome 14; and optionally no further mutation from the sequence of D2-2ref.
190. The vertebrate, cell or population of clause 188 or 189, wherein the plurality comprises a human D2-2 gene segment comprising a cytosine at a position corresponding to position 106,382,687 on human chromosome 14; and optionally no further mutation from the sequence of D2-2a.
191. The vertebrate, cell or population of any one of clauses 188 to 190, wherein the plurality comprises a human D2-2 gene segment comprising an adenine at a position corresponding to position 106,382,711 on human chromosome 14; and optionally no further mutation from the sequence of D2-2b.
192. The vertebrate, cell or population of any one of clauses 188 to 191, wherein the plurality comprises a human D2-2 gene segment comprising an thymine at a position corresponding to position 106,382,711 on human chromosome 14; and optionally no further mutation from the sequence of D2-2ref.
193. The vertebrate, cell or population of any one of clauses 154 to 192, comprising a plurality of D7-27 gene segments, wherein the plurality comprises D7-27 gene segments that vary from each other at a nucleotide position corresponding to position 106,331,767 on human chromosome 14.
194. The vertebrate, cell or population of clause 193, wherein the plurality comprises a human D7-27 gene segment (optionally two copies and/or in homozygous state) comprising a cytosine at a position corresponding to position 106,331,767 on human chromosome 14; and optionally no further mutation from the sequence of D7-27ref.
195. The vertebrate, cell or population of clause 193 or 194, wherein the plurality comprises a human D7-27 gene segment comprising a guanine at a position corresponding to position 106,331,767 on human chromosome 14; and optionally no further mutation from the sequence of D7-27a.
196. The vertebrate, cell or population of any one of clauses 154 to 195, comprising a plurality of D4-23 gene segments, wherein the plurality comprises D4-23 gene segments that vary from each other at a nucleotide position corresponding to position 106,350,740 on human chromosome 14.
197. The vertebrate, cell or population of clause 196, wherein the plurality comprises a human D4-23 gene segment (optionally two copies and/or in homozygous state) comprising an adenine at a position corresponding to position 106,350,740 on human chromosome 14; and optionally no further mutation from the sequence of D4-23ref.
198. The vertebrate, cell or population of clause 196 or 197, wherein the plurality comprises a human D4-23 gene segment (optionally two copies and/or in homozygous state) comprising an guanine at a position corresponding to position 106,350,740 on human chromosome 14; and optionally no further mutation from the sequence of D4-23a.
199. The vertebrate, cell or population of any one of clauses 154 to 197, comprising a plurality of D2-21 gene segments, wherein the plurality comprises D2-21 gene segments that vary from each other at a nucleotide position corresponding to position 106,354,418 on human chromosome 14.
200. The vertebrate, cell or population of clause 199, wherein the plurality comprises a human D2-21 gene segment (optionally two copies and/or in homozygous state) comprising an adenine at a position corresponding to position 106,354,418 on human chromosome 14; and optionally no further mutation from the sequence of D2-21ref.
201. The vertebrate, cell or population of clause 199 or 200, wherein the plurality comprises a human D2-21 gene segment (optionally two copies and/or in homozygous state) comprising a guanine at a position corresponding to position 106,354,418 on human chromosome 14; and optionally no further mutation from the sequence of D2-21a.
202. The vertebrate, cell or population of any one of clauses 154 to 201, comprising a plurality of D3-16 gene segments, wherein the plurality comprises D3-16 gene segments that vary from each other at a nucleotide position corresponding to position 106,354,418 on human chromosome 14.
203. The vertebrate, cell or population of clause 202, wherein the plurality comprises a human D3-16 gene segment (optionally two copies and/or in homozygous state) comprising a thymine at a position corresponding to position 106,361,515 on human chromosome 14; and optionally no further mutation from the sequence of D3-16ref.
204. The vertebrate, cell or population of clause 202 or 203, wherein the plurality comprises a human D3-16 gene segment (optionally two copies and/or in homozygous state) comprising a cytosine at a position corresponding to position 106,361,515 on human chromosome 14; and optionally no further mutation from the sequence of D3-16a.
205. The vertebrate, cell or population of any one of clauses 154 to 204, comprising a plurality of D6-13 gene segments, wherein the plurality comprises D6-13 gene segments that vary from each other at a nucleotide position corresponding to position 106,367,013 on human chromosome 14.
206. The vertebrate, cell or population of clause 205, wherein the plurality comprises a human D6-13 gene segment (optionally two copies and/or in homozygous state) comprising a thymine at a position corresponding to position 106,367,013 on human chromosome 14; and optionally no further mutation from the sequence of D6-13ref.
207. The vertebrate, cell or population of clause 205 or 206, wherein the plurality comprises a human D6-13 gene segment (optionally two copies and/or in homozygous state) comprising a cytosine at a position corresponding to position 106,367,013 on human chromosome 14; and optionally no further mutation from the sequence of D6-13a.
208. The vertebrate, cell or population of any one of clauses 154 to 207, comprising a plurality of D3-10 gene segments, wherein the plurality comprises D3-10 gene segments that vary from each other at one or more nucleotide positions corresponding to positions
   106,370,370 and
   106,370,371
   on human chromosome 14.
209. The vertebrate, cell or population of clause 208, wherein the plurality comprises a human D3-10 gene segment (optionally two copies and/or in homozygous state) comprising a thymine at a position corresponding to position 106,370,370 on human chromosome 14; and optionally no further mutation from the sequence of D3-10ref.
210. The vertebrate, cell or population of clause 208 or 209, wherein the plurality comprises a human D3-10 gene segment (optionally two copies and/or in homozygous state) comprising a cytosine at a position corresponding to position 106,370,370 on human chromosome 14; and optionally no further mutation from the sequence of D3-10a.
211. The vertebrate, cell or population of clause 208, 209 or 210 wherein the plurality comprises a human D3-10 gene segment (optionally two copies and/or in homozygous state) comprising an adenine at a position corresponding to position 106,370,371 on human chromosome 14; and optionally no further mutation from the sequence of D3-10ref.
212. The vertebrate, cell or population of any one of clauses 208 to 211, wherein the plurality comprises a human D3-10 gene segment (optionally two copies and/or in homozygous state) comprising a guanine at a position corresponding to position 106,370,371 on human chromosome 14; and optionally no further mutation from the sequence of D3-10b.
213. The vertebrate, cell or population of any one of clauses 154 to 212, comprising a plurality of D3-9 gene segments, wherein the plurality comprises D3-9 gene segments that vary from each other at a nucleotide position corresponding to position 106,370,567 on human chromosome 14.
214. The vertebrate, cell or population of clause 213, wherein the plurality comprises a human D3-9 gene segment (optionally two copies and/or in homozygous state) comprising an adenine at a position corresponding to position 106,370,567 on human chromosome 14; and optionally no further mutation from the sequence of D3-9ref.
215. The vertebrate, cell or population of clause 213 or 214, wherein the plurality comprises a human D3-9 gene segment (optionally two copies and/or in homozygous state) comprising a thymine at a position corresponding to position 106,370,567 on human chromosome 14; and optionally no further mutation from the sequence of D3-9a.
216. The vertebrate, cell or population of any one of clauses 154 to 215, comprising a plurality of D2-8 gene segments, wherein the plurality comprises D2-8 gene segments that vary from each other at one or more nucleotide positions corresponding to positions
   106,373,085;
   106,373,086 and
   106,373,089
   on human chromosome 14.
217. The vertebrate, cell or population of clause 216, wherein the plurality comprises a human D2-8 gene segment (optionally two copies and/or in homozygous state) comprising a cytosine at a position corresponding to position 106,373,085 on human chromosome 14.
218. The vertebrate, cell or population of clause 216 or 217, wherein the plurality comprises a human D2-8 gene segment (optionally two copies and/or in homozygous state) comprising a thymine at a position corresponding to position 106,373,085 on human chromosome 14; and optionally no further mutation from the sequence of D2-8b.
219. The vertebrate, cell or population of clause 216, 217 or 218 wherein the plurality comprises a human D2-8 gene segment (optionally two copies and/or in homozygous state) comprising a cytosine at a position corresponding to position 106,373,086 on human chromosome 14; and
   optionally no further mutation from the sequence of D2-8ref.
220. The vertebrate, cell or population of any one of clauses 216 to 219, wherein the plurality comprises a human D2-8 gene segment comprising a thymine at a position corresponding to position 106,373,086 on human chromosome 14; and optionally no further mutation from the sequence of D2-8ref.
221. The vertebrate, cell or population of any one of clauses 154 to 220, comprising a plurality of D4-4 gene segments, wherein the plurality comprises D4-4 gene segments that vary from each other at one or more nucleotide positions corresponding to positions
   106,379,086; and
   106,379,089
   on human chromosome 14.
222. The vertebrate, cell or population of clause 221, wherein the plurality comprises a D4-4 gene segment (optionally two copies and/or in homozygous state) comprising a cytosine at a position corresponding to position 106,379,086 on human chromosome 14; and optionally no further mutation from the sequence of D4-4ref.
223. The vertebrate, cell or population of clause 221 or 222, wherein the plurality comprises a human D4-4 gene segment (optionally two copies and/or in homozygous state) comprising a thymine at a position corresponding to position 106,379,086 on human chromosome 14; and optionally no further mutation from the sequence of D4-4a.
224. The vertebrate, cell or population of clause 221, 222 or 223 wherein the plurality comprises a human D4-4 gene segment (optionally two copies and/or in homozygous state) comprising a cytosine at a position corresponding to position 106,379,089 on human chromosome 14; and optionally no further mutation from the sequence of D4-4ref or a cytosine at a position corresponding to position 106,379,086 on human chromosome 14.
225. The vertebrate, cell or population of any one of clauses 221 to 224, wherein the plurality comprises a human D4-4 gene segment (optionally two copies and/or in homozygous state) comprising a thymine at a position corresponding to position 106,373,089 on human chromosome 14; and optionally no further mutation from the sequence of D4-4a.
226. The vertebrate, cell or population of any one of clauses 154 to 225, comprising a plurality of D3-3 gene segments, wherein the plurality comprises D3-3 gene segments that vary from each other at one or more nucleotide positions corresponding to positions
   106,380,241; and
   106,380,246
   on human chromosome 14.
227. The vertebrate, cell or population of clause 226, wherein the plurality comprises a D3-3 gene segment (optionally two copies and/or in homozygous state) comprising a thymine at a position corresponding to position 106,380,241 on human chromosome 14; and optionally no further mutation from the sequence of D3-3ref.
228. The vertebrate, cell or population of clause 226 or 227, wherein the plurality comprises a human D3-3 gene segment (optionally two copies and/or in homozygous state) comprising a cytosine at a position corresponding to position 106,380,241 on human chromosome 14; and optionally no further mutation from the sequence of D3-3a.
229. The vertebrate, cell or population of clause 226, 227 or 228 wherein the plurality comprises a human D3-3 gene segment (optionally two copies and/or in homozygous state) comprising an adenine at a position corresponding to position 106,380,246 on human chromosome 14; and optionally no further mutation from the sequence of D3-3ref.
230. The vertebrate, cell or population of any one of clauses 226 to 229, wherein the plurality comprises a human D3-3 gene segment (optionally two copies and/or in homozygous state) comprising a thymine at a position corresponding to position 106,380,246 on human chromosome 14; and optionally no further mutation from the sequence of D3-3a.

### Multiple Human JL Gene Segment Variants

A specific application of this configuration is the provision of multiple human JLgene segments (J_{K} and/or Jλ) as follows (as set out in numbered paragraphs, starting at paragraph number 80).
80. A non-human vertebrate (eg, a mouse or rat) or a non-human vertebrate cell (eg, an ES cell or a B-cell) having a genome comprising at least 3 human JLgene segments of the same type (eg, Jκ1), wherein at least two of the human JL gene segments are variants that are not identical to each other.
81. A non-human vertebrate (eg, a mouse or rat) or a non-human vertebrate cell (eg, an ES cell or a B-cell) having a genome comprising at least 2 different non-endogenous JL gene segments of the same type (eg, Jκ1) *cis* at the same Ig locus.
82. A non-human vertebrate (eg, a mouse or rat) or a non-human vertebrate cell (eg, an ES cell or a B-cell) having a genome comprising at least 2 different human JL gene segments of the same type (eg, Jκ1) *trans* at the same Ig locus; and optionally a third human JL gene segment of the same type, wherein the third JL is *cis* with one of said 2 different JL gene segments.
83. A population of non-human vertebrates (eg, mice or rats) comprising a repertoire of human JL gene segments, wherein the plurality comprises at least 2 different human JL gene segments of the same type (eg, Jκ1), a first of said different JL gene segments is provided in the genome of a first vertebrate of the population, and a second of said different JL gene segments being provided in the genome of a second vertebrate of the population, wherein the genome of the first vertebrate does not comprise the second JL gene segment.
84. A non-human vertebrate (eg, a mouse or rat) or a non-human vertebrate cell (eg, an ES cell or a B-cell) having a genome comprising at least 2 different non-endogenous JL gene segments of the same type (eg, Jκ1), wherein the JL gene segments are derived from the genome sequence of different human individuals that are not genetically related over at least 3 generations.
85. A method of enhancing the human immunoglobulin gene diversity of a non-human vertebrate (eg, a mouse or rat), the method comprising providing the vertebrate with a genome comprising at least 3 human JL gene segments of the same type (eg, Jκ1), wherein at least two of the human JL gene segments are variants that are not identical to each other.
86. A method of enhancing the immunoglobulin gene diversity of a non-human vertebrate (eg, a mouse or rat), the method comprising providing the vertebrate with a genome comprising at least 2 different non-endogenous JLgene segments of the same type (eg, Jκ1) *cis* at the same Ig locus.
87. A method of enhancing the immunoglobulin gene diversity of a non-human vertebrate (eg, a mouse or rat), the method comprising providing the vertebrate with a genome comprising at least 2 different human JLgene segments of the same type(eg, Jκ1) *trans* at the same Ig locus; and optionally a third human JL gene segment of the same type, wherein the third JL is *cis* with one of said 2 different JL gene segments.
88. A method of providing an enhanced human immunoglobulin JL gene segment repertoire, the method comprising providing a population of non-human vertebrates (eg, a mouse or rat) comprising a repertoire of human JL gene segments, wherein the method comprises providing at least 2 different human JLgene segments of the same type (eg, Jκ1), wherein a first of said different JLgene segments is provided in the genome of a first vertebrate of the population, and a second of said different JL gene segments is provided in the genome of a second vertebrate of the population, wherein the genome of the first vertebrate does not comprise the second JL gene segment.
89. A method of enhancing the human immunoglobulin gene diversity of a non-human vertebrate (eg, a mouse or rat), the method comprising providing the vertebrate with a genome comprising at least 2 different non-endogenous JLgene segments of the same type (eg, Jκ1), wherein the JL gene segments are derived from the genome sequence of different human individuals that are not genetically related over at least 3 generations.
90. The vertebrate or cell of paragraph 80, 82 or 84, or the method of paragraph 85, 82 or 89, wherein at least 2 or 3 of said different gene segments are provided *cis* at the same Ig locus in said genome.
91. The vertebrate or cell of paragraph 80, 81 or 82, or the method of paragraph 85, 86 or 87, wherein the JL gene segments are derived from the genome sequence of different human individuals that are not genetically related over at least 3 generations.
92. The vertebrate or cell of paragraph 80, 81 or 82, or the method of paragraph 85, 86 or 87, wherein the JLgene segments are derived from the genome sequence of two or more different human individuals; optionally wherein the different human individuals are from different human populations.
93. The vertebrate, cell or method of paragraph 92, wherein the individuals are not genetically related.
94. The vertebrate, cell or method of any one of paragraphs 80 to 93, wherein at least one of the different JL segments is a synthetic mutant of a human germline JL gene segment.
95. A method of enhancing the human immunoglobulin gene diversity of a non-human vertebrate (eg, a mouse or rat), the method comprising providing the vertebrate with a genome comprising at least 2 human JL gene segments of the same type (eg, Jκ1), wherein the JL gene segments are derived from the genome sequence of different human individuals that are not genetically related over at least 3 generations; optionally wherein at least 2 or 3 of said different gene segments are provided at the same IgL locus in said genome.
96. The vertebrate or cell of any one of paragraphs paragraph 80 to 82 and 84, wherein the genome comprises a substantially complete functional repertoire of human Jκ and/or Jλ gene segment types supplemented with one, two or more human Jκ and/or Jλ gene segments respectively, wherein said substantially complete functional repertoire and the supplementary gene segments are not found together in the germline genome of a human individual.
97. The population of paragraph 83, wherein the population comprises a substantially complete functional repertoire of human JL gene segment types supplemented with one, two or more human Jκ and/or Jλ gene segments respectively, wherein said substantially complete functional repertoire and the supplementary gene segments are not found together in the germline genome of a human individual.
98. A non-human vertebrate (eg, a mouse or rat) or a non-human cell (eg, an ES cell or a B-cell) having a genome comprising a substantially complete functional repertoire of human Jκ and/or Jλ gene segment types supplemented with one, two or more human Jκ and/or Jλ gene segments respectively, wherein said substantially complete functional repertoire and the supplementary gene segments are not found together in the germline genome of a human individual.
99. A population of non-human vertebrates (eg, mice or rats) comprising a substantially complete functional repertoire of human Jκ and/or Jλ gene segment types supplemented with one, two or more human Jκ and/or Jλ gene segments respectively, wherein said substantially complete functional repertoire and the supplementary gene segments are not found together in the germline genome of a human individual.
100. A non-human vertebrate or vertebrate cell according to paragraph 81, comprising a genome that comprises VL and JL gene repertoires comprising human gene segments, the JL gene repertoire comprising
   a plurality of human Jκ1 gene segments provided by at least 2 different human Jκ1 gene segments in *cis* at the same Ig locus in said genome;
   a plurality of human Jκ2 gene segments provided by at least 2 different human Jκ1 gene segments in *cis* at the same Ig locus in said genome;
   a plurality of human Jκ3 gene segments provided by at least 2 different human Jκ1 gene segments in *cis* at the same Ig locus in said genome;
   a plurality of human Jκ4 gene segments provided by at least 2 different human Jκ1 gene segments in *cis* at the same Ig locus in said genome;
   a plurality of human Jκ5 gene segments provided by at least 2 different human Jκ1 gene segments in *cis* at the same Ig locus in said genome;
   a plurality of human Jλ1 gene segments provided by at least 2 different human Jλ1 gene segments in *cis* at the same Ig locus in said genome;
   a plurality of human Jλ2 gene segments provided by at least 2 different human Jλ2 gene segments in *cis* at the same Ig locus in said genome;
   a plurality of human Jλ3 gene segments provided by at least 2 different human Jλ3 gene segments in *cis* at the same Ig locus in said genome;
   a plurality of human Jλ4 gene segments provided by at least 2 different human Jλ4 gene segments in *cis* at the same Ig locus in said genome;
   a plurality of human Jλ5 gene segments provided by at least 2 different human Jλ5 gene segments in *cis* at the same Ig locus in said genome;
   a plurality of human Jλ6 gene segments provided by at least 2 different human Jλ6 gene segments in *cis* at the same Ig locus in said genome; or
   a plurality of human Jλ7 gene segments provided by at least 2 different human Jλ7 gene segments in *cis* at the same Ig locus in said genome;
   optionally wherein the JL gene segments are derived from the genome sequence of two or more different human individuals.
101. A non-human vertebrate or vertebrate cell (optionally an ES cell or B-cell), according to paragraph 80, comprising a genome that comprises VL and JL gene repertoires comprising human gene segments, the JL gene repertoire comprising
   a plurality of human Jκ1 gene segments provided by at least 3 (eg, 3, 4, 5, 6, or 7) different human Jκ1 gene segments;
   a plurality of human Jκ2 gene segments provided by at least 3 (eg, 3, 4, 5, 6, or 7) different human Jκ1 gene segments;
   a plurality of human Jκ3 gene segments provided by at least 3 (eg, 3, 4, 5, 6, or 7) different human Jκ1 gene segments;
   a plurality of human Jκ4 gene segments provided by at least 3 (eg, 3, 4, 5, 6, or 7) different human Jκ1 gene segments;
   a plurality of human Jκ5 gene segments provided by at least 3 (eg, 3, 4, 5, 6, or 7) different human Jκ1 gene segments;
   a plurality of human Jλ1 gene segments provided by at least 3 (eg, 3, 4, 5, 6, or 7) different human Jλ1 gene segments;
   a plurality of human Jλ2 gene segments provided by at least 3 (eg, 3, 4, 5, 6, or 7) different human Jλ2 gene segments;
   a plurality of human Jλ3 gene segments provided by at least 3 (eg, 3, 4, 5, 6, or 7) different human Jλ3 gene segments;
   a plurality of human Jλ4 gene segments provided by at least 3 (eg, 3, 4, 5, 6, or 7) different human Jλ4 gene segments;
   a plurality of human Jλ5 gene segments provided by at least 3 (eg, 3, 4, 5, 6, or 7) different human Jλ5 gene segments;
   a plurality of human Jλ6 gene segments provided by at least 3 (eg, 3, 4, 5, 6, or 7) different human Jλ6 gene segments; or
   a plurality of human Jλ7 gene segments provided by at least 3 (eg, 3, 4, 5, 6, or 7) different human Jλ7 gene segments;
   optionally wherein the JL gene segments are derived from the genome sequence of two or three different human individuals;
   optionally wherein at least 2 or 3 of said different gene segments are provided in *cis* at the same Ig locus in said genome.
102. The vertebrate or cell of paragraph 104 or 105, wherein the different human individuals are from different human populations.
103. The vertebrate or cell of any one of paragraphs 104 to 106, wherein the individuals are not genetically related.
104. The vertebrate or cell of any one of paragraphs 104 to 107, wherein at least one of the different JL segments is a synthetic mutant of a human germline JL gene segment.
105. A non-human vertebrate or vertebrate cell (optionally an ES cell or B-cell) according to paragraph 84, comprising a genome comprising human VL and JL gene repertoires, the JL gene repertoire comprising
   a plurality of human Jκ1 gene segments provided by at least 2 different human Jκ1 gene segments, optionally in *cis* at the same Ig locus in said genome;
   a plurality of human Jκ2 gene segments provided by at least 2 different human Jκ1 gene segments, optionally in *cis* at the same Ig locus in said genome;
   a plurality of human Jκ3 gene segments provided by at least 2 different human Jκ1 gene segments, optionally in *cis* at the same Ig locus in said genome;
   a plurality of human Jκ4 gene segments provided by at least 2 different human Jκ1 gene segments, optionally in *cis* at the same Ig locus in said genome;
   a plurality of human Jκ5 gene segments provided by at least 2 different human Jκ1 gene segments, optionally in *cis* at the same Ig locus in said genome;
   a plurality of human Jλ1 gene segments provided by at least 2 different human Jλ1 gene segments, optionally in *cis* at the same Ig locus in said genome;
   a plurality of human Jλ2 gene segments provided by at least 2 different human Jλ2 gene segments, optionally in *cis* at the same Ig locus in said genome;
   a plurality of human Jλ3 gene segments provided by at least 2 different human Jλ3 gene segments, optionally in *cis* at the same Ig locus in said genome;
   a plurality of human Jλ4 gene segments provided by at least 2 different human Jλ4 gene segments, optionally in *cis* at the same Ig locus in said genome;
   a plurality of human Jλ5 gene segments provided by at least 2 different human Jλ5 gene segments, optionally in *cis* at the same Ig locus in said genome;
   a plurality of human Jλ6 gene segments provided by at least 2 different human Jλ6 gene segments, optionally in *cis* at the same Ig locus in said genome; or
   a plurality of human Jλ7 gene segments provided by at least 2 different human Jλ7 gene segments, optionally in *cis* at the same Ig locus in said genome;
   wherein the JL gene segments are derived from the genome sequence of different human individuals that are not genetically related over at least 3 generations.
106. The vertebrate or cell of paragraph 109, wherein the human individuals are from different human populations.

The skilled person will realise that standard molecular biology techniques can be used to provide vectors comprising synthetic combinations of immunoglobulin gene segments (eg, V, D and/or J) for use in the invention, such that the vectors can be used to build a transgenic immunoglobulin locus (eg, using homologous recombination and/or recombinase mediated cassette exchange as known in the art, eg, see US7501552 (Medarex), US5939598 (Abgenix), US6130364 (Abgenix), WO02/066630 (Regeneron), WO2011004192 (Genome Research Limited), WO2009076464, WO2009143472 and WO2010039900 (Ablexis), the disclosures of which are explicitly incorporated herein. For example, such synthetic combinations of gene segments can be made using standard recombineering techniques in E coli to construct BAC vectors harbouring the synthetic combination prior to insertion in embryonic stem cells using homologous recombination or RMCE (eg, using cre/lox site-specific recombination). Details of recombineering can be found at www.genebridges.com and in EP1034260 and EP1204740 the disclosures of which are explicitly incorporated herein.

In one embodiment, it is useful to bias the immune response of the vertebrate (and thus resultant lead antibodies) to a predetermined gene segment, eg, one known to be commonly used in natural human immune responses to antigens, such as antigens of infectious disease pathogens. For example, VH1-69 is commonly used to produce antibodies in humans against Infulenza virus; it is possible, therefore, to include two or more polymorphic DNA versions of the VH segment VH1-69 in the locus of the invention. The examples below illustrate how such a transgenic locus can be constructed in which diversity is extended by extending the VH1-69 gene segment repertoire based on naturally-occurring VH1-69 polymorphic variants.

In one embodiment in any configuration of the invention, the genome has been modified to prevent or reduce the expression of fully-endogenous antibody. Examples of suitable techniques for doing this can be found in PCT/GB2010/051122, US7501552, US6673986, US6130364, WO2009/076464, EP1399559 and US6586251, the disclosures of which are incorporated herein by reference. In one embodiment, the non-human vertebrate VDJ region of the endogenous heavy chain immunoglobulin locus, and optionally VJ region of the endogenous light chain immunoglobulin loci (lambda and/or kappa loci), have been inactivated. For example, all or part of the non-human vertebrate VDJ region is inactivated by inversion in the endogenous heavy chain immunoglobulin locus of the mammal, optionally with the inverted region being moved upstream or downstream of the endogenous Ig locus (see, eg, WO2011004192, the disclosure of which is incorporated herein by reference). For example, all or part of the non-human vertebrate VJ region is inactivated by inversion in the endogenous kappa chain immunoglobulin locus of the mammal, optionally with the inverted region being moved upstream or downstream of the endogenous Ig locus. For example, all or part of the non-human vertebrate VJ region is inactivated by inversion in the endogenous lambda chain immunoglobulin locus of the mammal, optionally with the inverted region being moved upstream or downstream of the endogenous Ig locus. In one embodiment the endogenous heavy chain locus is inactivated in this way as is one or both of the endogenous kappa and lambda loci.

Additionally or alternatively, the vertebrate has been generated in a genetic background which prevents the production of mature host B and T lymphocytes, optionally a RAG-1-deficient and/or RAG-2 deficient background. See US5859301 for techniques of generating RAG-1 deficient animals.

Thus, in one embodiment of any configuration or aspect of the invention herein, endogenous heavy and light chain expression has been inactivated.

In one embodiment each said locus constant region is a heavy chain endogenous non-human vertebrate (optionally host mouse or rat) constant region.

In one embodiment each said locus constant region is a light chain endogenous non-human vertebrate (optionally host mouse or rat) constant region.

The invention provides a monoclonal or polyclonal antibody composition prepared by immunisation of at least one vertebrate (eg, mouse or rat) according to the invention, optionally wherein the antigen is an antigen of an infectious disease pathogen (eg, a bacterial or viral pathogen antigen), optionally wherein the same antigen is used to immunise all the vertebrates; optionally wherein the antibody or antibodies are IgG-type (eg, IgG1).

The invention also provides a monoclonal or polyclonal antibody mixture produced by the method of the invention or a derivative antibody or mixture thereof, eg, where one or more constant region has been changed (eg, replaced with a different constant region such as a human constant region; or mutated to enhance or ablate Fc effector function). In an aspect of the invention, the monoclonal or polyclonal antibody mixture is provided for therapy and/or prophylaxis of a disease or condition in a human, eg, for the treatment and/or prevention of an infectious disease, wherein optionally wherein each antibody binds an antigen of an infectious disease pathogen, preferably the same antigen.

In an aspect of the invention, there is provided the use of an isolated, monoclonal or polyclonal antibody according to the invention, or a mutant or derivative antibody thereof in the manufacture of a medicament for the treatment and/or prevention of a disease or condition in a human, eg, an infectious disease, optionally wherein the infectious disease is a disease caused by a bacterial or viral pathogen.

An example of a mutant antibody is one that bears up to 15 or 10 amino acid mutations in its variable regions relative to an isolated antibody (eg, IgG-type, such as IgG1-type, antibody) obtainable or obtained by the method of the invention. An example of a derivative is one that has been modified to replace a constant region with a different constant region such as a human constant region; or mutated to enhance or ablate Fc effector function.

Examples of infectious diseases are diseases caused or mediated by a bacterial or viral pathogen. For example, the infectious disease is selected from the group consisting of a disease caused by a pathogen selected from the group consisting of Haemophilus influenza, E coli, Neisseria meningitidis, a herpes family virus, cytomegalovirus (CMV), HIV and influenza virus.

### Tailoring V(D)JIncorporation Into Immunoglobin Loci For The Generation of Antibodies Against Infectious Disease

The inventors realised that it would be desirable to provide for vertebrates, cells, methods etc for the production of therapeutic and/or prophylactic antibodies based on natural human immune responses to antigens, such as antigens of infectious disease pathogens. In this respect, the literature observes frequently used immunoglobulin gene segments to raise anti-infective responses in humans (Table 9).

In the various configurations, aspects, embodiments and examples above, the invention provides the skilled addressee with the possibility of choosing immunoglobulin gene segments in a way that tailors or biases the repertoire for application to generating antibodies to treat and/or prevent infectious diseases. The inventors have categorised the following groups of gene segments for use in the invention according to the desired application of resultant antibodies.

### List A:

### Immunoglobulin gene segments for antibodies that bind an antigen expressed by a Pathogen

(a) a VL gene segment selected from the group consisting of a V_{λ}II gene family member, V_{λ}VII 4A, V_{λ}II 2.1, V_{λ}VII 4A, a V_{λ}1 gene family member, a V_{λ}3gene family member, IGLV1S2, V_{λ}3-cML70, lalh2, lalvl, la3h3, Kv325, a VκI gene family member, κI-15A (KL012), VκII family member, a VκIII family member, a VκI gene family member, κI-15A (KL012), VκII A2 (optionally the A2a variant), Vκ A27 (Humkv325) and a gene segment at least 80% identical thereto.
(b) a V_{λ} gene segment selected from a V_{λ}II gene family member, V_{λ}VII 4A, V_{λ}II 2.1, V_{λ}VII 4A, a V_{λ}1 gene family member, a V_{λ}3gene family member, IGLV1S2, V_{λ}3-cML70, lalh2, lalvl, la3h3 and a gene segment at least 80% identical thereto.
(c) a V_{κ} gene segment selected from Kv325, a VκI gene family member, κI-15A (KL012), VκII family member, a V_{κ}III family member, a VκI gene family member, κI-15A (KL012), VκII A2 (optionally the A2a variant), V_{K} A27 (Humkv325) and a gene segment at least 80% identical thereto.
(d) a V_{H} gene segment a V_{H}III gene family member (optionally, a VHIIIa or VHIIIb family member), a V_{H}IV gene family member, V_{H}III 9.1 (VH3-15), V_{H}III VH26 (VH3-23), V_{H}3-21, LSG6.1, LSG12.1, DP77 (V3-21), V_{H}H11, VH1GRR, ha3h2, V_{H}I-ha1c1, V_{H}III-VH2-1, VH4.18, ha4h3, Hv1051, 71-2, Hv1f10, VH4.11, 71-4, VH251, VH1-69 and a gene segment at least 80% identical thereto.
(e) a J_{λ} gene segment selected from J_{λ}2, J_{λ}3 and a gene segment at least 80% identical thereto.
(f) a D gene segment selected from Dk1, Dxp'1, Dn4r, D2r and a gene segment at least 80% identical thereto.

### List A1:

### Immunoglobulin gene segments for antibodies that bind an antigen expressed by a Bacterial Pathogen

(a) a V_{λ} gene segment selected from a V_{λ}II gene family member, V_{λ}VII 4A, V_{λ}II 2.1, V_{λ}VII 4A and a gene segment at least 80% identical thereto.
(b) a V_{K} gene segment selected from a VκI gene family member, κI-15A (KL012), VκII family member, a V_{K}III family member, a VκI gene family member, κI-15A (KL012), VκII A2 (optionally the A2a variant), V_{K} A27 (Humkv325) and a gene segment at least 80% identical thereto.
(c) a V_{H} gene segment a VH3 gene family member (optionally, a VHIIIa or VHIIIb family member), V_{H}III 9.1 (VH3-15), V_{H}III VH26 (VH3-23), V_{H}3-21, LSG6.1, LSG12.1, DP77 (V3-21), V_{H} H11 and a gene segment at least 80% identical thereto.
(d) a J_{λ} gene segment selected from J_{λ}2, J_{λ}3 and a gene segment at least 80% identical thereto.
(e) a J_{H} gene segment selected from J_{H}2, J_{H}3, J_{H}4 and a gene segment at least 80% identical thereto.

### List A1.1:

### Immunoglobulin gene segments for antibodies that bind an antigen expressed bv H influenza

(a) a V_{λ} gene segment selected from a V_{λ}II gene family member, V_{λ}VII 4A, V_{λ}II 2.1, V_{λ}VII 4A and a gene segment at least 80% identical thereto.
(b) a V_{K} gene segment selected from a VκII family member, a V_{K}III family member, a VκI gene family member, κI-15A (KL012), V_{K}II A2 (optionally the A2a variant), V_{K} A27 (Humkv325) and a gene segment at least 80% identical thereto.
(c) a V_{H} gene segment a VH3 gene family member (optionally, a VHIIIb family member), V_{H}III 9.1 (VH3-15), V_{H}III VH26 (VH3-23), V_{H}3-21, LSG6.1, LSG12.1, DP77 (V3-21) and a gene segment at least 80% identical thereto.
(d) a J_{λ} gene segment selected from J_{λ}2, J_{λ}3 and a gene segment at least 80% identical thereto.

### List A1.2:

### Immunoglobulin gene segments for antibodies that bind an antigen expressed by E Coli or Neisseria meningitidis

(a) a V_{H} gene segment a VH3 gene family member (optionally a VHIIIa or VHIIIb member), V_{H}III 9.1 (VH3-15), V_{H} H11, V_{H}III VH26 (VH3-23) a gene segment at least 80% identical thereto, eg, V_{H}III 9.1 + J_{H}3; or V_{H} H11 + J_{H}4; or V_{H}III VH26 + J_{H}2.
(b) a V_{K} gene segment selected from a VκI gene family member, κI-15A (KL012) and a gene segment at least 80% identical thereto.
(c) a V_{λ} gene segment selected from a V_{λ}II gene family member, V_{λ}II 2.1 and a gene segment at least 80% identical thereto.
(d) a J_{H} gene segment selected from J_{H}2, J_{H}3, J_{H}4 and a gene segment at least 80% identical thereto.

### A2:

### Immunoglobulin gene segments for antibodies that bind an antigen expressed by a viral Pathogen

(a) a V_{H} gene segment selected from a V_{H}III gene family member, a V_{H}IV gene family member, V_{H}III-VH26 (VH3-23), VH1GRR, ha3h2, V_{H}I-ha1c1, V_{H}III-VH2-1, VH4.18, ha4h3, Hv1051, 71-2, Hv1f10, VH4.11, 71-4, VH251, VH1-69 and a gene segment at least 80% identical thereto.
(b) a V_{λ} gene segment selected from a V_{λ}1 gene family member, a V_{λ}3gene family member, IGLV1S2, V_{λ}3-cML70, lalh2, lalvl, la3h3 and a gene segment at least 80% identical thereto.
(c) a Vk gene segment selected from Kv325 and a gene segment at least 80% identical thereto.
(d) a J_{H} gene segment selected from J_{H}3, J_{H}5, J_{H}6 and a gene segment at least 80% identical thereto.
(e) a D gene segment selected from Dk1, Dxp'1, Dn4r, D2r and a gene segment at least 80% identical thereto.
(f) a J_{λ} gene segment selected from J_{λ}2, J_{λ}3 and a gene segment at least 80% identical thereto.

### A2.1:

### Immunoglobulin gene segments for antibodies that bind an antigen expressed bv Herpes Virus Family (eg, VZV or HSV)

(a) a V_{H} gene segment selected from a V_{H}III gene family member, a V_{H}IV gene family member, V_{H}III-VH26 (VH3-23), VH1GRR, ha3h2, V_{H}I-ha1c1, V_{H}III-VH2-1, VH4.18, ha4h3, and a gene segment at least 80% identical thereto.
(b) a V_{λ} gene segment selected from a V_{λ}1 gene family member, a V_{λ}3gene family member, IGLV1S2, V_{λ}3-cML70, lalh2, lalvl, la3h3 and a gene segment at least 80% identical thereto.
(c) a J_{H} gene segment selected from J_{H}3, J_{H}5, J_{H}6 and a gene segment at least 80% identical thereto.
(d) a D gene segment selected from Dk1, Dxp'1, Dn4r, D2r and a gene segment at least 80% identical thereto.
(e) a J_{λ} gene segment selected from J_{λ}2, J_{λ}3 and a gene segment at least 80% identical thereto.

### A2.2:

### Immunoglobulin gene segments for antibodies that bind an antigen expressed by CMV

(a) a V_{H} gene segment selected from Hvl051 and a gene segment at least 80% identical thereto.
(b) a Vk gene segment selected from Kv325 and a gene segment at least 80% identical thereto.

### A2.3:

### Immunoglobulin gene segments for antibodies that bind an antigen expressed bv HIV

(a) a V_{H} gene segment selected from 71-2, Hv1f10, VH4.11, 71-4, VH251, VH1-69 and a gene segment at least 80% identical thereto.

### A2.4:

### Immunoglobulin gene segments for antibodies that bind an antigen expressed bv Influenza Virus

(a) a V_{H} gene segment selected from VH1-69 and a gene segment at least 80% identical thereto.

Thus,
Where one wishes to generate an antibody or antibody mixture to treat and/or prevent an infectious disease, one or more V, D and/or or all J gene segments used in any configuration, aspect, method, example or embodiment of the invention can be selected from List A1. Thus, for example in (a) of the first configuration of the invention, the recited heavy chain V gene segment is selected from the VH gene segments in List A, optionally with a D in that list.

Where one wishes to generate an antibody or antibody mixture to treat and/or prevent an infectious disease caused or mediated by a bacterial pathogen, one or more or all V, D and/or J gene segments used in any configuration, aspect, method, example or embodiment of the invention can be selected from List A1.

Where one wishes to generate an antibody or antibody mixture to treat and/or prevent an infectious disease caused or mediated by a viral pathogen, one or more or all V, D and/or J gene segments used in any configuration, aspect, method, example or embodiment of the invention can be selected from List A2.

Where one wishes to generate an antibody or antibody mixture to treat and/or prevent an infectious disease caused or mediated by *H influenza,* one or more or all V, D and/or J gene segments used in any configuration, aspect, method, example or embodiment of the invention can be selected from List A1.1.

Where one wishes to generate an antibody or antibody mixture to treat and/or prevent an infectious disease caused or mediated by *E Coli or Neisseria meningitidis,* one or more or all V, D and/or J gene segments used in any configuration, aspect, method, example or embodiment of the invention can be selected from List A1.2.

Where one wishes to generate an antibody or antibody mixture to treat and/or prevent an infectious disease caused or mediated by Herpes Virus Family (eg, VZV or HSV), one or more or all V, D and/or J gene segments used in any configuration, aspect, method, example or embodiment of the invention can be selected from List A2.1.

Where one wishes to generate an antibody or antibody mixture to treat and/or prevent an infectious disease caused or mediated by CMV, one or more or all V, D and/or J gene segments used in any configuration, aspect, method, example or embodiment of the invention can be selected from List A2.2.

Where one wishes to generate an antibody or antibody mixture to treat and/or prevent an infectious disease caused or mediated by HIV, one or more or all V, D and/or J gene segments used in any configuration, aspect, method, example or embodiment of the invention can be selected from List A2.3.

Where one wishes to generate an antibody or antibody mixture to treat and/or prevent an infectious disease caused or mediated by Influenza Virus, one or more or all V, D and/or J gene segments used in any configuration, aspect, method, example or embodiment of the invention can be selected from List A2.4.

Optionally each VH segment in the locus of the invention is selected from List A1, A2, A1.1, A1.2, A2.1, A2.2, A2.3 or A2.4.

Optionally each VL segment in the locus of the invention is selected from List A1, A2, A1.1, A1.2, A2.1, A2.2, A2.3 or A2.4
Optionally each D segment in the locus of the invention is selected from List A1, A2, A1.1, A1.2, A2.1, A2.2, A2.3 or A2.4.

Optionally each J_{L} segment in the locus of the invention is selected from List A1, A2, A1.1, A1.2, A2.1, A2.2, A2.3 or A2.4.

### Antibodies For Therapy & Prophylaxis of Patients of Specific Ancestry

The inventors, having undertaken the extensive Bioinformatics analysis exercise described herein, realised that the output of that analysis has made it possible to identify specific gene segments that are useful to produce antibody- and VH domain-based drugs that are tailored specifically to a patient's ancestry (ie, genotype). That is, antibodies can be selected on the basis that they are made *in vivo* in a transgenic non-human vertebrate (eg, mouse or rat with transgenic IgH loci) and particularly derived from gene segments that are relatively prevalent in members of the patient's population, ie, from individuals of the same human ancestry. Since variant distributions differ across different populations (see Table 13), this presumably reflects the effects of evolution, adaptation and conservation of useful variant gene types in those populations. Thus, by tailoring the antibody-based drugs according to the invention, it is possible to match the drug to the population gene biases, thus with the aim of making better drugs for that specific population of humans. Better can, for example, mean more efficacious, better neutralising, higher target antigen affinity, less immunogenic, less patient reactions to the drug etc. This can be determined empirically, as is standard in drug research and development processes.

Thus, the invention provides the following embodiments (numbered from clause 345 onwards):-
345. An isolated antibody for administration to a Chinese patient, the antibody comprising a human heavy chain, the heavy chain comprising a variable domain that is specific for an antigen and a constant region, wherein the constant region is a human constant region selected from a constant region (eg, an IGHG constant region) inTable 13 found in a Chinese population and with a cumulative frequency of at least 1 or 5%;, and wherein
   (i) the variable domain is derived from the recombination of said human gene segments in a non-human vertebrate (eg, in a mouse or a rat); and/or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
   In another embodiment, the invention provides
   An isolated antibody for administration to a Chinese patient, the antibody comprising a human heavy chain, the heavy chain comprising a variable domain that is specific for an antigen and a constant region, wherein the constant region is a human constant region selected from a constant region (eg, an IGHG constant region) present in a Chinese population with a cumulative frequency of at least 5%;, and wherein
   (i) the variable domain is derived from the recombination of said human gene segments in a non-human vertebrate (eg, in a mouse or a rat);and/ or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
   In an example, the constant region is found in the 1000 Genomes database. In an example, the constant region is found in Table 13.
346. The antibody of clause 345 wherein the constant region is a IGHG1a, IGHG2a, IGHG3a, IGHG3b or IGHG4a constant region.
347. The antibody of clause 345 or 346, wherein the variable domain is derived from the recombination of a human VH gene segment with a human D gene segment and a human JH gene segment, the VH gene segment being selected from a VH in Table 13 found in a Chinese population and with a cumulative frequency of at least 5%.
   In another embodiment, the invention provides
   The antibody of clause 345 or 346, wherein the variable domain is derived from the recombination of a human VH gene segment with a human D gene segment and a human JH gene segment, the VH gene segment being selected from a VH present in a Chinese population with a cumulative frequency of at least 5%.
   In an example, the gene segment is found in the 1000 Genomes database. In an example, the gene segment is found in Table 13.
348. The antibody of clause 345, 346 or 347, wherein the variable domain is derived from the recombination of a human VH gene segment with a human D gene segment and a human JH gene segment, the D gene segment being selected from a D in Table 13 found in a Chinese population and with a cumulative frequency of at least 5%.
   In another embodiment, the invention provides
   The antibody of clause 345, 346 or 347, wherein the variable domain is derived from the recombination of a human VH gene segment with a human D gene segment and a human JH gene segment, the D gene segment being selected from a D present in a Chinese population with a cumulative frequency of at least 5%.
   In an example, the gene segment is found in the 1000 Genomes database. In an example, the gene segment is found in Table 13.
349. The antibody of clause 345, 346, 347 or 348 wherein the variable domain is derived from the recombination of a human VH gene segment with a human D gene segment and a human JH gene segment, the JH gene segment being selected from a JH in Table 13 found in a Chinese population and with a cumulative frequency of at least 5%.
   In another embodiment, the invention provides
   The antibody of clause 345, 346, 347 or 348 wherein the variable domain is derived from the recombination of a human VH gene segment with a human D gene segment and a human JH gene segment, the JH gene segment being selected from a JH present in a Chinese population with a cumulative frequency of at least 5%.
   In an example, the gene segment is found in the 1000 Genomes database. In an example, the gene segment is found in Table 13.
350. An isolated VH domain identical to a variable domain as recited in any one of clauses 347 to 349, optionally fused at its C-terminus to a polypeptide (eg, an antibody Fc).
   In an embodiment, there is provided an isolated VH domain identical to a variable domain as recited in any one of clauses 347 to 349 which is part of a conjugate, conjugated with a label (eg, for imaging in the patient) or a toxin (eg, a radioactive toxic payload, such as for cancer treatment in the patient) or a half-life-extending moiety (eg, PEG of human serum albumin).
351. A pharmaceutical composition comprising the antibody or variable domain of any one of clauses 345 to 350 together with a pharmaceutically-acceptable excipient, diluent or a medicament (eg, a further antigen-specific variable domain, antibody chain or antibody).
352. An isolated antibody for administration to a Chinese patient, the antibody comprising a human heavy chain, the heavy chain comprising a variable domain that is specific for an antigen and a constant region, wherein the variable domain is derived from the recombination of a human VH gene segment with a human D gene segment and a human JH gene segment, the VH gene segment being selected from a VH in Table 13 found in a Chinese population and with a cumulative frequency of at least 5%; and wherein
   (i) the variable domain is derived from the recombination of said human gene segments in a non-human vertebrate (eg, in a mouse or a rat); and/or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
   In another embodiment, the invention provides
   An isolated antibody for administration to a Chinese patient, the antibody comprising a human heavy chain, the heavy chain comprising a variable domain that is specific for an antigen and a constant region, wherein the variable domain is derived from the recombination of a human VH gene segment with a human D gene segment and a human JH gene segment, the VH gene segment being selected from a VH present in a Chinese population with a cumulative frequency of at least 5%; and wherein
   (i) the variable domain is derived from the recombination of said human gene segments in a non-human vertebrate (eg, in a mouse or a rat); and/or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
353. The antibody of clause 352, wherein the variable domain is derived from the recombination of a human VH gene segment with a human D gene segment and a human JH gene segment, the D gene segment being selected from a D in Table 13 found in a Chinese population and with a cumulative frequency of at least 5%.
   In another embodiment, the invention provides
   The antibody of clause 352, wherein the variable domain is derived from the recombination of a human VH gene segment with a human D gene segment and a human JH gene segment, the D gene segment being selected from a D present in a Chinese population with a cumulative frequency of at least 5%.
   In an example, the gene segment is found in the 1000 Genomes database. In an example, the gene segment is found in Table 13.
354. The antibody of clause 352 or 353, wherein the variable domain is derived from the recombination of a human VH gene segment with a human D gene segment and a human JH gene segment, the JH gene segment being selected from a JH in Table 13 found in a Chinese population and with a cumulative frequency of at least 5%.
   In another embodiment, the invention provides
   The antibody of clause 352 or 353, wherein the variable domain is derived from the recombination of a human VH gene segment with a human D gene segment and a human JH gene segment, the JH gene segment being selected from a JH present in a Chinese population with a cumulative frequency of at least 5%.
   In an example, the gene segment is found in the 1000 Genomes database. In an example, the gene segment is found in Table 13.
355. An isolated VH domain identical to a variable domain as recited in any one of clauses 352 to 354, optionally fused at its C-terminus to a polypeptide (eg, an antibody Fc).
   In an embodiment, there is provided a VH domain identical to a variable domain as recited in any one of clauses 352 to 354 which is part of a conjugate, conjugated with a label (eg, for imaging in the patient) or a toxin (eg, a radioactive toxic payload, such as for cancer treatment in the patient) or a half-life-extending moiety (eg, PEG of human serum albumin).
356. A pharmaceutical composition comprising the antibody or variable domain of any one of clauses 352 to 355 together with a pharmaceutically-acceptable excipient, diluent or a medicament (eg, a further antigen-specific variable domain, antibody chain or antibody).
357. An antibody heavy chain or VH domain (eg, provided as part of an antibody) for therapy and/or prophylaxis of a disease or medical condition in a Chinese patient, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH in Table 13 found in a Chinese population and with a cumulative frequency of at least 5%;
   (b) Optional humanisation of the heavy chain by combining the variable domain of the heavy chain with a human constant region; or optional humanisation of the selected VH domain by combining with a human constant region.
   In another embodiment, the invention provides
   An antibody heavy chain or VH domain (eg, provided as part of an antibody) for therapy and/or prophylaxis of a disease or medical condition in a Chinese patient, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH present in a Chinese population with a cumulative frequency of at least 5%;
   (b) Optional humanisation of the heavy chain by combining the variable domain of the heavy chain with a human constant region; or optional humanisation of the selected VH domain by combining with a human constant region.
   In an example, the VH gene segment is found in the 1000 Genomes database. In an example, the gene segment is found in Table 13.
358. The antibody heavy chain or VH domain of clause 357, wherein the human constant region is as recited in clause 345 or 346.
359. An antibody heavy chain or VH domain as recited in clause 357 or 358 for use in a medicament for therapy and/or prophylaxis of a disease or medical condition in a Chinese patient.
360. A method of treating and/or preventing a disease or medical condition in a Chinese patient, the method comprising administering to the patient a therapeutically or prophylactically-effective amount of the antibody heavy chain or VH domain as recited in clause 357 or 358.
361. An isolated antibody for administration to a patient of European, East Asian, West African, South Asian or Americas ancestry, the antibody comprising a human heavy chain, the heavy chain comprising a variable domain that is specific for an antigen and a constant region, wherein the constant region is a human constant region selected from a constant region (eg, an IGHG constant region) in Table 13 found in a population of European, East Asian, West African, South Asian or Americas ancestry respectively and with a cumulative frequency of at least 1 or 5%;, and wherein
   (i) the variable domain is derived from the recombination of said human gene segments in a non-human vertebrate (eg, in a mouse or a rat); or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
   In another embodiment, the invention provides
   An isolated antibody for administration to a patient of European, East Asian, West African, South Asian or Americas ancestry, the antibody comprising a human heavy chain, the heavy chain comprising a variable domain that is specific for an antigen and a constant region, wherein the constant region is a human constant region selected from a constant region (eg, an IGHG constant region) present in a population of European, East Asian, West African, South Asian or Americas ancestry respectively with a cumulative frequency of at least 1 or 5%;, and wherein
   (i) the variable domain is derived from the recombination of said human gene segments in a non-human vertebrate (eg, in a mouse or a rat); or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
   In an example, the constant region is found in the 1000 Genomes database. In an example, the constant region is found in Table 13.
362. The antibody of clause 361 wherein the constant region is a IGHG1a, IGHG2a, IGHG3a, IGHG3b or IGHG4a constant region and the patient is of European ancestry.
363. The antibody of clause 361 or 362, wherein the variable domain is derived from the recombination of a human VH gene segment with a human D gene segment and a human JH gene segment, the VH gene segment being selected from a VH in Table 13 found in said population and with a cumulative frequency of at least 1 or 5%.
   In another embodiment, the invention provides
   The antibody of clause 361 or 362, wherein the variable domain is derived from the recombination of a human VH gene segment with a human D gene segment and a human JH gene segment, the VH gene segment being selected from a VH present in a Chinese population with a cumulative frequency of at least 5%.
   In an example, the gene segment is found in the 1000 Genomes database. In an example, the gene segment is found in Table 13.
364. The antibody of clause 361, 362 or 363, wherein the variable domain is derived from the recombination of a human VH gene segment with a human D gene segment and a human JH gene segment, the D gene segment being selected from a D in Table 13 found in said population and with a cumulative frequency of at least 1 or 5%.
   In another embodiment, the invention provides
   The antibody of clause 361, 362 or 363, wherein the variable domain is derived from the recombination of a human VH gene segment with a human D gene segment and a human JH gene segment, the D gene segment being selected from a D present in a Chinese population with a cumulative frequency of at least 5%.
   In an example, the gene segment is found in the 1000 Genomes database. In an example, the gene segment is found in Table 13.
365. The antibody of clause 361, 362, 363 or 364 wherein the variable domain is derived from the recombination of a human VH gene segment with a human D gene segment and a human JH gene segment, the JH gene segment being selected from a JH in Table 13 found in said population and with a cumulative frequency of at least 1 or 5%.
   In another embodiment, the invention provides
   The antibody of clause 361, 362, 363 or 364 wherein the variable domain is derived from the recombination of a human VH gene segment with a human D gene segment and a human JH gene segment, the JH gene segment being selected from a JH present in a Chinese population with a cumulative frequency of at least 5%.
   In an example, the gene segment is found in the 1000 Genomes database. In an example, the gene segment is found in Table 13.
366. An isolated VH domain identical to a variable domain as recited in any one of clauses 363 to 365, optionally fused at its C-terminus to a polypeptide (eg, an antibody Fc).
367. A pharmaceutical composition comprising the antibody or variable domain of any one of clauses 361 to 366 together with a pharmaceutically-acceptable excipient, diluent or a medicament (eg, a further antigen-specific variable domain, antibody chain or antibody).
368. An isolated antibody for administration to a patient of European, East Asian, West African or Americas ancestry, the antibody comprising a human heavy chain, the heavy chain comprising a variable domain that is specific for an antigen and a constant region, wherein the variable domain is derived from the recombination of a human VH gene segment with a human D gene segment and a human JH gene segment, the VH gene segment being selected from a VH in Table 13 found in a population of European, East Asian, West African, South Asian or Americas ancestry respectively and with a cumulative frequency of at least 1 or 5%; and wherein
   (i) the variable domain is derived from the recombination of said human gene segments in a non-human vertebrate (eg, in a mouse or a rat); or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
   In another embodiment the invnention provides:-
   An isolated antibody for administration to a patient of European, East Asian, West African or Americas ancestry, the antibody comprising a human heavy chain, the heavy chain comprising a variable domain that is specific for an antigen and a constant region, wherein the variable domain is derived from the recombination of a human VH gene segment with a human D gene segment and a human JH gene segment, the VH gene segment being selected from a VH present in a population of European, East Asian, West African, South Asian or Americas ancestry respectively with a cumulative frequency of at least 1 or 5%; and wherein
   (i) the variable domain is derived from the recombination of said human gene segments in a non-human vertebrate (eg, in a mouse or a rat); or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
   In an example, the VH gene segment is found in the 1000 Genomes database. In an example, the gene segment is found in Table 13.
369. The antibody of clause 368, wherein the variable domain is derived from the recombination of a human VH gene segment with a human D gene segment and a human JH gene segment, the D gene segment being selected from a D in Table 13 found in said population and with a cumulative frequency of at least 1 or 5%.
   In another example there is provided
   The antibody of clause 368, wherein the variable domain is derived from the recombination of a human VH gene segment with a human D gene segment and a human JH gene segment, the D gene segment being selected from a D present in said population with a cumulative frequency of at least 1 or 5%.
   In an example, the D gene segment is found in the 1000 Genomes database. In an example, the gene segment is found in Table 13.
370. The antibody of clause 368 or 369, wherein the variable domain is derived from the recombination of a human VH gene segment with a human D gene segment and a human JH gene segment, the JH gene segment being selected from a JH in Table 13 found in said population and with a cumulative frequency of at least 1 or 5%.
   In another example there is provided
   The antibody of clause 368 or 369, wherein the variable domain is derived from the recombination of a human VH gene segment with a human D gene segment and a human JH gene segment, the JH gene segment being selected from a JH present in said population and with a cumulative frequency of at least 1 or 5%.
   In an example, the JH gene segment is found in the 1000 Genomes database. In an example, the gene segment is found in Table 13.
371. An isolated VH domain identical to a variable domain as recited in any one of clauses 368 to 370, optionally fused at its C-terminus to a polypeptide (eg, an antibody Fc).
372. A pharmaceutical composition comprising the antibody or variable domain of any one of clauses 368 to 371 together with a pharmaceutically-acceptable excipient, diluent or a medicament (eg, a further antigen-specific variable domain, antibody chain or antibody).
373. An antibody heavy chain or VH domain (eg, provided as part of an antibody) for therapy and/or prophylaxis of a disease or medical condition in a patient of European, East Asian, West African, South Asian or Americas ancestry, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH in Table 13 found in said population and with a cumulative frequency of at least 1 or 5%;
   (b) Optional humanisation of the heavy chain by combining the variable domain of the heavy chain with a human constant region; or optional humanisation of the selected VH domain by combining with a human constant region.
   In another embodiment, there is provided:-
   An antibody heavy chain or VH domain (eg, provided as part of an antibody) for therapy and/or prophylaxis of a disease or medical condition in a patient of European, East Asian, West African, South Asian or Americas ancestry, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH present in said population with a cumulative frequency of at least 1 or 5%;
   (b) Optional humanisation of the heavy chain by combining the variable domain of the heavy chain with a human constant region; or optional humanisation of the selected VH domain by combining with a human constant region.
   In an example, the VH gene segment is found in the 1000 Genomes database. In an example, the gene segment is found in Table 13.
374. The antibody heavy chain or VH domain of clause 373, wherein the human constant region is as recited in clause 361 or 362.
375. An antibody heavy chain or VH domain as recited in clause 373 or 374 for use in a medicament for therapy and/or prophylaxis of a disease or medical condition in a patient of said ancestry.
376. A method of treating and/or preventing a disease or medical condition in a patient of European, East Asian, West African, South Asian or Americas ancestry, the method comprising administering to the patient a therapeutically or prophylactically-effective amount of the antibody heavy chain or VH domain as recited in clause 373 or 374.

In embodiments herein, a Chinese patient can be a Han Chinese patient.

In embodiments herein, a patient of European ancestry can be a patient of Northern or Western European ancestry, Italian ancestry, British or Scottish ancestry, Finnish ancestry or Iberian ancestry.

In embodiments herein, a patient of East Asian ancestry can be a patient of Han Chinese ancestry, Japanese ancestry Chinese Dai ancestry, Vietnamese ancestry or Kinh ancestry.

In embodiments herein, a patient of West African ancestry can be a patient of Yoruba ancestry, Luhya ancestry, Gambian ancestry or Malawian ancestry.

In embodiments herein, a patient of Americas ancestry can be a patient of African American ancestry, African Caribbean ancestry, Mexican ancestry, Puerto Rican ancestry, Colombian ancestry or Peruvian ancestry.

In embodiments herein, a patient of South Asian ancestry can be a patient of Ahom ancestry, Kayadtha ancestry, Reddy ancestry, Maratha ancestry, or Punjabi ancestry.

In an example of any aspect, the cumulative frequency is at least 1, 2, 3, 4, 5, 6, 7, 8, 9, 10, 15, 20, 25, 30, 35, 40, 45, 50, 55, 60, 65, 70, 75, 80, 85, 90 or 95%.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps
The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

Any part of this disclosure may be read in combination with any other part of the disclosure, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. All such similar substitutes and modifications apparent to those skilled in the art are deemed to be within the spirit, scope and concept of the invention as defined by the appended claims.

The present invention is described in more detail in the following non limiting prophetic Examples.

### EXAMPLES

### EXAMPLE 1

### "Recombineered BAC Vectors to add Polymorphic V-regions to the Mouse Genome"

Figure 1 through 3 depict recombineering methods (see references above) that can be used to introduce polymorphic V-gene regions into genomic DNA. In one embodiment, a genomic fragment from the human heavy chain region is inserted into a bacterial artificial chromosome (BAC) vector by standard techniques. Preferably, such a BAC, which can range in size from 20-kb to 200-kb or more, can be isolated from libraries of BACs by standard techniques including sequence searches of commercially available libraries or by hybridization to bacterial colonies containing BACs to identify those with a BAC of interest.

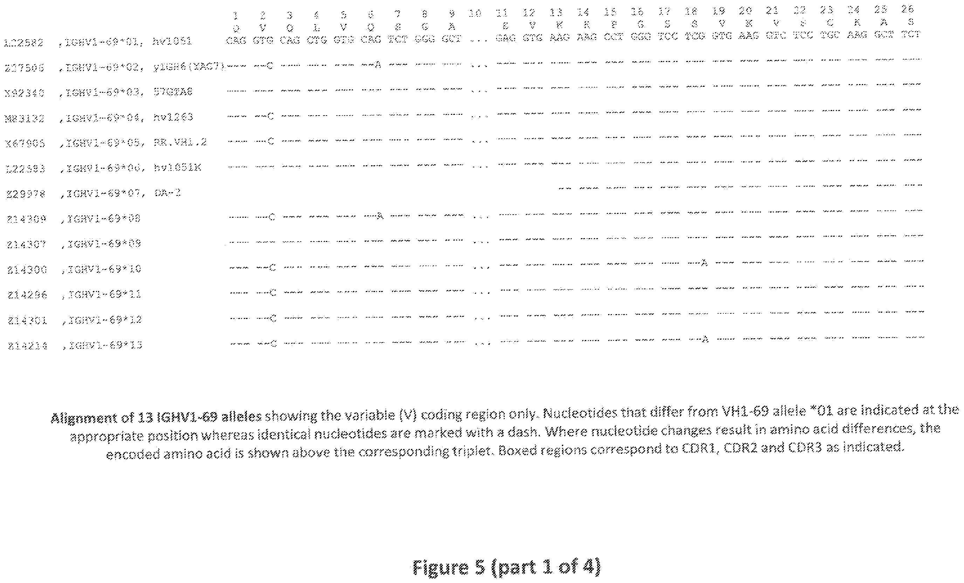

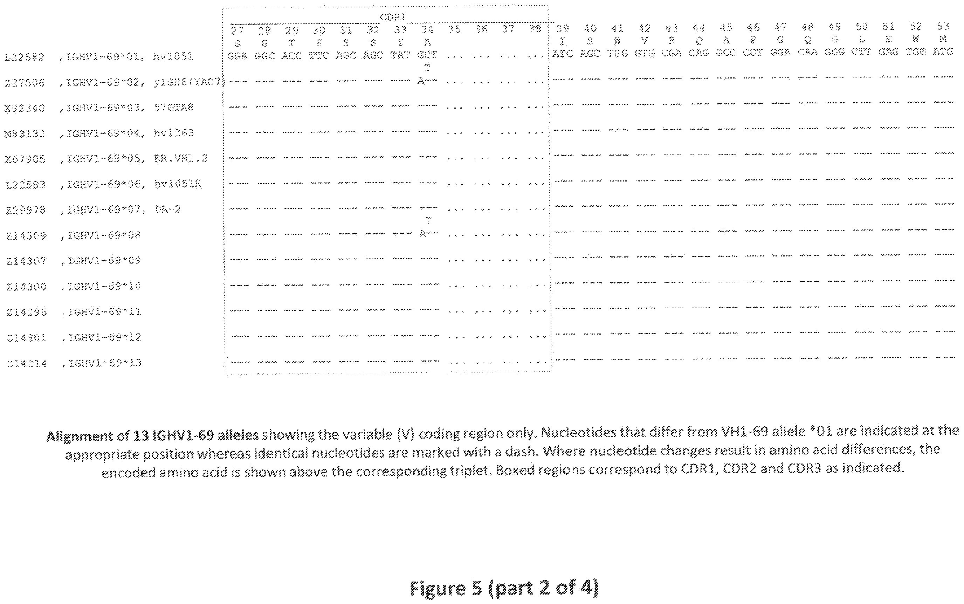

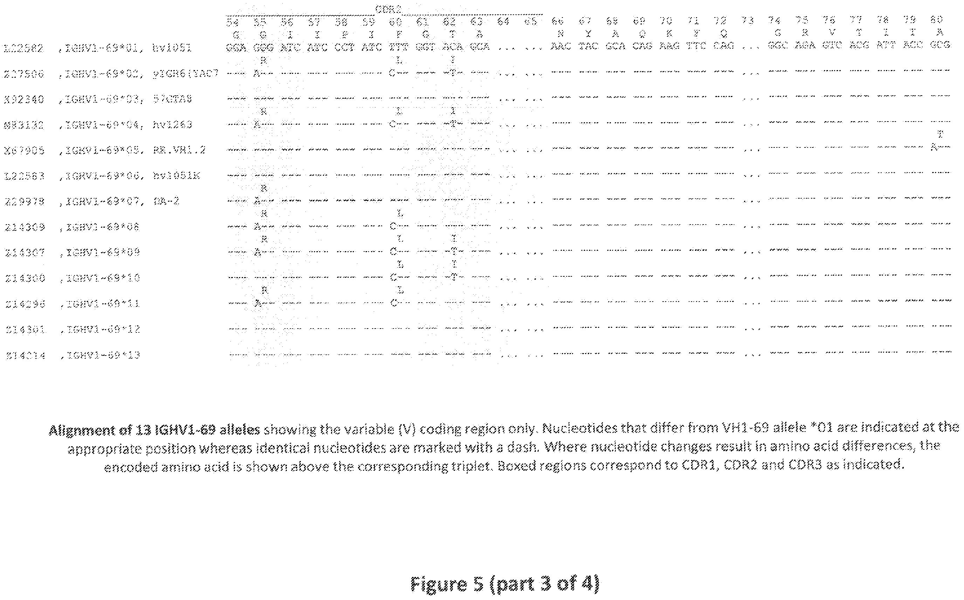

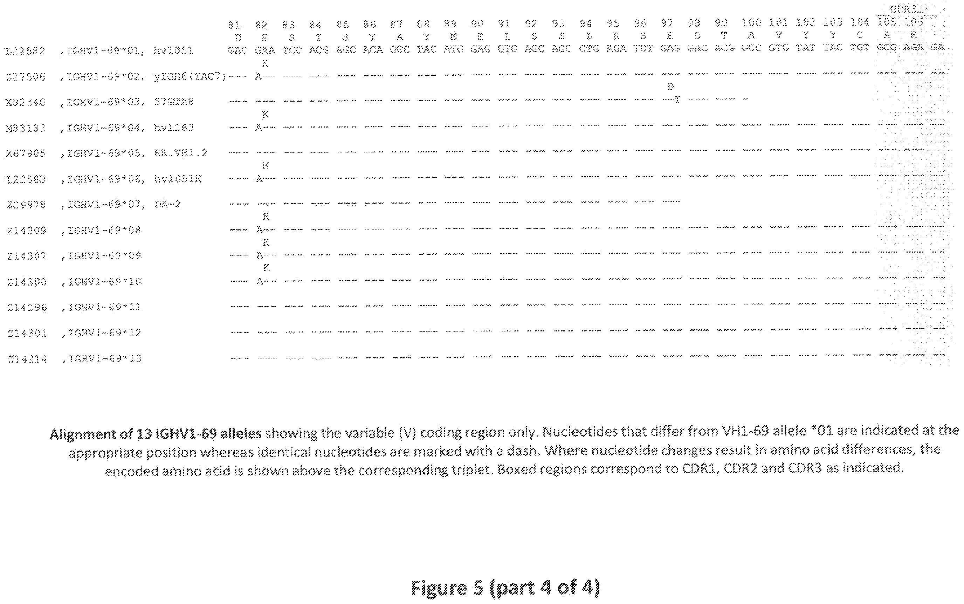

A BAC is chosen that has several VH gene segments; in Figure 1, these are generically identified as VH[a] through VH[z] for example. One skilled in the art will readily identify appropriate genomic fragments, for example, an approximately 120-kb fragment from human VH5-78 through VH1-68 which includes 5 endogenous active VH gene segments and 7 VH psuedogenes. Using recombineering techniques, the endogenous VH gene segments can be replaced by polymorphic VH or VL gene segments. In this example, two steps are required. The first step replaces the V-region coding exon of an endogenous VH gene segment with a positive-negative selection operon, in this example, an operon encoding an ampicillin resistance gene (*Amp*) and a streptomycin-sensitizing ribosomal protein (*rpsL*)*.* Certain strains of bacteria can be selected for the absence of the *rpsL* gene by resistance to streptomycin. Short stretches of DNA homologous to sequences flanking the endogenous VH gene exon are placed 5' and 3' of the rpsL-Amp operon. In the presence of appropriate recombination factors per standard recombineering techniques (see references above) recombination between the operon fragment and the BAC will result in replacement of the endogenous VH gene exon with the operon (Figure 1a) which are selected by resistance to ampicillin. The second step uses the same homologous sequences in order to replace the inserted operon with a desired polymorphic VH gene segment. In this example, a human VH1-69 gene is inserted (Figure 1b and 1c). In particular the *02 variant of VH1-69 is used [ref IMGT and Figure 5]. Successful integrations of the polymorphic VH gene segment are selected in bacteria that become resistant to streptomycin due to the loss of the operon, specifically the *rpsL* portion.

In this example, the two step process as described can be repeated for each of the endogenous VH gene segments or for as many endogenous gene segments that one wishes to replace with polymorphic V gene segments (Figure 1d).

As is apparent, any polymorphic V gene segment can be inserted in this manner and any endogenous V gene segment can act as a target, including pseudogenes. V gene segments in each of the heavy chain and two light chain loci can be replaced using this technique with appropriate genomic fragments available as BAC inserts.

Figure 2 depicts another method for creating a genomic fragment encoding polymorphic V gene segments. In this example, polymorphic V gene segments are inserted into a region of genomic DNA devoid of other genes, control elements or other functions. Such 'desert' regions can be selected based on sequence analysis and corresponding DNA fragments cloned into BACs or identified in existing BAC libraries. Starting with such a genomic fragment, recombineering techniques can be used to insert polymorphic V gene segments at intervals of, for example, 10-kb. In this example, a 150-kb genomic fragment might accommodate insertion of up to 15 polymorphic V gene segments. Insertion of the segments is a two-step process. The first recombineering step inserts the *rpsL-Amp* operon at a specific site. Sequences homologous to a specific site are used to flank the operon. These are used by the recombineering system to insert the element specifically into the BAC genomic fragment and positive events are selected by resistance to ampicillin (Figure 2a). The second step replaces the operon in the genomic fragment with a polymorphic V gene segment by a similar recombineering step using the same sequence homology (Figure 2b). In this example, both exons and promoter element of a polymorphic VH gene segment are inserted, resulting in replacement of the *rpsL-Amp* operon and therefore resistance to streptomycin (Figure 2c).

The two step technique for inserting polymorphic V gene segments into a specific site on the genomic fragment can be repeated multiple times resulting in a BAC genomic fragment with several polymorphic gene segments, including their promoter elements. It is apparent that the examples shown in Figures 1 and 2 can be combined wherein the technique for insertion can be used to add *extra* polymorphic V gene segments to a BAC genomic fragment as depicted in Figure 1. One might choose to add these extra segments to an IG genomic fragment since such a fragment would be more amenable to proper IG gene expression once inserted into a non-human mammal's genome. It is known that a genomic fragment can have elements such as enhancers or elements that contribute to certain chromatin conformations, both important in wild-type gene expression.

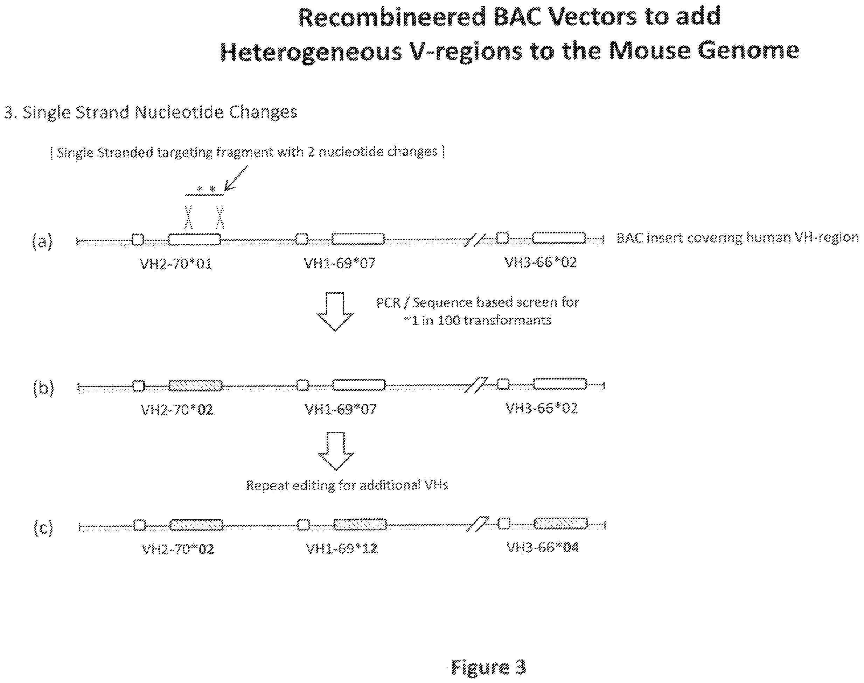

Figure 3 depicts an additional method to create genomic fragments with polymorphic V gene segments. This method depends upon the efficiency with which short (around 50 to 150 bases, preferably 100 bases) single stranded DNA fragments recombine with a homologous sequence using recombineering (Nat Rev Genet. 2001 Oct;2(10):769-79; Recombineering: a powerful new tool for mouse functional genomics; Copeland NG, Jenkins NA, Court DL). The recombinases used in recombineering preferentially bind and use such short single-stranded fragments of DNA as a substrate for initiating homologous recombination. The efficiency can be as high as 10-2, that is, a positive event can be found in approximately 100 randomly picked (not selected) clones resulting from recombineering. A positive event in this example occurring when one or more single nucleotide changes introduced into the single-stranded fragment get transferred to the BAC insert containing V gene segments and surrounding genomic DNA, said nucleotide change or changes occurring at a homologous sequence on the BAC.

Polymorphic V gene segments can differ from endogenous V gene segments by only 1 or 2, or up to 10 or 15 nucleotide changes, for example. An example of such nucleotide polymorphisms are depicted in Figure 5. Short single stranded regions that encompass the polymorphic nucleotide changes can be chemically synthesized using standard techniques. The resulting single stranded DNA fragments are introduced into bacteria and *via* recombineering techniques approximately 1 in 100 BAC fragments will have incorporated the polymorphic nucleotides *via* homologous incorporation of the single stranded fragment (Figure 3a). BACs with the desired nucleotide change can be identified by screening for example several hundred individual clones by polymerase chain reaction (PCR) amplification and sequencing, both by standard techniques. In the example, two nucleotide changes will convert a VH1-69*01 gene segment into a VH1-69*02 gene segment (Figure 3b).

It is clear that this process can be repeated for multiple endogenous V gene segments contained on a single BAC genomic fragment. In addition, the techniques depicted in Figure 2 can be used to add additional polymorphic V gene segments by insertion into regions between existing V gene segments. As would be evident to one skilled in the art, a combination of these techniques can be used to create numerous variations of both polymorphic and endogenous human V gene segments. And it would be evident that several different genomic fragments with engineered polymorphic V gene segments and endogenous human V gene segments can be combined to create even more variations.

### EXAMPLE 2

### "Adding Polymorphic V-regions to the Genome using SRMCE of Modified BACs"

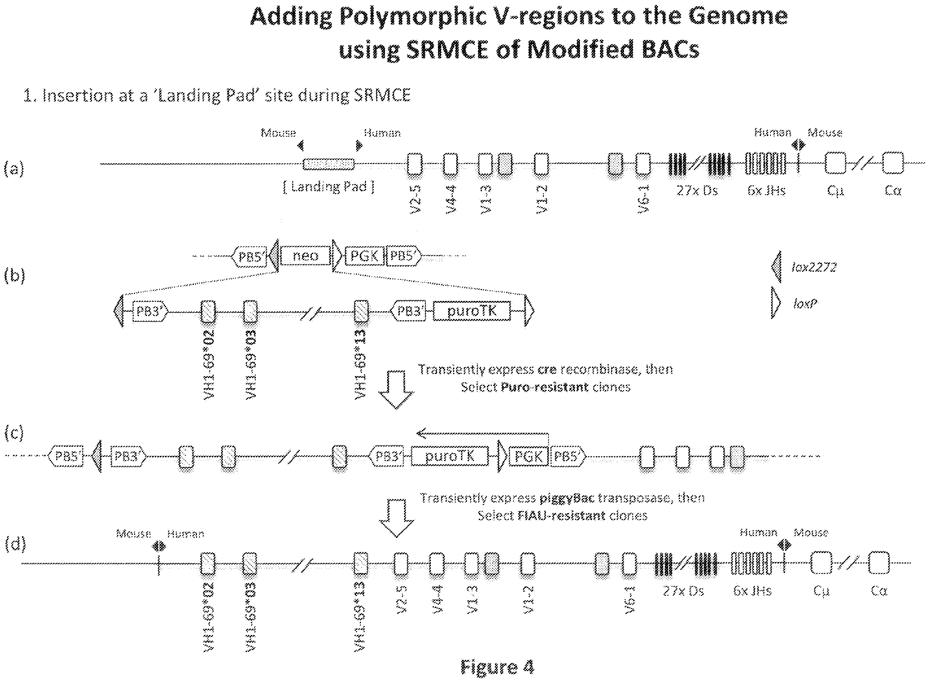

Modified BACs with polymorphic V gene segments created using the methods described in Example 1 can be used to alter the genome of non-human mammals. These alterations can result in an intact IG locus in which normal immunoglobin region recombination results in VDJ or VJ combinations which includes the human V gene segments. An example of how such an animal can be created is by altering the genome of, for example, mouse embryonic stem (ES) cells using the strategy outlined in Figure 4.

One technique to integrate modified BACs with polymorphic V gene segments into a genome is sequential recombinase mediated cassette exchange (SRMCE). The technique is described in WO2011004192 (Genome Research Limited), which is incorporated here in its entirety by reference. SRMCE provides for a locus modified with a 'landing pad' inserted at a specific location. This insertion can either be *de novo* via homologous recombination or as a consequence of a previous BAC insertion. In this example, the landing pad is inserted in the mouse IGH locus between the most 3' J gene segment and the Cµ gene segment and a previous BAC insertion *via* SRMCE techniques have resulted in the addition of 5 human V gene segments and 2 V region pseudogenes. The landing pad has elements as shown in Figure 4 that will allow the selection of correct insertion of a second targeting BAC fragment. The specificity of this insertion is provided by cre recombinase-mediated exchange between permissive *Iox* sites. A *Iox* site is permissive for recombination only with a compatible lox site. In this example, the *IoxP* site will only recombine with *IoxP* and *Iox2272* will only recombine with *Iox2272.* This provides directionality to the insertion of the BAC fragment as depicted in Figure 4b and 4c.

ES cell clones with correct insertions are selected from a pool of clones without insertions or with non-productive insertions by resistance to puromycin. Resistance to puromycin results from the juxtaposition of an active promoter element, PGK, with the *puroTK* coding region. Correct insertions are verified by standard techniques including PCR of junctions, PCR of internal elements, Southern blotting, comparative genomic hybridization (CGH), sequencing and *etc.* In the example, correct *Iox2272-Iox2272* and *IoxP-IoxP* recombination also results in two intact sets of *piggyBac* elements that did not exist prior to insertion. An intact piggyBac element is comprised of a set of inverted repeats which are depicted in the figure by "PB5"' and "PB3"'. An appropriated oriented set of *piggyBac* elements are the substrate of *piggyBac* transposase which can catalyse recombination between the elements, resulting in deletion of intervening sequences as well as both elements. The DNA remaining after a *piggyBac* transposition is left intact and is lacking any remnant of the *piggyBac* element. In the example, ES cell clones with successful piggyBac transposition are selected by loss of the active *puroTK* element which renders the cells resistant to the drug FIAU (Figure 4c and 4d).

The final product of the SRMCE method in this example is a IGH locus with several polymorphic V gene segments inserted along with a set of endogenous unmodified VH gene segments between sequences of the mouse genome on the 5' side and the mouse IGH constant region gene segments on the 3' side. The polymorphic V gene segments are positioned such that they can participate in the recombination events associated with B cell maturation yielding VDJ gene segments. These gene segments can then be transcribed and spliced to the mouse constant region. Translation of these transcripts will result in the production of an antibody heavy chain encoded by the polymorphic V gene segment, a human DH gene segment, a human JH gene segment and a mouse constant heavy chain gene segment.

As is well known to those skilled in the art, an ES cell clone can be used to create a line of genetically modified mice *via* injection of said cells into a mouse blastocyst embryo, transferring the injected embryo to a suitable recipient and breeding the chimeric offspring that result. The modified gene locus can be propagated through breeding and made either heterozygous or homozygous depending on the genetic cross.

It is evident from the structure of the IGH locus provided in this example and by knowledge of the mechanisms involved in B cell receptor (BCR) and antibody gene rearrangements that a large set of different combinations of polymorphic V gene segments with various DH and JH gene segments will result and these can contribute to a large repertoire of functional antibody genes in a population of B cells in genetically modified animals. In this example, several different human VH1-69 polymorphs are incorporated to provide superhuman VH diversity. This particular VH gene segment is known to be prevalent in antibodies that bind infectious disease pathogens (such as influenza virus) and therefore the antibody repertoire of a mouse with the genetic modification of this example would be expected to produce antibodies with a bias in favour of those that bind infectious disease pathogens. The repertoire, in other words, would have a larger subset of antibodies with superior affinities for pathogen antigens. Examples of such pathogens include influenza virus, hepatitis C virus (HCV) and human immunodeficiency virus-1 (HIV-1) (see also table above).

### EXAMPLE 3

### "Alignment of 13 VH1-69 Alleles"

Building a more diverse antibody repertoire by incorporating additional V gene segment polymorphs requires availability of polymorphic variants of V gene segments. One source of such variants include sequence databases. In this example, 13 distinct variants of the VH1-69 gene segment are provided.

These variant sequences and comparisons are drawn from the "IMmunoGeneTics" IMGT Information System (www.imgt.com) database. Figure 5 is a diagram of the alignment of variants *02 through *13 with the *01 variant. The VH1-69*01 nucleotide and amino acid sequence is provided at the top of the figure. Where the remaining variants are identical to the *01 variant sequence a dash is inserted below the sequence. Nucleotide differences are noted alongside the appropriate variant and if the sequence change results in a protein coding change, the amino acid change is indicated above the triplet.

Figure 5 depicts between 1 and 4 amino acid changes for each variant in comparison to the *01 variant. All of the amino acid changes occur in the part of the heavy chain protein encoding the complementarity determining regions (CDRs). These regions are responsible for antigen specificity and the affinity of the antibody for the antigen. It is evident that providing additional polymorphic CDRs in a repertoire of antibodies will increase the likelihood of there being an antibody with superior binding characteristics for various antigens. In several reports, it has been observed that the VH1-69-encoded variable region of the heavy chain is often found in antibodies that bind influenza virus, HCV and HIV-1 antigens (see table above). Therefore incorporating the polymorphic V gene segments of this example into a transgenic animal model using the methods of Examples 1 and 2 would likely result in an antibody repertoire in said transgenic animal with more antibodies that bind to antigens associated with these and other pathogens. And as is known in the art, a larger repertoire increases the probability of finding monoclonal antibodies using, for example, hybridoma technology, that bind with high affinity and specificity to a desired antigen.

This disclosure therefore describes in these examples a transgenic mouse model which can be immunized with pathogen or other antigens. Plasma B cells from such an immunized mouse can be used to make a hybridoma library that can be screened for production of antibodies that bind the pathogen antigens. This library will be superior to libraries from traditional transgenic mice for finding such antibodies given the addition of polymorphic VH1-69 gene segments to the IGH locus in said transgenic mouse.

These examples are not limiting to the human polymorphic V gene segments that can be chosen or to the methods used to introduce them into an animal model. The method can be used to construct a transgenic locus with immunoglobulin D and/or J segments. The V, D, J segments can be from a plurality of human sources (optionally more than one human ethnic population).

### EXAMPLE 4

### Human IgH JH Gene Variants Selected from the 1000 Genomes Database

Data is presented for human JH2, 5 and 6 variants. In Tables 10A, 11A and 12A samples from humans from various populations are listed where the sequence analysis of the inventors has revealed the presence of polymorphisms in one or both IgH JH alleles. The population codes are explained in Table 8 above. The polymorphisms are nucleotide variants from JH2, 5 and 6 reference sequences (SEQ ID NOs: 1, 2 and 3 respectively; see below). All references are sequences taken from the Ensembl database (*www.**ensembl**.org*). The JH5 reference is human IgH J5-001 disclosed in that database. The JH6 reference is human IgH J6-001 disclosed in that database. The JH2 reference is human IgH J2-001 disclosed in that database.

The reference nucleotide and encoded amino acid sequences are shown on the next page. Alignments with encoded amino acid sequences are also provided, including the corresponding position numbers on human chromosome 14.

Variant Frequencies are shown in Tables 10A, 11A and 12A and these relate to the frequency of the variants in the 1000 Genomes Database (release current at October 2011).

Tables 10B, 11B and 12B show the non-synonymous nucleotide polymorphisms in the human JH variants, as sorted by the present inventors from the 1000 Genomes database. Position numbers corresponding to nucleotide positions on human chromosome 14 are shown for variant positions (chromosome 14 being the chromosome bearing the IgH locus in humans). Thus, for example, the first entry in Table 11B is "14:106330027:A/C" which refers to a position in a variant JH5 sequence wherein the position corresponds to position 106,330,027 on human chromosome 14, such position being A (adenine) in the reference sequence. The "C" indicates that the present inventors observed a mutation to cytosine at this position in the variants found in the 1000 Genomes database. This change leads to a change at the amino acid level of the encoded sequence (ie, a "non-synonymous" change), in this case a change from a serine (found in the reference) to an alanine in the variant.

### Example 5:

### Human Antibody Gene Segment Variant Identification & Population Analysis

The genomic coding region coordinates for each target gene for variant analysis were identified from the Ensembl WWW site (www.ensembl.org) using coordinates from the GRCh.p8 Human Genome assembly (www.ncbi.nlm.nih.gov/projects/genome/assembly/grc). Using the collected gene location coordinates, variant data was extracted from the public ftp site of the 1000 Genomes Project using the Perl 'Variant Pattern Finder' (VPF - www.1000genomes.org/variation-pattern-finder-api-documentation).

Data extracted by VPF was post processed using software to extract all non-synonymous (NSS) variants with their associated genotype calls. Genotypes calls were assembled to form unique haplotypes, representing groups of NSS variants associated with 1000 Genome population groups and frequency of occurrence within those populations.

The output of the analysis results in tables such as in Table 13. The main body of the table describes each haplotype in turn giving a unique ID for that gene (in the range a-z,aa-zz), the population frequencies and occurrence in individuals and unique population groups; one or more subsequent columns describe the DNA base calls at each location that form the haplotype giving both the base from the reference sequence or the variant base call.

Table 13 was constructed in this manner. The table can be read as follows:
The first four columns (left to right) consist of (1) the haplotype ID letter ('ref' indicates reference - the DNA base call at each genomic location from the GRCh37 Human Reference Assembly) (2) the observed cumulative frequency of the haplotype among the different populations (3) the number of individuals in which a specific haplotype was observed (4) the number of unique population groups that the identified individuals belong to (the actual population group identifiers are displayed as a string of ID's in the most right hand column for each haplotype. For example haplotype 'a' has a population ID string of '3,4,9,13').

The populations are numbered as follows (population labels being according to 1000 Genomes Project nomenclature)
1= ASW;
2= CEU;
3=CHB;
4=CHS;
5=CLM;
6=FIN;
7=GBR;
8=IBS;
9=JPT;
10=LWK;
11=MXL;
12=PUR;
13=TSI;
14=YRI.

Subsequent columns detail a single point variant and have the following format (top to bottom) (1) the human genomic location of the variant (format [chromosome number]: [location] e.g. '14:106204113'); (2) The identifier for the point variant as defined in DbSNP (www.ncbi.nlm.nih.gov/proiects/SNP/); (3) One or additional rows show the amino acid change as result of the variant for a specific transcript (denoted by the Ensembl transcript ID in the most right-hand column for each row), the format is the amino acid in the reference sequence followed by '->' and the amino acid caused by the substitution of the variant in the reference sequence (e.g. 'Gly->Arg' means a that the translated reference sequence would result in a glycine at that location, whereas the substitution of the identified variant would result in translated protein containing arginine) using the IUPAC three letter amino acid codes (http://pac.iupac.org/publications/pac/pdf/1972/pdf/3104x0639.pdf). Subsequent rows (one per haplotype) show the DNA base at each location, bases matching the reference sequence are shown in black on white back ground, bases varying from the reference are shown as white text on a black background.

The most right-hand column contains the Ensembl transcript ID's (e.g. 'ENST00000390542') for each of the gene transcript and relates to the amino acid changes to the left of this column. Because the transcripts are differing lengths each variant position may or may not have an associated amino acid change at the that position.

### Example 6:

### Transgenic Mice, B-cells, Hybridomas, Antibodies & Heavy Chains Based on Human JH6*02

A functional human gene segment repertoire (from V_{H}2-26 to J_{H}6, see the IMGT database for the structure of the human IgH locus; http://www.imgt.org/IMGTrepertoire/index.php?section=LocusGenes&repertoire=locus&species=h uman&group=IGK) was sectored by the inventors to produce two different transgenic heavy chain alleles (denoted S2F and S3F) and corresponding mice. The transgenic alleles were expressed in the mice and the heavy chain repertoires were assessed at the RNA transcript level. Deep sequence analysis was carried out using Bioinformatics methods to assess V, D and JH gene usage, including in variable domain sequences having a HCDR3 length of at least 20 amino acids. Endogenous, mouse variable region gene segments were inactivated by inversion (as per the method described in WO2011004192, this disclosure being incorporated herein by reference).

### Sequencing of Human Donor DNA Samples: Identification of Conserved JH6*02 Variant

DNA samples from 9 anonymised consenting human donors were obtained by taking cheek swabs.

The samples were processed and the DNA Samples were extracted follow the protocol of QIAamp DNA Mini Kit (Cat.No.51304, Qiagen).

PCR reactions were set up to amplify the JH6 region and PCR products were sequenced (PCR Oligos sequence: Fwd. 5'-AGGCCAGCAGAGGGTTCCATG-3' (SEQ ID NO: 444), Rev. 5'-GGCTCCCAGATCCTCAAGGCAC-3' (SEQ ID NO: 445)).

Sequence analysis was carried out by comparing to the JH6 reference sequence from IMGT annotated database (http://www.imgt.org/), and this identified that all 9 donor genomes contained the human JH6*02 variant, with this variant being in the homozygous state in 7 out of the 9 donors. The inventors also consulted the genomic sequences publicly available for Jim Watson and Craig Venter at Ensembl human genome database [http://www.ensembl.org/]. These too contained the human JH6*02 variant. This confirmed to the inventors that human JH6*02 is a common, conserved variant in humans, and thus a good candidate for construction of a transgenic IgH locus as per the invention

### Identification of Suitable Human DNA Sequence BACs

A series of human bacterial artificial chromosome (BAC) clones were identified from Ensemble (http://www.ensembl.org/index.html) or UCSC (http://genome.ucsc.edu/) human database searches based on gene name (IGH) or location (chromosome 14: 106026574-107346185). Seven human RP11 BAC clones (see an extract of the UCSC databse in Figure 10, identified BACs being circled) were selected, RP11-1065N8 BAC carrying human JH6*02. In total, the following BACs were identified as sources of human IgH locus DNA: RP11-1065N8, RP11-659B19, RP11-141I7, RP-112H5, RP11-101G24, RP11-12F16 and RP11-47P23.

With a similar approach, different BAC clones (eg, different RP11 clone IDs or different sources from RP11) or genetically engineered BACs can be selected for insertion into the mouse IGH locus to provide different sets of human repertoires in the transgenic mouse.

### Construction of Transgenic IgH Loci

Insertion of human heavy gene segments from a 1st IGH BAC (RP11-1065N8) into the IGH locus of mouse AB2.1 ES cells (Baylor College of Medicine) was performed to create a heavy chain allele denoted the S1 allele. The inserted human sequence corresponds to the sequence of human chromosome 14 from position 106494908 to position 106328951 and comprises functional heavy gene segments V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6 (in 5' to 3' order), wherein the JH6 was chosen to be the human JH6*02 variant. The insertion was made between positions 114666435 and 114666436 on mouse chromosome 12, which is upstream of the mouse Cµ region. The mouse V_{H}, D and J_{H} gene segments were retained in the locus, immediately upstream of (5' of) the inserted human heavy chain DNA.

A second allele, S2 was constructed in which more human functional V_{H} gene segments were inserted upstream (5') of the 5'-most V_{H} inserted in the S1 allele by the sequential insertion of human DNA from a second BAC (BAC2). The inserted human sequence from BAC2 corresponds to the sequence of human chromosome 14 from position 106601551 to position 106494909 and comprises functional heavy chain gene segments V_{H}3-13, V_{H}3-11, V_{H}3-9, V_{H}1-8, V_{H}3-7. The mouse V_{H}, D and J_{H} gene segments were retained in the locus, immediately upstream of (5' of) the inserted human heavy chain DNA. In a subsequent step, these were inverted to inactivate them, thereby producing S2F mice in which only the human heavy chain variable region gene segments are active.

A third allele, S3 was constructed in which more human functional V_{H} gene segments were inserted upstream (5') of the 5'-most V_{H} inserted in the S2 allele by the sequential insertion of human DNA from a third BAC (BAC3). The inserted sequence corresponds to the sequence of human chromosome 14 from position 106759988 to position 106609301, and comprises functional heavy chain gene segments, V_{H}2-26, V_{H}1-24, V_{H}3-23, V_{H}3-21, V_{H}3-20, V_{H}1-18, and V_{H}3-15. The mouse V_{H}, D and J_{H} gene segments were retained in the locus, immediately upstream of (5' of) the inserted human heavy chain DNA. In a subsequent step, these were inverted to inactivate them, thereby producing S3F mice in which only the human heavy chain variable region gene segments are active.

Mice bearing either the S2F or S3F insertion into an endogenous heavy chain locus were generated from the ES cells using standard procedures. The other endogenous heavy chain locus was inactivated in the mice by insertion of an inactivating sequence comprising neo^{R} into the mouse J_{H}-Cµ intron (to produce the "HA" allele).

### Immunisation procedure

Transgenic mice of the S2F or S3F genotype were primed with 20-40ug recombinant proteins obtained commercially or produced in house with Antigen 1 (OVA (Sigma A7641); Antigen 2 (a human infectious disease pathogen antigen) and Antigen 3 (a human antigen) via the ip route in complete Freunds adjuvant (Sigma F 5881) and 10ug/animal CpG (CpG oligo; Invivogen, San Diego, California, USA) and then boosted twice in about two weekly intervals with about half the amount of antigen in incomplete Freunds adjuvant (Sigma F 5506) and 10ug/animal CpG. Final boosts were administered two weeks later iv without any adjuvant and contained 5-10 ug protein in PBS.

### Hvbridoma fusion procedure

Spleens were taken 3 days after the final boost and spleenocytes were treated with CpG (25 µm final concentration) for and left until the following day. Cells were then fused with SPO/2 Ag14 myeloma cells (HPA Cultures Cat No 85072401) using a BTX ECM2001 electrofusion instrument. Fused cells were left to recover for 20 minutes then seeded in a T75 flask until next morning. Then the cells were spun down and plated out by dilution series on 96-well culture plates and left for about 10 days before screening. Media was changed 1-3 times during this period.

### Screening

### Screening

Culture supernatants of the hybridoma wells above were screened using homogenious time resolved fluorescence assay (htrf) using Europium cryptate labelled anti-mouse IgG (Cisbio anti-mouse Ig Europium Cryptate) and a biotin tagged target antigen with a commercially available streptavidin conjucated donor (Cisbio; streptaviding conjugated D2) or by IgG-specific 384 well ELISA. Positive wells identified by htrf were scaled to 24-well plates or immediately counterscreened using an IgG-specific detection ELISA method. Positives identified by primary ELISA screen were immediately expanded to 24-well plates. Once cultures were expanded to 24-well stage and reached conflueny, supernatants were re-tested using htrf or IgG-specific ELISA to confirm binding to target antigen. Supernatant of such confirmed cultures were then also analysed by surface plasmon resonance using a BioRad ProteOn XPR36 instrument. For this, antibody expressed in the hybridoma cultures was captured on a biosensor GLM chip (BioRad 176-512) which had an anti-mouse IgG (GE Healthcare BR-1008-38))covalently coupled the biosensor chip surface. The antigen was then used as the analyte and passed over the captured hybridoma antibody surface. For Antigen 2 and Antigen 3, concentrations of 256nM, 64nM, 16nM, 4nM and 1nM were typically used, for Antigen 1, concentrations of 1028nM, 256nM, 64nM, 16nM and 4nM were typically used, binding curves were double referenced using a OnM injection (i.e. buffer alone). Kinetics and overall affinities were determined using the 1:1 model inherent to the BioRad ProteOn XPR36 analysis software.

Any clones with confirmed binding activity were used for preparing total RNA and followed by PCR to recover the heavy chain variable region sequences. Standard 5'-RACE was carried out to analyse RNA transcripts from the transgenic heavy chain loci in the S2F and S3F mice. Additionally, deep sequence analysis of almost 2000 sequences produced by the mice was carried out.

### Bionformatics Analysis

Sequences for analysis were obtained from two different methods:
- The first is from RNA extracted from the spleen: first cDNA strand was synthesized using an oligo based on the Cmu region of the mouse IGH locus as a PCR template. PCR was performed using this oligo with an oligo dT-anchor primer. Then PCR product was cloned into pDrive vector (Qiagen) and then sequenced.
- The second is from hybridomas generated through electro-fusion: total RNA was extracted from hybridoma lines of interest using standard Trizol methods and frozen at -80 °C for long term storage. cDNA was generated from 100ng total RNA using standard Superscript III reverse transcriptase and a gene-specific reverse primer binding to all mouse IgG isotypes for heavy chain and a mouse kappa constant region primer for the light chain amplification. 2-3 ul of cDNA were then used as template in a PCR reaction using Pfu DNA polymerase and a panel of degenerate forward primers annealing to the leader sequence of the human immunoglobulin variable domain as well as one mouse pan-IgG reverse primer. PCR products were run out of a 1% agarose gel and bands of approximately 350-450 basepairs extracted and purified. DNA was then sequenced.

The sequences from the first method can either be from IgM from Naive mice or IgG from immunised mice. The samples from the second method are all from IgG from immunised mice, and specific to the immunising antigen. Almost 2000 sequences were analysed.

The sequences were obtained as a pair of forward and reverse reads. These were first trimmed to remove low-quality base calls from the ends of the reads (trimmed from both ends until a 19 nucleotide window had an average quality score of 25 or more). The reads were combined together by taking the reverse complement of the reverse read, and aligning it against the forward read. The alignment scoring was 5 for a match, -4 for a mismatch, a gap open penalty of 10 and a gap extension penalty of 1. A consensus sequence was then produced by stepping through the alignment and comparing bases. When there was a disagreement the base with the highest quality value from sequencing was used.

The BLAST+ (Basic Local Alignment Search Tool) (Camacho C., Coulouris G., Avagyan V., Ma N., Papadopoulos J., Bealer K., & Madden T.L. (2008) "BLAST+: architecture and applications." BMC Bioinformatics 10:421 http://www.ncbi.nlm.nih.gov/pubmed/20003500) program 'blastn' was then used to find the germline J and V segments used in each sequence. A wordsize of 30 was used for V matching, and 15 for J matching. The database searched against was constructed from the NGS sequencing of the BACs which were used to generate the Kymouse.

If a sequence matched both a V and a J segment, the sequence between the two was then compared to a database of germline D segments in the mouse using 'blastn' with a wordsize of 4 and the options 'blastn-short' and 'ungapped'. This was used to assign a D segment, if possible. The CDR3 was identified by searching for the conserved "TATTACTGT" sequence in the V segment, and the "CTGGGG" in the J segment. If these motifs were not found, then up to 4 mismatches were allowed. The IMGT definition of CDR3 was used, so the CDR3 length is calculated from after the "TGT" in the V to before the "TGG" in the J. Sequences with an out of frame junction (those which do not have a CDR3 nucleotide length divisible by 3) or which contained a stop codon ("TAA", "TAG" or "TGA") were excluded.

The identity of the matching V, J and D segments as well as the CDR3 length from this assignment were then saved as a table for downstream analysis. The ratio of IGHJ6*02 used increased from the naïve to immunised mice, as well as being enriched in the sub-population of sequences with a long HCDR3 (defined as consisting of 20 or more amino acids):

| | All | | HCDR3>20 | | % HCDR3>20 |
|---|---|---|---|---|---|
| | JH6*02% | Total Count | JH6*02% | Total Count | |
| Naïve | 22.31% | 1340 | 91.11% | 45 | 3.36% |
| Immunised | 37.50% | 256 | 66.67% | 9 | 3.52% |
| Hybridoma | 36.13% | 119 | 63.64% | 11 | 9.24% |

This shows that the JH6*02 gene segment is selected for by immunisation, as the proportion of JH6*02 usage increases after immunisation. JH6*02 is also used in the majority of antibodies with a long HCDR3 length, which is desirable for targets which are specifically bound by long HCDR3 length antibodies.

SEQ ID NO: 1 (JH5 Reference)
T T G A C C A A G C T G G G G A C C C C
G G T C C C T T G G G A C C A G T G G C
A G A G G A G T C

JH5 Alignment: (top line=SEQ ID NO: 1, Middle line=-SEQ ID NO:5, Bottom line=SEQ ID NO:6)

100.57 Hg

870-033 601
470-033 601 N
G C
A T
860-033 601 W
250-033 601
660-033 601
560-033 601 F
G C
260-033 601
360-033 601 D
G C
G C P
G C
A T W
G C
C G
C G G
G C
T A Q
C G
C G
C G G
T A
540-033 601
440-033 601 T
G C
G C
140-033 601 L
C G
C G
A T V
G C
T A
G C T
G C
C G
280-033 601 V
G C
A T
G C S
G C
G C
220-033 601
G C
T A S
420-033 601

SEQ ID NO: 2 (JH6 Reference)
A T G A T G A T G A T G A T G A T G T A
C C T G C A G A C C C C G T T T C C C T
G G T G C C A G T G G C A G A G G A G T
C

JH6 Alignment: (top line=SEQ ID NO: 2, Middle line=-SEQ ID NO:7, Bottom line=SEQ ID NO:8)

100.97 Hd

864 923 601 T G A T G A T G A T G A T G 354 923 601 254 923 601 154 923 601 T A C C T G C A G A C C C G 834 923 601 434 923 601 T

A C T A C T A C T A C T A C [?] [?] [?] A T G G A C G T C T G G G C [?] [?] A

Y Y Y Y Y Y M D V W G K

C
C
C
T
G
G
624 923 601
G
C
C
A
G
T
914 923 601
G
714 923 601
G
A
G
414 923 601
314 923 601
G
114 923 601
G
T
804 923 601

G G G A C C A C G G T C A C C G T C T C C T C A G
G     T     T     V     T     V     S     S

SEQ ID NO: 3 (JH2 Reference)

A T G A C C A T G A A G C T A G A G A C

C C C G G C A C C G T G G G A C C A G T

G A C A G A G G A G T C

JH2 Alignment: (top line=SEQ ID NO: 3, Middle line=-SEQ ID NO:9, Bottom line=SEQ ID NO:10)

100-31 H9

601 133 490 T G A C 601 133 5[?] A 601 133 3[?] G A A G C T A G A G A C C C G G C A C C G T G G A C C A G T G A C A G A G G A G T 601 133 3[?]
A C T G T C T T C G A T C T C T G G G C C G T G G C A C C T G G T C A C T G T C T C C T C A
Y W Y F D L W G R G T L V T V S S

**TABLES**

| | | | |
|---|---|---|---|
| In the tables, the notation is illustrated by the following example | | | |
| IGLV1-40 | V1-40*02 | X53936 | g9>c\|c10>g,L4>V\| |

Polymorphic variant IGV lambda V1-40*02 has Genbank Accession No. X53936 and when compared to the *01 variant, the V1-40*02 variant has mutations at positions 9, 10 and 4. For example, at position 9, a "C" appears instead of a "G" that is present in the *01 variant. The "|" is simply a notation separator, and does not indicate any mutation. For example the "g282 I" notation indicates no change (ie, position 282 is a g). "del#" means that the residue at that position is absent.

**Table 1: Human IgH V Polymorphic Variants**

| | | | | |
|---|---|---|---|---|
| All variant sequences appearing in Genbank under the listed Accession Numbers are incorporated herein in their entirety by reference as though explicitly recited herein and for possible inclusion in any of the claims herein. | | | | |
| | | | | |

| **subgroup** | **IGHV** | **IGHV** | **Accession number** | **Description of mutations** |
|---|---|---|---|---|
| 1 | IGHV1-2 | IGHV1-2*01 | X07448 | SEQ ID NO: 11 |
| | | IGHV1-2*02 | X62106 | \|c163>t,R55>W\| \|g233>t,t234>g,S78>M\|t299>c,V100>A\| |
| | | IGHV1-2*03 | X92208 | c44>t,P15>L\|c163>t,R55>W\| \|g233>t,t234>g,S78>M\|t299>c,V100>A \| |
| | | IGHV1-2*04 | Z12310 | \|c163>t,R55>W\|a223>t,R75>W\|g233>t,t234>g,S78>M\|t299>c,V100 >A\| |
| | IGHV1-3 | IGHV1-3*01 | X62109 | SEQ ID NO: 12 c6 \|t12 \|t167 ,I56 \|a208 ,K70 \|a291 \|c296 ,T99 \| |
| | | IGHV1-3*02 | X62107 | c6>t\|t12>g\|t167>g,I56>S \|a208>g,K70>E \|a291>g \| c296>t,T99>M \| |
| | IGHV1-8 | IGHV1-8*01 | M99637 | SEQ ID NO: 13 |
| | IGHV1-18 | IGHV1-18*01 | M99641 | g282 \| |
| | | IGHV1-18*02 | X60503 | g282>a\| |
| | IGHV1-24 | IGHV1-24*01 | M99642 | SEQ ID NO: 14 |
| | IGHV1-45 | IGHV1-45*01 | X92209 | SEQ ID NO: 15 g139 ,G47 \|t237 \| |
| | | IGHV1-45*02 | AB019438 | \|t237>c\| |
| | | IGHV1-45*03 | Z17391 | g139>a,G47>R \|t237>c \| |
| | IGHV1-46 | IGHV1-46*01 | X92343 | SEQ ID NO: 16 c92 ,T31 \|g315 \| |
| | | IGHV1-46*02 | J00240 | c92>a,T31>N \| |
| | | IGHV1-46*03 | L06612 | \|g315>t\| |
| | IGHV1-58 | IGHV1-58*01 | M29809 | SEQ ID NO: 17 g115 ,V39 \| |
| | | IGHV1-58*02 | AB019438 | g115>a,V39>M \| |
| | IGHV1-69 | IGHV1-69*01 | L22582 | SEQ ID NO: 18 |
| | | IGHV1-69*02 | Z27506 | g6>c\|g18>a\| \|g100>a,A34>T\| g163>a,G55>R\|t178>c,F60>L\|cl85>t,T62>I \| \|g244>a,E82>K\| |
| | | IGHV1-69*03 | X92340 | \|g291>t,E97>D\| |
| | | IGHV1-69*04 | M83132 | g6>c\| \|g163>a,G55>R\|t178>c,F60>L\|c185>t,T62>I \|\|g2 44>a, E82>K\| |
| | | IGHV1-69*05 | X67905 | g6>c\| \|g238>a,A80>T\| |
| | | IGHV1-69*06 | L22583 | \|g244>a,E82>K\| |
| | | IGHV1-69*07 | Z29978 | \|g163>a,G55>R\| |
| | | IGHV1-69*08 | Z14309 | g6>c\|g18>a\| \|g100>a,A34>T\|g163>a,G55>R\|t178>C,F60>L\| \|g244>a,E82>K\| |
| | | IGHV1-69*09 | Z14307 | \|g163>a,G55>R\|t178>c,F60>L\|c185>t,T62>I \| \|g244> a,E82>K\| |
| | | IGHV1-69*10 | Z14300 | g6>c\| \|g54>a\| \|t178>c,F60>L\|c185>t,T62>I \| \|g244>a ,E82>K\| |
| | | IGHV1-69*11 | Z14296 | g6>c\| \|g163>a,G55>R\|t178>c,F60>L\| |
| | | IGHV1-69*12 | Z14301 | g6>c |
| | | IGHV1-69*13 | Z14214(st) | g6>c\| \|g54>a\| |
| | IGHV1-c | IGHV1-c*01 | Z18904 | SEQ ID NO: 19 |
| | IGHV1-f | IGHV1-f*01 | Z12305 | SEQ ID NO: 20 c201 \| |
| | | IGHV1-f*02 | Z29977 | c201>t\| |
| 2 | IGHV2-5 | IGHV2-5*01 | X62111 | SEQ ID NO: 21 |
| | | IGHV2-5*02 | Z14072 | \|a175>g,N59>D\| |
| | | IGHV2-5*03 | X93619 | \|a175>g,N59>D\| \|c234>t\| |
| | | IGHV2-5*04 | L21963 | \|c299>g,A100>G\|c314>t,A105>V\|a317>g,c318>g,H106>R\| |
| | | IGHV2-5*05 | L21964 | \|a175>g,N59>D\|a202>g,S68>G\| |
| | | IGHV2-5*06 | L21966 | \|g39>a \| \|a175>g,N59>D\|a202>g,S68>G\| |
| | | IGHV2-5*07 | L21968 | \|c299>g,A100>G \|c314>t,A105>V \| |
| | | IGHV2-5*08 | L21971 | a4>g,I2>V\|a31>g,T11>A\| \|g60>a\|g103>a,V35>M\|g106>c,G36>R\|g118>a ,G40>S\|a175>g,N59>D\| |
| | | IGHV2-5*09 | L21972 | a4>g,I2>V\| \|a175>g,N59>D\|a202>g,S68> G\| |
| | | IGHV2-5*10 | X69690 | \|a175>g,N59>D\| \|a317>g,c318>g,H106>R\| |
| | IGHV2-26 | IGHV2-26*01 | M99648 | SEQ ID NO: 22 |
| | IGHV2-70 | IGHV2-70*01 | L21969 | SEQ ID NO: 23 22 a2 ,Q1 \|g4 ,V2 \|g14 ,R5 \|g31 ,A11 \|a60 \|t67 ,C23 \|a70 ,T24 \|t10 6 ,C36 \|t116 ,V39 \|a138 \|c157 ,L53 \|t164 ,L55 \|a197 ,Y66 \|t210 \|g2 16 ,K72 \|a297 \|c299 ,A100 \|a301 ,c302 ,T101 \|g303 \|c309 \|c314 ,A1 05 \|g317 ,g318 ,R106 \|t320 ,I107 \| |
| | | IGHV2-70*02 | X92241 | \|a297>g\| \|a301>g,c302>t,T101>V\| |
| | | IGHV2-70*03 | X92238 | \|g14>a,R5>K\| \|t106>c,C36>R\| \|t164>g,L55>R\|a197>t,Y66>F\| \|a297>g\| \|a301>g,c3 02>t,T101>V\| |
| | | IGHV2-70*04 | Z12330 | \|g14>a,R5>K\| t106>c,C36>R \|t164>g,L55>R\|a197>t,Y66>F\| |
| | | IGHV2-70*05 | Z27502 | \|t106>c,C36>R\|t116>c,V39>A\| \|t164>g,L55>R\|a197>t,Y66>F\| |
| | | IGHV2-70*06 | X92239 | \|g14>a,R5>K\|\|t106>c,C36>R\| \|t164>g,L55>R\|a197>t,Y66>F\|t210>c\| \|a297>g\| \|a301> g,c302>t,T101>V\| |
| | | IGHV2-70*07 | X92243 | \|a138>g\| \|a297>g\| \|a301>g,c302>t,T101>V\| |
| | | IGHV2-70*08 | X92245 | \|a70>g,T24>A\| \|t 164>g,L55>R\| \|a297>g\| \|a301>g,c302>t,T101>V\| |
| | | IGHV2-70*09 | L21962 | \|g4>a,V2>I\|g14>a,R5>K\|g31>a,A11>T\|a60>g\|t67>c,C23>R\| \|g216>c,K72>N\| \|c299>g,A100> G\| \|g303>a \| \|c314>t,A105>V\| |
| | | IGHV2-70*10 | L21965 | \|g14>a,R5>K\| \|t106>c,C36>R\| \| c157>a,L53>I\|t164>g,L55>R\| |
| | | IGHV2-70*11 | L21967 | a2>g,Q1>R\| \|t1 64>g,L55>R\| |
| | | IGHV2-70*12 | L21970 | \|g4>a,V2>I\|g14>a,R5>K\|g31>a,A11>T\|a60>g\| \|g303>a\| \|g317a,g318>c,R106>H\|t320>g,I107>R\| |
| | | IGHV2-70*13 | AB019437 | \|c309>t\| |
| 3 | IGHV3-7 | IGHV3-7*01 | M99649 | SEQ ID NO: 24 |
| | | IGHV3-7*02 | X92288 | g144>a\| |
| | IGHV3-9 | IGHV3-9*01 | M99651 | SEQ ID NO: 25 |
| | IGH3-11 | IGHV3-11*01 | M99652 | SEQ ID NO: 26 \|g13 ,V5 \|g32 ,G11 \|g47 ,G16 \|g178 ,G60 \|a184 ,c185 ,T62 \|t 188 ,I63 \|t196 ,Y66 \|a206 ,D69 \|g240 \|c243 ,D81 \|c296 ,g297, T99 \|g301 ,g303 ,V101 \|t304 ,a305 ,Y102 \|t307 ,a308 ,c309 ,Y103 \|t 312 \|c314 ,A105 \| |
| | | IGHV3-11*02 | M15496 | 9^10>ins^t\| \| g32>del#,G11>del#\|g47>del#,G16>del#\| \|a206>del#,D69>del#\| \|c243>g,D81>E\|c296>del#,g297 >del#,T99>del#\|g301>t,g303>a,V101>L\|t304>c,a305>t,Y102>L\|t307>a,a30 8>c,c309>t,Y103>T\|t312>c\|c314>a,A105>E\| |
| | | IGHV3-11*03 | X92287 | \|g13>t,V5>L\| \|g178>a,G60>S\|a184>t,c185>a,T62> Y\|t188>c,I63>T\|t196>a,Y66>N\| \|g240>a\| |
| | IGHV3-13 | IGHV3-13*01 | X92217 | SEQ ID NO: 27 g9 ,Q3 \|t52 ,S18 \|g77 ,S26 \|g95 ,S32 \|t165 \|t167 ,I56 \|c222 \|g224, R75 \| |
| | | IGHV3-13*02 | M99653 | g9>t,Q3>H \|t52>g,S18>A\| \|g95>a,S32>N\|t165>c\|t167>a,I56>N\|c222> g\| |
| | | IGHV3-13*03 | U29582 | \|c77>g,S26>C\| \|g224>a, R75>Q\| |
| | IGHV3-15 | IGHV3-15*01 | X92216 | SEQ ID NO: 28 g23 ,g24 ,G8 \|g32 ,G11 \|a40 ,K14 \|a81 \|t89 ,F30 \|g119 ,S40 \|t159 \| c163 ,R55 \|a169 ,K57 \|a178 ,T60 \|g181 ,D61 \|g196 ,D66 \|c210 \|a242 ,D81 \|a275 ,N92 \|c279 \|a297 \|a320 \| |
| | | IGHV3-15*02 | M99654 | \|g32>c,G11>A\| |
| | | IGHV3-15*03 | M99408 | g23>c,g24>c,G8>A\|g32>c,G11>A\|a40>c,K14>Q\| \|t89>g,F30>C \|a178>g,T60>A\|g181>a,061>N\| \|c210>t\|a242>t,D81 >V\| |
| | | IGHV3-15*04 | M99402 | \|a169>g,K57>E\| |
| | | IGHV3-15*05 | M99403 | \|c279>t \| |
| | | IGHV3-15*06 | M99404 | \|t159>c \| \|g196>a,D66>N\| |
| | | IGHV3-15*07 | M99406 | \|a81>t\| \|g119>a,S40>N\|t159>c\| |
| | | IGHV3-15*08 | M99400 | g23>c ,G8>A\| \|a40>c,K14>Q\| \|t89>g,F30>C\| \|c163>t, R55>C\| \|a178>g,T60>A\|g181>a,D61>N\| \|c210>t\| \|a275 >t,N92>I\| \|a297>g\|a320>g\| |
| | IGHV3-16 | IGHV3-16*01 | M99655 | SEQ ID NO: 29 a6 \|a9 \| |
| | | IGHV3-16*02 | AB019440 | a6>g\|a9>g\| |
| | IGHV3-19 | IGHV3-19*01 | M99656 | SEQ ID NO: 30 |
| | IGHV3-20 | IGHV3-20*01 | M99657 | SEQ ID NO: 31 |
| | IGHV3-21 | IGHV3-21*01 | AB019439 | SEQ ID NO: 32 g9 I |
| | | IGHV3-21*02 | M99658 | g9>a\| |
| | IGHV3-23 | IGHV3-23*01 | M99660 | SEQ ID NO: 33 c164 ,A55 \|a169 ,g170 ,S57 \|g172 ,t174 ,G58 \|a175 ,S59 \|g181 ,G61 \|c201 \|e203 ,A68 \|c237 \|c243 \| |
| | | IGHV3-23*02 | M35415 | \|c203>g,A68>G \|c237>a\| |
| | | IGHV3-23*03 | U29481 | c164>t,A55>V\|a169>t,g170>a,S57>Y\|g172>a,t174>c,G58>S\|a175>g,S59>G \|g181>a,G61>S\|c201>t\|c243>t |
| | | IGHV3-23*04 | AJ879486 | t13>g\| |
| | | IGHV3-23*05 | AY757302 | a154>t, g155>a, S>Y\|g157>a, t159>a, G>S\|g166>a, G>S\| |
| | IGHV3-30 | IGHV3-30*01 | M83134 | SEQ ID NO: 34 g18 ,E6 \|a27 \|a49 ,R17 \|c75 \|g80 ,G27 \|c101 ,t102 ,A34 \|t135 \|a138 \|a150 \|g163 ,V55 \|t169 ,c170 ,a171 ,S57 \|c201 \|g202 ,A68 \|a229 ,T 77 \|c257 ,T86 \|g267 \|a275 ,N92 \|t288 \|a293 ,D98 \|g317 ,R106 \| |
| | | IGHV3-30*02 | L26401 | \|a49>g,R17>G \|c75>g\| \|c101>g,t102>c,A34>G\| \|a150 >g\|g163>t,V55>F\|t169>c,c170>g,a171>g,S57>R\|c201>t\| \|g317>a,R106>K\| |
| | | IGHV3-30*03 | M99663 | \|c101>g,t102>c,A34>G\| \|a150>g\| \|c201>t\| |
| | | IGHV3-30*04 | L06615 | \|a150>g\| |
| | | IGHV3-30*05 | M77323 | \|c101>g,t102>c,A34>G\| \|a293>g,D98>G\| |
| | | IGHV3-30*06 | L06617 | \|c75>g\| \|c101>c,t102>c,A34>G\| |
| | | IGHV3-30*07 | L06614 | \|t288>c\| |
| | | IGHV3-30*08 | M62737 | g18>c,E6>D\| \|g80>c,G27>A\| |
| | | IGHV3-30*09 | M77300 | \|a150>g\| \|a229>g,T77>A\| |
| | | IGHV3-30*10 | M77326 | \| g202>a,A68>T\| |
| | | IGHV3-30*11 | M77331 | \|c75>g\| |
| | | IGHV3-30*12 | M77338 | \|a27>g\| \|c75>g\| \|c101>g,t102>c,A34>G\| \|t288>c\| |
| | | IGHV3-30*13 | M77339 | \|c101>g,t102>c,A34>G\| \|c257>g,T86>R\| |
| | | IGHV3-30*14 | M77324 | \|a150>g\| \|g267>t\| |
| | | IGHV3-30*15 | M77327 | \|a275>g,N92>S\| |
| | | IGHV3-30*16 | M77328 | \|t135>c\| |
| | | IGHV3-30*17 | M77329 | \|a138>g\| |
| | | IGHV3-30*18 | X92214 | \|c101>g,t102>g,A34>G\| \|a150>g\| \|c201>t\| \|g317>a,R 106>K\| |
| | | IGHV3-30*19 | L06616 | \|c75>g\| \|c101>g,t102>g,A34>G\| \|a150>g\| |
| | IGHV3-30-3 | IGHV3-30-3*01 | X92283 | SEQ ID NO: 35 c75 \|g317 ,R106\| |
| | | IGHV3-30-3*02 | M77302 | c75>g\|g317>a,R106>K\| |
| | IGHV3-33 | IGHV3-33*01 | AB019439 | SEQ ID NO: 36 g6 \|g150 \|g170 ,g171 ,W57 \|t177 \|t212 ,V71 \|t246 \|a251 ,K84 \|a263 ,Y88 \|g317 ,R106 \| |
| | | IGHV3-33*02 | M99665 | g6>a\| \|t212>c,V71>A\| \|a251>c,K84>T\|a263>t,Y88>F\| |
| | | IGHV3-33*03 | M77305 | \|t246>c\| \|g317>a,R106>K\| |
| | | IGHV3-33*04 | M77335 | \|g150>a\| \|t177>c\| |
| | | IGHV3-33*05 | M77334 | \|g170>c,g171>a,W57>S\| |
| | IGHV3-35 | IGHV3-35*01 | M99666 | SEQ ID NO: 37 |
| | IGHV3-38 | IGHV3-38*01 | M99669 | SEQ ID NO: 38 c302 ,A101 \| |
| | | IGHV3-38*02 | AB019439 | c302>t,A101>V\| |
| | IGHV3-43 | IGHV3-43*01 | M99672 | SEQ ID NO: 39 t32 ,V11 \|a100 ,T34 \|g138 \|t172 ,W58\| |
| | | IGHV3-43*02 | Z18901 | t32>g,V11>G\|a100>g,T34>A\|g138>a\|t172>g,W58>G\| |
| | IGHV3-47 | IGHV3-47*01 | Z18900 | SEQ ID NO: 40 c58 \|c101 ,A34 \|t149 ,L50 \|t267 ,L89 \|t270 ,H90 \|t310\| |
| | | IGHV3-47*02 | AB019438 | c58>a\|c101>t,A34>V\|t149>c,L50>P\| \|t270>a, H90>Q\| |
| | | IGHV3-47*03 | M99674 | c58>a\|c101>t,A34>V\|t149>c,L50>P\|t267>del#,L89>del#\|t270>a, H90>Q\|t3 10>g\| |
| | IGHV3-48 | IGHV3-48*01 | M99675 | SEQ ID NO: 41 g48 \|c96 \|a100 ,g101 ,c102 ,S34 \|a178 ,S60 \|t246 \|c287 ,A96 \|g303 I |
| | | IGHV3-48*02 | AB019438 | \|c287>a,A96>D\| |
| | | IGHV3-48*03 | U03893 | g48>a\|c96>t\|a100>g,g101>a,c102>a,S34>E\|a178>g,S60>G\|t246>c\| \|g303>t\| |
| | IGHV3-49 | IGHV3-49*01 | M99676 | SEQ ID NO: 42 g50 ,R17 \|t93 \|g94 ,D32 \|g100 ,A34 \|t124 ,F42 \|a202 ,T68 \|g245 ,G8 2 I |
| | | IGHV3-49*02 | M99401 | g50>c,R17>P\|t93>g\|g94>t,D32>Y\|g100>c,A34>P\|t124>g,F42>V\|a202>g,T6 8>A\|g245>a,G82>D\| |
| | | IGHV3-49^{*}03 | AB019438 | \|a202>g,T68>A\|g245>a,G82>D\| |
| | | IGHV3-49*04 | AM940220 | \|t124>g ,F42>V \|a202>g,T68>A\|g245>a,G82>D\| |
| | | IGHV3-49*05 | AM940221 | \|a202>g,T68>A\|g245>a,G82>D\| |
| | IGHV3-53 | IGHV3-53*01 | M99679 | t19 ,S7 \|g133 ,A45 \|c210 \|g315 \|a318\| |
| | | IGHV3-53*02 | Z12342 | SEQ ID NO: 43 t19>a,S7>T\| |
| | | IGHV3-53*03 | J03617 | \|g133>c,A45>P\|c210>t\|g315>t\|a318>g\| |
| | IGHV3-64 | IGHV3-64*01 | M99682 | SEQ ID NO: 44 gl ,E1 \|g26 ,G9 \|g70 ,A24 \|t198 \|t201 \|a205 ,N69 \|t210 \|c265 ,t267 ,L89 \|g274 ,g275 ,G92 \|c279 \|t296 ,M99 \|c314 ,A105 \|g317 ,R106 \| |
| | | IGHV3-64*02 | AB019437 | \|g26>a,G9>E\| \|a205>g,N69>D\| |
| | | IGHV3-64*03 | M77298 | \|g70>t,A24>S\|t198>c\|t201>c\|a205>g,N69>D\|t210>a\|c265>g,t2 67>c,L89>V\|g274>a ,G92>S\|c279>t\|t296>c,M99>T\|c314>t,A105>V\|g31 7>a,R106>K\| |
| | | IGHV3-64*04 | M77299 | g1>c,E1>Q\| \|g70>t,A24>S\|t198>c\|t201>c\|a205>g,N69>D\|t210>a\| ,t267>g, \|g274>a,g275>a,G92>N\|t296>c,M99>T\| |
| | | IGHV3-64*05 | M77301 | \|g70>t,A24>S\|t198>c\|t201>c\|a205>g,N69>D\|t210>a\|c265>g, ,L89>V\|g274>a ,G92>S\|c279>t\|t296>c,M99>T\|c314>t,A105>V\|g317> a,R106>K\| |
| | IGHV3-66 | IGHV3-66*01 | X92218 | SEQ ID NO: 45 g24 \|g37 ,V13 \|a81 \|g175 ,G59 \|a223 \|c288 \|g319\| |
| | | IGHV3-66*02 | Z27504 | \|a223>c\|c288>t\| |
| | | IGHV3-66*03 | AB019437 | g24>a\|g37>a,V13>l\|a81>g\|g175>t,G59>C\|a223>c\|c288>t\| |
| | | IGHV3-66*04 | X70208 | \|g319>c\| |
| | IGHV3-72 | IGHV3-72*01 | X92206 | SEQ ID NO: 46 t186 \| |
| | | IGHV3-72*02 | Z29979 | t186>c\| |
| | IGHV3-73 | IGHV3-73*01 | X70197 | SEQ ID NO: 47 t21 \| |
| | | IGHV3-73*02 | AB019437 | t21>c\| |
| | IGHV3-74 | IGHV3-74*01 | L33851 | SEQ ID NO: 48 c21 \|g197 ,c198 ,S66\| |
| | | IGHV3-74*02 | Z17392 | c21>t\| |
| | | IGHV3-74*03 | J00239 | \|g197>c,c198>g,S66>T\| |
| | IGHV3-d | IGHV3-d*01 | Z18898 | SEQ ID NO: 49 |
| | IGHV3-h | IGHV3-h*01 | AJ879484 | SEQ ID NO: 50 |
| | | IGHV3-h*02 | AJ879485 | \|g303>t\| |
| 4 | IGHV4-4 | IGHV4-4*01 | X05713 | SEQ ID NO: 51 |
| | | IGHV4-4*02 | X92232 | \|c46>t,P16>S\| \|g308>a,C103 >Y\| |
| | | IGHV4-4*03 | X92252 | \|g308>a,C103>Y\| |
| | | IGHV4-4*04 | X92253 | \|g73>a,V25>l\| \|g308>a, C103>Y\| |
| | | IGHV4-4*05 | X92254 | c20>t,S7>L\| \|g308>a,C103>Y\| |
| | | IGHV4-4*06 | Z75355 | \|a234>g,178>M\|a245>c,K82>T \|g308>a,C103>Y\| |
| | | IGHV4-4*07 | X62112 | \|c46>t,P16>S\|g50>a,G17>E\|g70>a,A24>T\| \|c93>t\|a97>t,g98>a, t99>c,S33>Y\|a100>t,N34>Y\|t120>c\|g124>a,V42>1\|c129>g\|c136>g,a138>c, P46>A\|g147>a\|g163>c,a164>g,a165>t,E55>R\|c172>a,a173>c,t174>c,H58> T\|g207>c\|a234>g,178>M \|a245>c,K82>T\|g308>a,C103>Y\| \|a234>g,178>M \|a245>c,K82>T\|g308>a,C103>Y\| |
| | IGHV4-28 | IGHV4-28*01 | X05714 | SEQ ID NO: 52 g48 \|g49 ,c51 ,D17 \|c185 ,T62 \|g297 \|c299 ,A100 \|a319 \| |
| | | IGHV4-28*02 | M83133 | g48>a\|g49>c,c51>g,D17>Q\|c185>t,T62>1\| |
| | | IGHV4-28*03 | X92233 | \|a319>g\| |
| | | IGHV4-28*04 | X56358 | \|g297>c\|c299>g,A100>G\| |
| | | IGHV4-28*05 | X92260 | \|c185>t,T62>l\| |
| | IGHV4-30-2 | IGHV4-30-2*01 | L10089 | SEQ ID NO: 53 t163 ,a164 ,c165 ,Y55 \|c172 ,H58 \|a237 \|g245 ,R82 \|c288 \|g291 \|c30 0 \|c315 \|g319 \| |
| | | IGHV4-30-2*02 | M95122 | \|c288>t\| \|c315>g\| |
| | | IGHV4-30-2*03 | X92229 | t163>a,a164>g,c165>t,Y55>S\|c172>t,H58>Y\|a237>c\|g245>c,R82>T\|c288>t \|g291>a\|c300>t\|c315>g\|g319>c\| |
| | | IGHV4-30-2*04 | Z75351 | \|g245>c,R82>T\| \|g291>a\| \|c315>g\| |
| | IGHV4-30-4 | IGHV4-30-4*01 | Z14238 | SEQ ID NO: 54 g18 ,E6 \|a48 \|c49 ,g51 ,Q17 \|c134 ,P45 \|a166 ,156 \|t228 \|c288 \|a29 1 I |
| | | IGHV4-30-4*02 | Z14239 | \|a48>g\|c49>g,g51>c,Q17>D\| \|c288>a\| |
| | | IGHV4-30-4*03 | X92274 | \|a291>g\| |
| | | IGHV4-30-4*04 | X92275 | g18>c,E6>D\| \|a166>t,I56>F\| |
| | | IGHV4-30-4*05 | Z75353 | \|t228>c\| |
| | | IGHV4-30-4*06 | Z75360 | \|c134>a,P45>H\| |
| | IGHV4-31 | IGHV4-31*01 | L10098 | SEQ ID NO: 55 a8 ,Q3 \|g37 ,V13 \|c69 \|c84 \|g100 ,G34 \|a164 ,Y55 \|t224 ,L75 \|a237 \|a244 ,c245 ,T82 \|t249 \|t285 \|t285-c287 \|a295 ,T99 \|t304 ,Y102 \| |
| | | IGHV4-31*02 | M99683 | \|c69>t\| \|t224>g,L75>R\| |
| | | IGHV4-31*03 | Z14237 | \|t224>g,L75>R\| |
| | | IGHV4-31*04 | M95120 | a8>g,Q3>R\| \|t224>g,L75>R\| |
| | | IGHV4-31*05 | M95121 | \|t224>g,L75>R\| \|t28 5-c287>del(3nt)#\|a295>g,T99>A\| |
| | | IGHV4-31*06 | X92270 | \|g100>a,G34>S\| \|t224>g,L75>R\| |
| | | IGHV4-31*07 | X92271 | \|c84>a\| \|t224>g,L75>R\| |
| | | IGHV4-31*08 | X92272 | \|t224>g,L75>R\|a237>c\| \|t249>c \| |
| | | IGHV4-31*09 | X92273 | \|t224>g,L75>R\| \|,c245>a,T82>K\|t249>c\|t285>c\| |
| | | IGHV4-31*10 | Z14235 | \|g37>t,V13>L\| \|a164>g,Y55>C\|t224>g,L75>R\| \|a244> c, ,T82>P\|t249>c\| \|t304>g,Y102>D\| |
| | IGHV4-34 | IGHV4-34*01 | AB019439 | SEQ ID NO: 56 a12 \|g15 \|c16 ,Q6 \|g20 ,W7 \|g25 ,A9 \|t37 ,L13 \|g48 \|g49 ,E17 \|t85 ,S29 \|t88 ,F30 \|a118 ,S40 \|c129 \|c137 ,P46 \|g147 \|g163 ,a165 ,E55 \|a169 ,a170 ,t171 ,N57 \|c172 ,H58 \|a180 \|t199 ,Y67 \|g207 \|g226 ,t2 27 ,c228 ,V76 \|a234 ,178 \|c249 \|c263 ,S88 \|g270 ,K90 \|a274 ,S92 \|c2 85 \|t300 \|a308 ,Y103\| |
| | | IGHV4-34*02 | M99684 | \|g15>a\| |
| | | IGHV4-34*03 | X92255 | \|t300>c\| |
| | | IGHV4-34*04 | X92236 | \|t199>a,Y67>N\| \|,t227>c , V76>A\| |
| | | IGHV4-34*05 | X92237 | \|a118>t,S40>C\| \| c137>t,P46>L\| \|t199>a,Y67>N\| \|,t227>c ,V76>A\| |
| | | IGHV4-34*06 | X92256 | \|a274>g,S92>G\| \|t300>c\| |
| | | IGHV4-34*07 | X92258 | \| ,t171>c\| \|t300>c\| |
| | | IGHV4-34*08 | M95113 | \|t85>a,S29>T\| |
| | | IGHV4-34*09 | Z14241 | a12>g\| \|c16>g,Q6>E\|g20>c,W7>S\|g25>c,A9>P\|t37>g,L13>V\|g48>a\|g49 >c,E17>Q\| \|g147>a\| \|c228>t\| \|c249>t\| \|c285>t\|t300>c\| |
| | | IGHV4-34*10 | Z14242 | a12>g\| \|c16>g,Q6>E\|g20>c,W7>S\|g25>c,A9>P\|t37>g,L13>V\| \|g147>a\| \|g226>a, ,V76>l\|a234>g,178>M \| \|c263>a,S88>Y\| \|t300>c\| |
| | | IGHV4-34*11 | X05716 | \|t88>g,F30>V\| \|c129 >g\| \|g163>t,a165>t,E55>Y\|a169>t ,N57>Y\|c172>t,H58>Y\| a180>g\|t199>a,Y67>N\|g207>c\|t227>c ,V76>A\| \|g 270>c, K90>N \| \|t300>c\|a308>g,Y103>C\| |
| | | IGHV4-34*12 | X56591 | \| a170>t ,N57>l\| |
| | | IGHV4-34*13 | Z75356 | \|g207>c\| |
| | IGHV4-39 | IGHV4-39*01 | AB019439 | SEQ ID NO: 57 a2 ,Q1 \|t56 ,L19 \|c237 \|g258 ,Q86 \|t262 ,S88 \|a291 \|t300 \|c319 \| |
| | | IGHV4-39*02 | X05715 | \|g258>c,Q86>H\|\|c319>g\| |
| | | IGHV4-39*03 | X92259 | \|t300>c\| |
| | | IGHV4-39*04 | X92297 | \|a291>g\| |
| | | IGHV4-39*05 | M95116 | \|t56>c,L19>P\| |
| | | IGHV4-39*06 | Z14236 | a2>g,Q1>R\| \|c237>a\| \|t262>c,S88>P\|a291>g\|t300>c\| |
| | | IGHV4-39*07 | AM940222 | \|c237>a\| \|a291>g\|t300>c\| c319>g |
| | IGHV4-55 | IGHV4-55*01 | M99685 | SEQ ID NO: 58 c44 ,P15 \|c237 \|a251 ,K84 \|c255 ,N85 \|a263 ,Y88 \|c288 \|c290 ,A97 \|g291 \|t319 \| |
| | | IGHV4-55*02 | X92223 | \|c237>a\| |
| | | IGHV4-55*03 | X92263 | \|c237>a\| \|a263>c,Y88>S\| |
| | | IGHV4-55*04 | X92265 | c44>t,P15>L\|c237>a\| |
| | | IGHV4-55*05 | X92266 | c44>t,P15>L\| |
| | | IGHV4-55*06 | X92267 | \|c255>g,N85>K\| \|c288>t\| |
| | | IGHV4-55*07 | X92268 | \|a251>g,K84>R\| \|a263>c,Y88>S\| \|g291>a\| |
| | | IGHV4-55*08 | X92234 | \|c237>a\| \|t319>g\| |
| | | IGHV4-55*09 | X92235 | \|a263>c,Y88>S\| \|c290>t,A97>V\| \|t319>a\| |
| | IGHV4-59 | IGHV4-59*01 | AB019438 | SEQ ID NO: 59 g12 \|g16 ,E6 \|c20 ,S7 \|c25 ,P9 \|g37 \|g51 ,E17 \|a70 ,T24 \|t76 ,c77 , S26 \|a88 ,130 \|c135 \|c136 ,a138 ,P46 \|a147 \|t163 ,a164 ,t165 ,Y55 \| t172 ,a173 ,c174 ,Y58 \|a196 ,N66 \|c207 \|c228 \|a234 ,178 \|a237 \|c24 9 \|g258 \|c285 \|t288 \|g291 \|c300 \|g319 \|a320 \| |
| | | IGHV4-59*02 | M29812 | \|a88>g,l30>V\| |
| | | IGHV4-59*03 | M95114 | \|g258>a\| |
| | | IGHV4-59*04 | M95117 | \|,c174>t \|a196>t,N66>Y\|c207>g\| \|a234>g,178>M \| \|t288>c\|g291>a\|c300>t\| |
| | | IGHV4-59*05 | M95118 | \|c135>g\| ,a138>g \| \|t163>c,a164>g, ,Y55>R\| ,c174>t \|a196>t,N66>Y\|c207> g\| \|a237>c\| \|t288>c\|g291>a\|c300>t\| |
| | | IGHV4-59*06 | M95119 | \|t76>a ,S26>T\| \|c136>g, a138>t,P46>A\|a147>c\| ,t165>c, \| \|a196>t,N66>Y\|c2 07>g\|c228>t\| \|c249>t\| \|c285>t\|t288>c \| |
| | | IGHV4-59*07 | X56360 | \|g51>c,E17>D\| |
| | | IGHV4-59*08 | X87091 | \|t288>c\| g291>a\| |
| | | IGHV4-59*09 | Z75359 | \|a320>g\| |
| | | IGHV4-59*10 | Z14243 | g12>a\|g16>c,E6>Q\|c20>g,S7>W\|c25>g,P9>A\|g37>t\| \|a70>g,T24>A\| ,c77>a,S26>Y\| \|c136>g,a138>c,P46>A\|a147>g\|t163>c,a164>g, ,Y55>R\|t172>a,a173>c, Y58>T\| \|a234>g,178>M \| \|t288>c \| \|g319>t\| |
| | IGHV4-61 | IGHV4-61*01 | M29811 | SEQ ID NO: 60 g4 ,V2 \|g48 \|g49 ,E17 \|g88 ,V30 \|g97 ,G33 \|a100 ,S34 \|a118 ,S40 \|c 136 ,a138 ,P46 \|g162 \|t163 ,a164 ,Y55 \|t172 ,a173 ,Y58 \|c245 ,T82 \| g258 ,Q86 \|t288 \|g289-g291 \|g291 \|g315 \|g319 \| |
| | | IGHV4-61*02 | L10097 | \|g48>a\|g49>c,E17>Q\|g88>a,V30>l\| \|c136>g,a138 >c, P46>A\|t163>c,a164>g,Y55>R\|t172>a,a173>c,Y58>T\| t288>c\| \|g291>a\| |
| | | IGHV4-61*03 | X92230 | \|g258>c,Q86>H\| |
| | | IGHV4-61*04 | X92250 | \|g162>a\| \|g289-g291>del(3nt)\| |
| | | IGHV4-61*05 | X56356 | g4>c,V2>L\| \|g88>a,V30>I \|g97>a,G33>S\| \|a118>g,S40>G\| \|c245>a,T82>K\| \|t288>c\| |
| | | IGHV4-61*06 | Z75347 | \|t288>c\| \|g315>c\| |
| | | IGHV4-61*07 | Z75348 | \|g319>c\| |
| | | IGHV4-61*08 | AB019437 | \|a100>g,S34>G\| |
| | IGHV4-b | IGHV4-b*01 | Z12367 | SEQ ID NO:61 |
| | | IGHV4-b*02 | X56365 | g70>a,A24>T\| |
| 5 | IGHV5-51 | IGHV5-51*01 | M99686 | SEQ ID NO: 62 |
| | | IGHV5-51*02 | M18806 | \|t116>c,I39>T\| \|c148>t\| |
| | | IGHV5-51*03 | X56368 | c45>g\| |
| | | IGHV5-51*04 | X56367 | c45>g\| \|t247>c,S83>P\| |
| | | IGHV5-51*05 | Z27449 | \|g139>a,G47>R\| |
| | IGHV5-a | IGHV5-a*01 | X92227 | SEQ ID NO: 63 t21 \|c148 \|c225 ,H75 \|c288 ,A96\| |
| | | IGHV5-a*02 | X92279 | \|c148>t\| \|c288>del#,A96>del#\| |
| | | IGHV5-a*03 | X56375 | t21>c\| |
| | | IGHV5-a*04 | X56376 | \|c225>g,H75>Q\| |
| 6 | IGHV6-1 | IGHV6-1*01 | X92224/J04 097 | SEQ ID NO: 64 |
| | | IGHV6-1*02 | Z14223 | a27>g\| |
| 7 | IGHV7-4-1 | IGHV7-4-1*01 | L10057 | SEQ ID NO: 65 |
| | | IGHV7-4-1*02 | X62110 | t274>a,C92>S\| |
| | | IGHV7-4-1*03 | X92290 | t274>a,C92>S\|g278>c,c279>g,S93>T\| |
| | IGHV7-81 | IGHV7-81*01 | AB019437 | SEQ ID NO: 66 |

**Table 2: Human IgHD Polymorphic Variants**

| All variant sequences appearing in Genbank under the listed Accession Numbers are incorporated herein in their entirety by reference as though explicitly recited herein and for possible inclusion in any of the claims herein. | | | | |
|---|---|---|---|---|
| **IGHD subgro up** | **IGHD name** | **Variant name** | **Genbank Accession number** | **Sequence** |
| 1 | **IGHD1-1** | IGHD1-1*01 | X97051 | SEQ ID NO: 67 |
| | **IGHD1-7** | IGHD1-7*01 | X13972 | SEQ ID NO: 68 |
| | **IGHD1-14** | IGHD1-14*01 | X13972 | SEQ ID NO: 69 |
| | **IGHD1-20** | IGHD1-20*01 | X97051 | SEQ ID NO: 70 |
| | **IGHD1-26** | IGHD1-26*01 | X97051 | SEQ ID NO: 71 |
| 2 | **IGHD2-2** | IGHD2-2*01 | J00232 | SEQ ID NO: 72 |
| | | | | AGGATATTGTAGTAGTACCAGCTGCTATGCC |
| | | IGHD2-2*02 | X97051 | G>A |
| | | IGHD2-2*03 | M35648 | A>T |
| | **IGHD2-8** | IGHD2-8*01 | X13972 | SEQ ID NO: 73 |
| | | | | AGGATATTGTACTAATGGTGTATGCTATACC |
| | | IGHD2-8*02 | J00233 | AA>GG |
| | **IGHD2-15** | IGHD2-15*01 | J00234 | SEQ ID NO: 74 |
| | **IGHD2-21** | IGHD2-21*01 | J00235 | SEQ ID NO: 75 |
| | | | | AGCATATTGTGGTGGTGATTGCTATTCC |
| | | IGHD2-21*02 | X97051 | T>C |
| 3 | **IGHD3-3** | IGHD3-3*01 | X13972 | SEQ ID NO: 76 |
| | | | | GTATTACGATTTTTGGAGTGGTTATTATACC |
| | | IGHD3-3*02 | X93618 | CG>GC |
| | **IGHD3-9** | IGHD3-9*01 | X13972 | SEQ ID NO: 77 |
| | **IGHD3-10** | IGHD3-10*01 | X13972 | SEQ ID NO: 78 |
| | | | | GTATTACTATGGTTCGGGGAGTTATTATAAC |
| | | IGHD3-10*02 | X93615 | G>[OMITTED] |
| | **IGHD3-16** | IGHD3-16*01 | X93614 | SEQ ID NO: 79 |
| | | | | GTATTATGATTACGTTTGGGGGAGTTATGCTTATACC |
| | | IGHD3-16*02 | X97051 | GC>CG |
| | **IGHD3-22** | IGHD3-22*01 | X93616 | SEQ ID NO: 80 |
| 4 | **IGHD4-4** | IGHD4-4*01 | X13972 | SEQ ID NO: 81 |
| | **IGHD4-11** | D4-11*01 | X13972 | SEQ ID NO: 82 |
| | **IGHD4-17** | IGHD4-17*01 | X97051 | SEQ ID NO: 83 |
| | **IGHD4-23** | IGHD4-23*01 | X97051 | SEQ ID NO: 84 |
| 5 | **IGHD5-5** | IGHD5-5*01 | X13972 | SEQ ID NO: 85 |
| | **IGHD5-12** | IGHD5-12*01 | X13972 | SEQ ID NO: 86 |
| | **IGHD5-18** | IGHD5-18*01 | X97051</A<< B> | SEQ ID NO: 87 |
| | **IGHD5-24** | IGHD5-24*01 | X97051 | SEQ ID NO: 88 |
| 6 | **IGHD6-6** | IGHD6-6*01 | X13972 | SEQ ID NO: 89 |
| | **IGHD6-13** | IGHD6-13*01 | X13972 | SEQ ID NO: 90 |
| | **IGHD6-19** | IGHD6-19*01 | X97051 | SEQ ID NO: 91 |
| | **IGHD6-25** | IGHD6-25*01 | X97051 | SEQ ID NO: 92 |
| 7 | **IGHD7-27** | IGHD7-27*01 | J00256 | SEQ ID NO: 93 |

Underlined nucleotides in the *01 variant are positions that are different in other variants, eg, GC>CG for IGHD3-16*02 indicates that GC in the *01 variant is instead CG in the *02 variant. All other positions are identical to between the *01 and other variant. Similar notation is used in other tables below to denote changes between variants of gene segments.

**Table 3: Human IgH J Polymorphic Variants**

| All variant sequences appearing in Genbank under the listed Accession Numbers are incorporated herein in their entirety by reference as though explicitly recited herein and for possible inclusion in any of the claims herein. | | | |
|---|---|---|---|
| **IGHV name** | **Variant name** | **Genbank Accession number** | **Sequence** |
| **IGHJ1** | J1*01 | J00256 | SEQ ID NO: 94 |
| **IGHJ2** | J2*01 | J00256 | SEQ ID NO: 95 |
| **IGHJ3** | J3*01 | J00256 | T GAT GCT TTT GAT GTC TGG GGC CAA GGG ACA ATG GTC ACC GTC TCT TCA G SEQ ID NO: 96 |
| | J3*02 | X86355 | G>A |
| **IGHJ4** | J4*01 | J00256 | AC TAC TTT GAC TAC TGG GGC CAA GGA ACC CTG GTC ACC GTC TCC TCA G SEQ ID NO: 97 |
| | J4*02 | X86355 | AA>AG |
| | J4*03 | M25625 | AC>GC GA>GG |
| **IGHJ5** | J5*01 | J00256 | AC AAC TGG TTC GAC TCC TGG GGC CAA GGA ACC CTG GTC ACC GTC TCC TCA G SEQ ID NO: 98 |
| | J5*02 | X86355 | T>C A>G |
| **IGHJ6** | J6*01 | J00256 | AT TAC TAC TAC TAC TAC GGT ATG GAC GTC TGG GGG CAA GGG ACC ACG GTC ACC GTC TCC TCA G SEQ ID NO: 99 |
| | J6*02 | X86355 | GG>G C |
| | | | |
| | J6*03 | X86356 | GGT>TAC GG>G C |
| | | | CA>AA |
| | J6*04 | AJ8794887 | GG>GC CA>AA |

**Table 4: Human Ig Vk Polymorphic Variants**

| All variant sequences appearing in Genbank under the listed Accession Numbers are incorporated herein in their entirety by reference as though explicitly recited herein and for possible inclusion in any of the claims herein. | | | | |
|---|---|---|---|---|
| **IGKV subgroup** | **IGKV name** | **Variant name** | **Accession number** | **Sequence** |
| **1** | IGKV1-5 | IGKV1-5*01 | Z00001 | SEQ ID NO: 100 |
| | | | | GAC ATC CAG ATG ACC CAG TCT CCT TCC ACC CTG TCT GCA TCT GTA GGA GAC AGA GTC ACC ATC ACT TGC CGG GCC AGT CAG AGT ATT AGT AGC TGG … … … … … … TTG GCC TGG TAT CAG CAG AAA CCA GGG AAA GCC CCT AAG |
| | | | | CTC CTG ATC TAT GAT GCC TCC ... ...... ... ... ... ... AGT TTG GAA AGT GGG GTC CCA ... TCA AGG TTC AGC GGC AGT GGA ... ... TCT GGG ACA GAA TTC ACT CTC ACC ATC AGC AGC CTG CAG CCT GAT GAT TTT GCA ACT TAT TAC TGC CAA CAG TAT AAT AGT TAT TCT CC |
| | | IGKV1-5*02 | M23851 | AC>AT |
| | | IGKV1-5*03 | X72813 | GAT>AAG |
| | | | | GCC>GCG |
| | | | | TCC>TCT |
| | | | | TTG>TTA |
| | IGKV1-6 | IGKV1-6*01 | M64858 | SEQ ID NO: 101 |
| | IGKV1-8 | IGKV1-8*01 | Z00014 | SEQ ID NO: 102 |
| | IGKV1D-8 | IGKV1D-8*01 | Z00008 | SEQ ID NO: 103 |
| | IGKV1-9 | IGKV1-9*01 | Z00013 | SEQ ID NO: 104 |
| | IGKV1-12 | IGKV1-12*01 | V01577 | SEQ ID NO: 105 |
| | | | | GAC ATC CAG ATG ACC CAG TCT CCA TCT TCC GTG TCT GCA TCT GTA GGA GAC AGA GTC ACC ATC ACT TGT CGG GCG AGT CAG GGT ATT AGC AGC TGG … … … … … … TTA GCC TGG TAT CAG CAG AAA CCA GGG AAA GCC CCT AAG CTC CTG ATC TAT GCT GCA TCC … … … … … … AGT TTG CAA AGT GGG GTC CCA … TCA AGG TTC AGC GGC AGT GGA … … TCT GGG ACA GAT TTC ACT CTC ACC ATC AGC AGC CTG CAG CCT GAA GAT TTT GCA ACT TAC TAT TGT CAA CAG GCT AAC AGT TTC CCT CC |
| | IGKV1D-12 | IGKV1D-12*01 | X17263 | TCC>TCT |
| | | | | ACC>ACT |
| | IGKV1-12/IGKV1D-12 (1) | IGKV1-12*02/1D-12*02 | V01576 | CC>TC |
| | IGKV1-13 | IGKV1-13*01 | Z00010 | SEQ ID NO: 106 |
| | | | | GCC ATC CAG TTG ACC CAG TCT CCA TCC TCC CTG TCT GCA TCT GTA GGA GAC AGA GTC ACC ATC ACT TGC CGG GCA AGT CAG GGC ATT AGC AGT GCT ... ... ... ... ... ... TTA GCC TGA TAT CAG CAG AAA CCA GGG AAA GCT CCT AAG CTC CTG ATC TAT GAT GCC TCC ... ... ... ... ... ... ... AGT TTG GAA AGT GGG GTC CCA ... TCA AGG TTC AGC GGC AGT GGA ... ... TCT GGG ACA GAT TTC ACT CTC ACC ATC AGC AGC CTG CAG CCT GAA GAT TTT GCA ACT TAT TAC TGT CAA CAG TTT AAT AAT TAC CCT CA |
| | | | | |
| | | IGKV1-13*02 | Z00006 | TGA>TGG |
| | | | | AAT>AGT |
| | IGKV1D-13 | IGKV1D-13*01 | X17262 | TGA>TGG |
| | IGKV1-16 | IGKV1-16*01 | J00248 | SEQ ID NO: 107 |
| | | | | GAC ATC CAG ATG ACC CAG TCT CCA TCC TCA CTG TCT GCA TCT GTA GGA GAC AGA GTC ACC ATC ACT TGT CGG GCG AGT CAG GGC ATT AGC AAT TAT ... ... ... ... ... ... TTA GCC TGG TTT CAG CAG AAA CCA GGG AAA GCC CCT AAG TCC CTG ATC TAT GCT GCA TCC ... ... ... ... ... ... AGT TTG CAA AGT GGG GTC CCA ... TCA AGG TTC AGC GGC AGT GGA ... ... TCT GGG ACA GAT TTC ACT CTC ACC ATC AGC AGC CTG CAG CCT GAA GAT TTT GCA ACT TAT TAC TGC CAA CAG TAT AAT AGT TAC CCT CC |
| | | IGKV1-16*02 | FM164406 | AGG>AAG |
| | IGKV1D-16 | IGKV1D-16*01 | K01323 | SEQ ID NO: 108 |
| | | | | GAC ATC CAG ATG ACC CAG TCT CCA TCC TCA CTG TCT GCA TCT GTA GGA GAC AGA GTC ACC ATC ACT TGT CGG GCG AGT CAG GGT ATT AGC AGC TGG ... ... ... ... ... ... TTA GCC TGG TAT CAG CAG AAA CCA GAG AAA GCC CCT AAG TCC CTG ATC TAT GCT GCA TCC ... ... ... ... ... ... AGT TTG CAA AGT GGG GTC CCA ... TCA AGG TTC AGC GGC AGT GGA ... ... TCT GGG ACA GAT TTC ACT CTC ACC ATC AGC AGC CTG CAG CCT GAA GAT TTT GCA ACT TAT TAC |
| | | | | TGC CAA CAG TAT AAT AGT TAC CCT CC |
| | | IGKV1D-16*02 | V00558 | AGT>AGG |
| | IGKV1-17 | IGKV1-17*01 | X72808 | SEQ ID NO: 109 |
| | | | | GAC ATC CAG ATG ACC CAG TCT CCA TCC TCC CTG TCT GCA TCT GTA GGA GAC AGA GTC ACC ATC ACT TGC CGG GCA AGT CAG GGC ATT AGA AAT GAT ... ... ... ... ... ... TTA GGC TGG TAT CAG CAG AAA CCA GGG AAA GCC CCT AAG CGC CTG ATC TAT GCT GCA TCC ... ... ... ... ... ... AGT TTG CAA AGT GGG GTC CCA ... TCA AGG TTC AGC GGC AGT GGA ... ... TCT GGG ACA GAA TTC ACT CTC ACA ATC AGC AGC CTG CAG CCT GAA GAT TTT GCA ACT TAT TAC TGT CTA CAG CAT AAT AGT TAC CCT CC |
| | | IGKV1-17*02 | D88255 | AGC>AAC |
| | IGKV1D-17 | IGKV1D-17*01 | X63392 | SEQ ID NO: 110 |
| | | | | AAC ATC CAG ATG ACC CAG TCT CCA TCT GCC ATG TCT GCA TCT GTA GGA GAC AGA GTC ACC ATC ACT TGT CGG GCG AGG CAG GGC ATT AGC AAT TAT ... ... ... ... ... ... TTA GCC TGG TTT CAG CAG AAA CCA GGG AAA GTC CCT AAG CAC CTG ATC TAT GCT GCA TCC ... ... ... ... ... ... AGT TTG CAA AGT GGG GTC CCA ... TCA AGG TTC AGC GGC AGT GGA ... ... TCT GGG ACA GAA TTC ACT CTC ACA ATC AGC AGC CTG CAG CCT GAA GAT TTT GCA ACT TAT TAC TGT CTA CAG CAT AAT AGT TAC CCT CC |
| | | IGKV1D-17*02 | FM164407 | AAC>GACCAC>CGC |
| | IGKV1-27 | IGKV1-27*01 | X63398 | SEQ ID NO:111 |
| | IGKV1-33 | IGKV1-33*01 | M64856 | SEQ ID NO: 112 |
| | IGKV1D-33 | IGKV1D-33*01 | M64855 | SEQ ID NO: 113 |
| | IGKV1-37 | IGKV1-37*01 | X59316 | SEQ ID NO: 114 |
| | IGKV1D-37 | IGKV1D-37*01 | X71893 | SEQ ID NO: 115 |
| | IGKV1-39 | IGKV1-39*01 | X59315 | SEQ ID NO: 116 |
| | | | | GAC ATC CAG ATG ACC CAG TCT CCA TCC TCC CTG TCT GCA TCT GTA GGA GAC AGA GTC ACC ATC ACT TGC CGG GCA AGT CAG AGC ATT AGC AGC TAT ... ... ... ... ... ... TTA AAT TGG TAT CAG CAG AAA CCA GGG AAA GCC CCT AAG CTC CTG ATC TAT GCT GCA TCC ... ... ... ... ... ... AGT TTG CAA AGT GGG GTC CCA ... TCA AGG TTC AGT GGC AGT GGA ... ... TCT GGG ACA GAT TTC ACT CTC ACC ATC AGC AGT CTG CAA CCT GAA GAT TTT GCA ACT TAC TAC TGT CAA CAG AGT TAC AGT ACC CCT CC |
| | | IGKV1-39*02 | X59318 | TCC>TTC |
| | | | | TAC>TAT |
| | | | | CAA CAG AGT> CAG TGT GGT |
| | | | | ACC>ACA |
| | IGKV1D-39 | IGKV1D-39*01 | X59312 | SEQ ID NO: 117 |
| | IGKV1D-42 | IGKV1D-42*01 | X72816 | SEQ ID NO: 118 |
| | IGKV1D-43 | IGKV1D-43*01 | X72817 | SEQ ID NO: 119 |
| **2** | IGKV2-24 | IGKV2-24*01 | X12684 | SEQ ID NO: 120 |
| | IGKV2D-24 | IGKV2D-24*01 | X63401 | SEQ ID NO: 121 |
| | IGKV2-28 | IGKV2-28*01 | X63397 | SEQ ID NO: 122 |
| | IGKV2D-28 | IGKV2D-28*01 | X12691 | SEQ ID NO: 123 |
| | IGKV2-29 | IGKV2-29*01 | X63396 | SEQ ID NO: 124 |
| | | | | GAT ATT GTG ATG ACC CAG ACT CCA CTC TCT CTG TCC GTC ACC CCT GGA CAG CCG GCC TCC  ATC TCC TGC AAG TCT AGT CAG AGC CTC CTG CAT AGT GAT GGA AAG ACC TAT ... TTG TAT TGG TAC CTG CAG AAG CCA GGC CAG |
| | | | | TCT CCA CAG CTC CTG ATC TAT GAA GTT TCC ... ... ... ... ... ... ... AGC CGG TTC TCT GGA GTG CCA ... GAT AGG TTC AGT GGC AGC GGG ... ... TCA GGG ACA GAT TTC ACA CTG AAA ATC AGC CGG GTG GAG GCT GAG GAT GTT GGG GTT TAT TAC TGA ATG CAA GGT ATA CAC CTT CCT CC |
| | | IGKV2-29*02 | U41645 | CTG> CTA TGA> TGC |
| | | IGKV2-29*03 | AJ783437 | TGA> TGC |
| | IGKV2D-29 | IGKV2D-29*01 | M31952 | GAT ATT GTG ATG ACC CAG ACT CCA CTC TCT CTG TCC GTC ACC CCT GGA CAG CCG GCC TCC ATC TCC TGC AAG TCT AGT CAG AGC CTC CTG CAT AGT GAT GGA AAG ACC TAT ... TTG TAT TGG TAC CTG CAG AAG CCA GGC CAG CCT CCA CAG CTC CTG ATC TAT GAA GTT TCC ... ... ... ... ... ... AAC CGG TTC TCT GGA GTG CCA ... GAT AGG TTC AGT GGC AGC GGG ... ... TCA GGG ACA GAT TTC ACA CTG AAA ATC AGC CGG GTG GAG GCT GAG GAT GTT GGG GTT TAT TAC TGC ATG CAA AGT ATA CAG CTT CCT CC SEQ ID NO: 125 |
| | | IGKV2D-29*02 | U41644 | CCT>TCT |
| | IGKV2-30 | IGKV2-30*01 | X63403 | SEQ ID NO: 126 |
| | | | | GAT GTT GTG ATG ACT CAG TCT CCA CTC TCC CTG CCC GTC ACC CTT GGA CAG CCG GCC TCC ATC TCC TGC AGG TCT AGT CAA AGC CTC GTA TAC AGT GAT GGA AAC ACC TAC ... TTG AAT TGG TTT CAG CAG AGG CCA GGC CAA |
| | | | | TCT CCA AGG CGC CTA ATT TAT AAG GTT TCT … … … … … … … AAC CGG GAC TCT GGG GTC CCA … GAC AGA TTC AGC GGC AGT GGG … … TCA GGC ACT GAT TTC ACA CTG AAA ATC AGC AGG GTG GAG GCT GAG GAT GTT GGG GTT TAT TAC TGC ATG CAA GGT ACA CAC TGG CCT CC |
| | | IGKV2-30*02 | FM164408 | TAC>CAC |
| | IGKV2D-30 | IGKV2D-30*01 | X63402 | SEQ ID NO: 127 |
| | IGKV2-40 | IGKV2-40*01 | X59314 | GAT ATT GTG ATG ACC CAG ACT CCA CTC TCC CTG CCC GTC ACC CCT GGA GAG CCG GCC TCC ATC TCC TGC AGG TCT AGT CAG AGC CTC TTG GAT AGT GAT GAT GGA AAC ACC TAT TTG GAC TGG TAC CTG CAG AAG CCA GGG CAG TCT CCA CAG CTC CTG ATC TAT ACG CTT TCC ... ... ... ... ... ... ... TAT CGG GCC TCT GGA GTC CCA ... GAC AGG TTC AGT GGC AGT GGG ... ... TCA GGC ACT GAT TTC ACA CTG AAA ATC AGC AGG GTG GAG GCT GAG GAT GTT GGA GTT TAT TAC TGC ATG CAA CGT ATA GAG TTT CCT TC SEQ ID NO: 128 |
| | | IGKV2-40*02 | X59317 | GAC TGG>GAT TGT |
| | | | | GGC>GAC |
| | IGKV2D-40 | IGKV2D-40*01 | X59311 | SEQ ID NO: 129 |
| **3** | IGKV3-7 | IGKV3-7*01 | X02725 | SEQ ID NO: 130 |
| | | | | GAA ATT GTA ATG ACA CAG TCT CCA CCC ACC CTG TCT TTG TCT CCA GGG GAA AGA GTC ACC CTC TCC TGC AGG GCC AGT CAG AGT GTT AGC AGC AGC TAC ... ... ... ... ... TTA ACC TGG TAT CAG CAG AAA CCT GGC CAG GCG CCC AGG CTC CTC ATC TAT GGT GCA TCC ... ... ... ... ... ... ACC AGG GCC ACT AGC ATC CCA ... GCC AGG TTC AGT GGC AGT GGG ... ... TCT GGG ACA GAC TTC ACT CTC ACC ATC AGC AGC CTG CAG CCT GAA GAT TTT GCA GTT TAT TAC TGT CAG CAG GAT CAT AAC TTA CCT CC |
| | | IGKV3-7*02 | X72812 | ACC>TCC |
| | | | | TAT>TAC |
| | | | | GGC>GGG |
| | | | | GCG>GCT |
| | | | | AGC>GGC |
| | | | | ACA>AGA |
| | | | | CAT>TAT |
| | | IGKV3-7*03 | K02769 | SEQ ID NO: 131 |
| | | | | GAA ATT GTA ATG ACA CAG TCT CCA CCC ACC CTG TCT TTG TCT CCA GGG GAA AGA GTC ACC CTC TCC TGC AGG GCC AGT CAG AGT GTT AGC AGC AGC TAC ... ... ... ... ... TTA ACC TGG TAT CAG CAG AAA CCT GGC CAG GCG CCC AGG CTC CTC ATC TAT GGT GCA TCC ... ... ... ... ... ... ... ACC AGG GCC ACT AGC ATC CCA ... GCC AGG TTC AGT GGC AGT GGG ... ... TCT GGG ACA GAC TTC ACT CTC ACC ATC AGC AGC CTG CAG CCT GAA GAT TTT GCA GTT TAT |
| | | | | TAC TGT CAG CAG GAT CAT AAC TTA CCT CC |
| | | IGKV3-7*04 | FM164409 | CAT>TAT |
| | IGKV3D-7 | IGKV3D-7*01 | X72820 | SEQ ID NO: 132 |
| | IGKV3-11 | IGKV3-11*01 | X01668 | SEQ ID NO: 133 |
| | | | | GAA ATT GTG TTG ACA CAG TCT CCA GCC ACC CTG TCT TTG TCT CCA GGG GAA AGA GCC ACC CTC TCC TGC AGG GCC AGT CAG AGT GTT AGC AGC TAC ... ... ... ... ... ... TTA GCC TGG TAC CAA CAG AAA CCT GGC CAG GCT CCC AGG CTC CTC ATC TAT GAT GCA TCC ... ... ... ... ... ... AAC AGG GCC ACT GGC ATC CCA ... GCC AGG TTC AGT GGC AGT GGG ... ... TCT GGG ACA GAC TTC ACT CTC ACC ATC AGC AGC CTA GAG CCT GAA GAT TTT GCA GTT TAT TAC TGT CAG CAG CGT AGC AAC TGG CCT CC |
| | | IGKV3-11*02 | K02768 | ACA>AGA |
| | IGKV3D-11 | IGKV3D-11*01 | X17264 | SEQ ID NO: 134 |
| | IGKV3-15 | IGKV3-15*01 | M23090 | SEQ ID NO: 135 |
| | IGKV3D-15 | IGKV3D-15*01 | X72815 | SEQ ID NO: 136 |
| | | | | GAA ATA GTG ATG ACG CAG TCT CCA GCC ACC CTG TCT GTG TCT CCA GGG GAA AGA GCC ACC CTC TCC TGC AGG GCC AGT CAG AGT GTT AGC AGC AAC ... ... ... ... ... ... TTA GCC TGG TAC CAG CAG AAA CCT GGC CAG GCT CCC AGG CTC CTC ATC TAT GGT GCA TCC ... ... ... ... ... ... ... ACC AGG GCC ACT GGC ATC CCA ... GCC AGG TTC AGT GGC AGT GGG ... ... TCT GGG ACA GAG TTC ACT CTC ACC ATC AGC AGC CTG CAG TCT GAA GAT TTT GCA GTT TAT TAC TGT CAG CAG TAT AAT AAC TGG CCT CC |
| | | IGKV3D-15*02 | M23091 | ACG>ATG |
| | | | | TGG>TGA |
| | IGKV3-20 | IGKV3-20*01 | X12686 | SEQ ID NO: 137 |
| | | | | GAA ATT GTG TTG ACG CAG TCT CCA GGC ACC CTG TCT TTG TCT CCA GGG GAA AGA GCC ACC CTC TCC TGC AGG GCC AGT CAG AGT GTT AGC AGC AGC TAC ... ... ... ... ... TTA GCC TGG TAC CAG CAG AAA CCT GGC CAG GCT CCC AGG CTC CTC ATC TAT GGT GCA TCC ... ... ... ... ... ... ... AGC AGG GCC ACT GGC ATC CCA ... GAC AGG TTC AGT GGC AGT GGG ... ... TCT GGG ACA GAC TTC ACT CTC ACC ATC AGC AGA CTG GAG CCT GAA GAT TTT GCA GTG TAT TAC TGT CAG CAG TAT GGT AGC TCA CCT CC |
| | | IGKV3-20*01, Tou-kv325 | X56593 | ACC>CCC |
| | | | | CTG>TGT |
| | | | | TCT> CTT |
| | | | | TTG> TGT |
| | | | | TCT> CTC |
| | | | | |
| | | IGKV3-20*02 | L37729 | ACG>ACA |
| | | | | GGC>GCC |
| | | | | GAC>GCA |
| | IGKV3D-20 | IGKV3D-20*01 | X12687 | SEQ ID NO: 138 |
| **4** | IGKV4-1 | IGKV4-1*01 | Z00023 | SEQ ID NO: 139 |
| **5** | IGKV5-2 | IGKV5-2*01 | X02485 | SEQ ID NO: 140 |
| **6** | IGKV6-21 | IGKV6-21*01 | X63399 | SEQ ID NO: 141 |
| | IGKV6D-21 | IGKV6D-21*01 | X12683 | SEQ ID NO: 142 |
| | IGKV6D-41 | IGKV6D-41*01 | M27751 (3) | SEQ ID NO: 143 |

**Table 5: Human Ig Vλ Polymorphic Variants**

| All variant sequences appearing in Genbank under the listed Accession Numbers are incorporated herein in their entirety by reference as thoug explicitly recited herein and for possible inclusion in any of the claims herein. | | | | |
|---|---|---|---|---|
| | | | | |

| **IGLV subgroup** | **IGLV Gene name** | **IGLV variant name** | **Accession number** | **Sequences & Description of mutations** |
|---|---|---|---|---|
| 1 | IGLV1-36 | V1-36*01 | Z73653 | SEQ ID NO: 144 |
| | IGLV1-40 | V1-40*01 | M94116 | SEQ ID NO: 145 |
| | | V1-40*02 | X53936 | g9>c\|c10>g,L4>V\| |
| | | V1-40*03 | Z22192 | g9>c\|c10>g,L4>V\|a253>g,T85>A\| |
| | IGLV1-41 | V1-41*01 | M94118 | SEQ ID NO: 146 |
| | | V1-41*02 | D87010 | g295>t,E99>*\|c332>t,P111>L\| |
| | | | | |
| | IGLV1-44 | V1-44*01 | Z73654 | SEQ ID NO: 147 |
| | IGLV1-47 | V1-47*01 | Z73663 | SEQ ID NO: 148 |
| | | V1-47*02 | D87016 | g168>t,R56>S\| |
| | IGLV1-50 | V1-50*01 | M94112 | SEQ ID NO: 149 |
| | IGLV1-51 | V1-51*01 | Z73661 | SEQ ID NO: 150 |
| | | V1-51*02 | M30446 | t162>c\|c168>a,D56>E\| |
| 2 | IGLV2-8 | V2-8*01 | X97462 | SEQ ID NO: 151 |
| | | V2-8*02 | L27695 | g37>a,G13>R\| |
| | | V2-8*03 | Y12418 | \|c230>t,S77>F\| |
| | IGLV2-11 | V2-11*01 | Z73657 | SEQ ID NO: 152 |
| | | V2-11*02 | Z22198 | t96>g\| |
| | | V2-11*03 | Y12415 | \|g132>a\| |
| | IGLV2-14 | V2-14*01 | Z73664 | SEQ ID NO: 153 |
| | | V2-14*02 | L27822 | c87>t\|t93>g\|g94>a,G32>S\|t103>c,a104>t,Y35>L\| \|t170>g,V57>G\|t198>g,N66>K\| |
| | | V2-14*03 | Y12412 | \|g132>a\|g168>t,E56>D\| |
| | | V2-14*04 | Y12413 | \|g168>t,E56>D\| |
| | IGLV2-18 | V2-18*01 | Z73642 | SEQ ID NO: 154 |
| | | V2-18*02 | L27697 | \|t317>c, L106>S \| |
| | | V2-18*03 | L27694 | \|t272>c,I91>T\| t317>c,L106>S \| |
| | | V2-18*04 | L27692 | t227>c,F76>S\|t249>c\| \|t317>c,L106>S\| |
| | IGLV2-23 | V2-23*01 | X14616 | SEQ ID NO: 155 |
| | | V2-23*02 | Z73665 | g170>t,G57>V\|a339>c,L113>F\| |
| | | V2-23*03 | D86994 | \|a339>c,L113>F\| |
| | IGLV2-33 | V2-33*01 | Z73643 | SEQ ID NO: 156 |
| | | V2-33*02 | L27823 | a3>g\| |
| | | V2-33*03 | L27691 | \|t96>c\|a256>g,M86>V\| |
| 3 | IGLV3-1 | V3-1*01 | X57826 | SEQ ID NO: 157 |
| | IGLV3-9 | V3-9*01 | X97473 | a52 ,T18 \|a82 ,I28 \|a88 ,g89 ,S30 \|a95 ,N32 \|g173 ,S58 \| |
| | | V3-9*02 | X74288 | a52>9,T18>A\|a82>c,I28>L\|a88>t,g89>a,S30>Y\|a95>g ,N32>S \|g173>a,S58>N\| |
| | | V3-9*03 | X51754 | a52>g,T18>A\|a82>c,I28>L\|a88>t,g89>a,S30>Y\|a95>del#,N32>del# \|g173>a,S58>N\| |
| | IGLV3-10 | V3-10*01 | X97464 | SEQ ID NO: 158 |
| | | V3-10*02 | L29166 | g166>a,E56>K\| c207>a\|t270>c\|c299>a,A100>D\|a319>g,T107>A\|a325> t,g326>a,S109>Y\| |
| | IGLV3-12 | V3-12*01 | Z73658 | SEQ ID NO: 159 |
| | | V3-12*02 | D86998 | a259>g,T87>A\| |
| | | | | |
| | IGLV3-16 | V3-16*01 | X97471 | SEQ ID NO: 160 |
| | IGLV3-19 | V3-19*01 | X56178 | SEQ ID NO: 161 |
| | IGLV3-21 | V3-21*01 | X71966 | SEQ ID NO: 162 |
| | | V3-21*02 | D87007 | a27>g\|a49>c,K17>Q\|a160>g,154>V\|t166>g,Y56>D\|c324>t\| |
| | | V3-21*03 | M94115 | a27>g\| \|a160>g,I54>V\|t166>g,Y56>D\|c324>t\| |
| | IGLV3-22 | V3-22*01 | Z73666 | SEQ ID NO: 163 |
| | | V3-22*02 | X71967 | c53>a,T18>K\|g88>a, E30>K \|g140>del#,G47>del#\|c148>t,c149>g,t150> a,P50>*\| 150^151>ins^tatac#,50^51>ins^Y#\| g322>a,D108>N\|c330>t\| |
| | IGLV3-25 | V3-25*01 | X97474 | SEQ ID NO: 164 |
| | | V3-25*02 | D86994 | t14>c,g15>a,M5>T\| |
| | | V3-25*03 | L29165 | t14>c,g15>a,M5>T\|t294>c\| |
| | IGLV3-27 | V3-27*01 | D86994 | SEQ ID NO: 165 |
| | IGLV3-32 | V3-32*01 | Z73645 | SEQ ID NO: 166 |
| 4 | IGLV4-3 | V4-3*01 | X57828 | SEQ ID NO: 167 |
| | IGLV4-60 | V4-60*01 | Z73667 | SEQ ID NO: 168 |
| | | V4-60*02 | D87000 | a288>t,L96>F\| |
| | | V4-60*03 | AF073885 | t287>c,a288>t,L96>S\| |
| | IGLV4-69 | V4-69*01 | Z73648 | SEQ ID NO: 169 |
| | | V4-69*02 | U03868 | c198>t\| |
| 5 | IGLV5-37 | V5-37*01 | Z73672 | SEQ ID NO: 170 |
| | IGLV5-39 | V5-39*01 | Z73668 | t81 ,g135>t\| |
| | | V5-39*02 | AF216776 | t81>c,g135>t\| |
| | IGLV5-45 | V5-45*01 | Z73670 | SEQ ID NO: 171 |
| | | V5-45*02 | Z73671 | g22>t,A8>S\|g75>a\| |
| | | V5-45*03 | D86999 | g22>t,A8>S\| |
| | IGLV5-48 | V5-48*01 | Z73649 | SEQ ID NO: 172 |
| | IGLV5-52 | V5-52*01 | Z73669 | SEQ ID NO: 173 |
| 6 | IGLV6-57 | V6-57*01 | Z73673 | SEQ ID NO: 174 |
| 7 | IGLV7-43 | V7-43*01 | X14614 | SEQ ID NO: 175 |
| | IGLV7-46 | V7-46*01 | Z73674 | SEQ ID NO: 176 |
| | | V7-46*02 | D86999 | \|c275>t,S92>L\| |
| | | V7-46*03 | Z22210 | c271>del#\| L91>del# \| |
| 8 | IGLV8-61 | V8-61*01 | Z73650 | SEQ ID NO: 177 |
| | | V8-61*02 | U03637 | \|c223>t,R75>C\| |
| | | V8-61*03 | AF266511 | g210>c\| |
| 9 | IGLV9-49 | V9-49*01 | Z73675 | SEQ ID NO: 178 |
| | | V9-49*02 | Z73675 | \|g291>a\| |
| | | V9-49*03 | U03869 | g153>a\| |
| 10 | IGLV10 -54 | V10-54*01 | Z73676 | SEQ ID NO: 179 |
| | | V10-54*02 | D86996 | \|a86>t,N29>I\| \|a228>c,L76>F\|g320>t, W107>L\| |
| | | V10-54*03 | S70116 | g33>c\| \|t125>c,g126>t,L42>P\|c127>g,Q43>E\| |
| 11 | IGLV11 -55 | V11-55*01 | D86996 | SEQ ID NO: 180 |
| | | | | |
| | | | | |
| | | | | |
| | | | | |

**Table 6: Human IgH Jk Polymorphic Variants**

| All variant sequences appearing in Genbank under the listed Accession Numbers are incorporated herein in their entirety by reference as though explicitly recited herein and for possible inclusion in any of the claims herein. | | | |
|---|---|---|---|
| | | | |

| **IGKJ gene name** | **Variant name** | **Accession number** | **Sequence** |
|---|---|---|---|
| **IGKJ1** | IGKJ1*01 | J00242 | SEQ ID NO: 181 |
| **IGKJ2** | IGKJ2*01 | J00242 | SEQ ID NO: 182 |
| | | | TG TAC ACT TTT GGC CAG GGG ACC AAG CTG GAG ATC AAA C |
| | IGKJ2*02 | Z70260 #c | TAC>TGC |
| | IGKJ2*03 | U95246 #c | ACT>AGT |
| | | | |
| | IGKJ2*04 | L40735 #c | TAC>TGC |
| | | | ACT>AGT |
| **IGKJ3** | IGKJ3*01 | J00242 | SEQ ID NO: 183 |
| **IGKJ4** | IGKJ4*01 | J00242 | SEQ ID NO: 184 |
| | | | G CTC ACT TTC GGC GGA GGG ACC AAG GTG GAG ATC AAA C |
| | IGKJ4*02 | AF103571 #c | ACT>ACG |
| **IGKJ5** | IGKJ5*01 | J00242 | SEQ ID NO: 185 |

**Table 7: Human IgH Jλ Polymorphic Variants**

| All variant sequences appearing in Genbank under the listed Accession Numbers are incorporated herein in their entirety by reference as though explicitly recited herein and for possible inclusion in any of the claims herein. | | | |
|---|---|---|---|
| **IGLJ gene name** | **Variant name** | **Accession number** | **Sequence** |
| **IGLJ1** | J1*01 | X04457 | SEQ ID NO: 186 |
| **IGLJ2** | J2*01 | M15641 | SEQ ID NO: 187 |
| **IGLJ3** | J3*01 | M15642 | SEQ ID NO: 188 |
| | | | T GTG GTA TTC GGC GGA GGG ACC AAG CTG ACC GTC CTA G |
| | J3*02 | D87023(1) | GTG> TGG |
| | | | GTA> GTG |
| **IGLJ4** | J4*01 | X51755 | SEQ ID NO: 189 |
| **IGLJ5** | J5*01 | X51755 | SEQ ID NO: 190 |
| | | | C TGG GTG TTT GGT GAG GGG ACC GAG CTG ACC GTC CTA G |
| | J5*02 | D87017 | ACC>ACG |
| **IGLJ6** | J6*01 | M18338 | SEQ ID NO: 191 |
| **IGLJ7** | J7*01 | X51755 | SEQ ID NO: 192 |
| | | | T GCT GTG TTC GGA GGA GGC ACC CAG CTG ACC GTC CTC G |
| | J7*02 | D87017 | GTC>GCC |

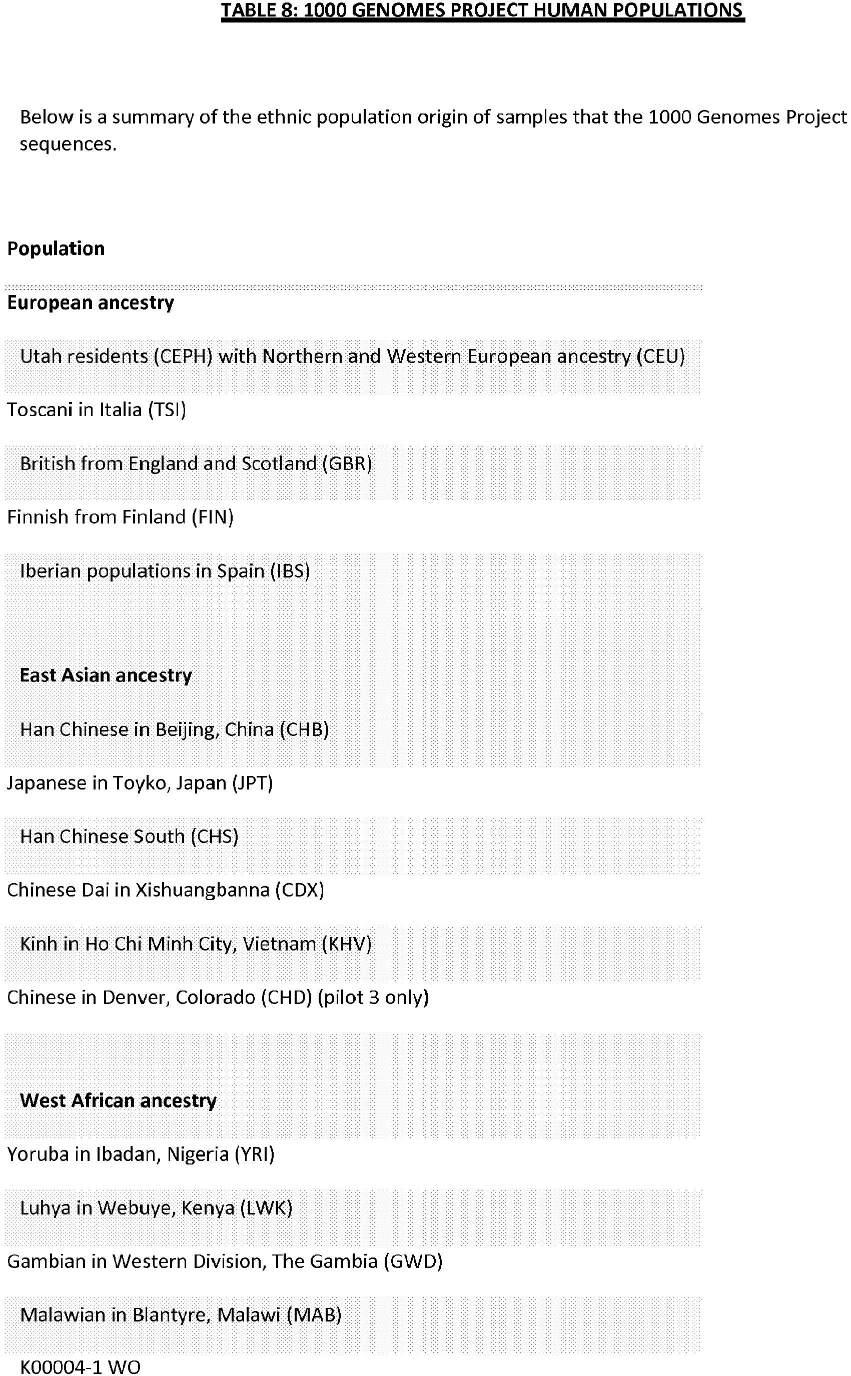

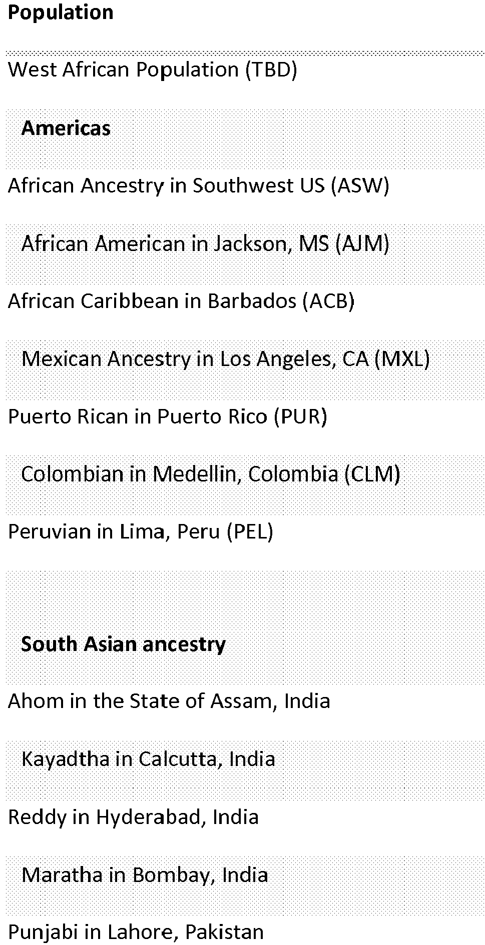

**Table 9: Immunoglobulin Gene Usage in Human Antibody Responses to Infectious Disease Pathogens**

| | | | |
|---|---|---|---|
| | | | |
| KAPPA V GENES | Haemophilus influenzae type b polysaccharide (HibPS) | Haemophilus influenzae | 1. Lonberg, Nat Biotech 2005; [human PBMCs] |
| • Vk II germline gene A2 + JK3 | | | |
| • Vk II family gene + JK4 | | | 2. Adderson et al, J Clin Invest 1992; [Human PBLs] |
| • 94% identical to the A27 (Humkv325) germ line gene | | | 3. Chung et al, J Immunol 1993 |
| • a VκI gene family member; κl-15A (KL012) | | | 4. Nadel et al, J Immunol 1998 |
| | | | 5. Feeney et al, J Clin Invest 1996 |
| LAMBDA V GENES | | | 6. Lucas et al, Infect Immun 1994; [Human PBLs] |
| • Four Vλ VII family members that are 96-98% identical to each other | | | 7. Adderson et al, J Clin Invest 1993; [Human PBLs] |
| | | | 8. Granoff et al, J Clin Invest 1993; [human PBLs] |
| • Vλ II family members (82, 89 and 91% homologous to Vλ2.1 gene) + VHIII segments closely homologous to germline gene 9.1 | | | 9. Azmi et al, Infect Immun 1994; [human tonsil cells] |
| • V_{λ}VII 4A | | | |
| • All with Jλ homologous to germline Jλ2 and Jλ3 | | | |
| VH GENES | | | |
| • VH 96% identical to the VH germ line gene segment DP77 (V3-21) | | | |
| • LSG6.1, LSG12.1, V_{H}III VH26, V_{H}III 9.1 | | | |
| VH and VL COMBINATIONS | | | |
| • V_{H}III 9.1+ V_{λ}VII 4A | | | |
| • V_{H}III 9.1 + V_{λ}II 2.1 | | | |
| • V_{H}III 9.1 + V_{κ}II A2 | | | |
| • V_{H}III VH26 + V_{λ}II 2.1 | | | |
| • V_{H}III 9.1; V_{H}III H11; V_{H}III VH26 | • Polysaccharide capsule of E coli K1 | • E coli K1 | 9. Azmi et al, Infect Immun 1994 |
| • κI 15A | | | |
| • Vλ2.1 | • Meningococcal B polysaccharide; Poly[α(2→8)-N-acetylneuramic acid | • Neisseria meningitidis Group B | |
| | | | |
| • VHIII or VHIV family member | | • Herpes family virus | 10. Huang et al, J Clin Invest 1992; [human tonsils] |
| • VλI or Vλ3 member | • HSV 120-kD glycoprotein | • Herpes simplex virus (HSV); HSV-1; HSV-2 | |
| • VH26 + Dk1 + JH6 with IGLV1S2 + Jλ2 | • 116-, 105-, 64-kD glycoproteins of VZV | • Varicella zoster virus (VZV) | |
| • VH4.18 | | | |
| • VH2-1 (VH3) + D region Dxp'1 + JH5 with Vλ3 cML70 + Jλ3 | | | |
| • VH1GRR+ JH3 + Dn4r or D2r with IGLV1S2 + Jλ2 | | | |
| • For VZV Abs: ha3h2 (VH3) with lalh2 (Vλ); or ha1c1 (VH1) with lalvl (Vλ1) | | | |
| • For VZV Abs: ha4h3 (VH4) with la3h3 (Vλ3) | | | |
| • Hv1051 (VH) | | • Cytomegalovirus (CMV) | 10. Huang et al, J Clin Invest 1992; |
| • Kv325 (Vk) | | | |
| • 71-2 (VH) | | • HIV | 10. Huang et al, J Clin Invest 1992; |
| • Hv1f10 (VH) | | | 11. Wang & Palese, Science 2011 |
| • VH4.11 | | | |
| • 71-4 (VH) | | | |
| • VH251 | | | |
| • VH1-69 | | | |
| • VH1-69 | Haemagglutinin (HA) | • Influenza virus, eg, Group 1 | 12. Ekiert et al, Science 2009 |
| | | and/or Group 2 Infulenza A | 13. Throsby et al, PLoS One 2008 |
| | | virus; eg, H1N1, H2N2, or | 14. Sui et al, Nat Struct Mol Biol 2009 |
| | | H3N2 or H7N2 or H7N7 influenza virus | 15. Ekiert et al, Science 2011 |

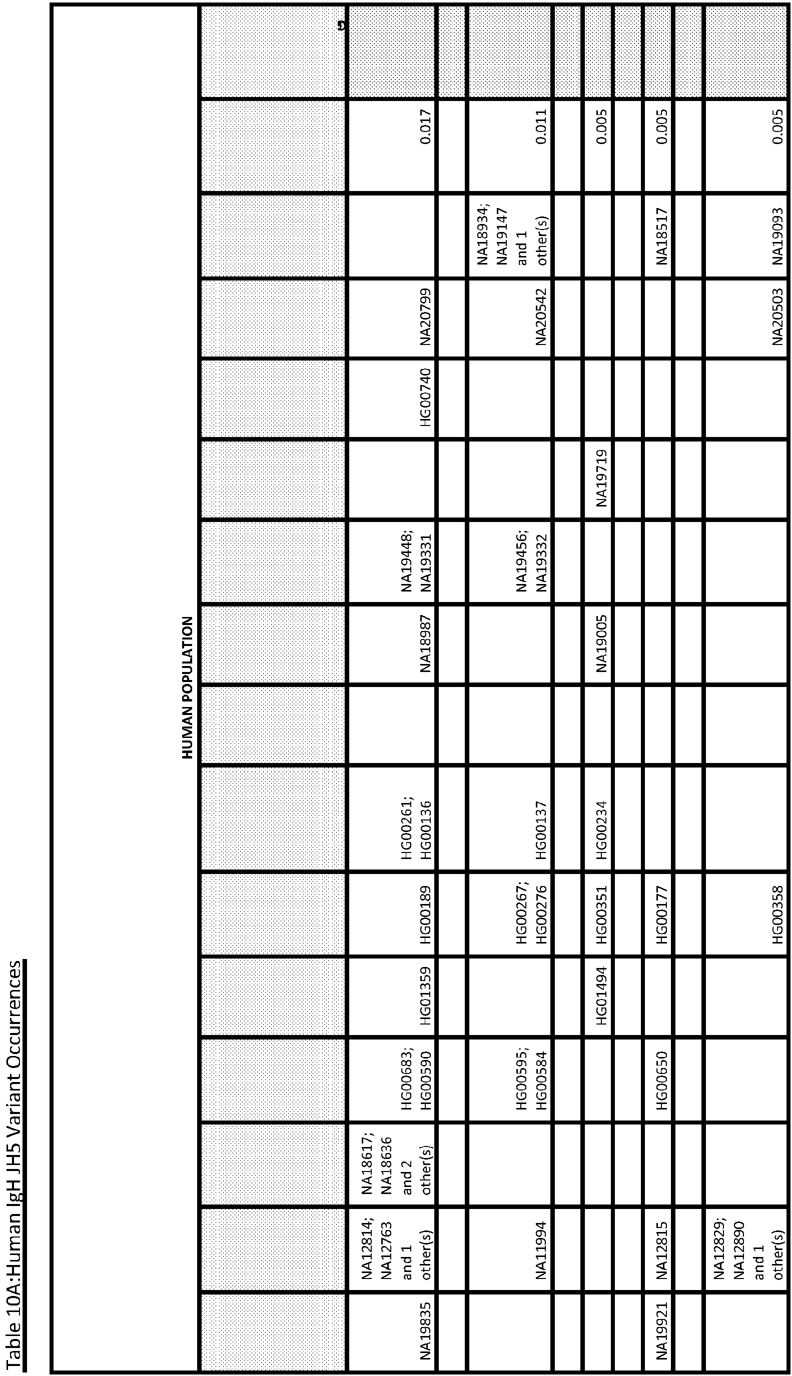

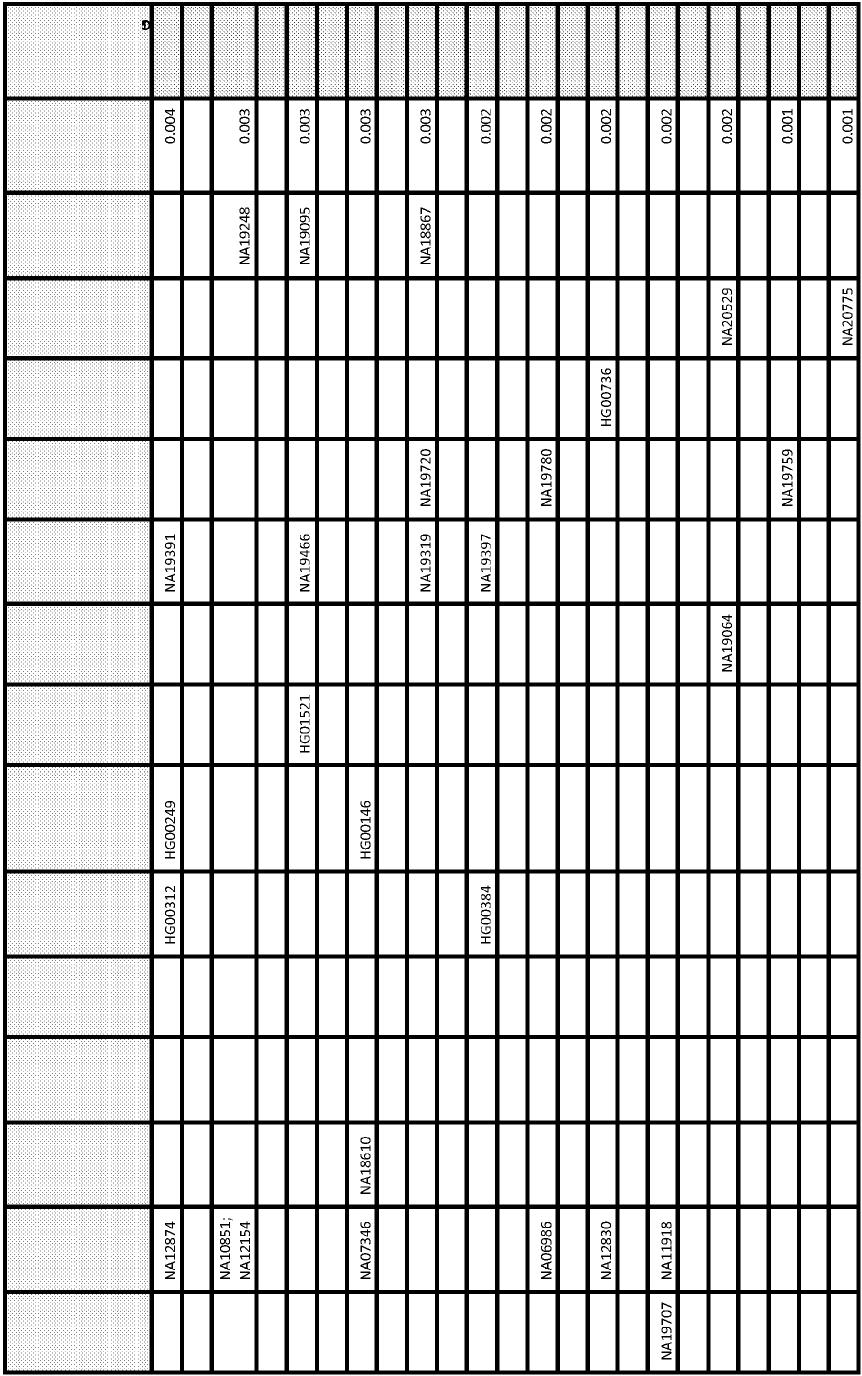

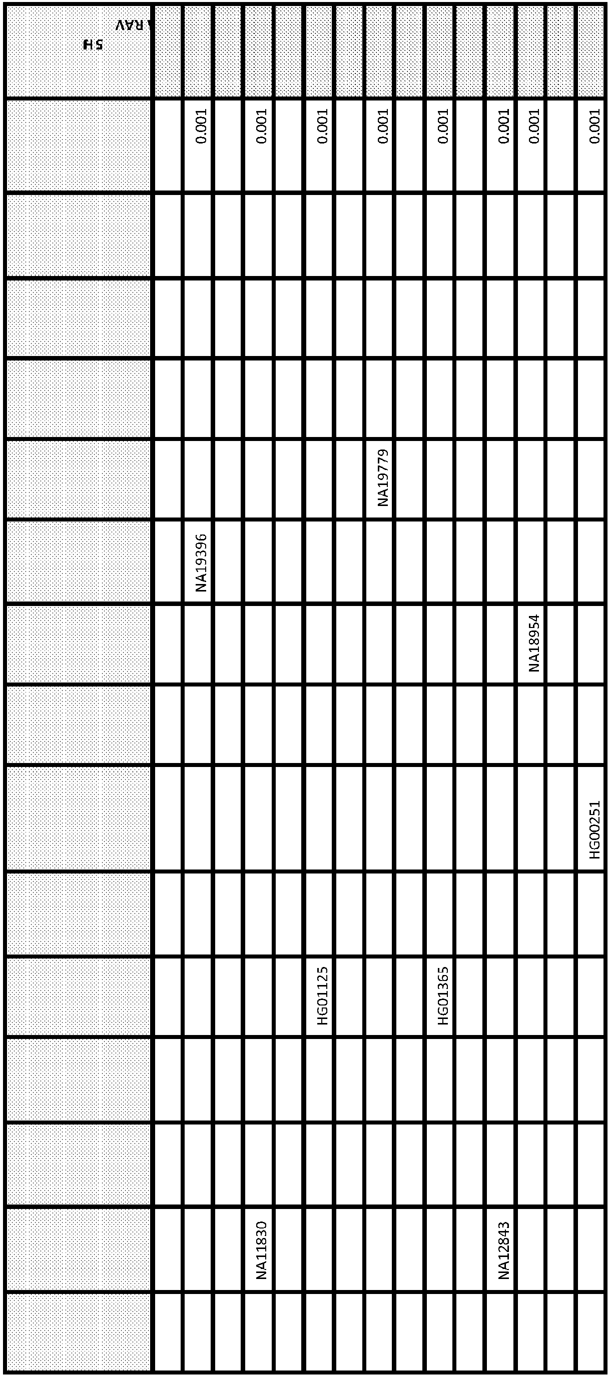

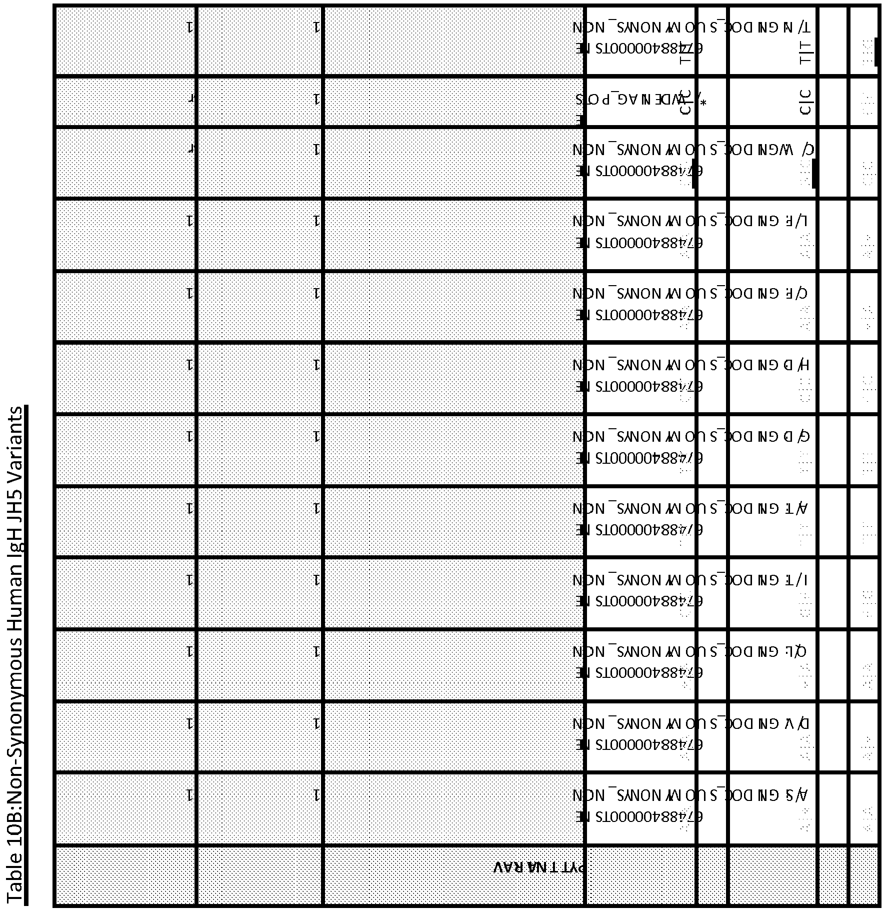

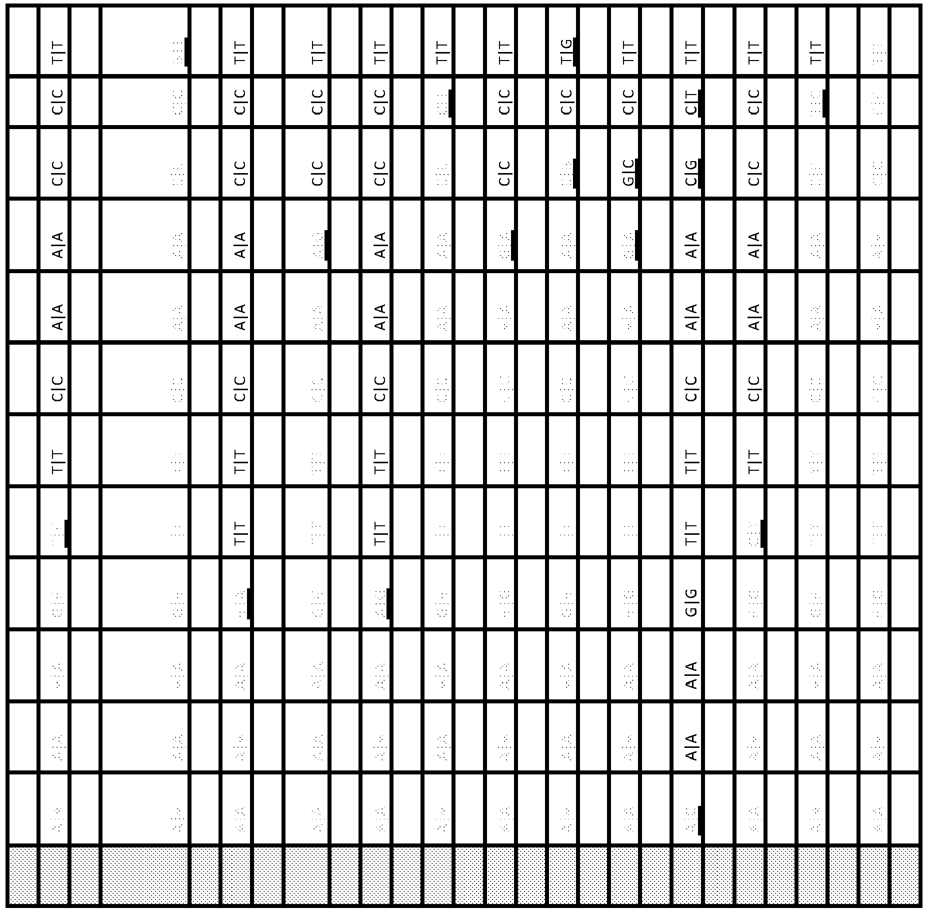

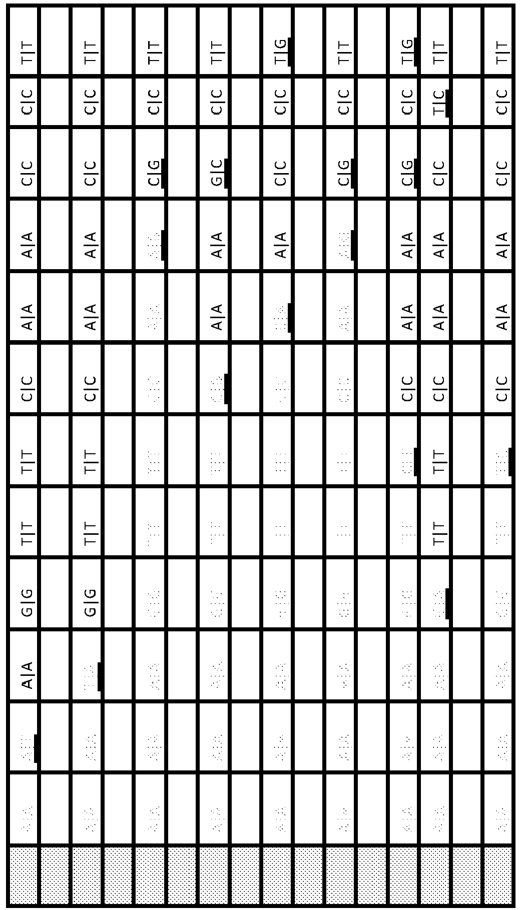

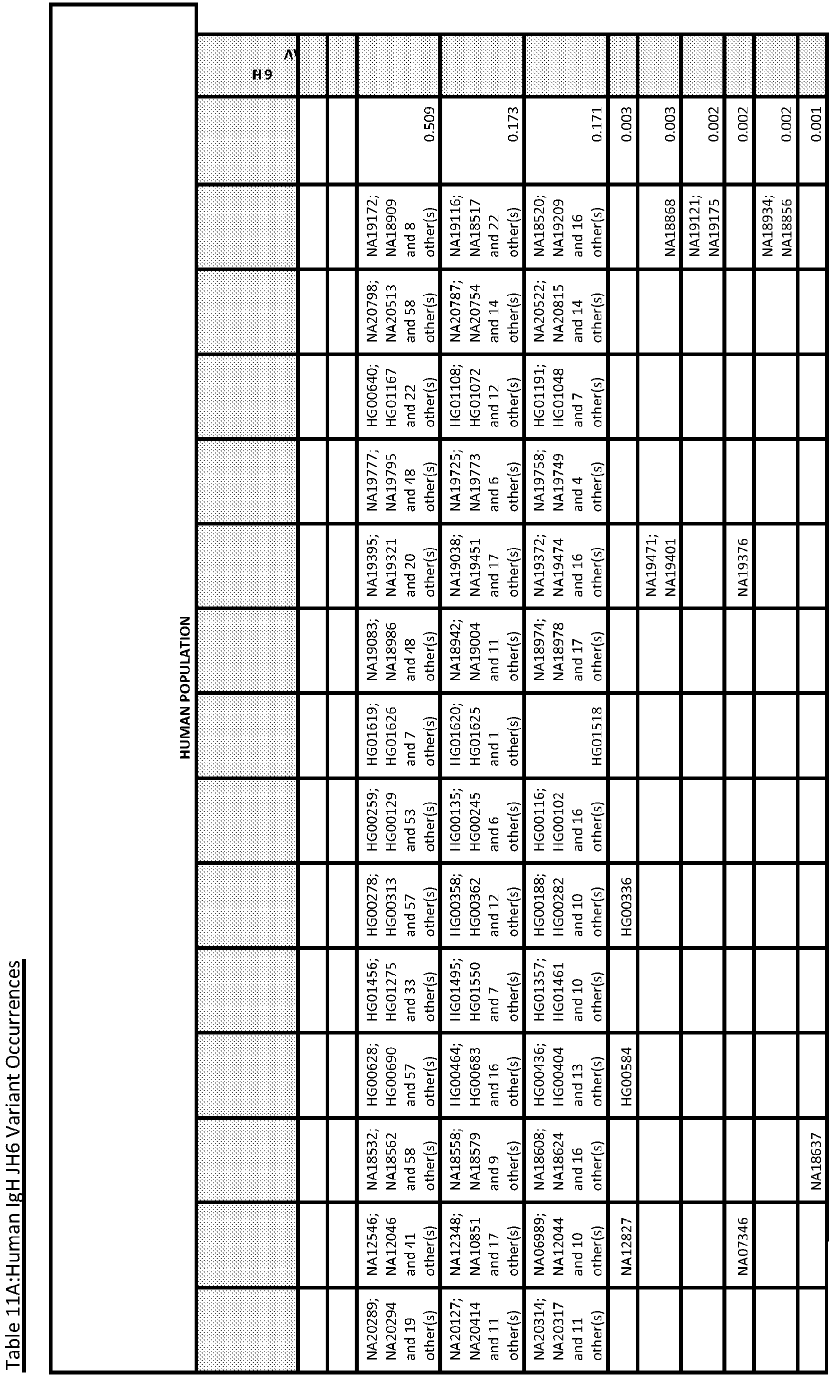

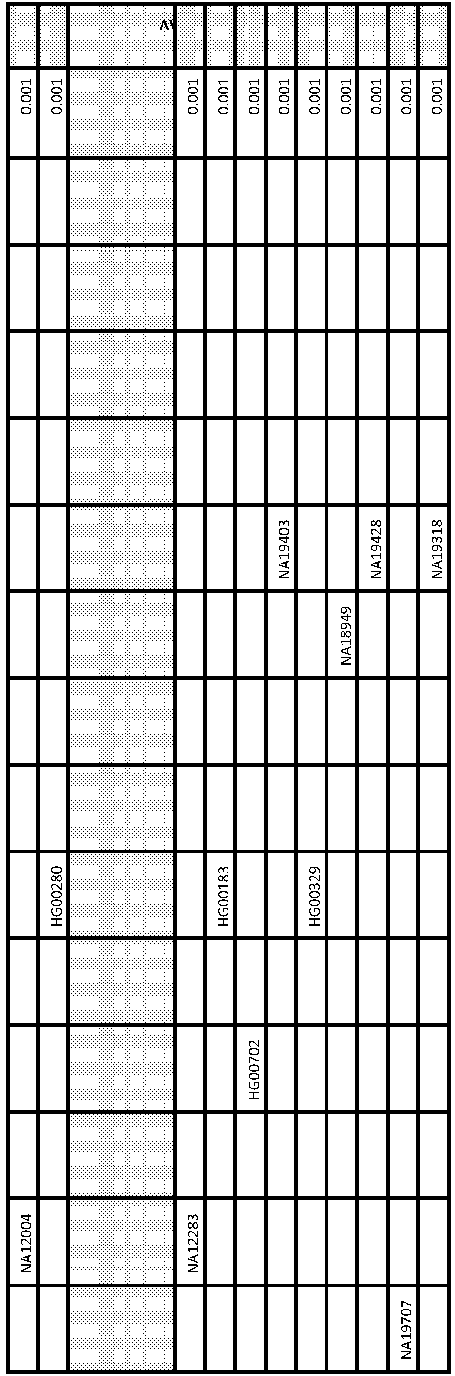

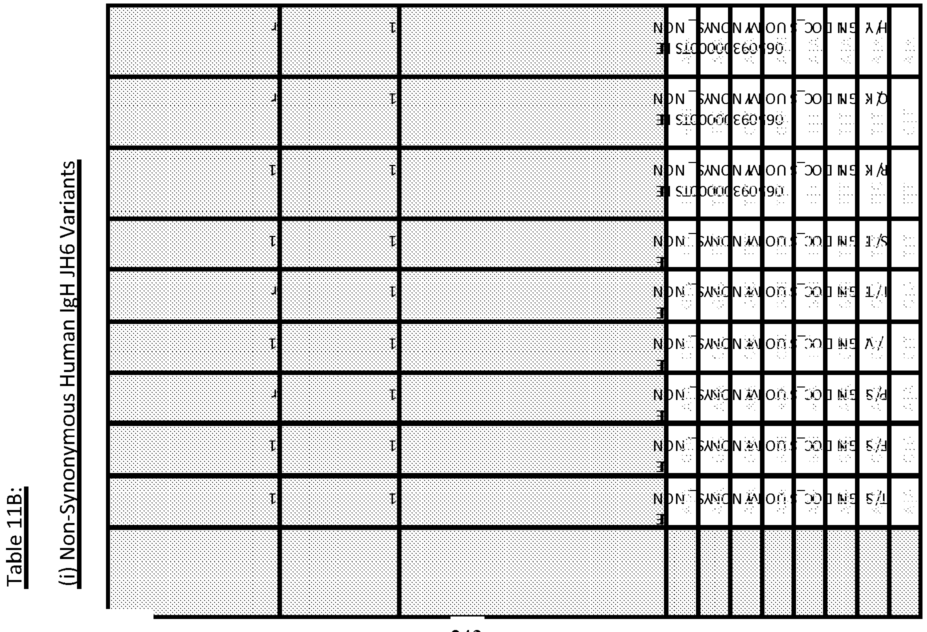

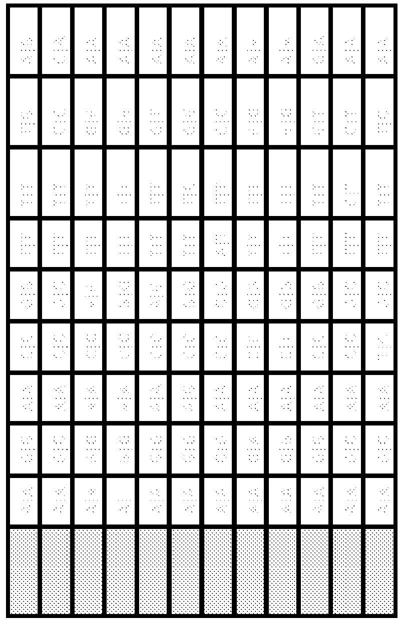

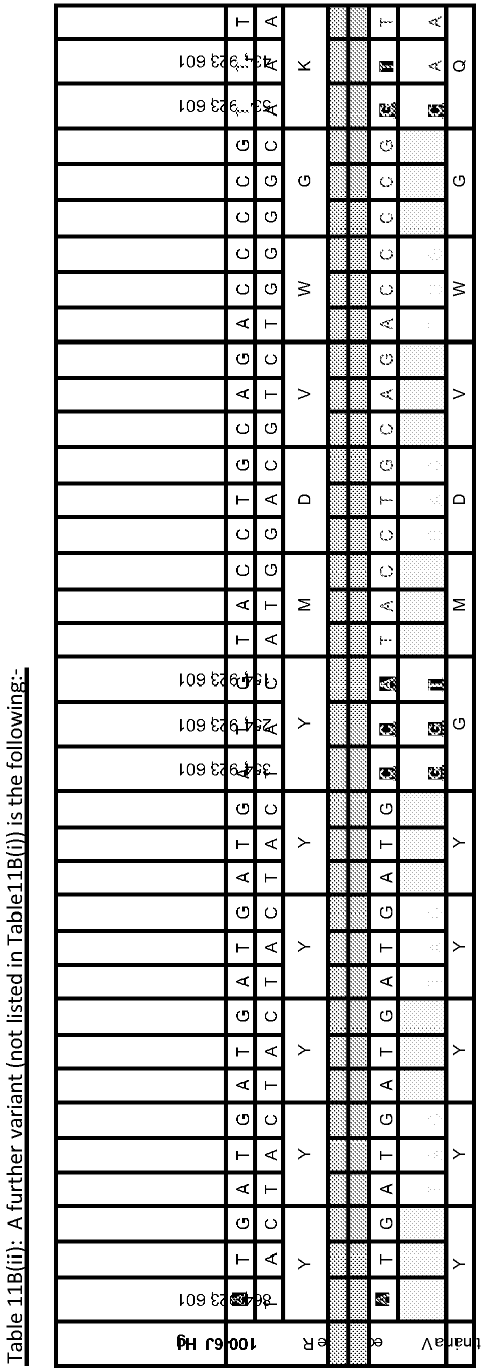

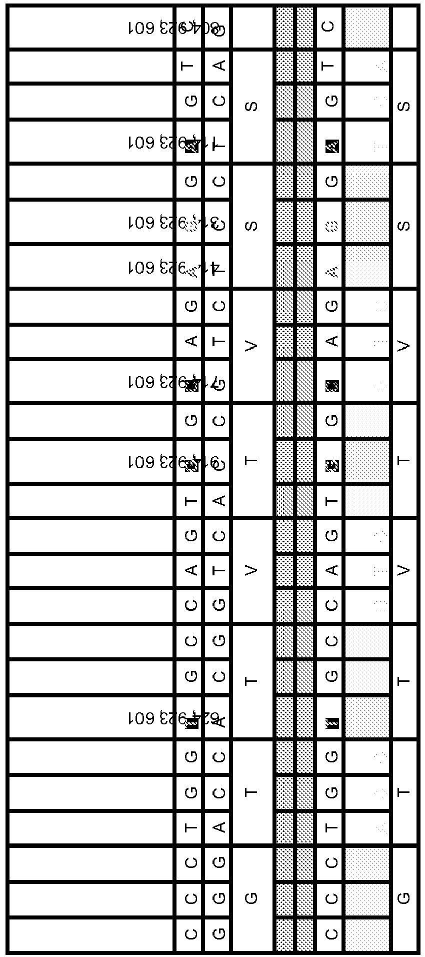

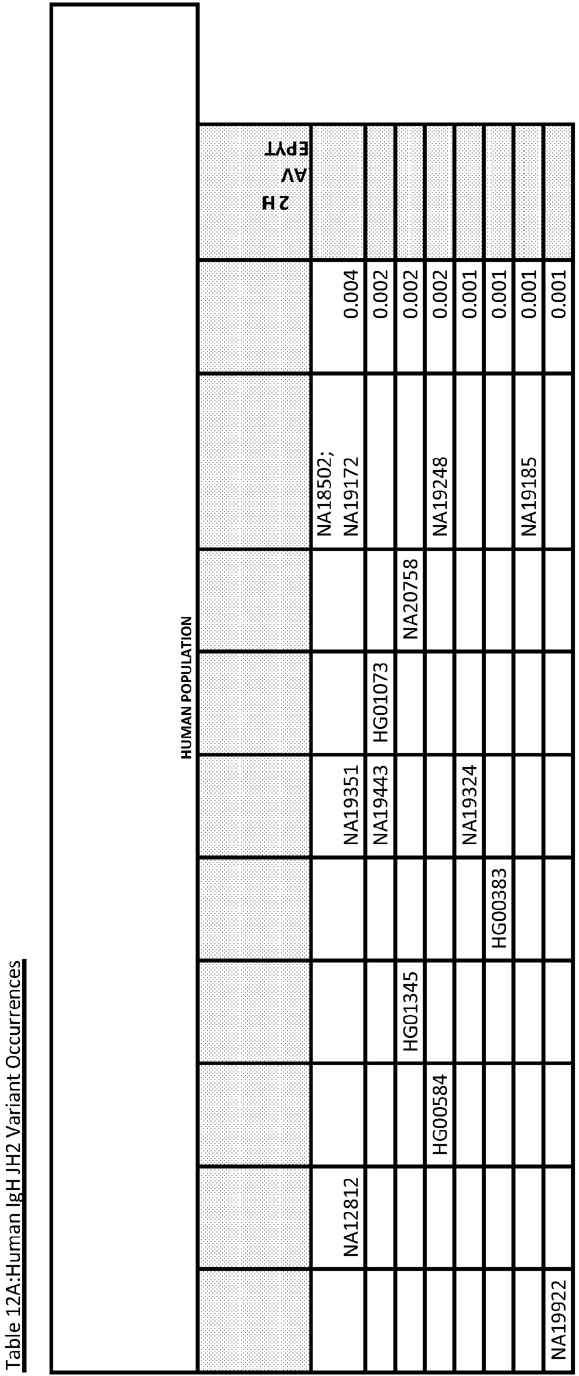

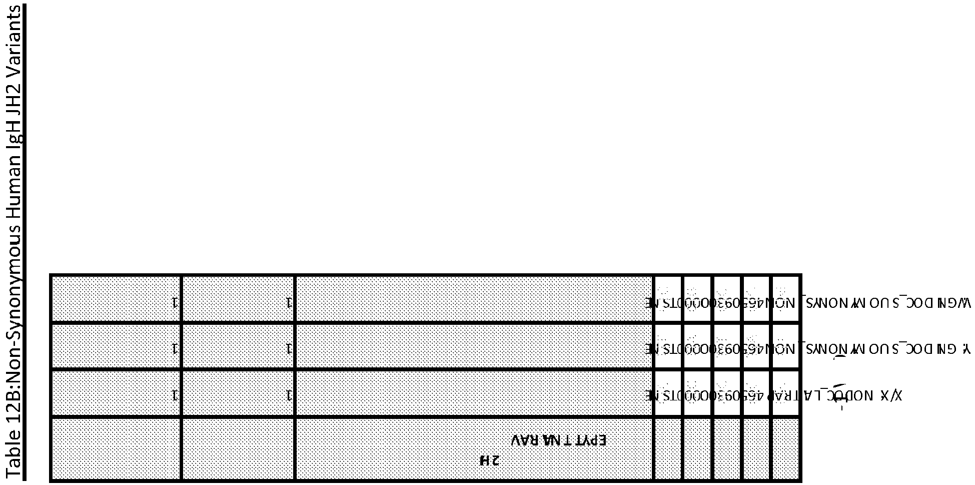

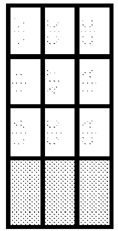

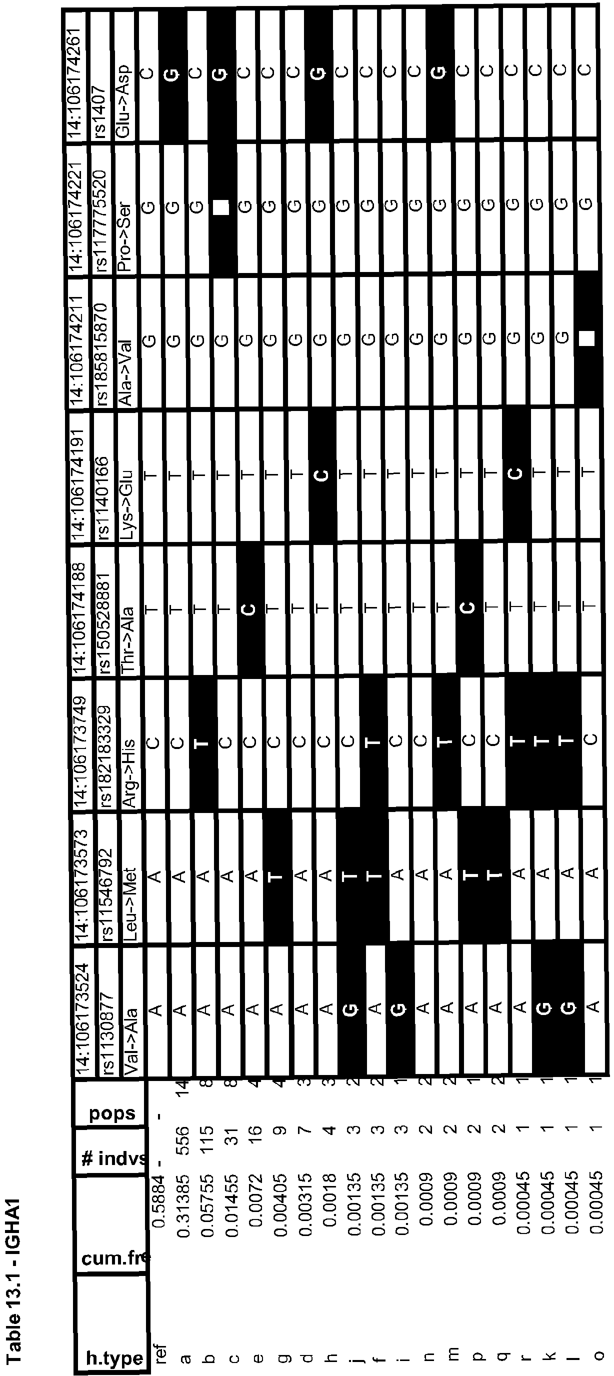

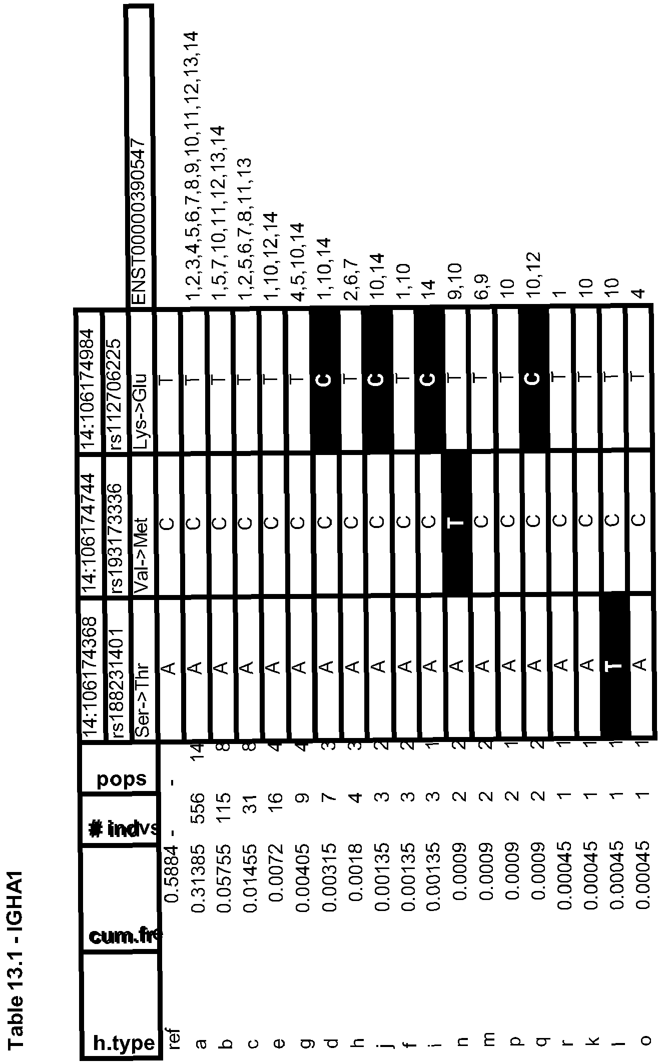

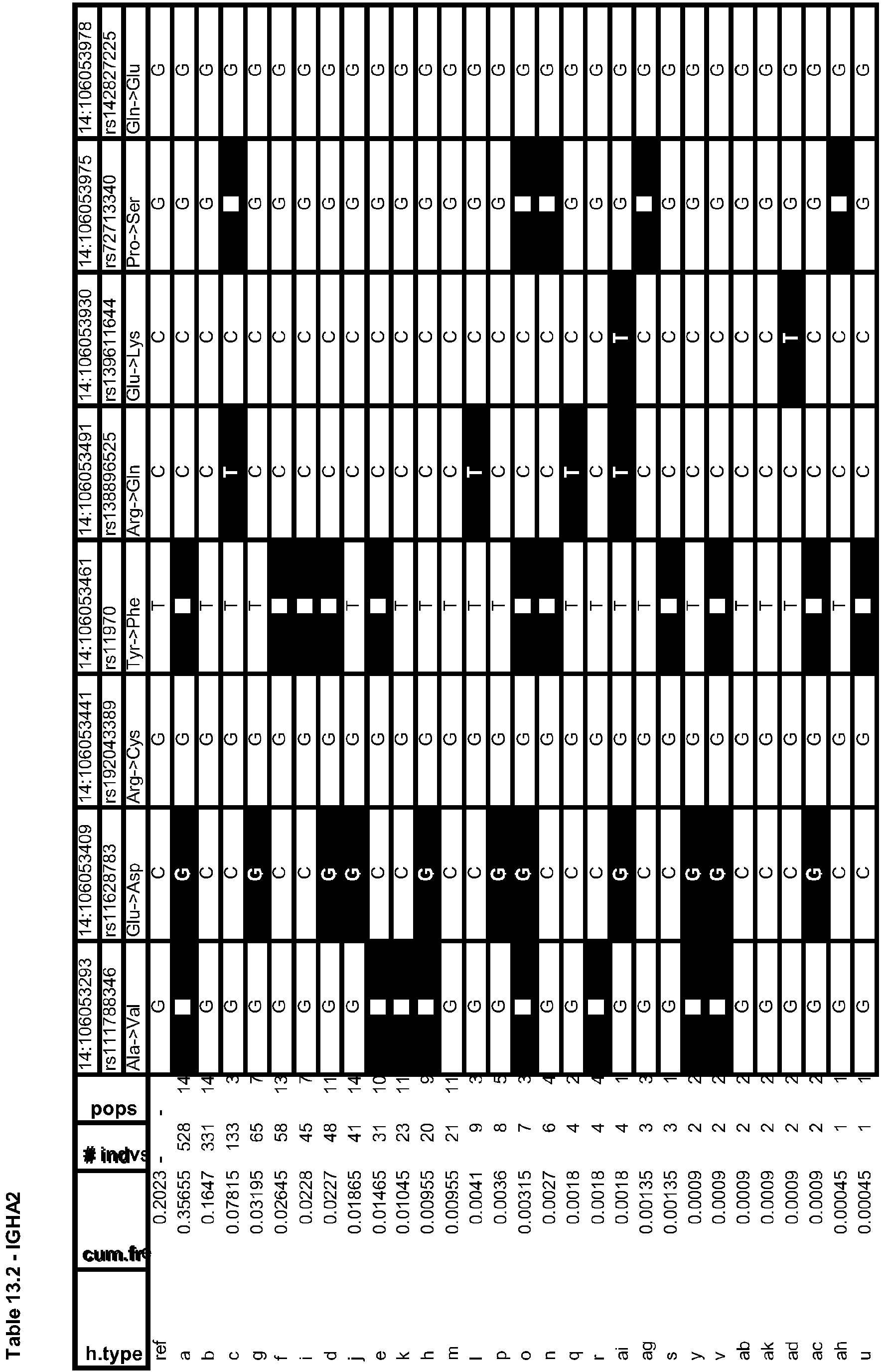

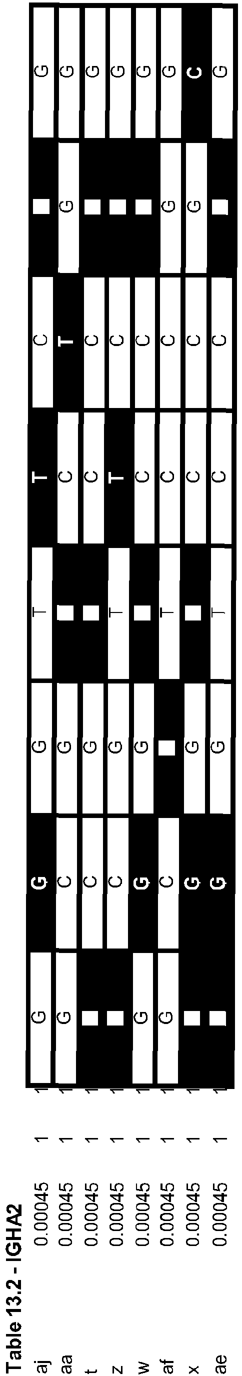

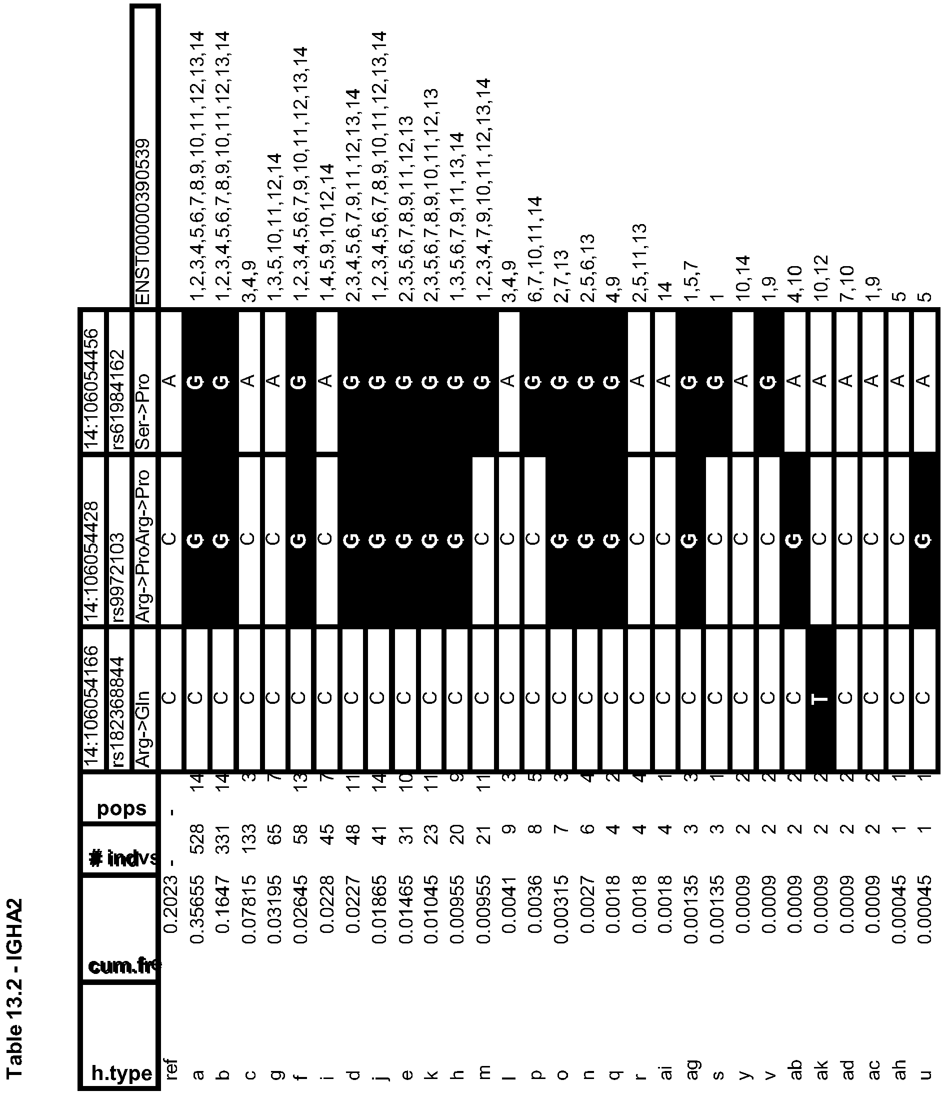

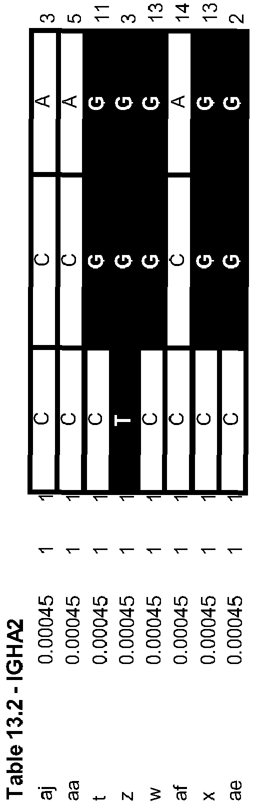

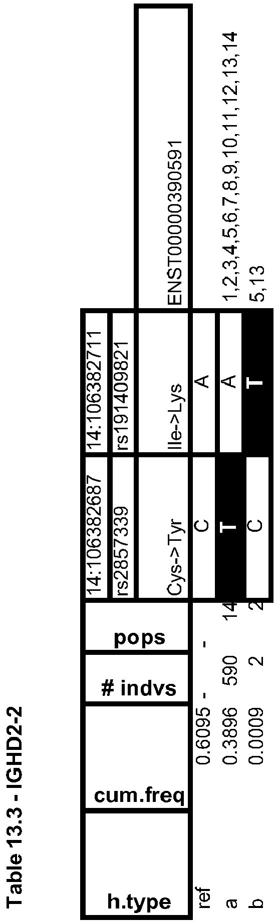

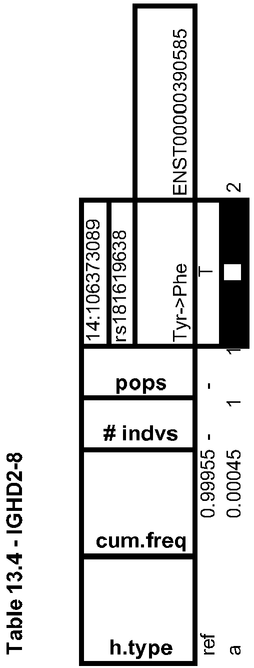

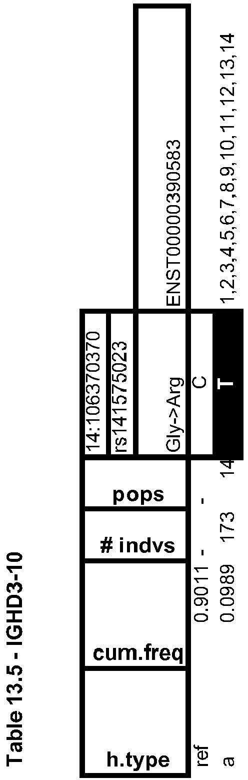

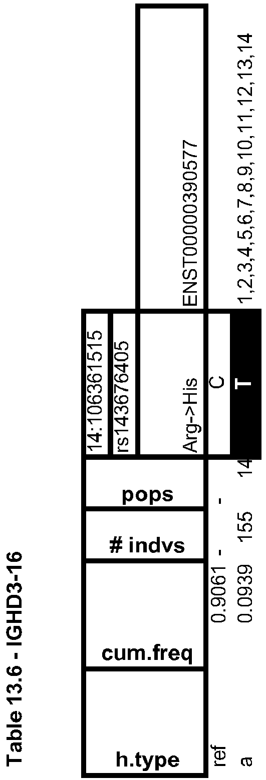

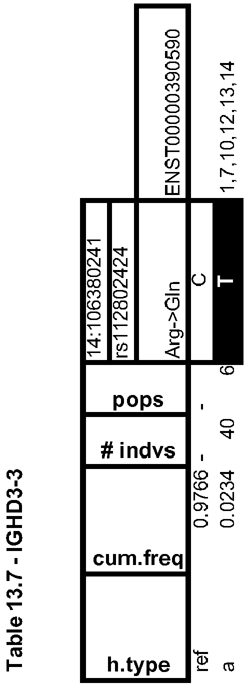

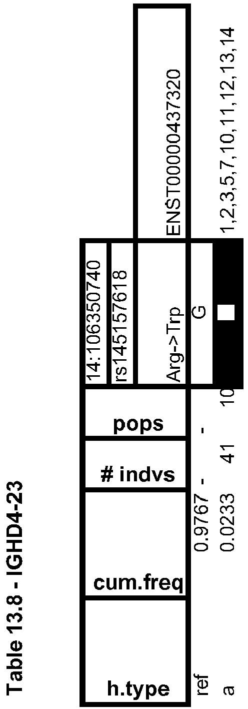

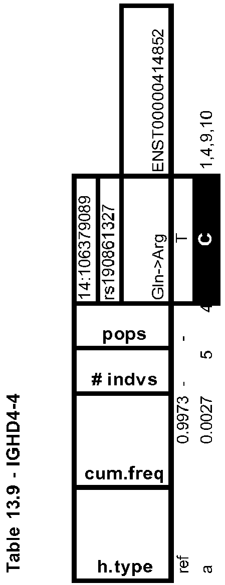

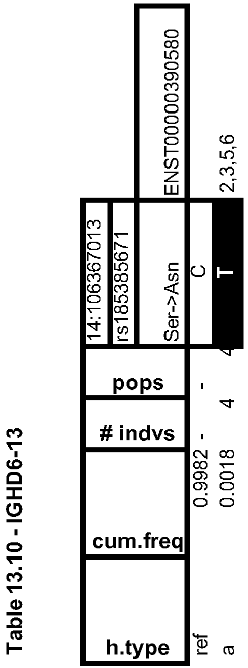

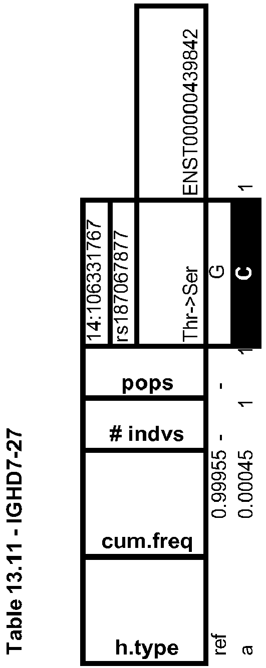

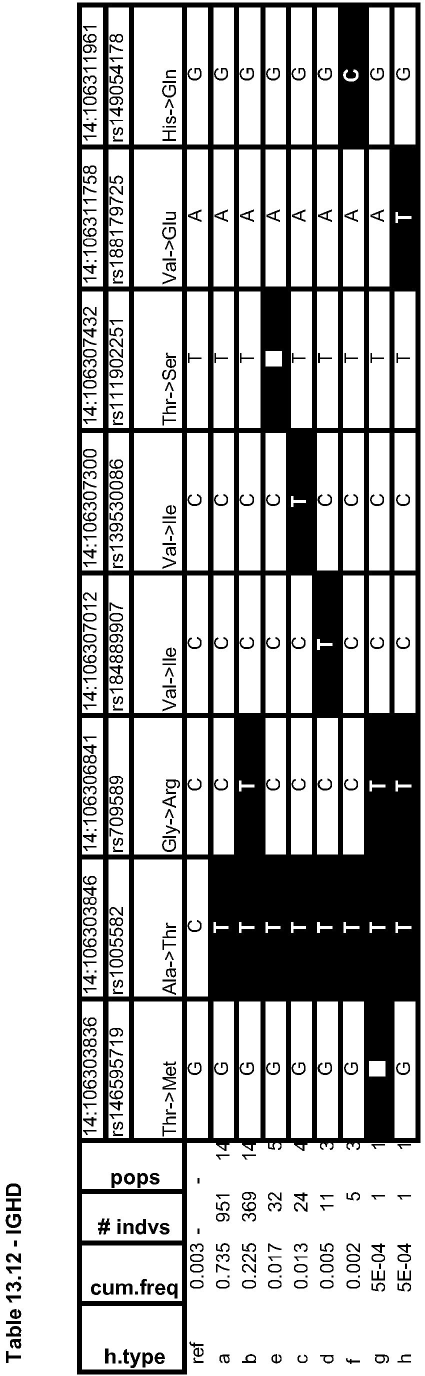

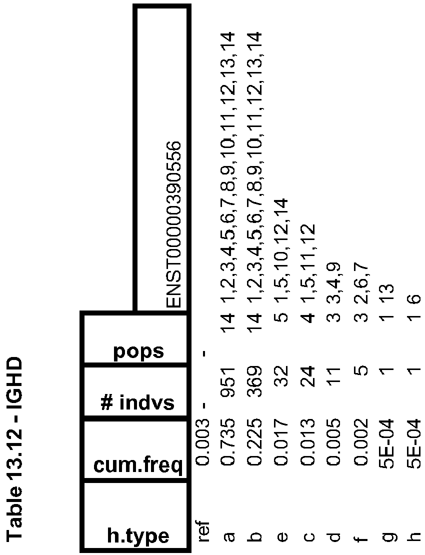

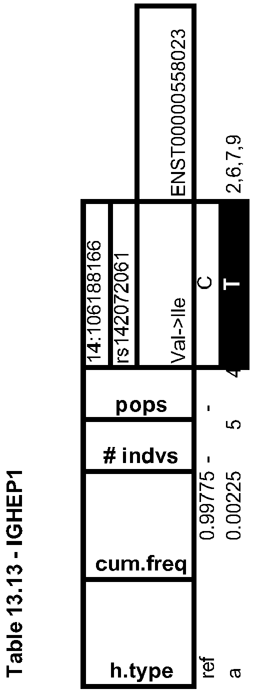

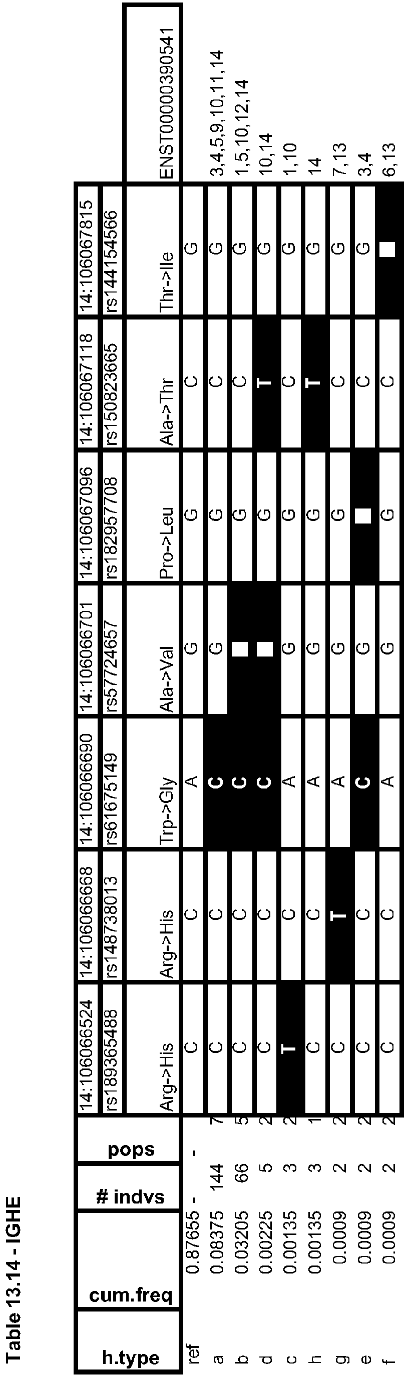

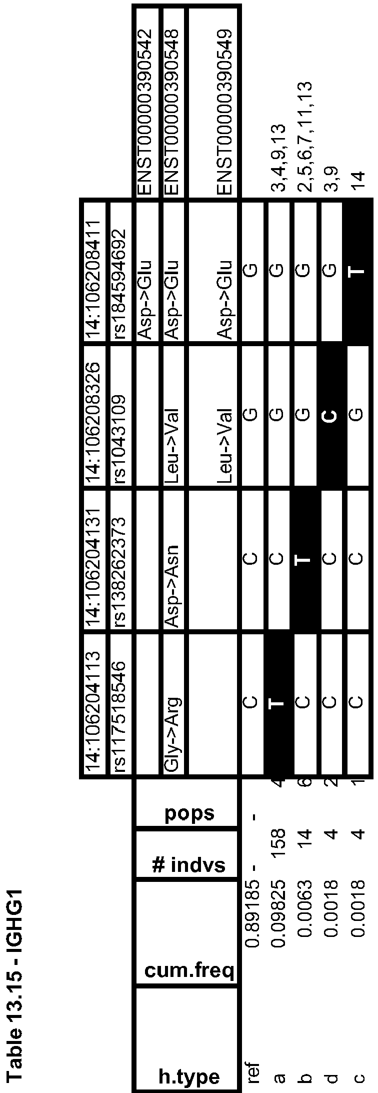

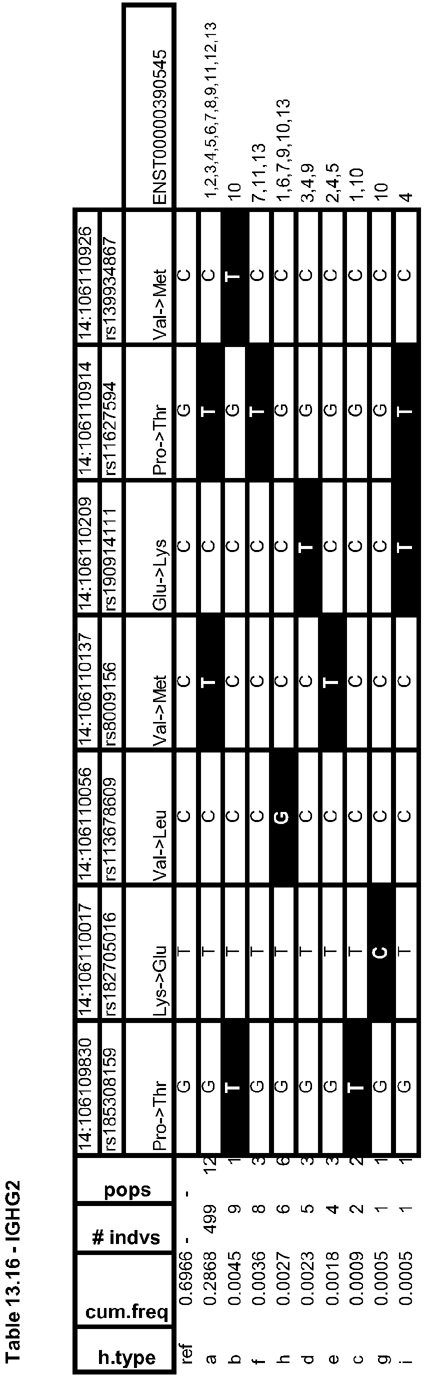

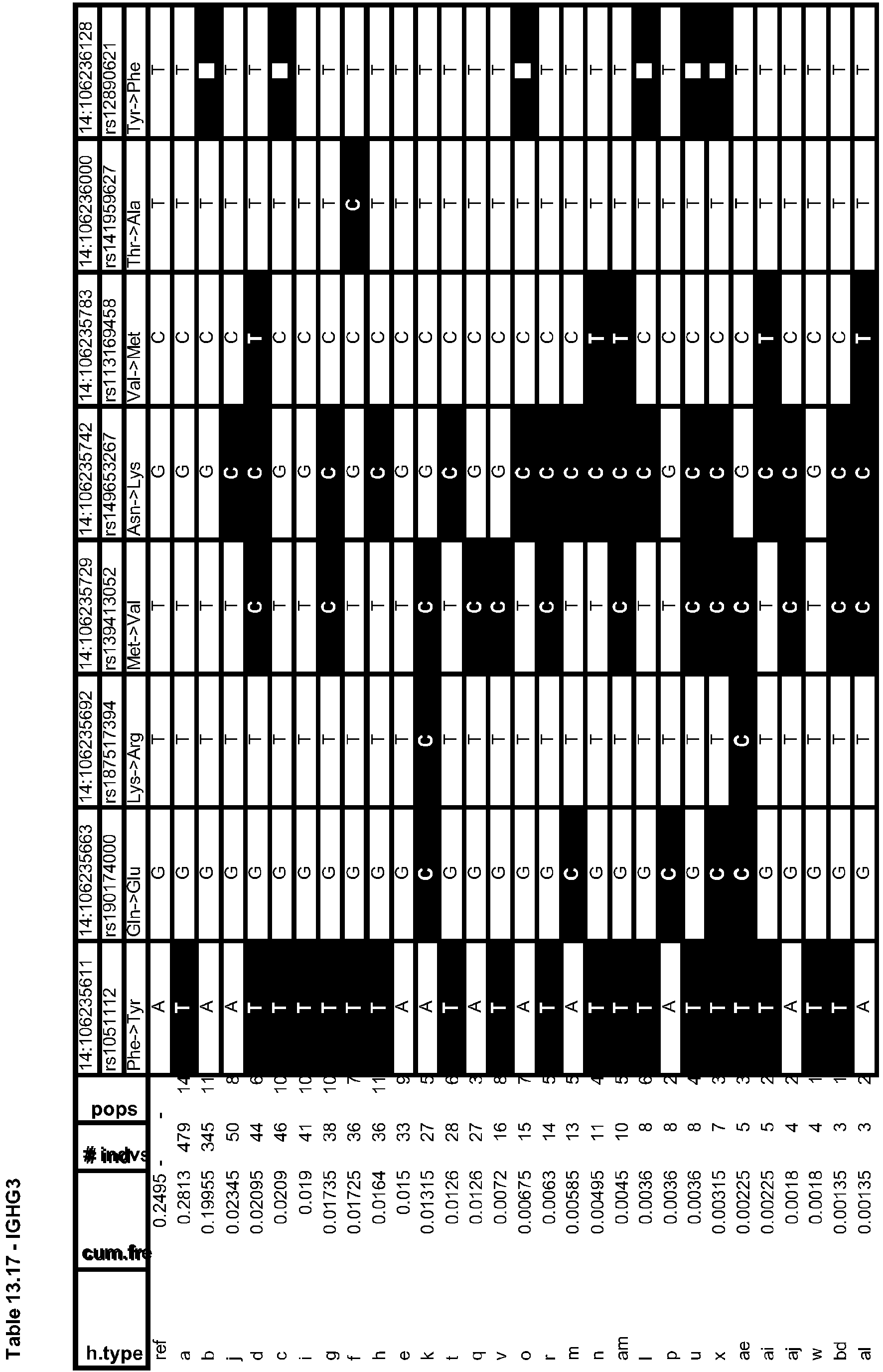

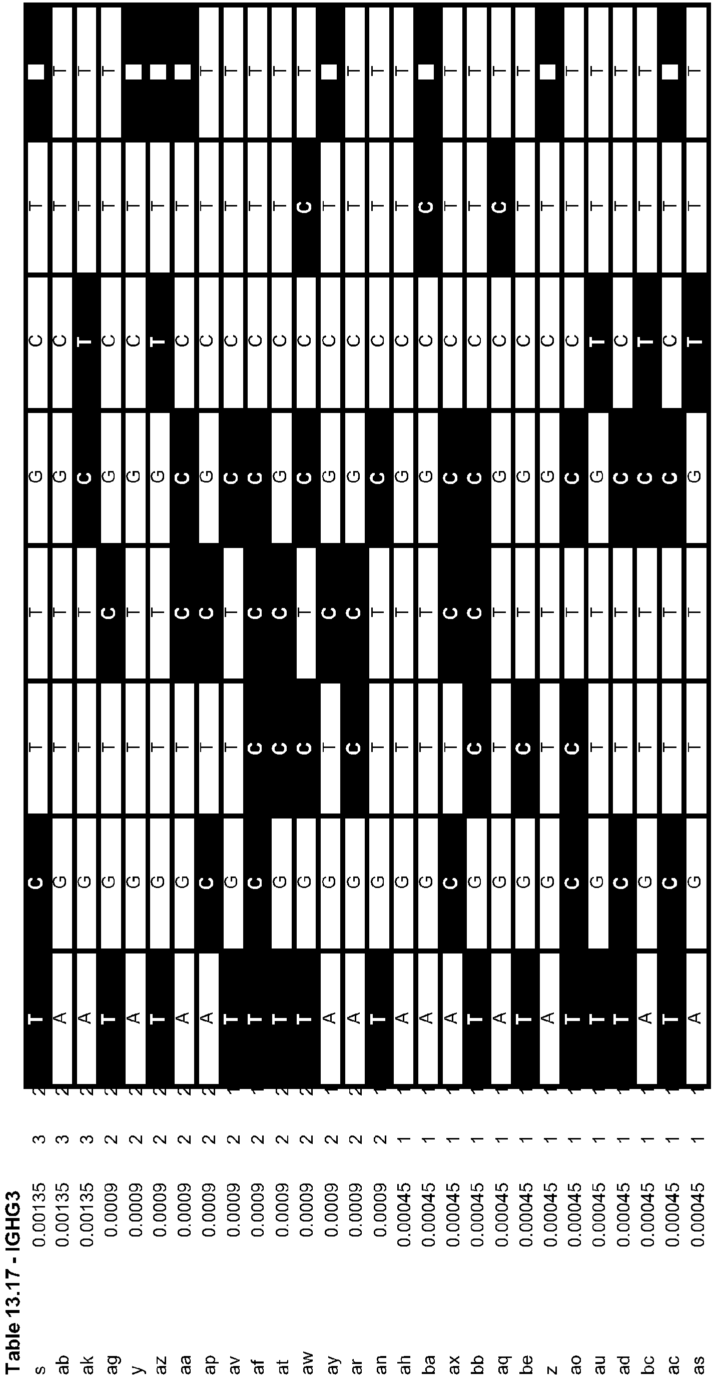

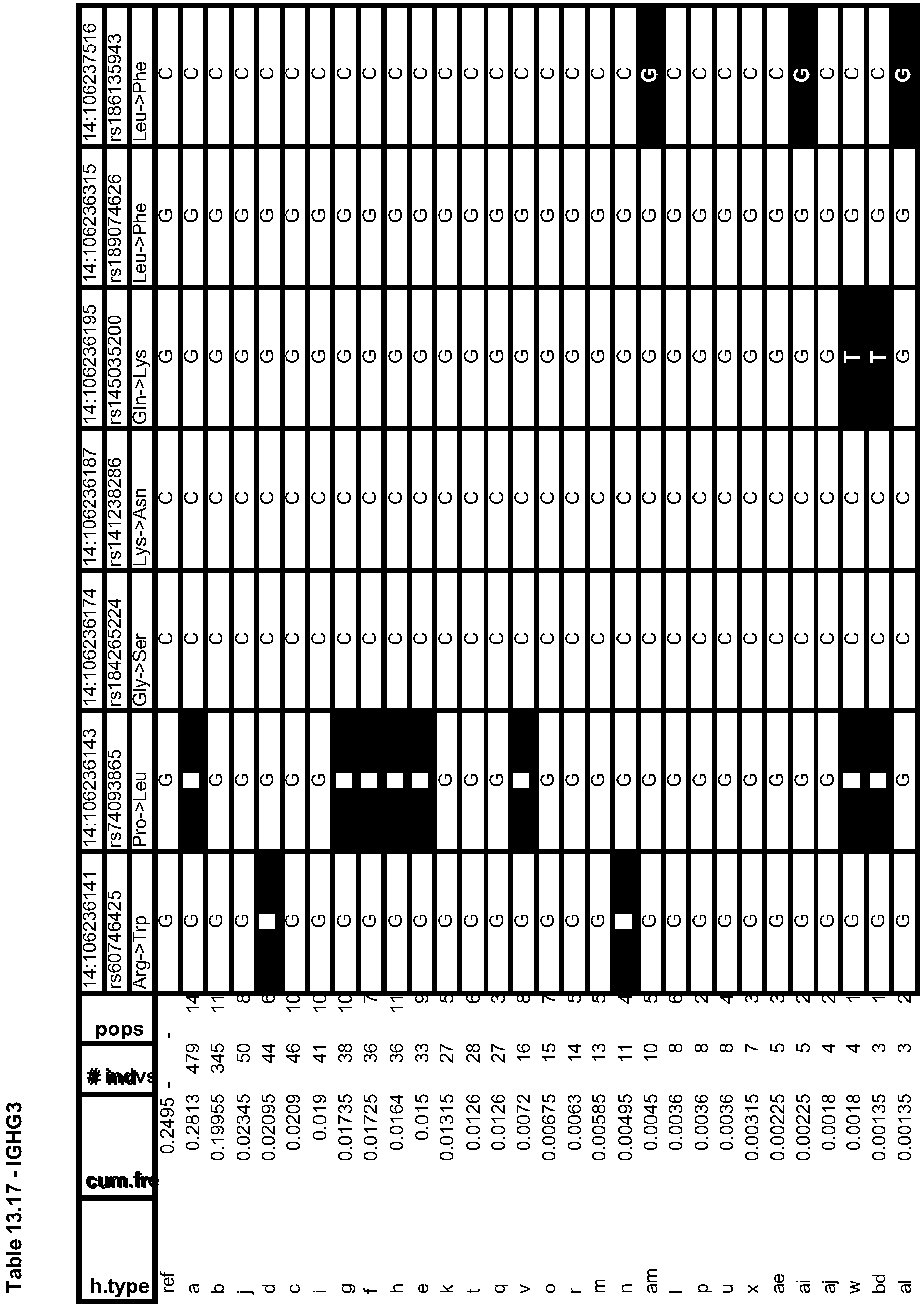

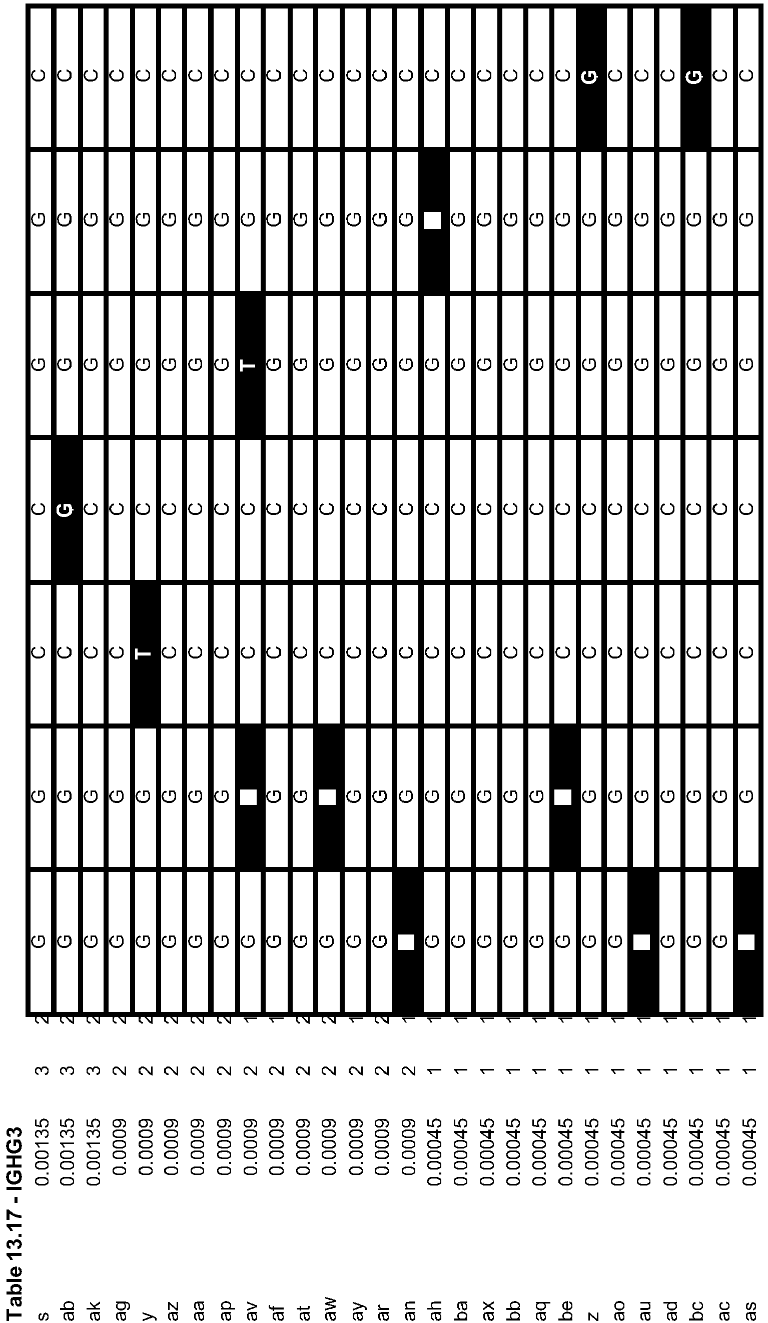

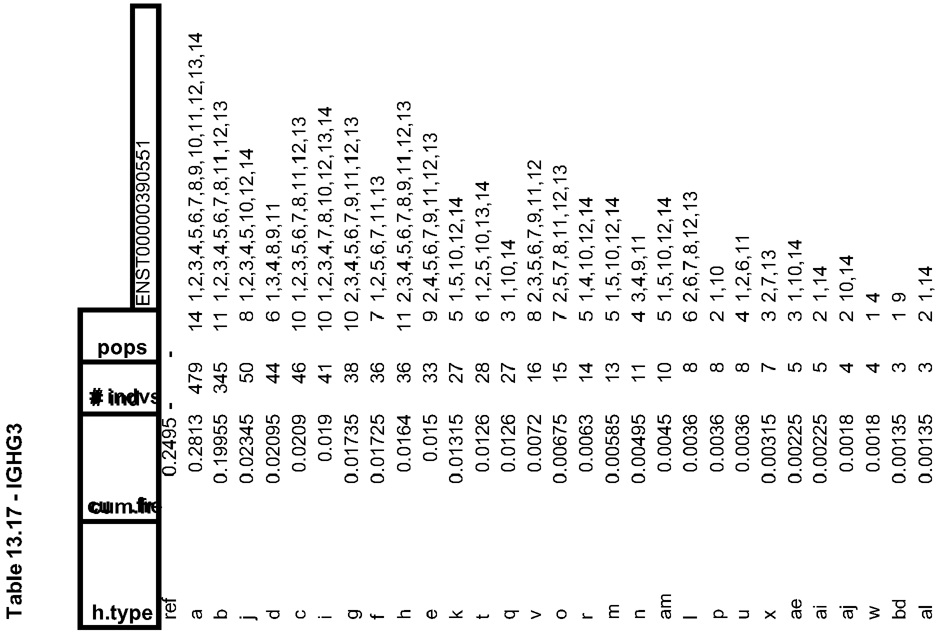

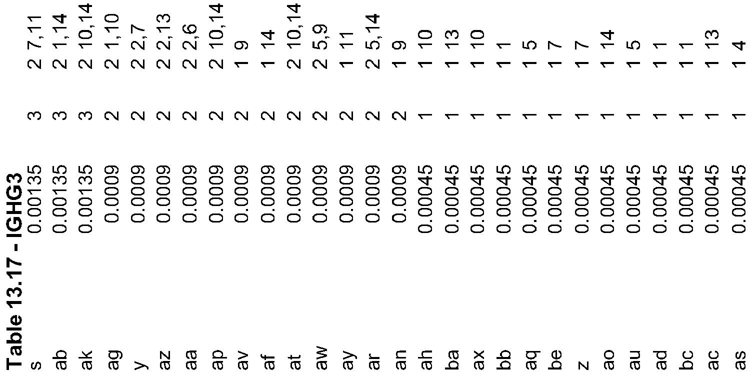

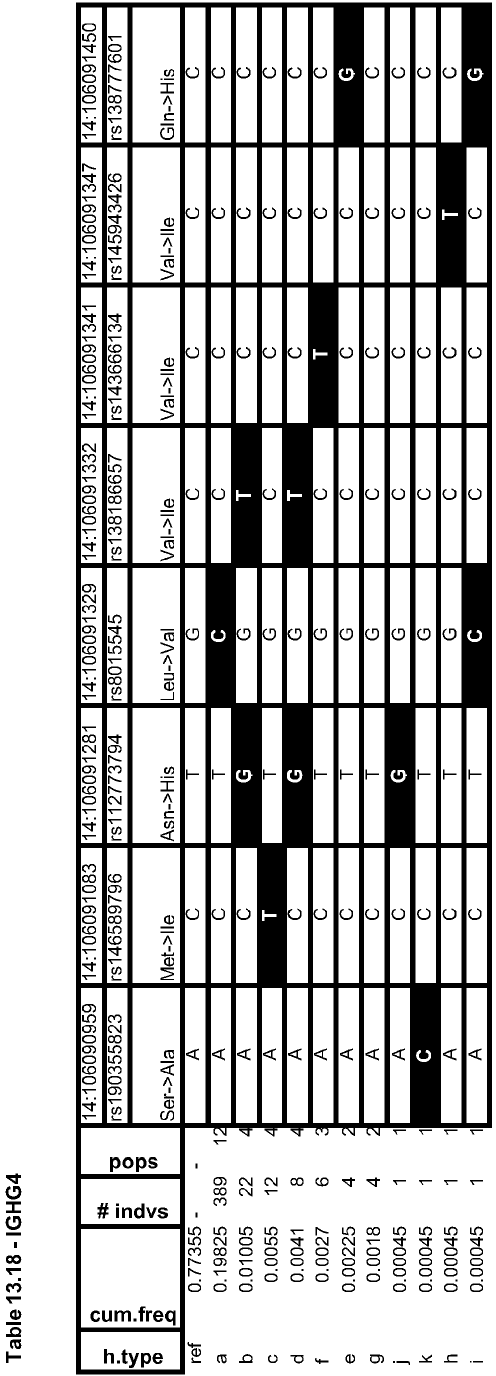

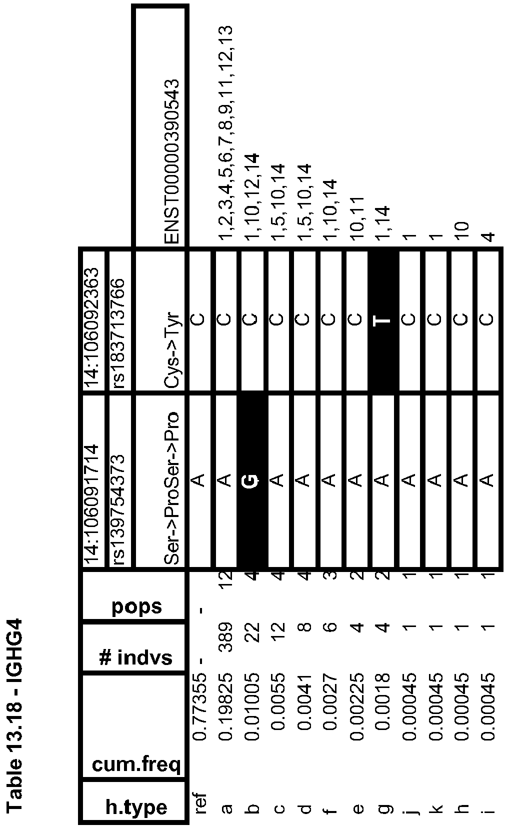

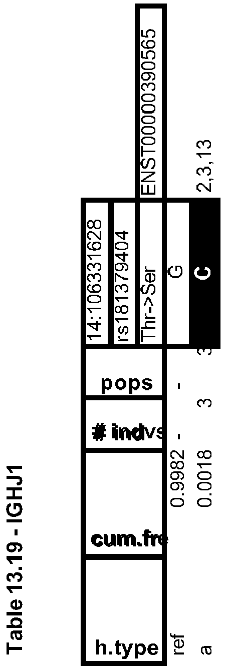

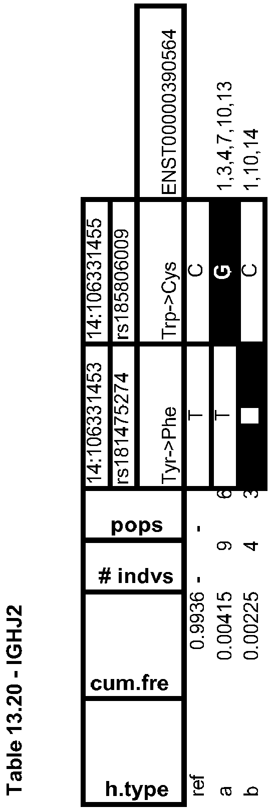

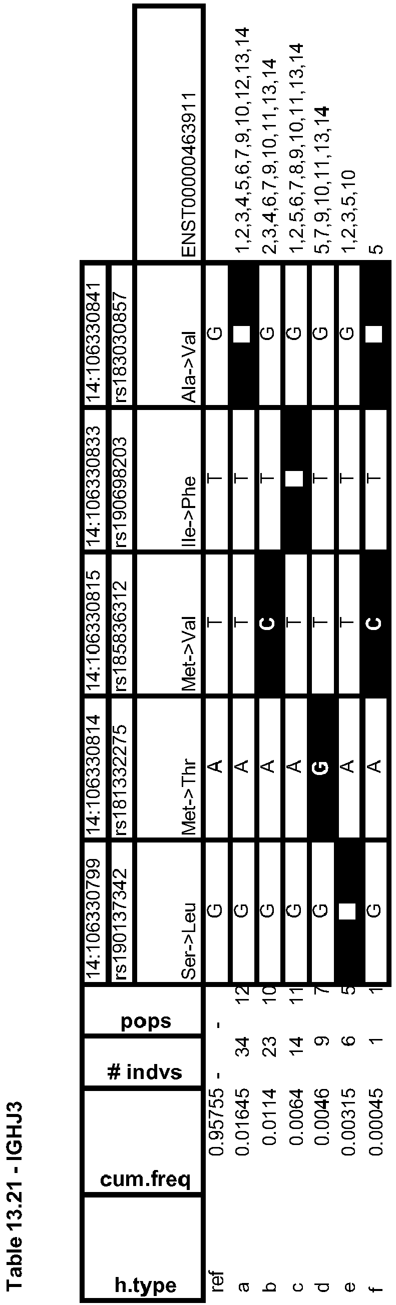

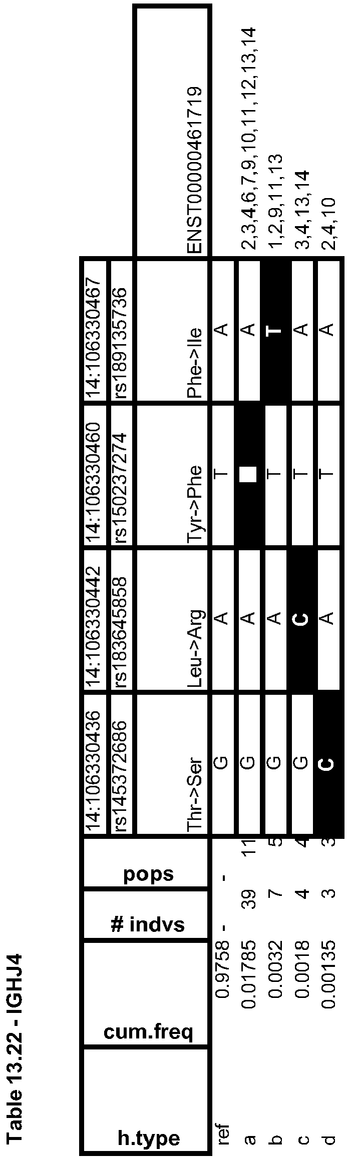

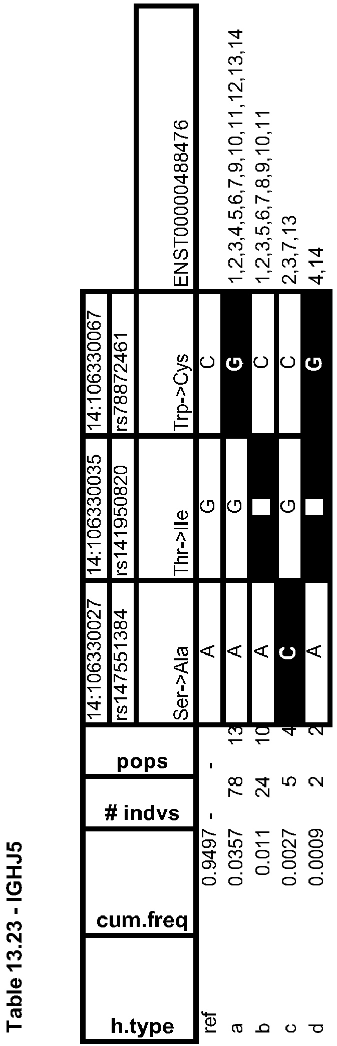

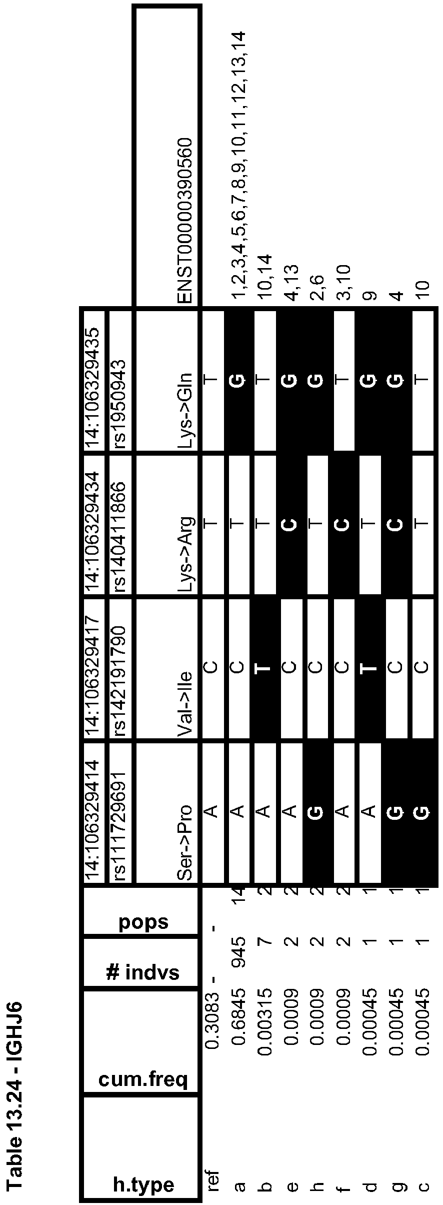

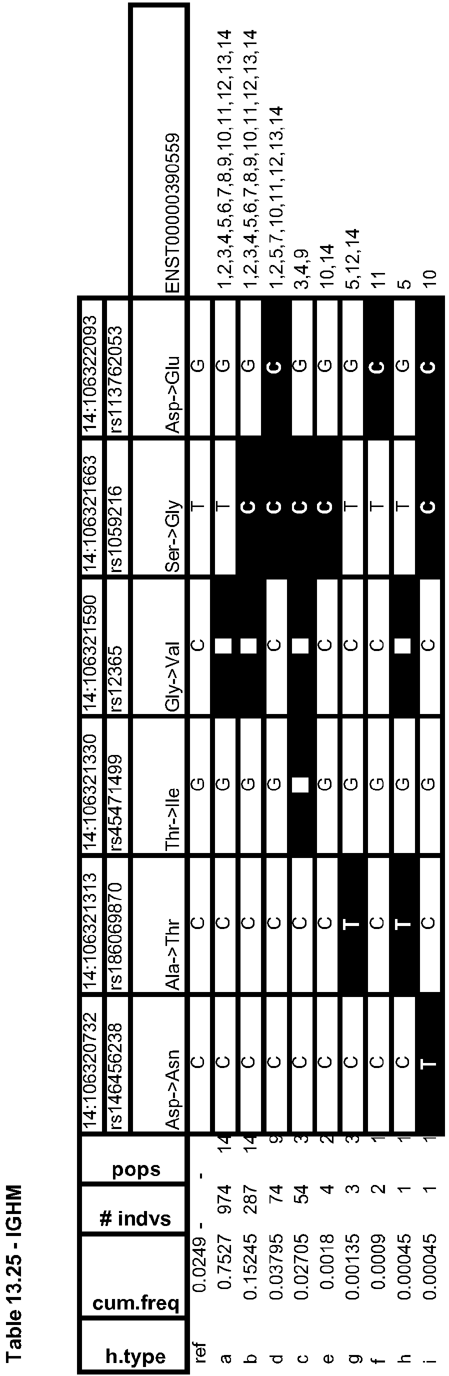

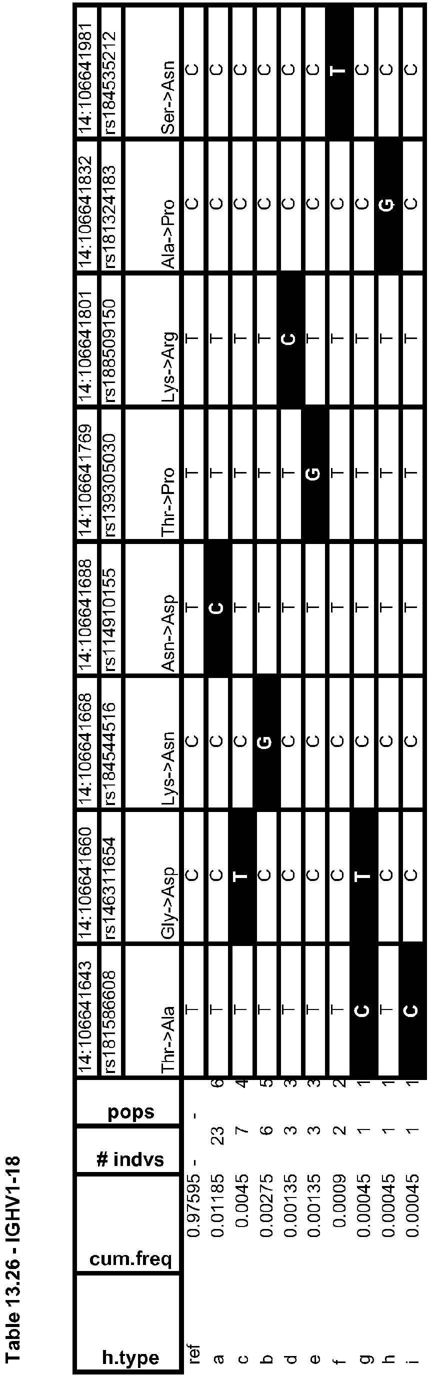

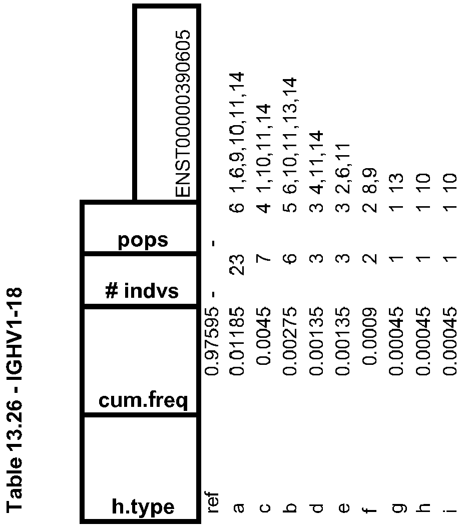

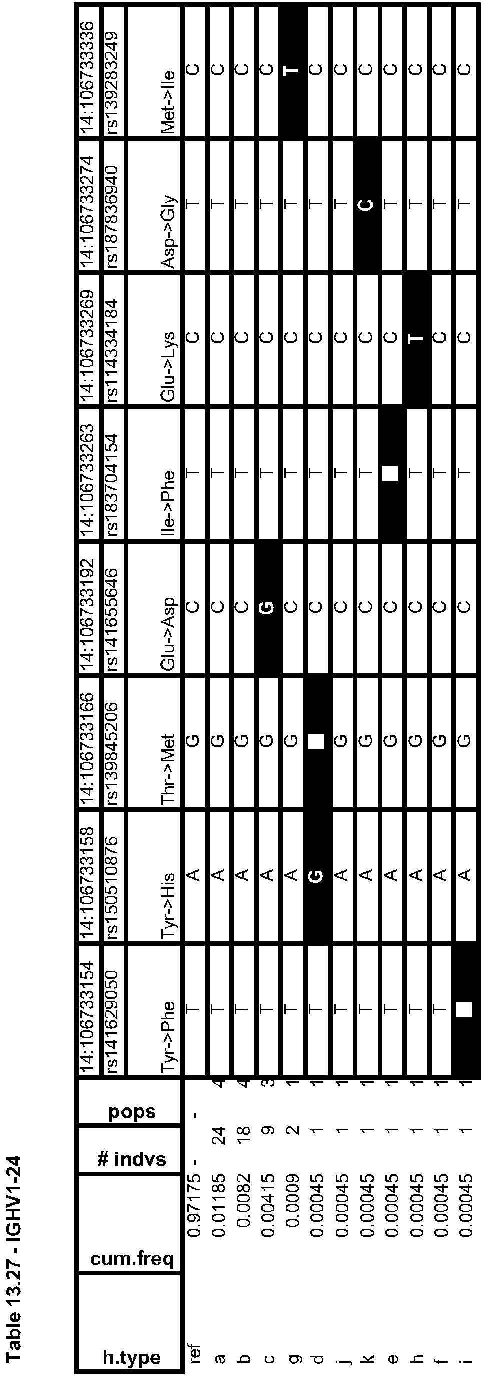

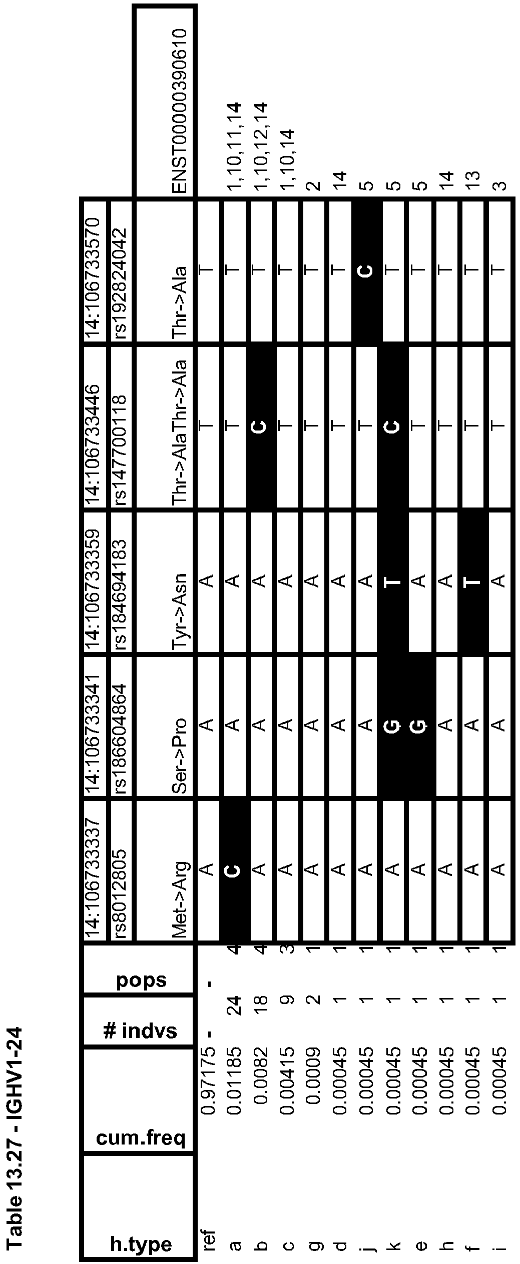

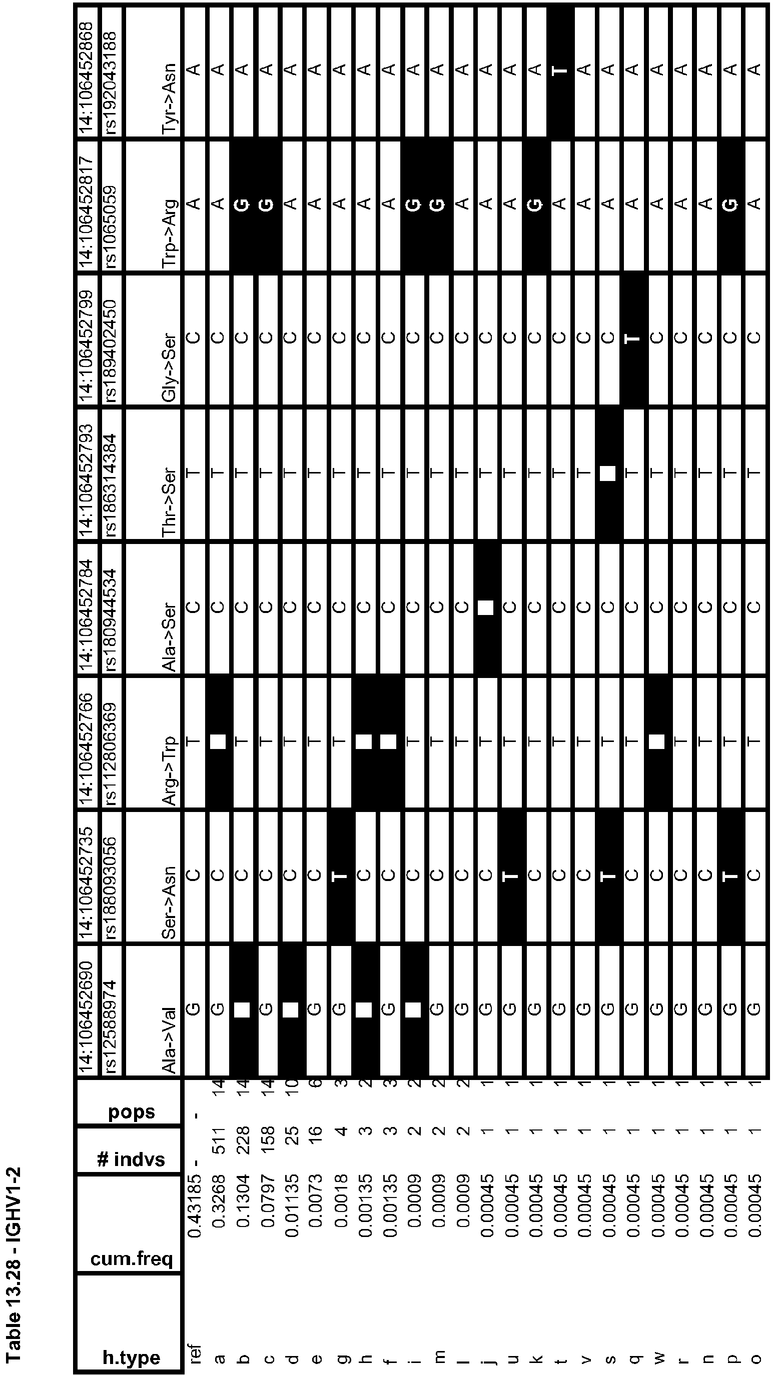

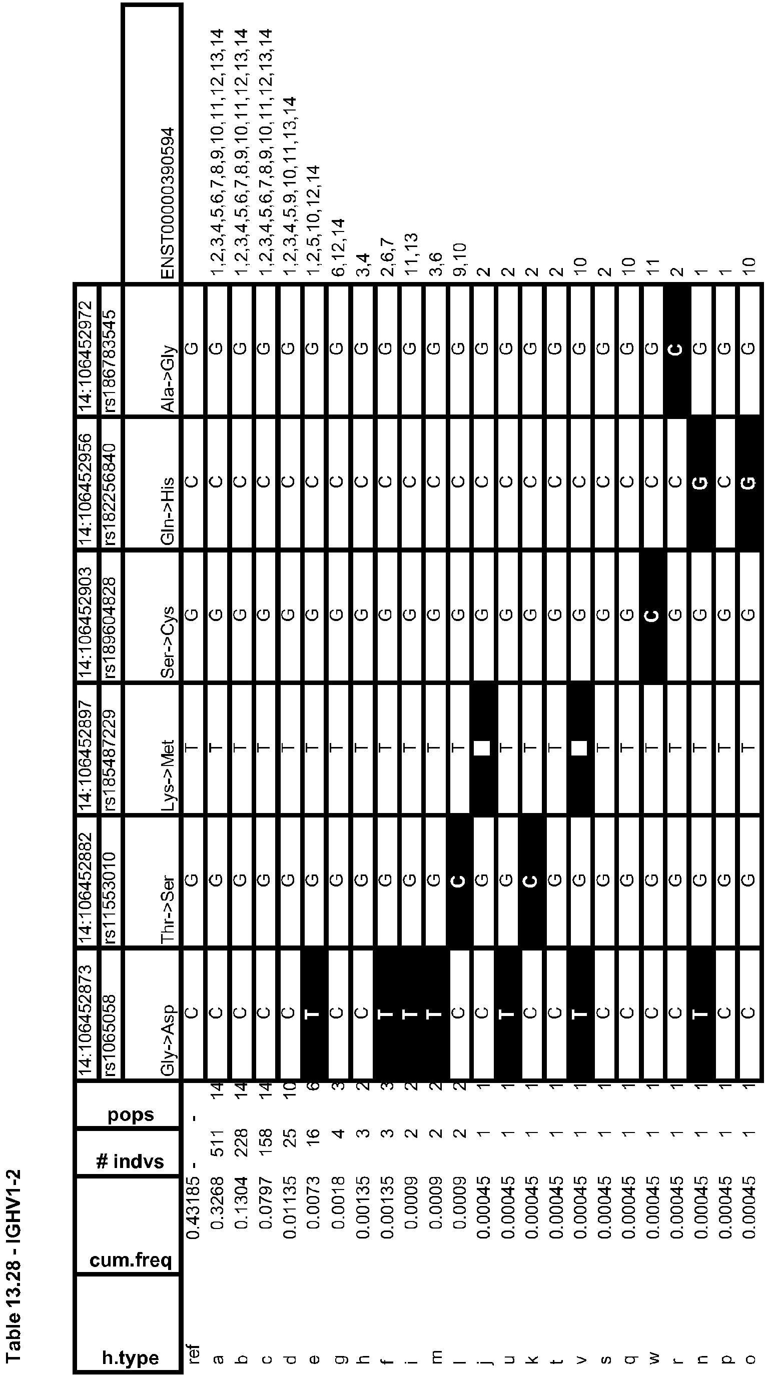

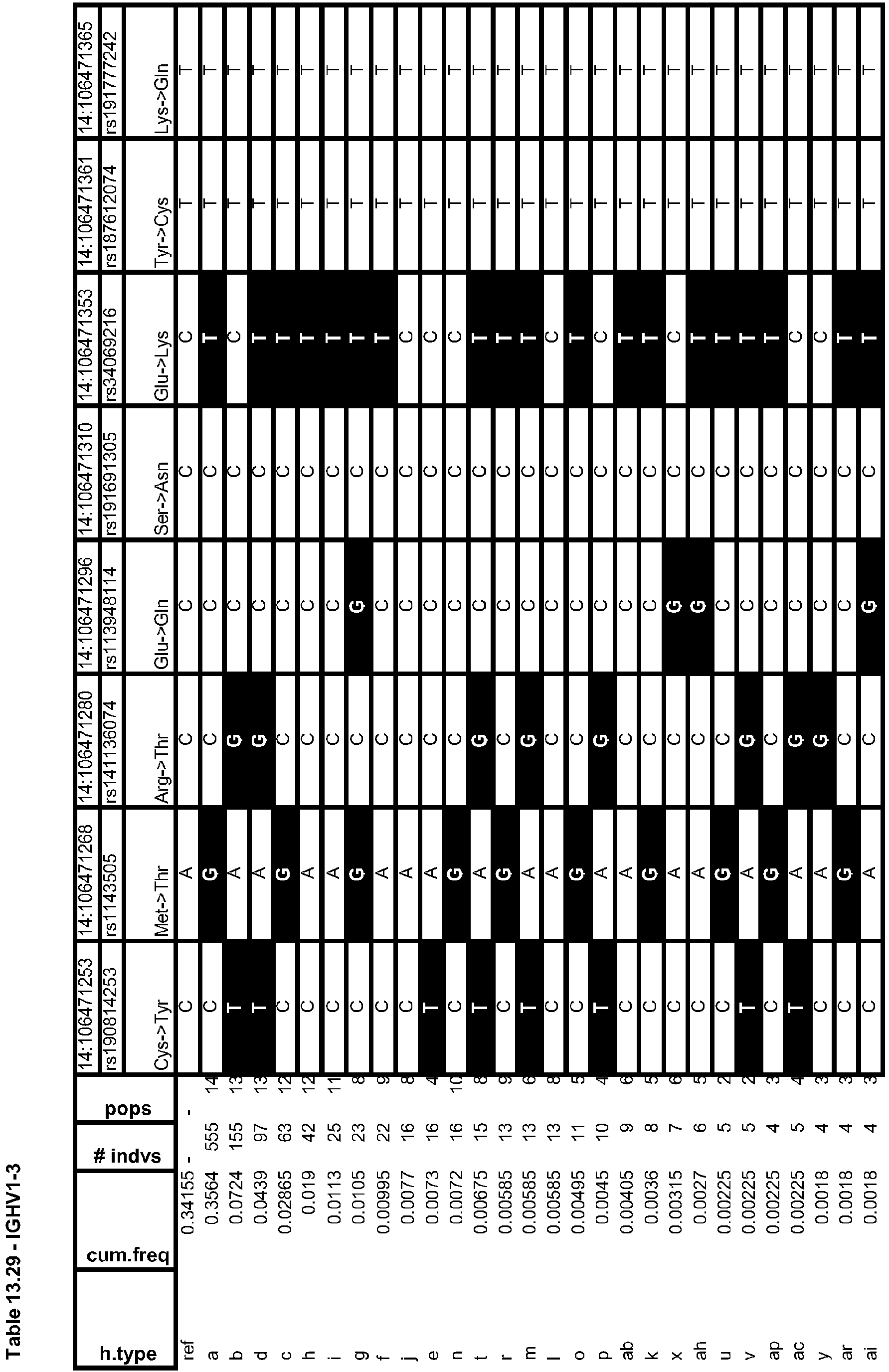

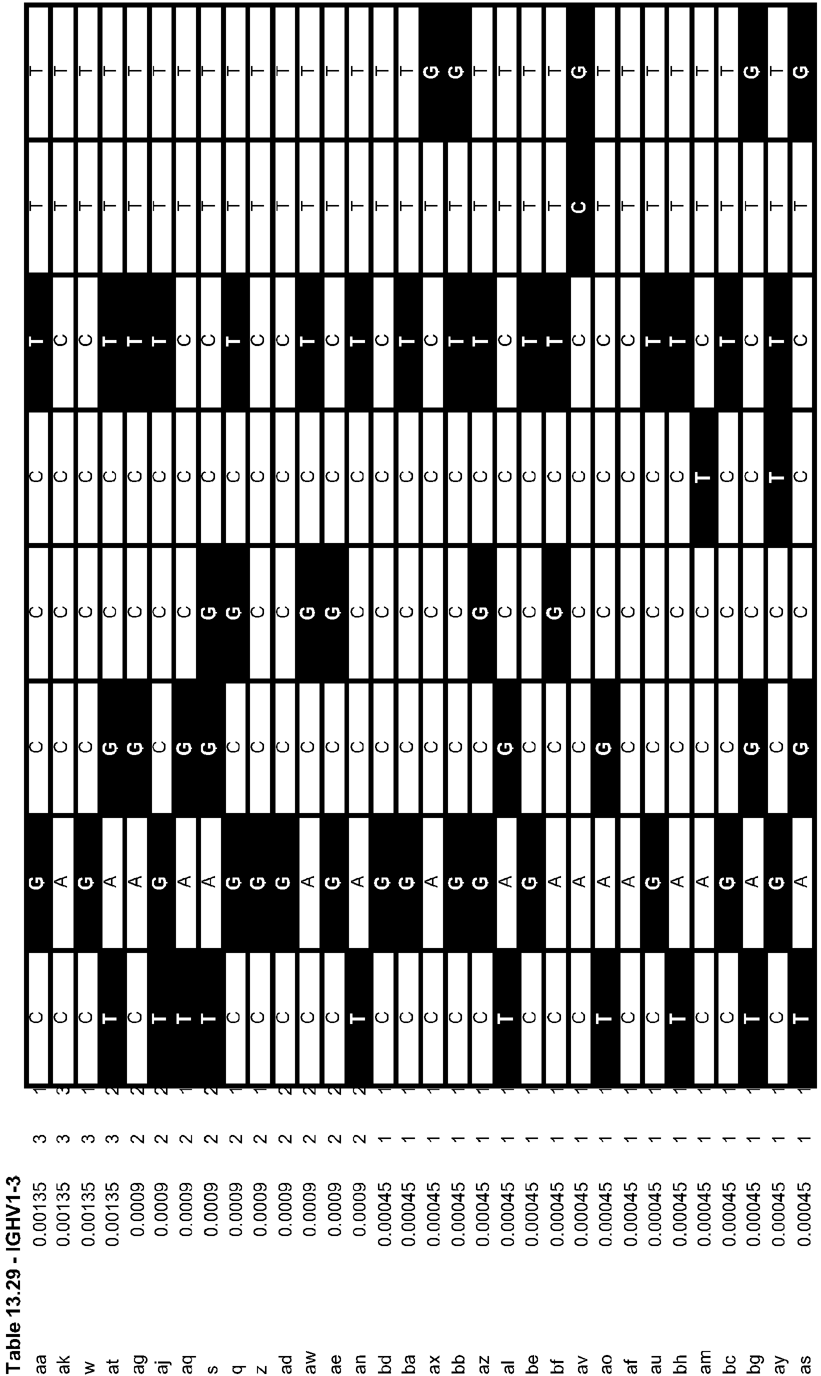

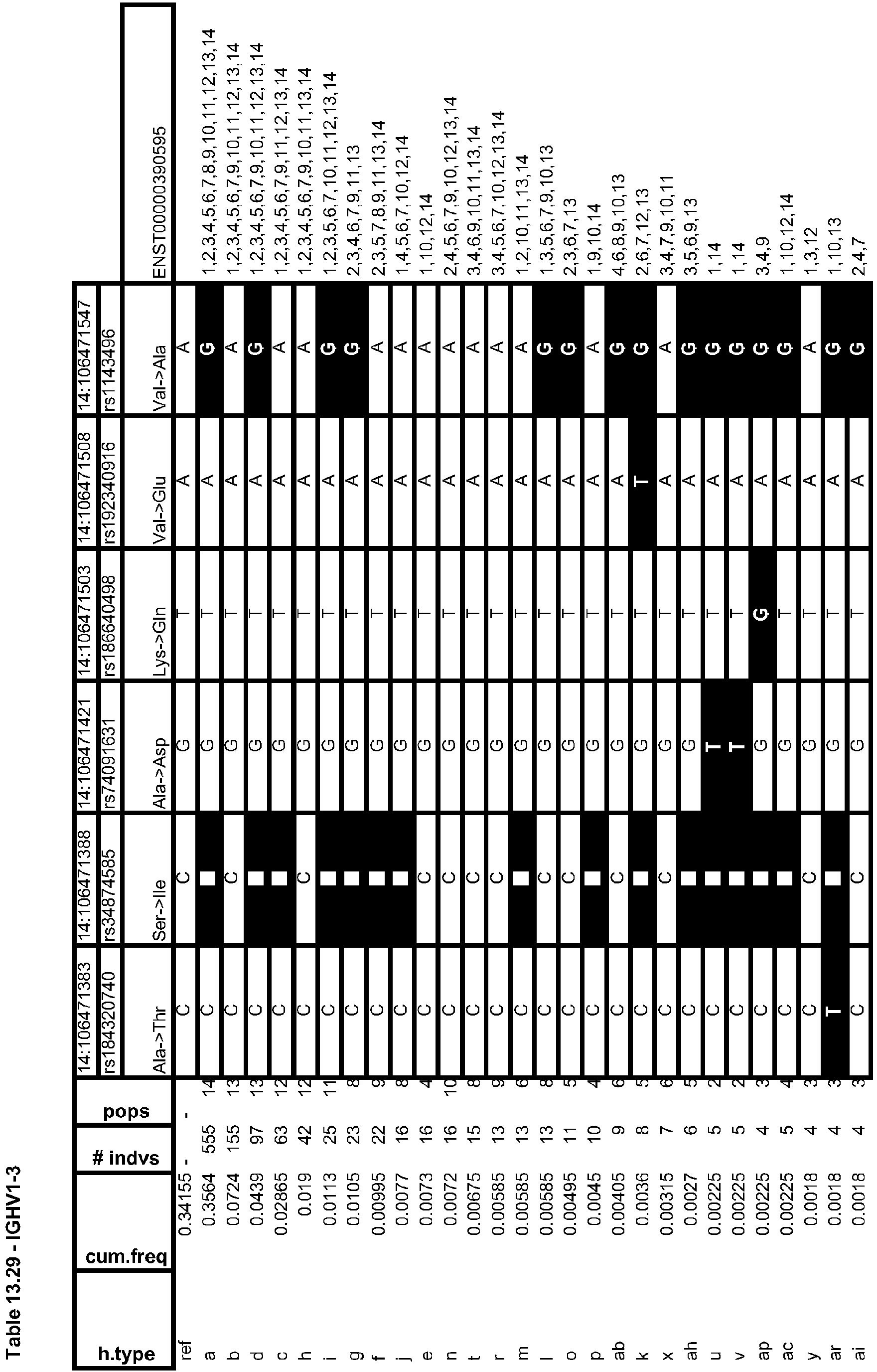

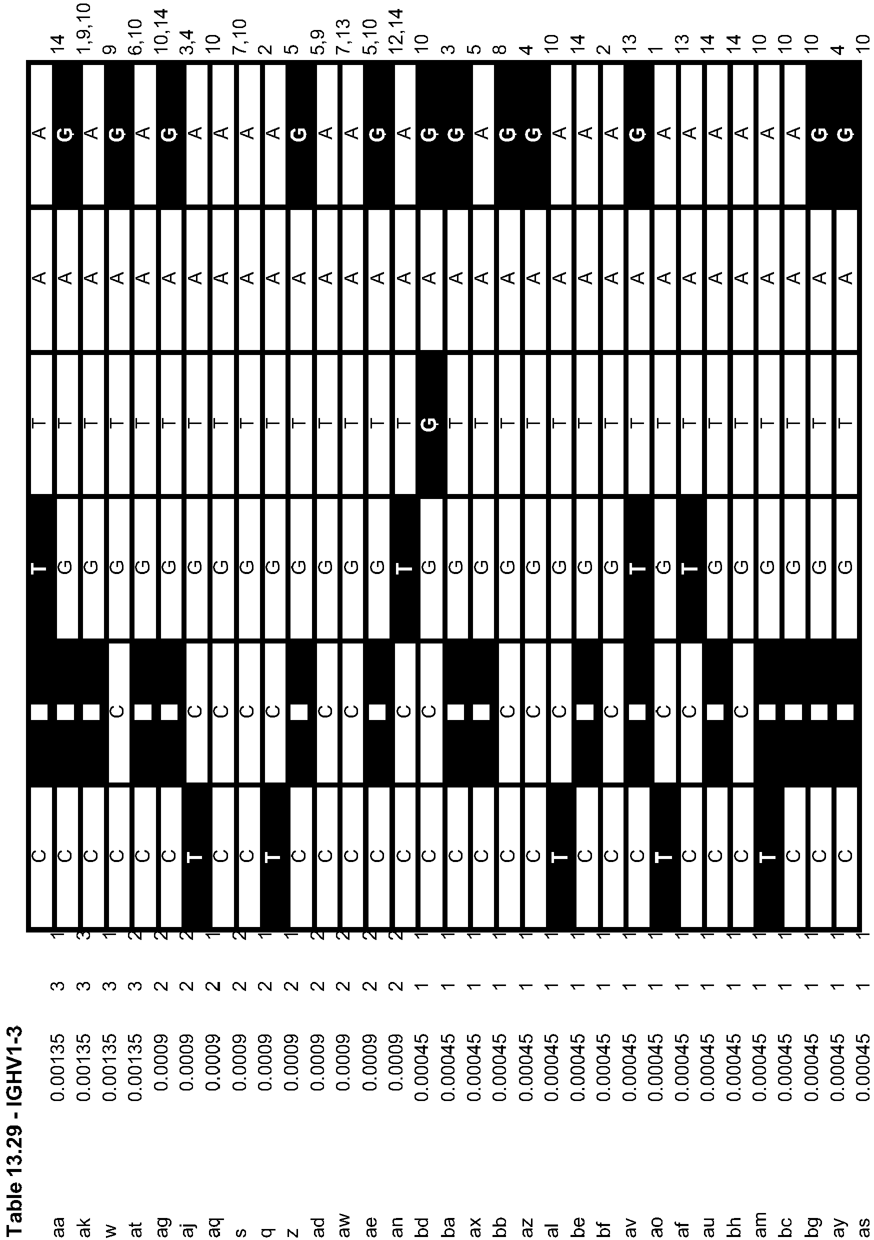

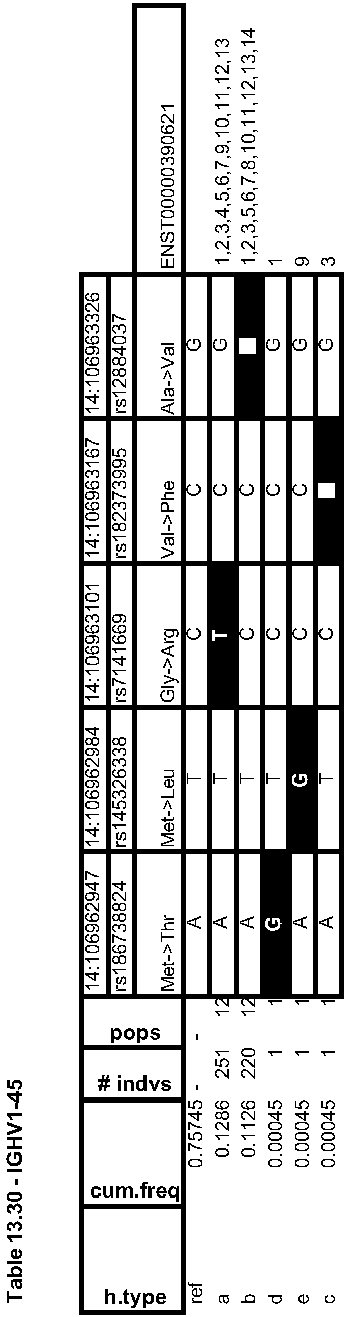

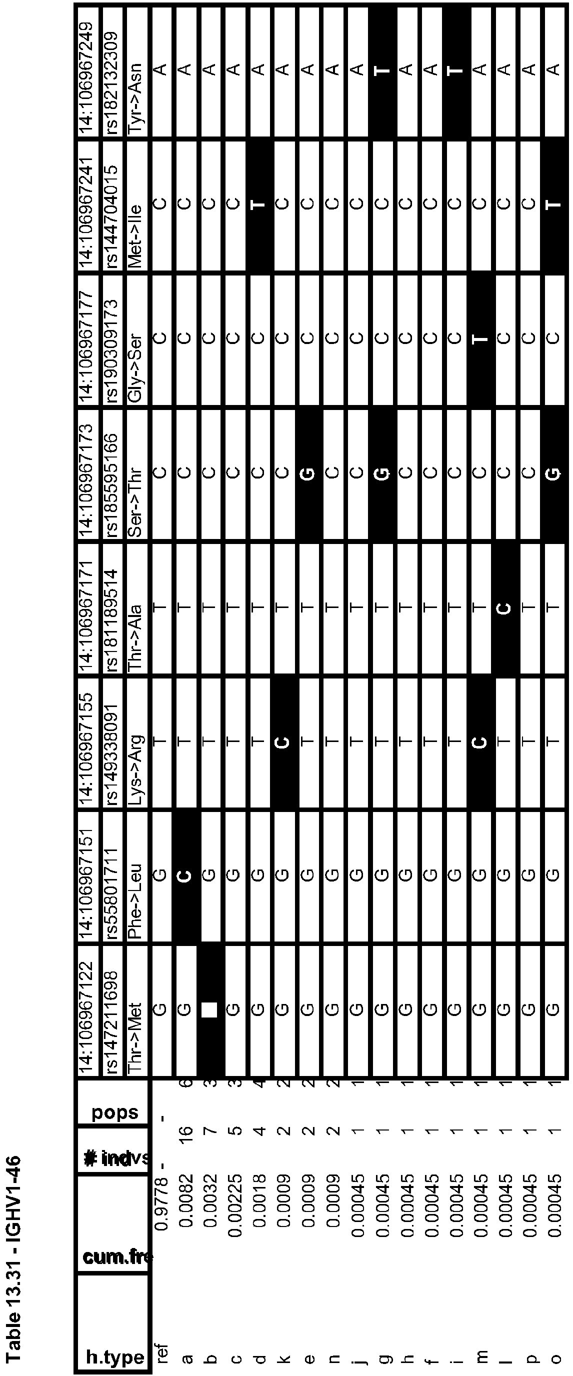

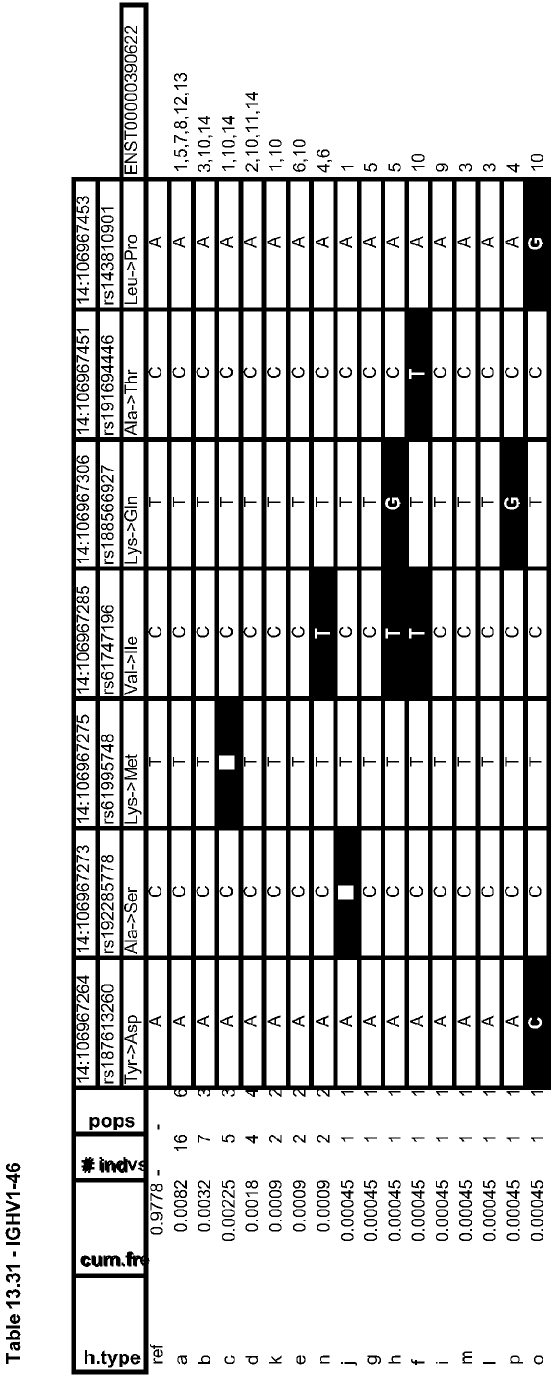

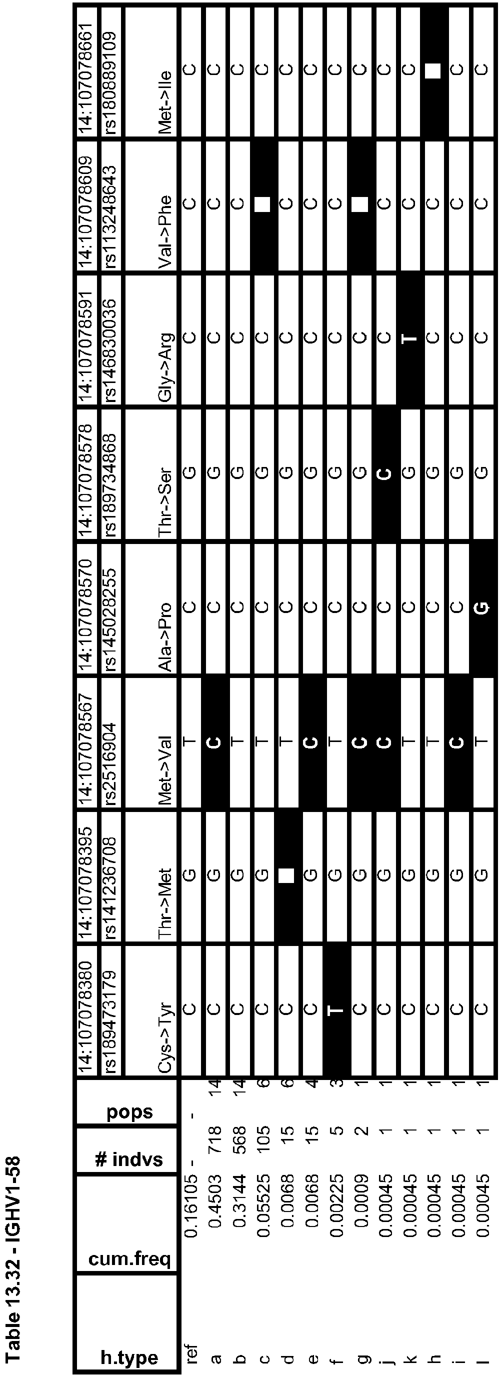

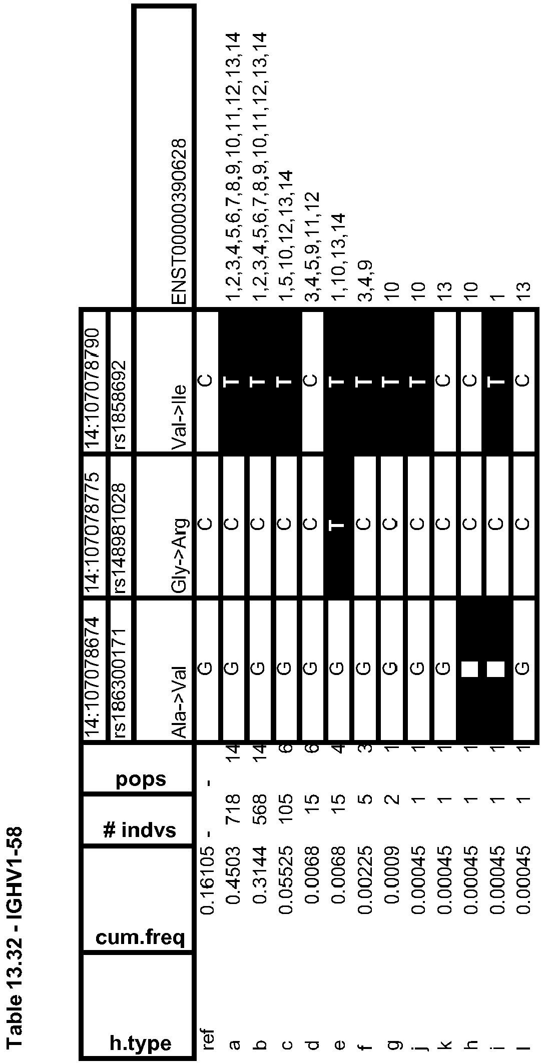

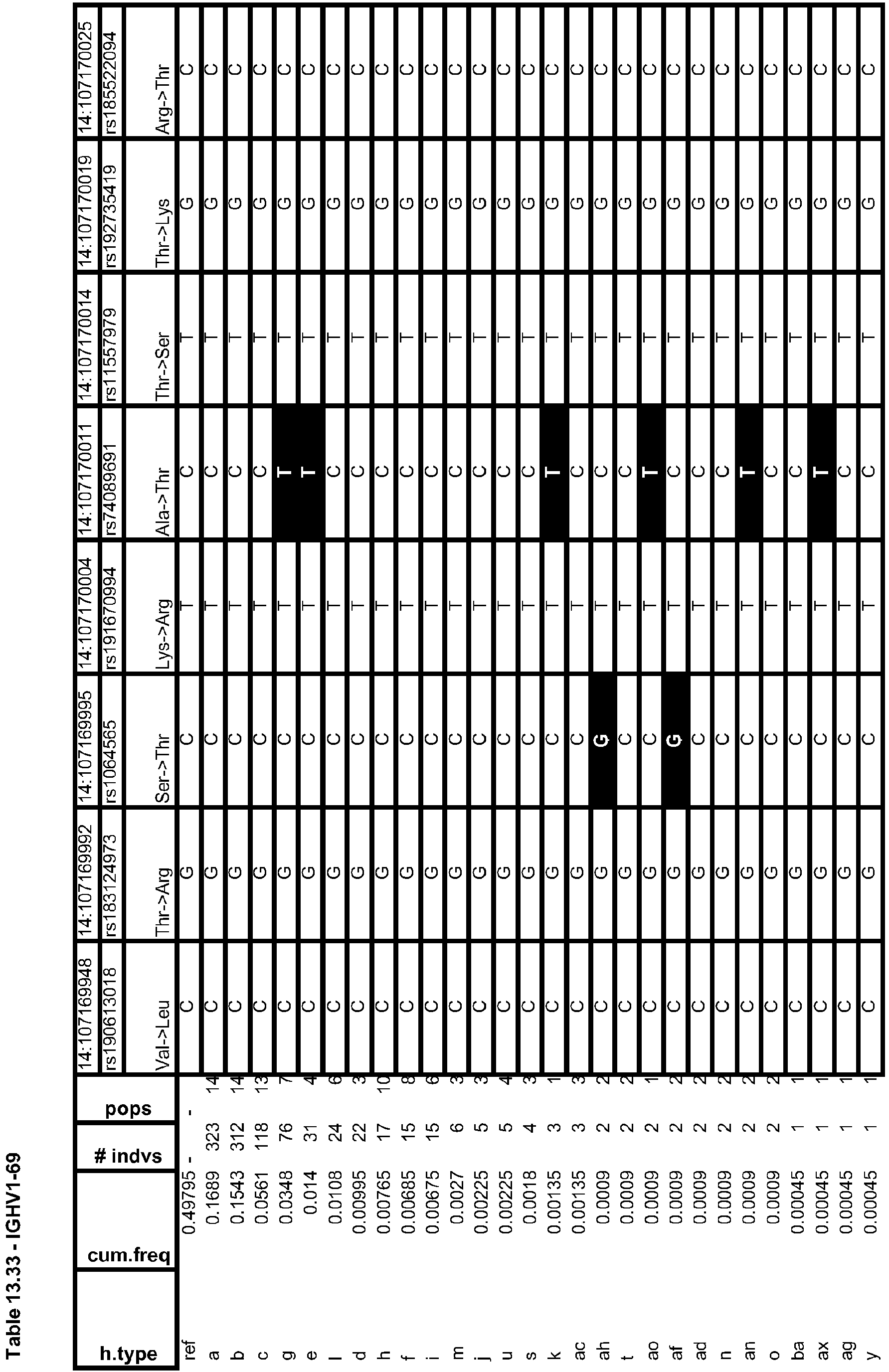

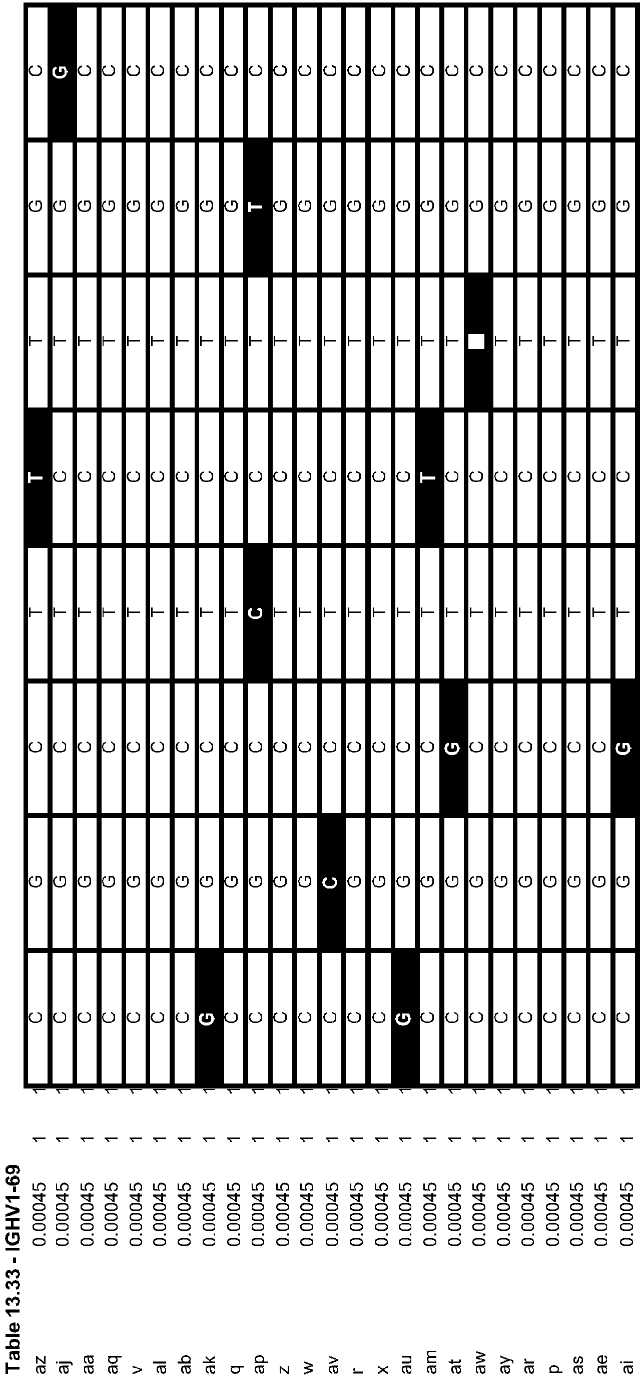

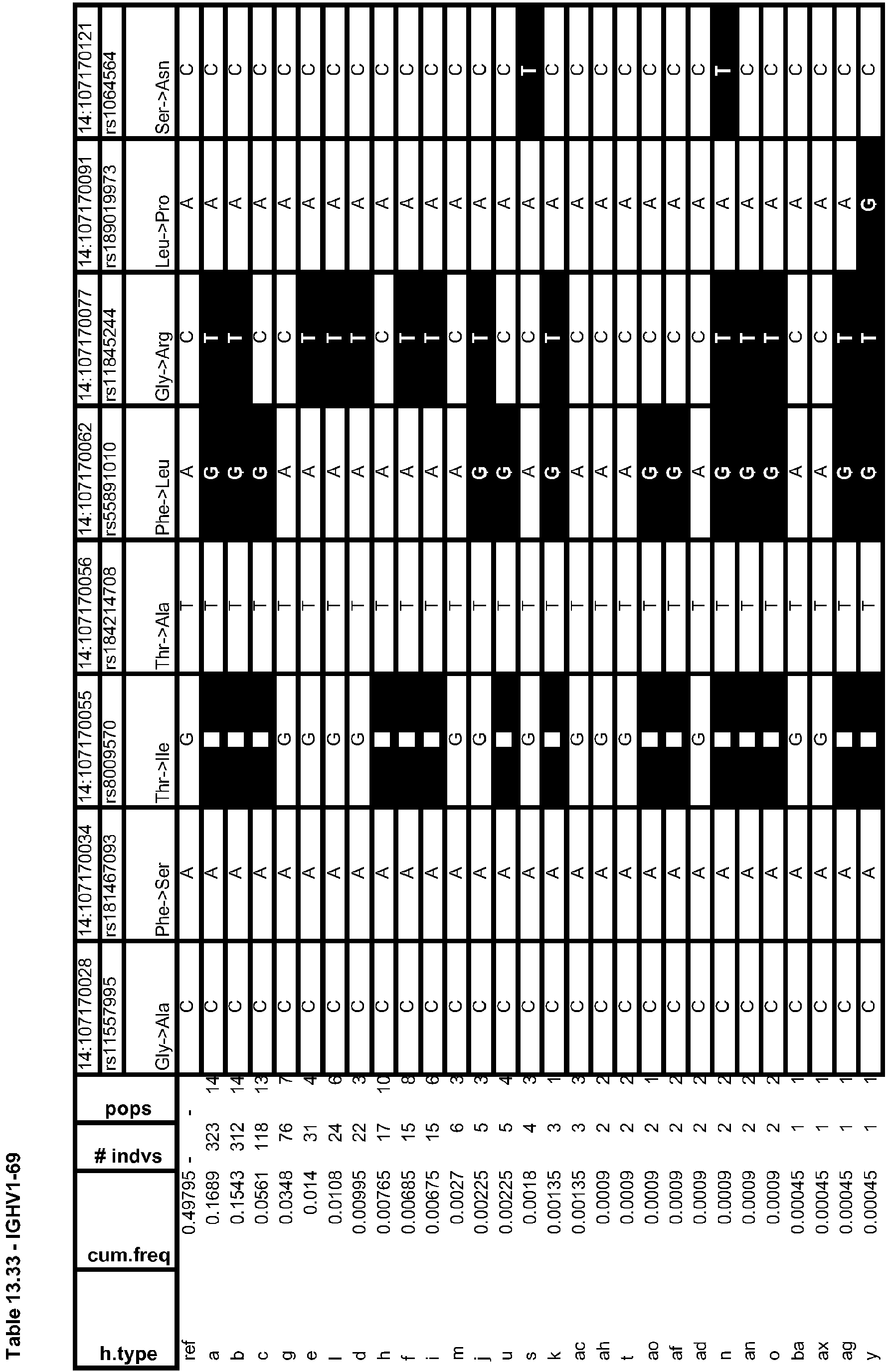

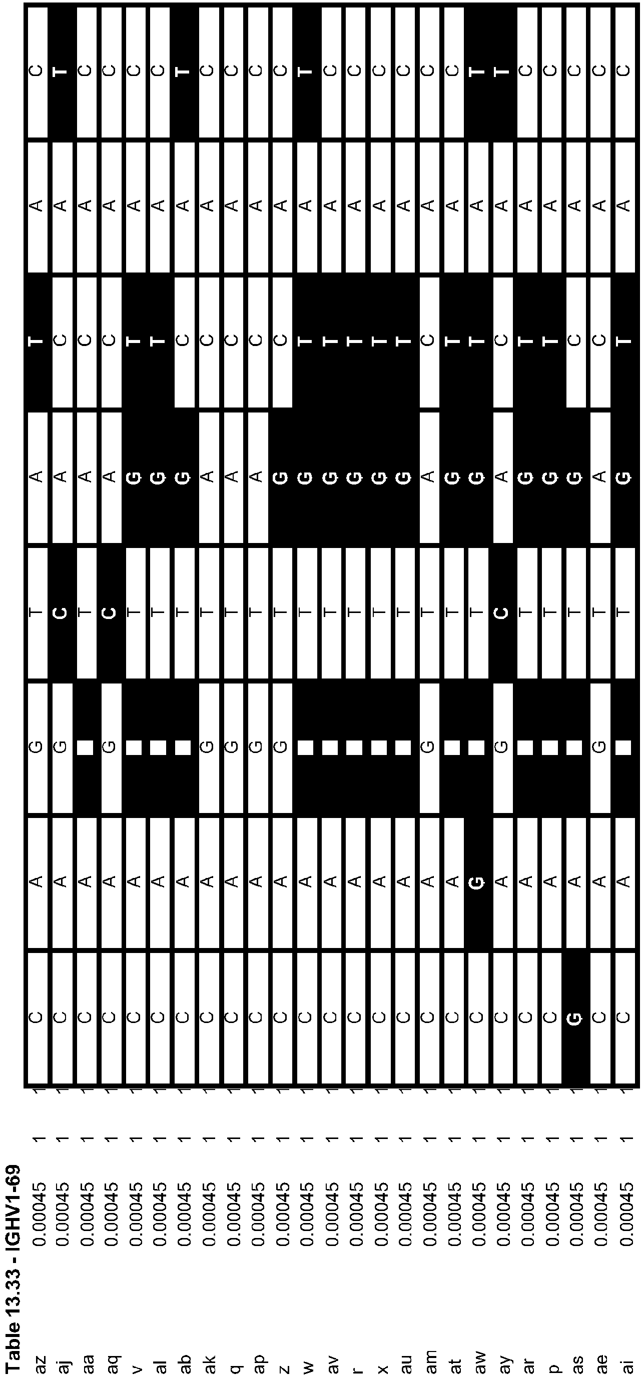

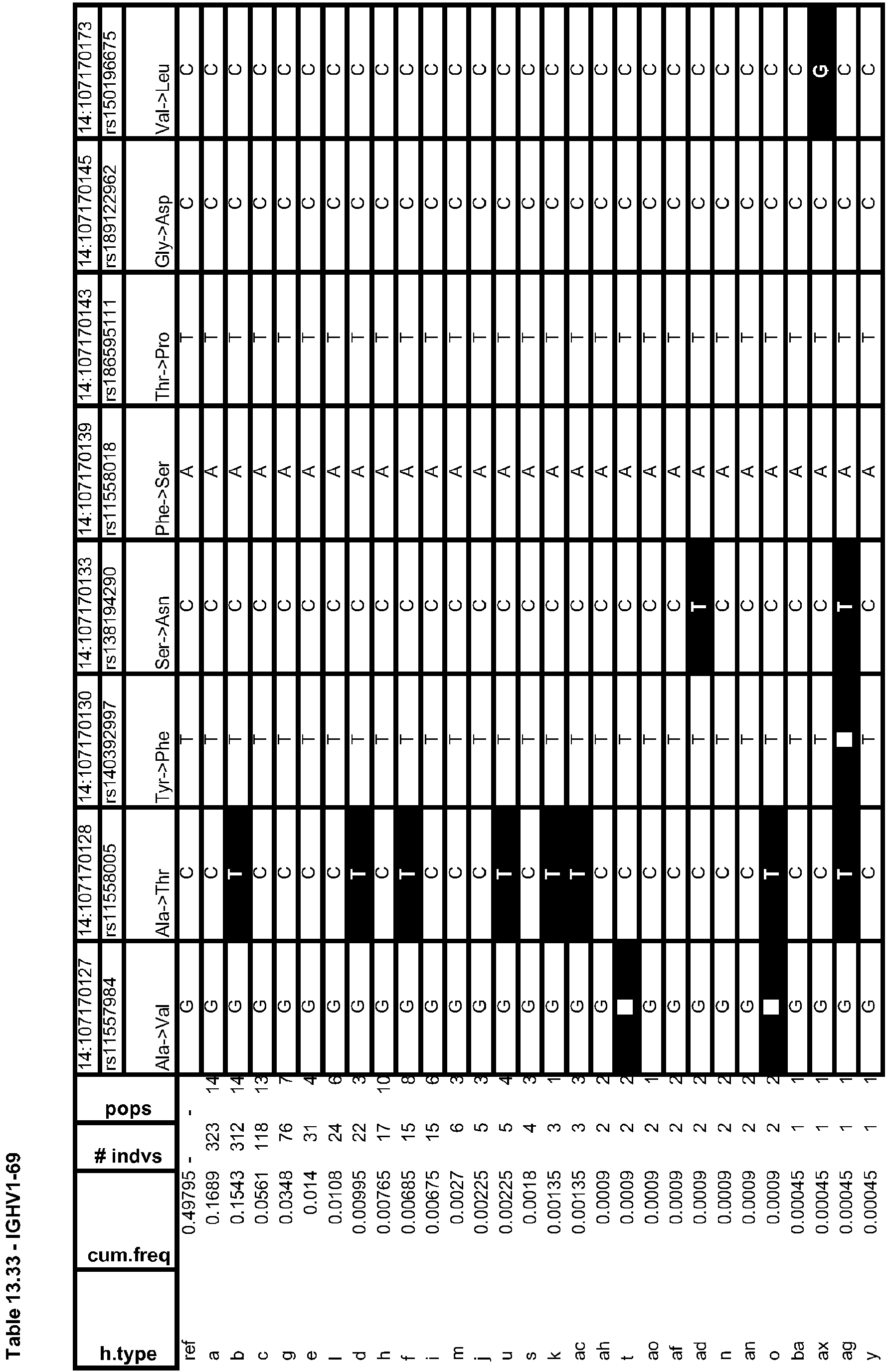

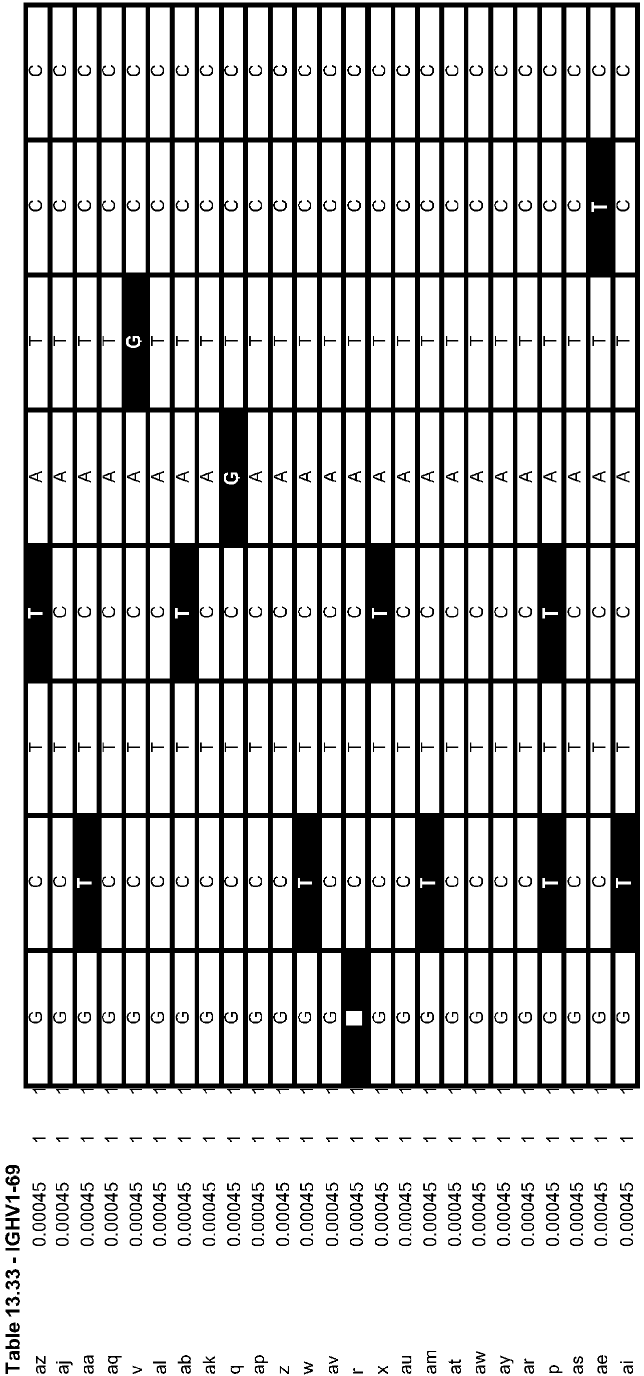

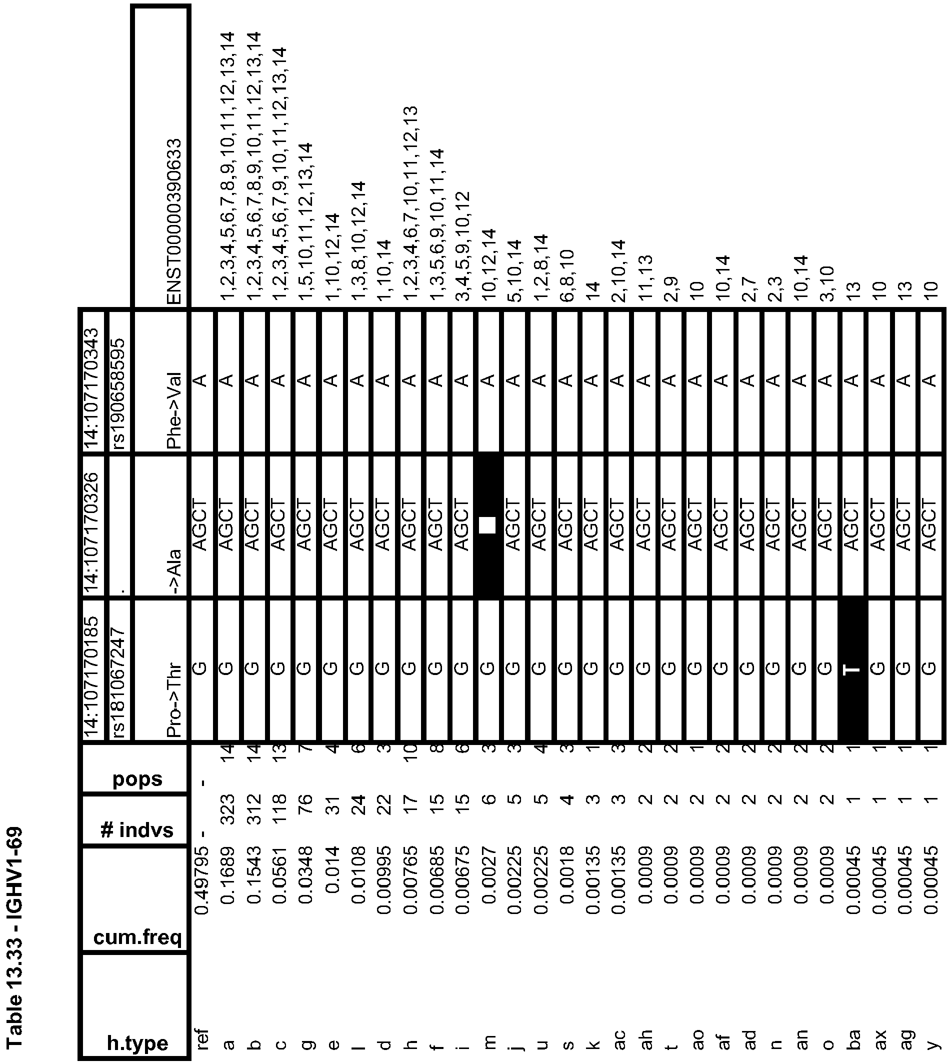

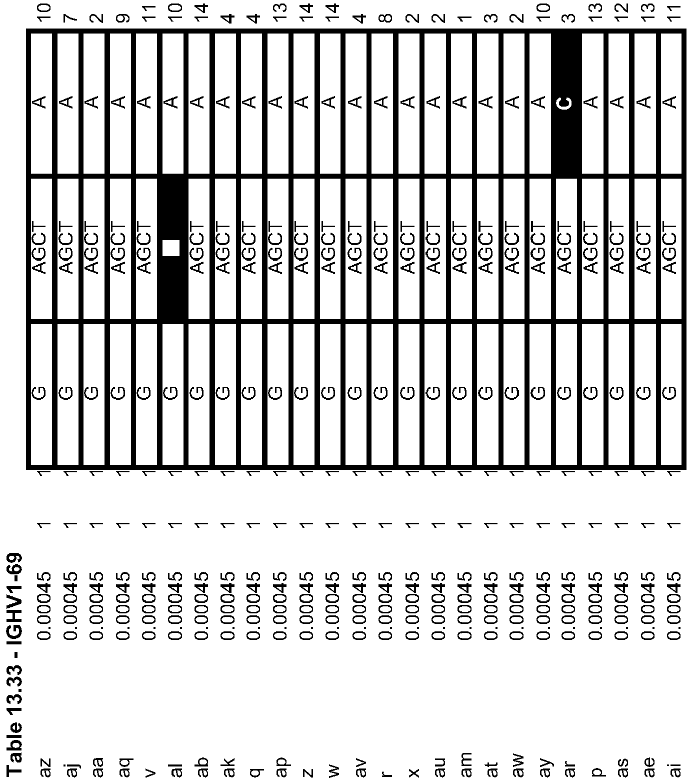

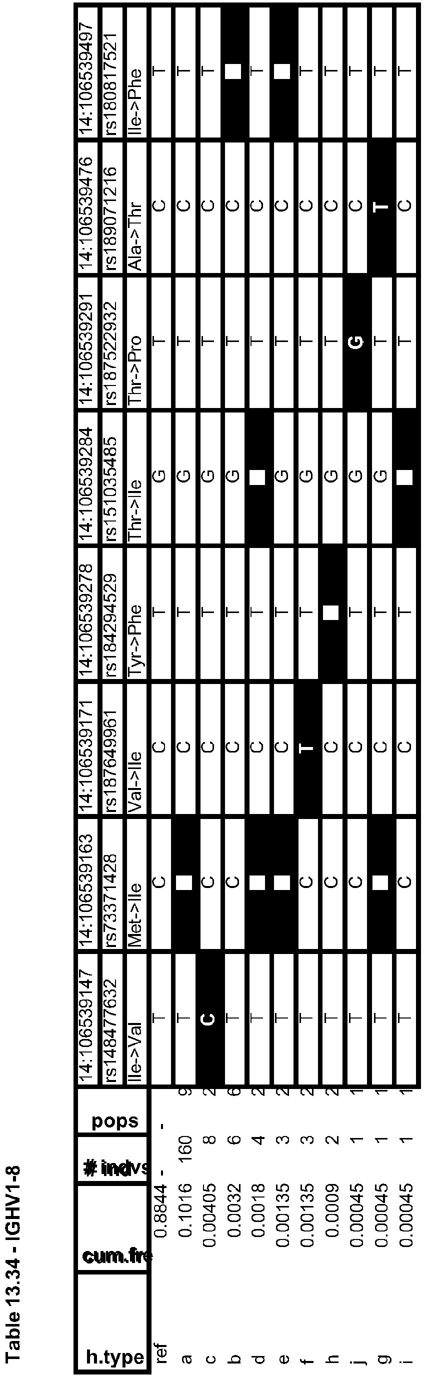

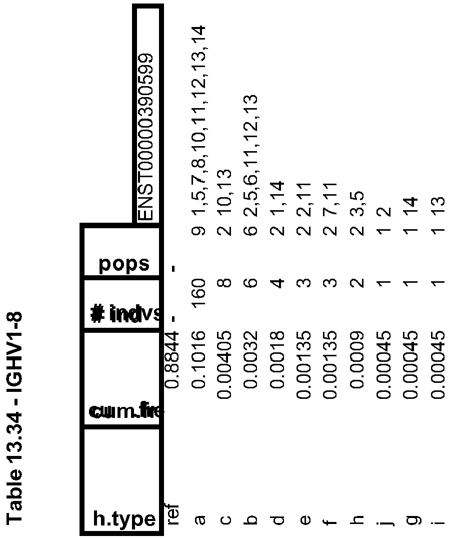

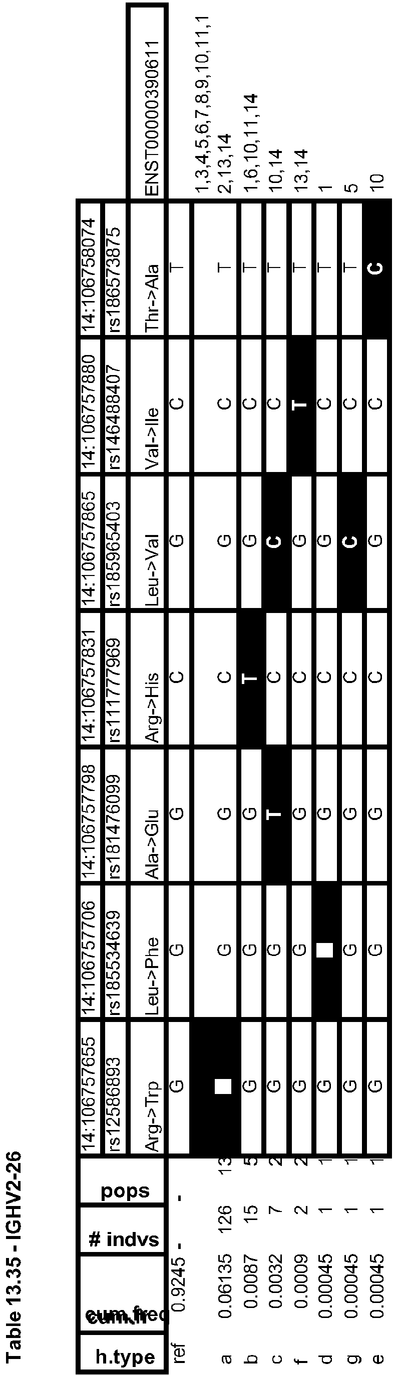

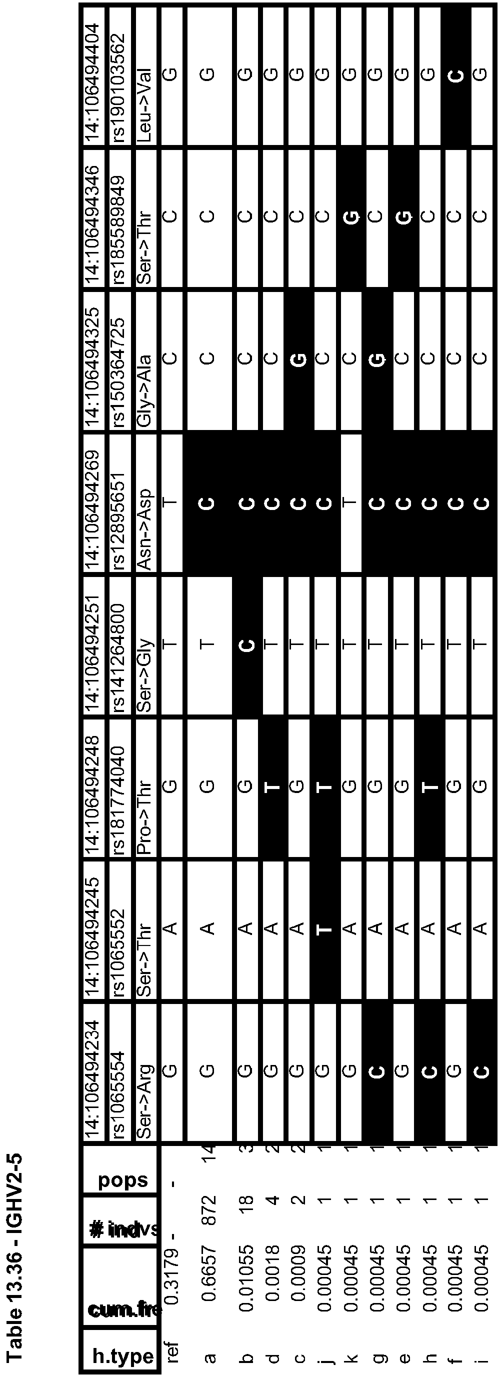

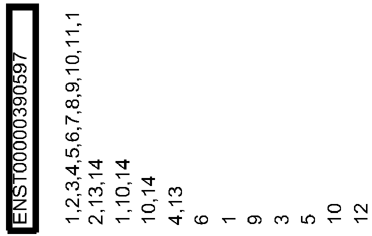

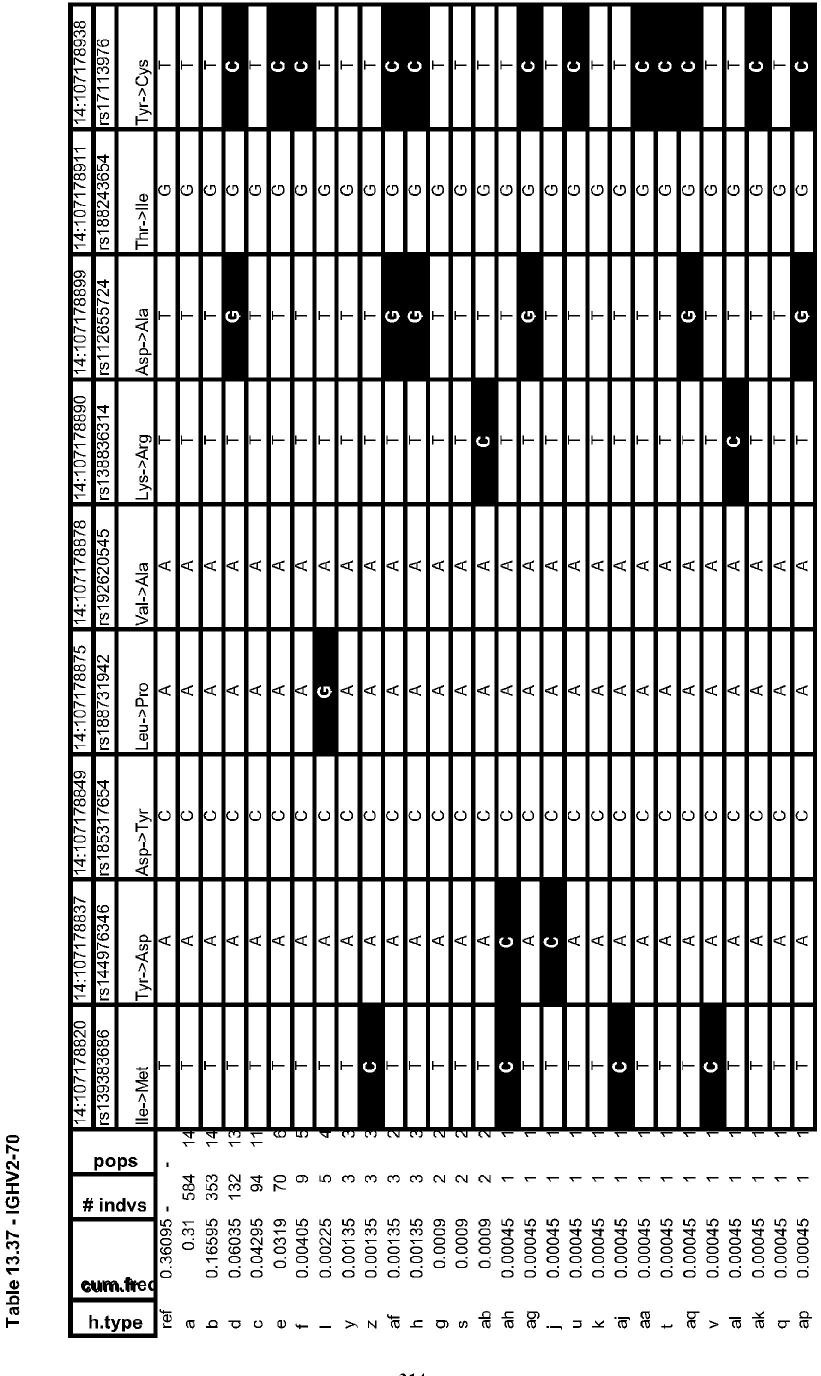

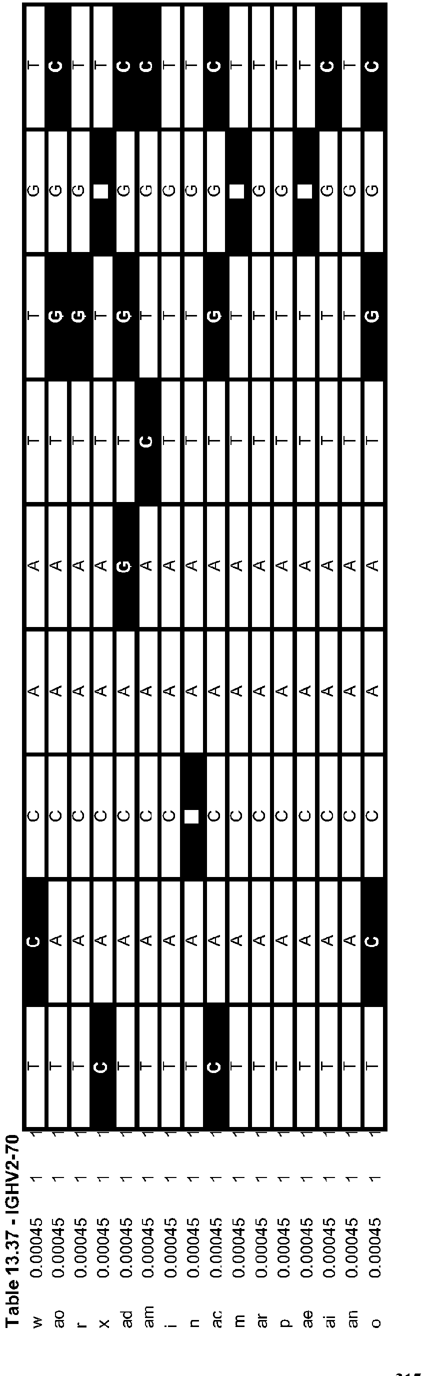

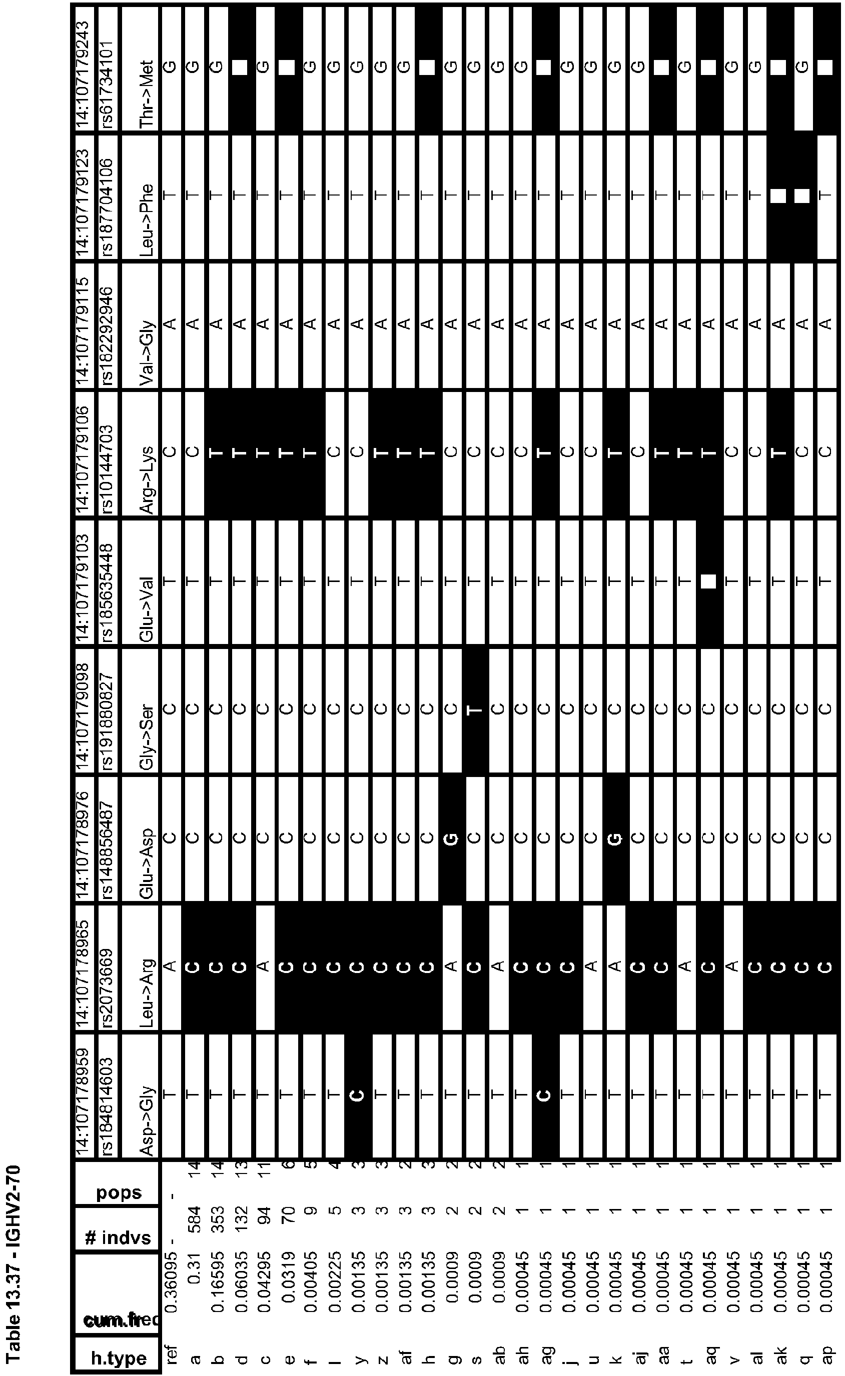

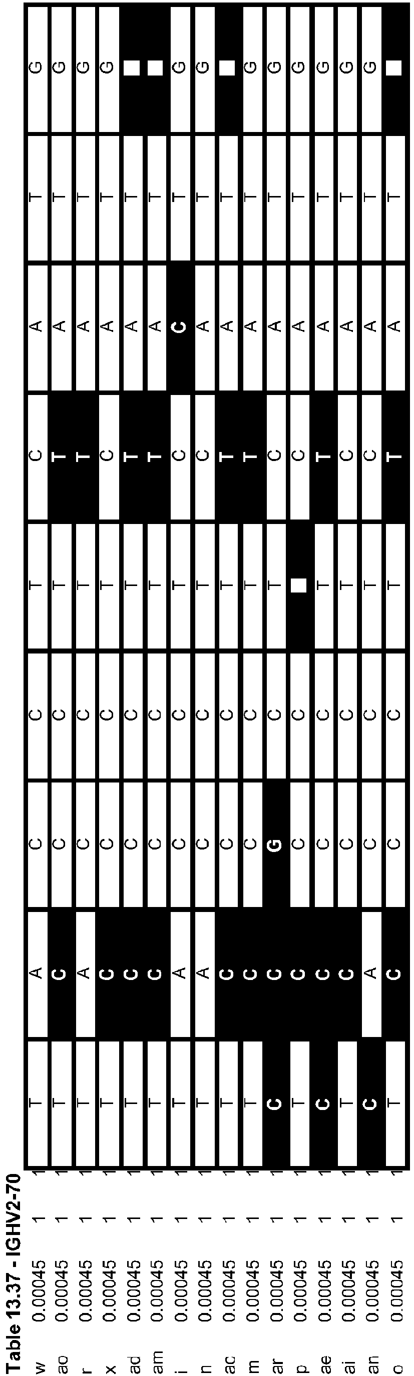

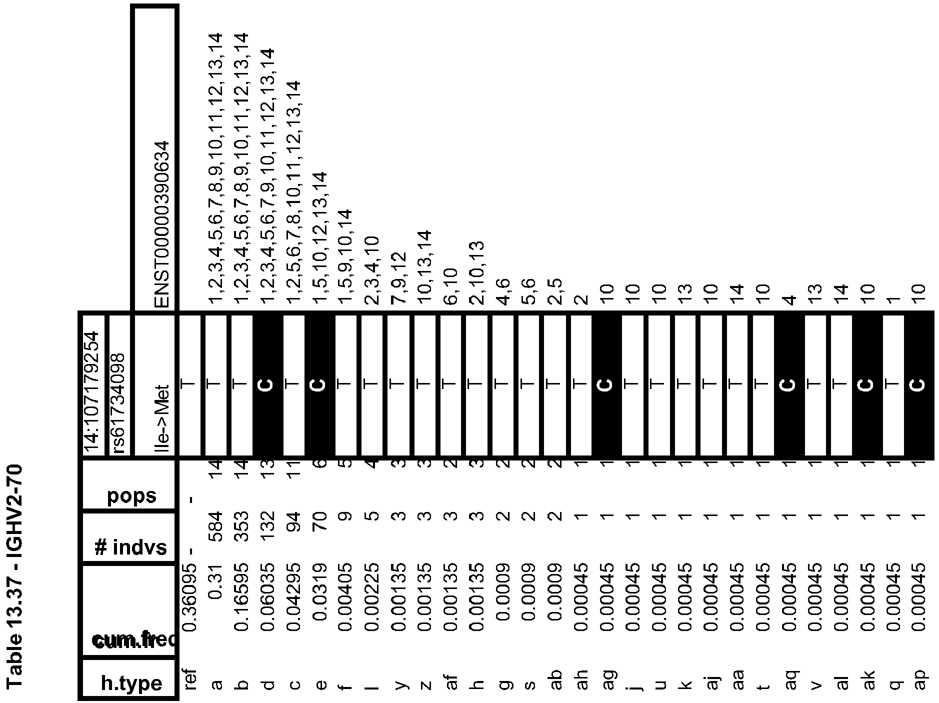

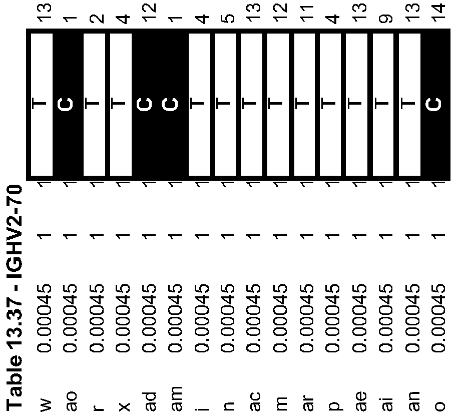

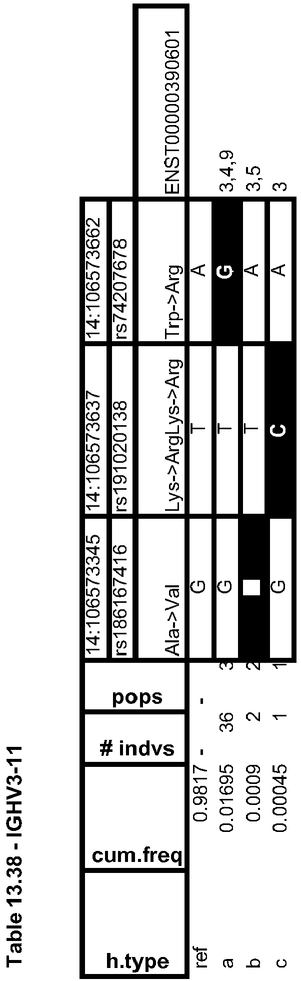

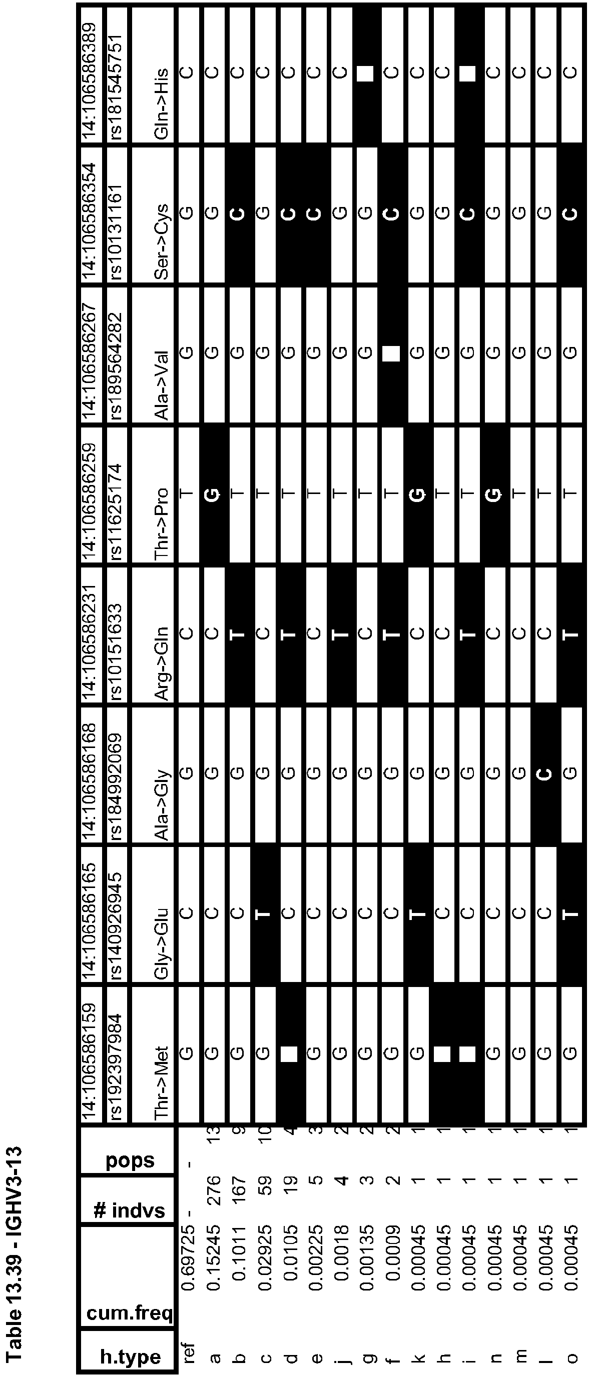

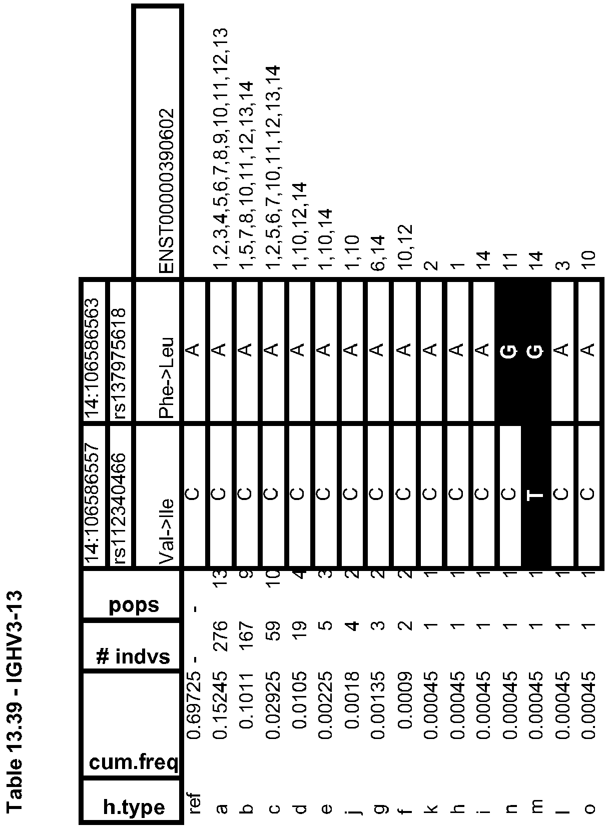

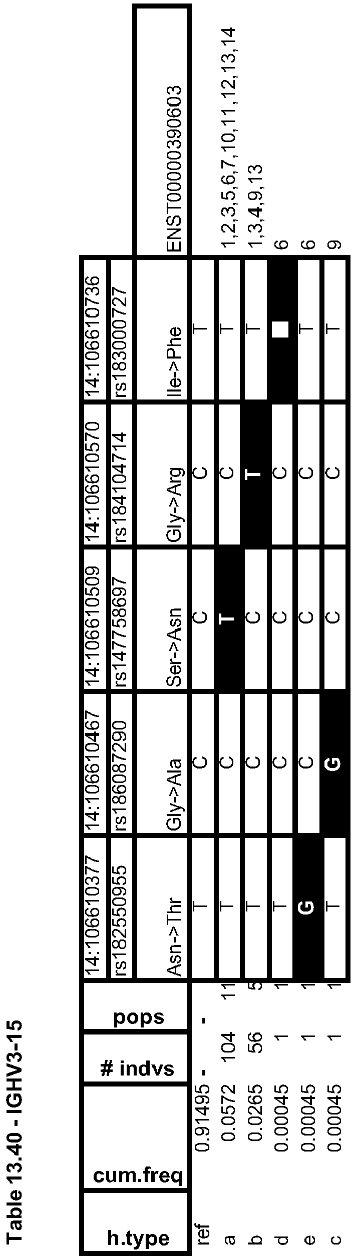

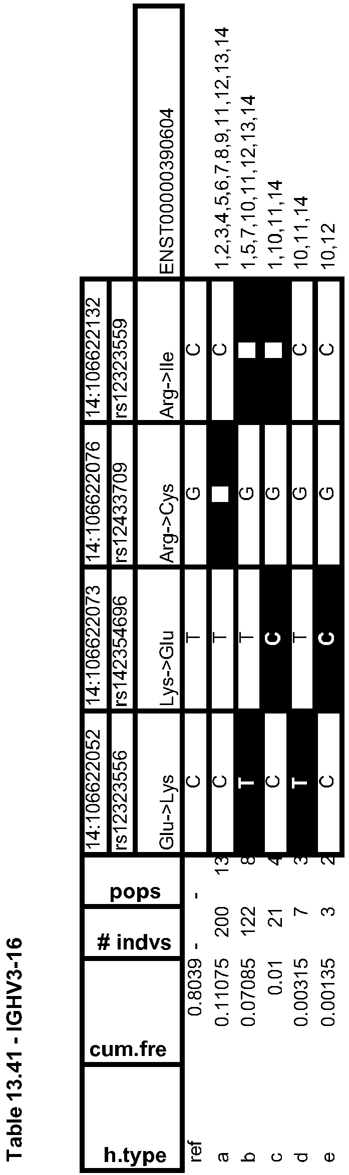

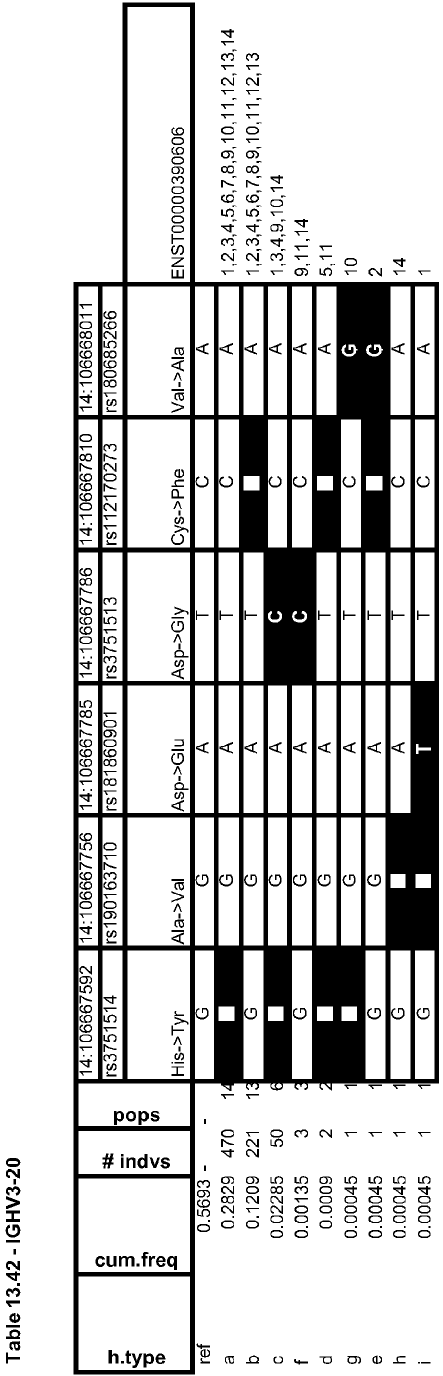

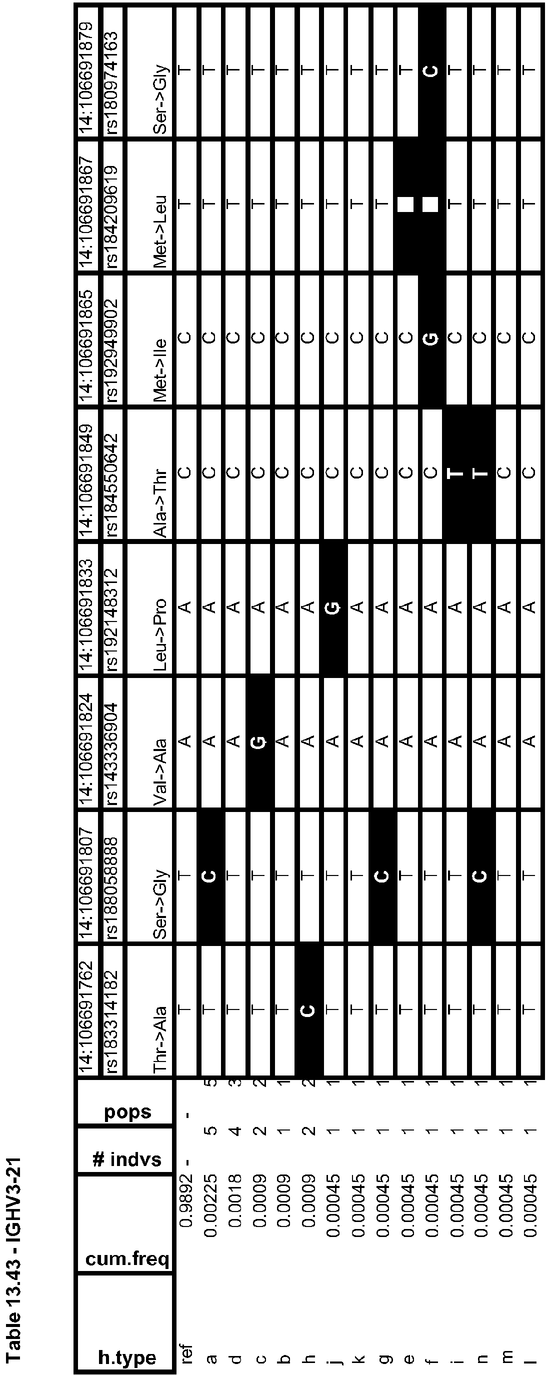

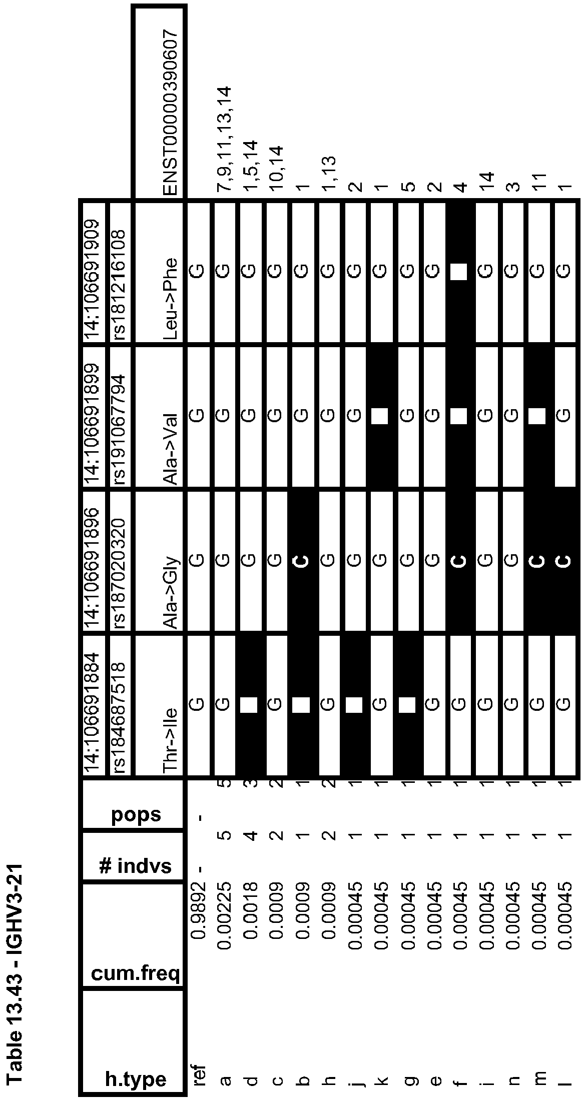

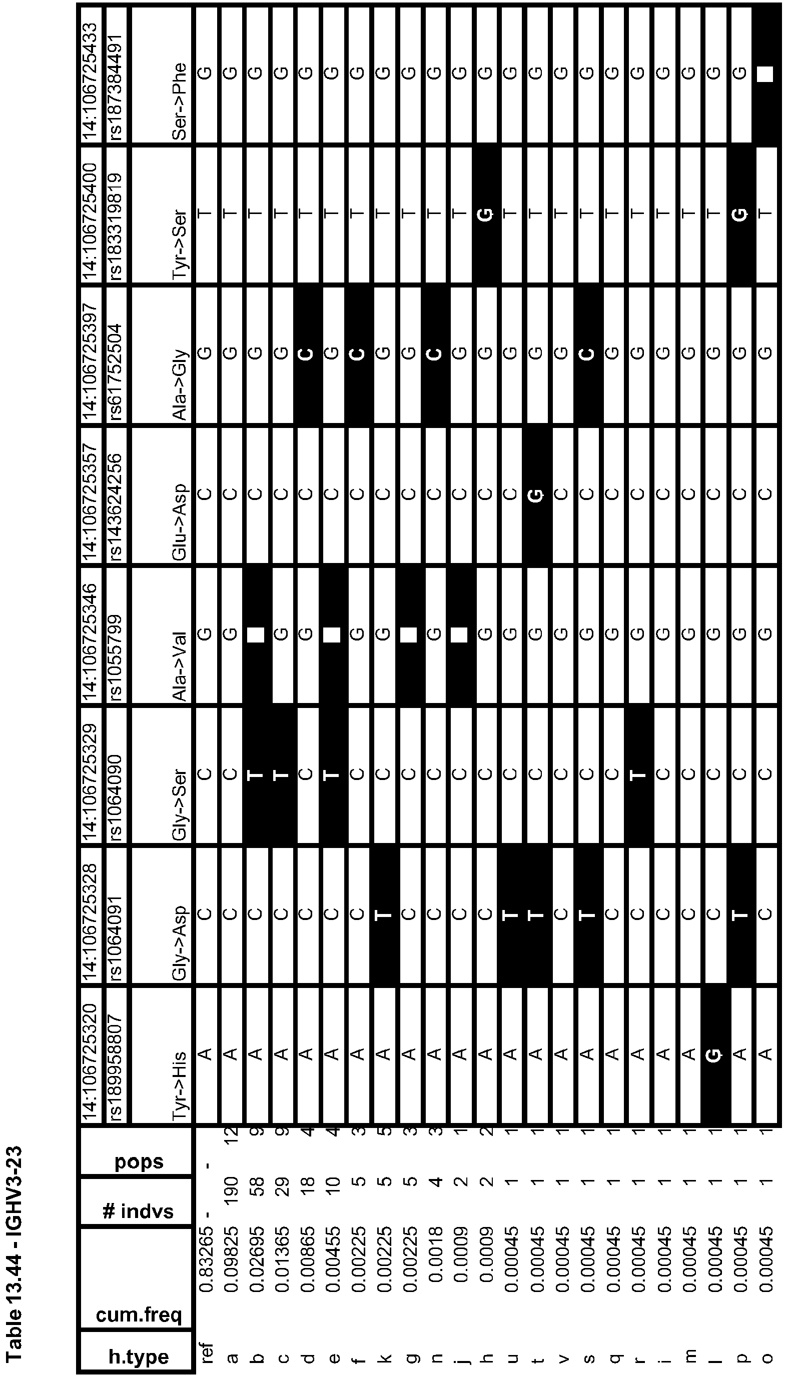

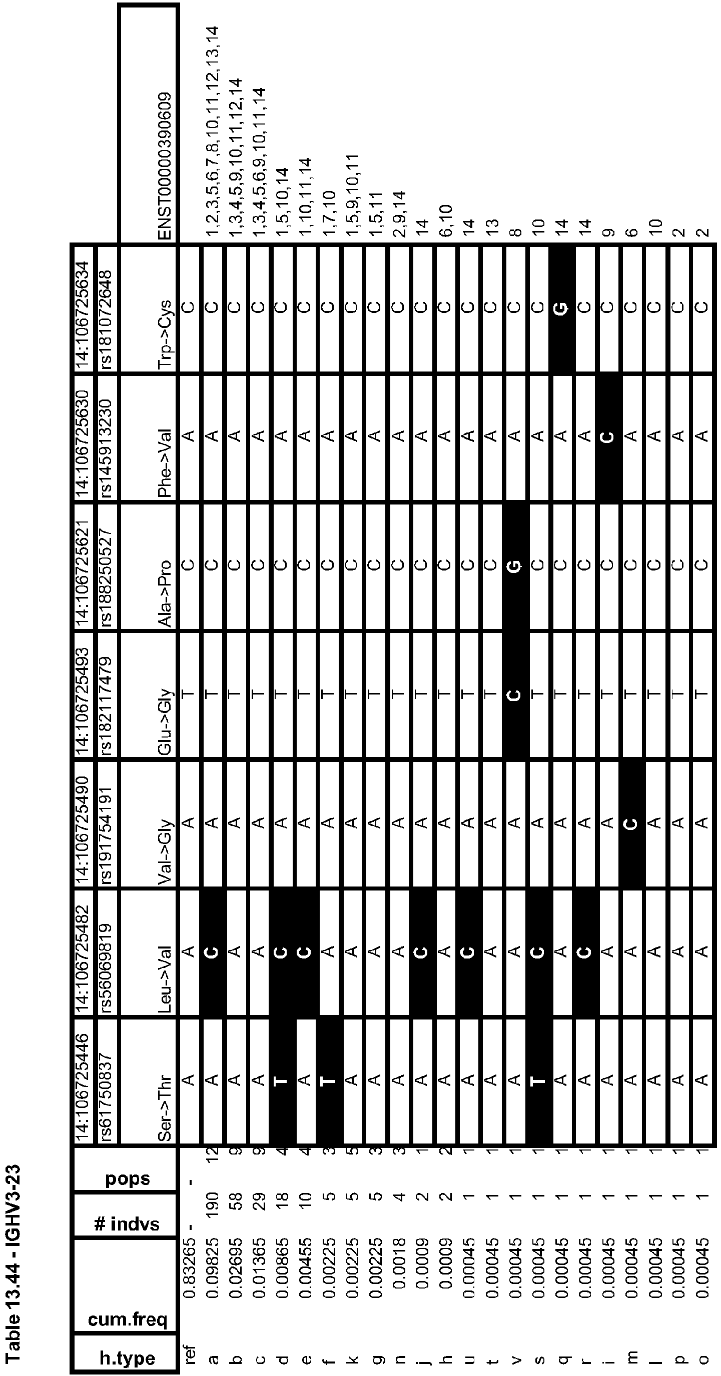

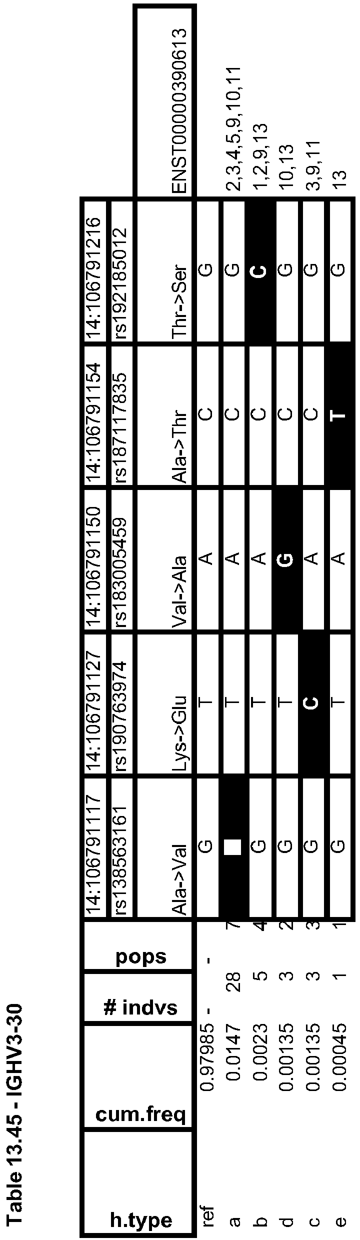

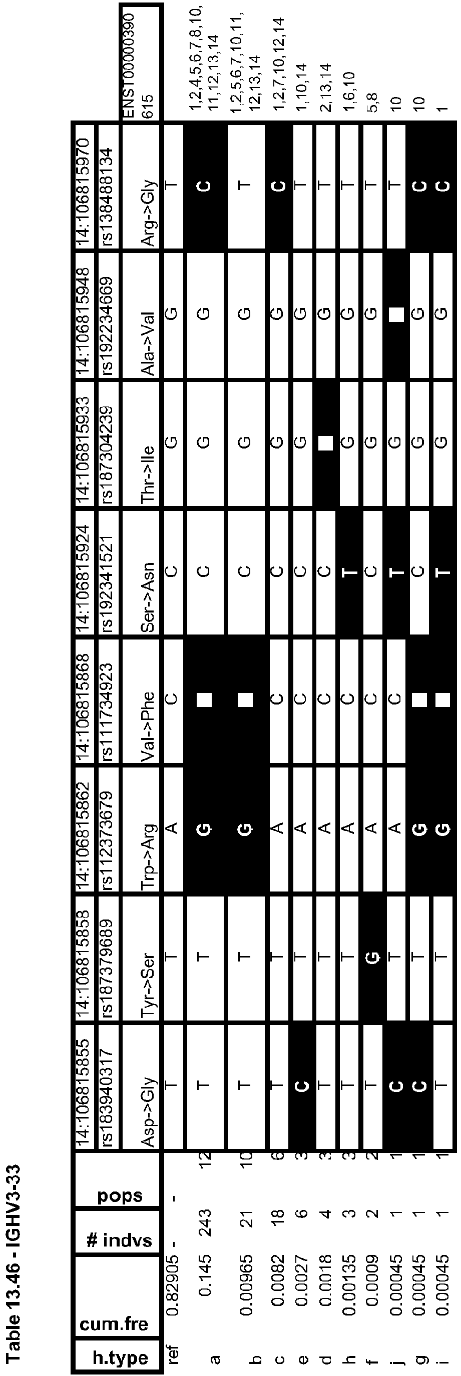

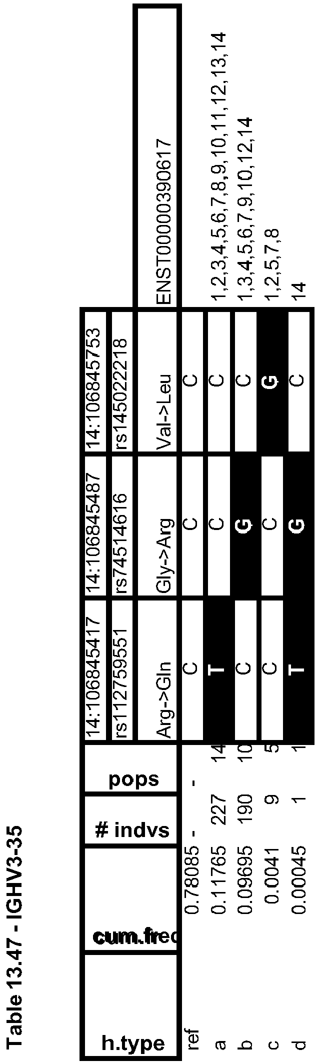

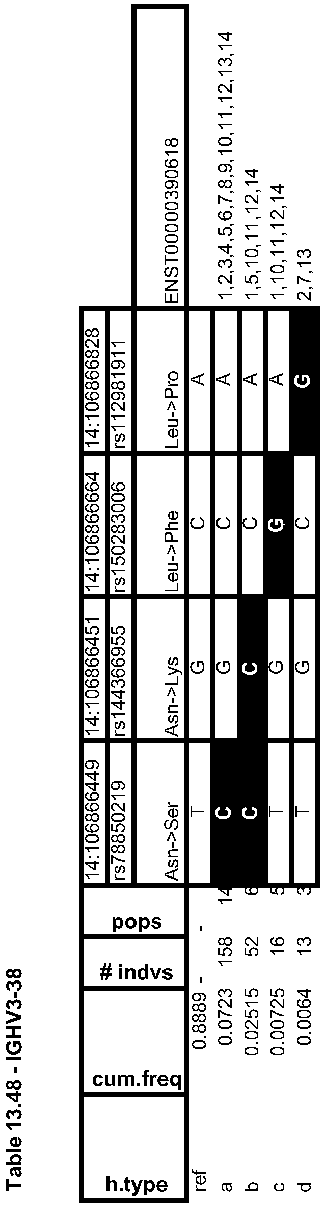

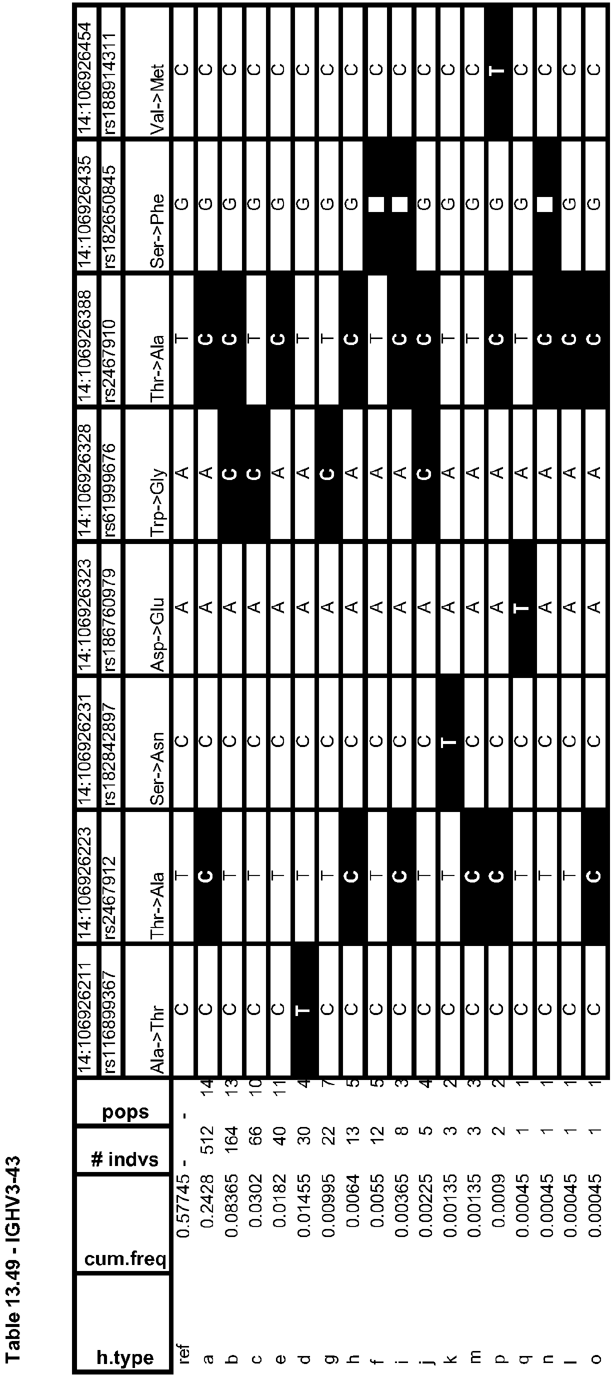

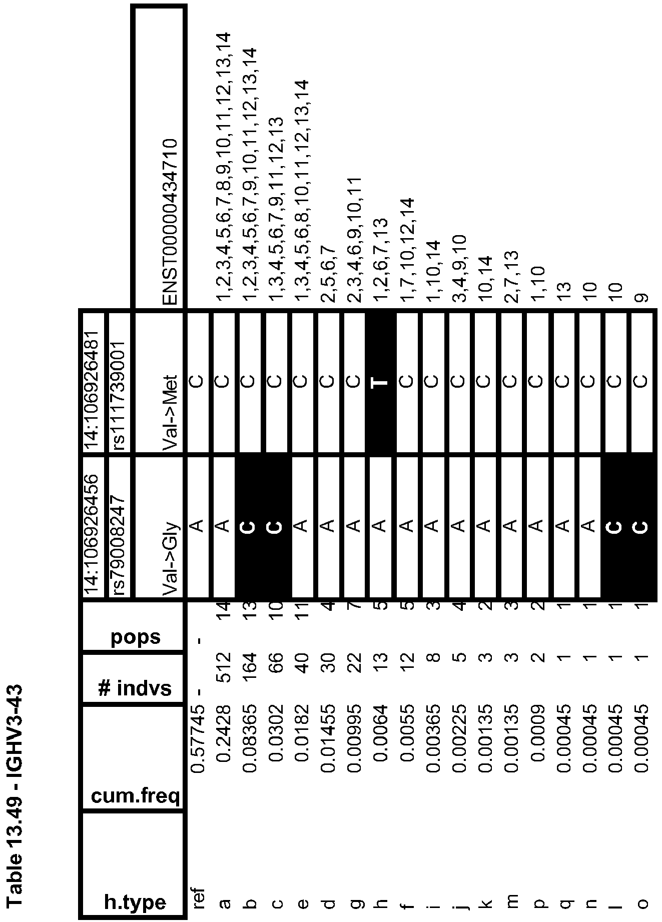

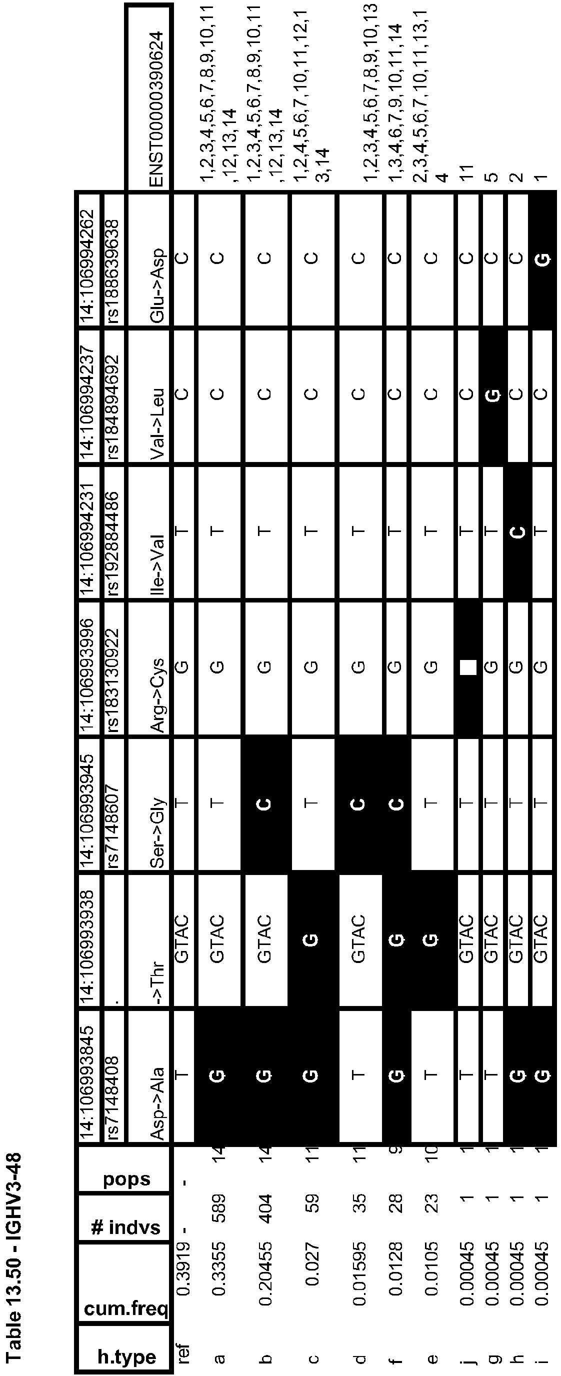

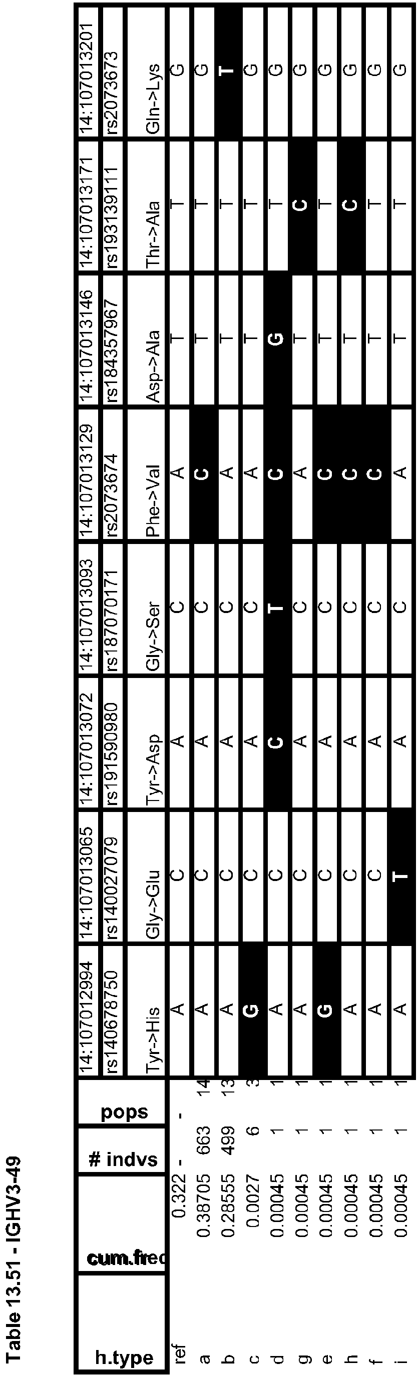

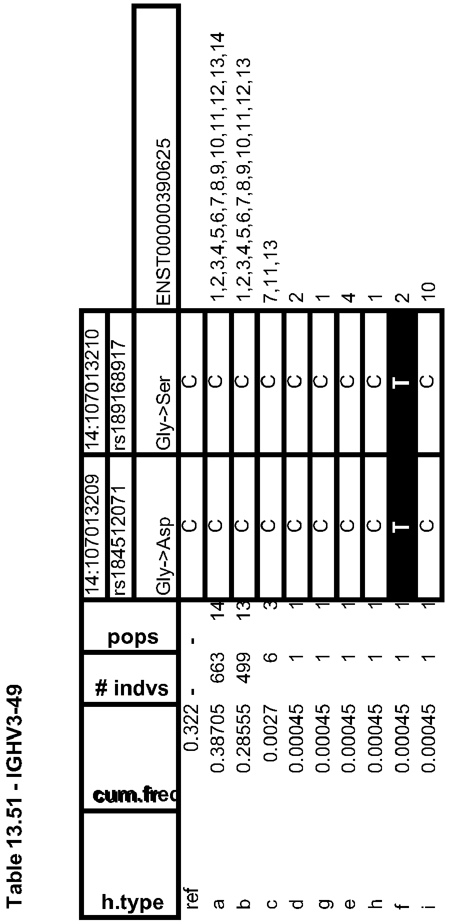

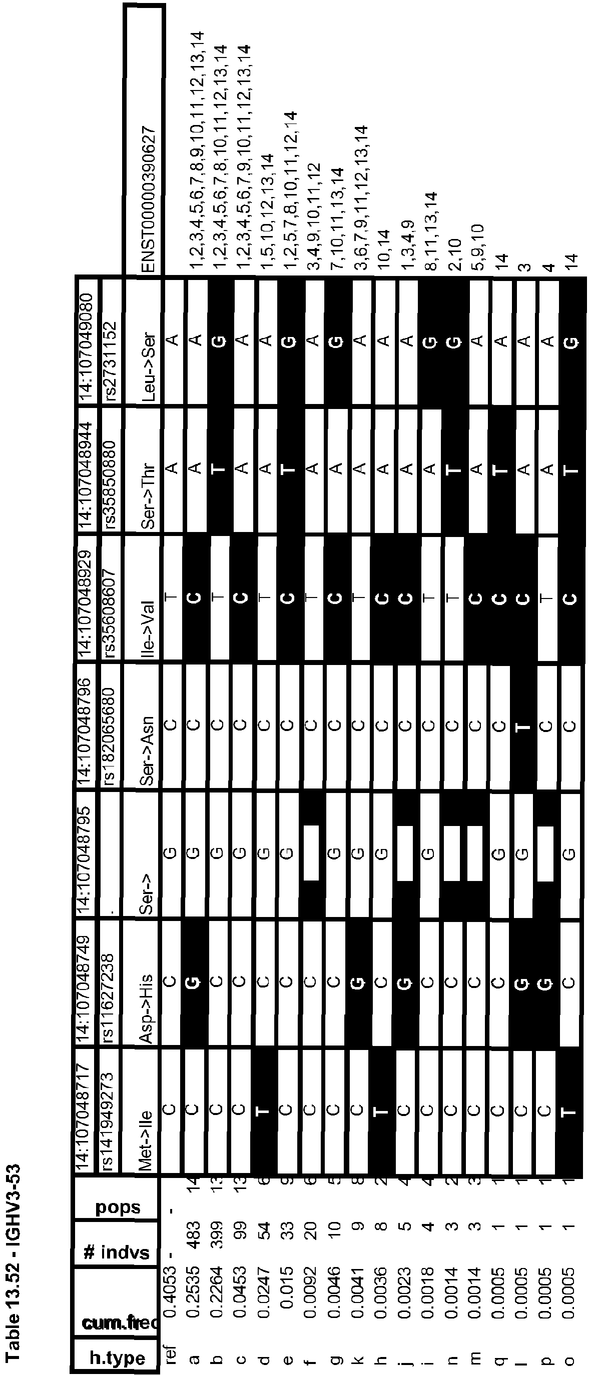

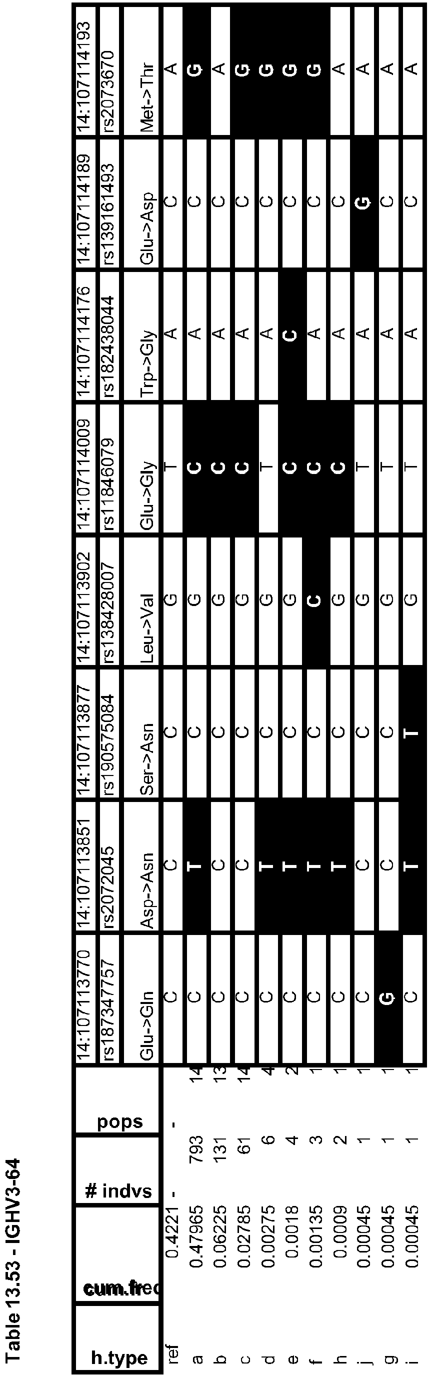

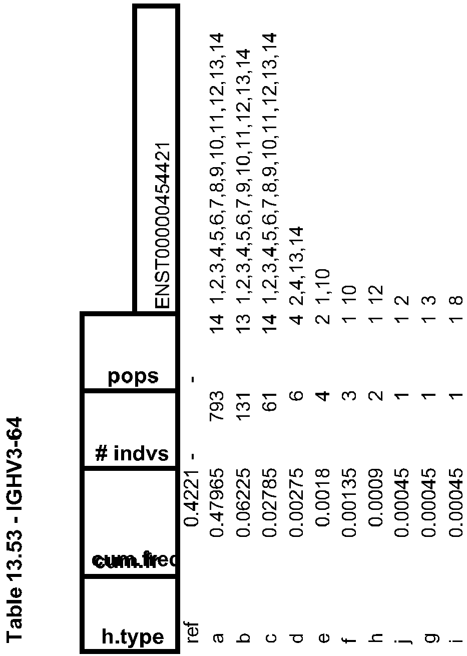

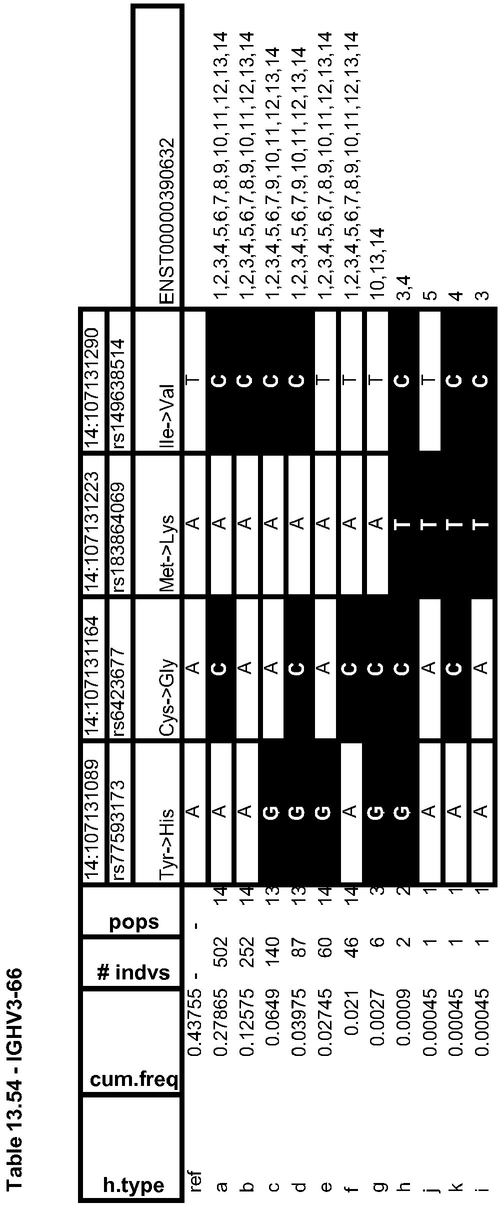

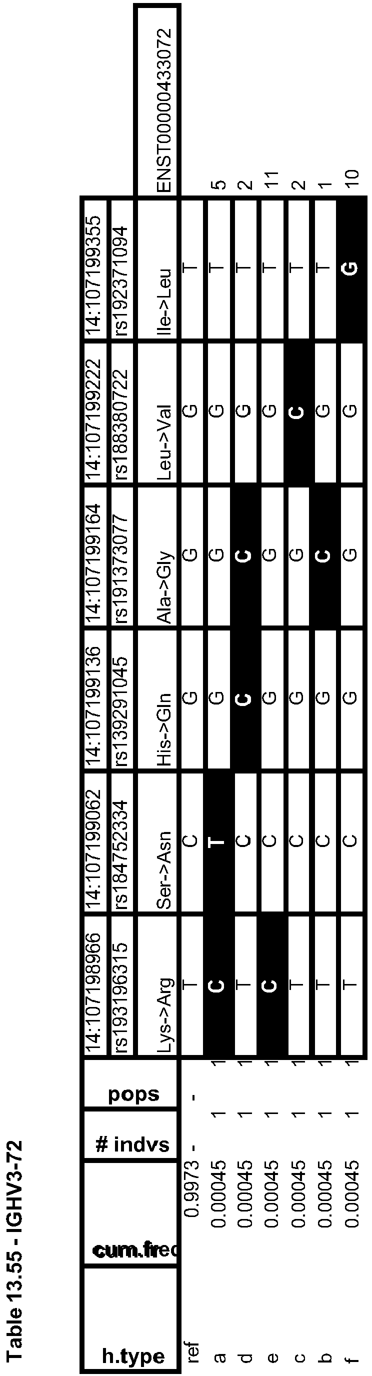

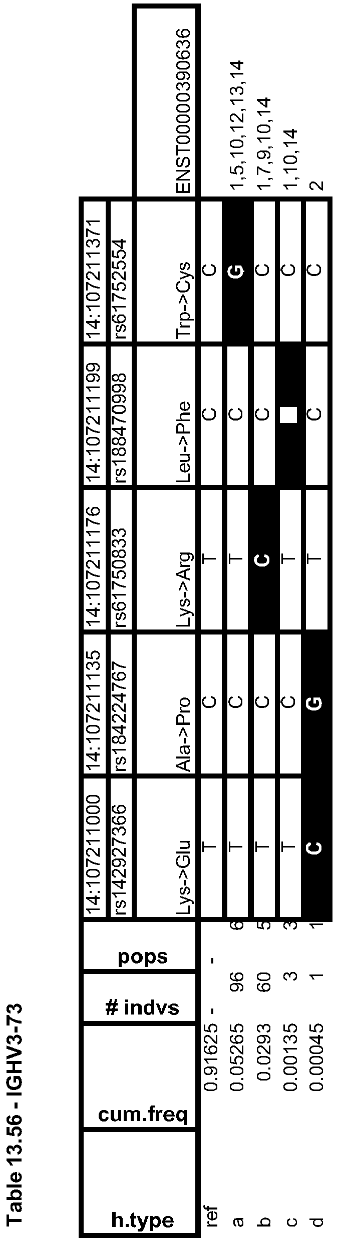

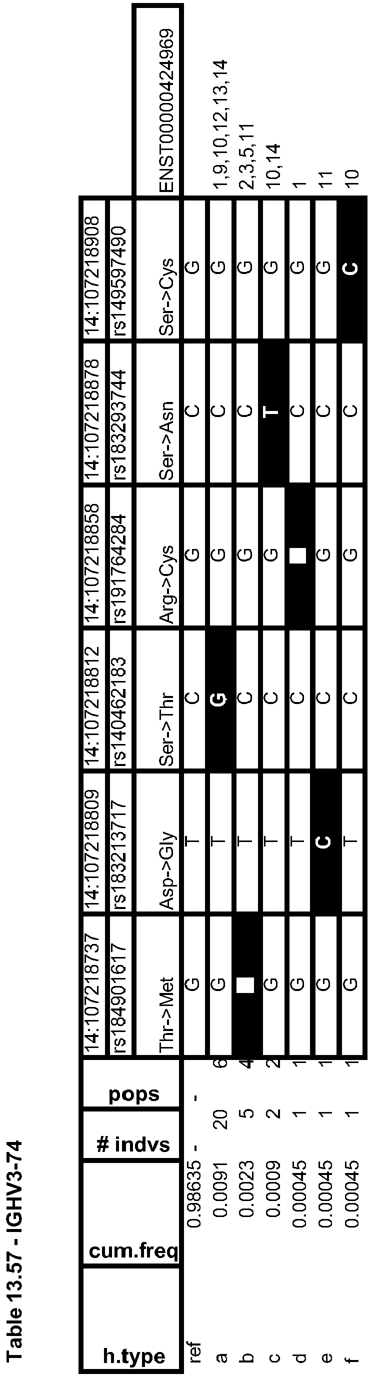

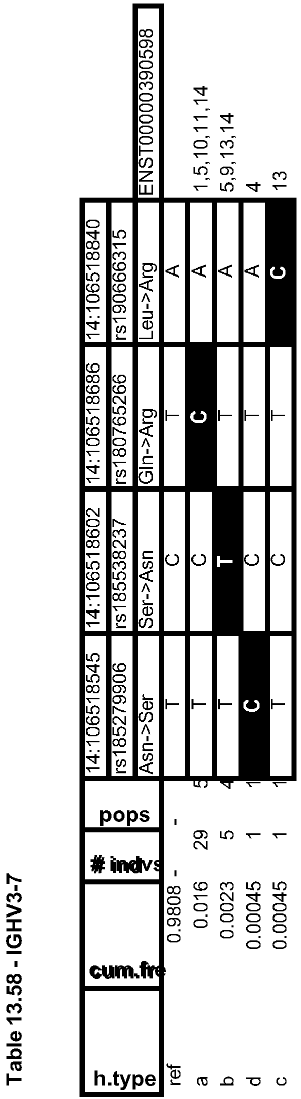

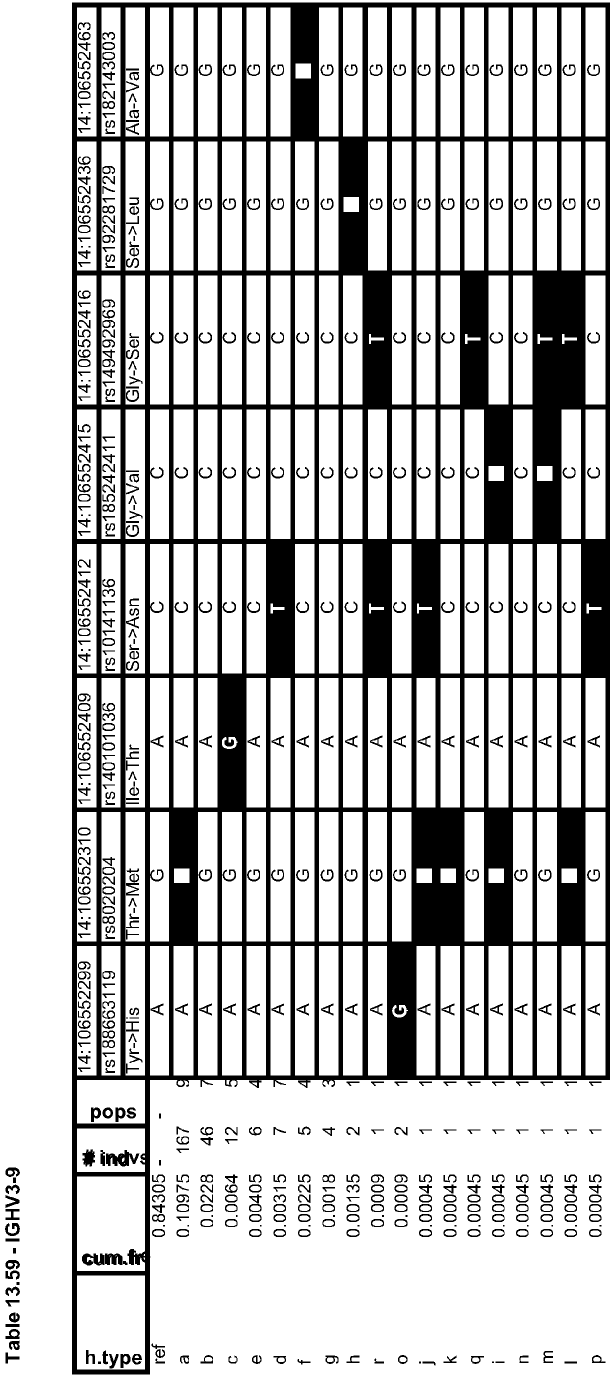

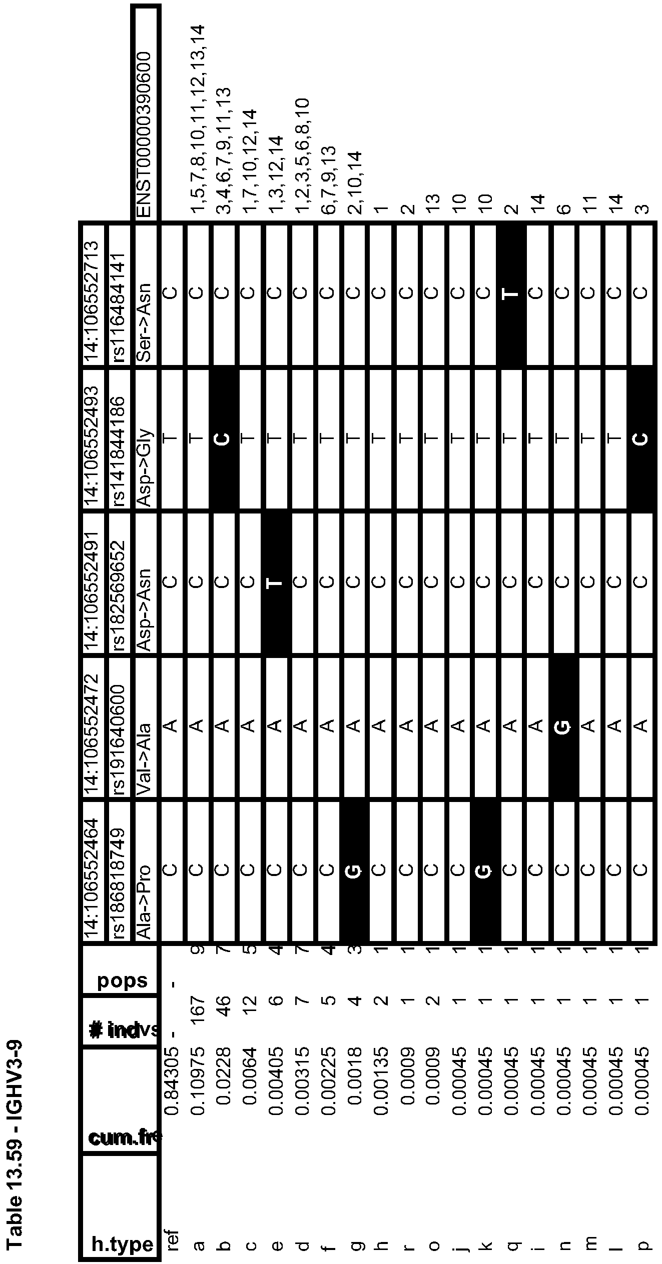

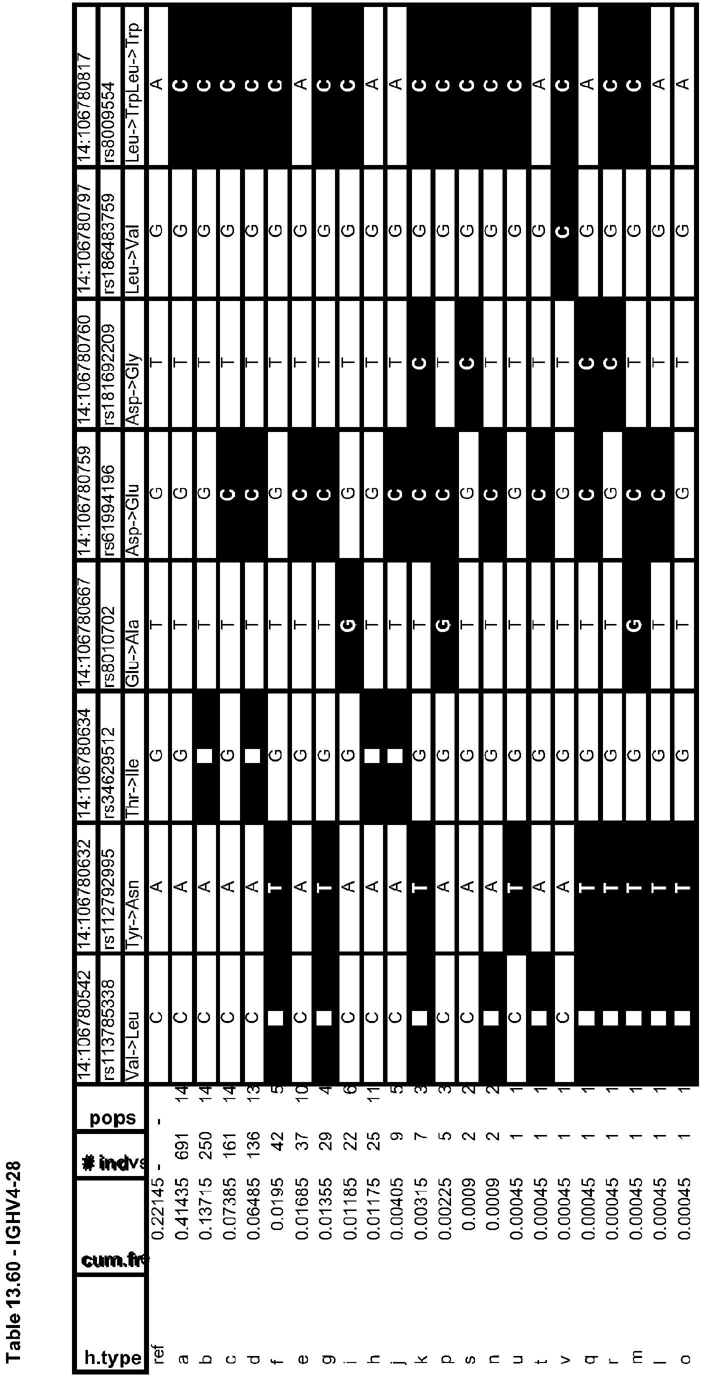

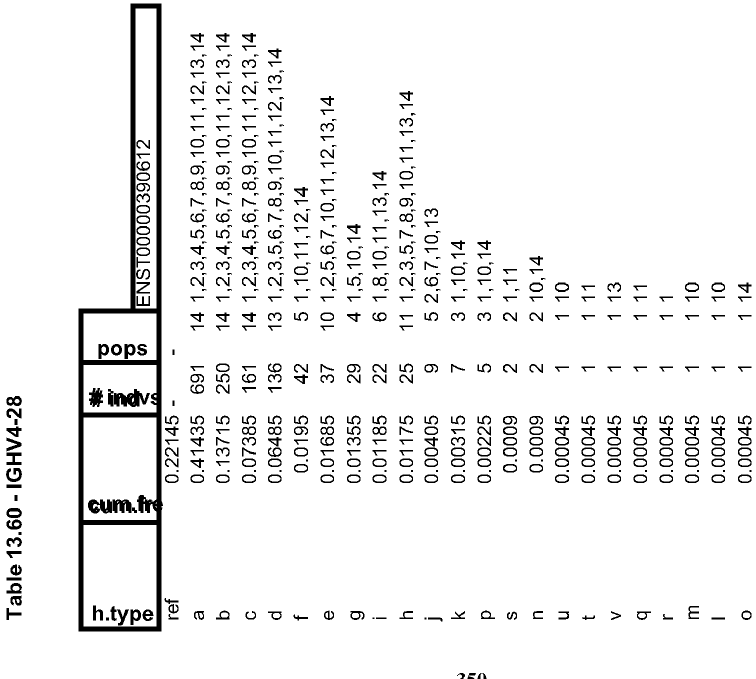

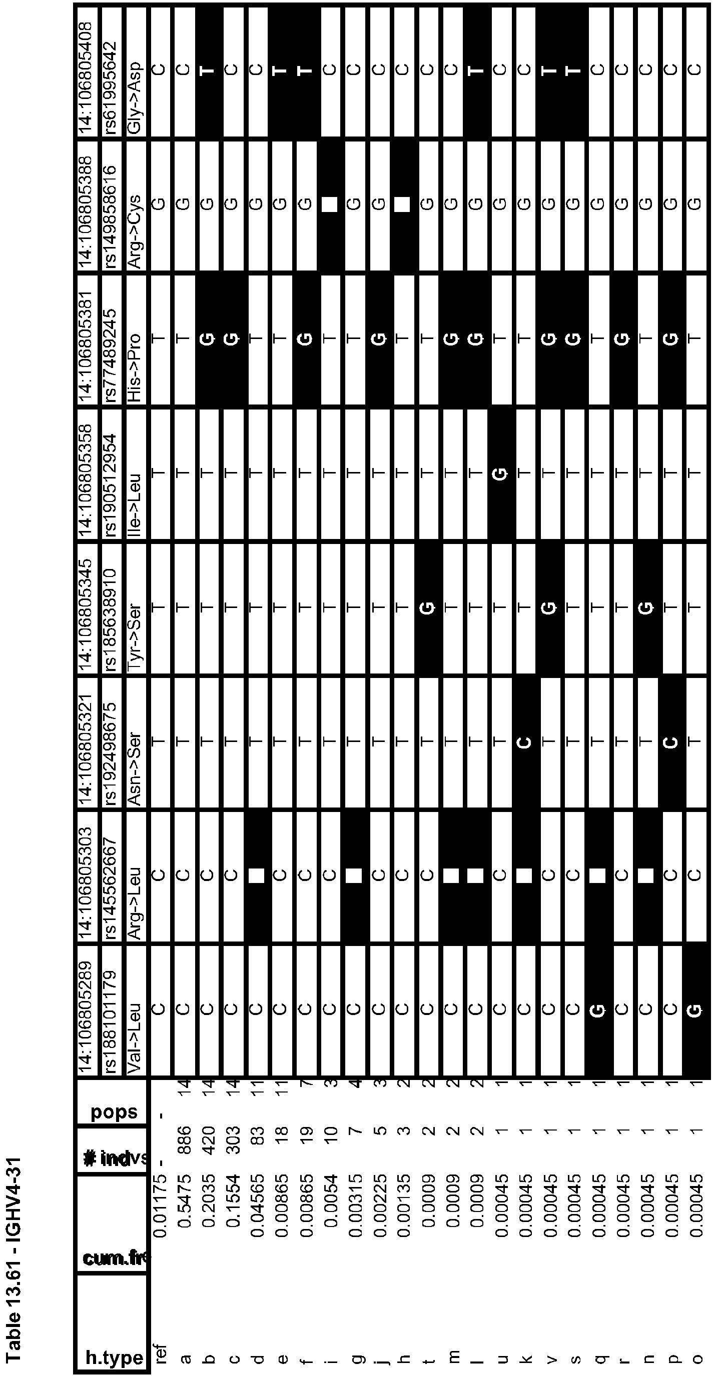

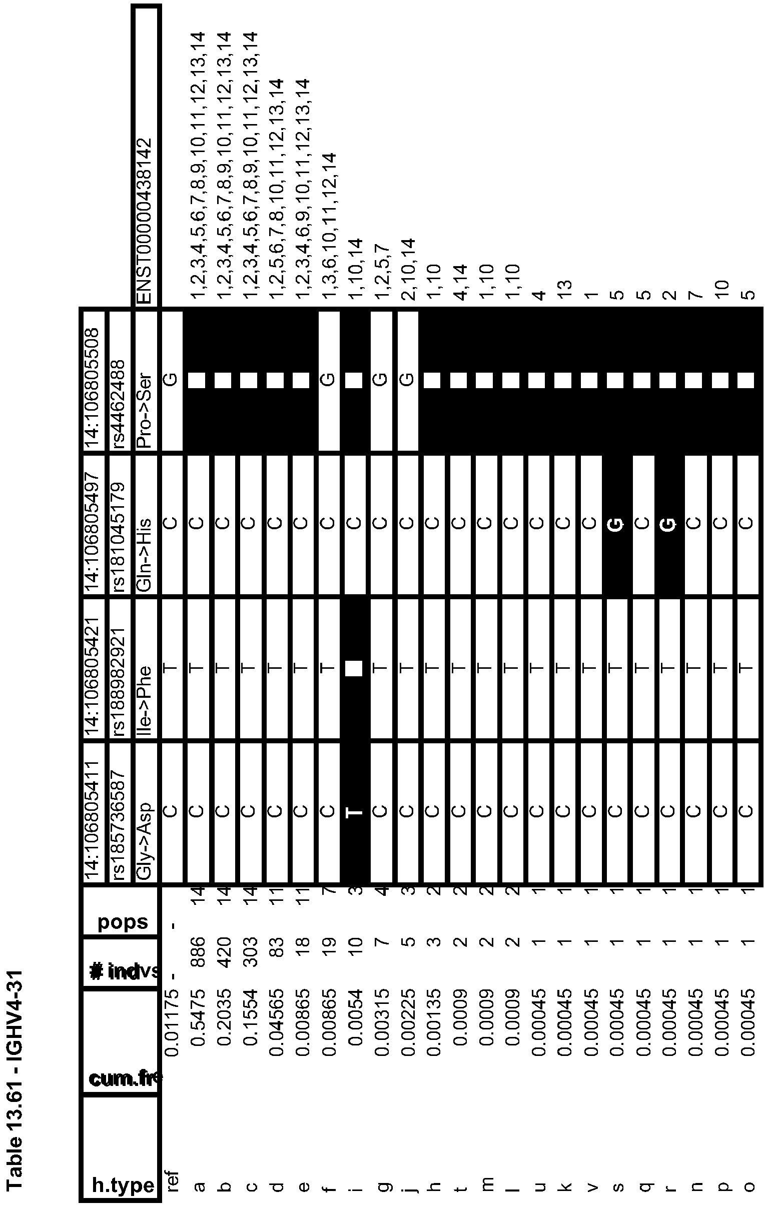

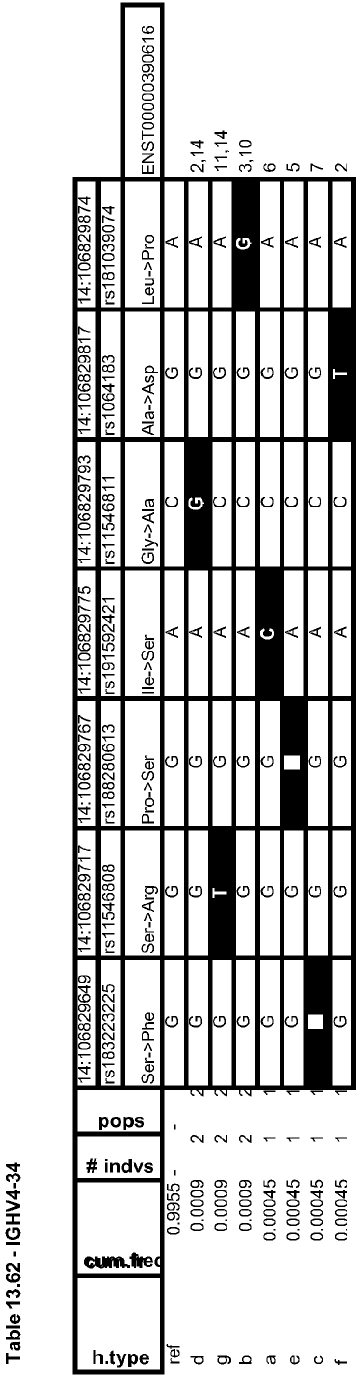

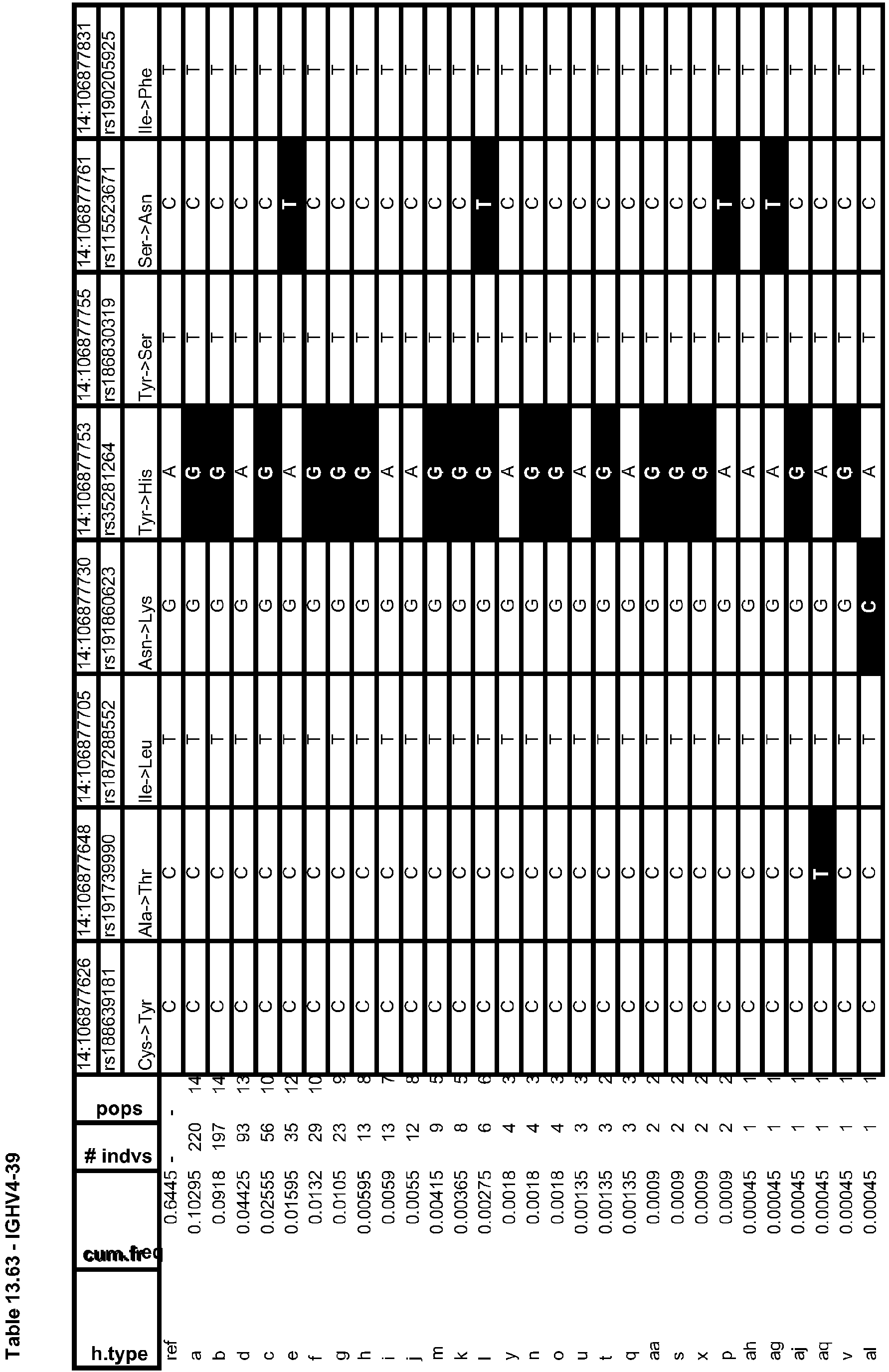

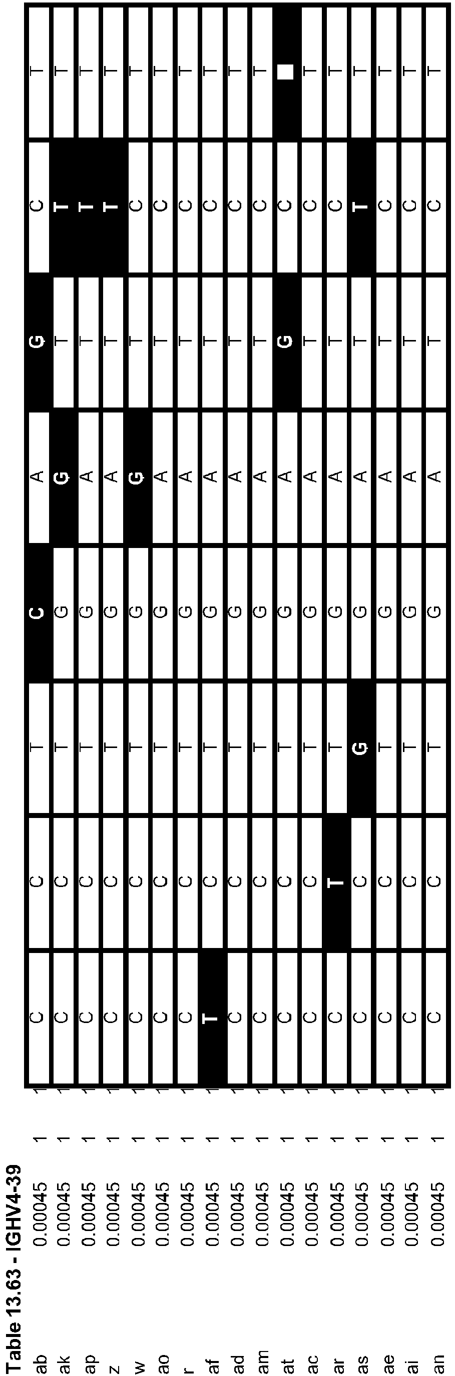

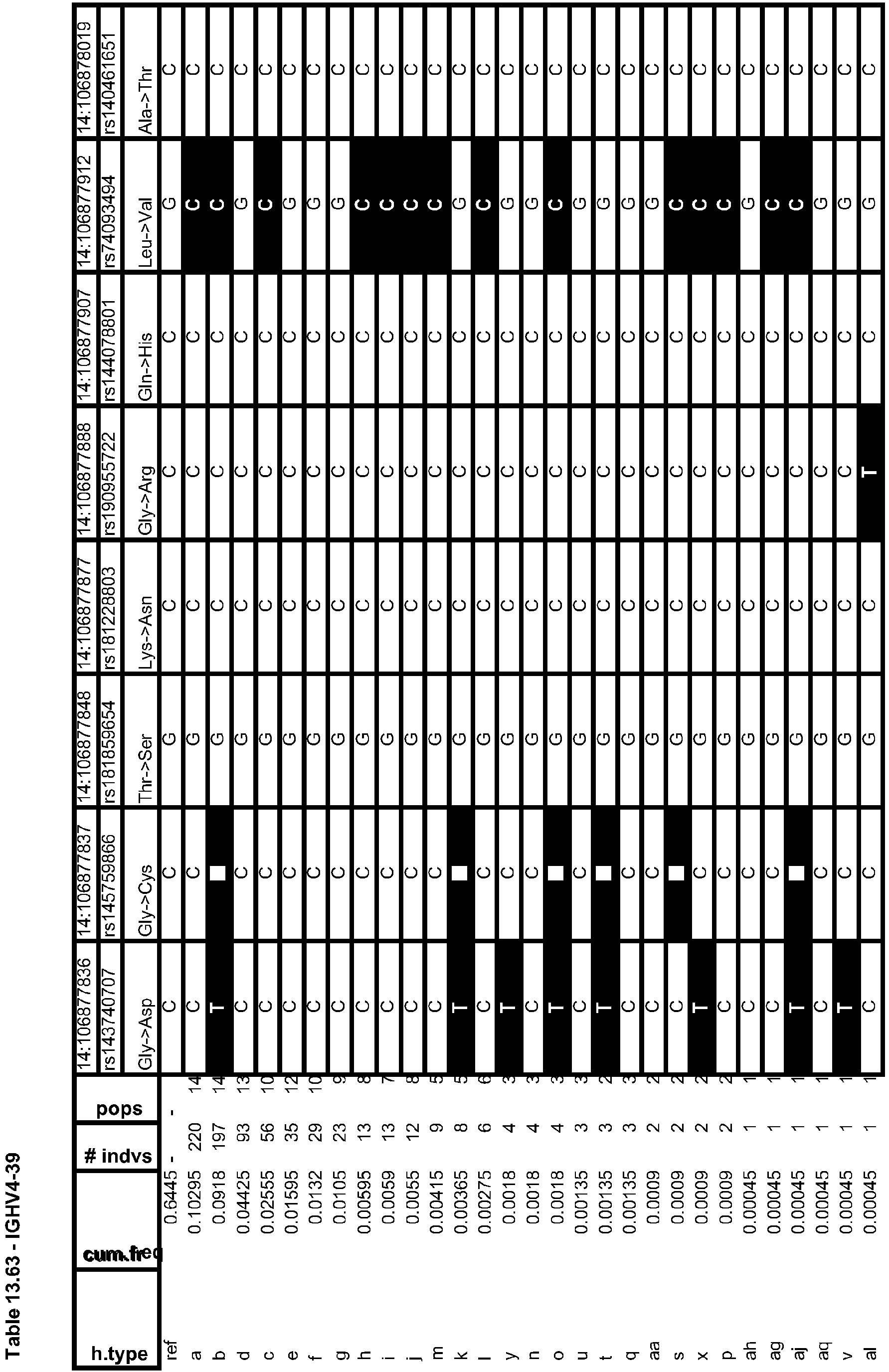

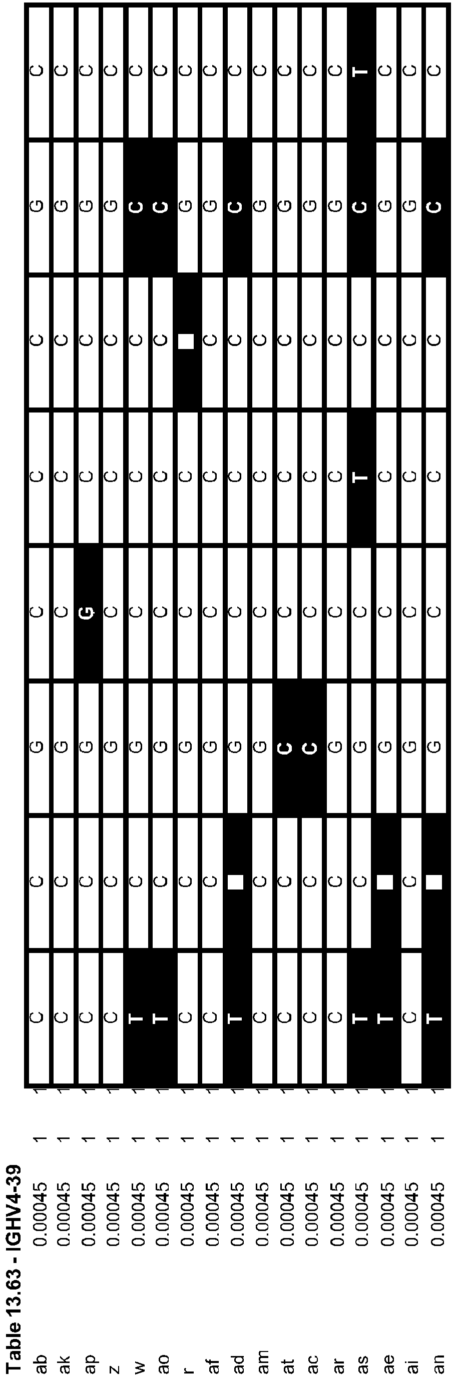

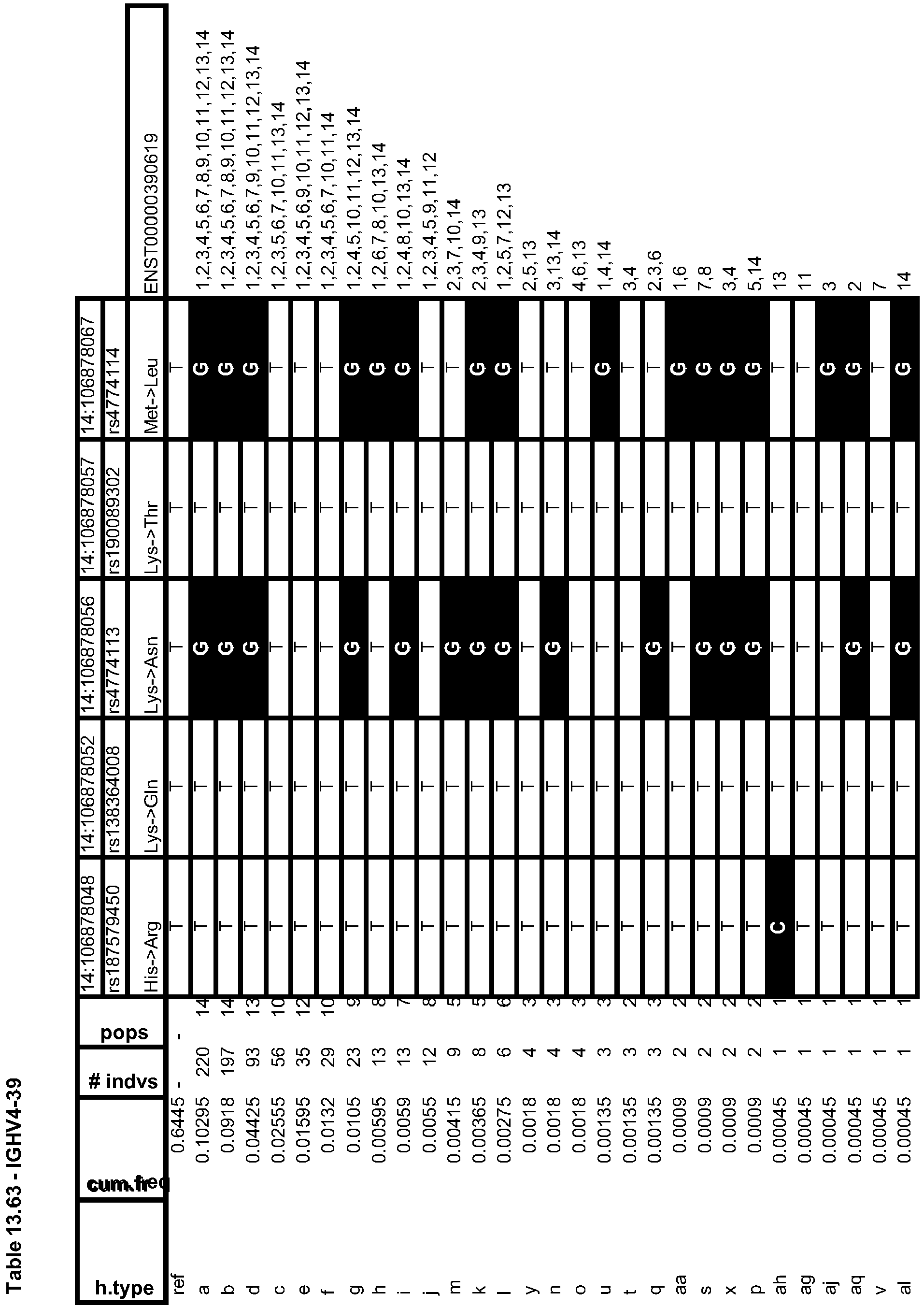

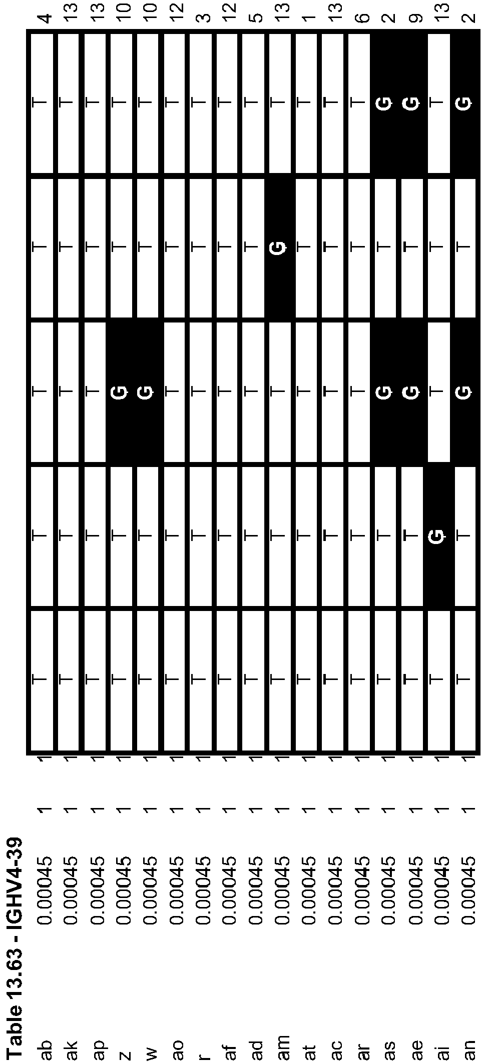

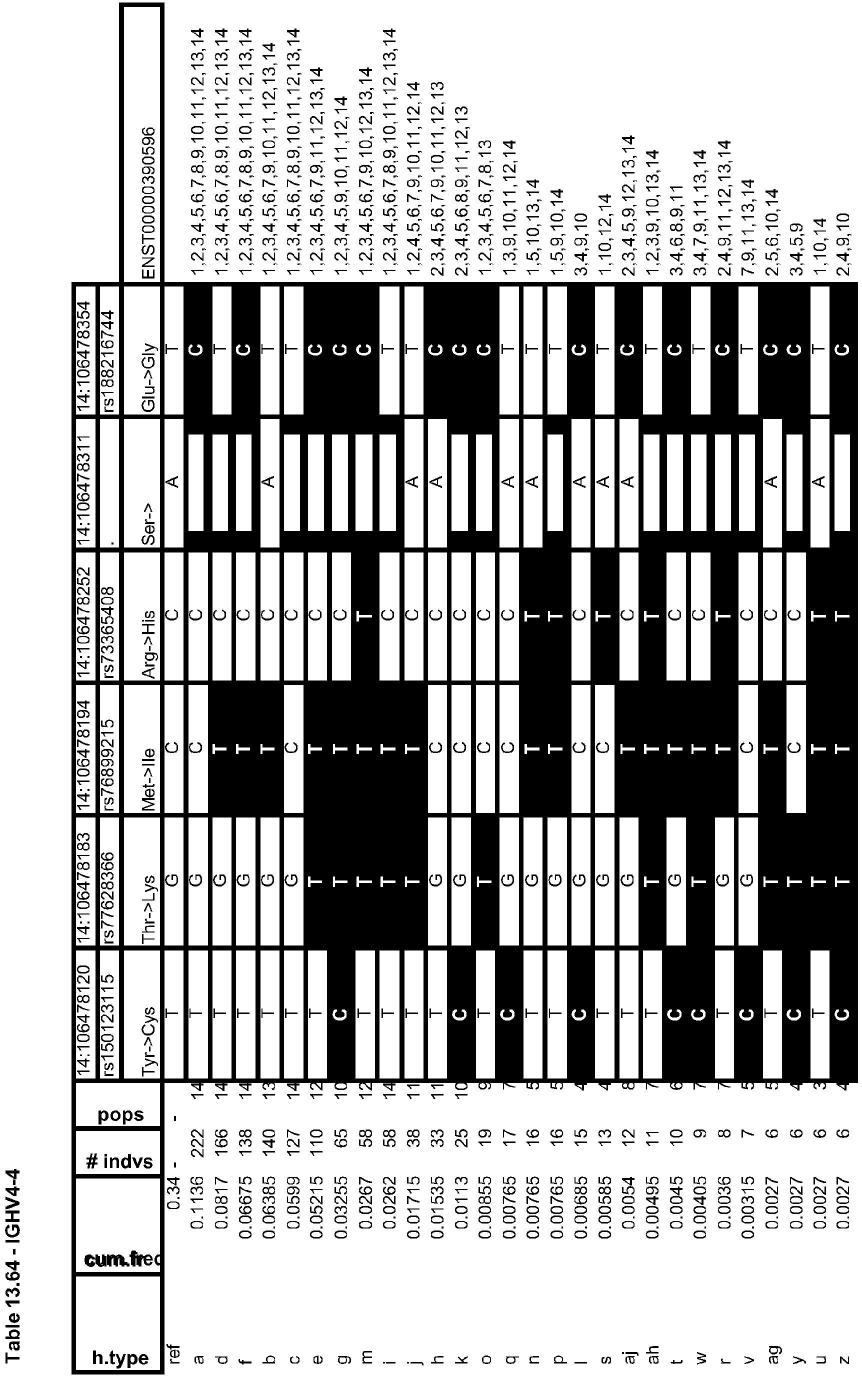

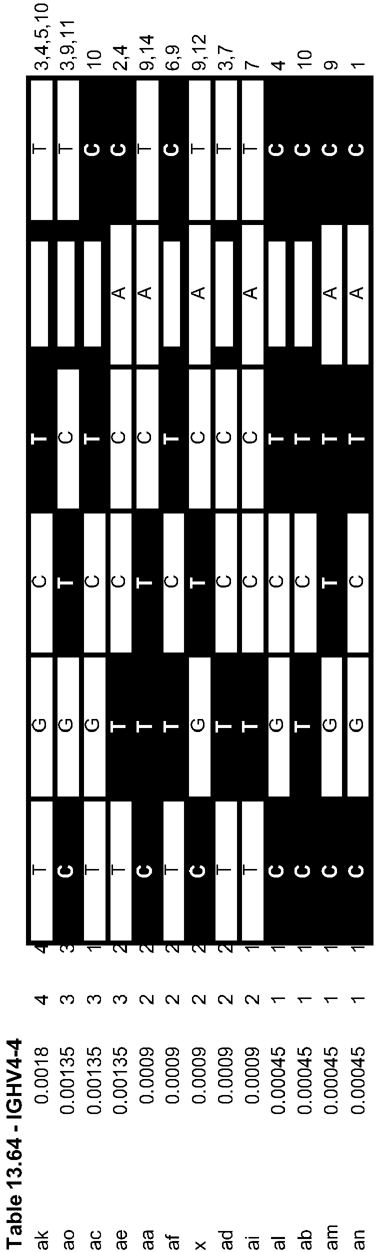

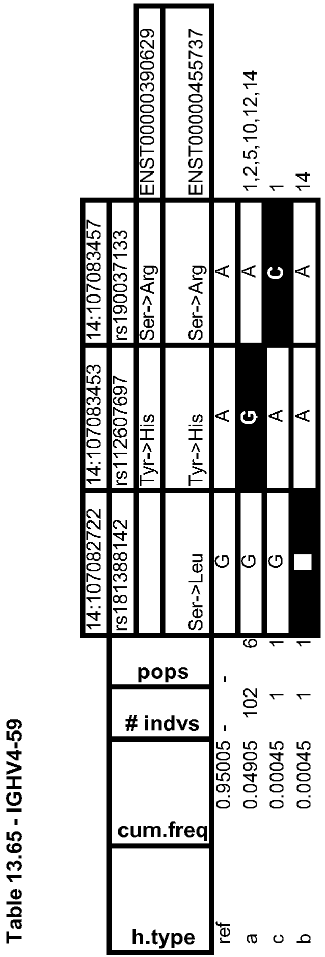

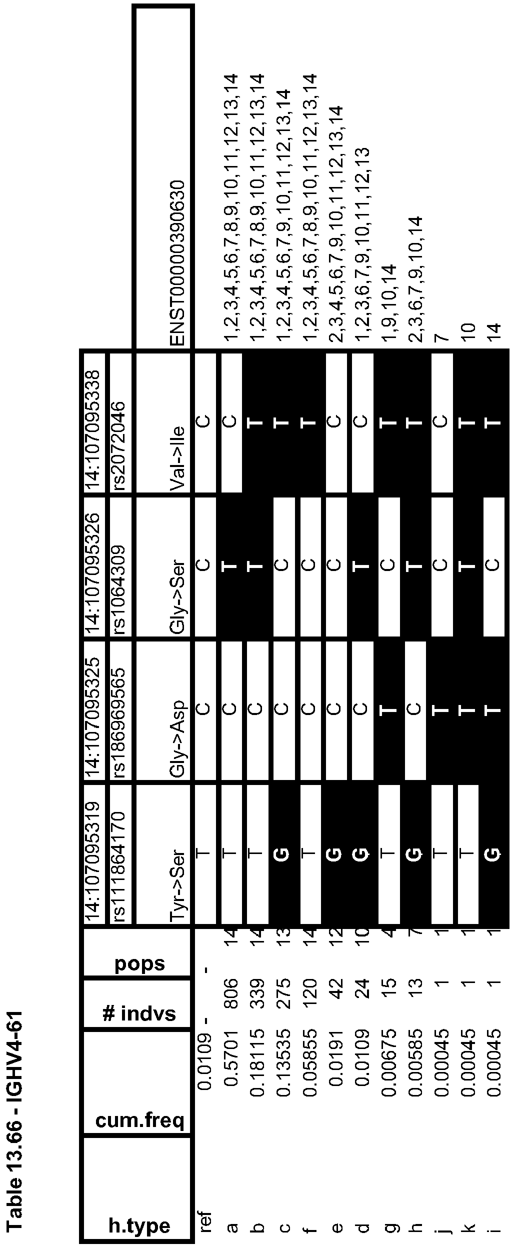

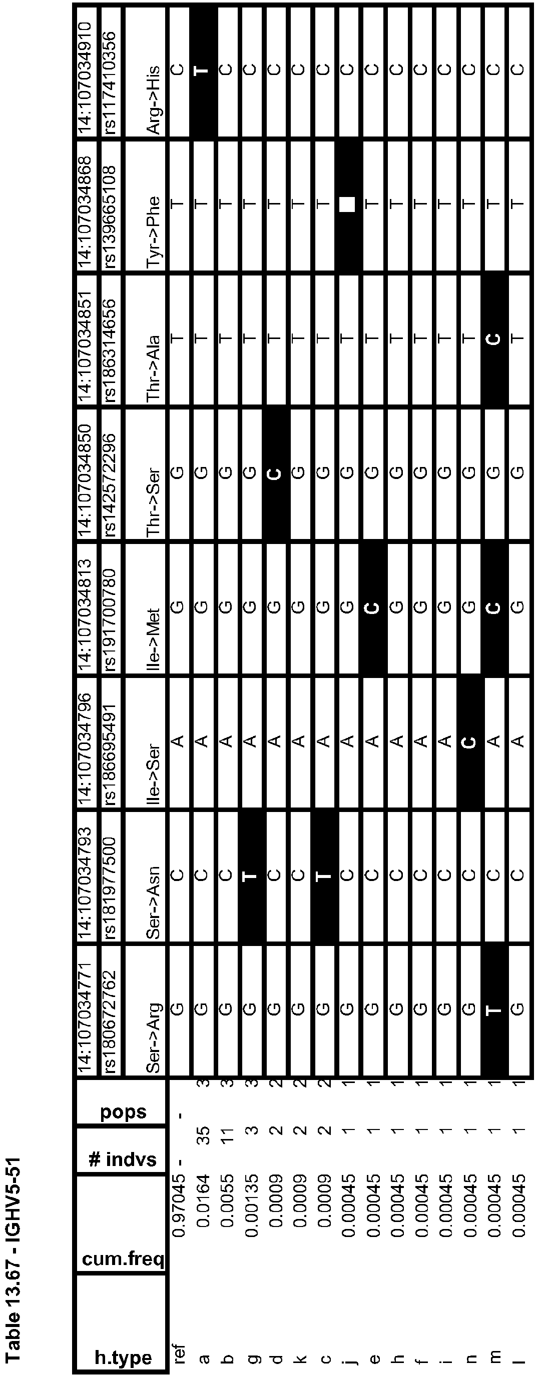

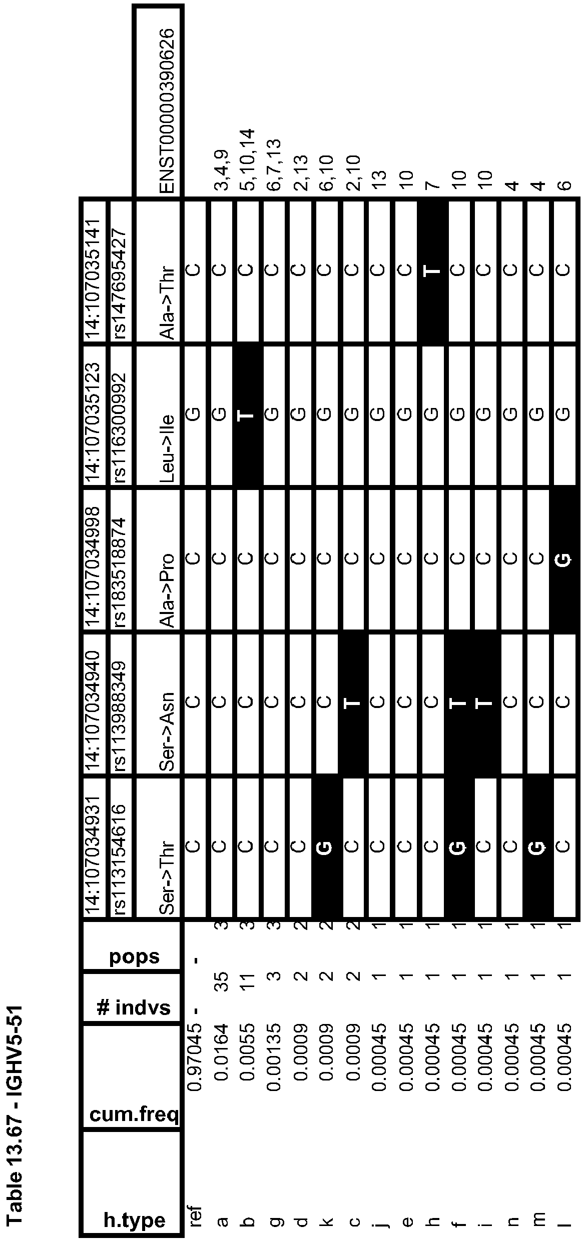

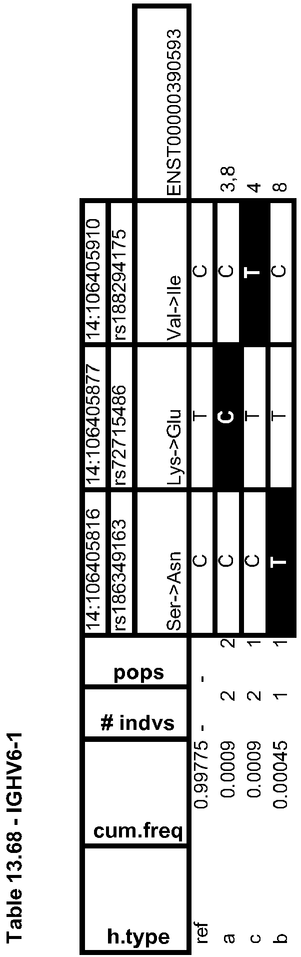

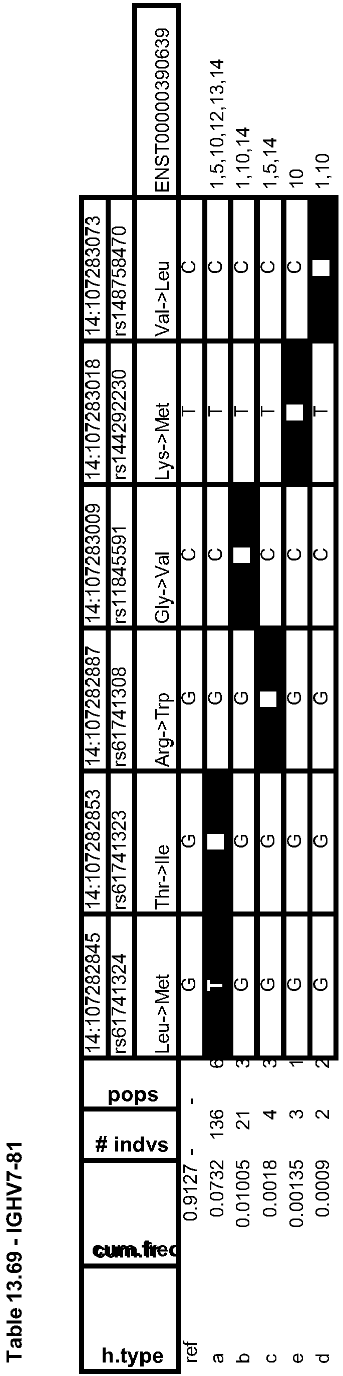

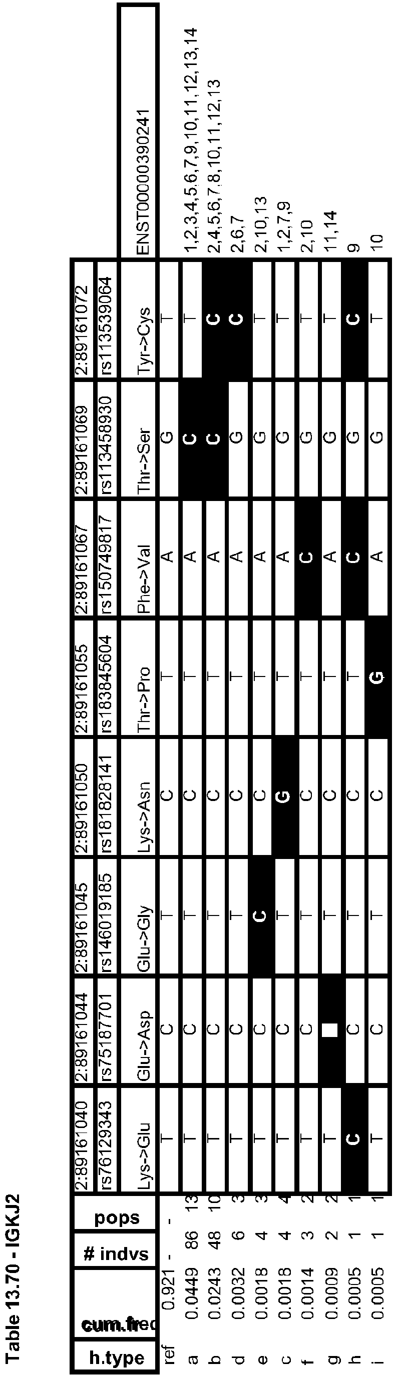

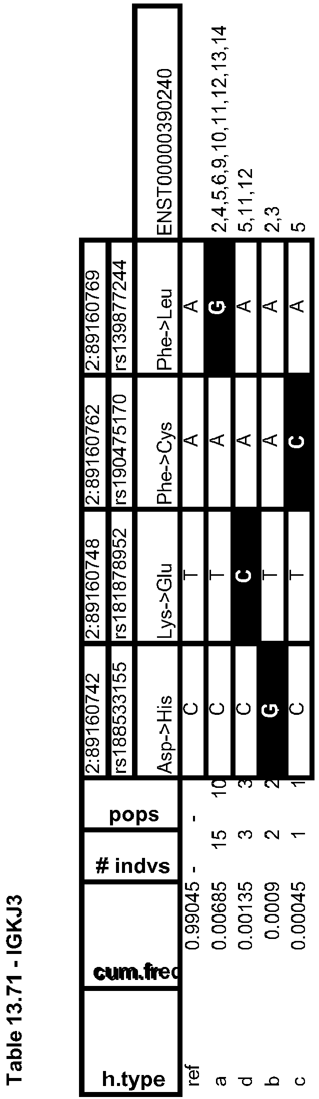

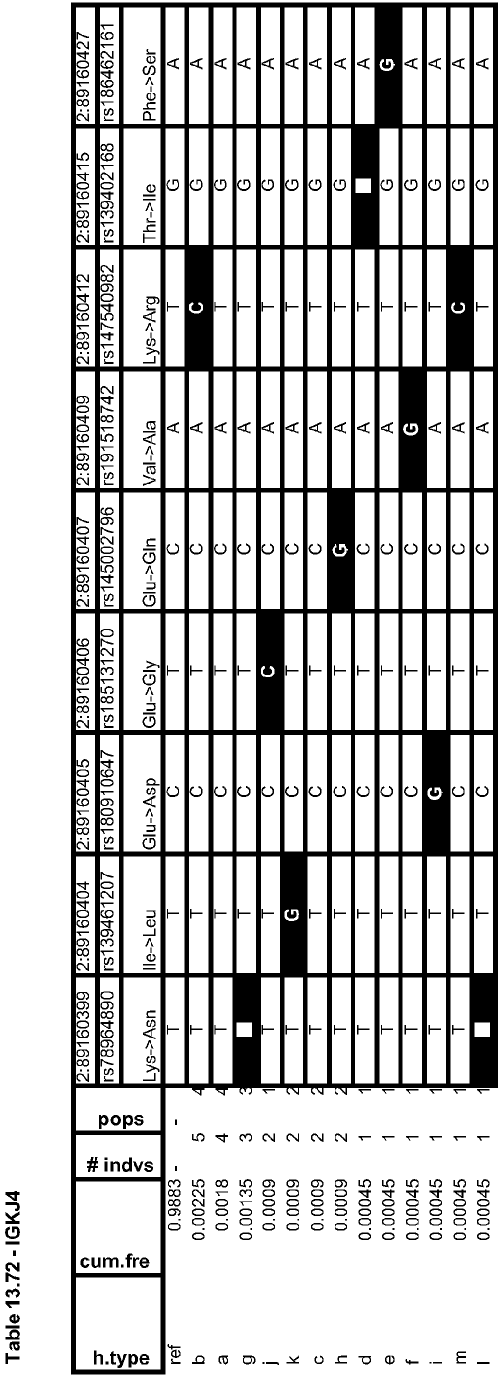

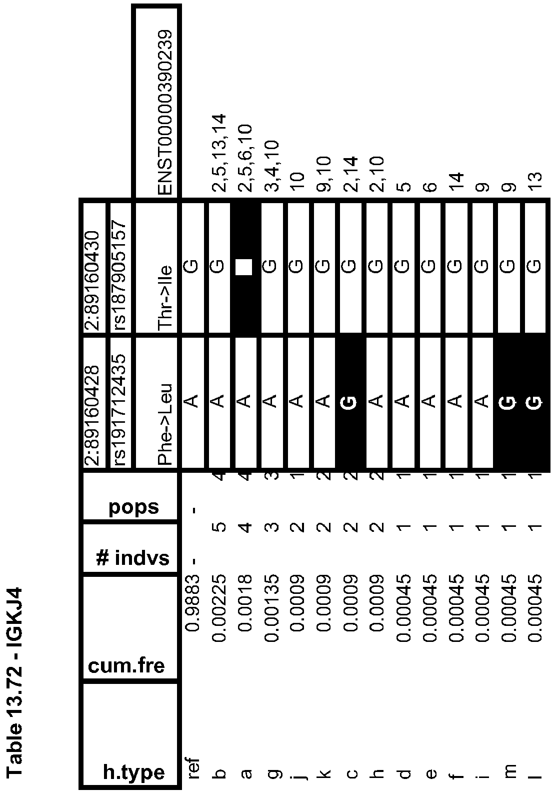

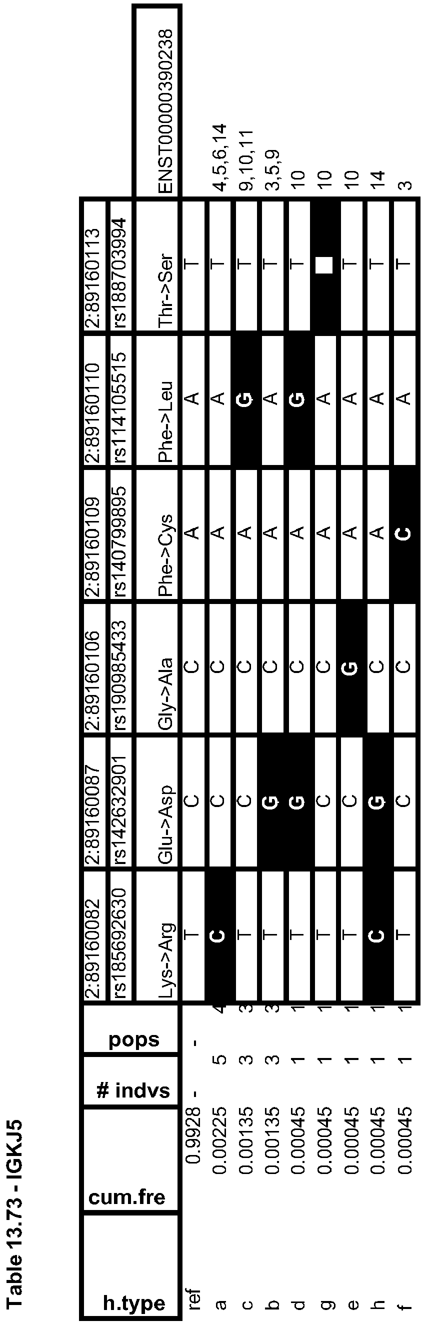

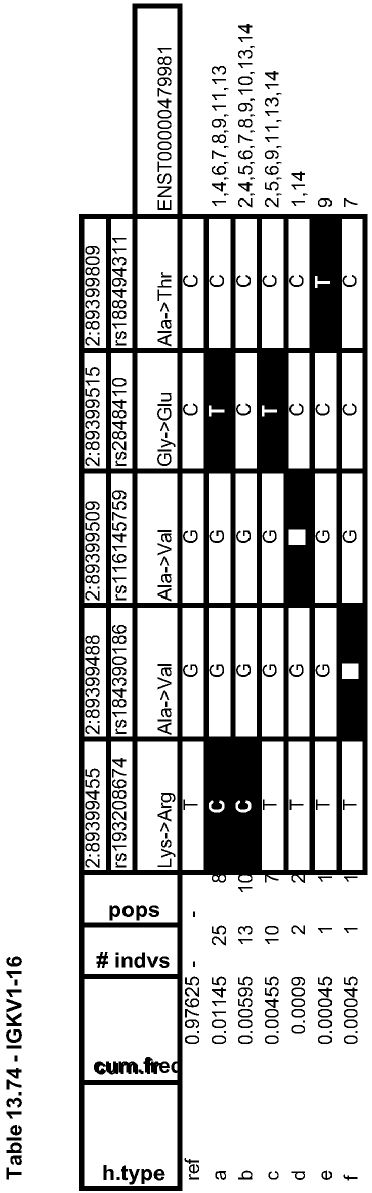

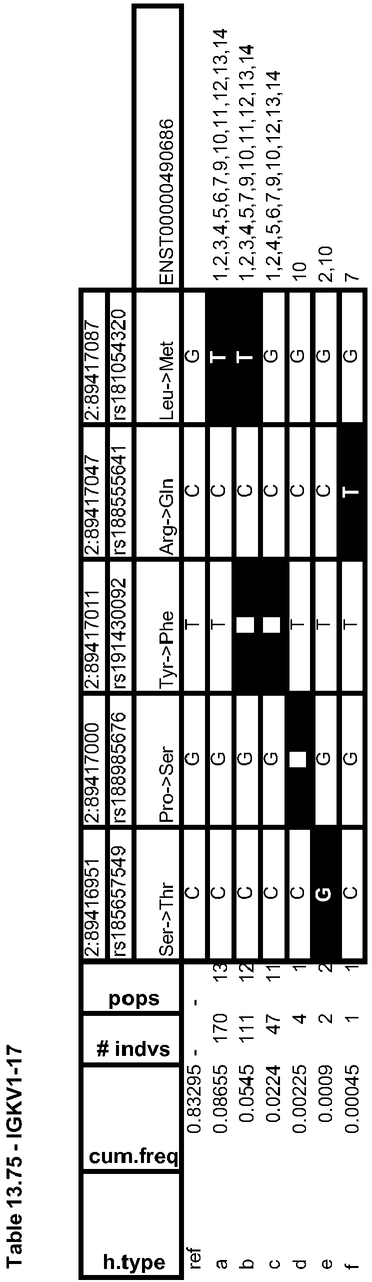

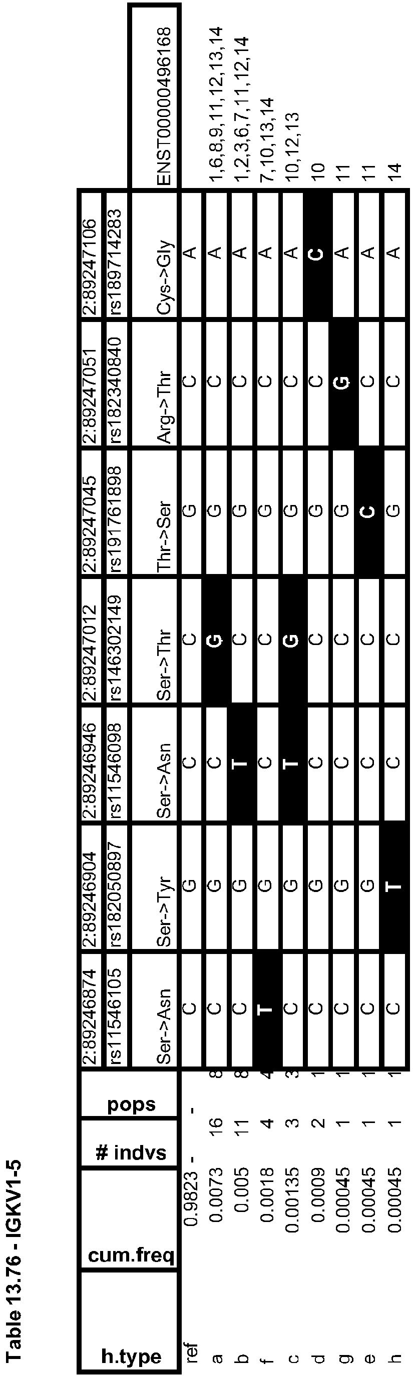

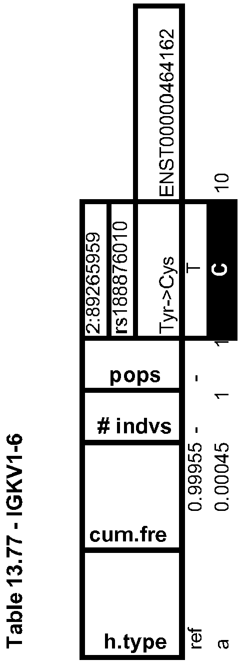

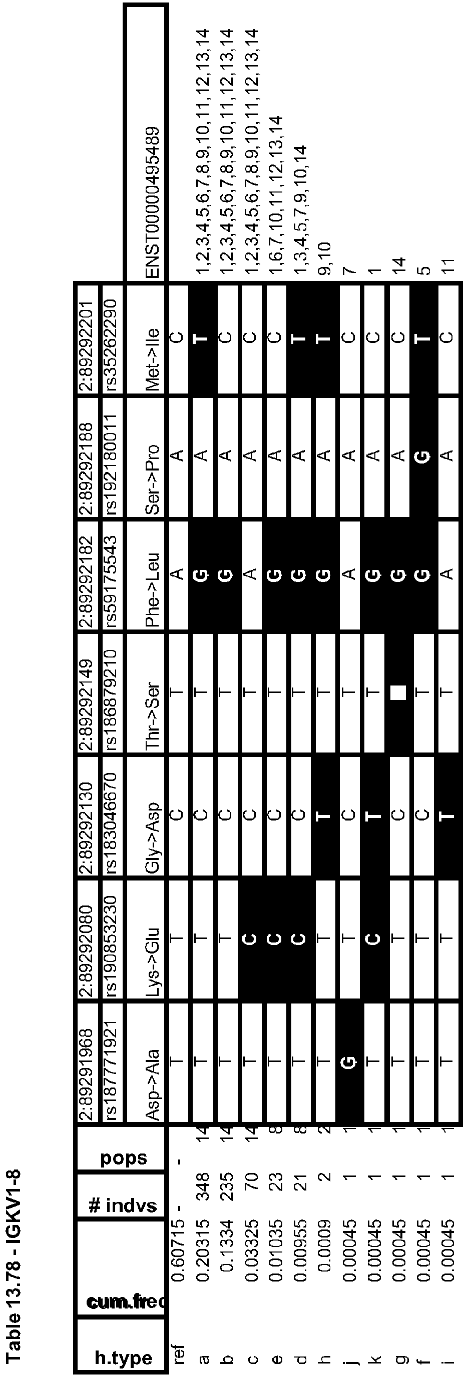

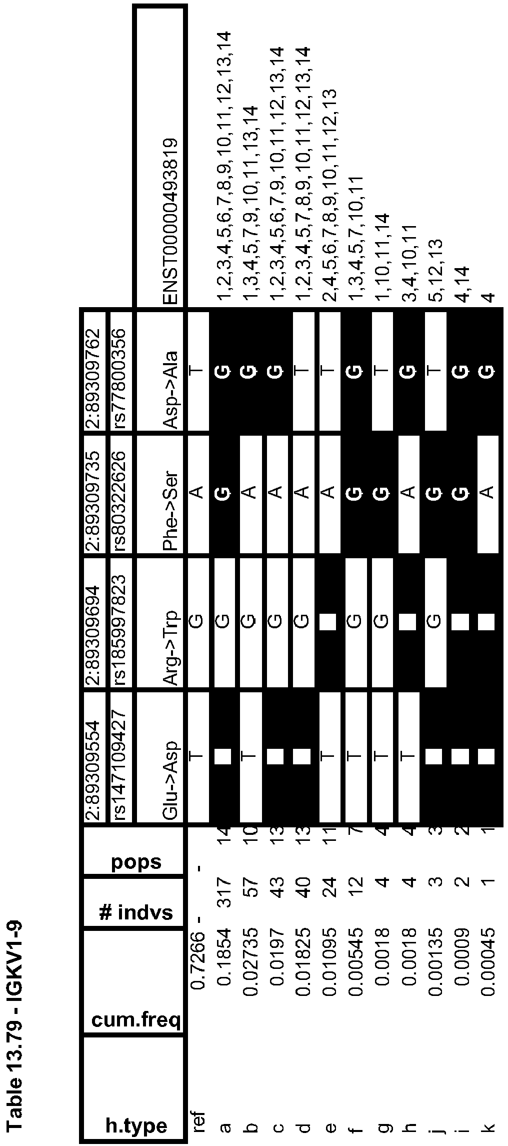

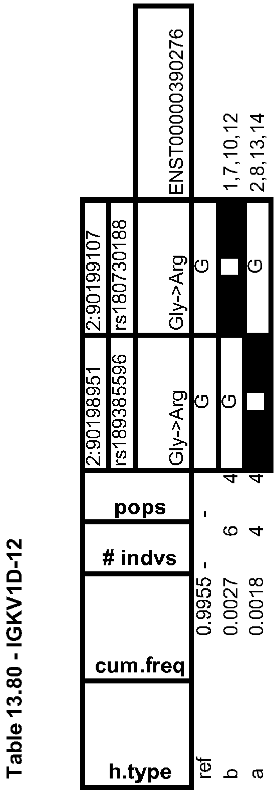

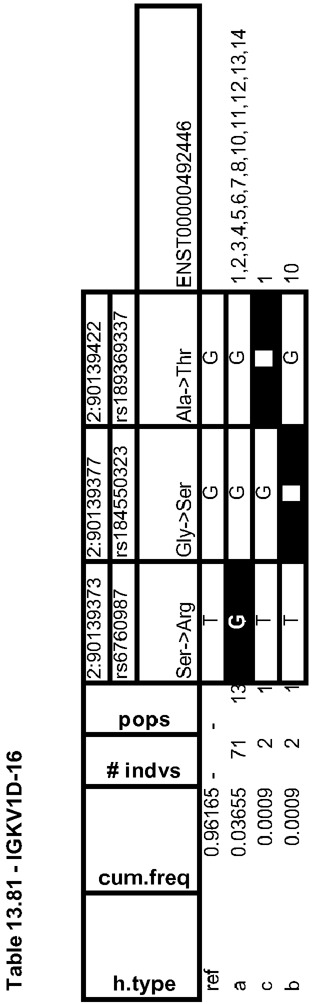

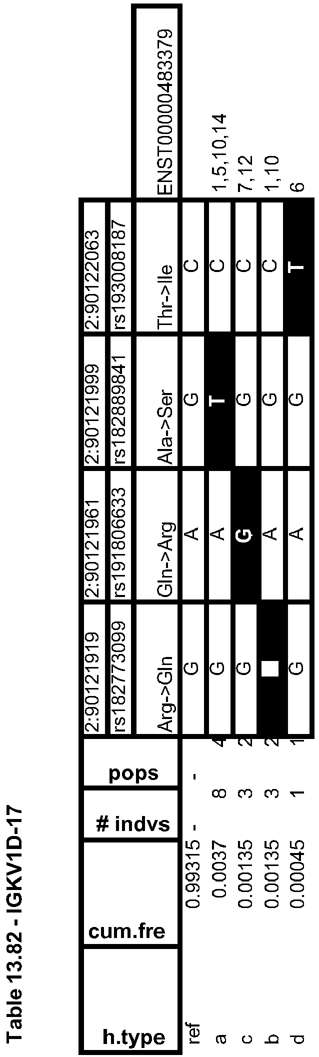

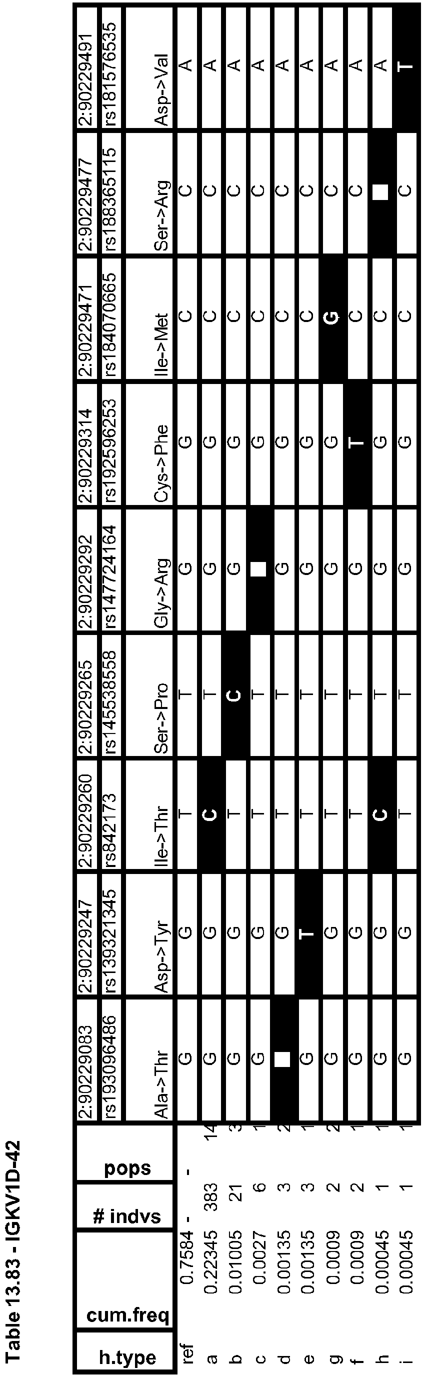

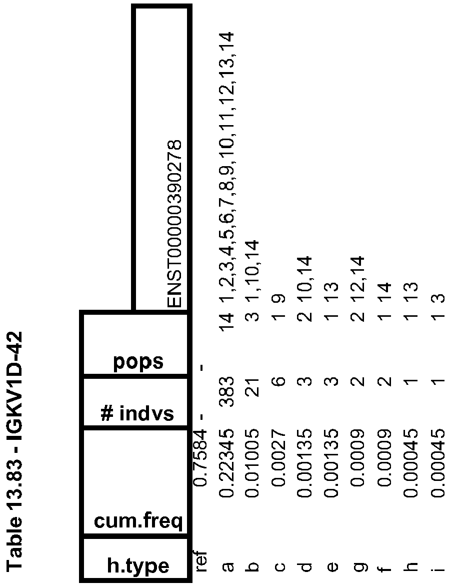

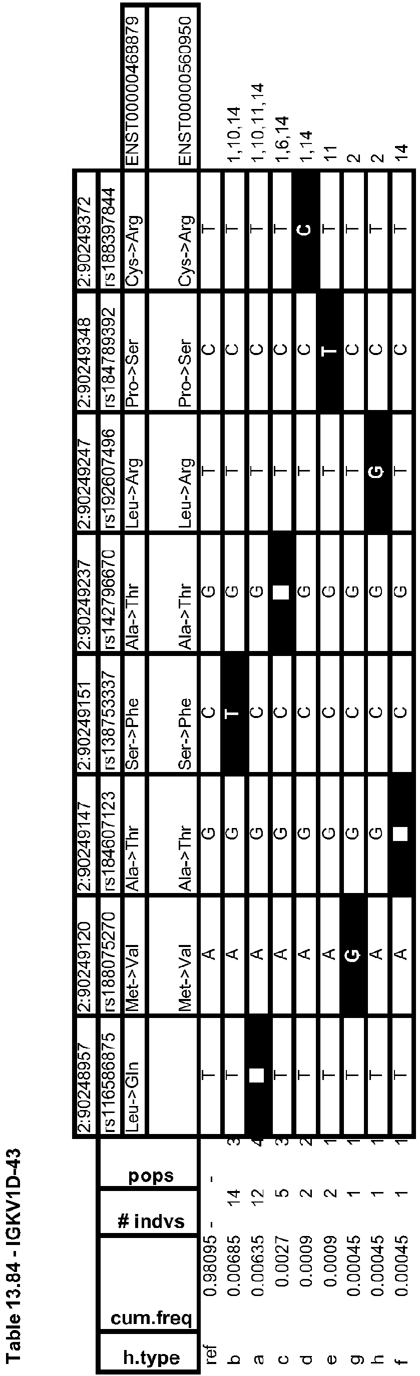

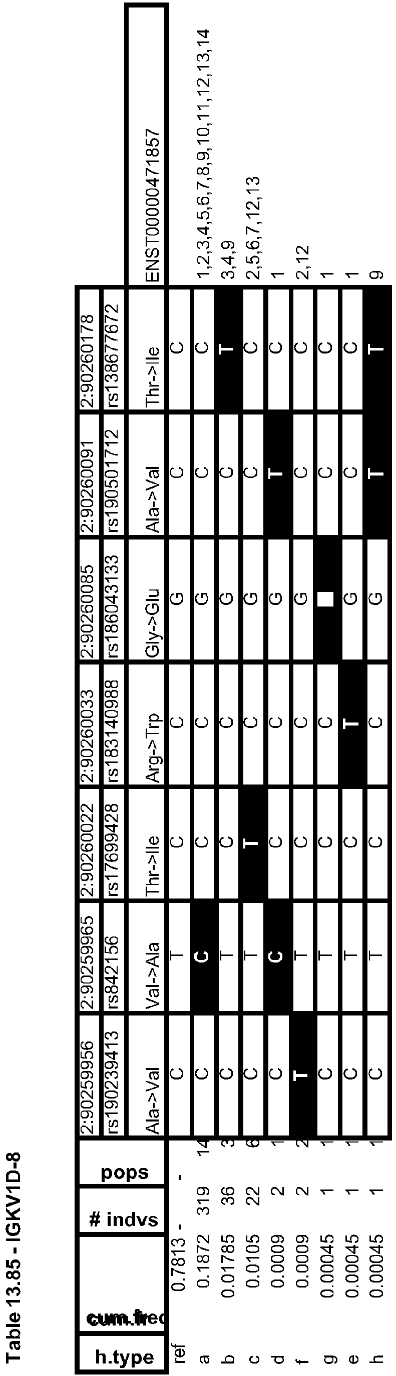

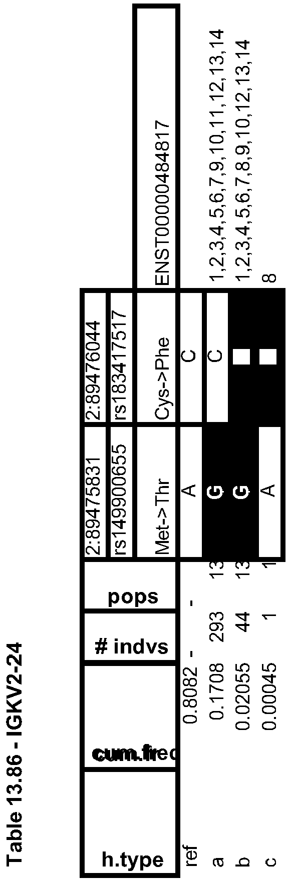

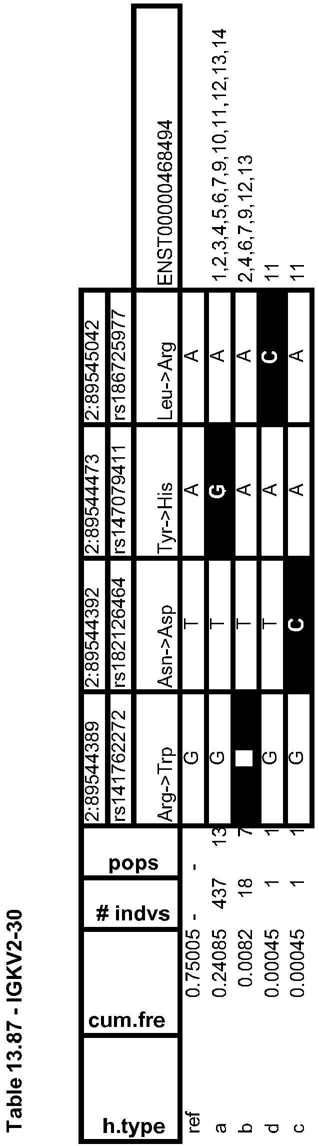

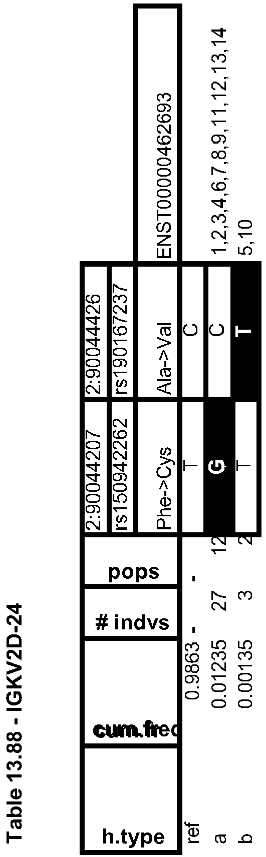

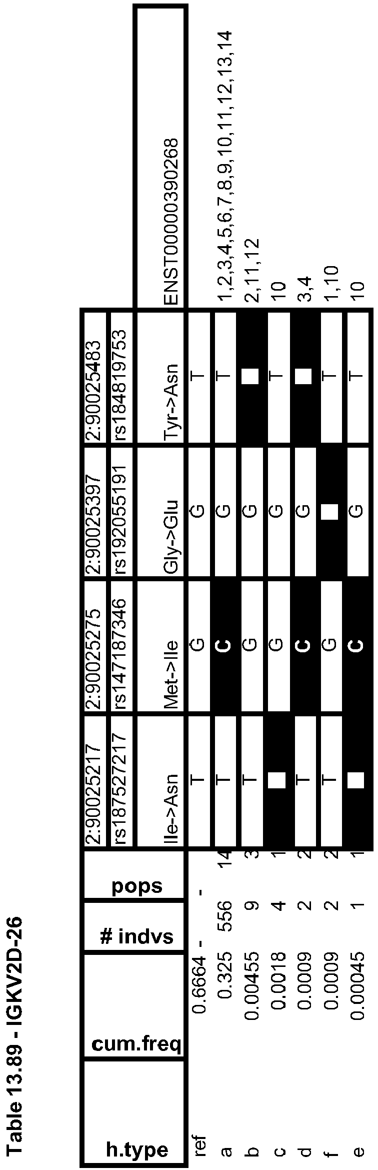

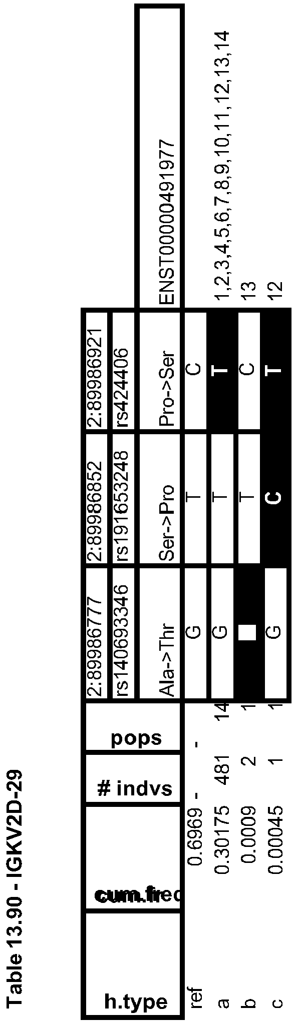

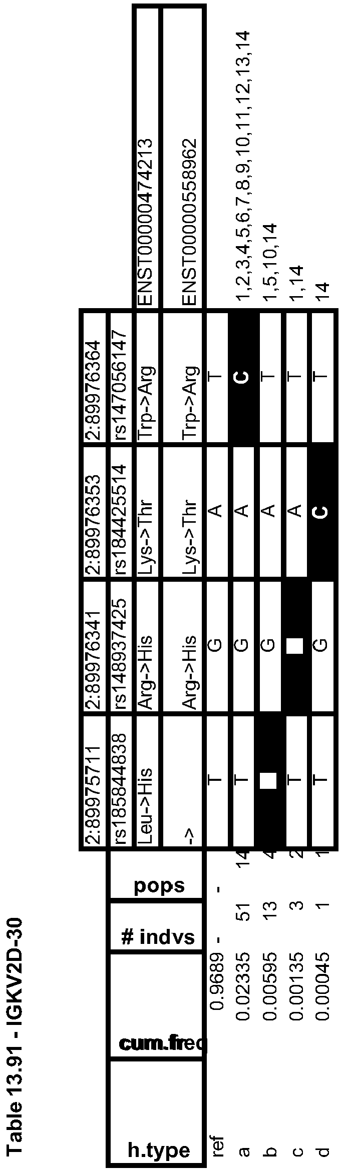

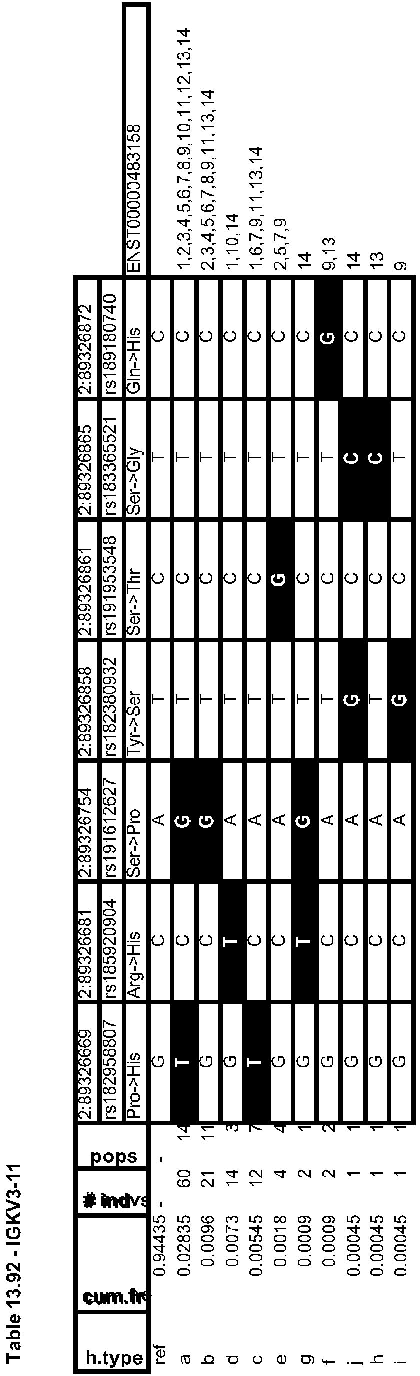

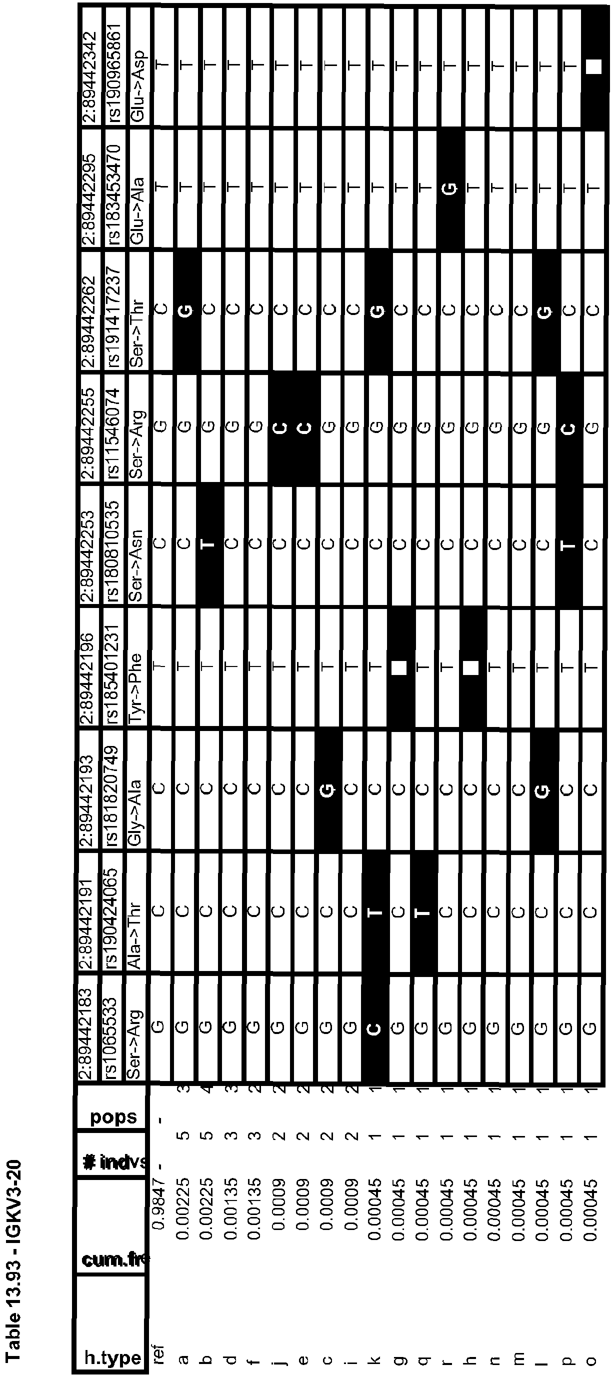

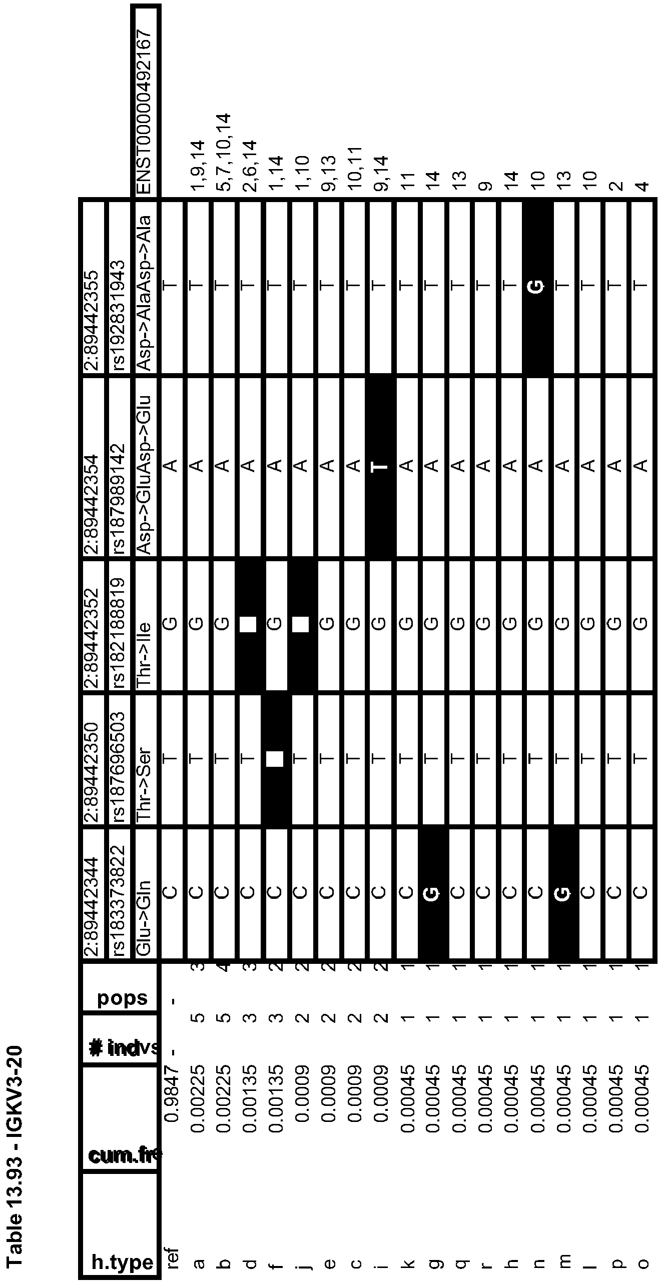

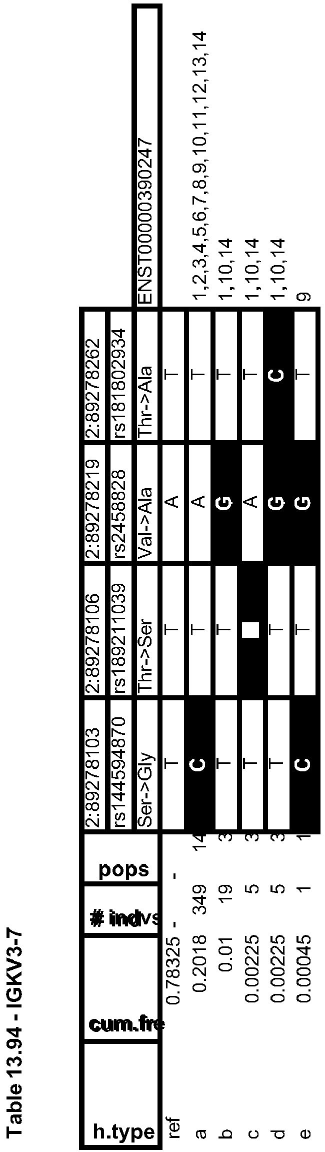

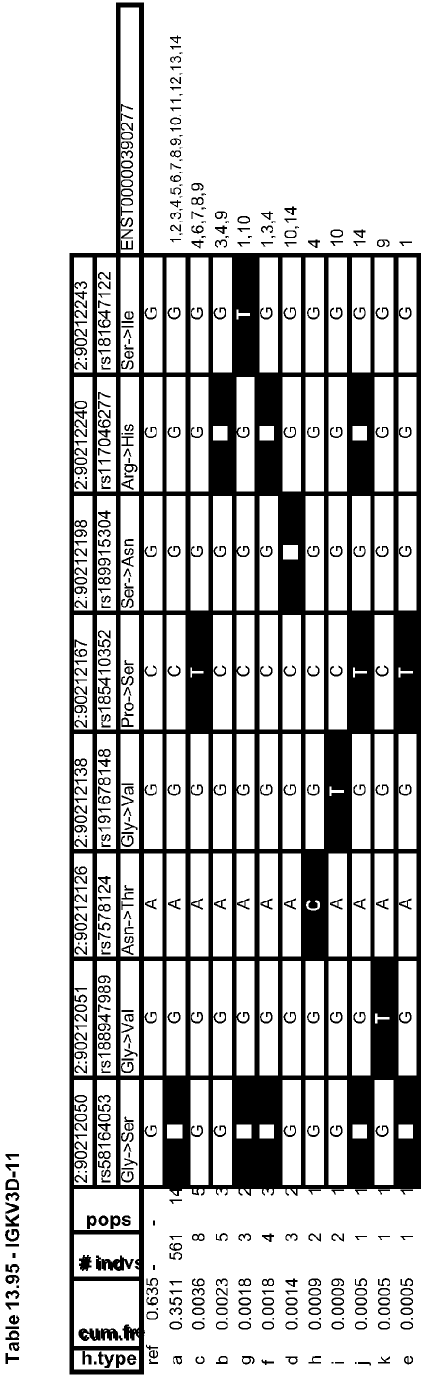

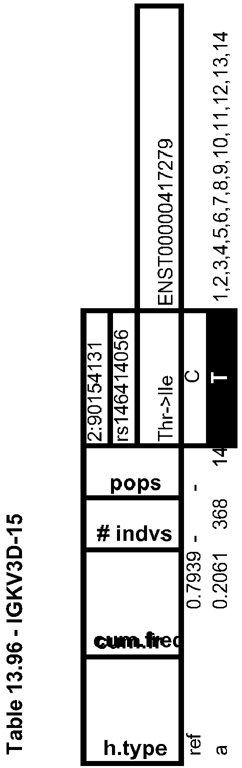

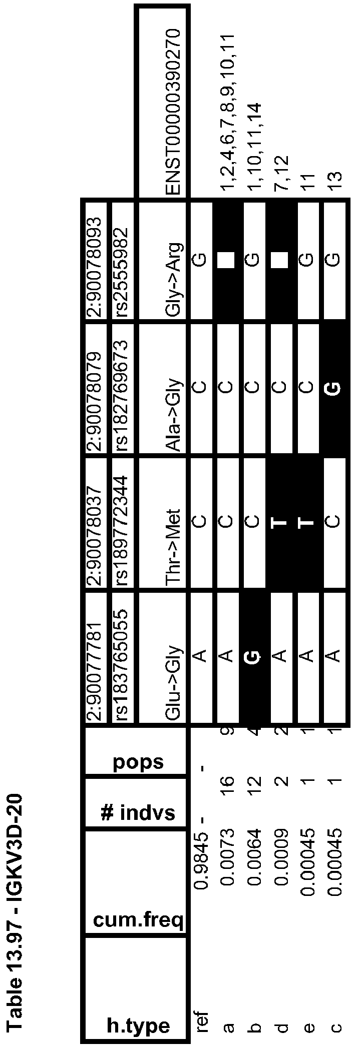

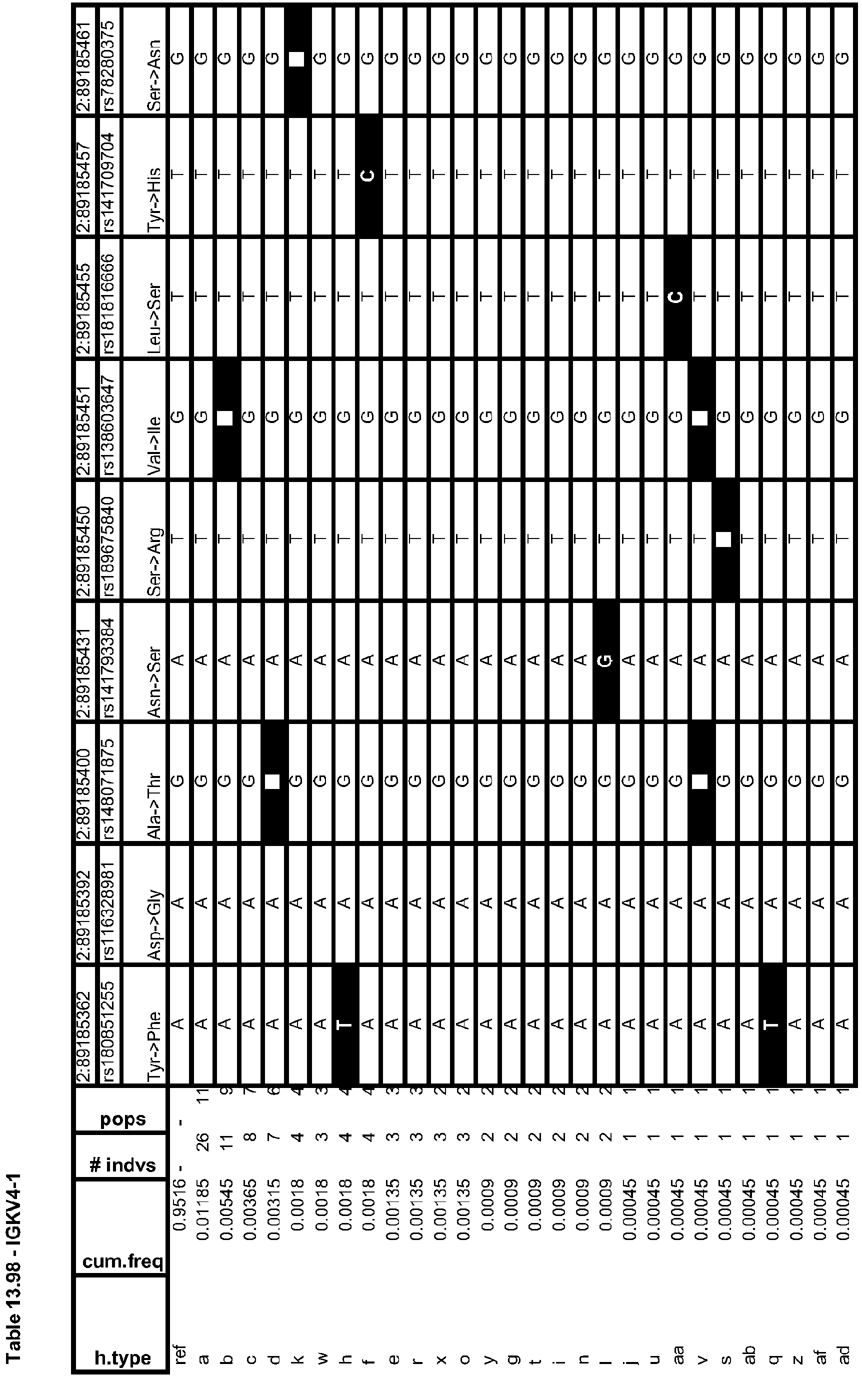

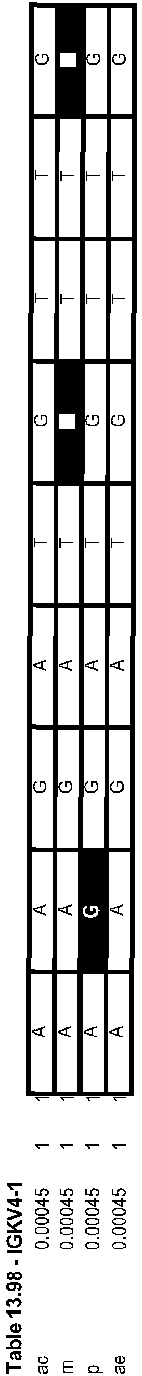

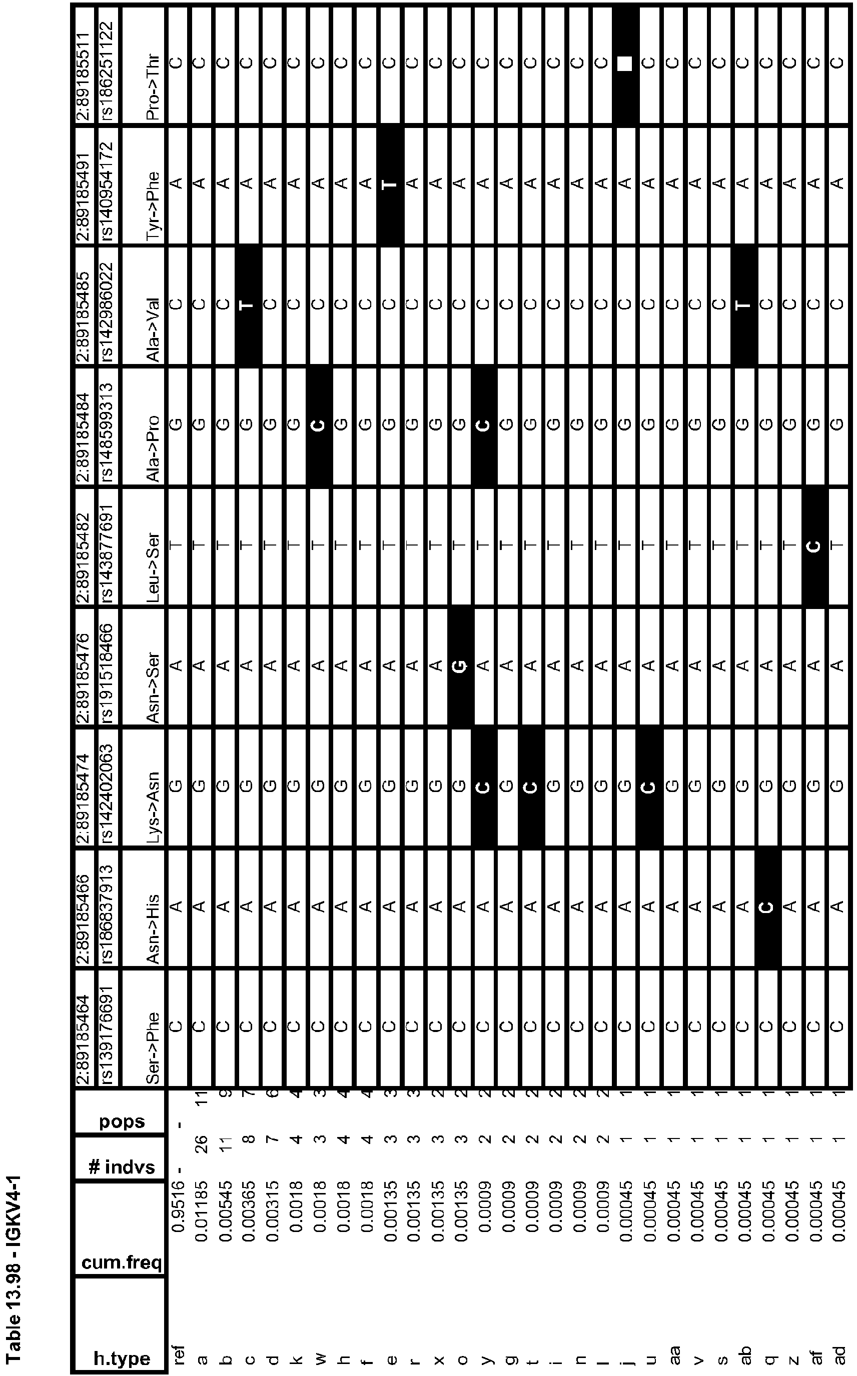

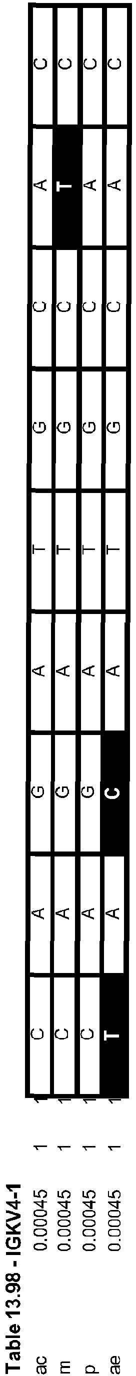

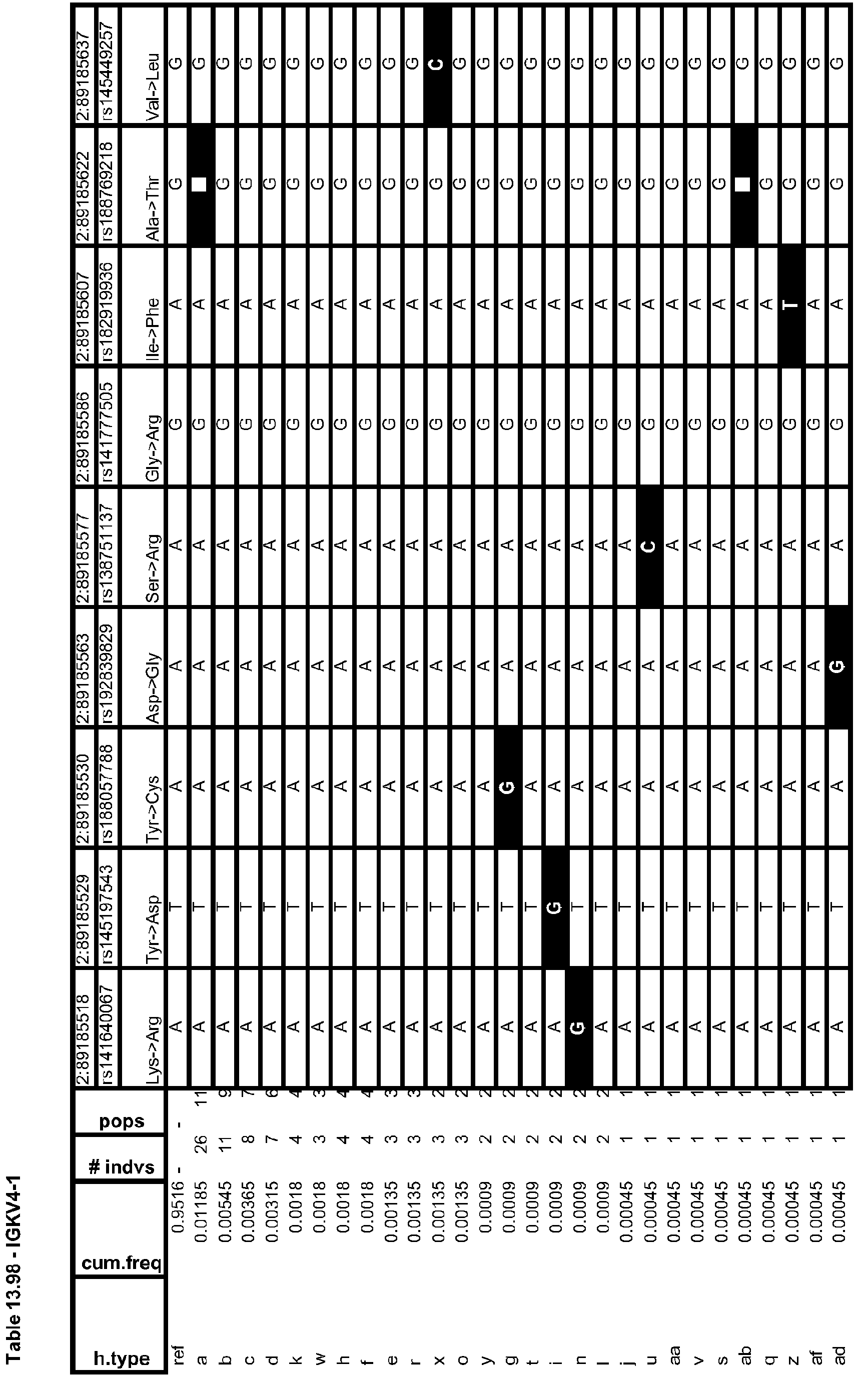

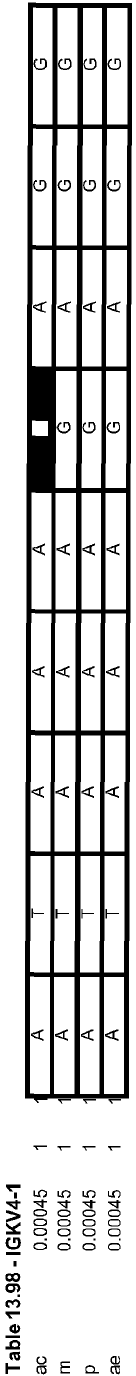

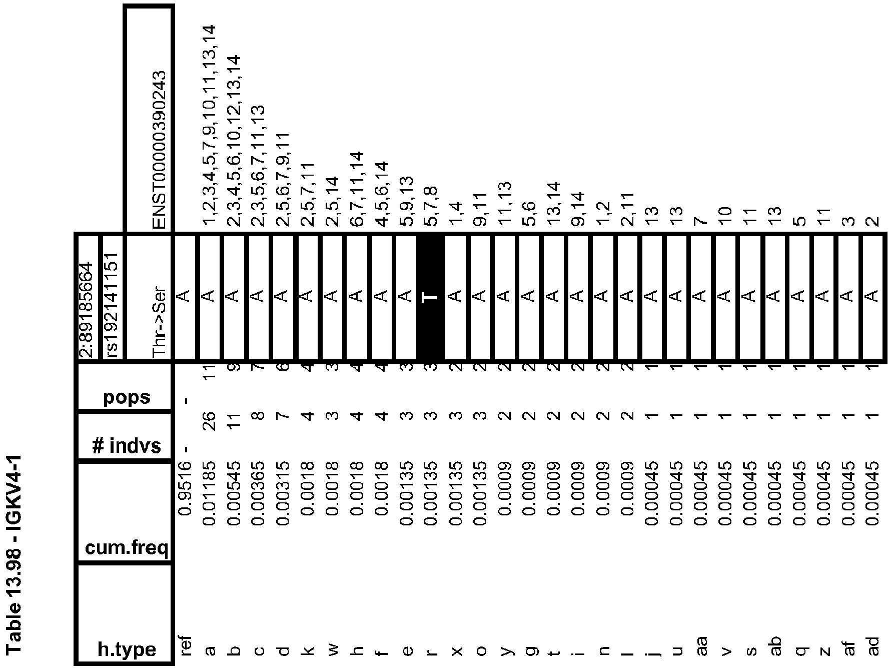

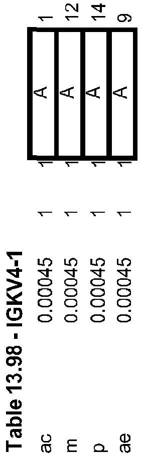

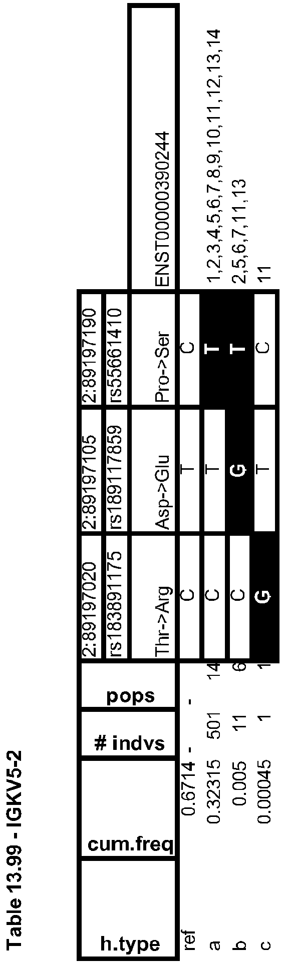

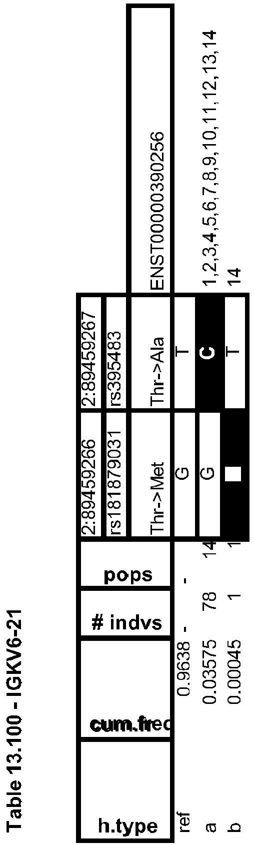

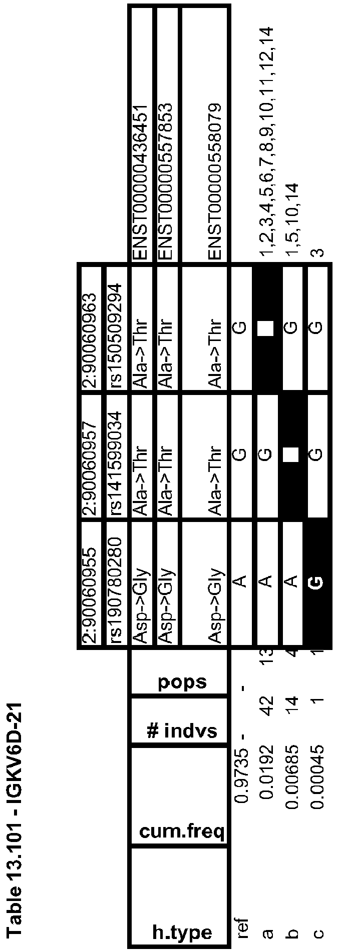

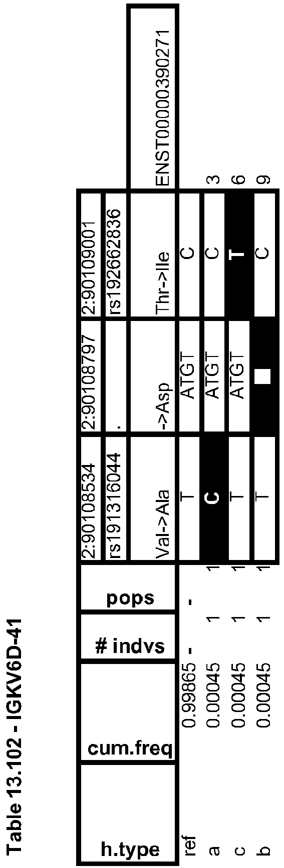

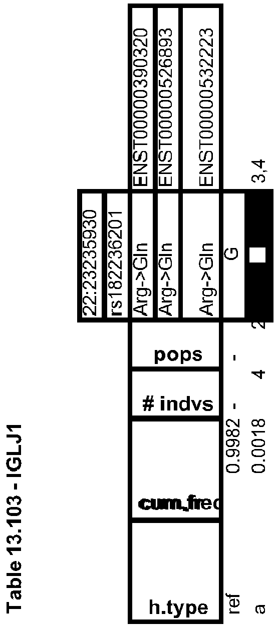

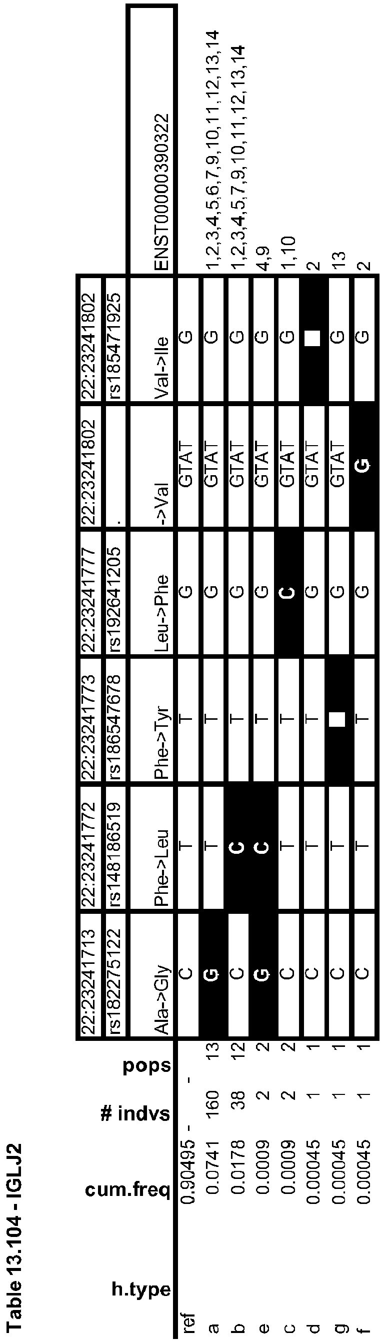

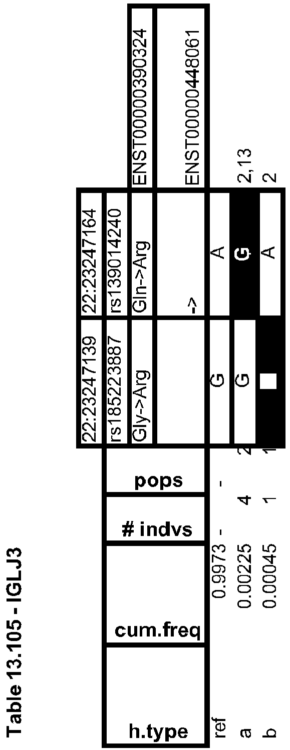

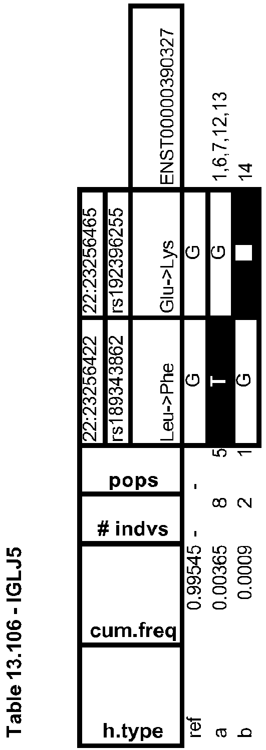

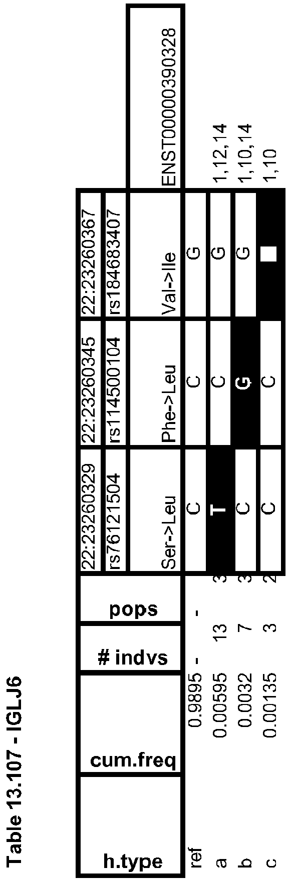

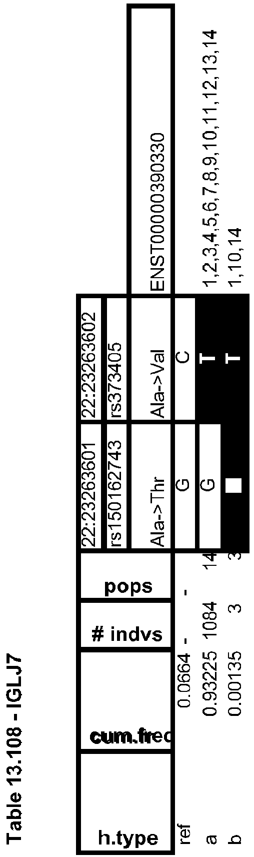

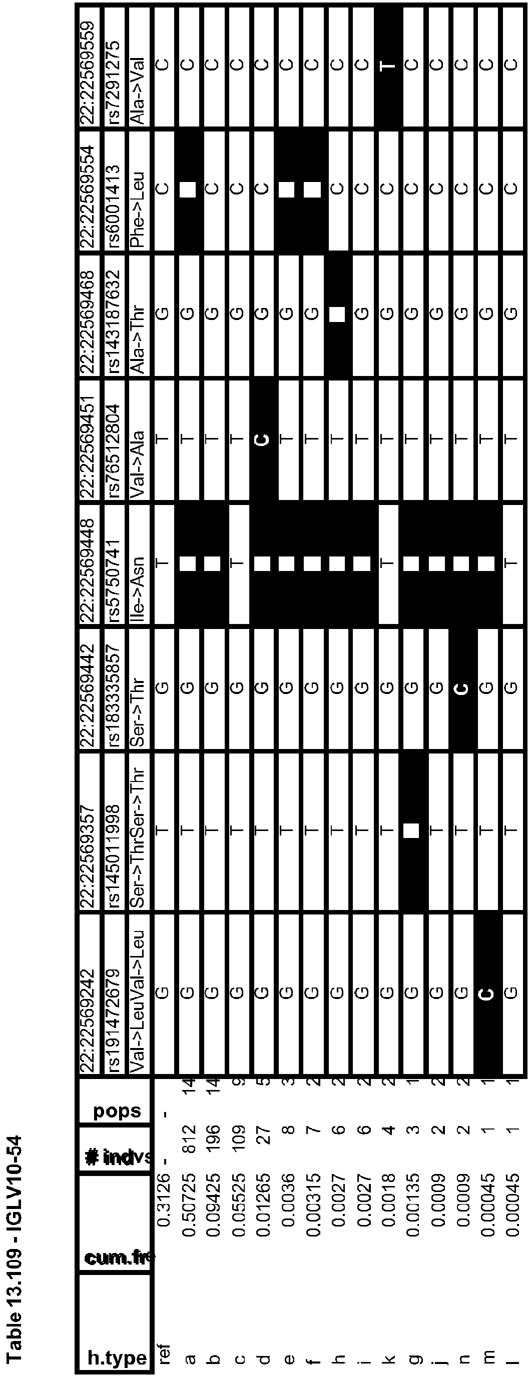

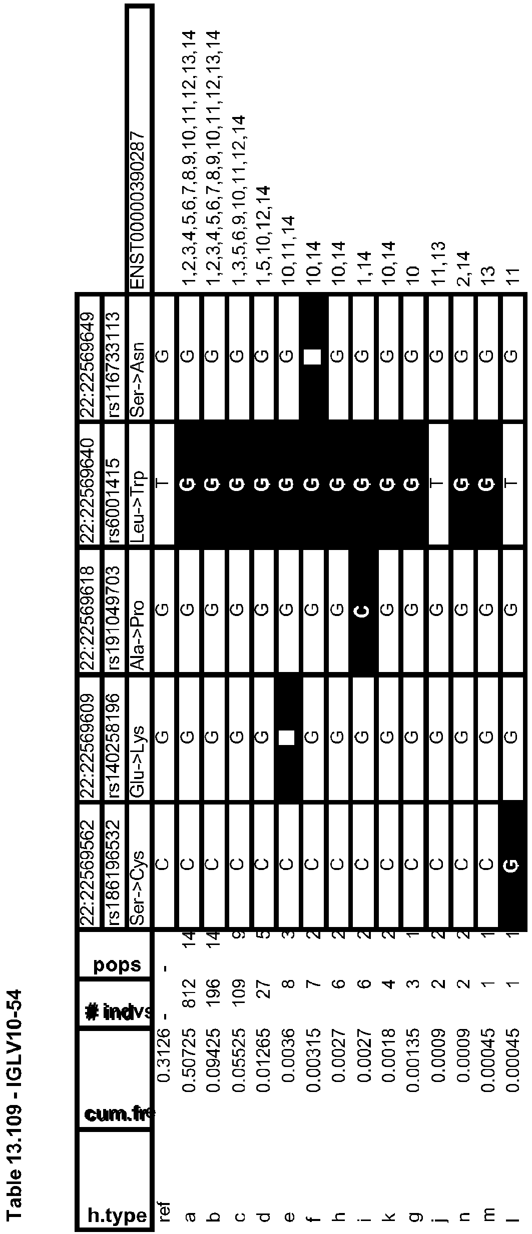

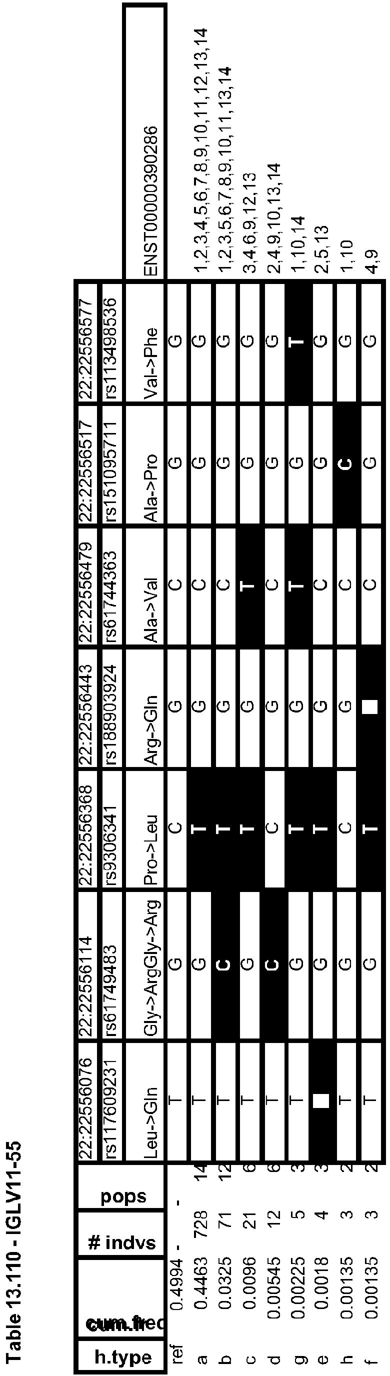

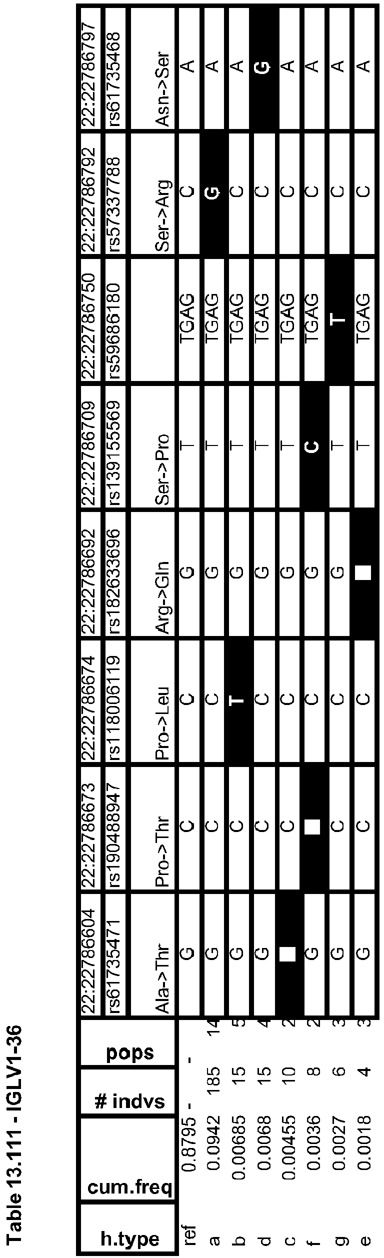

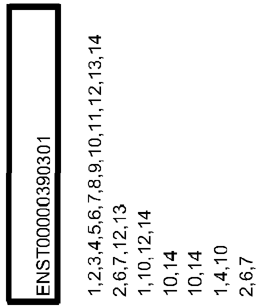

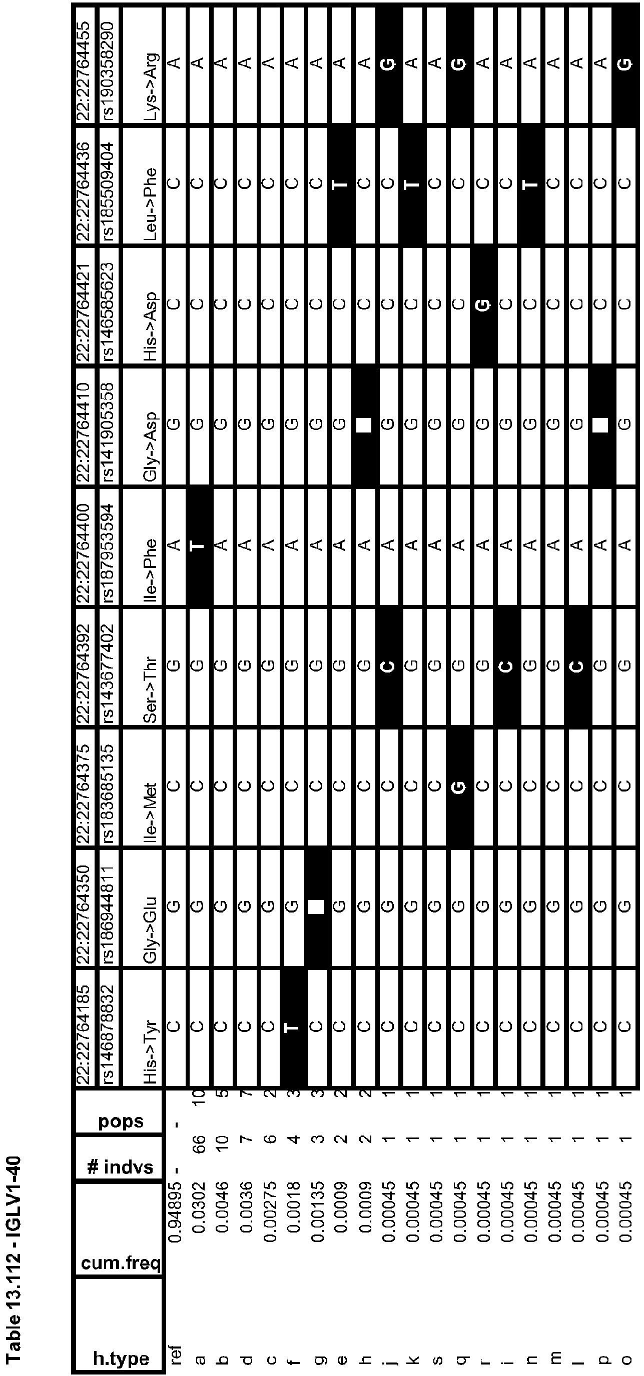

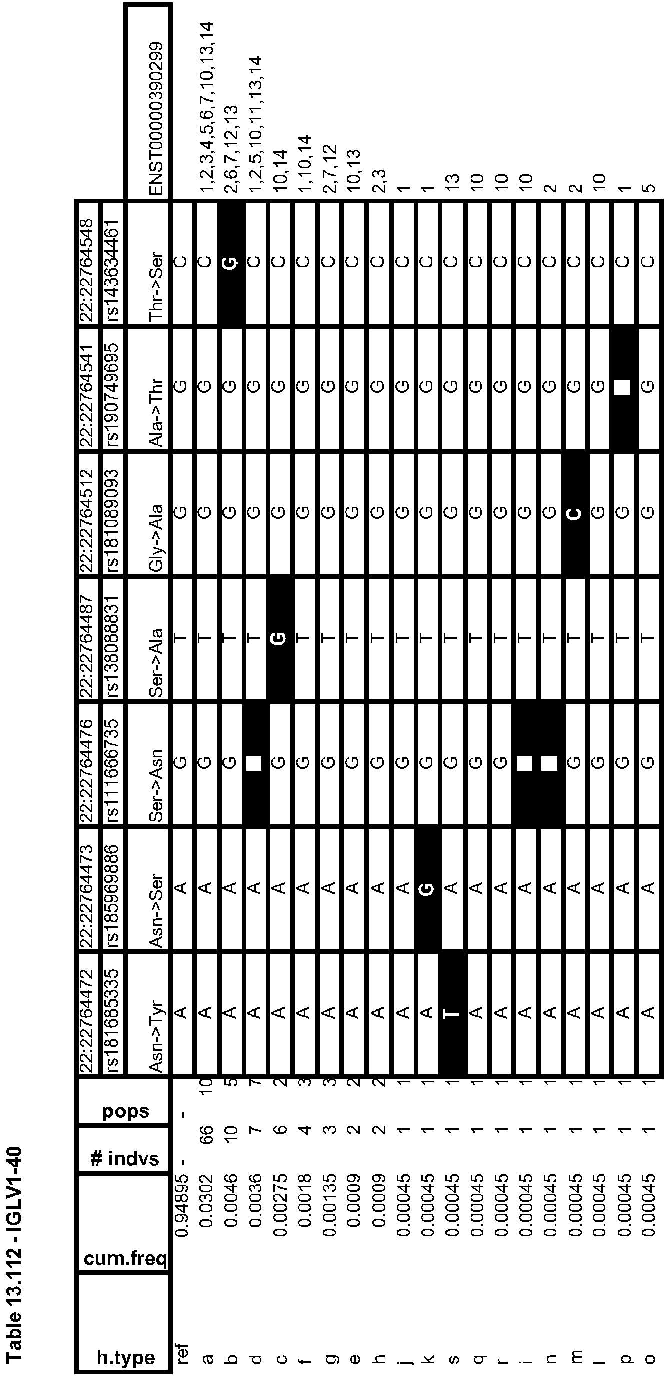

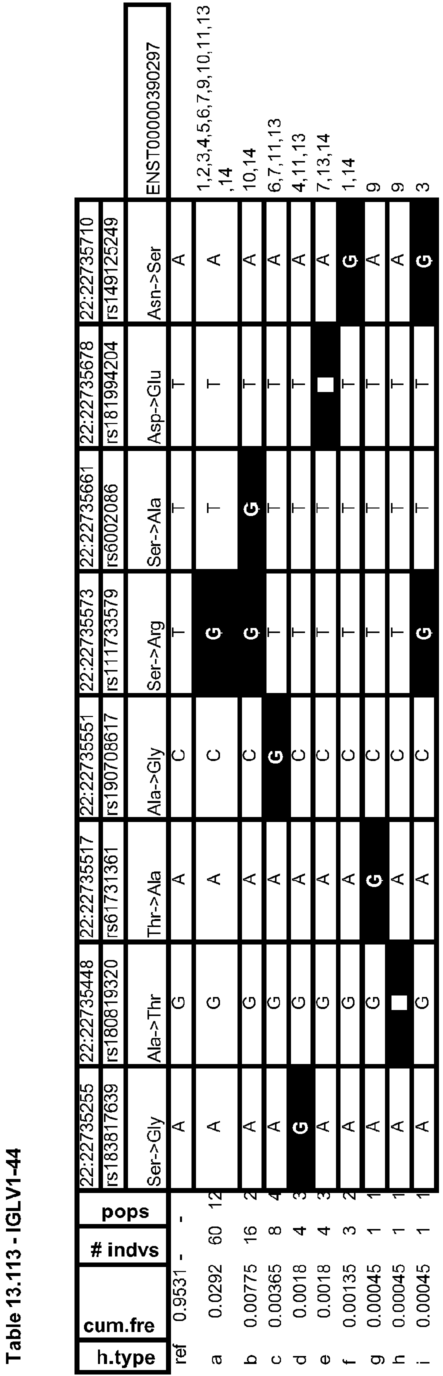

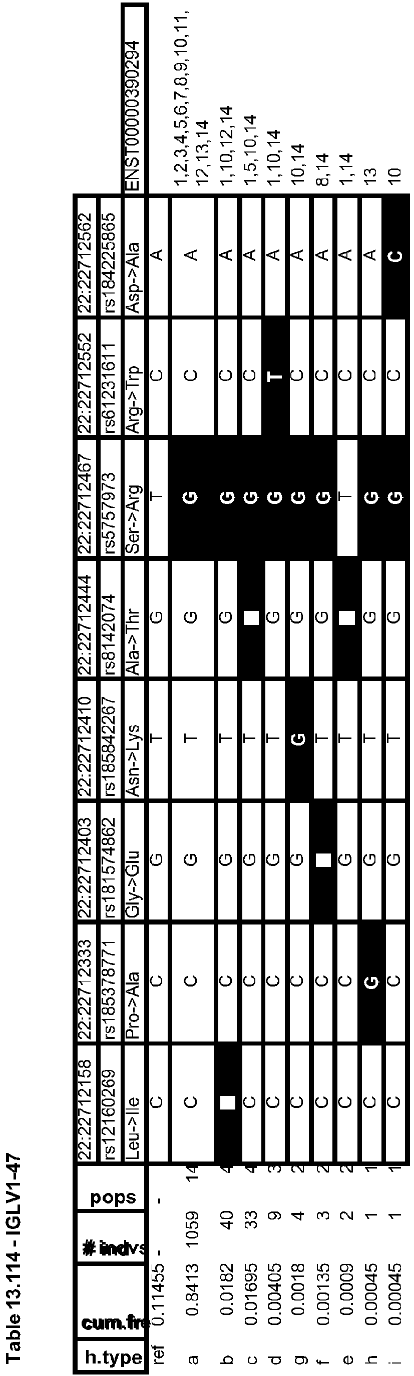

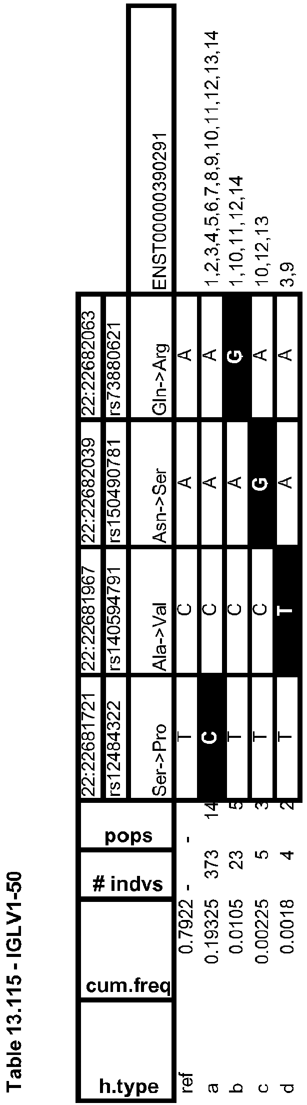

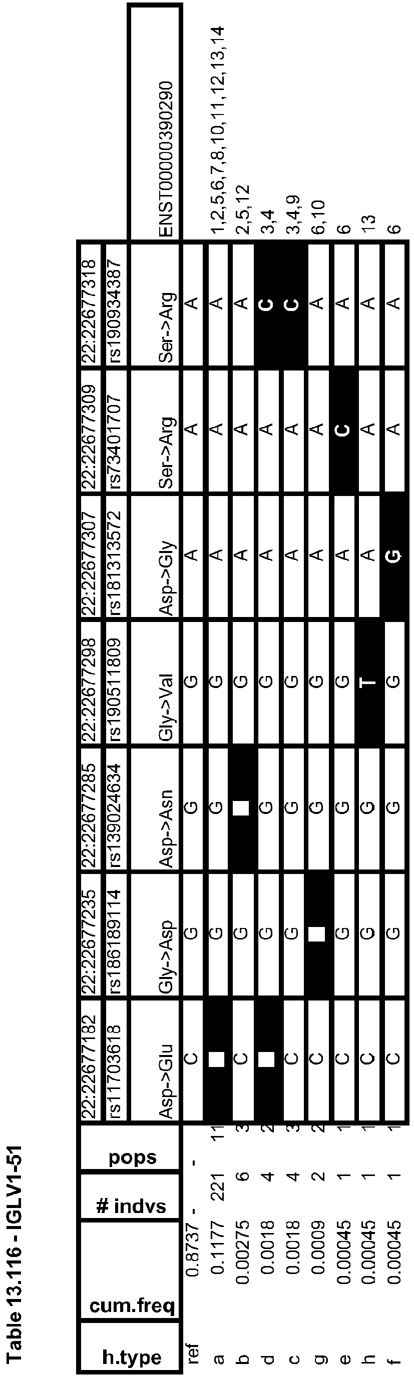

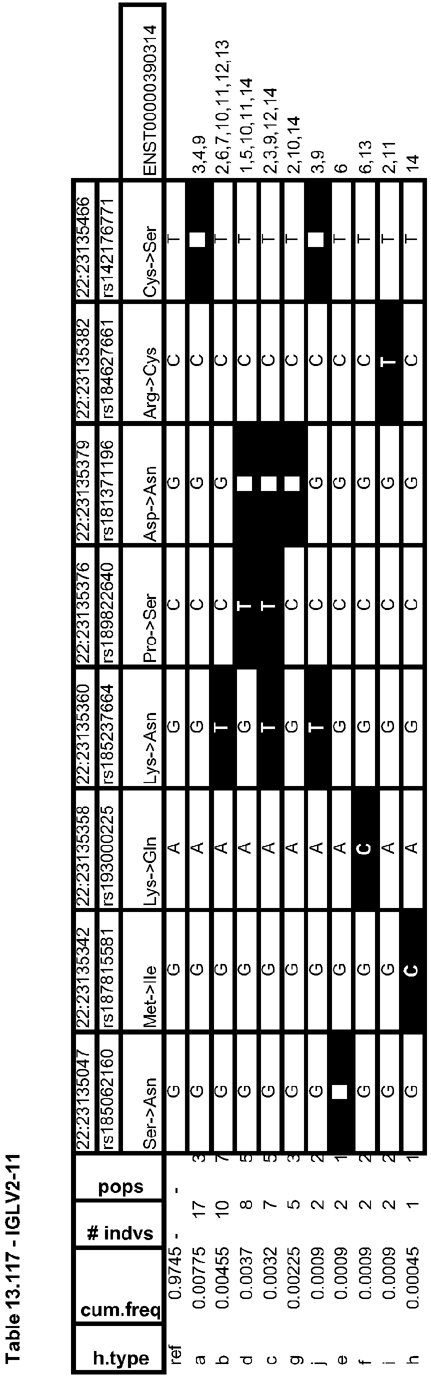

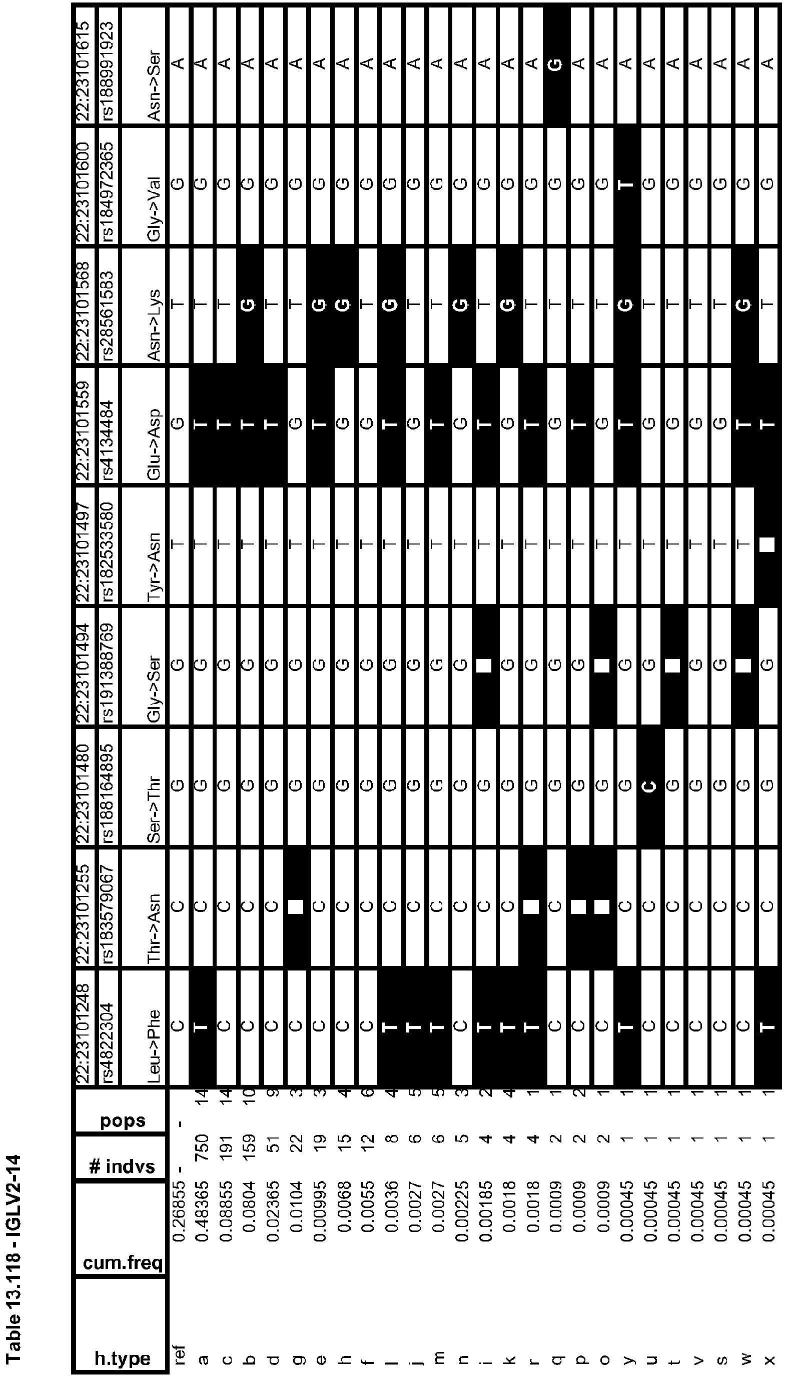

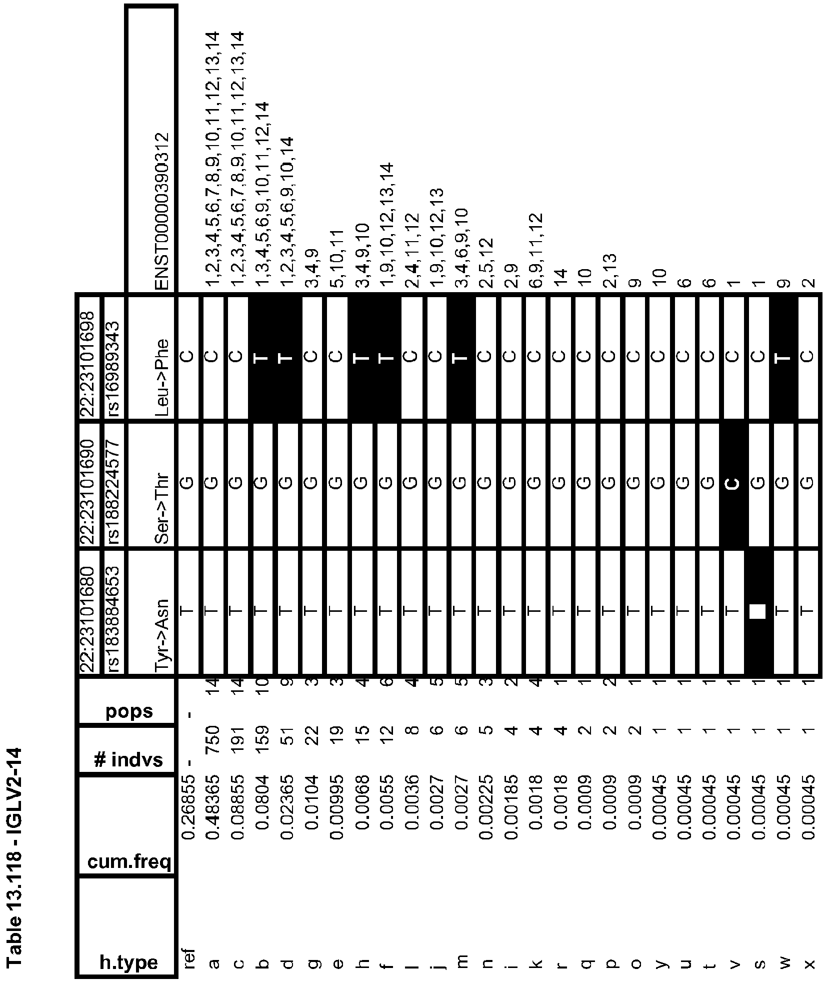

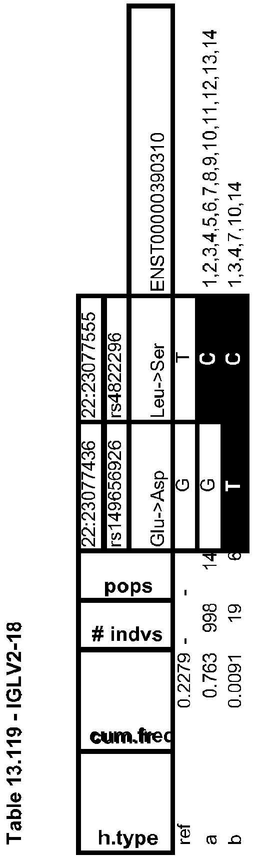

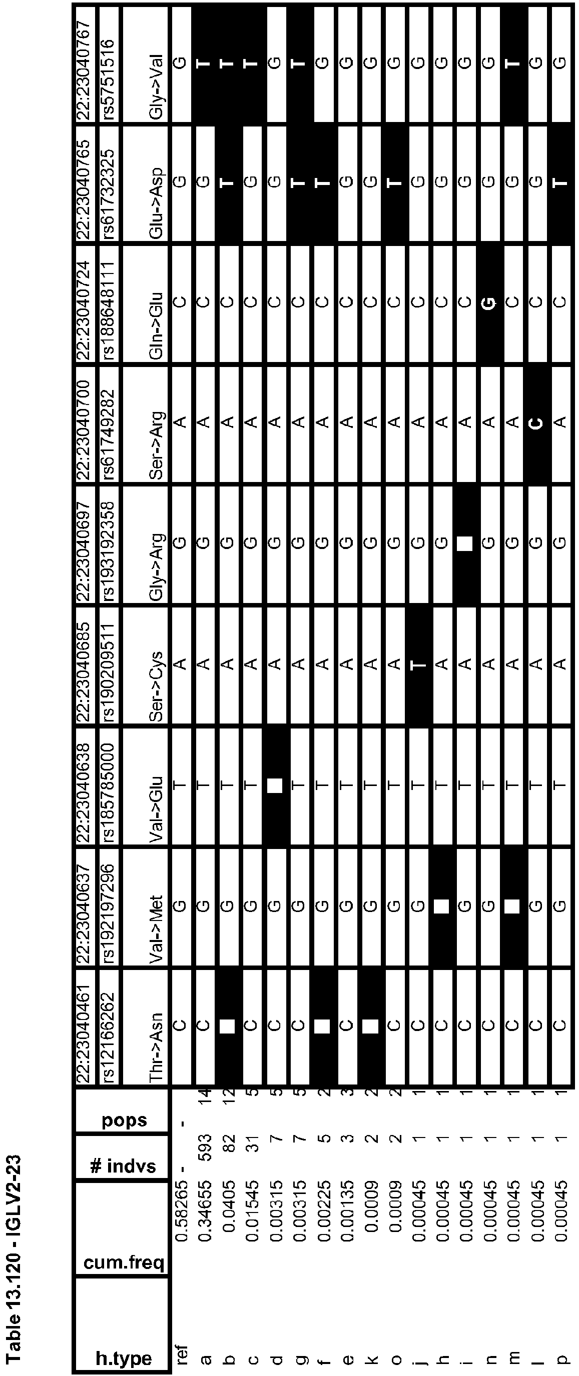

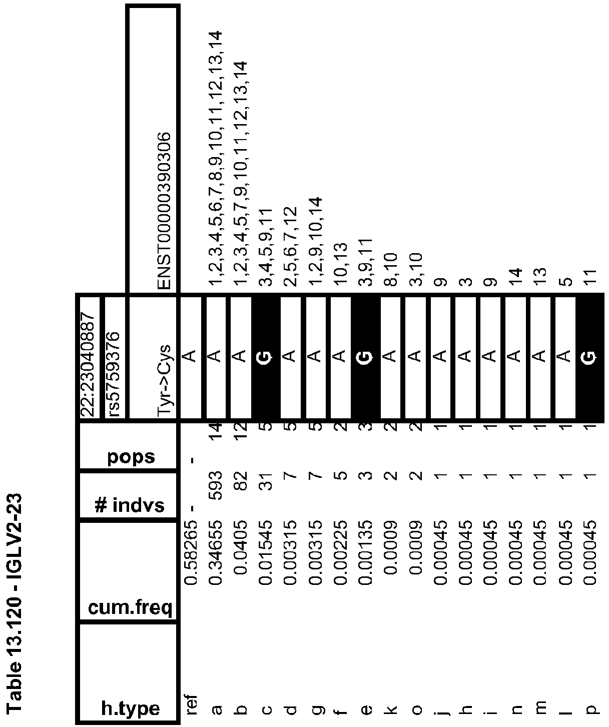

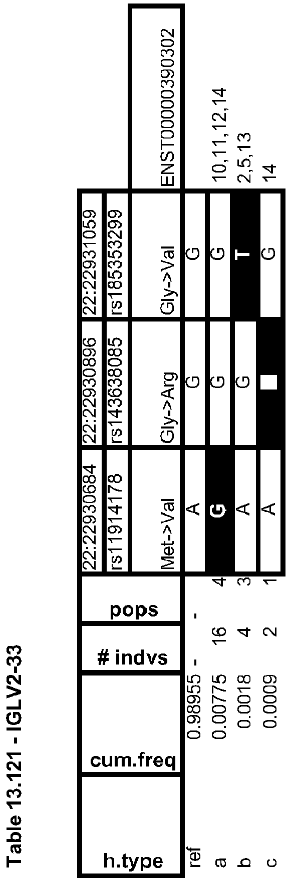

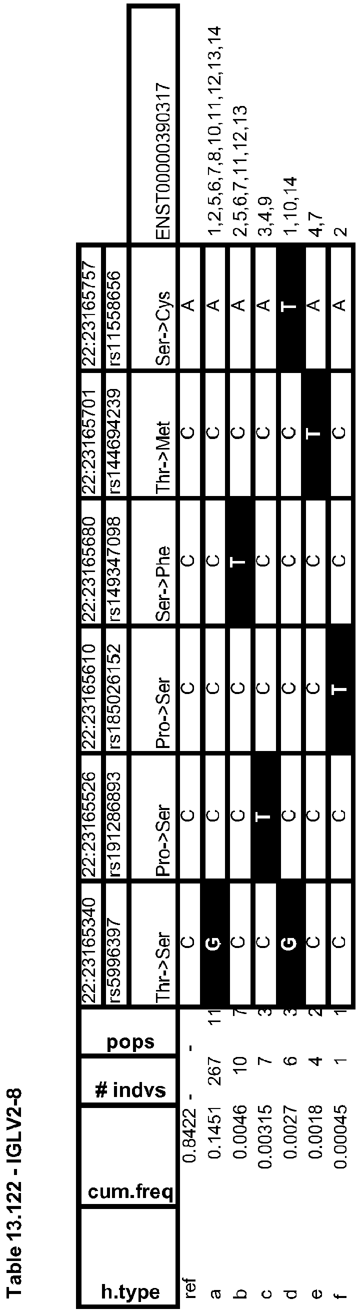

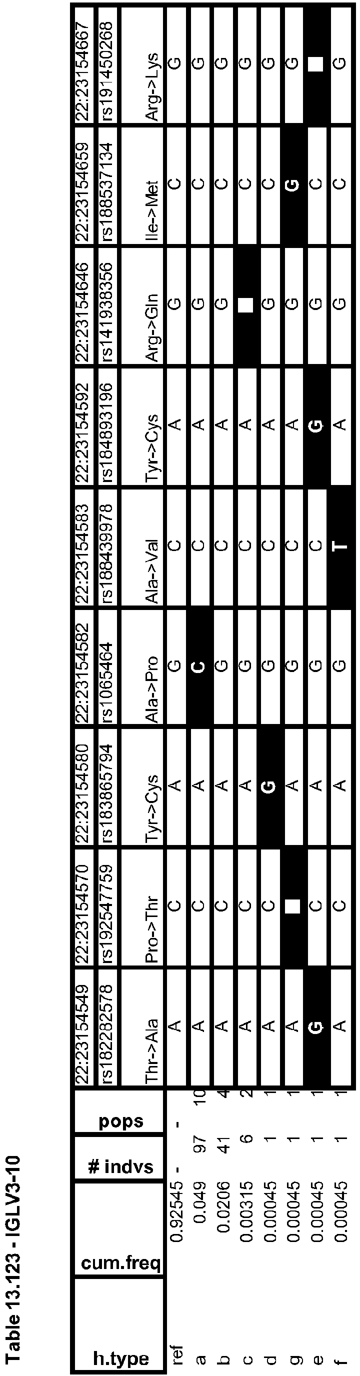

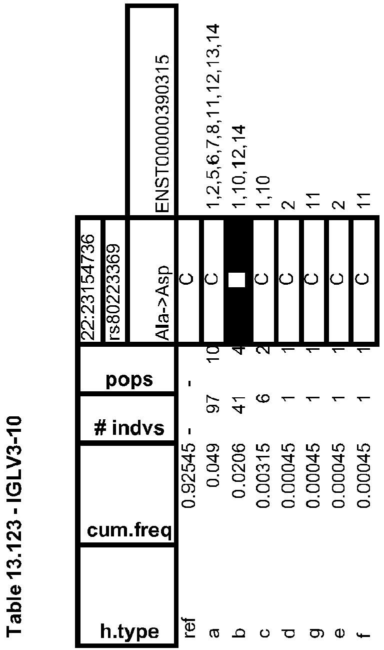

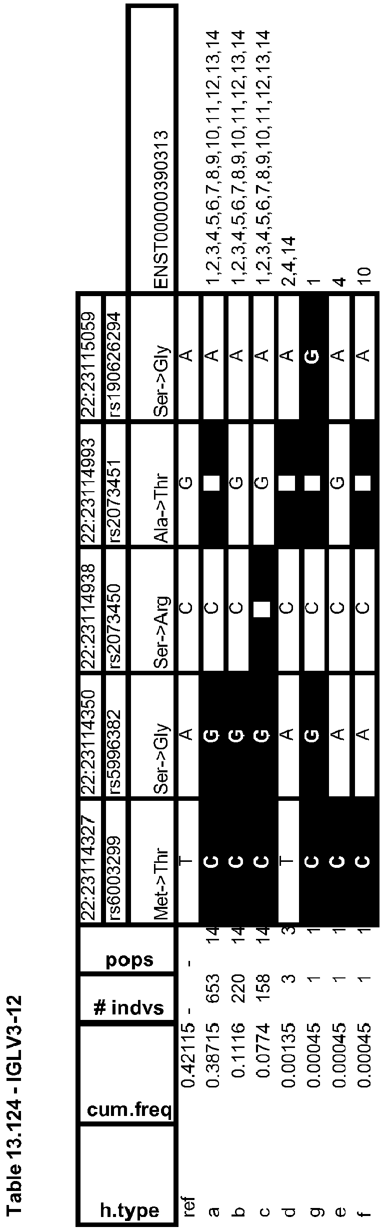

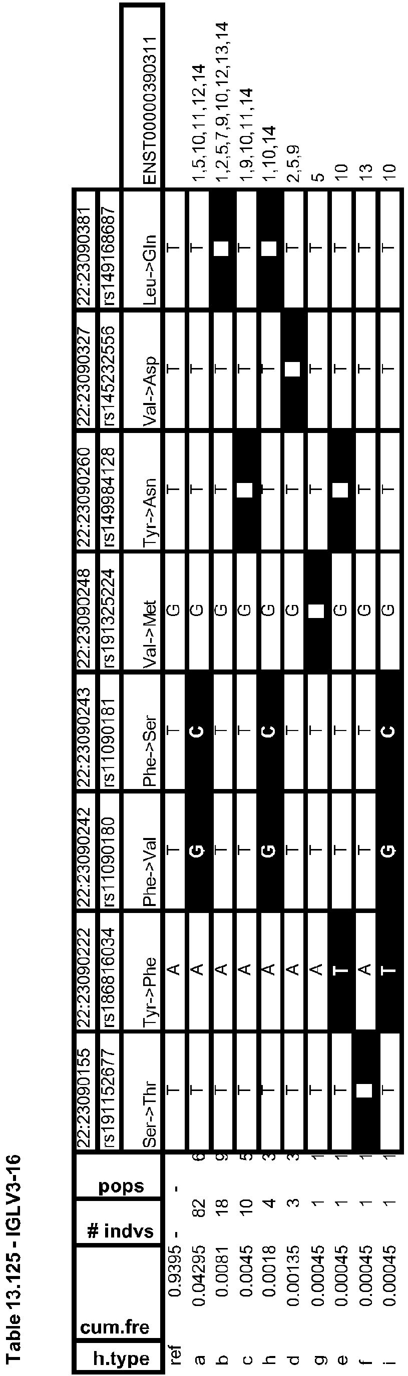

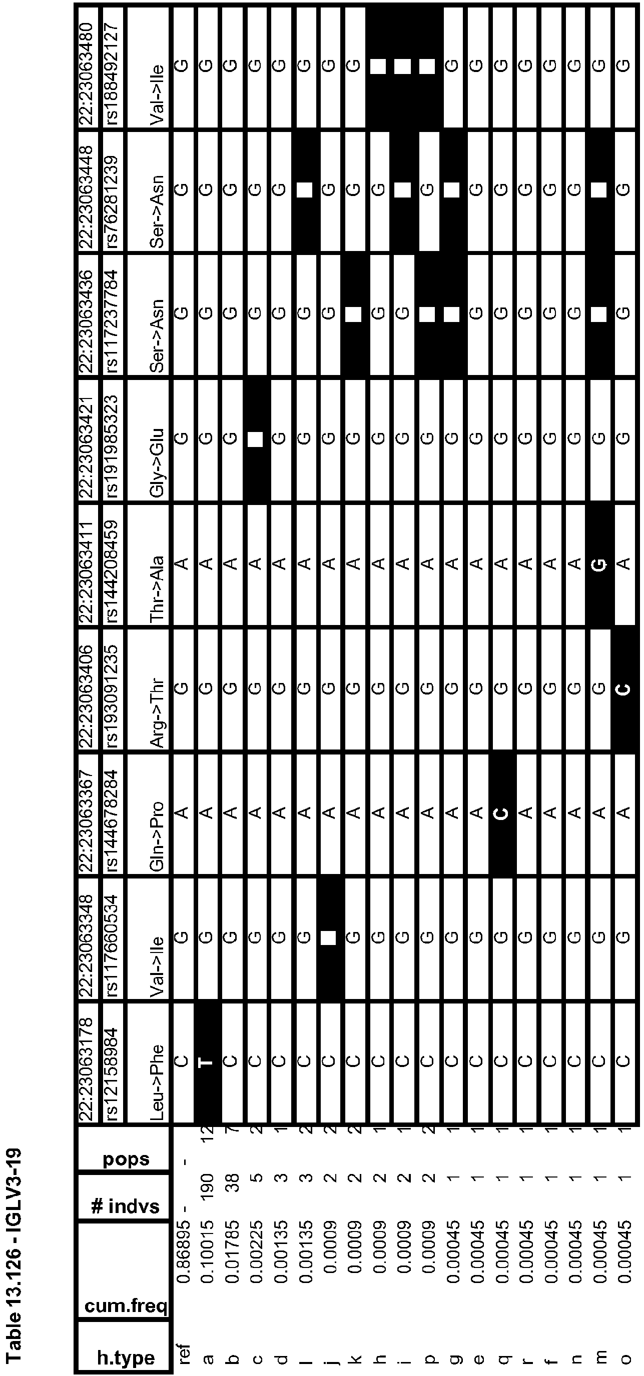

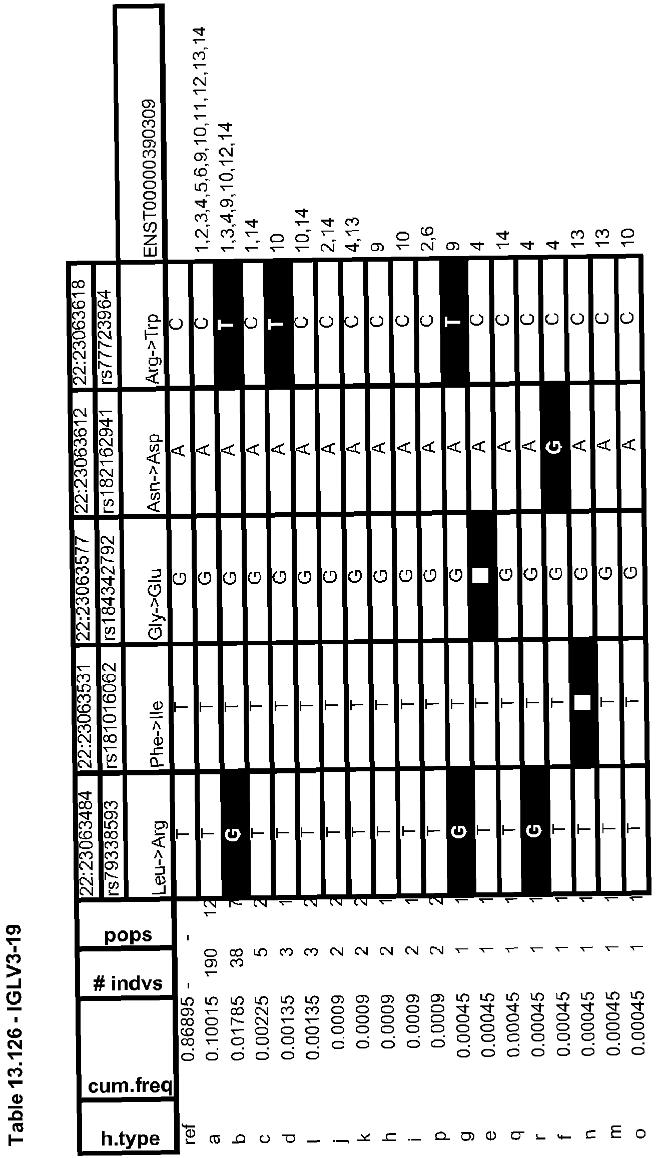

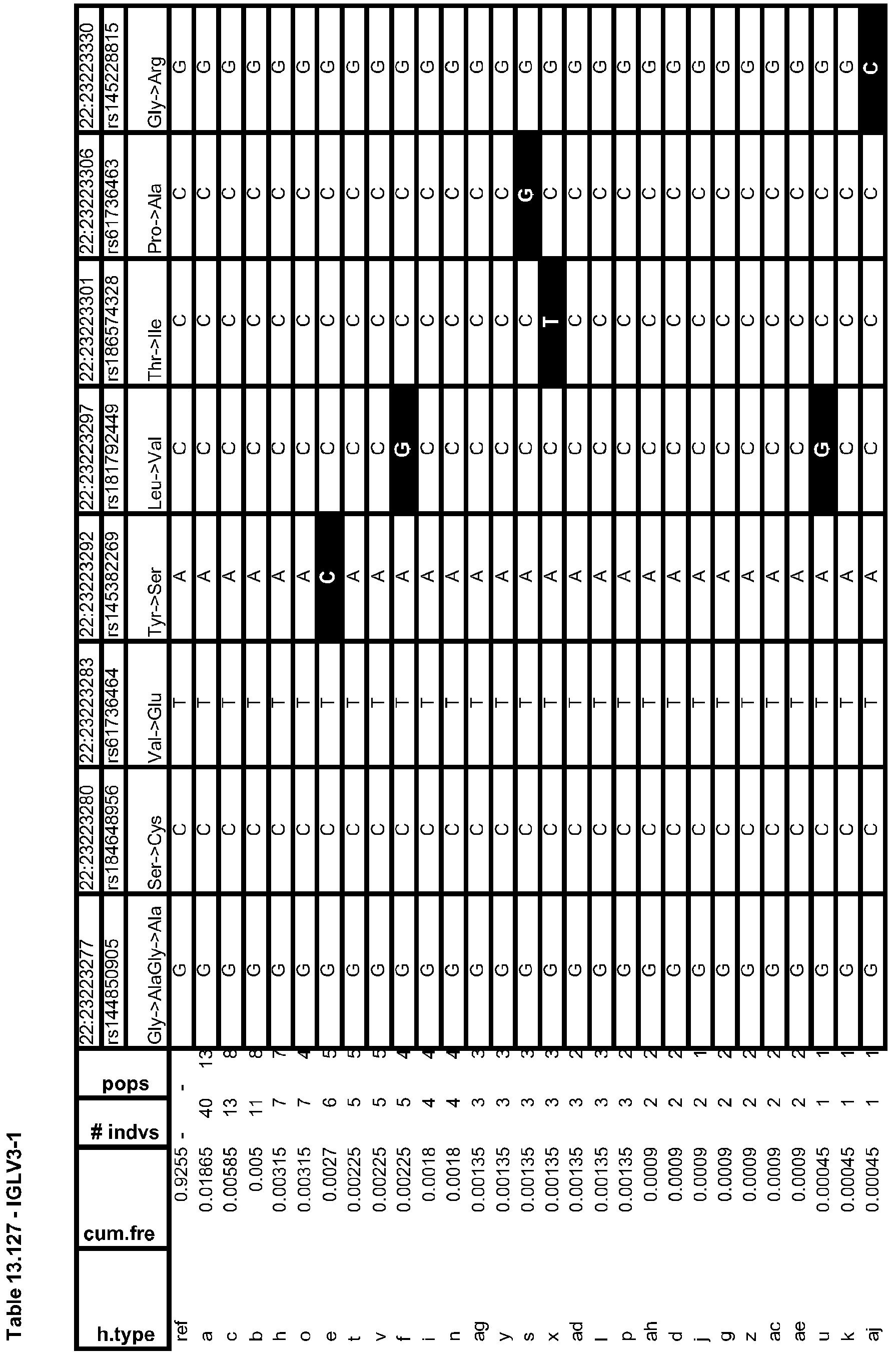

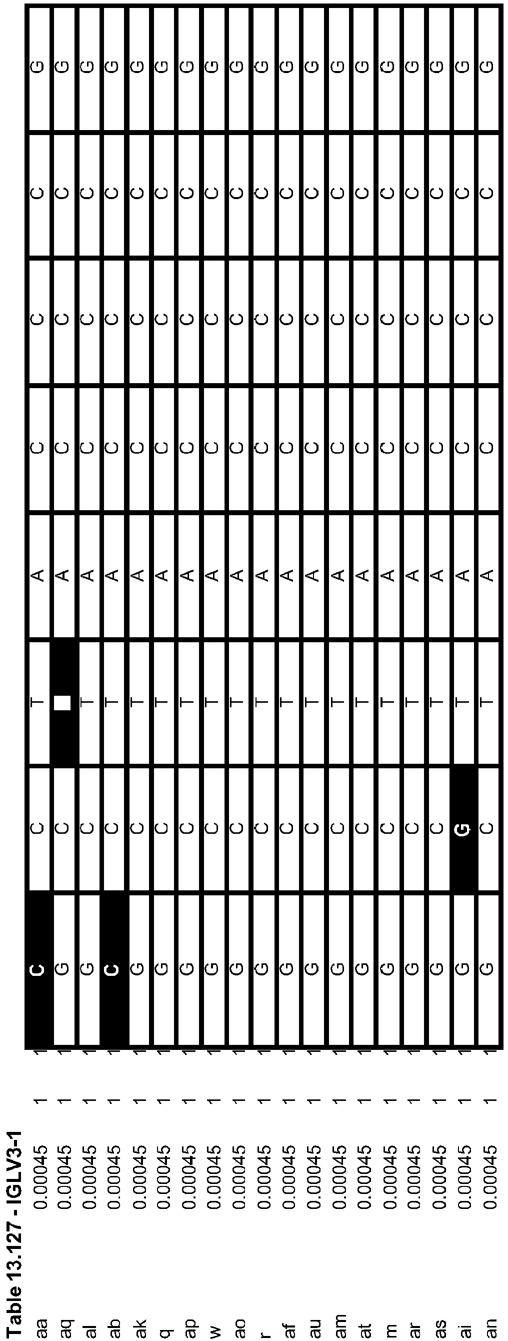

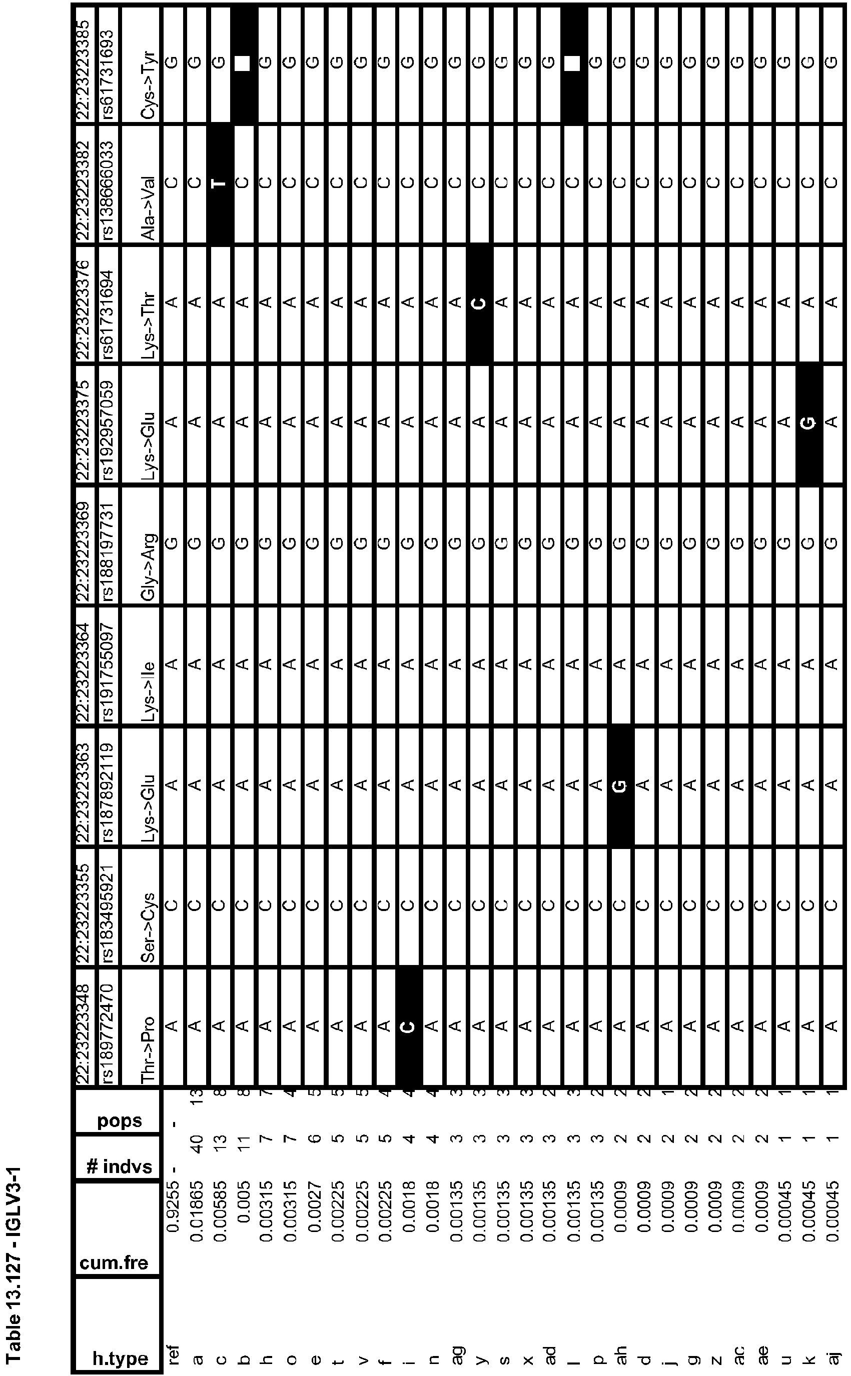

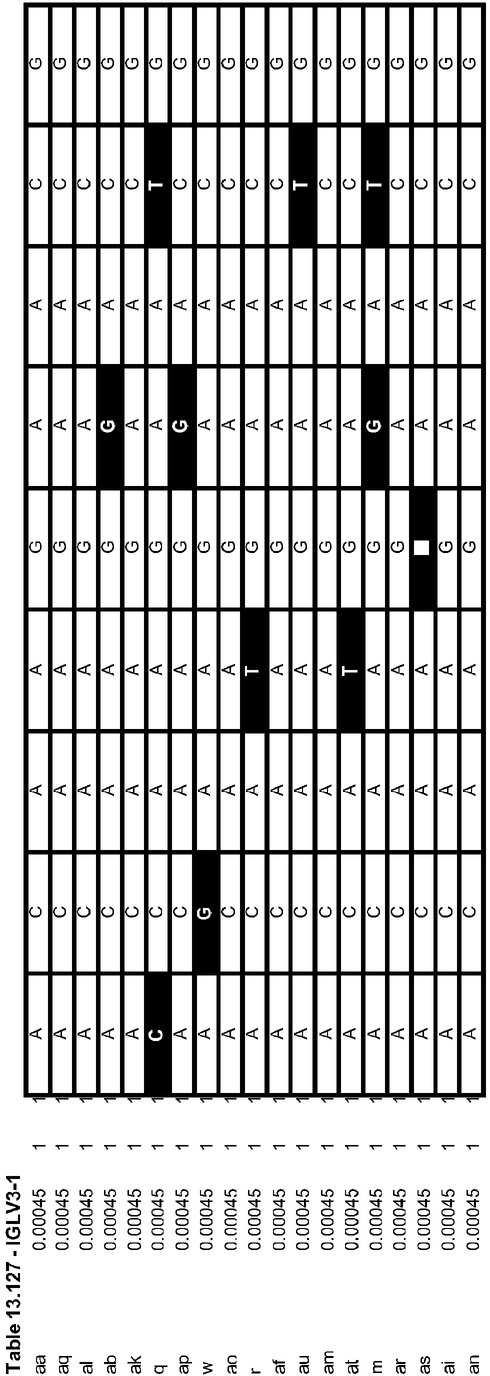

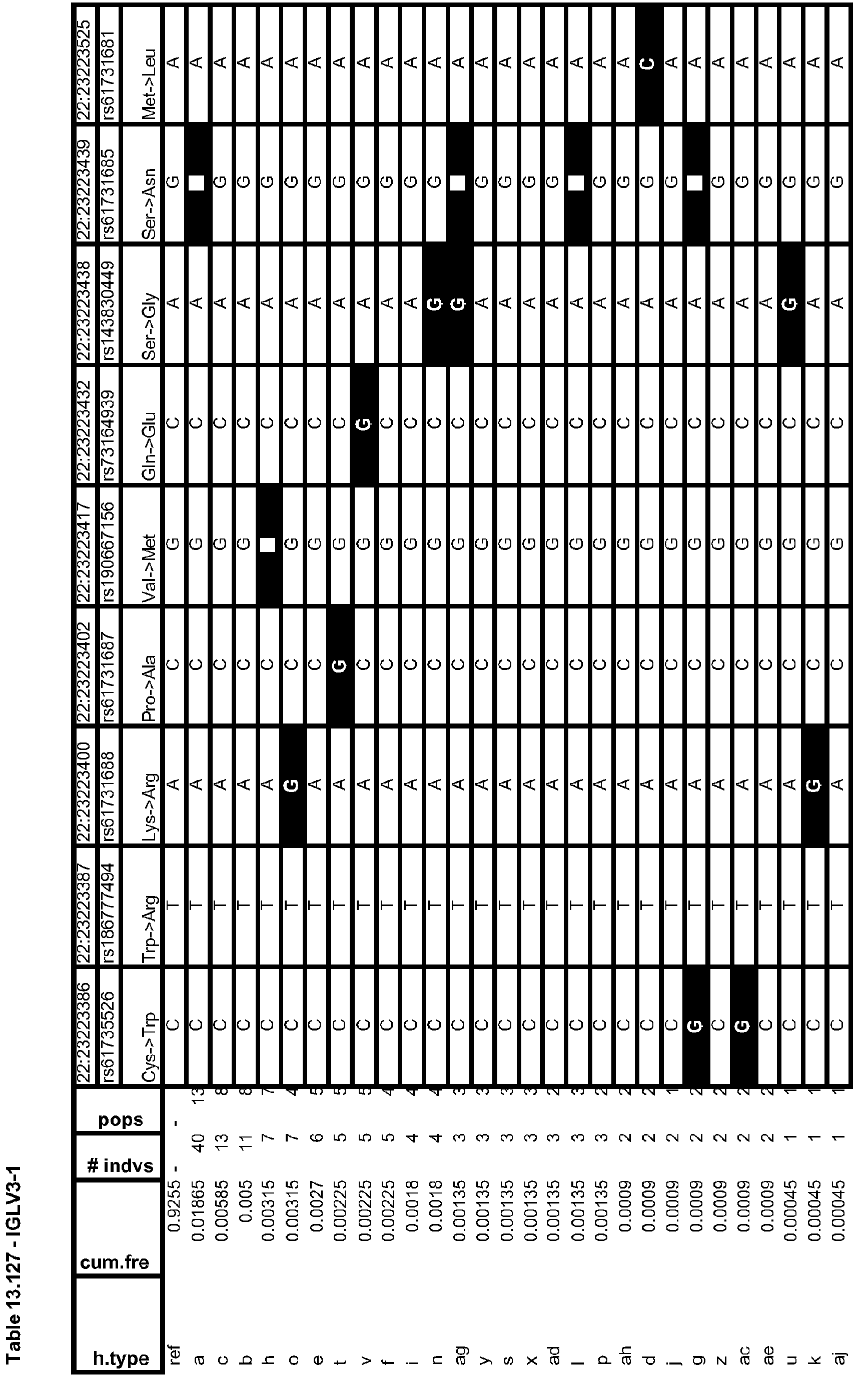

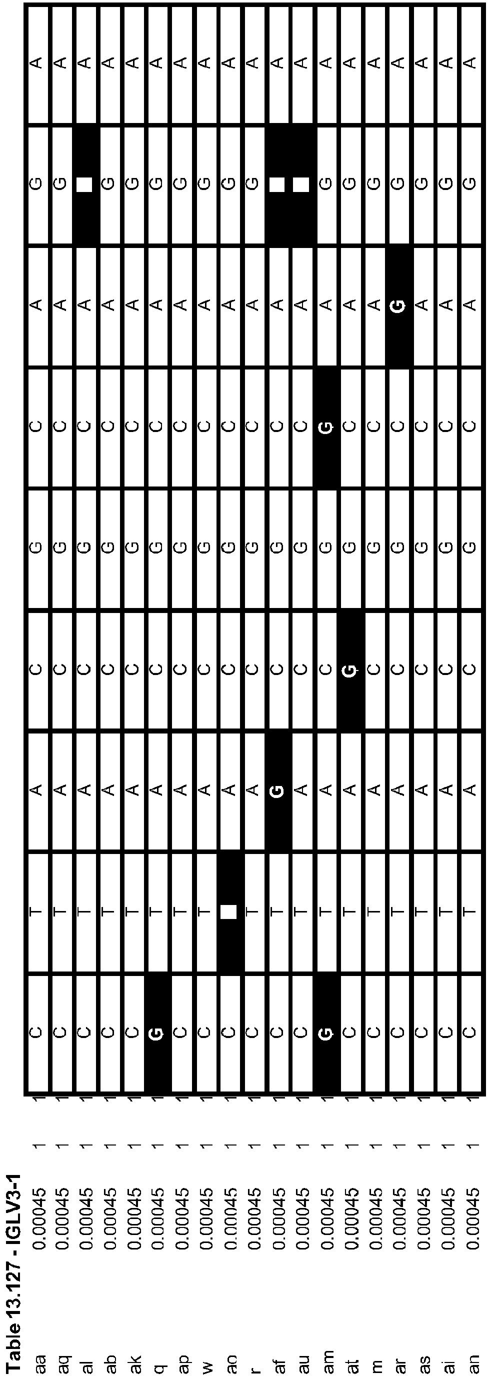

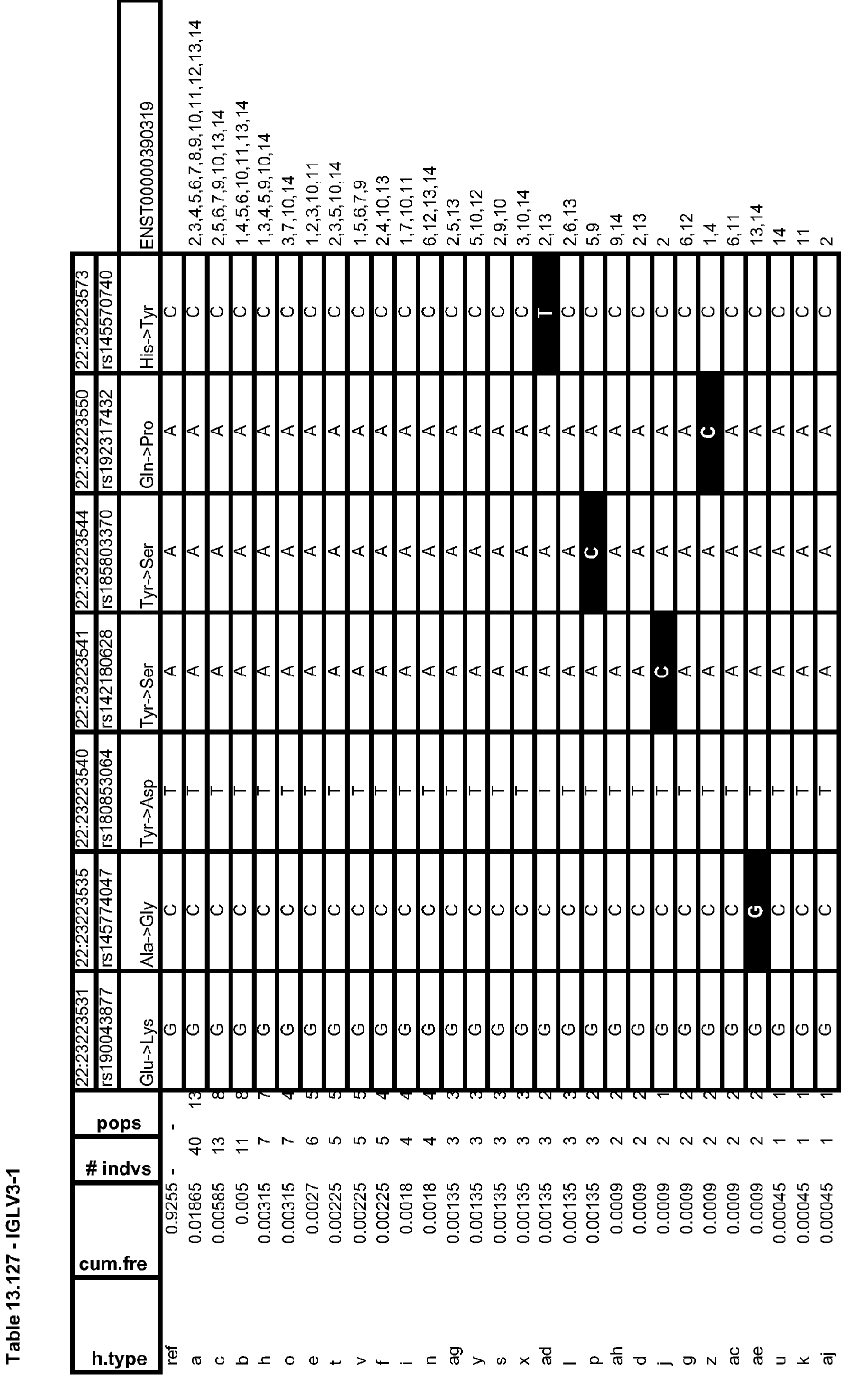

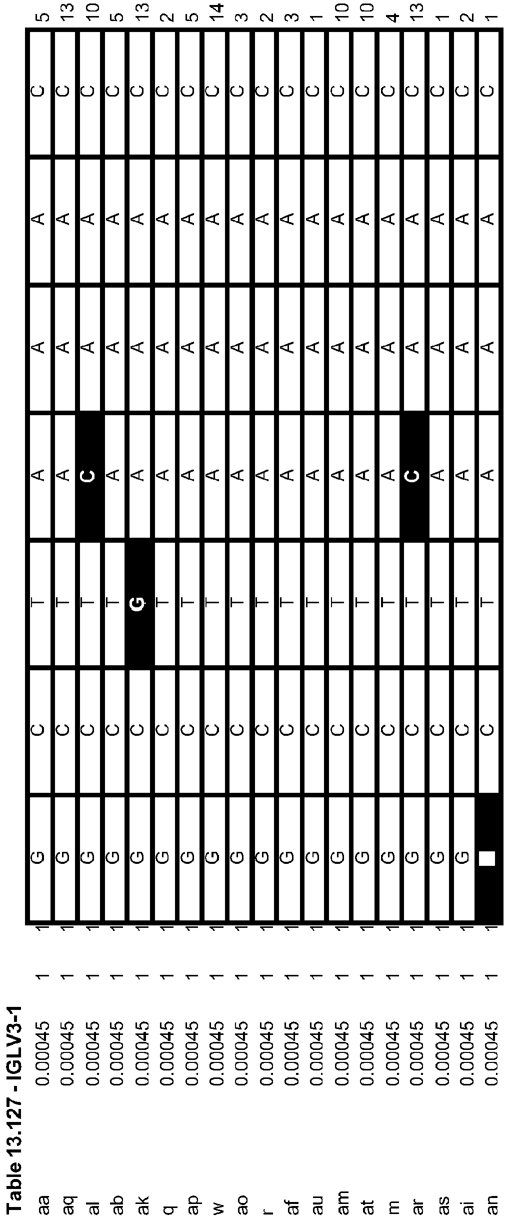

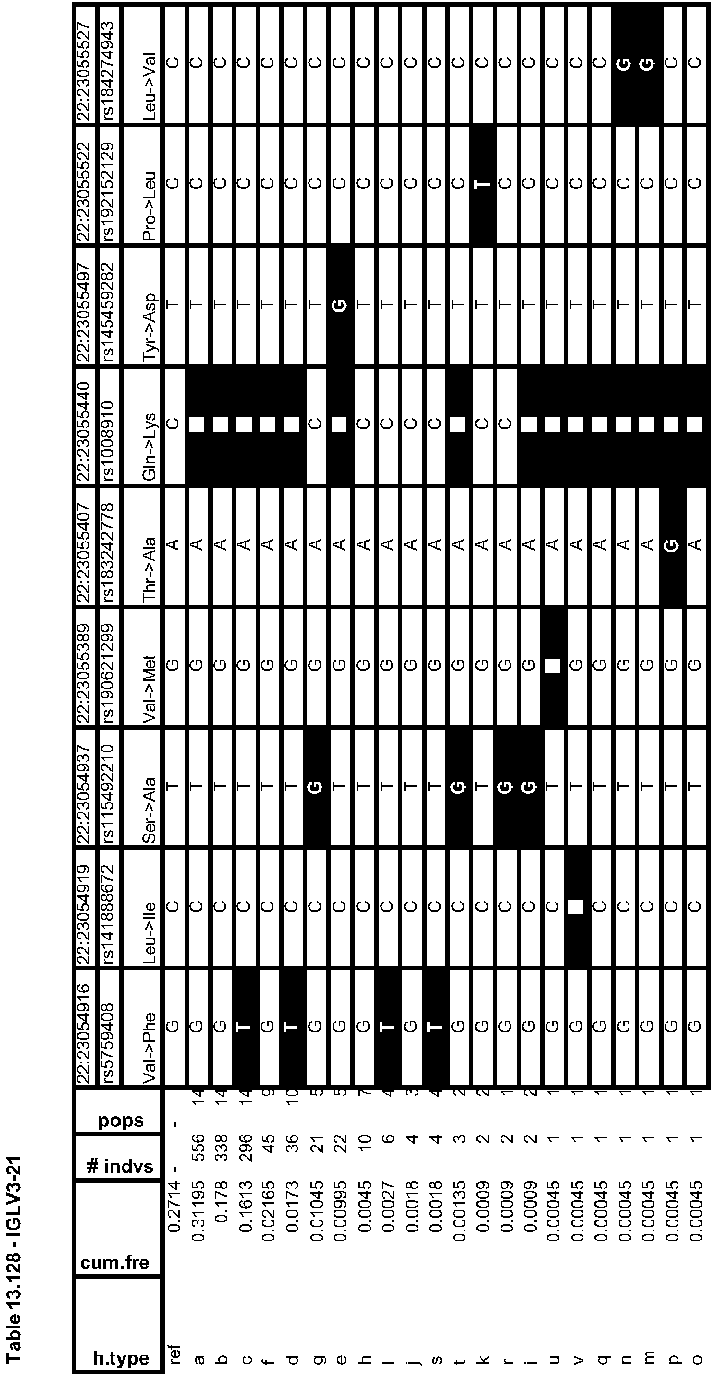

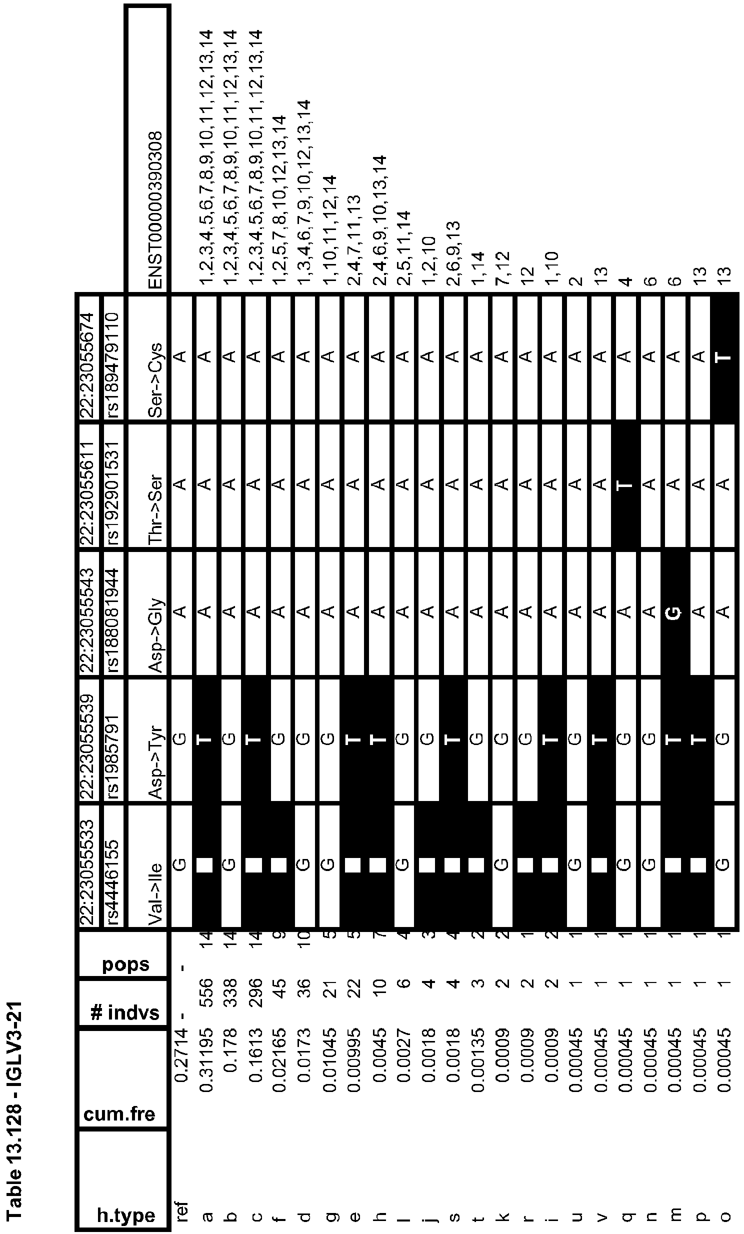

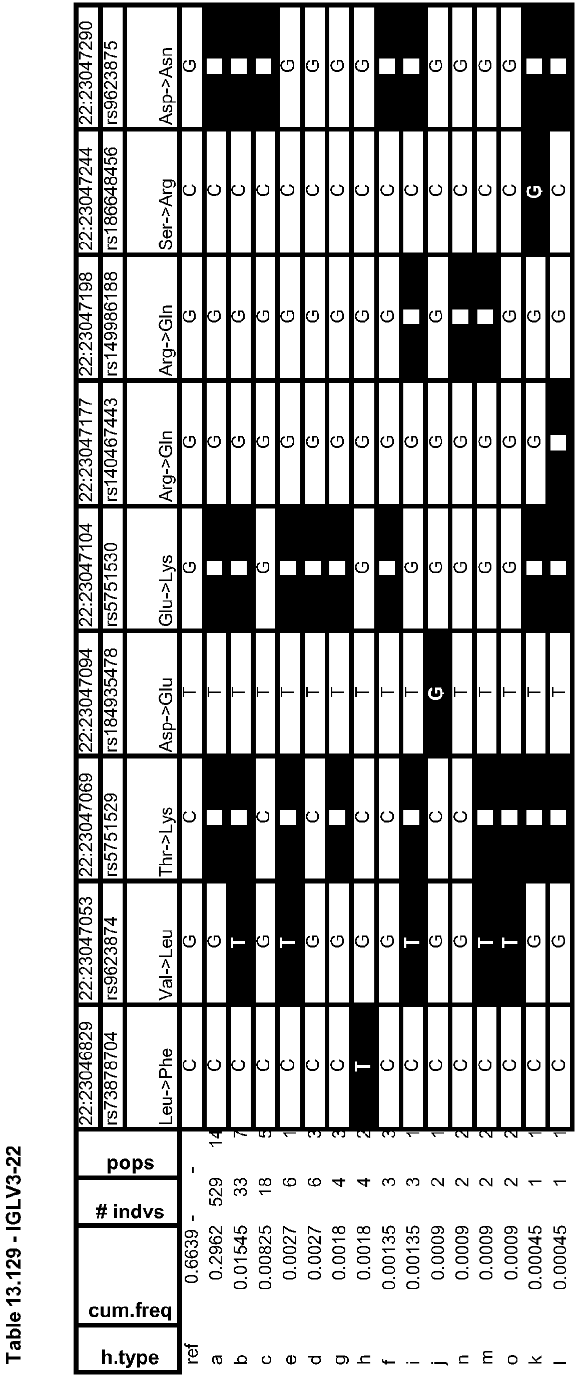

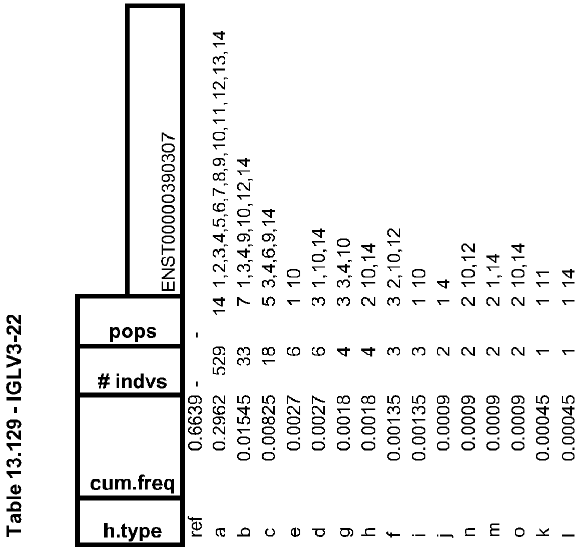

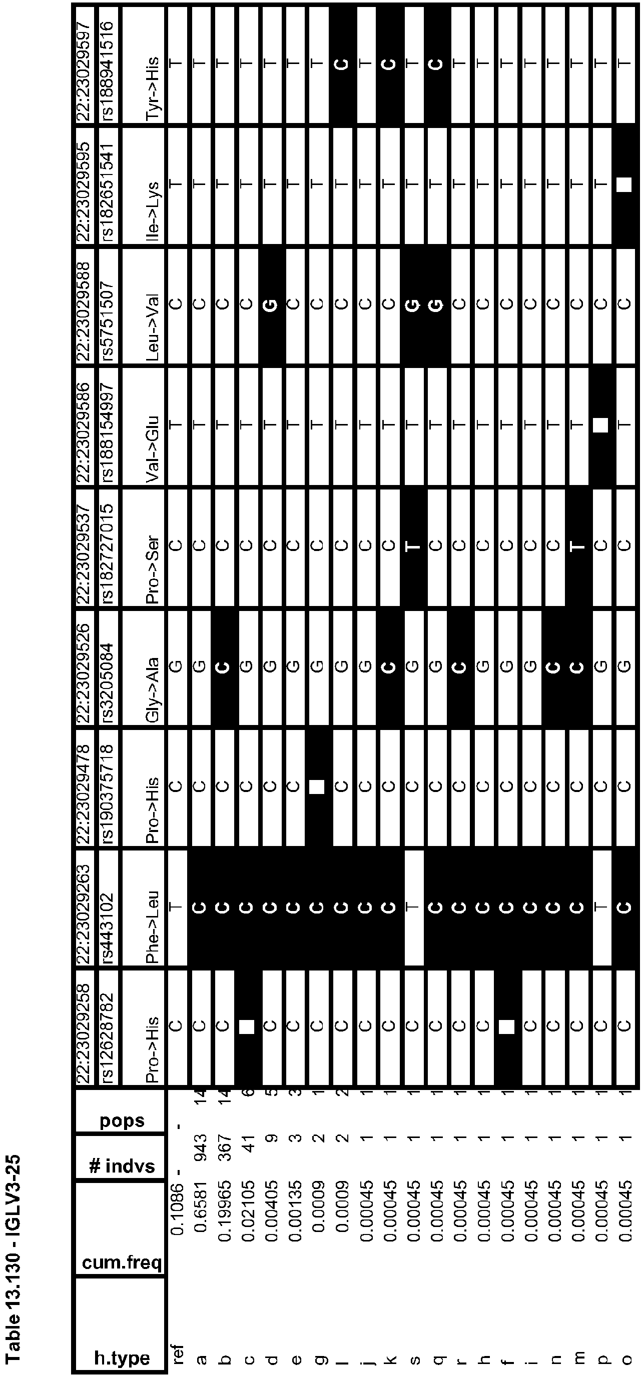

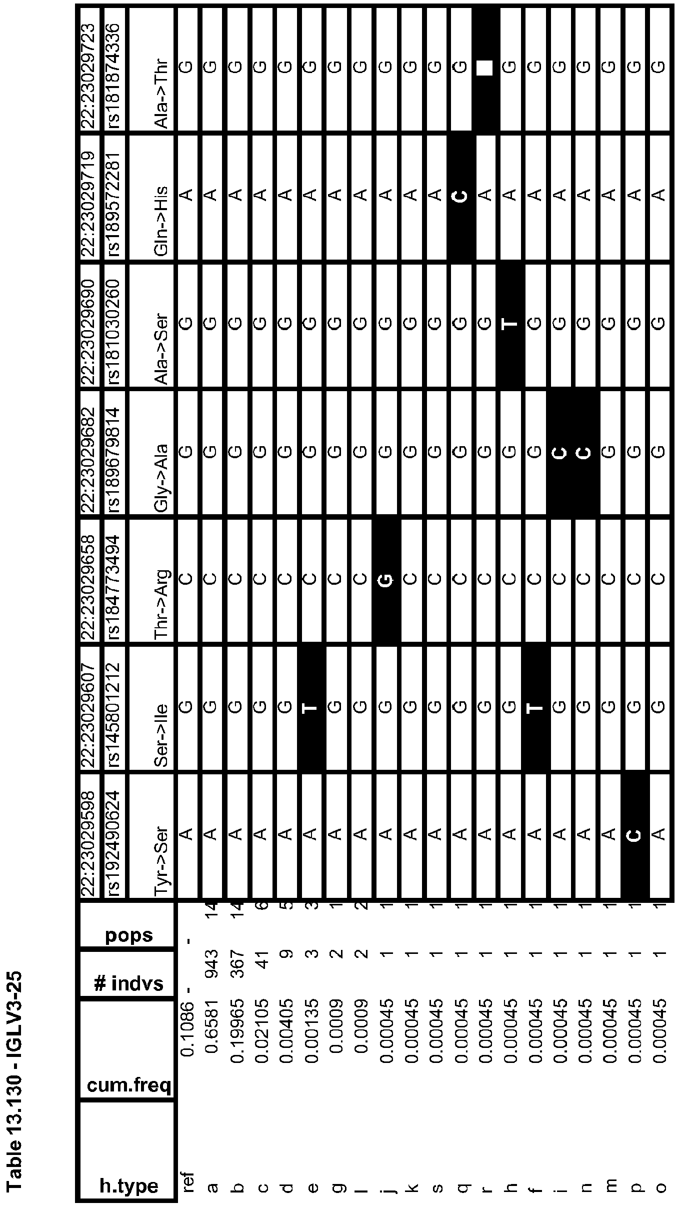

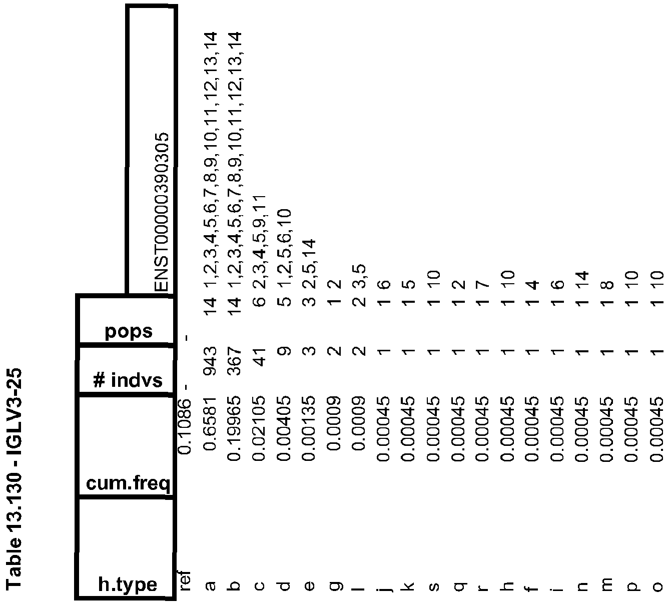

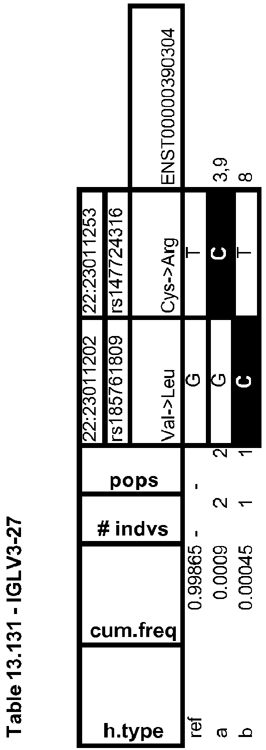

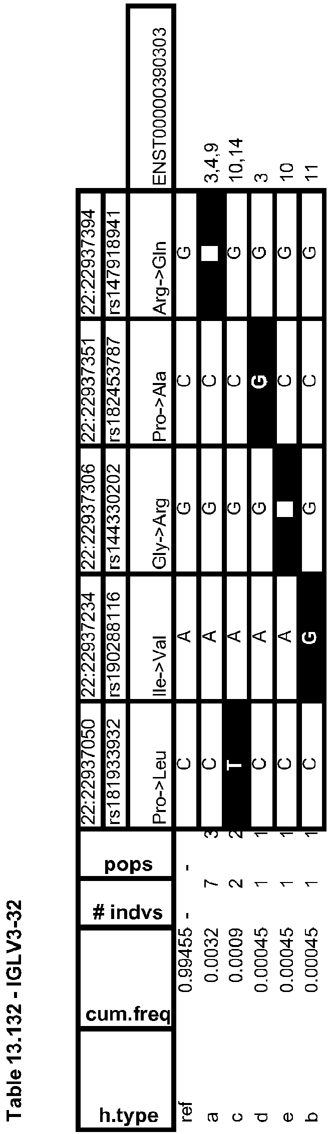

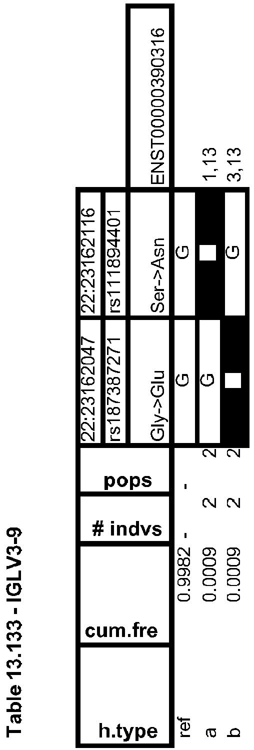

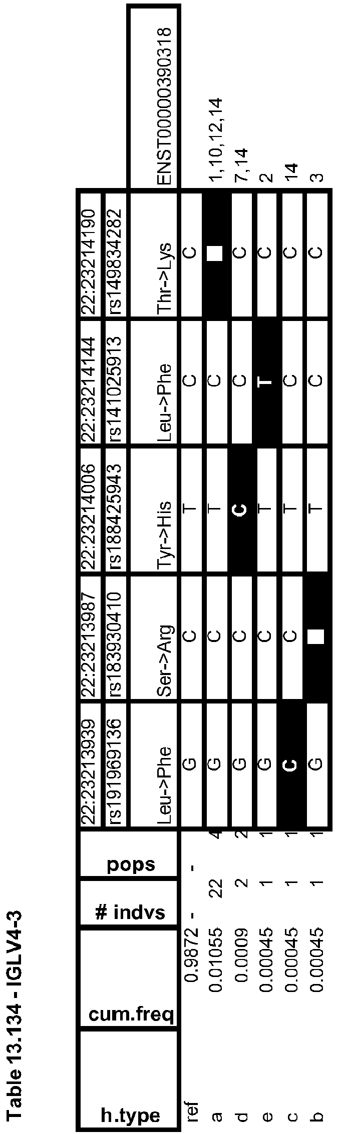

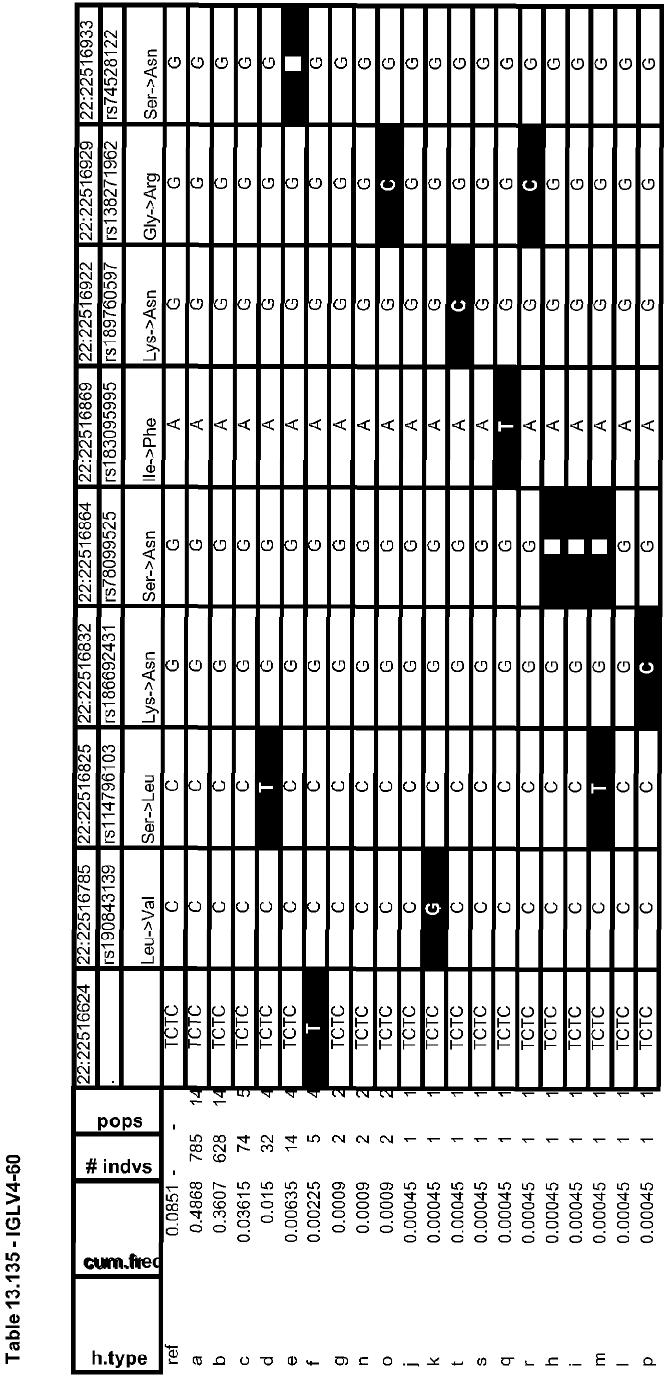

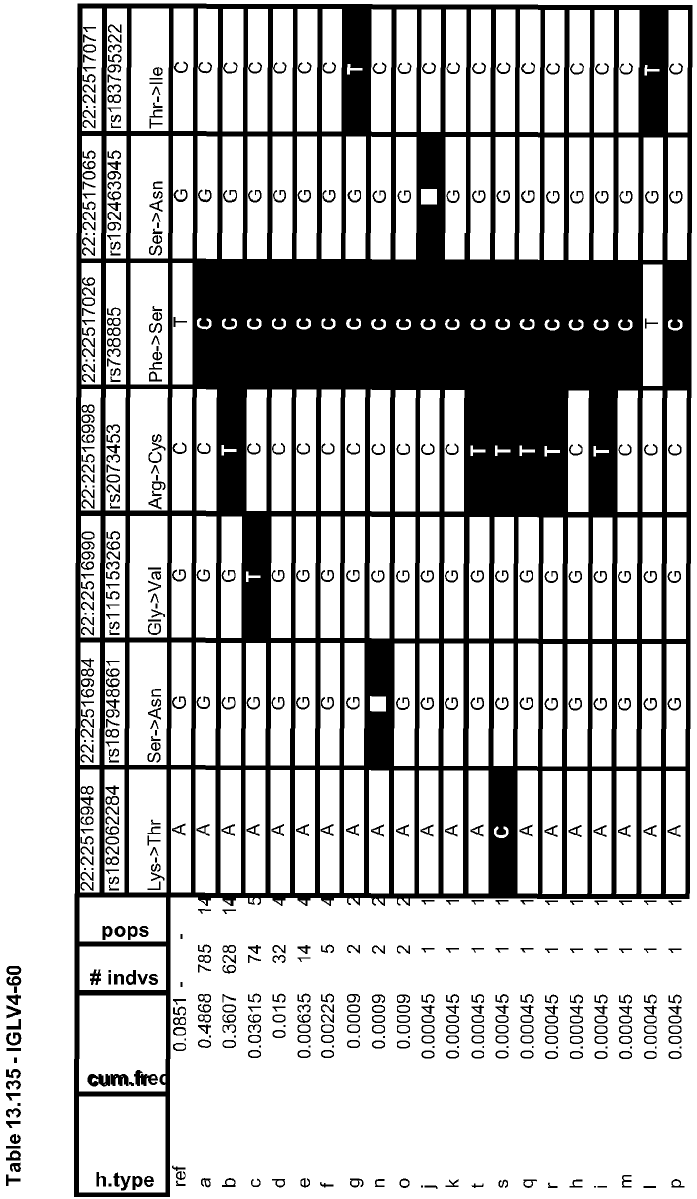

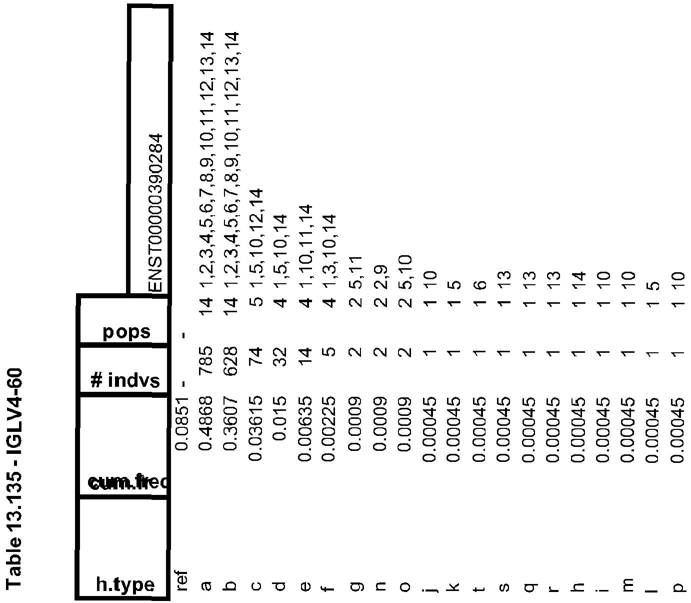

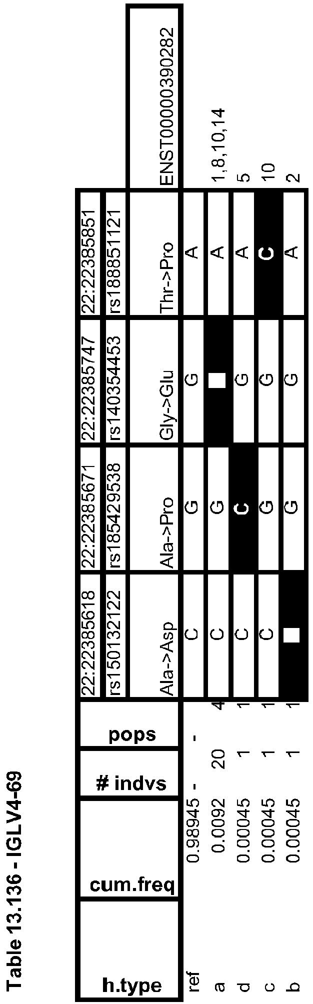

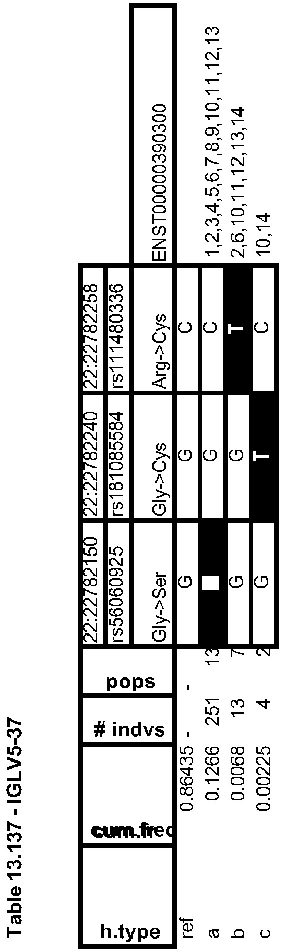

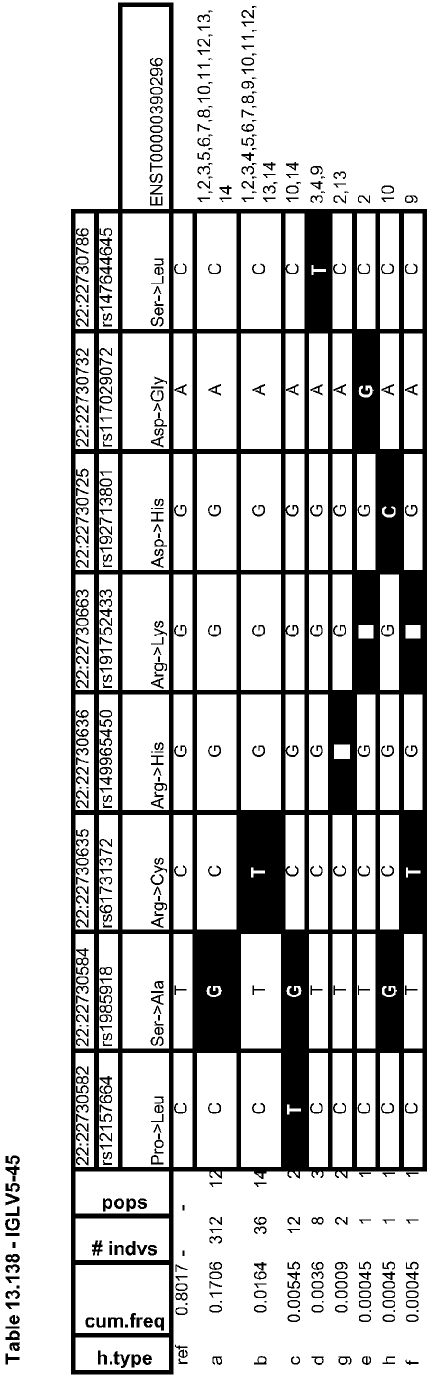

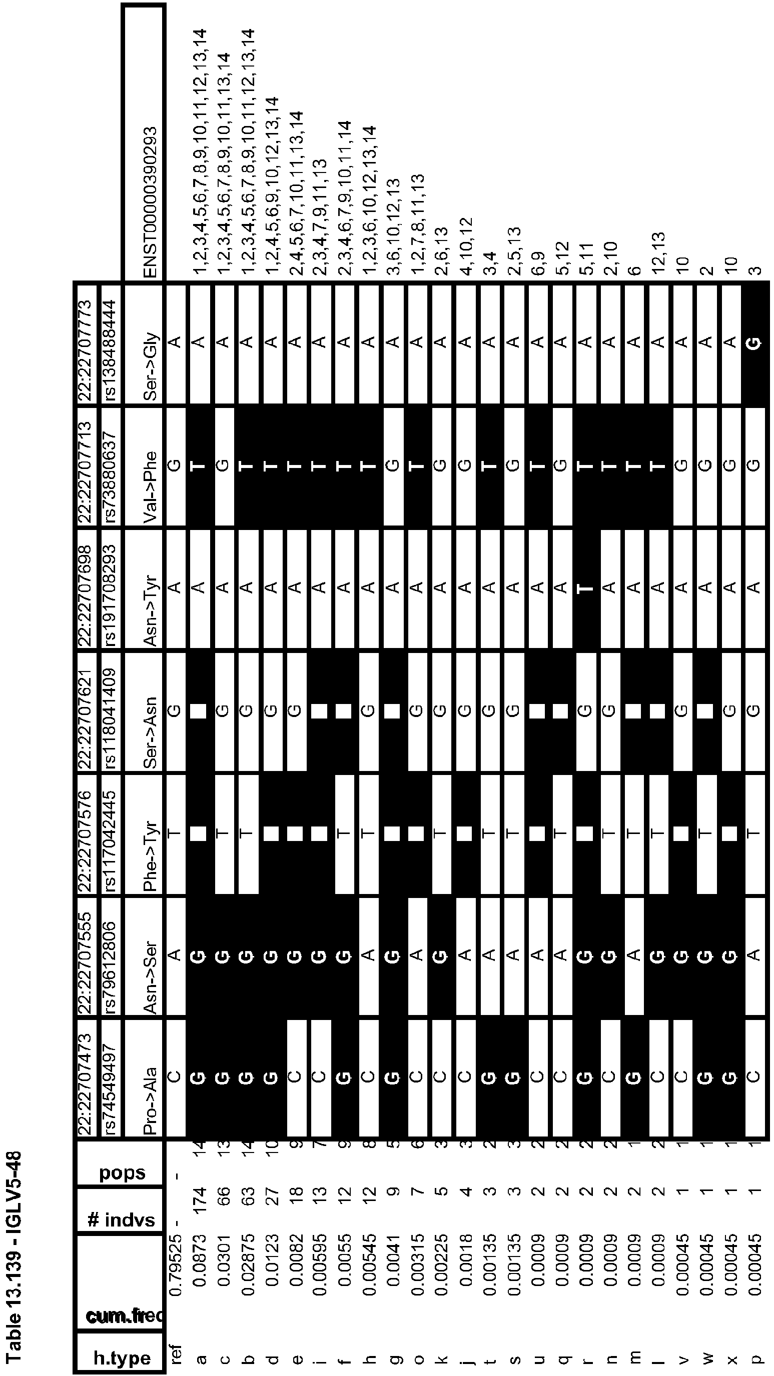

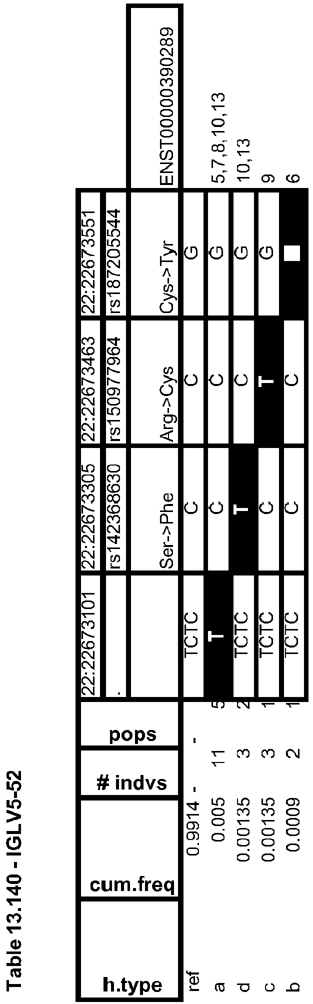

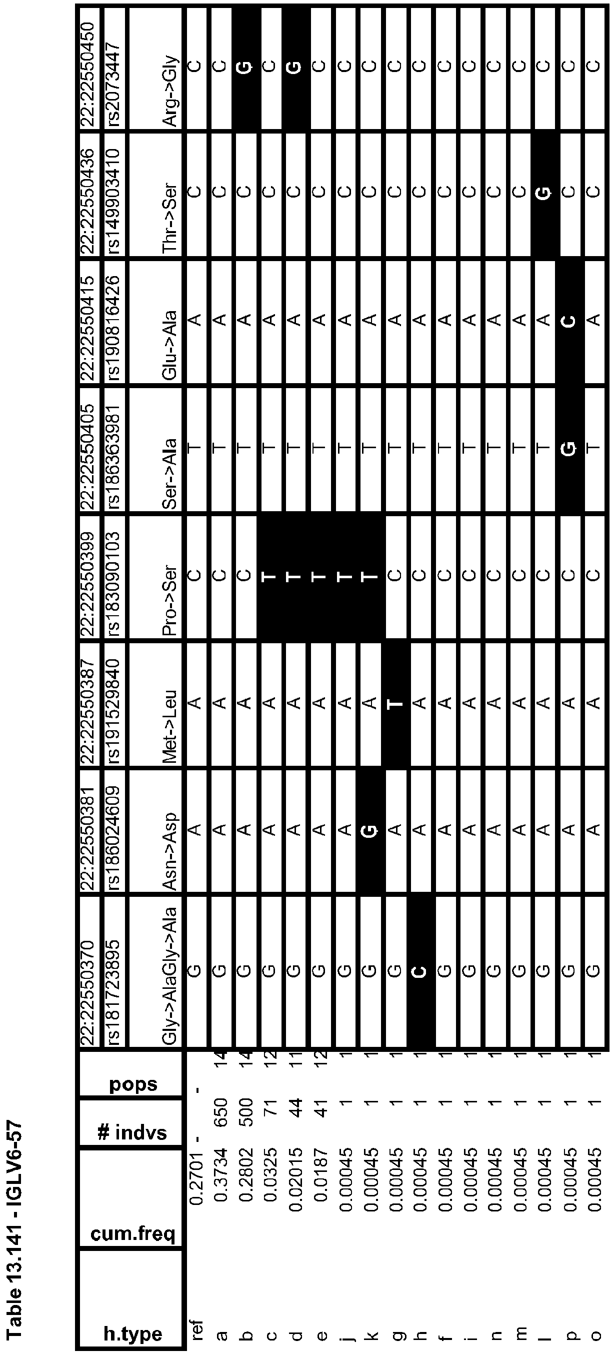

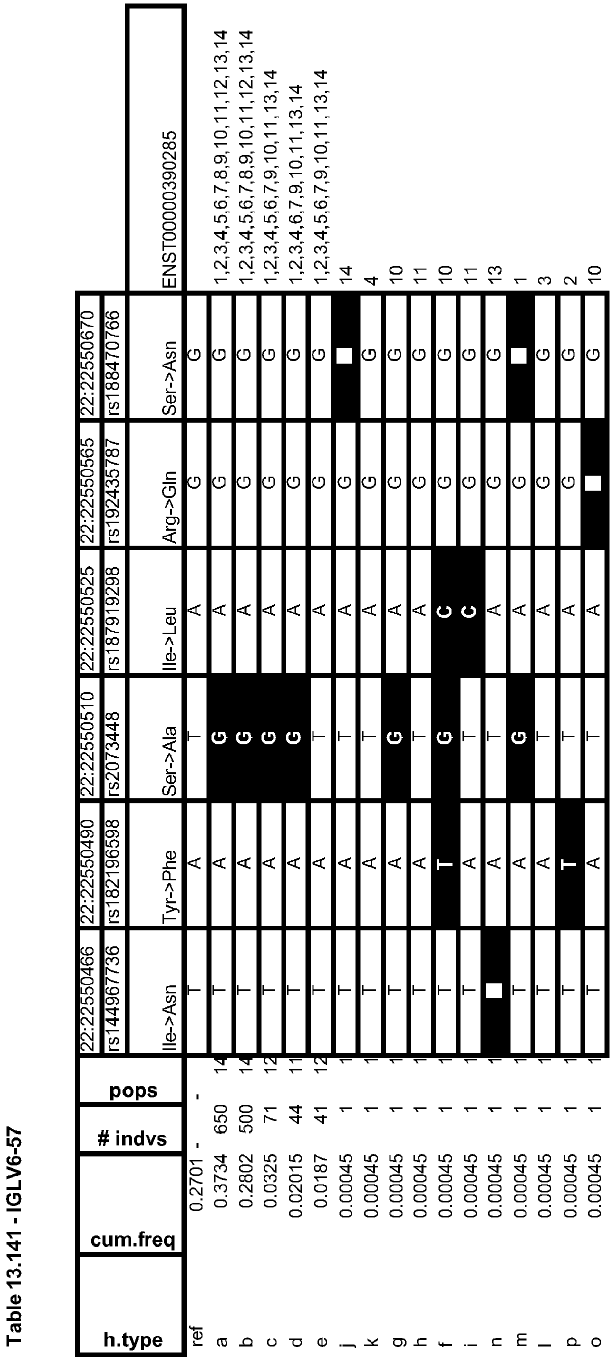

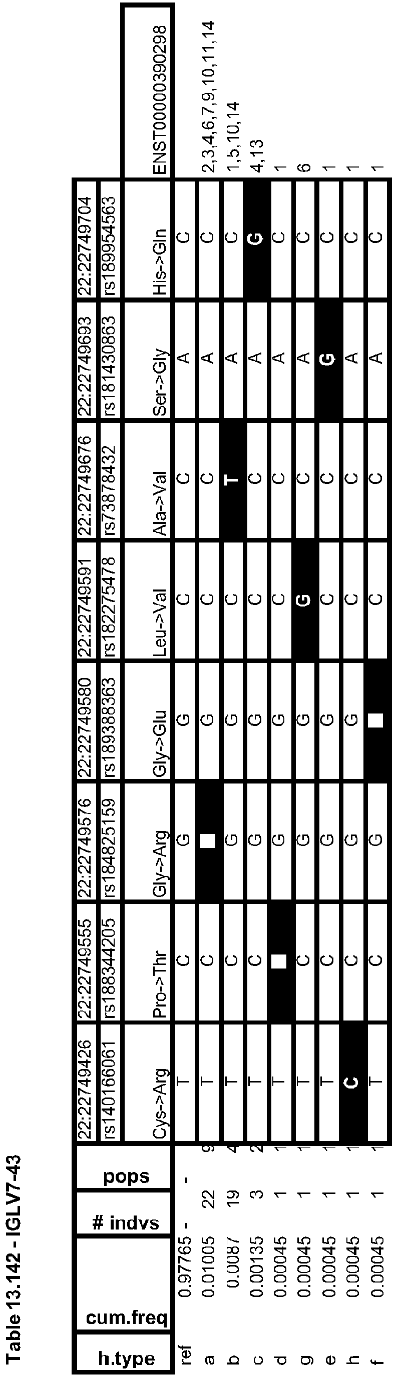

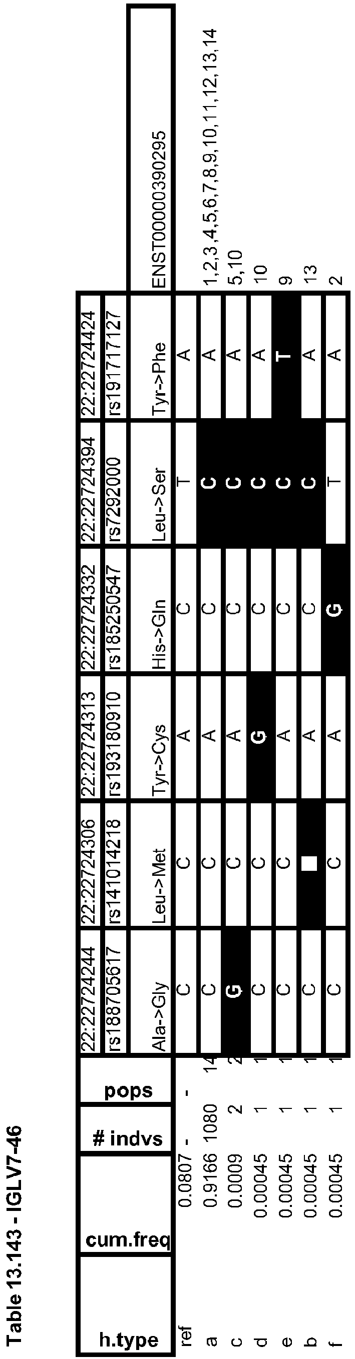

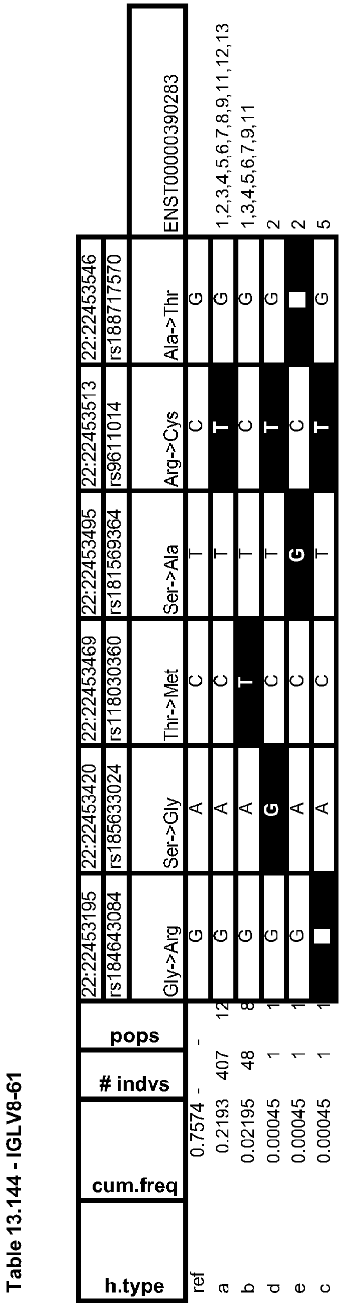

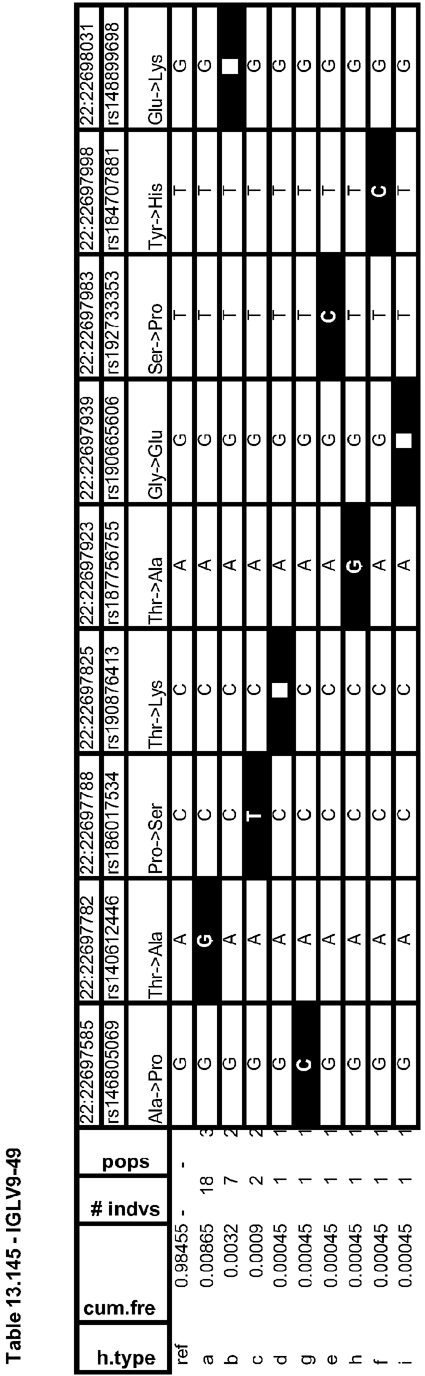

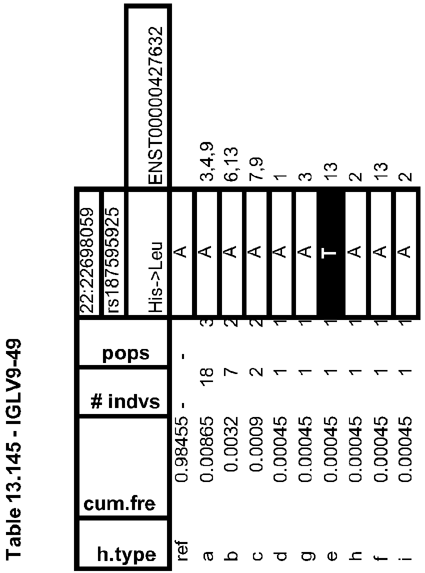

SEQ ID NO: 4 (Nucleotide sequence of JH6 variant)

A T G A T G A T G A T G C C A T A
C C T G A C G A C C G G T C C C T
G G T G C A G T G C G A G G A G T
C

**Table 14A: Variants Having a Cumulative Frequency Greater than 5% or 10%**

| **GENE SEGMENT** | **VARIANT NAME** | **CUMULATIVE FREQUENCY** | **CUMULATIVE FREQUENCY >10%** | **NO UNIQUE POPULATIONS** |
|---|---|---|---|---|
| **Heavy C** | | | | |
| IGHA | IGHA1-a | 0.31385 | Yes | 14 |
| IGHA | IGHA2-a | 0.35655 | Yes | 14 |
| IGHA | IGHA2-b | 0.1647 | Yes | 14 |
| IGHG | IGHG2-a | 0.28675 | Yes | 12 |
| IGHG | IGHG3-a | 0.2813 | Yes | 14 |
| IGHG | IGHG3-b | 0.19955 | Yes | 11 |
| IGHG | IGHG4-a | 0.19825 | Yes | 12 |
| IGHM | IGHM-a | 0.7527 | Yes | 14 |
| IGHM | IGHM-b | 0.15245 | Yes | 14 |

| **Heavy J** | | | | |
|---|---|---|---|---|
| IGHJ | IGHJ6-a | 0.6845 | Yes | 14 |

| **Heavy V** | | | | |
|---|---|---|---|---|
| IGHV | IGHV1-2-a | 0.3268 | Yes | 14 |
| IGHV | IGHV1-2-b | 0.1304 | Yes | 14 |
| IGHV | IGHV1-2-c | 0.0797 | No | 14 |
| IGHV | IGHV1-3-a | 0.3564 | Yes | 14 |
| IGHV | IGHV1-3-b | 0.0724 | No | 13 |
| IGHV | IGHV1-45-a | 0.1286 | Yes | 12 |
| IGHV | IGHV1-45-b | 0.1126 | Yes | 12 |
| IGHV | IGHV1-58-a | 0.4503 | Yes | 14 |
| IGHV | IGHV1-58-b | 0.3144 | Yes | 14 |
| IGHV | IGHV1-69-a | 0.1689 | Yes | 14 |
| IGHV | IGHV1-69-b | 0.1543 | Yes | 14 |
| IGHV | IGHV1-69-c | 0.0561 | No | 13 |
| IGHV | IGHV2-26-a | 0.06135 | No | 13 |
| IGHV | IGHV2-5-a | 0.6657 | Yes | 14 |
| IGHV | IGHV2-70-a | 0.31 | Yes | 14 |
| IGHV | IGHV2-70-b | 0.16595 | Yes | 14 |
| IGHV | IGHV2-70-d | 0.06035 | No | 13 |
| IGHV | IGHV3-13-a | 0.15245 | Yes | 13 |
| IGHV | IGHV3-15-a | 0.0572 | No | 11 |
| IGHV | IGHV3-16-a | 0.11075 | Yes | 13 |
| IGHV | IGHV3-20-a | 0.2829 | Yes | 14 |
| IGHV | IGHV3-20-b | 0.1209 | Yes | 13 |
| IGHV | IGHV3-23-a | 0.09825 | No | 12 |
| IGHV | IGHV3-33-a | 0.145 | Yes | 12 |
| IGHV | IGHV3-35-a | 0.11765 | Yes | 14 |
| IGHV | IGHV3-35-b | 0.09695 | No | 10 |
| IGHV | IGHV3-38-a | 0.0723 | No | 14 |
| IGHV | IGHV3-43-a | 0.2428 | Yes | 14 |
| IGHV | IGHV3-43-b | 0.08365 | No | 13 |
| IGHV | IGHV3-48-a | 0.3355 | Yes | 14 |
| IGHV | IGHV3-48-b | 0.20455 | Yes | 14 |
| IGHV | IGHV3-49-a | 0.38705 | Yes | 14 |
| IGHV | IGHV3-49-b | 0.28555 | Yes | 13 |
| IGHV | IGHV3-53-a | 0.2535 | Yes | 14 |
| IGHV | IGHV3-53-b | 0.2264 | Yes | 13 |
| IGHV | IGHV3-64-a | 0.47965 | Yes | 14 |
| IGHV | IGHV3-64-b | 0.06225 | No | 13 |
| IGHV | IGHV3-66-a | 0.27865 | Yes | 14 |
| IGHV | IGHV3-66-b | 0.12575 | Yes | 14 |
| IGHV | IGHV3-66-c | 0.0649 | No | 13 |
| IGHV | IGHV4-28-a | 0.41435 | Yes | 14 |
| IGHV | IGHV4-28-b | 0.13715 | Yes | 14 |
| IGHV | IGHV4-28-c | 0.07385 | No | 14 |
| IGHV | IGHV4-28-d | 0.06485 | No | 13 |
| IGHV | IGHV4-31-a | 0.5475 | Yes | 14 |
| IGHV | IGHV4-31-b | 0.2035 | Yes | 14 |
| IGHV | IGHV4-31-c | 0.1554 | Yes | 14 |
| IGHV | IGHV4-39-a | 0.10295 | Yes | 14 |
| IGHV | IGHV4-39-b | 0.0918 | No | 14 |
| IGHV | IGHV4-4-a | 0.1136 | Yes | 14 |
| IGHV | IGHV4-4-d | 0.0817 | No | 14 |
| IGHV | IGHV4-4-f | 0.06675 | No | 14 |
| IGHV | IGHV4-4-b | 0.06385 | No | 13 |
| IGHV | IGHV4-4-c | 0.0599 | No | 14 |
| IGHV | IGHV4-4-e | 0.05215 | No | 12 |
| IGHV | IGHV4-61-a | 0.5701 | Yes | 14 |
| IGHV | IGHV4-61-b | 0.18115 | Yes | 14 |
| IGHV | IGHV4-61-c | 0.13535 | Yes | 13 |
| IGHV | IGHV4-61-f | 0.05855 | No | 14 |

| Kappa C | | | | |
|---|---|---|---|---|
| IGKC | IGKC-a | 0.2427 | Yes | 14 |

| Kappa V | | | | |
|---|---|---|---|---|
| IGKV | IGKV1-17-a | 0.08655 | No | 13 |
| IGKV | IGKV1-17-b | 0.0545 | No | 12 |
| IGKV | IGKV1-8-a | 0.20315 | Yes | 14 |
| IGKV | IGKV1-8-b | 0.1334 | Yes | 14 |
| IGKV | IGKV1-9-a | 0.1854 | Yes | 14 |
| IGKV | IGKV1D-42-a | 0.22345 | Yes | 14 |
| IGKV | IGKV1D-8-a | 0.1872 | Yes | 14 |
| IGKV | IGKV2-24-a | 0.1708 | Yes | 13 |
| IGKV | IGKV2-30-a | 0.24085 | Yes | 13 |
| IGKV | IGKV2D-26-a | 0.325 | Yes | 14 |
| IGKV | IGKV2D-29-a | 0.30175 | Yes | 14 |
| IGKV | IGKV3-7-a | 0.2018 | Yes | 14 |
| IGKV | IGKV3D-11-a | 0.35105 | Yes | 14 |
| IGKV | IGKV3D-15-a | 0.2061 | Yes | 14 |
| IGKV | IGKV5-2-a | 0.32315 | Yes | 14 |

| **Lambda C** | | | | |
|---|---|---|---|---|
| IGLC | IGLC7-a | 0.0751 | No | 14 |

| **Lambda J** | | | | |
|---|---|---|---|---|
| IGLJ | IGU2-a | 0.0741 | No | 13 |
| IGLJ | IGU7-a | 0.93225 | Yes | 14 |

| **Lambda V** | | | | |
|---|---|---|---|---|
| IGLV | IGLV10-54-a | 0.50725 | Yes | 14 |
| IGLV | IGLV10-54-b | 0.09425 | No | 14 |
| IGLV | IGLV11-55-a | 0.4463 | Yes | 14 |
| IGLV | IGLV1-36-a | 0.0942 | No | 14 |
| IGLV | IGLV1-47-a | 0.8413 | Yes | 14 |
| IGLV | IGLV1-50-a | 0.19325 | Yes | 14 |
| IGLV | IGLV1-51-a | 0.1177 | Yes | 11 |
| IGLV | IGLV2-14-a | 0.48365 | Yes | 14 |
| IGLV | IGLV2-14-c | 0.08855 | No | 14 |
| IGLV | IGLV2-14-b | 0.0804 | No | 10 |
| IGLV | IGLV2-18-a | 0.763 | Yes | 14 |
| IGLV | IGLV2-23-a | 0.34655 | Yes | 14 |
| IGLV | IGLV2-8-a | 0.1451 | Yes | 11 |
| IGLV | IGLV3-12-a | 0.38715 | Yes | 14 |
| IGLV | IGLV3-12-b | 0.1116 | Yes | 14 |
| IGLV | IGLV3-12-c | 0.0774 | No | 14 |
| IGLV | IGLV3-19-a | 0.10015 | Yes | 12 |
| IGLV | IGLV3-21-a | 0.31195 | Yes | 14 |
| IGLV | IGLV3-21-b | 0.178 | Yes | 14 |
| IGLV | IGLV3-21-c | 0.1613 | Yes | 14 |
| IGLV | IGLV3-22-a | 0.2962 | Yes | 14 |
| IGLV | IGLV3-25-a | 0.6581 | Yes | 14 |
| IGLV | IGLV3-25-b | 0.19965 | Yes | 14 |
| IGLV | IGLV4-60-a | 0.4868 | Yes | 14 |
| IGLV | IGLV4-60-b | 0.3607 | Yes | 14 |
| IGLV | IGLV5-37-a | 0.1266 | Yes | 13 |
| IGLV | IGLV5-45-a | 0.1706 | Yes | 12 |
| IGLV | IGLV5-48-a | 0.0873 | No | 14 |
| IGLV | IGLV6-57-a | 0.3734 | Yes | 14 |
| IGLV | IGLV6-57-b | 0.2802 | Yes | 14 |
| IGLV | IGLV7-46-a | 0.9166 | Yes | 14 |
| IGLV | IGLV8-61-a | 0.2193 | Yes | 12 |

**Table 14B: Variants Having a Cumulative Frequency Greater than 20%**

| **GENE SEGMENT** | **VARIANT NAME** | **CUMULATIAVE FREQUENCY** | **NO UNIQUE POPULATIONS** |
|---|---|---|---|
| **Heavy** C | | | |
| IGHA | IGHA1-a | 0.31385 | 14 |
| IGHA | IGHA2-a | 0.35655 | 14 |
| IGHG | IGHG2-a | 0.28675 | 12 |
| IGHG | IGHG3-a | 0.2813 | 14 |
| IGHM | IGHM-a | 0.7527 | 14 |

| **Heavy J** | | | |
|---|---|---|---|
| IGHJ | IGHJ6-a | 0.6845 | 14 |

| **Heavy V** | | | |
|---|---|---|---|
| IGHV | IGHV1-2-a | 0.3268 | 14 |
| IGHV | IGHV1-3-a | 0.3564 | 14 |
| IGHV | IGHV1-58-a | 0.4503 | 14 |
| IGHV | IGHV1-58-b | 0.3144 | 14 |
| IGHV | IGHV2-5-a | 0.6657 | 14 |
| IGHV | IGHV2-70-a | 0.31 | 14 |
| IGHV | IGHV3-20-a | 0.2829 | 14 |
| IGHV | IGHV3-43-a | 0.2428 | 14 |
| IGHV | IGHV3-48-a | 0.3355 | 14 |
| IGHV | IGHV3-48-b | 0.20455 | 14 |
| IGHV | IGHV3-49-a | 0.38705 | 14 |
| IGHV | IGHV3-49-b | 0.28555 | 13 |
| IGHV | IGHV3-53-a | 0.2535 | 14 |
| IGHV | IGHV3-53-b | 0.2264 | 13 |
| IGHV | IGHV3-64-a | 0.47965 | 14 |
| IGHV | IGHV3-66-a | 0.27865 | 14 |
| IGHV | IGHV4-28-a | 0.41435 | 14 |
| IGHV | IGHV4-31-a | 0.5475 | 14 |
| IGHV | IGHV4-31-b | 0.2035 | 14 |
| IGHV | IGHV4-61-a | 0.5701 | 14 |

| **Kappa C** | | | |
|---|---|---|---|
| IGKC | IGKC-a | 0.2427 | 14 |
| | IGKV1-8-a | 0.20315 | 14 |

| **Kappa V** | | | |
|---|---|---|---|
| IGKV | | | |
| IGKV | IGKV1D-42-a | 0.22345 | 14 |
| IGKV | IGKV2-30-a | 0.24085 | 13 |
| IGKV | IGKV2D-26-a | 0.325 | 14 |
| IGKV | IGKV2D-29-a | 0.30175 | 14 |
| IGKV | IGKV3-7-a | 0.2018 | 14 |
| IGKV | IGKV3D-11-a | 0.35105 | 14 |
| IGKV | IGKV3D-15-a | 0.2061 | 14 |
| IGKV | IGKV5-2-a | 0.32315 | 14 |

| **Lambda J** | | | |
|---|---|---|---|
| IGLJ | IGU7-a | 0.93225 | 14 |

| **Lambda V** | | | |
|---|---|---|---|
| IGLV | IGLV10-54-a | 0.50725 | 14 |
| IGLV | IGLV11-55-a | 0.4463 | 14 |
| IGLV | IGLV1-47-a | 0.8413 | 14 |
| IGLV | IGLV2-14-a | 0.48365 | 14 |
| IGLV | IGLV2-18-a | 0.763 | 14 |
| IGLV | IGLV2-23-a | 0.34655 | 14 |
| IGLV | IGLV3-12-a | 0.38715 | 14 |
| IGLV | IGLV3-21-a | 0.31195 | 14 |
| IGLV | IGLV3-22-a | 0.2962 | 14 |
| IGLV | IGLV3-25-a | 0.6581 | 14 |
| IGLV | IGLV4-60-a | 0.4868 | 14 |
| IGLV | IGLV4-60-b | 0.3607 | 14 |
| IGLV | IGLV6-57-a | 0.3734 | 14 |
| IGLV | IGLV6-57-b | 0.2802 | 14 |
| IGLV | IGLV7-46-a | 0.9166 | 14 |
| IGLV | IGLV8-61-a | 0.2193 | 12 |

**Table 14C: Variants Having a Cumulative Frequency Greater than 30%**

| **GENE SEGMENT** | **VARIANT NAME** | **CUMULATIVE FREQUENCY** | **NO UNIQUE POPULATIONS** |
|---|---|---|---|
| **Heavy C** | | | |
| IGHA | IGHA1-a | 0.31385 | 14 |
| IGHA | IGHA2-a | 0.35655 | 14 |
| IGHM | IGHM-a | 0.7527 | 14 |

| **Heavy J** | | | |
|---|---|---|---|
| IGHJ | IGHJ6-a | 0.6845 | 14 |
| | | | |

| **Heavy V** | | | |
|---|---|---|---|
| IGHV | IGHV1-2-a | 0.3268 | 14 |
| IGHV | IGHV1-3-a | 0.3564 | 14 |
| IGHV | IGHV1-58-a | 0.4503 | 14 |
| IGHV | IGHV1-58-b | 0.3144 | 14 |
| IGHV | IGHV2-5-a | 0.6657 | 14 |
| IGHV | IGHV2-70-a | 0.31 | 14 |
| IGHV | IGHV3-48-a | 0.3355 | 14 |
| IGHV | IGHV3-49-a | 0.38705 | 14 |
| IGHV | IGHV3-64-a | 0.47965 | 14 |
| IGHV | IGHV4-28-a | 0.41435 | 14 |
| IGHV | IGHV4-31-a | 0.5475 | 14 |
| IGHV | IGHV4-61-a | 0.5701 | 14 |
| IGKV | IGKV2D-26-a | 0.325 | 14 |
| IGKV | IGKV2D-29-a | 0.30175 | 14 |
| IGKV | IGKV3D-11-a | 0.35105 | 14 |
| IGKV | IGKV5-2-a | 0.32315 | 14 |

| **Lambda J** | | | |
|---|---|---|---|
| IGLJ | IGU7-a | 0.93225 | 14 |

| **Lambda V** | | | |
|---|---|---|---|
| IGLV | IGLV10-54-a | 0.50725 | 14 |
| IGLV | IGLV11-55-a | 0.4463 | 14 |
| IGLV | IGLV1-47-a | 0.8413 | 14 |
| IGLV | IGLV2-14-a | 0.48365 | 14 |
| IGLV | IGLV2-18-a | 0.763 | 14 |
| IGLV | IGLV2-23-a | 0.34655 | 14 |
| IGLV | IGLV3-12-a | 0.38715 | 14 |
| IGLV | IGLV3-21-a | 0.31195 | 14 |
| IGLV | IGLV3-25-a | 0.6581 | 14 |
| IGLV | IGLV4-60-a | 0.4868 | 14 |
| IGLV | IGLV4-60-b | 0.3607 | 14 |
| IGLV | IGLV6-57-a | 0.3734 | 14 |
| IGLV | IGLV7-46-a | 0.9166 | 14 |

**Table 14D: Variants Having a Cumulative Frequency Greater than 40%**

| **GENE SEGMENT** | **VARIANT NAME** | **CUMULATIAVE FREQUENCY** | **NO UNIQUE POPULATIONS** |
|---|---|---|---|
| **Heavy** C | | | |
| IGHM | IGHM-a | 0.7527 | 14 |

| **Heavy J** | | | |
|---|---|---|---|
| IGHJ | IGHJ6-a | 0.6845 | 14 |
| | | | |

| **Heavy V** | | | |
|---|---|---|---|
| IGHV | IGHV1-58-a | 0.4503 | 14 |
| IGHV | IGHV2-5-a | 0.6657 | 14 |
| IGHV | IGHV3-64-a | 0.47965 | 14 |
| IGHV | IGHV4-28-a | 0.41435 | 14 |
| IGHV | IGHV4-31-a | 0.5475 | 14 |
| IGHV | IGHV4-61-a | 0.5701 | 14 |

| **Lambda J** | | | |
|---|---|---|---|
| IGLJ | IGU7-a | 0.93225 | 14 |

| **Lambda V** | | | |
|---|---|---|---|
| IGLV | IGLV10-54-a | 0.50725 | 14 |
| IGLV | IGLV11-55-a | 0.4463 | 14 |
| IGLV | IGLV1-47-a | 0.8413 | 14 |
| IGLV | IGLV2-14-a | 0.48365 | 14 |
| IGLV | IGLV2-18-a | 0.763 | 14 |
| IGLV | IGLV3-25-a | 0.6581 | 14 |
| IGLV | IGLV4-60-a | 0.4868 | 14 |
| IGLV | IGLV7-46-a | 0.9166 | 14 |

**Table 14E: Variants Having a Cumulative Frequency Greater than 50%**

| **GENE SEGMENT** | **VARIANT NAME** | **CUMULATIVE FREQUENCY** | **NO UNIQUE POPULATIONS** |
|---|---|---|---|
| **Heavy C** | | | |
| IGHM | IGHM-a | 0.7527 | 14 |

| **Heavy J** | | | |
|---|---|---|---|
| IGHJ | IGHJ6-a | 0.6845 | 14 |
| | | | |

| **Heavy V** | | | |
|---|---|---|---|
| IGHV | IGHV2-5-a | 0.6657 | 14 |
| IGHV | IGHV4-31-a | 0.5475 | 14 |
| IGHV | IGHV4-61-a | 0.5701 | 14 |

| Lambda J | | | |
|---|---|---|---|
| IGLJ | IGU7-a | 0.93225 | 14 |
| **Lambda V** | IGLV10-54-a | 0.50725 | 14 |
| IGLV | | | |
| IGLV | IGLV1-47-a | 0.8413 | 14 |
| IGLV | IGLV2-18-a | 0.763 | 14 |
| IGLV | IGLV3-25-a | 0.6581 | 14 |
| IGLV | IGLV7-46-a | 0.9166 | 14 |

**Table 14F: Variants Having a Cumulative Frequency Greater than 60%**

| **GENE SEGMENT** | **VARIANT NAME** | **CUMULATIVE FREQUENCY** | **NO UNIQUE POPULATIONS** |
|---|---|---|---|
| **Heavy C** | | | |
| IGHM | IGHM-a | 0.7527 | 14 |

| **Heavy J** | | | |
|---|---|---|---|
| IGHJ | IGHJ6-a | 0.6845 | 14 |
| | | | |

| **Heavy V** | | | |
|---|---|---|---|
| IGHV | IGHV2-5-a | 0.6657 | 14 |

| **Lambda J** | | | |
|---|---|---|---|
| IGLJ | IGU7-a | 0.93225 | 14 |

| **Lambda V** | | | |
|---|---|---|---|
| IGLV | IGLV1-47-a | 0.8413 | 14 |
| IGLV | IGLV2-18-a | 0.763 | 14 |
| IGLV | IGLV3-25-a | 0.6581 | 14 |
| IGLV | IGLV7-46-a | 0.9166 | 14 |

**Table 14G: Variants Having a Cumulative Frequency Greater than 70%**

| **GENE SEGMENT** | **VARIANT NAME** | **CUMULATIVE FREQUENCY** | **NO UNIQUE POPULATIONS** |
|---|---|---|---|
| **Heavy C** | | | |
| IGHM | IGHM-a | 0.7527 | 14 |

| **Lambda J** | | | |
|---|---|---|---|
| IGLJ | IGU7-a | 0.93225 | 14 |

| **Lambda V** | | | |
|---|---|---|---|
| IGLV | IGLV1-47-a | 0.8413 | 14 |
| IGLV | IGLV2-18-a | 0.763 | 14 |
| IGLV | IGLV7-46-a | 0.9166 | 14 |

**Table 14H: Variants Having a Cumulative Frequency Greater than 80%**

| **GENE SEGMENT** | **VARIANT NAME** | **CUMULATIVE FREQUENCY** | **NO UNIQUE POPULATIONS** |
|---|---|---|---|
| **Lambda J** | | | |
| IGLJ | IGLJ7-a | 0.93225 | 14 |
| **Lambda V** | IGLV1-47-a | 0.8413 | 14 |
| IGLV | | | |
| IGLV | IGLV7-46-a | 0.9166 | 14 |

**Table 14I: Variants Having a Cumulative Frequency Greater than 90%**

| **GENE SEGMENT** | **VARIANT NAME** | **CUMULATIVE FREQUENCY** | **NO UNIQUE POPULATIONS** |
|---|---|---|---|
| **Lambda J** | | | |
| IGLJ | IGU7-a | 0.93225 | 14 |

| **Lambda V** | | | |
|---|---|---|---|
| IGLV | IGLV7-46-a | 0.9166 | 14 |

**Table 15: NAÏVE HEAVY CHAIN REPERTOIRES**

| **TABLE 15A**: **All Naïve - J Usage** | | | |
|---|---|---|---|
| J | Average HCDR3 Length | Count | % |
| IGHJ1*01 | 11 | 12 | 0.90% |
| IGHJ2*01 | 12 | 47 | 3.51% |
| IGHJ3*02 | 12 | 205 | 15.30% |
| IGHJ4*02 | 11 | 689 | 51.42% |
| IGHJ5*02 | 13 | 88 | 6.57% |
| **IGHJ6*02** | **16** | **299** | **22.31%** |

| **Naive HCDR3>=20** | | | |
|---|---|---|---|
| | Average | | |
| | HCDR3 | | |
| J | Length | Count | % |
| IGHJ3*02 | 20 | 1 | 2.22% |
| IGHJ4*02 | 20 | 1 | 2.22% |
| IGHJ5*02 | 20 | 2 | 4.44% |
| **IGHJ6*02** | **21** | **41** | **91.11%** |

**Naïve - Long HCDR3 using IGHJ6*02**

| HCDR3Length | Count |
|---|---|
| 20 | 19 |
| 21 | 10 |
| 22 | 7 |
| 23 | 3 |
| 24 | 1 |
| 26 | 1 |

| **TABLE 15B: All Naive - J & D Usage** | | | |
|---|---|---|---|
| J | D | Average HCDR3 Length | Count |
| IGHJ6*02 | IGHD3-9*01 | 19 | 21 |
| IGHJ6*02 | IGHD4-17*01 | 18 | 7 |
| IGHJ6*02 | IGHD3-10*01 | 17 | 98 |
| IGHJ6*02 | IGHD2-2*02 | 17 | 4 |
| IGHJ6*02 | IGHD5-24*01 | 17 | 1 |
| IGHJ6*02 | IGHD6-19*01 | 16 | 30 |
| IGHJ6*02 | IGHD3-22*01 | 16 | 5 |
| IGHJ6*02 | IGHD6-13*01 | 16 | 33 |
| IGHJ6*02 | IGHD5-12*01 | 15 | 7 |
| IGHJ6*02 | IGHD1-26*01 | 15 | 25 |
| IGHJ6*02 | IGHD1-20*01 | 14 | 6 |
| IGHJ6*02 | IGHD5-18*01 | 14 | 3 |
| IGHJ6*02 | IGHD3-16*02 | 14 | 6 |
| IGHJ6*02 | IGHD2-21*02 | 13 | 5 |
| IGHJ6*02 | IGHD1-14*01 | 13 | 4 |
| IGHJ6*02 | IGHD7-27*02 | 12 | 8 |
| IGHJ6*02 | IGHD1-1*01 | 12 | 8 |
| IGHJ6*02 | IGHD6-25*01 | 12 | 3 |
| IGHJ6*02 | IGHD4-23*01 | 11 | 2 |

| **Naive HCDR3>=20** | | | |
|---|---|---|---|
| J | D | Average HCDR3 Length | Count |
| IGHJ6*02 | IGHD6-19*01 | 23 | 1 |
| IGHJ6*02 | IGHD4-17*01 | 22 | 2 |
| IGHJ6*02 | IGHD3-9*01 | 21 | 11 |
| IGHJ6*02 | IGHD3-10*01 | 21 | 25 |
| IGHJ6*02 | IGHD6-13*01 | 20 | 1 |
| IGHJ6*02 | IGHD3-22*01 | 20 | 1 |

| **TABLE 15C: All Naïve - V & J Usage** | | | |
|---|---|---|---|
| V | J | Average HCDR3 Length | Count |
| IGHV3-21*03 | IGHJ6*02 | 20 | 5 |
| IGHV1-18*01 | IGHJ6*02 | 20 | 10 |
| IGHV1-2*02 | IGHJ6*02 | 19 | 5 |
| IGHV3-13*01 | IGHJ6*02 | 19 | 3 |
| IGHV3-7*01 | IGHJ6*02 | 19 | 6 |
| IGHV1-8*01 | IGHJ6*02 | 19 | 21 |
| IGHV7-4-1*01 | IGHJ6*02 | 19 | 9 |
| IGHV3-9*01 | IGHJ6*02 | 18 | 13 |
| IGHV4-61*01 | IGHJ6*02 | 17 | 20 |
| IGHV3-23*04 | IGHJ6*02 | 17 | 6 |
| IGHV4-4*02 | IGHJ6*02 | 16 | 34 |
| IGHV1-3*01 | IGHJ6*02 | 16 | 39 |
| IGHV3-20*d01 | IGHJ6*02 | 16 | 1 |
| IGHV6-1*01 | IGHJ6*02 | 14 | 69 |
| IGHV2-5*10 | IGHJ6*02 | 14 | 8 |
| IGHV3-11*01 | IGHJ6*02 | 14 | 3 |
| IGHV3-66*03 | IGHJ6*02 | 13 | 21 |
| IGHV3-15*01 | IGHJ6*02 | 12 | 3 |

| **Naive HCDR3>=20** | | | |
|---|---|---|---|
| V | J | Average | Count |
| | | HCDR3 | |
| | | Length | |
| IGHV3-21*03 | IGHJ6*02 | 23 | 3 |
| IGHV3-13*01 | IGHJ6*02 | 22 | 1 |
| IGHV3-7*01 | IGHJ6*02 | 22 | 3 |
| IGHV6-1*01 | IGHJ6*02 | 21 | 3 |
| IGHV1-8*01 | IGHJ6*02 | 21 | 10 |
| IGHV1-2*02 | IGHJ6*02 | 21 | 3 |
| IGHV7-4-1*01 | IGHJ6*02 | 21 | 3 |
| IGHV1-3*01 | IGHJ6*02 | 21 | 4 |
| IGHV1-18*01 | IGHJ6*02 | 21 | 5 |
| IGHV4-4*02 | IGHJ6*02 | 20 | 3 |
| IGHV3-9*01 | IGHJ6*02 | 20 | 2 |
| IGHV3-23*04 | IGHJ6*02 | 20 | 1 |

**TABLE 16: IMMUNISED HEAVY CHAIN REPERTOIRES**

| **TABLE 16A: All Immunised - J Usage** | | | |
|---|---|---|---|
| J | HCDR3 | Count | % |
| | Length | | |
| IGHJ1*01 | 11 | 2 | 0.78% |
| IGHJ2*01 | 14 | 7 | 2.73% |
| IGHJ3*02 | 15 | 12 | 4.69% |
| IGHJ4*02 | 15 | 120 | 46.88% |
| IGHJ5*02 | 15 | 19 | 7.42% |
| **IGHJ6*02** | **17** | **96** | **37.50%** |

| **HCDR3>20** | | | |
|---|---|---|---|
| J | HCDR3 Length | Count | % |
| IGHJ4*02 | 22 | 2 | 22.22% |
| IGHJ5*02 | 25 | 1 | 11.11% |
| **IGHJ6*02** | **21** | **6** | **66.67%** |

**Immunised - Long HCDR3 using IGHJ6*02**

| HCDR3 Length | Count |
|---|---|
| 20 | 4 |
| 21 | 1 |
| 24 | 1 |

**TABLE 16B:**

| | | | |
|---|---|---|---|
| **All Immunised** - **J & D Usage** | | | |
| J | D | HCDR3 Length | Count |
| IGHJ6*02 | IGHD4-17*01 | 18 | 1 |
| IGHJ6*02 | IGHD1-26*01 | 18 | 5 |
| IGHJ6*02 | IGHD6-19*01 | 17 | 27 |
| IGHJ6*02 | IGHD3-10*01 | 17 | 42 |
| IGHJ6*02 | IGHD6-13*01 | 16 | 2 |
| IGHJ6*02 | IGHD5-18*01 | 15 | 2 |
| IGHJ6*02 | IGHD4-23*01 | 15 | 5 |
| IGHJ6*02 | IGHD5-12*01 | 14 | 3 |
| IGHJ6*02 | IGHD3-16*02 | 14 | 3 |
| IGHJ6*02 | IGHD3-22*01 | 13 | 3 |
| IGHJ6*02 | IGHD3-9*01 | 12 | 1 |
| IGHJ6*02 | IGHD1-20*01 | 11 | 1 |

| **HCDR3>20** | | | |
|---|---|---|---|
| J | D | HCDR3 Length | Count |
| IGHJ6*02 | IGHD3-10*01 | 21 | 4 |
| IGHJ6*02 | IGHD6-19*01 | 20 | 1 |
| IGHJ6*02 | IGHD1-26*01 | 20 | 1 |

**TABLE 16C:**

| **All Immunised** - **V & J Usage** | | | |
|---|---|---|---|
| V | J | HCDR3 Length | Count |
| IGHV3-7*01 | IGHJ6*02 | 18 | 33 |
| IGHV1-8*01 | IGHJ6*02 | 17 | 3 |
| IGHV3-9*01 | IGHJ6*02 | 17 | 22 |
| IGHV3-13 *01 | IGHJ6*02 | 17 | 5 |
| IGHV1-2*02 | IGHJ6*02 | 16 | 8 |
| IGHV3-11 *01 | IGHJ6*02 | 16 | 3 |
| IGHV4-4*02 | IGHJ6*02 | 15 | 9 |
| IGHV6-1 *01 | IGHJ6*02 | 15 | 6 |
| IGHV1-3*01 | IGHJ6*02 | 15 | 6 |

| **HCDR3>20** | | | |
|---|---|---|---|
| V | J | HCDR3 Length | Count |
| IGHV3-7*01 | IGHJ6*02 | 21 | 4 |
| IGHV3-11*01 | IGHJ6*02 | 21 | 1 |
| IGHV4-4*02 | IGHJ6*02 | 20 | 1 |

**TABLE 17: ANTIGEN-SPECIFIC HEAVY CHAIN REPERTOIRES**

| **TABLE 17A: All Antigen-Specific - J Usage** | | | |
|---|---|---|---|
| J | HCDR3 Length | Count | % |
| IGHJ1 *01 | 12 | 2 | 1.68% |
| IGHJ3*02 | 17 | 4 | 3.36% |
| IGHJ4*02 | 13 | 64 | 53.78% |
| IGHJ5*02 | 19 | 6 | 5.04% |
| **IGHJ6*02** | **17** | **43** | **36.13%** |
| | | | |
| | | | |

| **HCDR3>20** | | | |
|---|---|---|---|
| J | HCDR3 **Length** | Count | % |
| IGHJ4*02 | 22 | 1 | 9.09% |
| IGHJ5*02 | 21 | 3 | 27.27% |
| **IGHJ6*02** | **21** | 7 | **63.64%** |

**Immunised - Long HCDR3 using IGHJ6*02**

| HCDR3Length | Count |
|---|---|
| 20 | 4 |
| 21 | 1 |
| 22 | 1 |
| 24 | 1 |

| **TABLE 17B: All Antigen-Specific** - **J & D Usage** | | | |
|---|---|---|---|
| J | D | HCDR3 Length | Count |
| IGHJ6*02 | IGHD3-9*01 | 19 | 6 |
| IGHJ6*02 | IGHD3-10*01 | 19 | 12 |
| IGHJ6*02 | IGHD6-19*01 | 17 | 13 |
| IGHJ6*02 | IGHD5-12*01 | 16 | 2 |
| IGHJ6*02 | IGHD2-15*01 | 16 | 1 |
| IGHJ6*02 | IGHD1-26*01 | 15 | 7 |
| IGHJ6*02 | IGHD3-16*02 | 12 | 1 |
| IGHJ6*02 | IGHD5-18*01 | 11 | 1 |
| | | | |

| **HCDR3>20** | | | |
|---|---|---|---|
| J | D | HCDR3 Length | Count |
| IGHJ6*02 | IGHD3-9*01 | 21 | 1 |
| IGHJ6*02 | IGHD3-10*01 | 21 | 6 |

| **TABLE 17C: All Antigen-Specific - V & J Usage** | | | |
|---|---|---|---|
| V | J | HCDR3 Length | Count |
| IGHV1-3*01 | IGHJ6*02 | 18 | 1 |
| IGHV3-7*01 | IGHJ6*02 | 18 | 6 |
| IGHV3-9*01 | IGHJ6*02 | 18 | 6 |
| IGHV1-8*01 | IGHJ6*02 | 17 | 6 |
| IGHV3-20*d01 | IGHJ6*02 | 17 | 3 |
| IGHV3-11*01 | IGHJ6*02 | 17 | 8 |
| IGHV4-4*02 | IGHJ6*02 | 17 | 5 |
| IGHV3-15*01 | IGHJ6*02 | 15 | 5 |
| IGHV3-13*01 | IGHJ6*02 | 15 | 3 |
| | I | | |

| **HCDR3>20** | | | |
|---|---|---|---|
| V | J | HCDR3 Length | Count |
| IGHV4-4*02 | IGHJ6*02 | 20 | 2 |

| | | | |
|---|---|---|---|
| IGHV1-8*01 | IGHJ6*02 | 22 | 2 |
| IGHV3-9*01 | IGHJ6*02 | 20 | 1 |
| IGHV3-11 *01 | IGHJ6*02 | 21 | 1 |
| IGHV3-20*d01 | IGHJ6*02 | 22 | 1 |

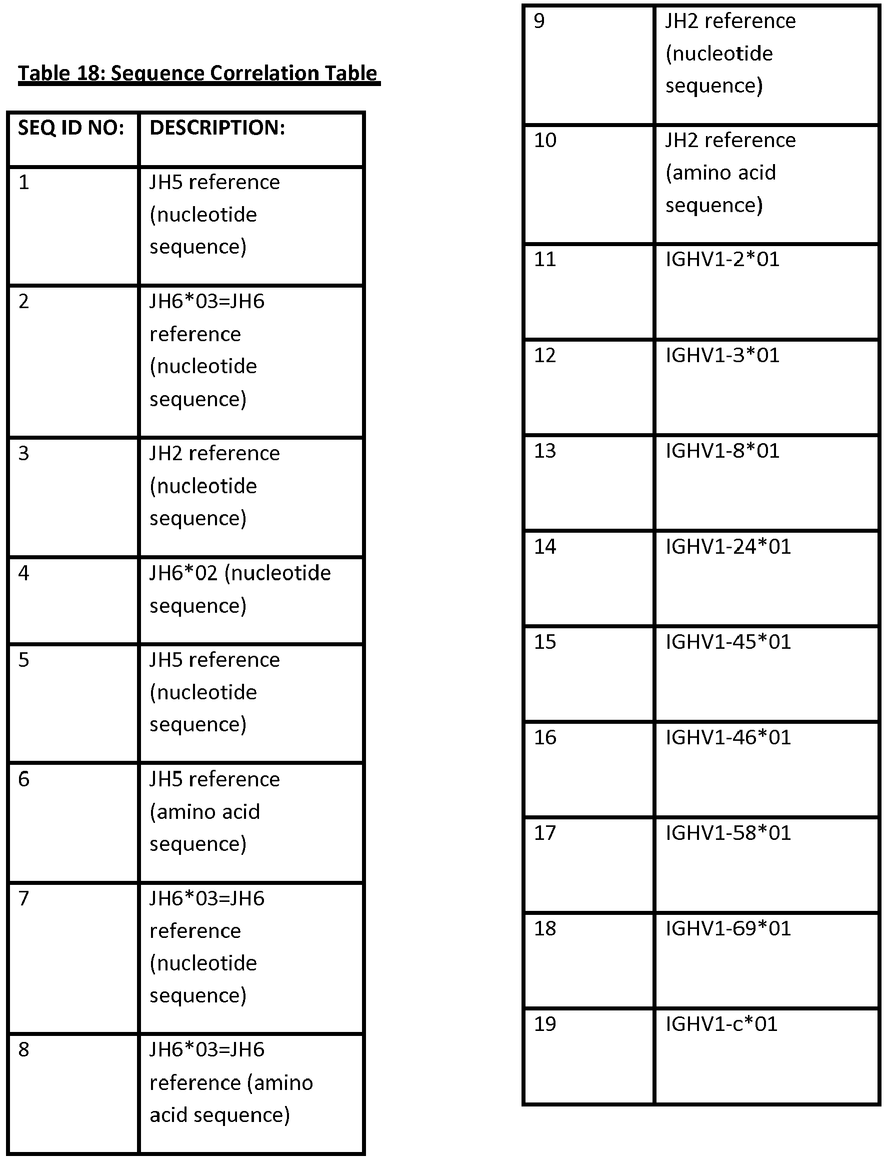

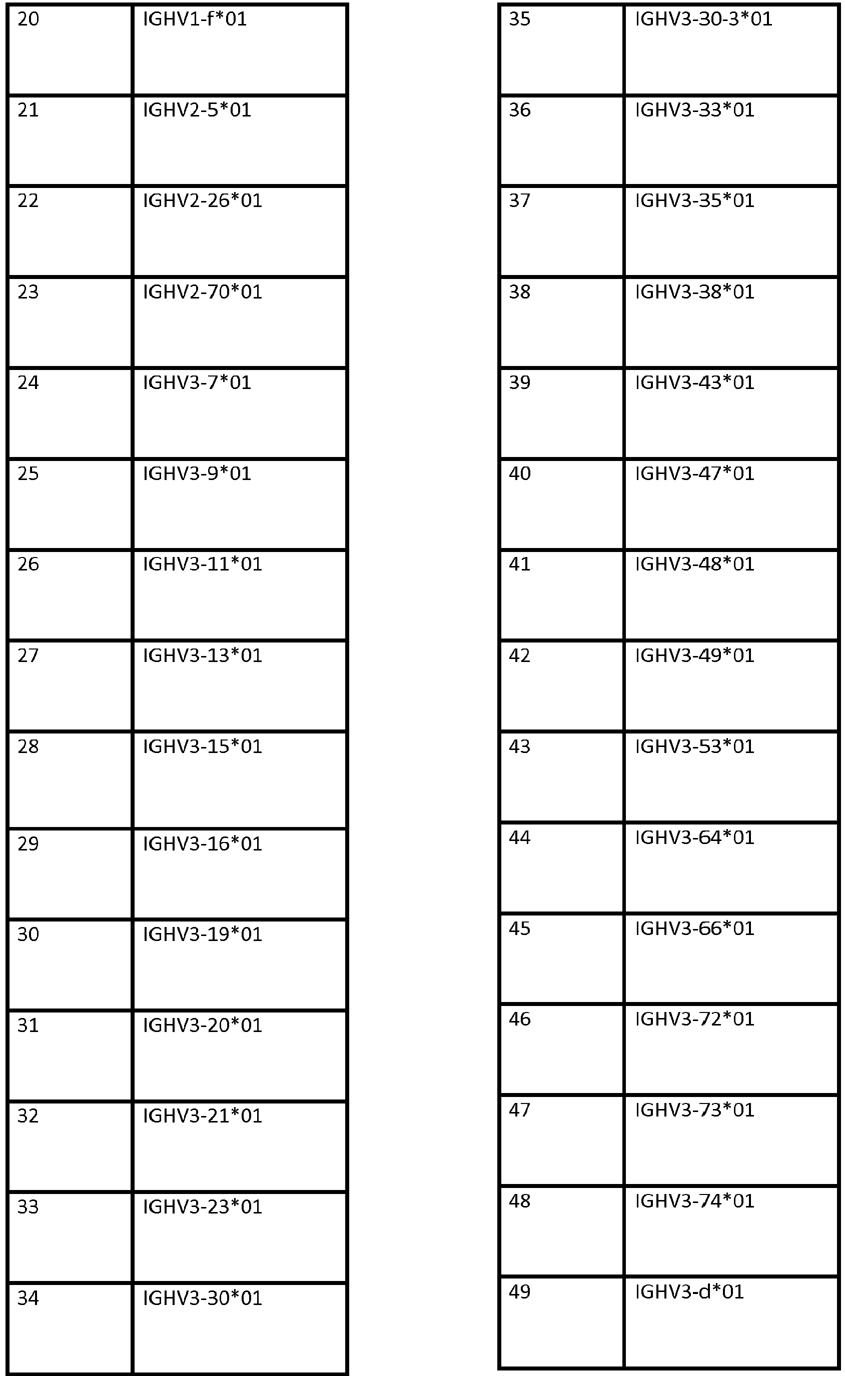

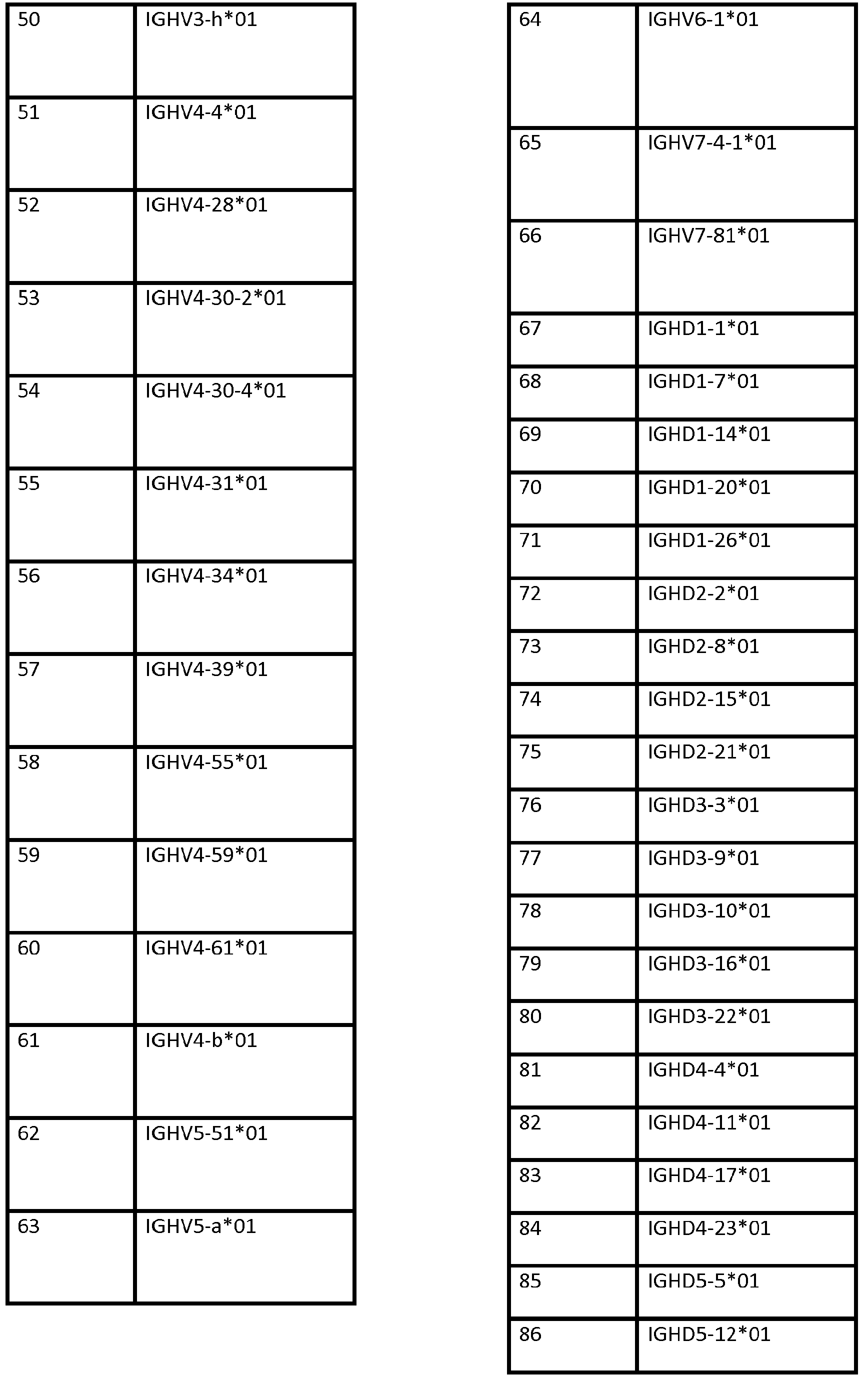

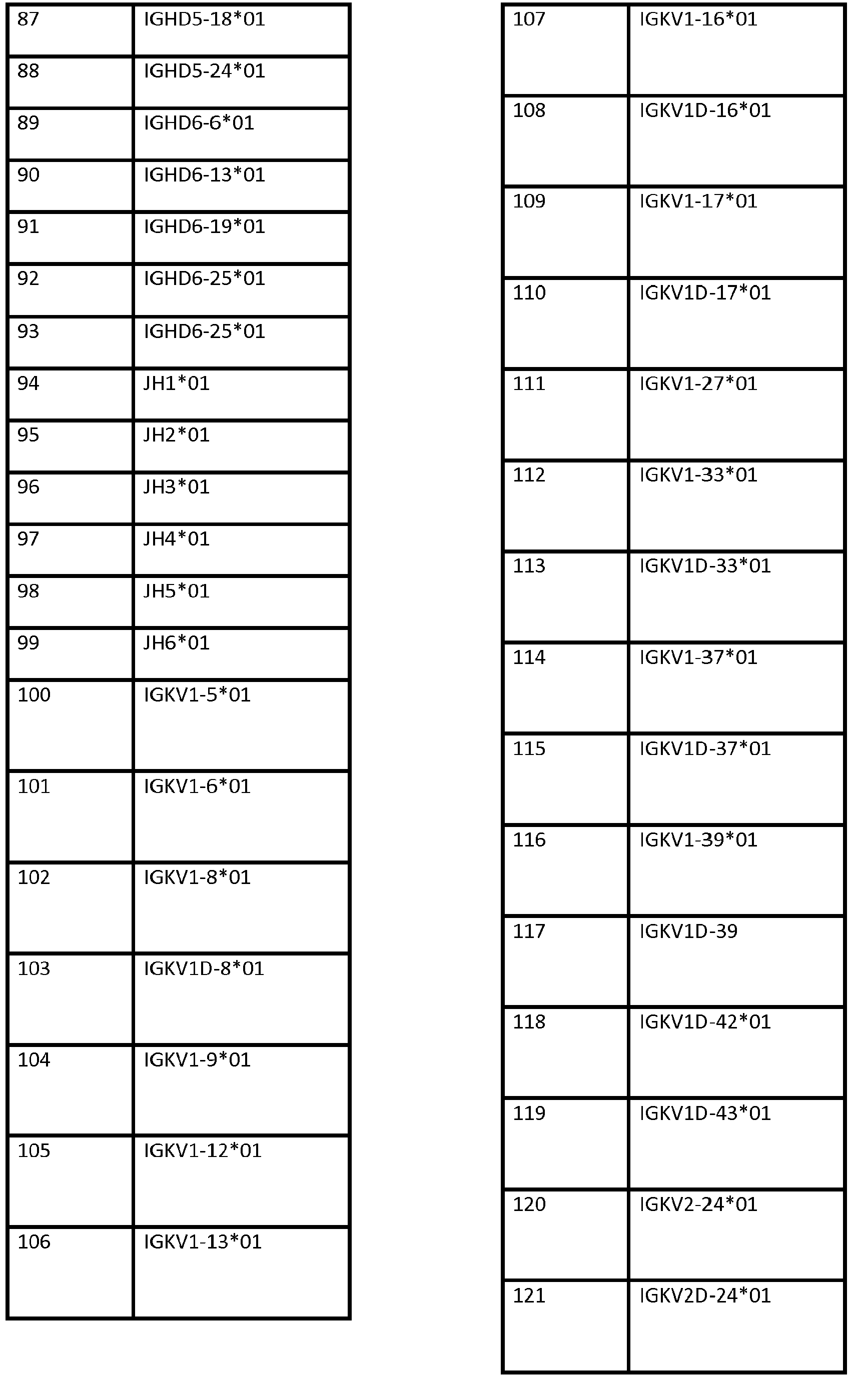

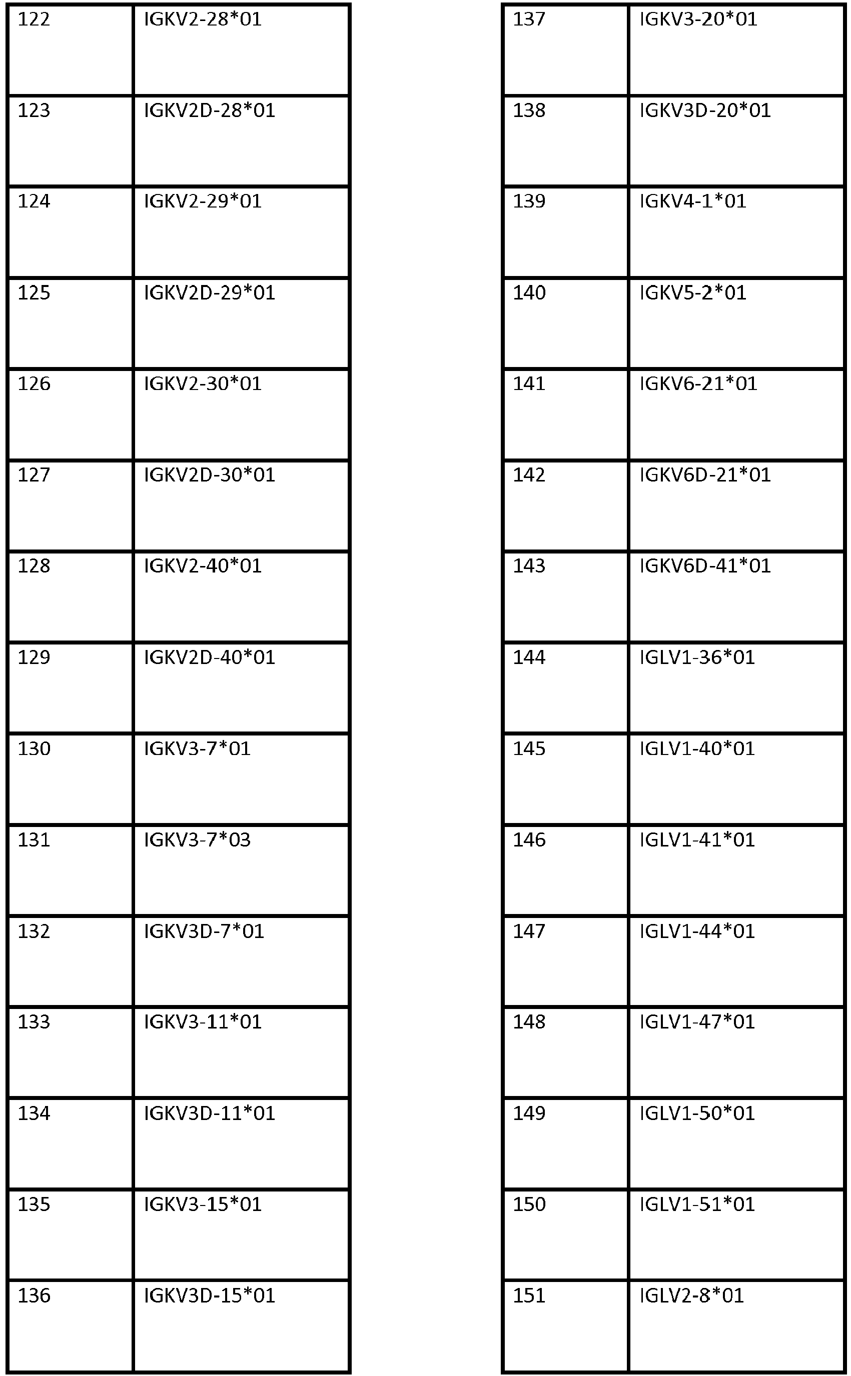

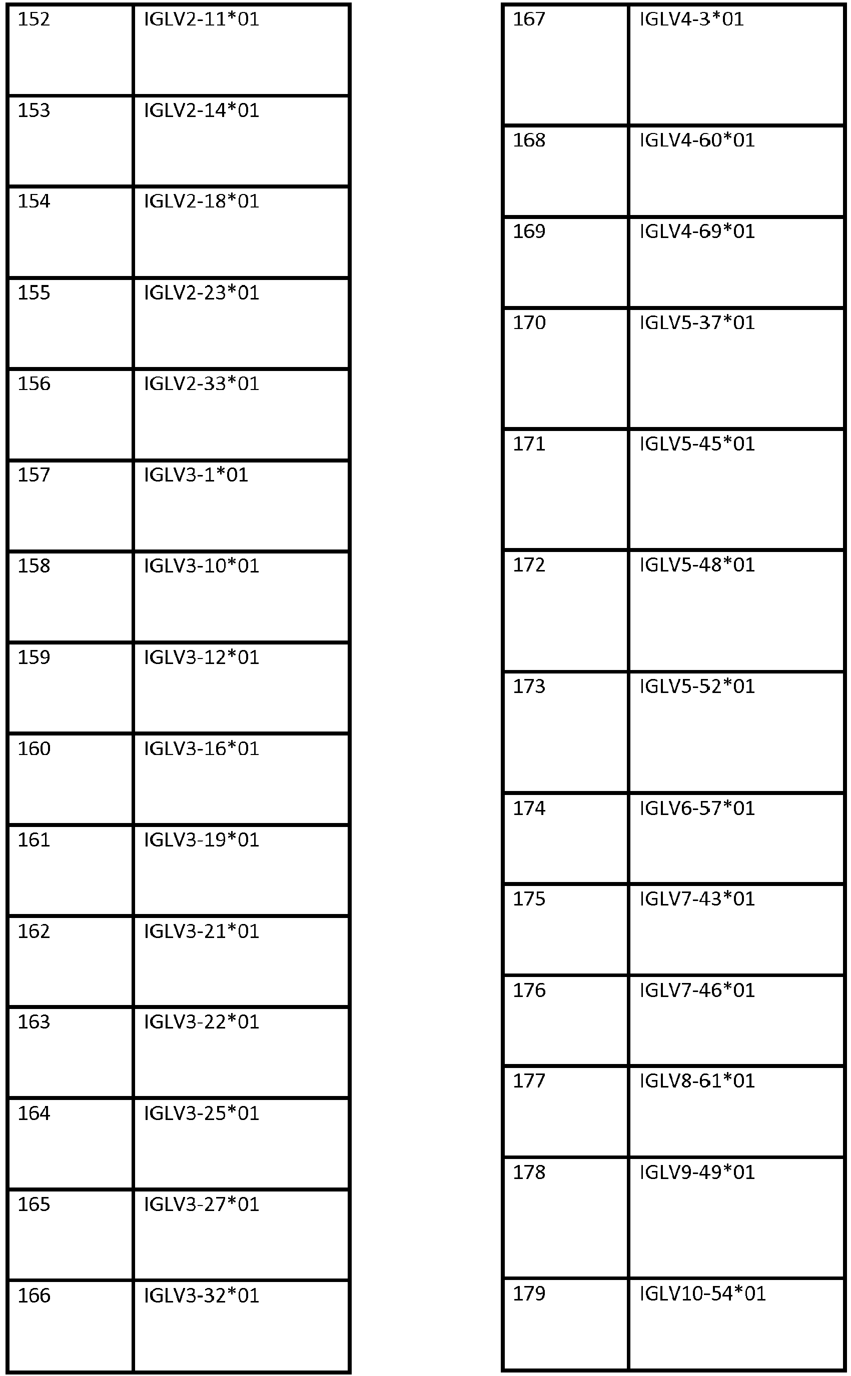

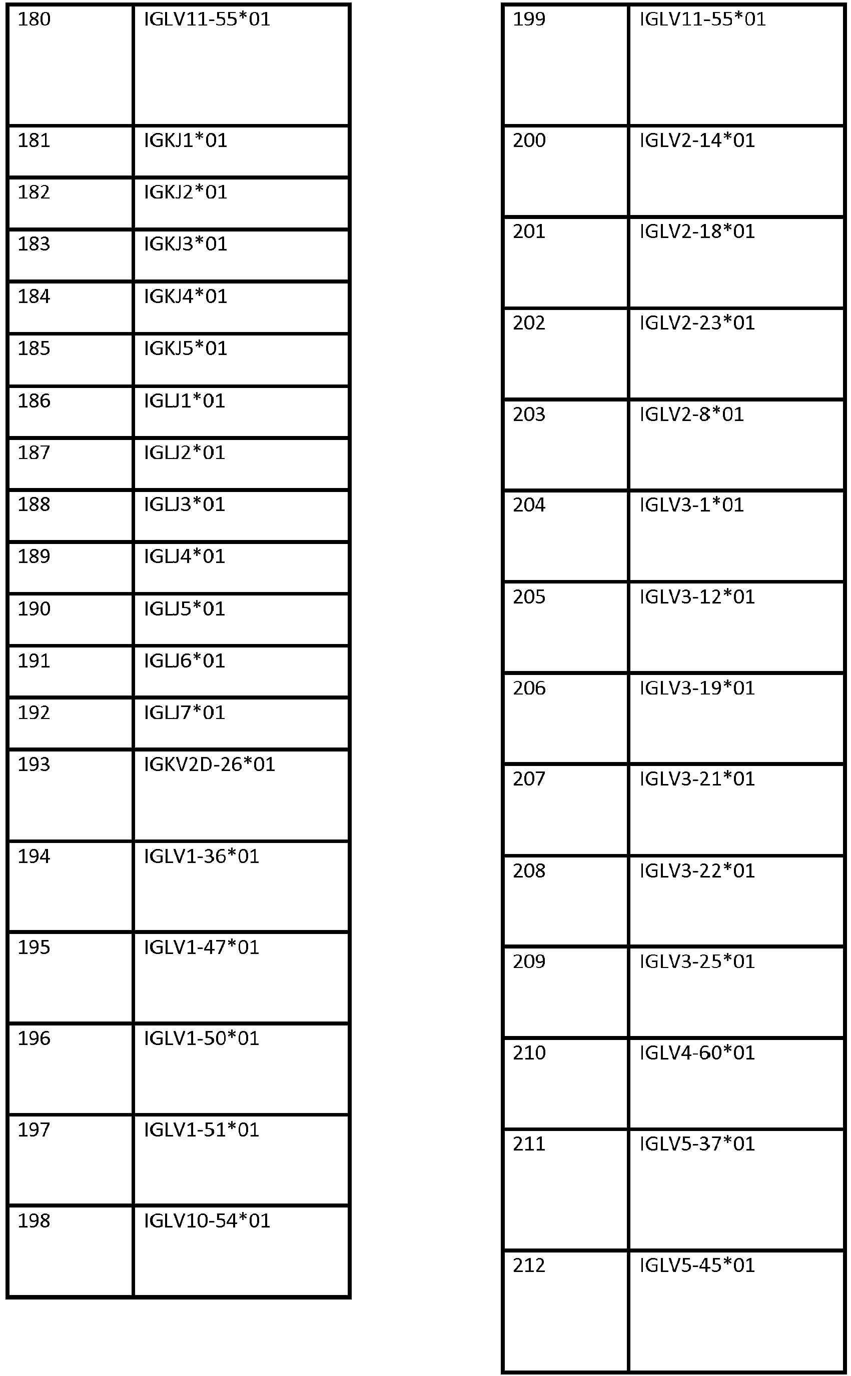

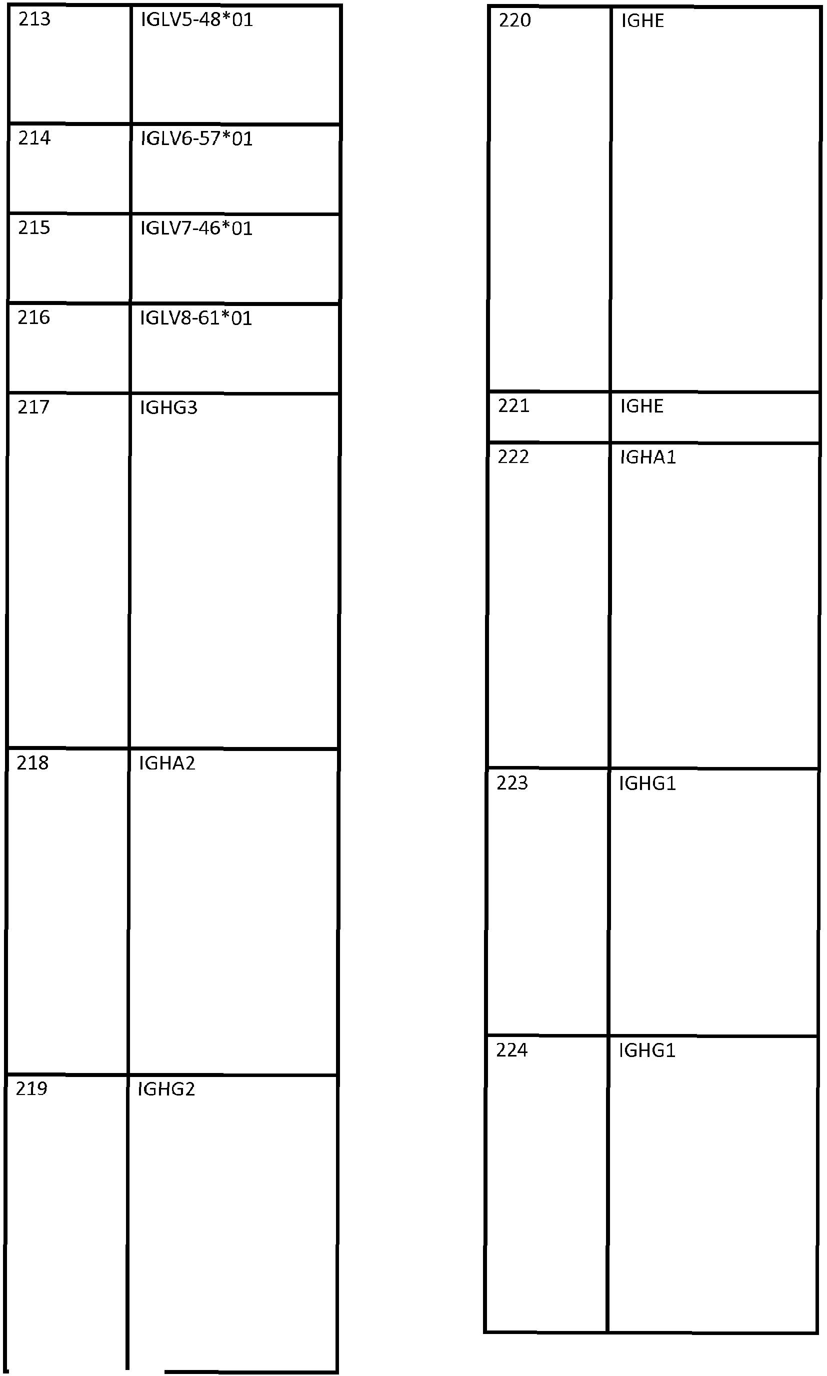

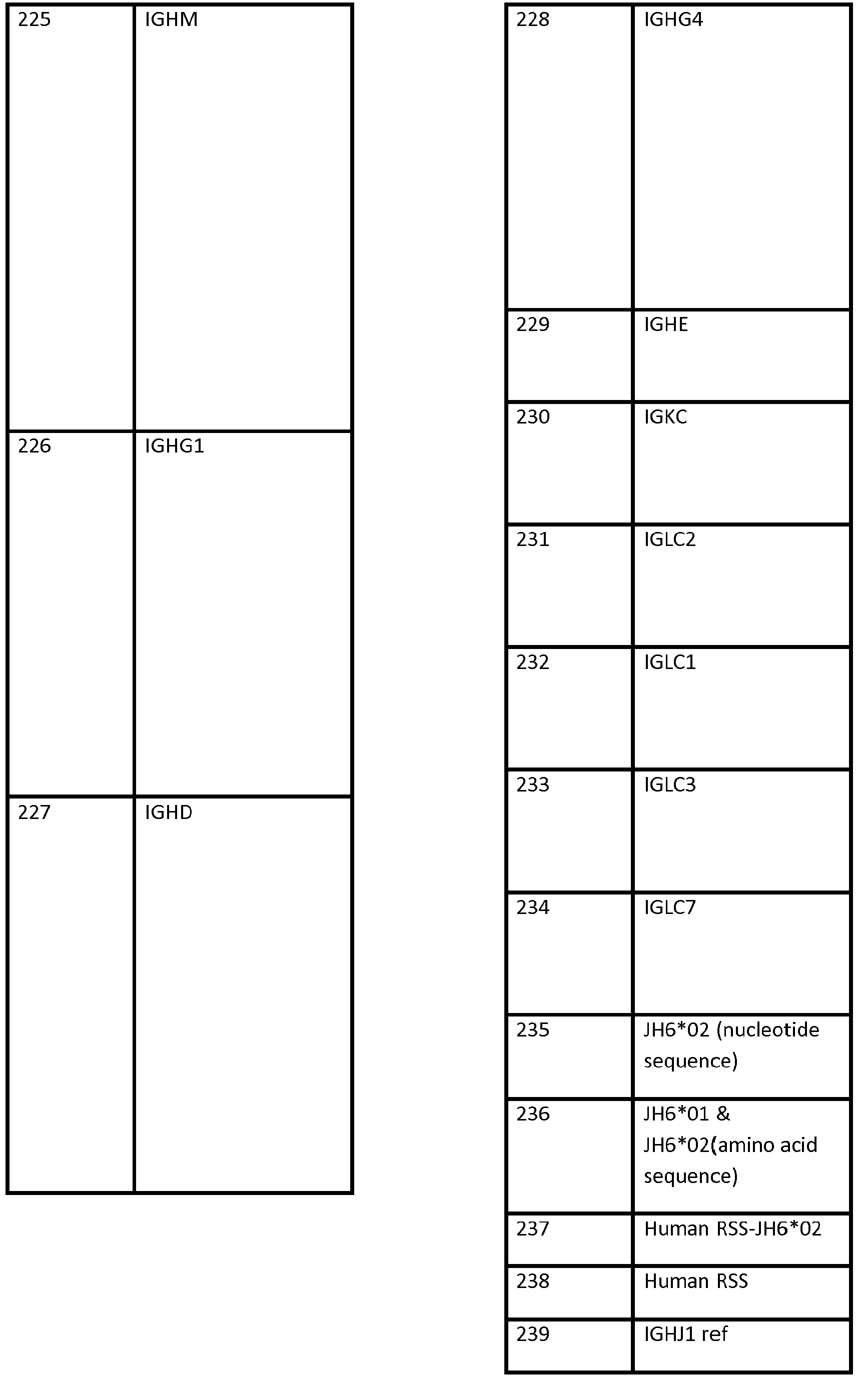

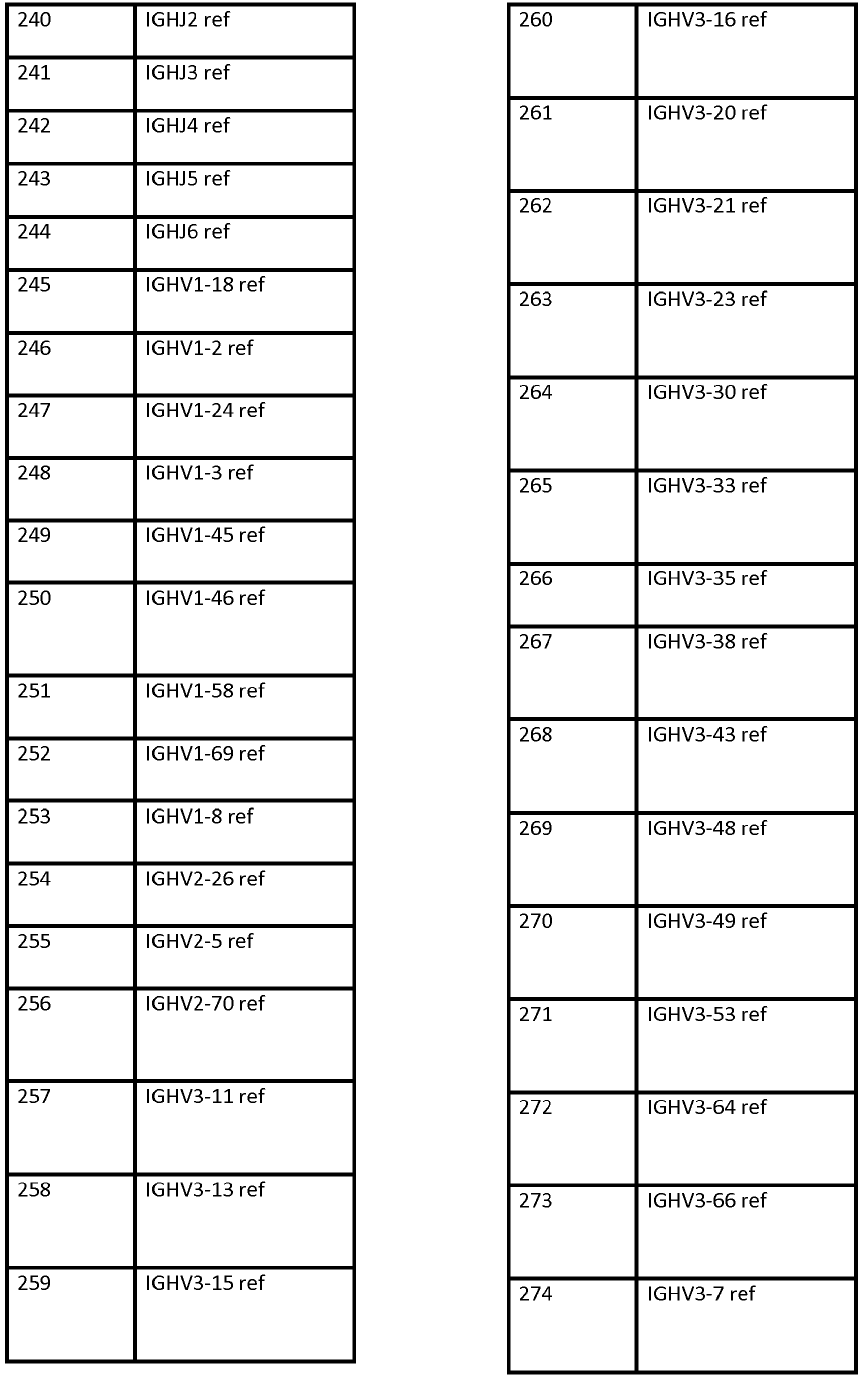

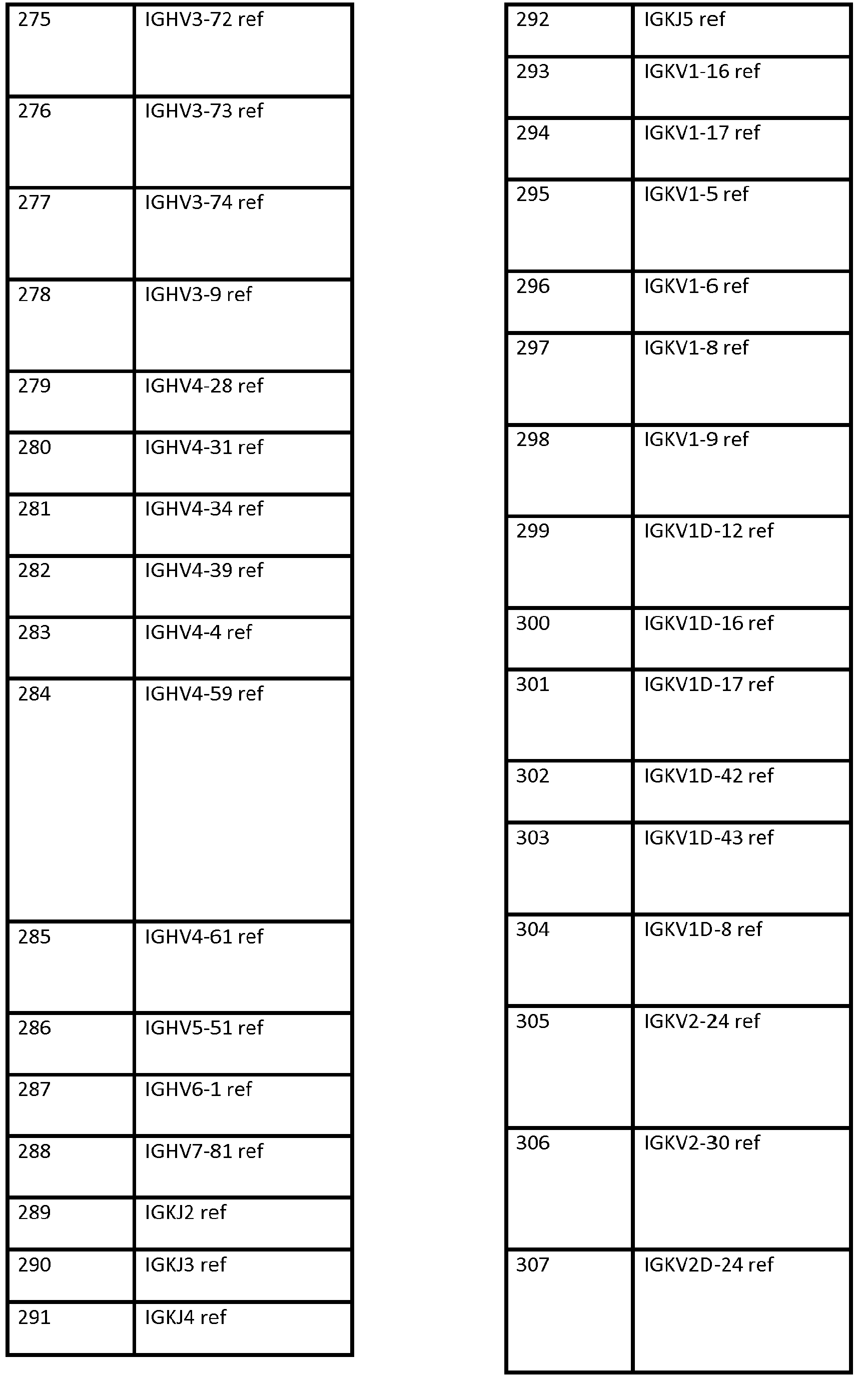

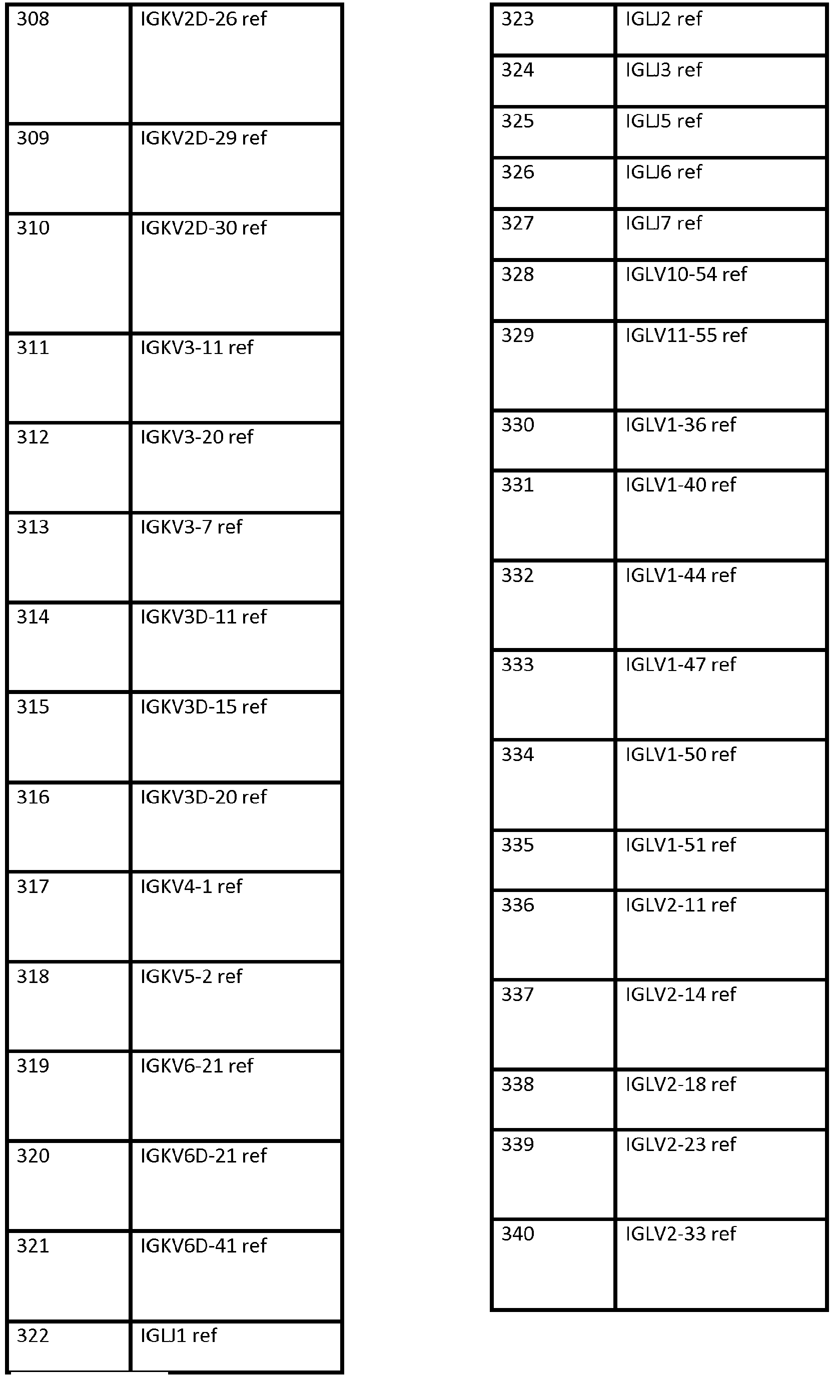

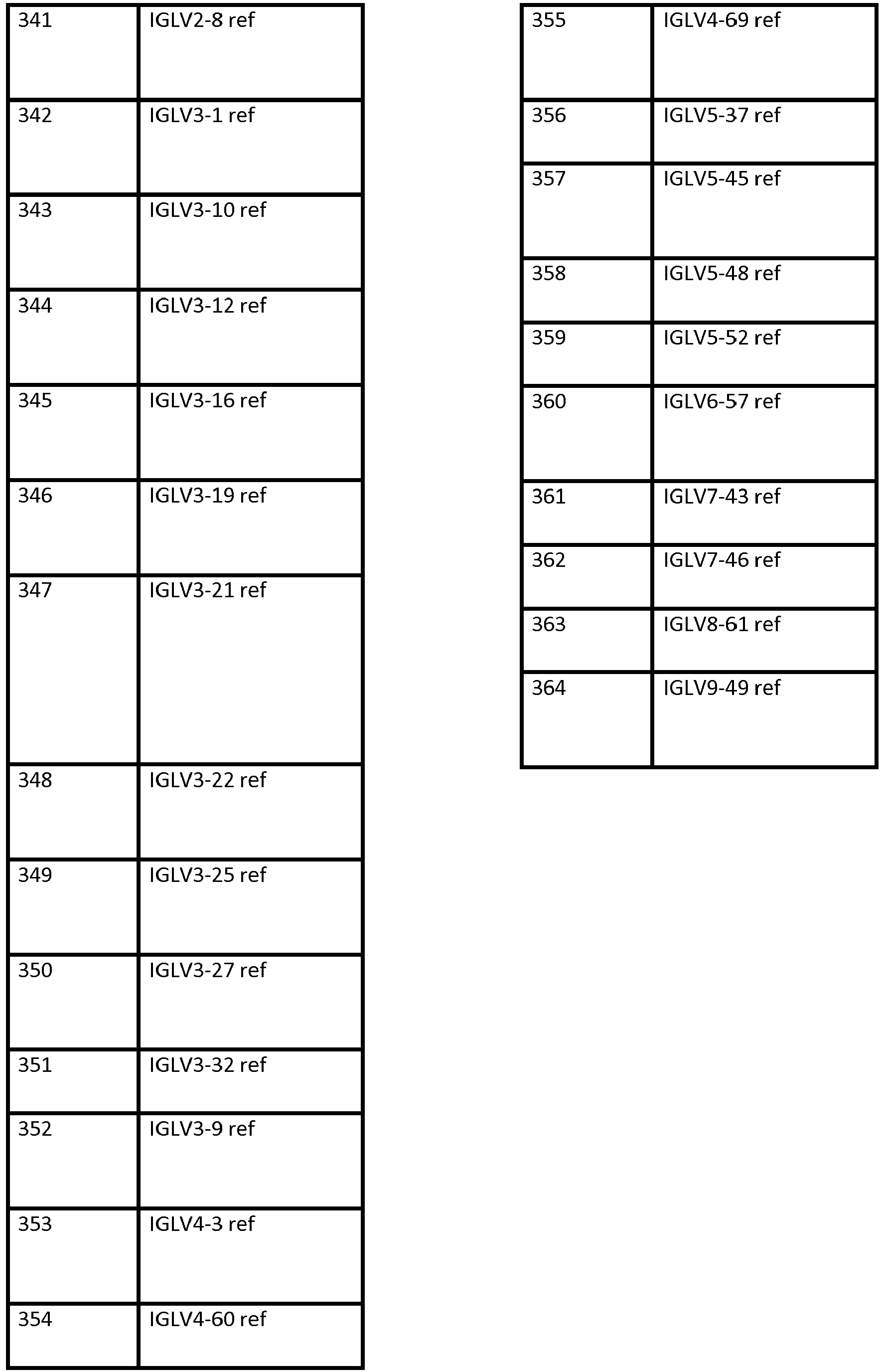

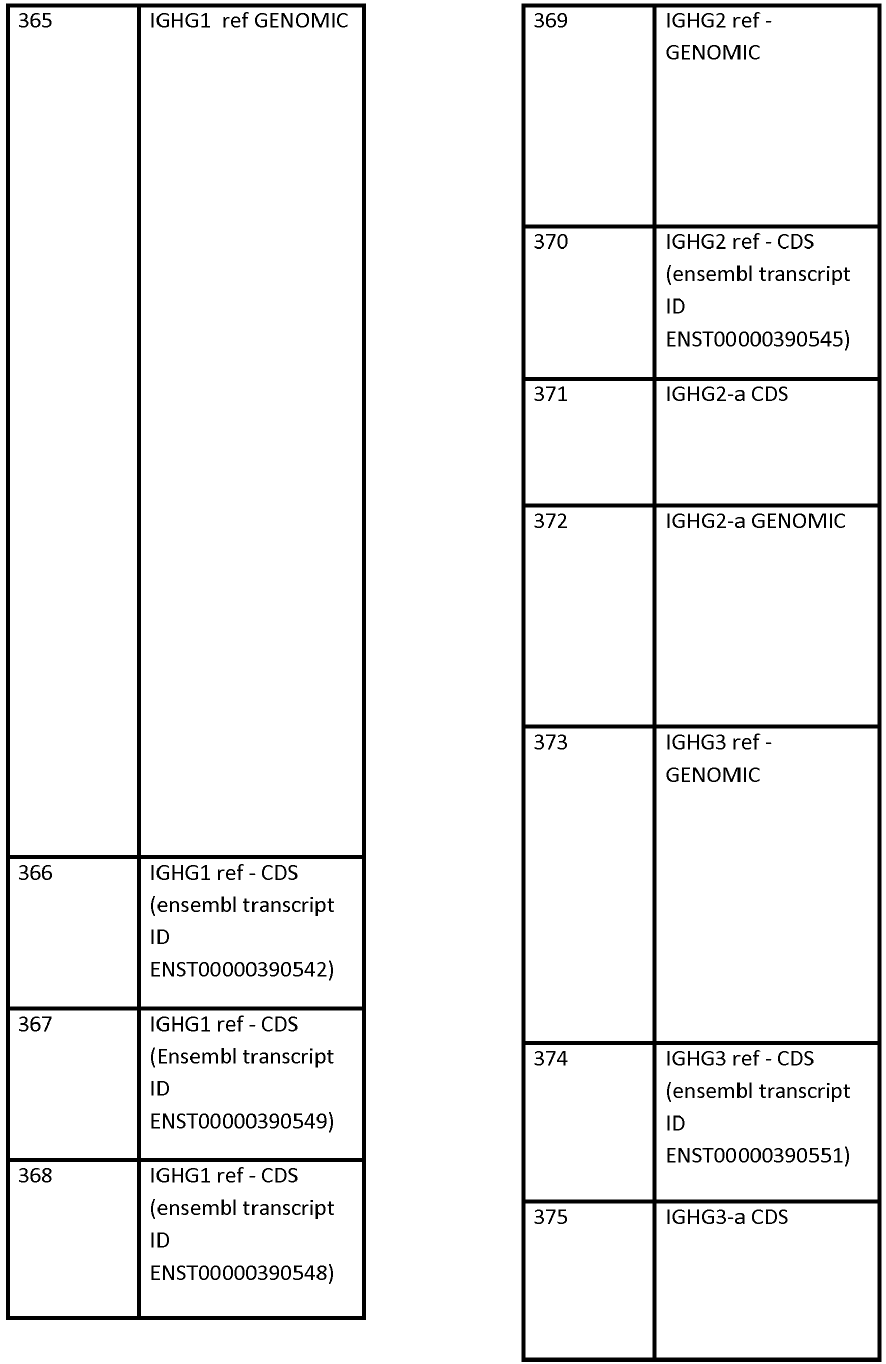

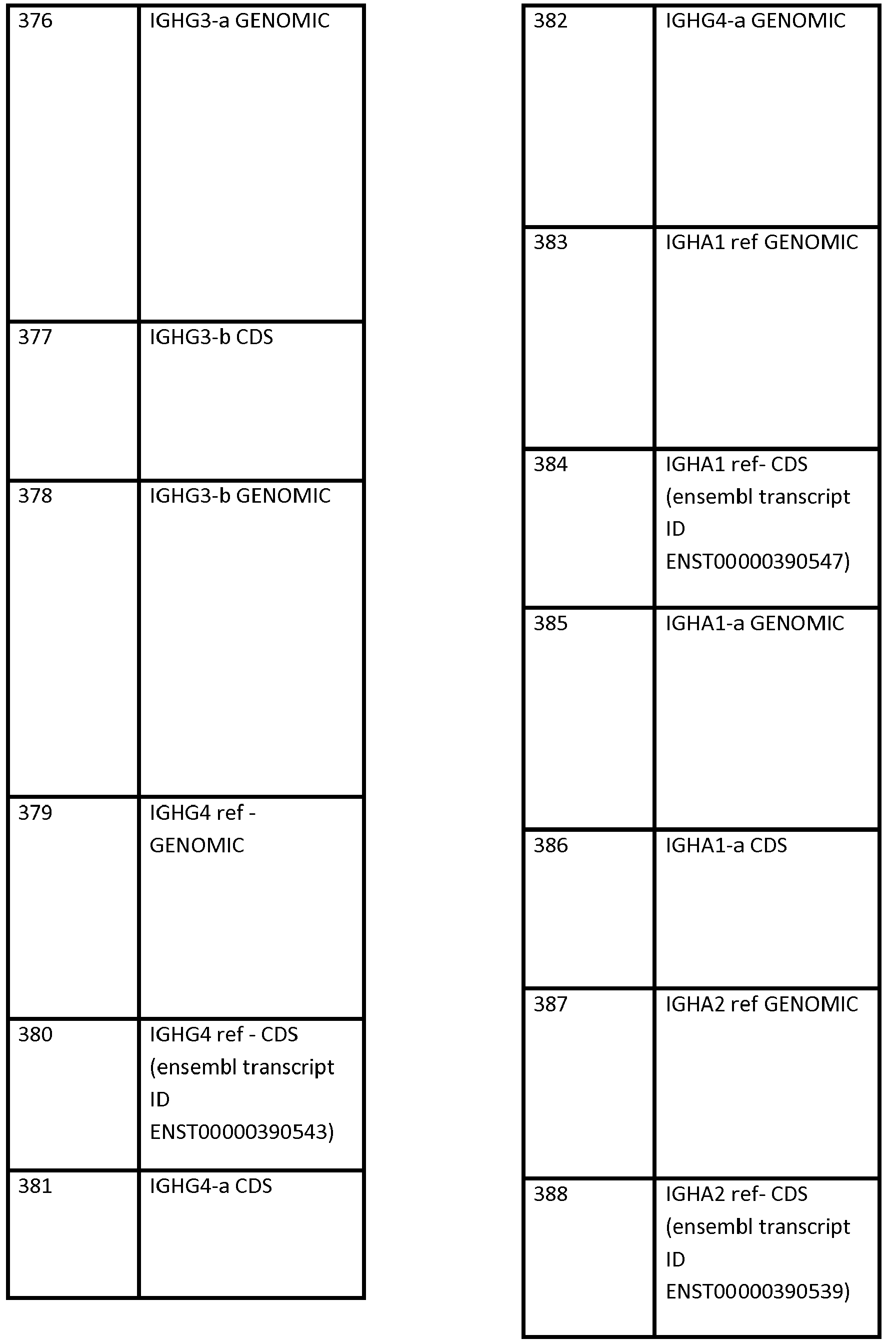

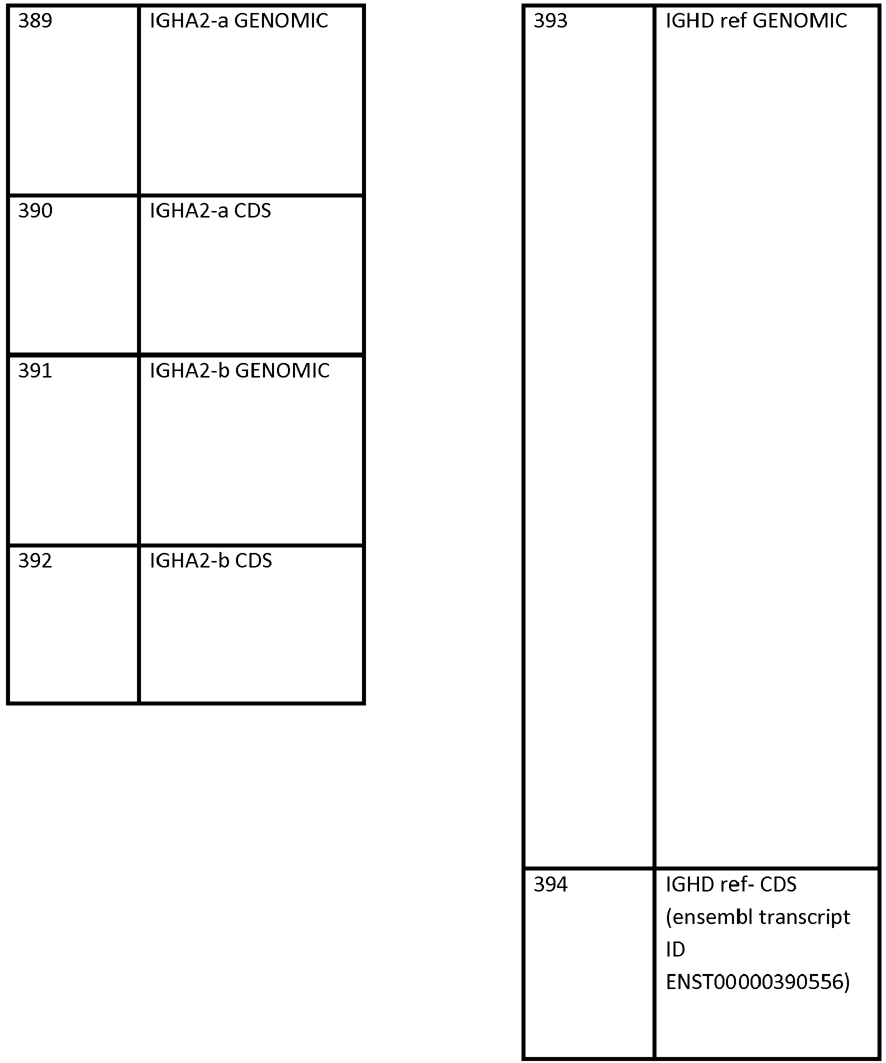

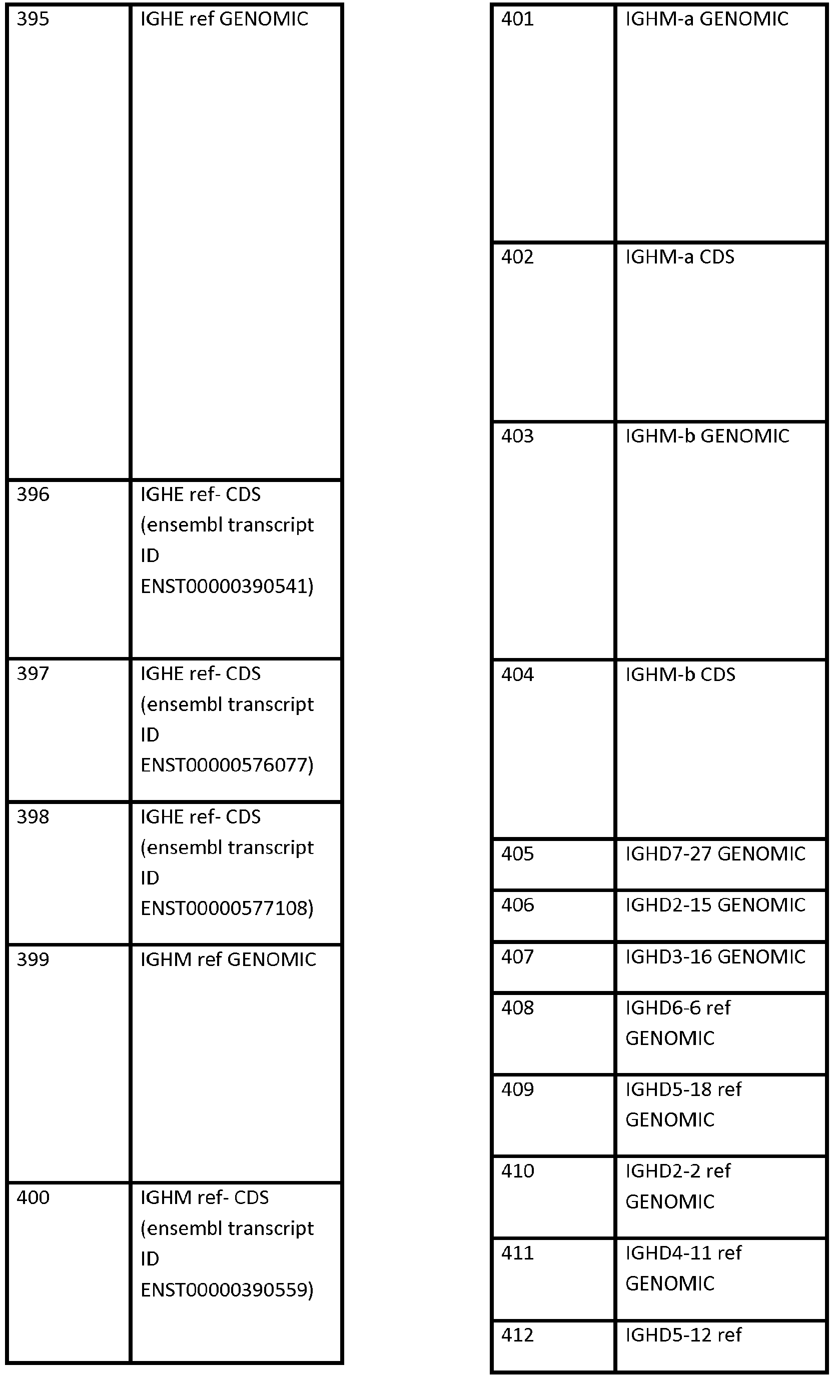

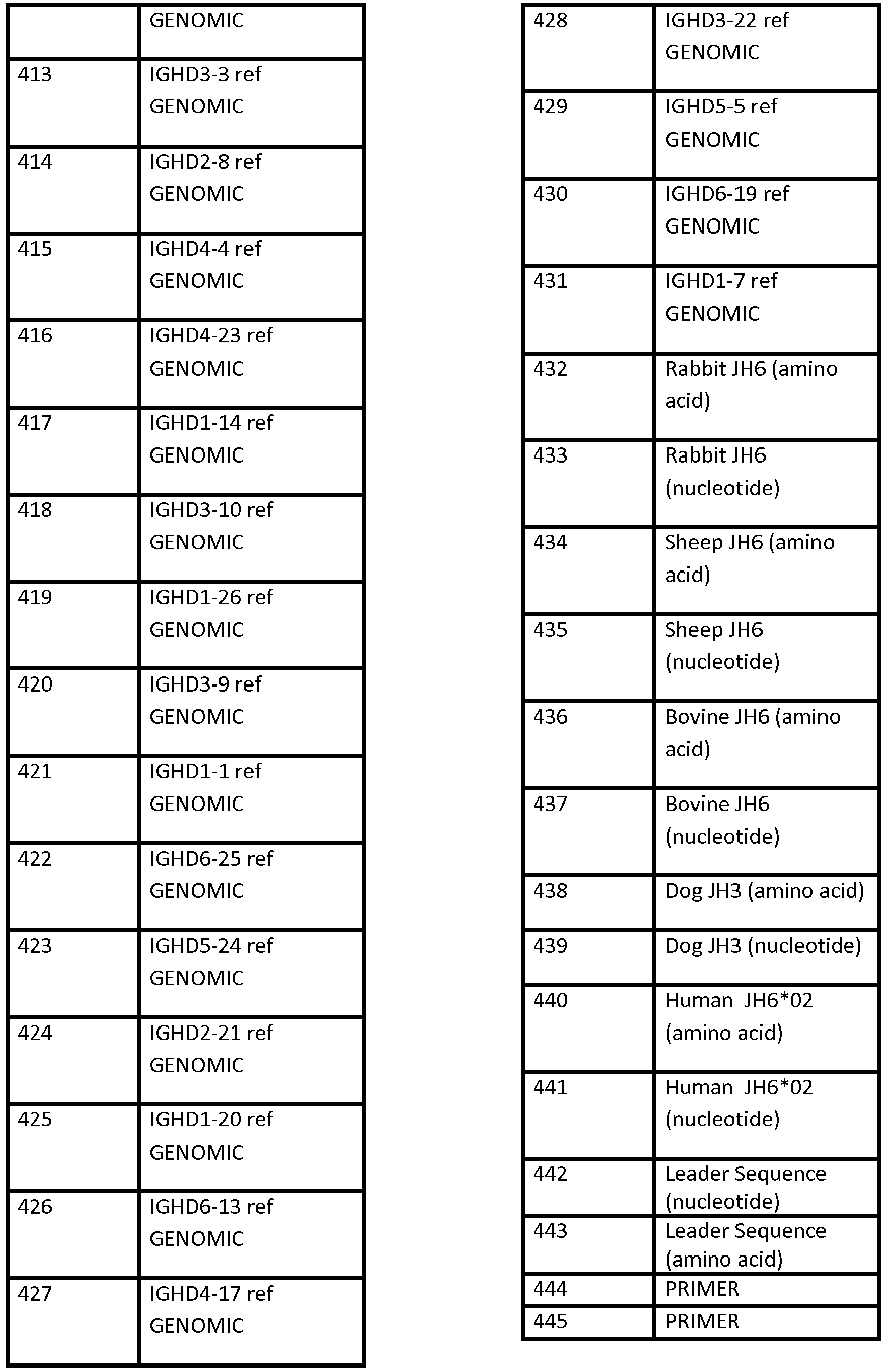

**Table 19**

| | IgG1 | IgG2 | IgG3 | IgG4 |
|---|---|---|---|---|
| Complement activation | | | | |
| Classical pathway | +++ | + | +++ | - |
| Alternative pathway | - | + | - | - |

| Fc receptor recognition | | | | |
|---|---|---|---|---|
| FcγRI | +++ | - | +++ | ++ |
| FcγRIIa, 131R/R | ++ | - | ++ | - |
| FcγRIIa, 131H/H | + | + | ++ | - |
| FcγRIIb | ++ | - | ++ | + |
| FcγRIII | + | +/- | + | +/- |

### Table 20

**Table 20A: Variants Found In No More Than 3 Human Populations**

| **TYPE** | **VARIANT** | **C.FREQ** | **C.FREQ (%)** | **#INDIVIDUALS** | **#POPULATIONS** |
|---|---|---|---|---|---|
| IGKV | IGKV1D-8-b | 0.01785 | 1.79% | 36 | 3 |
| IGHV | IGHV3-11-a | 0.01695 | 1.70% | 36 | 3 |
| IGHV | IGHV5-51-a | 0.0164 | 1.64% | 35 | 3 |
| IGHV | IGHV2-5-b | 0.01055 | 1.06% | 18 | 3 |
| IGLV | IGLV2-14-g | 0.0104 | 1.04% | 22 | 3 |
| IGHV | IGHV7-81-b | 0.01005 | 1.01% | 21 | 3 |
| IGKV | IGKV1D-42-b | 0.01005 | 1.01% | 21 | 3 |
| IGKV | IGKV3-7-b | 0.01 | 1.00% | 19 | 3 |
| IGHV | IGHV1-69-d | 0.00995 | 1.00% | 22 | 3 |
| IGLV | IGLV2-14-e | 0.00995 | 1.00% | 19 | 3 |
| IGLV | IGLV9-49-a | 0.00865 | 0.87% | 18 | 3 |
| IGLV | IGLV1-44-b | 0.00775 | 0.78% | 16 | 2 |
| IGLV | IGLV2-11-a | 0.00775 | 0.78% | 17 | 3 |
| IGKV | IGKV3-11-d | 0.0073 | 0.73% | 14 | 3 |
| IGKV | IGKV1D-43-b | 0.00685 | 0.69% | 14 | 3 |
| IGHV | IGHV3-38-d | 0.0064 | 0.64% | 13 | 3 |
| IGU | IGU6-a | 0.00595 | 0.60% | 13 | 3 |
| IGHV | IGHV5-51-b | 0.0055 | 0.55% | 11 | 3 |
| IGLV | IGLV5-45-c | 0.00545 | 0.55% | 12 | 2 |
| IGHV | IGHV4-31-i | 0.0054 | 0.54% | 10 | 3 |
| IGKV | IGKV2D-26-b | 0.00455 | 0.46% | 9 | 3 |
| IGLV | IGLV1-36-c | 0.00455 | 0.46% | 10 | 2 |
| IGHV | IGHV1-24-c | 0.00415 | 0.42% | 9 | 3 |
| IGHV | IGHV1-8-c | 0.00405 | 0.41% | 8 | 2 |
| IGLV | IGLV1-47-d | 0.00405 | 0.41% | 9 | 3 |
| IGHV | IGHV3-43-i | 0.00365 | 0.37% | 8 | 3 |
| IGHV | IGHV3-53-h | 0.0036 | 0.36% | 8 | 2 |
| IGLV | IGLV10-54-e | 0.0036 | 0.36% | 8 | 3 |
| IGLV | IGLV1-36-f | 0.0036 | 0.36% | 8 | 2 |
| IGLV | IGLV5-45-d | 0.0036 | 0.36% | 8 | 3 |
| IGHV | IGHV1-46-b | 0.0032 | 0.32% | 7 | 3 |
| IGHV | IGHV2-26-c | 0.0032 | 0.32% | 7 | 2 |
| IGU | IGU6-b | 0.0032 | 0.32% | 7 | 3 |
| IGLV | IGLV3-32-a | 0.0032 | 0.32% | 7 | 3 |
| IGLV | IGLV9-49-b | 0.0032 | 0.32% | 7 | 2 |
| IGHJ | IGHJ6-b | 0.00315 | 0.32% | 7 | 2 |
| IGHV | IGHV3-16-d | 0.00315 | 0.32% | 7 | 3 |
| IGHV | IGHV4-28-k | 0.00315 | 0.32% | 7 | 3 |
| IGKJ | IGKJ2-d | 0.00315 | 0.32% | 6 | 3 |
| IGLV | IGLV10-54-f | 0.00315 | 0.32% | 7 | 2 |
| IGLV | IGLV2-8-c | 0.00315 | 0.32% | 7 | 3 |
| IGLV | IGLV3-10-c | 0.00315 | 0.32% | 6 | 2 |
| IGLV | IGLV1-40-c | 0.00275 | 0.28% | 6 | 2 |
| IGLV | IGLV1-51-b | 0.00275 | 0.28% | 6 | 3 |
| IGHV | IGHV1-69-m | 0.0027 | 0.27% | 6 | 3 |
| IGHV | IGHV3-33-e | 0.0027 | 0.27% | 6 | 3 |
| IGHV | IGHV3-49-c | 0.0027 | 0.27% | 6 | 3 |
| IGHV | IGHV3-66-g | 0.0027 | 0.27% | 6 | 3 |
| IGHV | IGHV4-4-u | 0.0027 | 0.27% | 6 | 3 |
| IGKV | IGKV1D-42-c | 0.0027 | 0.27% | 6 | 1 |
| IGKV | IGKV1D-43-c | 0.0027 | 0.27% | 5 | 3 |
| IGLV | IGLV10-54-h | 0.0027 | 0.27% | 6 | 2 |
| IGLV | IGLV10-54-i | 0.0027 | 0.27% | 6 | 2 |
| IGLV | IGLV1-36-g | 0.0027 | 0.27% | 6 | 3 |
| IGLV | IGLV2-8-d | 0.0027 | 0.27% | 6 | 3 |
| IGLV | IGLV3-22-e | 0.0027 | 0.27% | 6 | 1 |
| IGLV | IGLV3-22-d | 0.0027 | 0.27% | 6 | 3 |
| IGHJ | IGHJ2-b | 0.00225 | 0.23% | 4 | 3 |
| IGHV | IGHV1-3-u | 0.00225 | 0.23% | 5 | 2 |
| IGHV | IGHV1-3-v | 0.00225 | 0.23% | 5 | 2 |
| IGHV | IGHV1-3-ap | 0.00225 | 0.23% | 4 | 3 |
| IGHV | IGHV1-46-c | 0.00225 | 0.23% | 5 | 3 |
| IGHV | IGHV1-58-f | 0.00225 | 0.23% | 5 | 3 |
| IGHV | IGHV1-69-j | 0.00225 | 0.23% | 5 | 3 |
| IGHV | IGHV3-13-e | 0.00225 | 0.23% | 5 | 3 |
| IGHV | IGHV3-23-f | 0.00225 | 0.23% | 5 | 3 |
| IGHV | IGHV3-23-g | 0.00225 | 0.23% | 5 | 3 |
| IGHV | IGHV4-28-p | 0.00225 | 0.23% | 5 | 3 |
| IGHV | IGHV4-31-j | 0.00225 | 0.23% | 5 | 3 |
| IGKV | IGKV1-17-d | 0.00225 | 0.23% | 4 | 1 |
| IGKV | IGKV3-20-a | 0.00225 | 0.23% | 5 | 3 |
| IGKV | IGKV3-7-c | 0.00225 | 0.23% | 5 | 3 |
| IGKV | IGKV3-7-d | 0.00225 | 0.23% | 5 | 3 |
| IGKV | IGKV3D-11-b | 0.00225 | 0.23% | 5 | 3 |
| IGU | IGU3-a | 0.00225 | 0.23% | 4 | 2 |
| IGLV | IGLV11-55-g | 0.00225 | 0.23% | 5 | 3 |
| IGLV | IGLV1-50-c | 0.00225 | 0.23% | 5 | 3 |
| IGLV | IGLV2-11-g | 0.00225 | 0.23% | 5 | 3 |
| IGLV | IGLV2-14-n | 0.00225 | 0.23% | 5 | 3 |
| IGLV | IGLV2-23-f | 0.00225 | 0.23% | 5 | 2 |
| IGLV | IGLV3-19-c | 0.00225 | 0.23% | 5 | 2 |
| IGLV | IGLV5-37-c | 0.00225 | 0.23% | 4 | 2 |
| IGLV | IGLV5-48-k | 0.00225 | 0.23% | 5 | 3 |
| IGLV | IGLV2-14-i | 0.00185 | 0.19% | 4 | 2 |
| IGHJ | IGHJ1-a | 0.0018 | 0.18% | 3 | 3 |
| IGHV | IGHV1-2-g | 0.0018 | 0.18% | 4 | 3 |
| IGHV | IGHV1-3-y | 0.0018 | 0.18% | 4 | 3 |
| IGHV | IGHV1-3-ar | 0.0018 | 0.18% | 4 | 3 |
| IGHV | IGHV1-3-ai | 0.0018 | 0.18% | 4 | 3 |
| IGHV | IGHV1-69-s | 0.0018 | 0.18% | 4 | 3 |
| IGHV | IGHV1-8-d | 0.0018 | 0.18% | 4 | 2 |
| IGHV | IGHV2-5-d | 0.0018 | 0.18% | 4 | 2 |
| IGHV | IGHV3-13-j | 0.0018 | 0.18% | 4 | 2 |
| IGHV | IGHV3-21-d | 0.0018 | 0.18% | 4 | 3 |
| IGHV | IGHV3-23-n | 0.0018 | 0.18% | 4 | 3 |
| IGHV | IGHV3-33-d | 0.0018 | 0.18% | 4 | 3 |
| IGHV | IGHV3-64-e | 0.0018 | 0.18% | 4 | 2 |
| IGHV | IGHV3-9-g | 0.0018 | 0.18% | 4 | 3 |
| IGHV | IGHV4-39-y | 0.0018 | 0.18% | 4 | 3 |
| IGHV | IGHV4-39-n | 0.0018 | 0.18% | 4 | 3 |
| IGHV | IGHV4-39-o | 0.0018 | 0.18% | 4 | 3 |
| IGHV | IGHV7-81-c | 0.0018 | 0.18% | 4 | 3 |
| IGKJ | IGKJ2-e | 0.0018 | 0.18% | 4 | 3 |
| IGKV | IGKV2D-26-c | 0.0018 | 0.18% | 4 | 1 |
| IGKV | IGKV3D-11-g | 0.0018 | 0.18% | 3 | 2 |
| IGKV | IGKV3D-11-f | 0.0018 | 0.18% | 4 | 3 |
| IGKV | IGKV4-1-w | 0.0018 | 0.18% | 3 | 3 |
| IGU | IGU1-a | 0.0018 | 0.18% | 4 | 2 |
| IGLV | IGLV10-54-k | 0.0018 | 0.18% | 4 | 2 |
| IGLV | IGLV11-55-e | 0.0018 | 0.18% | 4 | 3 |
| IGLV | IGLV1-36-e | 0.0018 | 0.18% | 4 | 3 |
| IGLV | IGLV1-40-f | 0.0018 | 0.18% | 4 | 3 |
| IGLV | IGLV1-44-d | 0.0018 | 0.18% | 4 | 3 |
| IGLV | IGLV1-44-e | 0.0018 | 0.18% | 4 | 3 |
| IGLV | IGLV1-47-g | 0.0018 | 0.18% | 4 | 2 |
| IGLV | IGLV1-50-d | 0.0018 | 0.18% | 4 | 2 |
| IGLV | IGLV1-51-d | 0.0018 | 0.18% | 4 | 2 |
| IGLV | IGLV1-51-c | 0.0018 | 0.18% | 4 | 3 |
| IGLV | IGLV2-14-r | 0.0018 | 0.18% | 4 | 1 |
| IGLV | IGLV2-33-b | 0.0018 | 0.18% | 4 | 3 |
| IGLV | IGLV2-8-e | 0.0018 | 0.18% | 4 | 2 |
| IGLV | IGLV3-16-h | 0.0018 | 0.18% | 4 | 3 |
| IGLV | IGLV3-21-j | 0.0018 | 0.18% | 4 | 3 |
| IGLV | IGLV3-22-g | 0.0018 | 0.18% | 4 | 3 |
| IGLV | IGLV3-22-h | 0.0018 | 0.18% | 4 | 2 |
| IGLV | IGLV5-48-j | 0.0018 | 0.18% | 4 | 3 |
| IGHJ | IGHJ4-d | 0.00135 | 0.14% | 3 | 3 |
| IGHV | IGHV1-18-d | 0.00135 | 0.14% | 3 | 3 |
| IGHV | IGHV1-18-e | 0.00135 | 0.14% | 3 | 3 |
| IGHV | IGHV1-2-h | 0.00135 | 0.14% | 3 | 2 |
| IGHV | IGHV1-2-f | 0.00135 | 0.14% | 3 | 3 |
| IGHV | IGHV1-3-aa | 0.00135 | 0.14% | 3 | 1 |
| IGHV | IGHV1-3-ak | 0.00135 | 0.14% | 3 | 3 |
| IGHV | IGHV1-3-w | 0.00135 | 0.14% | 3 | 1 |
| IGHV | IGHV1-3-at | 0.00135 | 0.14% | 3 | 2 |
| IGHV | IGHV1-69-k | 0.00135 | 0.14% | 3 | 1 |
| IGHV | IGHV1-69-ac | 0.00135 | 0.14% | 3 | 3 |
| IGHV | IGHV1-8-e | 0.00135 | 0.14% | 3 | 2 |
| IGHV | IGHV1-8-f | 0.00135 | 0.14% | 3 | 2 |
| IGHV | IGHV2-70-y | 0.00135 | 0.14% | 3 | 3 |
| IGHV | IGHV2-70-z | 0.00135 | 0.14% | 3 | 3 |
| IGHV | IGHV2-70-af | 0.00135 | 0.14% | 3 | 2 |
| IGHV | IGHV2-70-h | 0.00135 | 0.14% | 3 | 3 |
| IGHV | IGHV3-13-g | 0.00135 | 0.14% | 3 | 2 |
| IGHV | IGHV3-16-e | 0.00135 | 0.14% | 3 | 2 |
| IGHV | IGHV3-20-f | 0.00135 | 0.14% | 3 | 3 |
| IGHV | IGHV3-30-d | 0.00135 | 0.14% | 3 | 2 |
| IGHV | IGHV3-30-c | 0.00135 | 0.14% | 3 | 3 |
| IGHV | IGHV3-33-h | 0.00135 | 0.14% | 3 | 3 |
| IGHV | IGHV3-43-k | 0.00135 | 0.14% | 3 | 2 |
| IGHV | IGHV3-43-m | 0.00135 | 0.14% | 3 | 3 |
| IGHV | IGHV3-53-n | 0.00135 | 0.14% | 3 | 2 |
| IGHV | IGHV3-53-m | 0.00135 | 0.14% | 3 | 3 |
| IGHV | IGHV3-64-f | 0.00135 | 0.14% | 3 | 1 |
| IGHV | IGHV3-73-c | 0.00135 | 0.14% | 3 | 3 |
| IGHV | IGHV3-9-h | 0.00135 | 0.14% | 2 | 1 |
| IGHV | IGHV4-31-h | 0.00135 | 0.14% | 3 | 2 |
| IGHV | IGHV4-39-u | 0.00135 | 0.14% | 3 | 3 |
| IGHV | IGHV4-39-t | 0.00135 | 0.14% | 3 | 2 |
| IGHV | IGHV4-39-q | 0.00135 | 0.14% | 3 | 3 |
| IGHV | IGHV4-4-ao | 0.00135 | 0.14% | 3 | 3 |
| IGHV | IGHV4-4-ac | 0.00135 | 0.14% | 3 | 1 |
| IGHV | IGHV4-4-ae | 0.00135 | 0.14% | 3 | 2 |
| IGHV | IGHV5-51-g | 0.00135 | 0.14% | 3 | 3 |
| IGHV | IGHV7-81-e | 0.00135 | 0.14% | 3 | 1 |
| IGKJ | IGKJ2-f | 0.00135 | 0.14% | 3 | 2 |
| IGKJ | IGKJ3-d | 0.00135 | 0.14% | 3 | 3 |
| IGKJ | IGKJ4-g | 0.00135 | 0.14% | 3 | 3 |
| IGKJ | IGKJ5-c | 0.00135 | 0.14% | 3 | 3 |
| IGKJ | IGKJ5-b | 0.00135 | 0.14% | 3 | 3 |
| IGKV | IGKV1-5-c | 0.00135 | 0.14% | 3 | 3 |
| IGKV | IGKV1-9-j | 0.00135 | 0.14% | 3 | 3 |
| IGKV | IGKV1D-17-c | 0.00135 | 0.14% | 3 | 2 |
| IGKV | IGKV1D-17-b | 0.00135 | 0.14% | 3 | 2 |
| IGKV | IGKV1D-42-e | 0.00135 | 0.14% | 3 | 1 |
| IGKV | IGKV1D-42-d | 0.00135 | 0.14% | 3 | 2 |
| IGKV | IGKV2D-24-b | 0.00135 | 0.14% | 3 | 2 |
| IGKV | IGKV2D-30-c | 0.00135 | 0.14% | 3 | 2 |
| IGKV | IGKV3-20-d | 0.00135 | 0.14% | 3 | 3 |
| IGKV | IGKV3-20-f | 0.00135 | 0.14% | 3 | 2 |
| IGKV | IGKV3D-11-d | 0.00135 | 0.14% | 3 | 2 |
| IGKV | IGKV4-1-r | 0.00135 | 0.14% | 3 | 3 |
| IGKV | IGKV4-1-x | 0.00135 | 0.14% | 3 | 2 |
| IGKV | IGKV4-1-e | 0.00135 | 0.14% | 3 | 3 |
| IGKV | IGKV4-1-o | 0.00135 | 0.14% | 3 | 2 |
| IGU | IGU6-c | 0.00135 | 0.14% | 3 | 2 |
| IGU | IGU7-b | 0.00135 | 0.14% | 3 | 3 |
| IGLV | IGLV10-54-g | 0.00135 | 0.14% | 3 | 1 |
| IGLV | IGLV11-55-h | 0.00135 | 0.14% | 3 | 2 |
| IGLV | IGLV11-55-f | 0.00135 | 0.14% | 3 | 2 |
| IGLV | IGLV1-40-g | 0.00135 | 0.14% | 3 | 3 |
| IGLV | IGLV1-44-f | 0.00135 | 0.14% | 3 | 2 |
| IGLV | IGLV1-47-f | 0.00135 | 0.14% | 3 | 2 |
| IGLV | IGLV2-23-e | 0.00135 | 0.14% | 3 | 3 |
| IGLV | IGLV3-12-d | 0.00135 | 0.14% | 3 | 3 |
| IGLV | IGLV3-16-d | 0.00135 | 0.14% | 3 | 3 |
| IGLV | IGLV3-19-d | 0.00135 | 0.14% | 3 | 1 |
| IGLV | IGLV3-19-l | 0.00135 | 0.14% | 3 | 2 |
| IGLV | IGLV3-1-ag | 0.00135 | 0.14% | 3 | 3 |
| IGLV | IGLV3-1-y | 0.00135 | 0.14% | 3 | 3 |
| IGLV | IGLV3-1-s | 0.00135 | 0.14% | 3 | 3 |
| IGLV | IGLV3-1-x | 0.00135 | 0.14% | 3 | 3 |
| IGLV | IGLV3-1-ad | 0.00135 | 0.14% | 3 | 2 |
| IGLV | IGLV3-1-l | 0.00135 | 0.14% | 3 | 3 |
| IGLV | IGLV3-1-p | 0.00135 | 0.14% | 3 | 2 |
| IGLV | IGLV3-21-t | 0.00135 | 0.14% | 3 | 2 |
| IGLV | IGLV3-22-f | 0.00135 | 0.14% | 3 | 3 |
| IGLV | IGLV3-22-i | 0.00135 | 0.14% | 3 | 1 |
| IGLV | IGLV3-25-e | 0.00135 | 0.14% | 3 | 3 |
| IGLV | IGLV5-48-t | 0.00135 | 0.14% | 3 | 2 |
| IGLV | IGLV5-48-s | 0.00135 | 0.14% | 3 | 3 |
| IGLV | IGLV5-52-d | 0.00135 | 0.14% | 3 | 2 |
| IGLV | IGLV5-52-c | 0.00135 | 0.14% | 3 | 1 |
| IGLV | IGLV7-43-c | 0.00135 | 0.14% | 3 | 2 |
| IGHJ | IGHJ5-d | 0.0009 | 0.09% | 2 | 2 |
| IGHJ | IGHJ6-e | 0.0009 | 0.09% | 2 | 2 |
| IGHJ | IGHJ6-h | 0.0009 | 0.09% | 2 | 2 |
| IGHJ | IGHJ6-f | 0.0009 | 0.09% | 2 | 2 |
| IGHV | IGHV1-18-f | 0.0009 | 0.09% | 2 | 2 |
| IGHV | IGHV1-24-g | 0.0009 | 0.09% | 2 | 1 |
| IGHV | IGHV1-2-i | 0.0009 | 0.09% | 2 | 2 |
| IGHV | IGHV1-2-m | 0.0009 | 0.09% | 2 | 2 |
| IGHV | IGHV1-2-l | 0.0009 | 0.09% | 2 | 2 |
| IGHV | IGHV1-3-ag | 0.0009 | 0.09% | 2 | 2 |
| IGHV | IGHV1-3-aj | 0.0009 | 0.09% | 2 | 2 |
| IGHV | IGHV1-3-aq | 0.0009 | 0.09% | 2 | 1 |
| IGHV | IGHV1-3-s | 0.0009 | 0.09% | 2 | 2 |
| IGHV | IGHV1-3-q | 0.0009 | 0.09% | 2 | 1 |
| IGHV | IGHV1-3-z | 0.0009 | 0.09% | 2 | 1 |
| IGHV | IGHV1-3-ad | 0.0009 | 0.09% | 2 | 2 |
| IGHV | IGHV1-3-aw | 0.0009 | 0.09% | 2 | 2 |
| IGHV | IGHV1-3-ae | 0.0009 | 0.09% | 2 | 2 |
| IGHV | IGHV1-3-an | 0.0009 | 0.09% | 2 | 2 |
| IGHV | IGHV1-46-k | 0.0009 | 0.09% | 2 | 2 |
| IGHV | IGHV1-46-e | 0.0009 | 0.09% | 2 | 2 |
| IGHV | IGHV1-46-n | 0.0009 | 0.09% | 2 | 2 |
| IGHV | IGHV1-58-g | 0.0009 | 0.09% | 2 | 1 |
| IGHV | IGHV1-69-ah | 0.0009 | 0.09% | 2 | 2 |
| IGHV | IGHV1-69-t | 0.0009 | 0.09% | 2 | 2 |
| IGHV | IGHV1-69-ao | 0.0009 | 0.09% | 2 | 1 |
| IGHV | IGHV1-69-af | 0.0009 | 0.09% | 2 | 2 |
| IGHV | IGHV1-69-ad | 0.0009 | 0.09% | 2 | 2 |
| IGHV | IGHV1-69-n | 0.0009 | 0.09% | 2 | 2 |
| IGHV | IGHV1-69-an | 0.0009 | 0.09% | 2 | 2 |
| IGHV | IGHV1-69-o | 0.0009 | 0.09% | 2 | 2 |
| IGHV | IGHV1-8-h | 0.0009 | 0.09% | 2 | 2 |
| IGHV | IGHV2-26-f | 0.0009 | 0.09% | 2 | 2 |
| IGHV | IGHV2-5-c | 0.0009 | 0.09% | 2 | 2 |
| IGHV | IGHV2-70-g | 0.0009 | 0.09% | 2 | 2 |
| IGHV | IGHV2-70-s | 0.0009 | 0.09% | 2 | 2 |
| IGHV | IGHV2-70-ab | 0.0009 | 0.09% | 2 | 2 |
| IGHV | IGHV3-11-b | 0.0009 | 0.09% | 2 | 2 |
| IGHV | IGHV3-13-f | 0.0009 | 0.09% | 2 | 2 |
| IGHV | IGHV3-20-d | 0.0009 | 0.09% | 2 | 2 |
| IGHV | IGHV3-21-c | 0.0009 | 0.09% | 2 | 2 |
| IGHV | IGHV3-21-h | 0.0009 | 0.09% | 2 | 2 |
| IGHV | IGHV3-23-j | 0.0009 | 0.09% | 2 | 1 |
| IGHV | IGHV3-23-h | 0.0009 | 0.09% | 2 | 2 |
| IGHV | IGHV3-33-f | 0.0009 | 0.09% | 2 | 2 |
| IGHV | IGHV3-43-p | 0.0009 | 0.09% | 2 | 2 |
| IGHV | IGHV3-64-h | 0.0009 | 0.09% | 2 | 1 |
| IGHV | IGHV3-66-h | 0.0009 | 0.09% | 2 | 2 |
| IGHV | IGHV3-74-c | 0.0009 | 0.09% | 2 | 2 |
| IGHV | IGHV3-9-o | 0.0009 | 0.09% | 2 | 1 |
| IGHV | IGHV4-28-s | 0.0009 | 0.09% | 2 | 2 |
| IGHV | IGHV4-28-n | 0.0009 | 0.09% | 2 | 2 |
| IGHV | IGHV4-31-t | 0.0009 | 0.09% | 2 | 2 |
| IGHV | IGHV4-31-m | 0.0009 | 0.09% | 2 | 2 |
| IGHV | IGHV4-31-l | 0.0009 | 0.09% | 2 | 2 |
| IGHV | IGHV4-34-d | 0.0009 | 0.09% | 2 | 2 |
| IGHV | IGHV4-34-g | 0.0009 | 0.09% | 2 | 2 |
| IGHV | IGHV4-34-b | 0.0009 | 0.09% | 2 | 2 |
| IGHV | IGHV4-39-aa | 0.0009 | 0.09% | 2 | 2 |
| IGHV | IGHV4-39-s | 0.0009 | 0.09% | 2 | 2 |
| IGHV | IGHV4-39-x | 0.0009 | 0.09% | 2 | 2 |
| IGHV | IGHV4-39-p | 0.0009 | 0.09% | 2 | 2 |
| IGHV | IGHV4-4-aa | 0.0009 | 0.09% | 2 | 2 |
| IGHV | IGHV4-4-af | 0.0009 | 0.09% | 2 | 2 |
| IGHV | IGHV4-4-x | 0.0009 | 0.09% | 2 | 2 |
| IGHV | IGHV4-4-ad | 0.0009 | 0.09% | 2 | 2 |
| IGHV | IGHV4-4-ai | 0.0009 | 0.09% | 2 | 1 |
| IGHV | IGHV5-51-d | 0.0009 | 0.09% | 2 | 2 |
| IGHV | IGHV5-51-k | 0.0009 | 0.09% | 2 | 2 |
| IGHV | IGHV5-51-c | 0.0009 | 0.09% | 2 | 2 |
| IGHV | IGHV6-1-a | 0.0009 | 0.09% | 2 | 2 |
| IGHV | IGHV6-1-c | 0.0009 | 0.09% | 2 | 1 |
| IGHV | IGHV7-81-d | 0.0009 | 0.09% | 2 | 2 |
| IGKJ | IGKJ2-g | 0.0009 | 0.09% | 2 | 2 |
| IGKJ | IGKJ3-b | 0.0009 | 0.09% | 2 | 2 |
| IGKJ | IGKJ4-j | 0.0009 | 0.09% | 2 | 1 |
| IGKJ | IGKJ4-c | 0.0009 | 0.09% | 2 | 2 |
| IGKJ | IGKJ4-k | 0.0009 | 0.09% | 2 | 2 |
| IGKJ | IGKJ4-h | 0.0009 | 0.09% | 2 | 2 |
| IGKV | IGKV1-16-d | 0.0009 | 0.09% | 2 | 2 |
| IGKV | IGKV1-17-e | 0.0009 | 0.09% | 2 | 2 |
| IGKV | IGKV1-5-d | 0.0009 | 0.09% | 2 | 1 |
| IGKV | IGKV1-8-h | 0.0009 | 0.09% | 2 | 2 |
| IGKV | IGKV1-9-i | 0.0009 | 0.09% | 2 | 2 |
| IGKV | IGKV1D-16-c | 0.0009 | 0.09% | 2 | 1 |
| IGKV | IGKV1D-16-b | 0.0009 | 0.09% | 2 | 1 |
| IGKV | IGKV1D-42-g | 0.0009 | 0.09% | 2 | 2 |
| IGKV | IGKV1D-42-f | 0.0009 | 0.09% | 2 | 1 |
| IGKV | IGKV1D-43-e | 0.0009 | 0.09% | 2 | 1 |
| IGKV | IGKV1D-43-d | 0.0009 | 0.09% | 2 | 2 |
| IGKV | IGKV1D-8-d | 0.0009 | 0.09% | 2 | 1 |
| IGKV | IGKV1D-8-f | 0.0009 | 0.09% | 2 | 2 |
| IGKV | IGKV2D-26-d | 0.0009 | 0.09% | 2 | 2 |
| IGKV | IGKV2D-26-f | 0.0009 | 0.09% | 2 | 2 |
| IGKV | IGKV2D-29-b | 0.0009 | 0.09% | 2 | 1 |
| IGKV | IGKV3-11-g | 0.0009 | 0.09% | 2 | 1 |
| IGKV | IGKV3-11-f | 0.0009 | 0.09% | 2 | 2 |
| IGKV | IGKV3-20-j | 0.0009 | 0.09% | 2 | 2 |
| IGKV | IGKV3-20-i | 0.0009 | 0.09% | 2 | 2 |
| IGKV | IGKV3-20-e | 0.0009 | 0.09% | 2 | 2 |
| IGKV | IGKV3-20-c | 0.0009 | 0.09% | 2 | 2 |
| IGKV | IGKV3D-11-h | 0.0009 | 0.09% | 2 | 1 |
| IGKV | IGKV3D-11-i | 0.0009 | 0.09% | 2 | 1 |
| IGKV | IGKV3D-20-d | 0.0009 | 0.09% | 2 | 2 |
| IGKV | IGKV4-1-y | 0.0009 | 0.09% | 2 | 2 |
| IGKV | IGKV4-1-g | 0.0009 | 0.09% | 2 | 2 |
| IGKV | IGKV4-1-t | 0.0009 | 0.09% | 2 | 2 |
| IGKV | IGKV4-1-i | 0.0009 | 0.09% | 2 | 2 |
| IGKV | IGKV4-1-n | 0.0009 | 0.09% | 2 | 2 |
| IGKV | IGKV4-1-l | 0.0009 | 0.09% | 2 | 2 |
| IGU | IGU2-e | 0.0009 | 0.09% | 2 | 2 |
| IGU | IGU2-c | 0.0009 | 0.09% | 2 | 2 |
| IGU | IGU5-b | 0.0009 | 0.09% | 2 | 1 |
| IGLV | IGLV10-54-n | 0.0009 | 0.09% | 2 | 2 |
| IGLV | IGLV10-54-j | 0.0009 | 0.09% | 2 | 2 |
| IGLV | IGLV1-40-h | 0.0009 | 0.09% | 2 | 2 |
| IGLV | IGLV1-40-e | 0.0009 | 0.09% | 2 | 2 |
| IGLV | IGLV1-47-e | 0.0009 | 0.09% | 2 | 2 |
| IGLV | IGLV1-51-g | 0.0009 | 0.09% | 2 | 2 |
| IGLV | IGLV2-11-j | 0.0009 | 0.09% | 2 | 2 |
| IGLV | IGLV2-11-f | 0.0009 | 0.09% | 2 | 2 |
| IGLV | IGLV2-11-i | 0.0009 | 0.09% | 2 | 2 |
| IGLV | IGLV2-11-e | 0.0009 | 0.09% | 2 | 1 |
| IGLV | IGLV2-14-p | 0.0009 | 0.09% | 2 | 2 |
| IGLV | IGLV2-14-q | 0.0009 | 0.09% | 2 | 1 |
| IGLV | IGLV2-14-o | 0.0009 | 0.09% | 2 | 1 |
| IGLV | IGLV2-23-k | 0.0009 | 0.09% | 2 | 2 |
| IGLV | IGLV2-23-o | 0.0009 | 0.09% | 2 | 2 |
| IGLV | IGLV2-33-c | 0.0009 | 0.09% | 2 | 1 |
| IGLV | IGLV3-19-j | 0.0009 | 0.09% | 2 | 2 |
| IGLV | IGLV3-19-k | 0.0009 | 0.09% | 2 | 2 |
| IGLV | IGLV3-19-h | 0.0009 | 0.09% | 2 | 1 |
| IGLV | IGLV3-19-i | 0.0009 | 0.09% | 2 | 1 |
| IGLV | IGLV3-19-p | 0.0009 | 0.09% | 2 | 2 |
| IGLV | IGLV3-1-ah | 0.0009 | 0.09% | 2 | 2 |
| IGLV | IGLV3-1-d | 0.0009 | 0.09% | 2 | 2 |
| IGLV | IGLV3-1-j | 0.0009 | 0.09% | 2 | 1 |
| IGLV | IGLV3-1-g | 0.0009 | 0.09% | 2 | 2 |
| IGLV | IGLV3-1-z | 0.0009 | 0.09% | 2 | 2 |
| IGLV | IGLV3-1-ac | 0.0009 | 0.09% | 2 | 2 |
| IGLV | IGLV3-1-ae | 0.0009 | 0.09% | 2 | 2 |
| IGLV | IGLV3-21-k | 0.0009 | 0.09% | 2 | 2 |
| IGLV | IGLV3-21-r | 0.0009 | 0.09% | 2 | 1 |
| IGLV | IGLV3-21-i | 0.0009 | 0.09% | 2 | 2 |
| IGLV | IGLV3-22-j | 0.0009 | 0.09% | 2 | 1 |
| IGLV | IGLV3-22-n | 0.0009 | 0.09% | 2 | 2 |
| IGLV | IGLV3-22-m | 0.0009 | 0.09% | 2 | 2 |
| IGLV | IGLV3-22-o | 0.0009 | 0.09% | 2 | 2 |
| IGLV | IGLV3-25-g | 0.0009 | 0.09% | 2 | 1 |
| IGLV | IGLV3-25-l | 0.0009 | 0.09% | 2 | 2 |
| IGLV | IGLV3-27-a | 0.0009 | 0.09% | 2 | 2 |
| IGLV | IGLV3-32-c | 0.0009 | 0.09% | 2 | 2 |
| IGLV | IGLV3-9-a | 0.0009 | 0.09% | 2 | 2 |
| IGLV | IGLV3-9-b | 0.0009 | 0.09% | 2 | 2 |
| IGLV | IGLV4-3-d | 0.0009 | 0.09% | 2 | 2 |
| IGLV | IGLV4-60-g | 0.0009 | 0.09% | 2 | 2 |
| IGLV | IGLV4-60-n | 0.0009 | 0.09% | 2 | 2 |
| IGLV | IGLV4-60-o | 0.0009 | 0.09% | 2 | 2 |
| IGLV | IGLV5-45-g | 0.0009 | 0.09% | 2 | 2 |
| IGLV | IGLV5-48-u | 0.0009 | 0.09% | 2 | 2 |
| IGLV | IGLV5-48-q | 0.0009 | 0.09% | 2 | 2 |
| IGLV | IGLV5-48-r | 0.0009 | 0.09% | 2 | 2 |
| IGLV | IGLV5-48-n | 0.0009 | 0.09% | 2 | 2 |
| IGLV | IGLV5-48-m | 0.0009 | 0.09% | 2 | 1 |
| IGLV | IGLV5-48-l | 0.0009 | 0.09% | 2 | 2 |
| IGLV | IGLV5-52-b | 0.0009 | 0.09% | 2 | 1 |
| IGLV | IGLV7-46-c | 0.0009 | 0.09% | 2 | 2 |
| IGLV | IGLV9-49-c | 0.0009 | 0.09% | 2 | 2 |

**Table 20B: Variants Found In No More Than 3 Human Populations & In At Least 10 Individuals**

| **TYPE** | **VARIANT** | **C.FREQ** | **C.FREQ (%)** | **#INDIVIDUALS** | **#POPULATIONS** |
|---|---|---|---|---|---|
| IGKV | IGKV1D-8-b | 0.01785 | 1.79% | 36 | 3 |
| IGHV | IGHV3-11-a | 0.01695 | 1.70% | 36 | 3 |
| IGHV | IGHV5-51-a | 0.0164 | 1.64% | 35 | 3 |
| IGHV | IGHV2-5-b | 0.01055 | 1.06% | 18 | 3 |
| IGLV | IGLV2-14-g | 0.0104 | 1.04% | 22 | 3 |
| IGHV | IGHV7-81-b | 0.01005 | 1.01% | 21 | 3 |
| IGKV | IGKV1D-42-b | 0.01005 | 1.01% | 21 | 3 |
| IGKV | IGKV3-7-b | 0.01 | 1.00% | 19 | 3 |
| IGHV | IGHV1-69-d | 0.00995 | 1.00% | 22 | 3 |
| IGLV | IGLV2-14-e | 0.00995 | 1.00% | 19 | 3 |
| IGLV | IGLV9-49-a | 0.00865 | 0.87% | 18 | 3 |
| IGLV | IGLV1-44-b | 0.00775 | 0.78% | 16 | 2 |
| IGLV | IGLV2-11-a | 0.00775 | 0.78% | 17 | 3 |
| IGKV | IGKV3-11-d | 0.0073 | 0.73% | 14 | 3 |
| IGKV | IGKV1D-43-b | 0.00685 | 0.69% | 14 | 3 |
| IGHV | IGHV3-38-d | 0.0064 | 0.64% | 13 | 3 |
| IGU | IGU6-a | 0.00595 | 0.60% | 13 | 3 |
| IGHV | IGHV5-51-b | 0.0055 | 0.55% | 11 | 3 |
| IGLV | IGLV5-45-c | 0.00545 | 0.55% | 12 | 2 |
| IGHV | IGHV4-31-i | 0.0054 | 0.54% | 10 | 3 |
| IGLV | IGLV1-36-c | 0.00455 | 0.46% | 10 | 2 |

**Table 20C: Variants Found In No More Than 3 Human Populations & In At Least 20 Individuals**

| **TYPE** | **VARIANT** | **C.FREQ** | **C.FREQ (%)** | **#INDIVIDUALS** | **#POPULATIONS** |
|---|---|---|---|---|---|
| IGKV | IGKV1D-8-b | 0.01785 | 1.79% | 36 | 3 |
| IGHV | IGHV3-11-a | 0.01695 | 1.70% | 36 | 3 |
| IGHV | IGHV5-51-a | 0.0164 | 1.64% | 35 | 3 |
| IGLV | IGLV2-14-g | 0.0104 | 1.04% | 22 | 3 |
| IGHV | IGHV7-81-b | 0.01005 | 1.01% | 21 | 3 |
| IGKV | IGKV1D-42-b | 0.01005 | 1.01% | 21 | 3 |
| IGHV | IGHV1-69-d | 0.00995 | 1.00% | 22 | 3 |

**Table 20D: Variants Found In No More Than 3 Human Populations & In At Least 30 Individuals**

| **TYPE** | **VARIANT** | **C.FREQ** | **C.FREQ (%)** | **#INDIVIDUALS** | **#POPULATIONS** |
|---|---|---|---|---|---|
| IGKV | IGKV1D-8-b | 0.01785 | 1.79% | 36 | 3 |
| IGHV | IGHV3-11-a | 0.01695 | 1.70% | 36 | 3 |
| IGHV | IGHV5-51-a | 0.0164 | 1.64% | 35 | 3 |

**Table 21**

| **Seq ID #** | **Associated Gene Name** | **Ensembl Gene ID** | **Gene Location [Chromosome Name:Gene Chr Start (bp)-GeneChr End (bp)]** | **Ensembl Transcript ID** | **Exon Positions [Chromosome Name:Exon Chr Start (bp)-Exon Chr End (bp)]** |
|---|---|---|---|---|---|
| 239 | IGHJ1 | ENSG00000211905 | 14:106331617-106331668 | ENST00000390565 | 14:106331617-106331668 |
| 240 | IGHJ2 | ENSG00000211904 | 14:106331409-106331460 | ENST00000390564 | 14:106331409-106331460 |
| 241 | IGHJ3 | ENSG00000242887 | 14:106330797-106330845 | ENST00000463911 | 14:106330797-106330845 |
| 242 | IGHJ4 | ENSG00000240041 | 14:106330425-106330470 | ENST00000461719 | 14:106330425-106330470 |
| 243 | IGHJ5 | ENSG00000242472 | 14:106330024-106330072 | ENST00000488476 | 14:106330024-106330072 |
| 244 | IGHJ6 | ENSG00000211900 | 14:106329408-106329468 | ENST00000390560 | 14:106329408-106329468 |
| 245 | IGHV1-18 | ENSG00000211945 | 14:106641563-106642056 | ENST00000390605 | 14:106641952-106642056 14:106641563-106641867 |
| 246 | IGHV1-2 | ENSG00000211934 | 14:106452671-106453170 | ENST00000390594 | 14:106453061-106453170 14:106452671-106452975 |
| 247 | IGHV1-24 | ENSG00000211950 | 14:106733144-106733639 | ENST00000390610 | 14:106733534-106733639 14:106733144-106733448 |
| 248 | IGHV1-3 | ENSG00000211935 | 14:106471246-106471723 | ENST00000390595 | 14:106471636-106471723 14:106471246-106471550 |
| 249 | IGHV1-45 | ENSG00000211961 | 14:106962931-106963424 | ENST00000390621 | 14:106963321-106963424 14:106962931-106963235 |
| 250 | IGHV1-46 | ENSG00000211962 | 14:106967049-106967788 | ENST00000390622 | 14:106967439-106967788 14:106967049-106967353 |
| 251 | IGHV1-58 | ENSG00000211968 | 14:107078373-107078869 | ENST00000390628 | 14:107078763-107078869 14:107078373-107078677 |
| 252 | IGHV1-69 | ENSG00000211973 | 14:107169931-107170428 | ENST00000390633 | 14:107170322-107170428 14:107169931-107170235 |
| 253 | IGHV1-8 | ENSG00000211939 | 14:106539079-106539577 | ENST00000390599 | 14:106539470-106539577 14:106539079-106539383 |
| 254 | IGHV2-26 | ENSG00000211951 | 14:106757650-106758116 | ENST00000390611 | 14:106758047-106758116 14:106757650-106757960 |
| 255 | IGHV2-5 | ENSG00000211937 | 14:106494135-106494597 | ENST00000390597 | 14:106494532-106494597 14:106494135-106494445 |
| 256 | IGHV2-70 | ENSG00000211974 | 14:107178820-107179338 | ENST00000390634 | 14:107179217-107179338 14:107178820-107179130 |
| 257 | IGHV3-11 | ENSG00000211941 | 14:106573233-106573800 | ENST00000390601 | 14:106573635-106573800 14:106573233-106573537 |
| 258 | IGHV3-13 | ENSG00000211942 | 14:106586137-106586667 | ENST00000390602 | 14:106586542-106586667 14:106586137-106586438 |
| 259 | IGHV3-15 | ENSG00000211943 | 14:106610313-106610852 | ENST00000390603 | 14:106610727-106610852 14:106610313-106610623 |
| 260 | IGHV3-16 | ENSG00000211944 | 14:106621894-106622419 | ENST00000390604 | 14:106622300-106622419 14:106621894-106622198 |
| 261 | IGHV3-20 | ENSG00000211946 | 14:106667581-106668095 | ENST00000390606 | 14:106667988-106668095 14:106667581-106667885 |
| 262 | IGHV3-21 | ENSG00000211947 | 14:106691673-106692203 | ENST00000390607 | 14:106692079-106692203 14:106691673-106691977 |
| 263 | IGHV3-23 | ENSG00000211949 | 14:106725201-106725733 | ENST00000390609 | 14:106725609-106725733 14:106725201-106725505 |
| 264 | IGHV3-30 | ENSG00000211953 | 14:106791005-106791536 | ENST00000390613 | 14:106791411-106791536 14:106791005-106791309 |
| 265 | IGHV3-33 | ENSG00000211955 | 14:106815722-106816253 | ENST00000390615 | 14:106816128-106816253 14:106815722-106816026 |
| 266 | IGHV3-35 | ENSG00000211957 | 14:106845323-106845789 | ENST00000390617 | 14:106845729-106845789 14:106845323-106845627 |
| 267 | IGHV3-38 | ENSG00000211958 | 14:106866406-106866934 | ENST00000390618 | 14:106866811-106866934 14:106866406-106866707 |
| 268 | IGHV3-43 | ENSG00000232216 | 14:106926188-106926724 | ENST00000434710 | 14:106926599-106926724 14:106926188-106926495 |
| 269 | IGHV3-48 | ENSG00000211964 | 14:106993814-106994346 | ENST00000390624 | 14:106994222-106994346 14:106993814-106994118 |
| 270 | IGHV3-49 | ENSG00000211965 | 14:107012938-107013477 | ENST00000390625 | 14:107013352-107013477 14:107012938-107013248 |
| 271 | IGHV3-53 | ENSG00000211967 | 14:107048672-107049341 | ENST00000390627 | 14:107049075-10704934114:107048672-107048973 |
| 272 | IGHV3-64 | ENSG00000223648 | 14:107113741-107114274 | ENST00000454421 | 14:107114149-107114274 14:107113741-107114045 |
| 273 | IGHV3-66 | ENSG00000211972 | 14:107131033-107131560 | ENST00000390632 | 14:107131436-107131560 14:107131033-107131334 |
| 274 | IGHV3-7 | ENSG00000211938 | 14:106518400-106518932 | ENST00000390598 | 14:106518808-106518932 14:106518400-106518704 |
| 275 | IGHV3-72 | ENSG00000225698 | 14:107198932-107199471 | ENST00000433072 | 14:107199346-107199471 14:107198932-107199242 |
| 276 | IGHV3-73 | ENSG00000211976 | 14:107210932-107211471 | ENST00000390636 | 14:107211346-107211471 14:107210932-107211242 |
| 277 | IGHV3-74 | ENSG00000224650 | 14:107218676-107219365 | ENST00000424969 | 14:107219084-107219365 14:107218676-107218980 |
| 278 | IGHV3-9 | ENSG00000211940 | 14:106552285-106552809 | ENST00000390600 | 14:106552684-106552809 14:106552285-106552592 |
| 279 | IGHV4-28 | ENSG00000211952 | 14:106780513-106781017 | ENST00000390612 | 14:106780900-106781017 14:106780513-106780817 |
| 280 | IGHV4-31 | ENSG00000231475 | 14:106805209-106805716 | ENST00000438142 | 14:106805599-106805716 14:106805209-106805516 |
| 281 | IGHV4-34 | ENSG00000211956 | 14:106829594-106830076 | ENST00000390616 | 14:106829979-106830076 14:106829594-106829895 |
| 282 | IGHV4-39 | ENSG00000211959 | 14:106877619-106878126 | ENST00000390619 | 14:106878010-106878126 14:106877619-106877926 |
| 283 | IGHV4-4 | ENSG00000211936 | 14:106478110-106478603 | ENST00000390596 | 14:106478494-106478603 14:106478110-106478411 |
| 284 | IGHV4-59 | ENSG00000224373 | 14:107081806-107083830 | ENST00000390629 | 14:107083640-107083830 14:107083256-107083557 |
| 284 | IGHV4-59 | ENSG00000224373 | 14:107081806-107083830 | ENST00000455737 | 14:107083640-107083725 14:107083240-107083557 |
| | | | | | 14:107081806-107082728 |
| 285 | IGHV4-61 | ENSG00000211970 | 14:107095126-107095662 | ENST00000390630 | 14:107095516-107095662 14:107095126-107095433 |
| 286 | IGHV5-51 | ENSG00000211966 | 14:107034729-107035221 | ENST00000390626 | 14:107035117-107035221 14:107034729-107035033 |
| 287 | IGHV6-1 | ENSG00000211933 | 14:106405611-106406108 | ENST00000390593 | 14:106406008-106406108 14:106405611-106405924 |
| 288 | IGHV7-81 | ENSG00000211979 | 14:107282792-107283280 | ENST00000390639 | 14:107283181-107283280 14:107282792-107283096 |
| 289 | IGKJ2 | ENSG00000211596 | 2:89161037-89161074 | ENST00000390241 | 2:89161037-89161074 |
| 290 | IGKJ3 | ENSG00000211595 | 2:89160733-89160770 | ENST00000390240 | 2:89160733-89160770 |
| 291 | IGKJ4 | ENSG00000211594 | 2:89160398-89160434 | ENST00000390239 | 2:89160398-89160434 |
| 292 | IGKJ5 | ENSG00000211593 | 2:89160080-89160117 | ENST00000390238 | 2:89160080-89160117 |
| 293 | IGKV1-16 | ENSG00000240864 | 2:89399352-89399854 | ENST00000479981 | 2:89399773-89399854 2:89399352-89399647 |
| 294 | IGKV1-17 | ENSG00000240382 | 2:89416833-89417335 | ENST00000490686 | 2:89417254-89417335 2:89416833-89417128 |
| 295 | IGKV1-5 | ENSG00000243466 | 2:89246819-89247475 | ENST00000496168 | 2:89247240-89247475 2:89246819-89247114 |
| 296 | IGKV1-6 | ENSG00000239855 | 2:89265781-89266286 | ENST00000464162 | 2:89266203-89266286 2:89265781-89266076 |
| 297 | IGKV1-8 | ENSG00000240671 | 2:89291928-89292450 | ENST00000495489 | 2:89292349-89292450 2:89291928-89292223 |
| 298 | IGKV1-9 | ENSG00000241755 | 2:89309479-89310012 | ENST00000493819 | 2:89309900-89310012 2:89309479-89309774 |
| 299 | IGKV1D-12 | ENSG00000240834 | 2:90198535-90199190 | ENST00000390276 | 2:90198535-90198770 2:90198895-90199190 |
| 300 | IGKV1D-16 | ENSG00000241244 | 2:90139078-90139580 | ENST00000492446 | 2:90139078-90139159 2:90139285-90139580 |
| 301 | IGKV1D-17 | ENSG00000242766 | 2:90121477-90122133 | ENST00000483379 | 2:90121477-90121712 2:90121838-90122133 |
| 302 | IGKV1D-42 | ENSG00000211633 | 2:90229045-90229531 | ENST00000390278 | 2:90229045-90229119 2:90229236-90229531 |
| 303 | IGKV1D-43 | ENSG00000242580 | 2:90248739-90249395 | ENST00000468879 | 2:90248739-90248974 2:90249100-90249395 |
| 304 | IGKV1D-8 | ENSG00000239819 | 2:90259593-90260248 | ENST00000471857 | 2:90259593-90259828 2:90259953-90260248 |
| 305 | IGKV2-24 | ENSG00000241294 | 2:89475812-89476644 | ENST00000484817 | 2:89476566-89476644 2:89475812-89476122 |
| 306 | IGKV2-30 | ENSG00000243238 | 2:89544264-89545079 | ENST00000468494 | 2:89545001-89545079 2:89544264-89544574 |
| 307 | IGKV2D-24 | ENSG00000241566 | 2:90043607-90044439 | ENST00000462693 | 2:90043607-90043685 2:90044129-90044439 |
| 308 | IGKV2D-26 | ENSG00000211623 | 2:90024732-90025512 | ENST00000390268 | 2:90024732-90024810 2:90025202-90025512 |
| 309 | IGKV2D-29 | ENSG00000243264 | 2:89986322-89987079 | ENST00000491977 | 2:89986322-89986400 2:89986769-89987079 |
| 310 | IGKV2D-30 | ENSG00000239571 | 2:89975669-89976489 | ENST00000474213 | 2:89975669-89975752 2:89976179-89976489 |
| 311 | IGKV3-11 | ENSG00000241351 | 2:89326668-89327228 | ENST00000483158 | 2:89327133-89327228 2:89326668-89326963 |
| 312 | IGKV3-20 | ENSG00000239951 | 2:89442057-89442643 | ENST00000492167 | 2:89442543-89442643 2:89442057-89442355 |
| 313 | IGKV3-7 | ENSG00000243063 | 2:89277987-89278600 | ENST00000390247 | 2:89278455-89278600 2:89277987-89278285 |
| 314 | IGKV3D-11 | ENSG00000211632 | 2:90211643-90212253 | ENST00000390277 | 2:90211643-90211788 2:90211958-90212253 |
| 315 | IGKV3D-15 | ENSG00000224041 | 2:90153696-90154258 | ENST00000417279 | 2:90153696-90153793 2:90153963-90154258 |
| 316 | IGKV3D-20 | ENSG00000211625 | 2:90077680-90078311 | ENST00000390270 | 2:90077680-90077825 2:90078013-90078311 |
| 317 | IGKV4-1 | ENSG00000211598 | 2:89184913-89185669 | ENST00000390243 | 2:89184913-89185136 2:89185356-89185669 |
| 318 | IGKV5-2 | ENSG00000211599 | 2:89196748-89197300 | ENST00000390244 | 2:89196748-89196859 2:89197005-89197300 |
| 319 | IGKV6-21 | ENSG00000211611 | 2:89459235-89459850 | ENST00000390256 | 2:89459741-89459850 2:89459235-89459530 |
| 320 | IGKV6D-21 | ENSG00000225523 | 2:90060377-90060995 | ENST00000436451 | 2:90060377-90060489 2:90060700-90060995 |
| 321 | IGKV6D-41 | ENSG00000211626 | 2:90108504-90109080 | ENST00000390271 | 2:90108504-90108578 2:90108785-90109080 |
| 322 | IGLJ1 | ENSG00000211674 | 22:23235872-23235998 | ENST00000390320 | 22:23235872-23235998 |
| 323 | IGU2 | ENSG00000211676 | 22:23241661-23241835 | ENST00000390322 | 22:23241661-23241835 |
| 324 | IGLJ3 | ENSG00000211678 | 22:23247030-23247205 | ENST00000390324 | 22:23247030-23247205 |
| 325 | IGLJ5 | ENSG00000211681 | 22:23256408-23256479 | ENST00000390327 | 22:23256408-23256479 |
| 326 | IGLJ6 | ENSG00000211682 | 22:23260304-23260373 | ENST00000390328 | 22:23260304-23260373 |
| 327 | IGLJ7 | ENSG00000211684 | 22:23263562-23263607 | ENST00000390330 | 22:23263562-23263607 |
| 328 | IGLV10-54 | ENSG00000211642 | 22:22569184-22569660 | ENST00000390287 | 22:22569184-22569242 22:22569355-22569660 |
| 329 | IGLV11-55 | ENSG00000211641 | 22:22556057-22556600 | ENST00000390286 | 22:22556057-22556114 22:22556233-22556600 |
| 330 | IGLV1-36 | ENSG00000211655 | 22:22786296-22786802 | ENST00000390301 | 22:22786296-22786381 22:22786497-22786802 |
| 331 | IGLV1-40 | ENSG00000211653 | 22:22764098-22764614 | ENST00000390299 | 22:22764098-22764194 22:22764305-22764614 |
| 332 | IGLV1-44 | ENSG00000211651 | 22:22735135-22735715 | ENST00000390297 | 22:22735135-22735294 22:22735410-22735715 |
| 333 | IGLV1-47 | ENSG00000211648 | 22:22712087-22712608 | ENST00000390294 | 22:22712087-22712188 22:22712304-22712608 |
| 334 | IGLV1-50 | ENSG00000211645 | 22:22681658-22682172 | ENST00000390291 | 22:22681658-22681754 22:22681865-22682172 |
| 335 | IGLV1-51 | ENSG00000211644 | 22:22676828-22677336 | ENST00000390290 | 22:22676828-22676909 22:22677019-22677336 |
| 336 | IGLV2-11 | ENSG00000211668 | 22:23134980-23135496 | ENST00000390314 | 22:23134980-23135067 22:23135185-23135496 |
| 337 | IGLV2-14 | ENSG00000211666 | 22:23101189-23101707 | ENST00000390312 | 22:23101189-23101275 22:23101393-23101707 |
| 338 | IGLV2-18 | ENSG00000211664 | 22:23077095-23077584 | ENST00000390310 | 22:23077095-23077153 22:23077270-23077584 |
| 339 | IGLV2-23 | ENSG00000211660 | 22:23040274-23040892 | ENST00000390306 | 22:23040274-23040481 22:23040599-23040892 |
| 340 | IGLV2-33 | ENSG00000211656 | 22:22930626-22931145 | ENST00000390302 | 22:22930626-22930729 22:22930852-22931145 |
| 341 | IGLV2-8 | ENSG00000211671 | 22:23165153-23165787 | ENST00000390317 | 22:23165153-23165360 22:23165476-23165787 |
| 342 | IGLV3-1 | ENSG00000211673 | 22:23222886-23223576 | ENST00000390319 | 22:23222886-23222983 22:23223277-23223576 |
| 343 | IGLV3-10 | ENSG00000211669 | 22:23154244-23154782 | ENST00000390315 | 22:23154244-23154324 22:23154478-23154782 |
| 344 | IGLV3-12 | ENSG00000211667 | 22:23114317-23115079 | ENST00000390313 | 22:23114317-23114371 22:23114775-23115079 |
| 345 | IGLV3-16 | ENSG00000211665 | 22:23089870-23090398 | ENST00000390311 | 22:23089870-23089953 22:23090108-23090398 |
| 346 | IGLV3-19 | ENSG00000211663 | 22:23063108-23063630 | ENST00000390309 | 22:23063108-23063193 22:23063340-23063630 |
| 347 | IGLV3-21 | ENSG00000211662 | 22:23054174-23055688 | ENST00000390308 | 22:23054174-23054290 22:23054770-23054949 22:23055384-23055688 |
| 348 | IGLV3-22 | ENSG00000211661 | 22:23046750-23047307 | ENST00000390307 | 22:23046750-23046859 22:23047009-23047307 |
| 349 | IGLV3-25 | ENSG00000211659 | 22:23029190-23029735 | ENST00000390305 | 22:23029190-23029278 22:23029445-23029735 |
| 350 | IGLV3-27 | ENSG00000211658 | 22:23010758-23011276 | ENST00000390304 | 22:23010758-23010841 22:23010984-23011276 |
| 351 | IGLV3-32 | ENSG00000211657 | 22:22936998-22937501 | ENST00000390303 | 22:22936998-22937079 22:22937226-22937501 |
| 352 | IGLV3-9 | ENSG00000211670 | 22:23161507-23162253 | ENST00000390316 | 22:23161507-23161667 22:23161954-23162253 |
| 353 | IGLV4-3 | ENSG00000211672 | 22:23213686-23214214 | ENST00000390318 | 22:23213686-23213763 22:23213890-23214214 |
| 354 | IGLV4-60 | ENSG00000211639 | 22:22516592-22517074 | ENST00000390284 | 22:22516592-22516643 22:22516765-22517074 |
| 355 | IGLV4-69 | ENSG00000211637 | 22:22385332-22385870 | ENST00000390282 | 22:22385332-22385440 22:22385561-22385870 |
| 356 | IGLV5-37 | ENSG00000211654 | 22:22781876-22782371 | ENST00000390300 | 22:22781876-22781926 22:22782049-22782371 |
| 357 | IGLV5-45 | ENSG00000211650 | 22:22730355-22730874 | ENST00000390296 | 22:22730355-22730428 22:22730552-22730874 |
| 358 | IGLV5-48 | ENSG00000211647 | 22:22707289-22707781 | ENST00000390293 | 22:22707289-22707334 22:22707459-22707781 |
| 359 | IGLV5-52 | ENSG00000211643 | 22:22673082-22673581 | ENST00000390289 | 22:22673082-22673132 22:22673254-22673581 |
| 360 | IGLV6-57 | ENSG00000211640 | 22:22550113-22550686 | ENST00000390285 | 22:22550113-22550244 22:22550370-22550686 |
| 361 | IGLV7-43 | ENSG00000211652 | 22:22749356-22749827 | ENST00000390298 | 22:22749356-22749435 22:22749523-22749827 |
| 362 | IGLV7-46 | ENSG00000211649 | 22:22723982-22724454 | ENST00000390295 | 22:22723982-22724061 22:22724151-22724454 |
| 363 | IGLV8-61 | ENSG00000211638 | 22:22453110-22453622 | ENST00000390283 | 22:22453110-22453216 22:22453316-22453622 |
| 364 | IGLV9-49 | ENSG00000223350 | 22:22697539-22698084 | ENST00000427632 | 22:22697539-22697621 22:22697759-22698084 |

### Refences:

1. Nat Biotechnol. 2005 Sep;23(9):1117-25; Human antibodies from transgenic animals; Lonberg N.
2. J Clin Invest. 1992 Mar;89(3):729-38; Immunoglobulin light chain variable region gene sequences for human antibodies to Haemophilus influenzae type b capsular polysaccharide are dominated by a limited number of V kappa and V lambda segments and VJ combinations; Adderson EE, Shackelford PG, Insel RA, Quinn A, Wilson PM, Carroll WL.
3. J Immunol. 1993 Oct 15;151(8):4352-61; Clonal characterization of the human IgG antibody repertoire to Haemophilus influenzae type b polysaccharide. V. In vivo expression of individual antibody clones is dependent on Ig CH haplotypes and the categories of antigen; Chung GH, Scott MG, Kim KH, Kearney J, Siber GR, Ambrosino DM, Nahm MH.
4. J Immunol. 1998 Dec 1;161(11):6068-73; Decreased frequency of rearrangement due to the synergistic effect of nucleotide changes in the heptamer and nonamer of the recombination signal sequence of the V kappa gene A2b, which is associated with increased susceptibility of Navajos to Haemophilus influenzae type b disease; Nadel B, Tang A, Lugo G, Love V, Escuro G, Feeney AJ.
5. J Clin Invest. 1996 May 15;97(10):2277-82; A defective Vkappa A2 allele in Navajos which may play a role in increased susceptibility to haemophilus influenzae type b disease; Feeney AJ, Atkinson MJ, Cowan MJ, Escuro G, Lugo G.
6. Infect Immun. 1994 Sep;62(9):3873-80; Variable region sequences of a protective human monoclonal antibody specific for the Haemophilus influenzae type b capsular polysaccharide; Lucas AH, Larrick JW, Reason DC.
7. J Clin Invest. 1993 Jun;91(6):2734-43; Restricted immunoglobulin VH usage and VDJ combinations in the human response to Haemophilus influenzae type b capsular polysaccharide. Nucleotide sequences of monospecific anti-Haemophilus antibodies and polyspecific antibodies cross-reacting with self antigens; Adderson EE, Shackelford PG, Quinn A, Wilson PM, Cunningham MW, Insel RA, Carroll WL.
8. J Clin Invest. 1993 Mar;91(3):788-96; Variable region expression in the antibody responses of infants vaccinated with Haemophilus influenzae type b polysaccharide-protein conjugates. Description of a new lambda light chain-associated idiotype and the relation between idiotype expression, avidity, and vaccine formulation. The Collaborative Vaccine Study Group; Granoff DM, Shackelford PG, Holmes SJ, Lucas AH.
9. Infect Immun. 1994 May;62(5):1776-86; Variable region sequences and idiotypic expression of a protective human immunoglobulin M antibody to capsular polysaccharides of Neisseria meningitidis group B and Escherichia coli K1; Azmi FH, Lucas AH, Raff HV, Granoff DM.
10. J Clin Invest. 1992 Dec;90(6):2197-208; Sequence analyses of three immunoglobulin G antivirus antibodies reveal their utilization of autoantibody-related immunoglobulin Vh genes, but not V lambda genes; Huang DF, Olee T, Masuho Y, Matsumoto Y, Carson DA, Chen PP.
11. Science. 2011 Aug 12;333(6044):834-5, Biochemistry. Catching a moving target, Wang TT, Palese P
12. Science. 2009 Apr 10;324(5924):246-51. Epub 2009 Feb 26; Antibody recognition of a highly conserved influenza virus epitope; Ekiert DC, Bhabha G, Elsliger MA, Friesen RH, Jongeneelen M, Throsby M, Goudsmit J, Wilson IA.
13. PLoS One. 2008;3(12):e3942. Epub 2008 Dec 16; Heterosubtypic neutralizing monoclonal antibodies cross-protective against H5N1 and H1N1 recovered from human IgM+ memory B cells; Throsby M, van den Brink E, Jongeneelen M, Poon LL, Alard P, Cornelissen L, Bakker A, Cox F, van Deventer E, Guan Y, Cinatl J, ter Meulen J, Lasters I, Carsetti R, Peiris M, de Kruif J, Goudsmit J.
14. Nat Struct Mol Biol. 2009 Mar;16(3):265-73. Epub 2009 Feb 22,Structural and functional bases for broad-spectrum neutralization of avian and human influenza A viruses, Sui J, Hwang WC, Perez S, Wei G, Aird D, Chen LM, Santelli E, Stec B, Cadwell G, Ali M, Wan H, Murakami A, Yammanuru A, Han T, Cox NJ, Bankston LA, Donis RO, Liddington RC, Marasco WA.
15. Science. 2011 Aug 12;333(6044):843-50. Epub 2011 Jul 7, A highly conserved neutralizing epitope on group 2 influenza A viruses, Ekiert DC, Friesen RH, Bhabha G, Kwaks T, Jongeneelen M, Yu W, Ophorst C, Cox F, Korse HJ, Brandenburg B, Vogels R, Brakenhoff JP, Kompier R, Koldijk MH, Cornelissen LA, Poon LL, Peiris M, Koudstaal W, Wilson IA, Goudsmit J.

Aspects of the invention are disclosed in the following numbered clauses
1. A non-human vertebrate (optionally a mouse or a rat) or vertebrate cell whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof so that the mouse is capable of producing an antibody heavy chain produced by recombination of the human JH6*02 with a D segment and a VH segment.
2. The vertebrate of clause 1, wherein the vertebrate has been immunised with a target antigen and wherein the variable domain of the heavy chain is the product of recombination between a VH, D and JH6*02 and wherein the HCDR3 length is at least 20 amino acids.
3. A non-human vertebrate cell (optionally a mouse cell or a rat cell) whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof for producing (eg, in a subsequent progeny cell) an antibody heavy chain produced by recombination of the human JH6*02 with a D segment and a VH segment.
4. The cell of clause 3, which is an ES cell capable of differentiation into a progeny antibody-producing cell that expresses said heavy chain.
5. The vertebrate or cell of any preceding clause wherein the heavy chain locus comprises a human JH6*02 recombination signal sequence (RSS) operably connected 5' to the JH6*02 gene segment.
6. The vertebrate or cell ofclause 5, wherein the RSS is SEQ ID NO: 238 or a sequence having an identical 9mer and 7mer sequence flanking a sequence that is at least 70% identical to the 22mer sequence of SEQ ID NO: 238.
7. The vertebrate or cell of clause6, wherein the RSS and JH6*02 are provided as SEQ ID NO: 237.
8. The vertebrate or cell of any preceding clause, wherein the JH6*02 is the only JH6-type gene segment in the genome.
9. The vertebrate or cell of any preceding clause, wherein the JH6*02 is the closest JH gene segment to the constant region in the locus.
10. The vertebrate or cell of any preceding clause, wherein the locus comprises one, more or all human D gene segments D3-9; D4-17; D3-10; D2-2; D5-24; D6-19; D3-22; D6-13; D5-12; D1-26; D1-20; D5-18; D3-16; D2-21; D1-14; D7-27; D1-1; D6-25; D2-14; and D4-23.
11. The vertebrate or cell of clause 10, wherein the locus comprises one, more or all human D gene segments D3-9, D3-10, D6-19, D4-17, D6-13, D3-22, D2-2, D2-25 and D3-3.
12. The vertebrate or cell of any preceding clause, wherein the locus comprises a plurality of human D gene segments and the JH6*02 is in human germline configuration with respect to the 3'-most human D gene segment.
13. The vertebrate or cell of any preceding clause, wherein the locus comprises one, more or all of IGHV gene segments selected from V3-21, V3-13, V3-7, V6-1, V1-8, V1-2, V7-4-1, V1-3, V1-18, V4-4, V3-9, V3-23, V3-11 and V3-20.
14. The vertebrate or cell of any preceding clause wherein the locus comprises one, more or all of human D3-9*01, D3-10*01, D6-19*01, D6-13*01, D1-26*01, IGHV1-8*01, IGHV4-61*01, IGHV6-1*01, IGHV4-4*02, IGHV1-3*01, IGHV3-66*03, IGHV3-7*01 and IGHV3-9*01.
15. An antibody-producing cell (eg, a B-cell) that is a progeny of the cell of any one of clauses3 to 14, wherein the antibody-producing cell comprises a heavy chain locus comprising a rearranged variable region produced by recombination of human JH6*02 with a D segment and a VH segment.
16. The cell of clause 15, which is a B-cell or hybridoma that expresses a target antigen-specific antibody comprising a heavy chain that comprises a rearranged variable region produced by recombination of human JH6*02 with a D segment and a VH segment.
17. The vertebrate or cell of any preceding clause, wherein the antibody heavy chain specifically binds a target antigen.
18. The vertebrate or cell of any preceding clause wherein the antibody heavy chain has a HCDR3 length of at least 20 amino acids.
19. The vertebrate or cell of any preceding clause wherein the antibody heavy chain is a product of the recombination of JH6*02 with a human VH gene segment recited inclause 13 or 14 and/or a D gene segment recited in clause 10, 11 or 14.
20. The vertebrate or cell of any preceding clause, wherein all endogenous non-human vertebrate heavy chain variable region gene segments have been inactivated in the genome.
21. The vertebrate or cell of any preceding clause, wherein the genome is homozygous for said heavy chain locus.
22. A heavy chain (eg, comprised by an antibody) isolated from a vertebrate of any one of clause 1, 2, 5 to 14 and 17 to 21 wherein the heavy chain comprises a HCDR3 of at least 20 amino acids.
23. The heavy chain of clause22, wherein the HCDR3 is the product of recombination of human JH6*02 with a human VH gene segment recited in clause 13 or 14 and/or a D gene segment recited in clause 10, 11 or 14.
24. A heavy chain (eg, comprised by an antibody) whose VH variable domain is identical to the VH variable domain of the heavy chain of clause 22 or 23, and which comprises a human constant region or a human-mouse chimaeric constant region.
25. The heavy chain of clause 22, 23 or 24, whose VH variable domain is specific for a target antigen.
26. A method for producing a heavy clause, VH domain or an antibody specific to a target antigen, the method comprising immunizing a non-human vertebrate according to any one of clauses 1, 2, 5 to 14 and 17 to 21 with the antigen and isolating the heavy chain, VH domain or an antibody specific to a target antigen or a cell producing the heavy chain, VH domain or an antibody, wherein the heavy chain, VH domain or an antibody comprises a HCDR3 that is derived from the recombination of human JH6*02 with a VH gene segment and a D gene segment.
27. A method for producing a human heavy chain or antibody comprising carrying out the method of claim 26, wherein the constant region of the locus is a non-human vertebrate (eg, mouse or rat) constant region, and then replacing the non-human constant region of the isolated heavy chain or antibody with a human constant region.
28. A heavy chain, VH domain or an antibody produced by the method of clause 26 or 27.
29. A B-cell or hybridoma expressing a heavy chain VH domain that is identical to the VH domain of the heavy chain of clause 22, 23 or 28.
30. A nucleic acid encoding the VH domain of the heavy chain of clause 22, 23 or 28, or encoding the heavy chain of clause 22, 23, 24, 25 or 28.
31. A vector (eg, a CHO cell or HEK293 cell vector) comprising the nucleic acid of clause 30; optionally wherein the vector is in a host cell (eg, a CHO cell or HEK293 cell).
32. A pharmaceutical composition comprising the antibody, heavy chain or VH domain (eg, comprised by an antibody) of any one ofclauses 22 to 25 and 28, together with a pharmaceutically-acceptable excipient, diluent or a medicament (eg, a further antigen-specific variable domain, heavy chain or antibody).
33. The antibody, heavy chain or VH domain (eg, comprised by an antibody) of any one of clauses 22 to 25 and 28 for use in medicine.
34. A non-human vertebrate (eg, a mouse or rat) or a non-human vertebrate cell (eg, an ES cell or a B-cell) having a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein at least two of the human gene segments are variants that are not identical to each other.
35. A non-human vertebrate (eg, a mouse or rat) or a non-human vertebrate cell (eg, an ES cell or a B-cell) having a genome comprising at least 2 different non-endogenous variable region gene segments of the same type (eg, at least 2 human VH6-1 gene segments, at least 3 human JH6 gene segments, at least 2 human Vκ1-39 gene segments, at least 2 human D2-2 gene segments or at least 2 human Jκ1 gene segments) *cis* at the same Ig locus.
36. A non-human vertebrate (eg, a mouse or rat) or a non-human vertebrate cell (eg, an ES cell or a B-cell) having a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) *trans* at the same Ig locus; and optionally a third human gene segment of the same type, wherein the third gene segment is *cis* with one of said 2 different gene segments.
37. A population of non-human vertebrates (eg, mice or rats) comprising a repertoire of human variable region gene segments, wherein the plurality comprises at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), a first of said different gene segments is provided in the genome of a first vertebrate of the population, and a second of said different gene segments being provided in the genome of a second vertebrate of the population, wherein the genome of the first vertebrate does not comprise the second gene segment.
38. A non-human vertebrate (eg, a mouse or rat) or a non-human vertebrate cell (eg, an ES cell or a B-cell) having a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.
39. A method of enhancing the human immunoglobulin gene diversity of a non-human vertebrate (eg, a mouse or rat), the method comprising providing the vertebrate with a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein at least two of the human gene segments are variants that are not identical to each other.
40. A method of enhancing the immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) *cis* at the same Ig locus.
41. A method of enhancing the immunoglobulin gene diversity of a non-human vertebrate (eg, a mouse or rat), the method comprising providing the vertebrate with a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) *trans* at the same Ig locus; and optionally a third human gene segment of the same type, wherein the third gene segment is *cis* with one of said 2 different gene segments.
42. A method of providing an enhanced human immunoglobulin variable region gene segment repertoire, the method comprising providing a population of non-human vertebrates (eg, a mouse or rat) comprising a repertoire of human variable region gene segments, wherein the method comprises providing at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein a first of said different gene segments is provided in the genome of a first vertebrate of the population, and a second of said different gene segments is provided in the genome of a second vertebrate of the population, wherein the genome of the first vertebrate does not comprise the second gene segment.
43. A method of enhancing the human immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.
44. The vertebrate, cell or method of any one of clauses 34 to 43, wherein at least 2 or 3 of said different gene segments are provided *cis* at the same Ig locus in said genome.
45. The vertebrate, cell or method of any one of clauses 34 to 44, wherein the gene segments are derived from the genome sequence of different human individuals that are not genetically related over at least 3 generations.
46. The vertebrate, cell or method of any one of clauses 34 to 45, wherein the gene segments are derived from the genome sequence of two or more different human individuals; optionally wherein the different human individuals are from different human populations.
47. The vertebrate, cell or method of clause 46, wherein the individuals are not genetically related.
48. A method of enhancing the human immunoglobulin gene diversity of a non-human vertebrate (eg, a mouse or rat), the method comprising providing the vertebrate with a genome comprising at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations; optionally wherein at least 2 or 3 of said different gene segments are provided at the same Ig locus in said genome.
49. The method of clause 48, wherein the different human individuals are from different human populations.
50. The method of clause 48, wherein the individuals are not genetically related.
51. The vertebrate, cell or method of preceding clause, wherein at least one of the different segments is a synthetic mutant of a human germline gene segment.
52. The vertebrate, cell or method of any one of clauses 34 to 51, wherein each of said gene segments occurs in 10 or more different human populations.
53. The vertebrate, cell or method of preceding clause wherein each of said gene segments has a human frequency of 5% or greater.
54. The vertebrate, cell or method of clause53, wherein each of said gene segments occurs in 10 or more different human populations.
55. The vertebrate, cell or method of any one of clauses 34 to 54, wherein each of said gene segments occurs in the 1000 Genomes database in more than 50 individuals.
56. The vertebrate, cell or method of preceding clause, wherein each of said gene segments (i) has a human frequency of 5% or greater; and (ii) occurs in 10 or more different human populations.
57. A non-human vertebrate (eg, a mouse or rat) or a non-human vertebrate cell (eg, an ES cell or a B-cell) having a genome comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 to 7 and 9 to 14 (eg, IGHJ6-a) and the second gene segment is the corresponding reference sequence (eg, IGHJ6 ref; SEQ ID NO: 244).
58. The vertebrate or cell of clause 57, wherein the genome comprises a third human gene segment of said type, the third gene segment being different from the first and second gene segments.
59. The vertebrate or cell of clause57 or 58, wherein the first and second gene segments are *cis* on the same chromosome; and optionally the third gene segment is also *cis* on said chromosome.
60. The vertebrate or cell of clause 59, wherein the gene segments are targeted insertions into an endogenous non-human Ig locus.
61. The vertebrate or cell of clause57 or 58, wherein the first and second gene segments are *trans* on different chromosomes.
62. The vertebrate or cell of any one of clauses57 to 61, wherein the first gene segment is a gene segment selected any one of Tables 1 to 7 and 9 to 14 and the second gene segment is the corresponding reference sequence.
63. A population of non-human vertebrates (eg, mice or rats) comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 to 7 and 9 to 14 (eg, IGHJ6-a) and the second gene segment is the corresponding reference sequence (eg, SEQ ID NO: 244), wherein the first gene segment is provided in the genome of a first vertebrate of the population, and the second gene segment is provided in the genome of a second vertebrate of the population.
64. The population of clause 63, wherein the genome of the first vertebrate does not comprise the second gene segment.
65. The population of clause 63 or 64, wherein the population comprises a third human gene segment of said type, the third gene segment being different from the first and second gene segments and optionally wherein the first and third gene segments are present in the genome of the first vertebrate.
66. The population ofclause 63, 64 or 65, wherein the gene segments are targeted insertions into an endogenous non-human Ig locus in the respective genome.
67. The population of any one of clauses 63 to 66, wherein the first gene segment is a gene segment selected from any one of Tables 1 to 7 and 9 to 14 and the second gene segment is the corresponding reference sequence.
68. A method of enhancing the human immunoglobulin gene diversity of a non-human vertebrate (eg, a mouse or rat), the method comprising providing the vertebrate with a genome comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 to 7 and 9 to 14 (eg, IGHJ6-a) and the second gene segment is the corresponding reference sequence (eg, SEQ ID NO: 244).
69. A method of providing an enhanced human immunoglobulin gene segment repertoire, the method comprising providing a population according to any one of clauses 63 to 66.
70. A non-human vertebrate (eg, a mouse or rat) or a non-human vertebrate cell (eg, an ES cell or a B-cell) according to clause34, having a genome comprising at least 3 human D gene segments of the same type (eg, D2-2 gene segments), wherein at least two of the human D gene segments are variants that are not identical to each other (eg, D2-2ref and D2-2a).
71. A non-human vertebrate (eg, a mouse or rat) or a non-human vertebrate cell (eg, an ES cell or a B-cell) according to clause35, having a genome comprising at least 2 different non-endogenous D gene segments of the same type type (eg, D2-2ref and D2-2a) *cis* at the same Ig locus.
72. A non-human vertebrate (eg, a mouse or rat) or a non-human vertebrate cell (eg, an ES cell or a B-cell) according to clause36, having a genome comprising at least 2 different human D gene segments of the same type (eg, D2-2ref and D2-2a) *trans* at the same Ig locus; and optionally a third human D gene segment (eg, (eg, D2-2ref, D2-2a or D2-2b) of the same type, wherein the third D is *cis* with one of said 2 different D gene segments.
73. A population of non-human vertebrates (eg, mice or rats) according to clause 37, comprising a repertoire of human D gene segments, wherein the plurality comprises at least 2 different human D gene segments of the same type (eg, D2-2 gene segments), a first of said different D gene segments (eg, D2-2ref) is provided in the genome of a first vertebrate of the population, and a second of said different D gene segment (eg, D2-2a) being provided in the genome of a second vertebrate of the population, wherein the genome of the first vertebrate does not comprise the second D gene segment.
74. A non-human vertebrate (eg, a mouse or rat) or a non-human vertebrate cell (eg, an ES cell or a B-cell) according to clause38, having a genome comprising at least 2 different non-endogenous D gene segments of the same type (eg, human D2-2 gene segments), wherein the D gene segments are derived from the genome sequence of different human individuals that are not genetically related over at least 3 generations.
75. A method of enhancing the human immunoglobulin gene diversity of a non-human vertebrate (eg, a mouse or rat) according to clause 39, the method comprising providing the vertebrate with a genome comprising at least 3 human D gene segments of the same type (eg, D2-2 gene segments), wherein at least two of the human D gene segments are variants that are not identical to each other (eg, D2-2ref and D2-2a).
76. A method of enhancing the immunoglobulin gene diversity of a non-human vertebrate (eg, a mouse or rat) according to clause 40, the method comprising providing the vertebrate with a genome comprising at least 2 different non-endogenous D gene segments of the same type (eg, human D2-2 gene segments) *cis* at the same Ig locus.
77. A method of enhancing the immunoglobulin gene diversity of a non-human vertebrate (eg, a mouse or rat) according to clause41, the method comprising providing the vertebrate with a genome comprising at least 2 different human D gene segments of the same type (eg, D2-2ref and D2-2a) *trans* at the same Ig locus; and optionally a third human D gene segment (eg, D2-2ref, D2-2a or D2-2b) of the same type, wherein the third D is *cis* with one of said 2 different D gene segments.
78. A method of providing an enhanced human immunoglobulin D gene segment repertoire according to clause 42, the method comprising providing a population of non-human vertebrates (eg, a mouse or rat) comprising a repertoire of human D gene segments, wherein the method comprises providing at least 2 different human D gene segments of the same type (eg, D2-2ref and D2-2a), wherein a first of said different D gene segments is provided in the genome of a first vertebrate of the population, and a second of said different D gene segments is provided in the genome of a second vertebrate of the population, wherein the genome of the first vertebrate does not comprise the second D gene segment.
79. A method of enhancing the human immunoglobulin gene diversity of a non-human vertebrate (eg, a mouse or rat) according to clause 43, the method comprising providing the vertebrate with a genome comprising at least 2 different non-endogenous D gene segments of the same type (eg, D2-2ref and D2-2a), wherein the D gene segments are derived from the genome sequence of different human individuals that are not genetically related over at least 3 generations.
80. The vertebrate or cell of clause 70, 71 or 74, or the method of clause 75, 77 or 79, wherein at least 2 or 3 of said different gene segments are provided *cis* at the same Ig locus in said genome.
81. The vertebrate or cell of clause 70, 71 or 71, or the method of any one ofclauses75 to 78 and 80, wherein the D gene segments are derived from the genome sequence of different human individuals that are not genetically related over at least 3 generations.
82. The vertebrate or cell of any one of clauses70 to 73, or the method of any one of clauses 75 to 78 and 81, wherein the D gene segments are derived from the genome sequence of two or more different human individuals; optionally wherein the different human individuals are from different human populations.
83. The vertebrate, cell or method of clause 82, wherein the individuals are not genetically related.
84. The vertebrate, cell or method of any one of clauses 70 to 83, wherein at least one of the different D segments is a synthetic mutant of a human germline D gene segment.
85. A method of enhancing the human immunoglobulin gene diversity of a non-human vertebrate (eg, a mouse or rat) according to clause48, the method comprising providing the vertebrate with a genome comprising at least 2 human D gene segments (eg, D2-2ref and D2-2a), wherein the D gene segments are derived from the genome sequence of different human individuals that are not genetically related over at least 3 generations; optionally wherein at least 2 or 3 of said different gene segments are provided at the same IgH locus in said genome.
86. The vertebrate or cell of any one of clauses 70 to 74 and 80 to 84, wherein the genome comprises a substantially complete functional repertoire of human D gene segment types supplemented with one, two or more variant human D gene segments, wherein said substantially complete functional repertoire and the supplementary D gene segments are not found together in the germline genome of a human individual.
87. The population of clause 73, wherein the population comprises a substantially complete functional repertoire of human D gene segment types supplemented with one, two or more variant human D gene segments, wherein said substantially complete functional repertoire and the supplementary D gene segments are not found together in the germline genome of a human individual.
88. A non-human vertebrate (eg, a mouse or rat) or a non-human cell (eg, an ES cell or a B-cell) having a genome comprising a substantially complete functional repertoire of human D gene segment types supplemented with one, two or more variant human D gene segments, wherein said substantially complete functional repertoire and the supplementary D gene segments are not found together in the germline genome of a human individual.
89. A population of non-human vertebrates (eg, mice or rats) comprising a substantially complete functional repertoire of human JH gene segment types supplemented with one, two or more variant human D gene segments, wherein said substantially complete functional repertoire and the supplementary D gene segments are not found together in the germline genome of a human individual.
90. The vertebrate or cell of clause 88 or the population of clause 89, comprising first and second D gene segments selected from
   D2-2ref and D2-2a; or
   D2-21ref and D2-21a; or
   D3-10ref and D3-10a; or
   D3-16ref and D3-16a; or
   D2-8ref and D2-8a; or
   D3-3ref and D3-3a; or
   D4-23ref and D4-23a; or
   D6-13ref and D6-13a; or
   D3-9ref and D3-9a; or
   D4-4ref and D4-4a; or
   D7-27ref and D7-27a;
   optionally wherein the first and/or second D gene segment is present in two or more copies.
91. The vertebrate, cell or population of clause90, comprising human gene segments D2-2ref and D2-2a; and D3-3ref and D3-3a; and optionally also D2-15.
92. A non-human vertebrate or vertebrate cell according to clause71, comprising a genome that comprises VH, D and JH gene repertoires comprising human gene segments, the D gene repertoire comprising one or more of
   a plurality of D2-2 gene segments provided by at least 2 different D2-2 gene segments in *cis* at the same Ig locus in said genome;
   a plurality of D2-21 gene segments provided by at least 2 different D2-21 gene segments in *cis* at the same Ig locus in said genome;
   a plurality of D3-10 gene segments provided by at least 2 different D3-10 gene segments in *cis* at the same Ig locus in said genome;
   a plurality of D3-16 gene segments provided by at least 2 different D3-16 gene segments in *cis* at the same Ig locus in said genome;
   a plurality of D2-8 gene segments provided by at least 2 different D2-8 gene segments in *cis* at the same Ig locus in said genome;
   a plurality of D3-3 gene segments provided by at least 2 different D3-3 gene segments in *cis* at the same Ig locus in said genome;
   a plurality of D4-23 gene segments provided by at least 2 different D4-23 gene segments in *cis* at the same Ig locus in said genome;
   a plurality of D6-13 gene segments provided by at least 2 different D6-13 gene segments in *cis* at the same Ig locus in said genome;
   a plurality of D3-9 gene segments provided by at least 2 different D3-9 gene segments in *cis* at the same Ig locus in said genome;
   a plurality of D4-4 gene segments provided by at least 2 different D4-4 gene segments in *cis* at the same Ig locus in said genome; and
   a plurality of D7-27 gene segments provided by at least 2 different D7-27 gene segments in *cis* at the same Ig locus in said genome;
   optionally wherein the D gene segments are derived from the genome sequence of two or more different human individuals.
93. A non-human vertebrate or vertebrate cell according to clause 71, comprising a genome that comprises VH, D and JH gene repertoires comprising human gene segments, the D gene repertoire comprising one or more of
   a plurality of D2-2 gene segments provided by at least 2 different D2-2 gene segments in *trans* in said genome;
   a plurality of D2-21 gene segments provided by at least 2 different D2-21 gene segments in *trans* in said genome;
   a plurality of D3-10 gene segments provided by at least 2 different D3-10 gene segments in *trans* in said genome;
   a plurality of D3-16 gene segments provided by at least 2 different D3-16 gene segments in *trans* in said genome;
   a plurality of D2-8 gene segments provided by at least 2 different D2-8 gene segments in *trans* in said genome;
   a plurality of D3-3 gene segments provided by at least 2 different D3-3 gene segments in *trans* in said genome;
   a plurality of D4-23 gene segments provided by at least 2 different D4-23 gene segments in *trans* in said genome;
   a plurality of D6-13 gene segments provided by at least 2 different D6-13 gene segments in *trans* in said genome;
   a plurality of D3-9 gene segments provided by at least 2 different D3-9 gene segments in *trans* in said genome;
   a plurality of D4-4 gene segments provided by at least 2 different D4-4 gene segments in *trans* in said genome; and
   a plurality of D7-27 gene segments provided by at least 2 different D7-27 gene segments in *trans* in said genome;
   optionally wherein the D gene segments are derived from the genome sequence of two or more different human individuals.
94. A non-human vertebrate or vertebrate cell (optionally an ES cell or B-cell), according to clause70, comprising a genome that comprises VH, D and JH gene repertoires comprising human gene segments, the D gene repertoire comprising one or more of
   a plurality of D2-2 gene segments provided by at least 3 different D2-2 gene segments;
   a plurality of D2-21 gene segments provided by at least 3 different D2-21 gene segments;
   a plurality of D3-10 gene segments provided by at least 3 different D3-10 gene segments;
   a plurality of D3-16 gene segments provided by at least 3 different D3-16 gene segments;
   a plurality of D2-8 gene segments provided by at least 3 different D2-8 gene segments;
   a plurality of D3-3 gene segments provided by at least 3 different D3-3 gene segments;
   a plurality of D4-23 gene segments provided by at least 3 different D4-23 gene segments;
   a plurality of D6-13 gene segments provided by at least 3 different D6-13 gene segments;
   a plurality of D3-9 gene segments provided by at least 3 different D3-9 gene segments;
   a plurality of D4-4 gene segments provided by at least 3 different D4-4 gene segments; and
   a plurality of D7-27 gene segments provided by at least 3 different D7-27 gene segments;
   optionally wherein the D gene segments are derived from the genome sequence of two or three different human individuals;
   optionally wherein at least 2 or 3 of said different gene segments are provided in *cis* at the same Ig locus in said genome.
95. The vertebrate or cell of clause 92, 93 or 94, wherein the different human individuals are from different human populations.
96. The vertebrate or cell of any one of clauses 92 to 95, wherein the individuals are not genetically related.
97. The vertebrate or cell of any one ofclauses92 to 96, wherein at least one of the different D segments is a synthetic mutant of a human germline D gene segment.
98. A non-human vertebrate or vertebrate cell (optionally an ES cell or B-cell) according to clause 74, comprising a genome comprising human VH, D and JH gene repertoires, the D gene repertoire comprising of one or more of
   a plurality of D2-2 gene segments provided by at least 2 different D2-2 gene ; optionally in *cis* in said genome;
   a plurality of D2-21 gene segments provided by at least 2 different D2-21 gene ; optionally in *cis* in said genome;
   a plurality of D3-10 gene segments provided by at least 2 different D3-10 gene ; optionally in *cis* in said genome;
   a plurality of D3-16 gene segments provided by at least 2 different D3-16 gene ; optionally in *cis* in said genome;
   a plurality of D2-8 gene segments provided by at least 2 different D2-8 gene ; optionally in *cis* in said genome;
   a plurality of D3-3 gene segments provided by at least 2 different D3-3 gene ; optionally in *cis* in said genome;
   a plurality of D4-23 gene segments provided by at least 2 different D4-23 gene ; optionally in *cis* in said genome;
   a plurality of D6-13 gene segments provided by at least 2 different D6-13 gene ; optionally in *cis* in said genome;
   a plurality of D3-9 gene segments provided by at least 2 different D3-9 gene ; optionally in *cis* in said genome;
   a plurality of D4-4 gene segments provided by at least 2 different D4-4 gene ; optionally in *cis* in said genome; and
   a plurality of D7-27 gene segments provided by at least 2 different D7-27 gene ; optionally in *cis* in said genome;
   wherein the D gene segments are derived from the genome sequence of different human individuals that are not genetically related over at least 3 generations.
99. The vertebrate or cell of clause 98, wherein the human individuals are from different human populations.
100. The vertebrate, cell or population of any one of clauses 70 to 99, wherein one or more of the D gene segments is a variant of a human germline D gene segment, wherein the variant gene segment encodes an amino acid sequence that differs by 1, 2 or 3 amino acids from the corresponding amino acid sequence encoded by the human germline D gene segment, provided in that said amino acid sequence encoded by the variant does not include a stop codon when said corresponding amino acid sequence does not include a stop codon.
101. The vertebrate, cell or population of clause 100, wherein said corresponding amino acid sequence encoded by the human germline D gene segment is a hydrophilic or hydrophobic sequence (according to J Mol Biol. 1997 Jul 25;270(4):587-97; Corbett SJ et *al*; Table 2).
102. The vertebrate, cell or population of clause 100 or 101, comprising said variant and said germline human D gene segments; optionally wherein the variant and germline human D gene segments are *cis* on the same chromosome.
103. The vertebrate, cell or population of any one of clauses 100 to 102, wherein germline human D gene segment is a D2, D3, D5 or D6 family gene segment; optionally a D2-2, D2-15, D3-3, D3-9, D3-10, D3-22, D5-5, D5-18, D6-6, D6-13, D6-19 gene segment.
104. The vertebrate, cell or population of any one of clauses 70 to 103, comprising a plurality of D2-2 gene segments, wherein the plurality comprises D2-2 gene segments that vary from each other at one or more nucleotide positions corresponding to positions
   106,382,687 and
   106,382,711
   on human chromosome 14.
105. The vertebrate, cell or population of clause 104, wherein the plurality comprises a human D2-2 gene segment comprising a thymine at a position corresponding to position 106,382,687 on human chromosome 14; and optionally no further mutation from the sequence of D2-2ref.
106. The vertebrate, cell or population of clause 104 or 105, wherein the plurality comprises a human D2-2 gene segment comprising a cytosine at a position corresponding to position 106,382,687 on human chromosome 14; and optionally no further mutation from the sequence of D2-2a.
107. The vertebrate, cell or population of any one of clauses 104 to 106, wherein the plurality comprises a human D2-2 gene segment comprising an adenine at a position corresponding to position 106,382,711 on human chromosome 14; and optionally no further mutation from the sequence of D2-2b.
108. The vertebrate, cell or population of any one of clauses 104 to 107, wherein the plurality comprises a human D2-2 gene segment comprising an thymine at a position corresponding to position 106,382,711 on human chromosome 14; and optionally no further mutation from the sequence of D2-2ref.
109. The vertebrate, cell or population of any one of clauses70 to 108, comprising a plurality of D7-27 gene segments, wherein the plurality comprises D7-27 gene segments that vary from each other at a nucleotide position corresponding to position 106,331,767 on human chromosome 14.
110. The vertebrate, cell or population of clause 109, wherein the plurality comprises a human D7-27 gene segment comprising a cytosine at a position corresponding to position 106,331,767 on human chromosome 14; and optionally no further mutation from the sequence of D7-27ref.
111. The vertebrate, cell or population ofclause 109 or 110, wherein the plurality comprises a human D7-27 gene segment comprising a guanine at a position corresponding to position 106,331,767 on human chromosome 14; and optionally no further mutation from the sequence of D7-27a.
112. The vertebrate, cell or population of any one of clauses 70 to 111, comprising a plurality of D4-23 gene segments, wherein the plurality comprises D4-23 gene segments that vary from each other at a nucleotide position corresponding to position 106,350,740 on human chromosome 14.
113. The vertebrate, cell or population of clause 112, wherein the plurality comprises a human D4-23 gene segment comprising an adenine at a position corresponding to position 106,350,740 on human chromosome 14; and optionally no further mutation from the sequence of D4-23ref.
114. The vertebrate, cell or population of clause 112 or 113, wherein the plurality comprises a human D4-23 gene segment comprising an guanine at a position corresponding to position 106,350,740 on human chromosome 14; and optionally no further mutation from the sequence of D4-23a.
115. The vertebrate, cell or population of any one of clauses 70 to 113, comprising a plurality of D2-21 gene segments, wherein the plurality comprises D2-21 gene segments that vary from each other at a nucleotide position corresponding to position 106,354,418 on human chromosome 14.
116. The vertebrate, cell or population of clause 115, wherein the plurality comprises a human D2-21 gene segment comprising an adenine at a position corresponding to position 106,354,418 on human chromosome 14; and optionally no further mutation from the sequence of D2-21ref.
117. The vertebrate, cell or population of clause 115 or 116, wherein the plurality comprises a human D2-21 gene segment comprising a guanine at a position corresponding to position 106,354,418 on human chromosome 14; and optionally no further mutation from the sequence of D2-21a.
118. The vertebrate, cell or population of any one of clauses 70 to 117, comprising a plurality of D3-16 gene segments, wherein the plurality comprises D3-16 gene segments that vary from each other at a nucleotide position corresponding to position 106,354,418 on human chromosome 14.
119. The vertebrate, cell or population of clause 118, wherein the plurality comprises a human D3-16 gene segment comprising a thymine at a position corresponding to position 106,361,515 on human chromosome 14; and optionally no further mutation from the sequence of D3-16ref.
120. The vertebrate, cell or population of clause 118 or 119, wherein the plurality comprises a human D3-16 gene segment comprising a cytosine at a position corresponding to position 106,361,515 on human chromosome 14; and optionally no further mutation from the sequence of D3-16a.
121. The vertebrate, cell or population of any one of clauses 70 to 120, comprising a plurality of D6-13 gene segments, wherein the plurality comprises D6-13 gene segments that vary from each other at a nucleotide position corresponding to position 106,367,013 on human chromosome 14.
122. The vertebrate, cell or population of clause 121, wherein the plurality comprises a human D6-13 gene segment comprising a thymine at a position corresponding to position 106,367,013 on human chromosome 14; and optionally no further mutation from the sequence of D6-13ref.
123. The vertebrate, cell or population ofclause 121 or 122, wherein the plurality comprises a human D6-13 gene segment comprising a cytosine at a position corresponding to position 106,367,013 on human chromosome 14; and optionally no further mutation from the sequence of D6-13a.
124. The vertebrate, cell or population of any one of clauses 70 to 123, comprising a plurality of D3-10 gene segments, wherein the plurality comprises D3-10 gene segments that vary from each other at one or more nucleotide positions corresponding to positions
   106,370,370 and
   106,370,371
   on human chromosome 14.
125. The vertebrate, cell or population of clause 124, wherein the plurality comprises a human D3-10 gene segment comprising a thymine at a position corresponding to position 106,370,370 on human chromosome 14; and optionally no further mutation from the sequence of D3-10ref.
126. The vertebrate, cell or population of clause 124 or 125, wherein the plurality comprises a human D3-10 gene segment comprising a cytosine at a position corresponding to position 106,370,370 on human chromosome 14; and optionally no further mutation from the sequence of D3-10a.
127. The vertebrate, cell or population of clause 124, 125 or 126 wherein the plurality comprises a human D3-10 gene segment comprising an adenine at a position corresponding to position 106,370,371 on human chromosome 14; and optionally no further mutation from the sequence of D3-10ref.
128. The vertebrate, cell or population of any one of clauses 124 to 127, wherein the plurality comprises a human D3-10 gene segment comprising a guanine at a position corresponding to position 106,370,371 on human chromosome 14; and optionally no further mutation from the sequence of D3-10b.
129. The vertebrate, cell or population of any one of clauses70 to 128, comprising a plurality of D3-9 gene segments, wherein the plurality comprises D3-9 gene segments that vary from each other at a nucleotide position corresponding to position 106,370,567 on human chromosome 14.
130. The vertebrate, cell or population of clause 129, wherein the plurality comprises a human D3-9 gene segment comprising an adenine at a position corresponding to position 106,370,567 on human chromosome 14; and optionally no further mutation from the sequence of D3-9ref.
131. The vertebrate, cell or population of clause 129 or 130, wherein the plurality comprises a human D3-9 gene segment comprising a thymine at a position corresponding to position 106,370,567 on human chromosome 14; and optionally no further mutation from the sequence of D3-9a.
132. The vertebrate, cell or population of any one of clauses 70 to 131, comprising a plurality of D2-8 gene segments, wherein the plurality comprises D2-8 gene segments that vary from each other at one or more nucleotide positions corresponding to positions
   106,373,085;
   106,373,086 and
   106,373,089
   on human chromosome 14.
133. The vertebrate, cell or population of clause 132, wherein the plurality comprises a human D2-8 gene segment comprising a cytosine at a position corresponding to position 106,373,085 on human chromosome 14.
134. The vertebrate, cell or population of clause 132 or 133, wherein the plurality comprises a human D2-8 gene segment comprising a thymine at a position corresponding to position 106,373,085 on human chromosome 14; and optionally no further mutation from the sequence of D2-8b.
135. The vertebrate, cell or population of clause 132, 133 or 134 wherein the plurality comprises a human D2-8 gene segment comprising a cytosine at a position corresponding to position 106,373,086 on human chromosome 14; and optionally no further mutation from the sequence of D2-8ref.
136. The vertebrate, cell or population of any one of clauses132 to 135, wherein the plurality comprises a human D2-8 gene segment comprising a thymine at a position corresponding to position 106,373,086 on human chromosome 14; and optionally no further mutation from the sequence of D2-8ref.
137. The vertebrate, cell or population of any one of clauses70 to 136, comprising a plurality of D4-4 gene segments, wherein the plurality comprises D4-4 gene segments that vary from each other at one or more nucleotide positions corresponding to positions
   106,379,086; and
   106,379,089
   on human chromosome 14.
138. The vertebrate, cell or population of clause 137, wherein the plurality comprises a D4-4 gene segment comprising a cytosine at a position corresponding to position 106,379,086 on human chromosome 14; and optionally no further mutation from the sequence of D4-4ref.
139. The vertebrate, cell or population of clause 137 or 138, wherein the plurality comprises a human D4-4 gene segment comprising a thymine at a position corresponding to position 106,379,086 on human chromosome 14; and optionally no further mutation from the sequence of D4-4a.
140. The vertebrate, cell or population of clause 137, 138 or 139 wherein the plurality comprises a human D4-4 gene segment comprising a cytosine at a position corresponding to position 106,379,089 on human chromosome 14; and optionally no further mutation from the sequence of D4-4ref or a cytosine at a position corresponding to position 106,379,086 on human chromosome 14.
141. The vertebrate, cell or population of any one of clauses 137 to 140, wherein the plurality comprises a human D4-4 gene segment comprising a thymine at a position corresponding to position 106,373,089 on human chromosome 14; and optionally no further mutation from the sequence of D4-4a.
142. The vertebrate, cell or population of any one of clauses70 to 141, comprising a plurality of D3-3 gene segments, wherein the plurality comprises D3-3 gene segments that vary from each other at one or more nucleotide positions corresponding to positions
   106,380,241; and
   106,380,246
   on human chromosome 14.
143. The vertebrate, cell or population of clause 142, wherein the plurality comprises a D3-3 gene segment comprising a thymine at a position corresponding to position 106,380,241 on human chromosome 14; and optionally no further mutation from the sequence of D3-3ref.
144. The vertebrate, cell or population of clause 142 or 143, wherein the plurality comprises a human D3-3 gene segment comprising a cytosine at a position corresponding to position 106,380,241 on human chromosome 14; and optionally no further mutation from the sequence of D3-3a.
145. The vertebrate, cell or population of clause 142, 143 or 144 wherein the plurality comprises a human D3-3 gene segment comprising an adenine at a position corresponding to position 106,380,246 on human chromosome 14; and optionally no further mutation from the sequence of D3-3ref.
146. The vertebrate, cell or population of any one of clauses 142 to 145, wherein the plurality comprises a human D3-3 gene segment comprising a thymine at a position corresponding to position 106,380,246 on human chromosome 14; and optionally no further mutation from the sequence of D3-3a.
147. A non-human vertebrate (eg, a mouse or rat) or a non-human vertebrate cell (eg, an ES cell or a B-cell) according toclause34, having a genome comprising at least 3 human JH gene segments of the same type (JH1, JH2, JH3, JH4, JH5 or JH6), wherein at least two of the human JH gene segments are variants that are not identical to each other.
148. A non-human vertebrate (eg, a mouse or rat) or a non-human vertebrate cell (eg, an ES cell or a B-cell) according toclause35, having a genome comprising at least 2 different non-endogenous JH gene segments of the same type (JH1, JH2, JH3, JH4, JH5 or JH6) *cis* at the same Ig locus.
149. A non-human vertebrate (eg, a mouse or rat) or a non-human vertebrate cell (eg, an ES cell or a B-cell) according to clause 36, having a genome comprising at least 2 different human JH gene segments of the same type (JH1, JH2, JH3, JH4, JH5 or JH6) *trans* at the same Ig locus; and optionally a third human JH gene segments of the same type, wherein the third JH is *cis* with one of said 2 different JH gene segments.
150. A population of non-human vertebrates (eg, mice or rats) comprising a repertoire of human JH gene segments according to clause37, wherein the plurality comprises at least 2 different human JH gene segments of the same type (JH1, JH2, JH3, JH4, JH5 or JH6), a first of said different JH gene segments is provided in the genome of a first vertebrate of the population, and a second of said different JH gene segments being provided in the genome of a second vertebrate of the population, wherein the genome of the first vertebrate does not comprise the second JH gene segment.
151. A non-human vertebrate (eg, a mouse or rat) or a non-human vertebrate cell (eg, an ES cell or a B-cell) according toclause38, having a genome comprising at least 2 different non-endogenous JH gene segments of the same type (JH1, JH2, JH3, JH4, JH5 or JH6), wherein the JH gene segments are derived from the genome sequence of different human individuals that are not genetically related over at least 3 generations.
152. A method of enhancing the human immunoglobulin gene diversity of a non-human vertebrate (eg, a mouse or rat) according toclause 39, the method comprising providing the vertebrate with a genome comprising at least 3 human JH gene segments of the same type (JH1, JH2, JH3, JH4, JH5 or JH6), wherein at least two of the human JH gene segments are variants that are not identical to each other.
153. A method of enhancing the immunoglobulin gene diversity of a non-human vertebrate (eg, a mouse or rat) according to clause 40, the method comprising providing the vertebrate with a genome comprising at least 2 different non-endogenous JH gene segments of the same type (JH1, JH2, JH3, JH4, JH5 or JH6) *cis* at the same Ig locus.
154. A method of enhancing the immunoglobulin gene diversity of a non-human vertebrate (eg, a mouse or rat) according to clause 41, the method comprising providing the vertebrate with a genome comprising at least 2 different human JH gene segments of the same type (JH1, JH2, JH3, JH4, JH5 or JH6) *trans* at the same Ig locus; and optionally a third human JH gene segments of the same type, wherein the third JH is *cis* with one of said 2 different JH gene segments.
155. A method of providing an enhanced human immunoglobulin JH gene segment repertoire according to clause 42, the method comprising providing a population of non-human vertebrates (eg, a mouse or rat) comprising a repertoire of human JH gene segments, wherein the method comprises providing at least 2 different human JH gene segments of the same type (JH1, JH2, JH3, JH4, JH5 or JH6), wherein a first of said different JH gene segments is provided in the genome of a first vertebrate of the population, and a second of said different JH gene segments is provided in the genome of a second vertebrate of the population, wherein the genome of the first vertebrate does not comprise the second JH gene segment.
156. A method of enhancing the human immunoglobulin gene diversity of a non-human vertebrate (eg, a mouse or rat) according to clause 43, the method comprising providing the vertebrate with a genome comprising at least 2 different non-endogenous JH gene segments of the same type (JH1, JH2, JH3, JH4, JH5 or JH6), wherein the JH gene segments are derived from the genome sequence of different human individuals that are not genetically related over at least 3 generations.
157. The vertebrate or cell of clause 147, 149 or 151, or the method of clause 152, 154, 155 or 156, wherein at least 2 or 3 of said different gene segments are provided *cis* at the same Ig locus in said genome.
158. The vertebrate or cell of clause 147, 148 or 149, or the method of clause 152, 153, 154 or 155, wherein the JH gene segments are derived from the genome sequence of different human individuals that are not genetically related over at least 3 generations.
159. The vertebrate or cell of clause 147, 148 or 149, or the method of clause 152, 153, 154 or 155, wherein the JH gene segments are derived from the genome sequence of two or more different human individuals; optionally wherein the different human individuals are from different human populations.
160. The vertebrate, cell or method of clause 159, wherein the individuals are not genetically related.
161. The vertebrate, cell or method of any one of clauses 147 to 160, wherein at least one of the different JH segments is a synthetic mutant of a human germline JH gene segment.
162. A method of enhancing the human immunoglobulin gene diversity of a non-human vertebrate (eg, a mouse or rat), the method comprising providing the vertebrate with a genome comprising at least 2 human JH gene segments of the same type (JH1, JH2, JH3, JH4, JH5 or JH6), wherein the JH gene segments are derived from the genome sequence of different human individuals that are not genetically related over at least 3 generations; optionally wherein at least 2 or 3 of said different gene segments are provided at the same IgH locus in said genome.
163. The vertebrate or cell of any one of clauses 147 to 149 and 151, wherein the genome comprises a substantially complete functional repertoire of human JH gene segment types supplemented with one, two or more human JH gene segments, wherein said substantially complete functional repertoire and the supplementary JH gene segments are not found together in the germline genome of a human individual.
164. The population of clause 150, wherein the population comprises a substantially complete functional repertoire of human JH gene segment types supplemented with one, two or more human JH gene segments, wherein said substantially complete functional repertoire and the supplementary JH gene segments are not found together in the germline genome of a human individual.
165. A non-human vertebrate (eg, a mouse or rat) or a non-human cell (eg, an ES cell or a B-cell) having a genome comprising a substantially complete functional repertoire of human JH gene segment types supplemented with one, two or more human JH gene segments, wherein said substantially complete functional repertoire and the supplementary JH gene segments are not found together in the germline genome of a human individual.
166. A population of non-human vertebrates (eg, mice or rats) comprising a substantially complete functional repertoire of human JH gene segment types supplemented with one, two or more human JH gene segments, wherein said substantially complete functional repertoire and the supplementary JH gene segments are not found together in the germline genome of a human individual.
167. The vertebrate of clause 165 or the population of clause 166, wherein at least one of said JH gene segments is SEQ ID NO: 1, 2, 3 or 4.
168. The vertebrate or population of clause 167, wherein at least one of said JH gene segments is SEQ ID NO: 1 and at least one, two or more of said supplementary JH gene segments is a variant according to any one of clauses 178 to 196.
169. The vertebrate or population of clause 167 or 168, wherein at least one of said JH gene segments is SEQ ID NO: 2 and at least one, two or more of said supplementary JH gene segments is a variant according to any one of clauses 198 to 209.
170. The vertebrate or population of clause 167, 168 or 169, wherein at least one of said JH gene segments is SEQ ID NO: 2 and at least one, two or more of said supplementary JH gene segments is a variant according to any one ofclauses 212 to 217.
171. A non-human vertebrate or vertebrate cell according to clause 148, comprising a genome that comprises VH, D and JH gene repertoires comprising human gene segments, the JH gene repertoire comprising
   a plurality of JH1 gene segments provided by at least 2 different JH1 gene segments in *cis* at the same Ig locus in said genome;
   a plurality of JH2 gene segments provided by at least 2 different JH2 gene segments in *cis* at the same Ig locus in said genome;
   a plurality of JH3 gene segments provided by at least 2 different JH3 gene segments in *cis* at the same Ig locus in said genome;
   a plurality of JH4 gene segments provided by at least 2 different JH4 gene segments in *cis* at the same Ig locus in said genome;
   a plurality of JH5 gene segments provided by at least 2 different JH5 gene segments in *cis* at the same Ig locus in said genome; or
   a plurality of JH6 gene segments provided by at least 2 different JH6 gene segments in *cis* at the same Ig locus in said genome;
   optionally wherein the JH gene segments are derived from the genome sequence of two or more different human individuals.
172. A non-human vertebrate or vertebrate cell (optionally an ES cell or B-cell), according to clause 147, comprising a genome that comprises VH, D and JH gene repertoires comprising human gene segments, the JH gene repertoire comprising
   a plurality of JH1 gene segments provided by at least 3 different JH1 gene segments;
   a plurality of JH2 gene segments provided by at least 3 different JH2 gene segments;
   a plurality of JH3 gene segments provided by at least 3 different JH3 gene segments;
   a plurality of JH4 gene segments provided by at least 3 different JH4 gene segments;
   a plurality of JH5 gene segments provided by at least 3 different JH5 gene segments; or
   a plurality of JH6 gene segments provided by at least 3 different JH6 gene segments;
   optionally wherein the JH gene segments are derived from the genome sequence of two or three different human individuals;
   optionally wherein at least 2 or 3 of said different gene segments are provided in *cis* at the same Ig locus in said genome.
173. The vertebrate or cell of clause 171 or 172, wherein the different human individuals are from different human populations.
174. The vertebrate or cell of any one of clauses 171 to 173, wherein the individuals are not genetically related.
175. The vertebrate or cell of any one of clauses 171 to 174, wherein at least one of the different JH segments is a synthetic mutant of a human germline JH gene segment.
176. A non-human vertebrate or vertebrate cell (optionally an ES cell or B-cell) according to clause 151, comprising a genome comprising human VH, D and JH gene repertoires, the JH gene repertoire comprising
   a plurality of JH1 gene segments provided by at least 2 different human JH1 gene segments, optionally in *cis* at the same Ig locus in said genome;
   a plurality of JH2 gene segments provided by at least 2 different human JH2 gene segments, optionally in *cis* at the same Ig locus in said genome;
   a plurality of JH3 gene segments provided by at least 2 different human JH3 gene segments, optionally in *cis* at the same Ig locus in said genome;
   a plurality of JH4 gene segments provided by at least 2 different human JH4 gene segments, optionally in *cis* at the same Ig locus in said genome;
   a plurality of JH5 gene segments provided by at least 2 different human JH5 gene segments, optionally in *cis* at the same Ig locus in said genome; or
   a plurality of JH6 gene segments provided by at least 2 different human JH6 gene segments, optionally in *cis* at the same Ig locus in said genome;
   wherein the JH gene segments are derived from the genome sequence of different human individuals that are not genetically related over at least 3 generations.
177. The vertebrate or cell of clause 176, wherein the human individuals are from different human populations.
178. The vertebrate, cell or population of any one of clauses 147 to 151, 157 to 161 and 163 to 177, comprising a plurality of JH5 gene segments, wherein the plurality comprises a human JH5 gene variant of SEQ ID NO: 1, wherein the variant comprises a nucleotide mutation at one or more positions corresponding to positions
   106,330,024
   106,330,027
   106,330,032
   106,330,041
   106,330,044
   106,330,045
   106,330,062
   106,330,063
   106,330,065
   106,330,066
   106,330,067
   106,330,068 and
   106,330,071
   on human chromosome 14.
179. The vertebrate, cell or population of clause 178, wherein the plurality comprises a human JH5 gene variant of SEQ ID NO: 1, wherein the variant comprises a guanine at a position corresponding to position 106,330,067 on human chromosome 14; and optionally no further mutation from the sequence of SEQ ID NO: 1.
180. The vertebrate, cell or population of clause 179, wherein the variant comprises additionally a mutation at a position corresponding to (i) position 106,330,071 on human chromosome 14 (optionally the additional mutation being a guanine); (ii) position 106,330,066 on human chromosome 14 (optionally the additional mutation being a guanine); and/or (iii) position 106,330,068 on human chromosome 14 (optionally the additional mutation being a thymine).
181. The vertebrate, cell or population of clause 178, 179 or 180, wherein the plurality comprises a human JH5 gene variant of SEQ ID NO: 1, wherein the variant comprises a guanine at a position corresponding to position 106,330,071 on human chromosome 14; and optionally no further mutation from the sequence of SEQ ID NO: 1.
182. The vertebrate, cell or population of clause 181, wherein the variant comprises additionally a mutation at a position corresponding to (i) position 106,330,063 on human chromosome 14 (optionally the additional mutation being an adenine); and/or (ii) position 106,330,067 on human chromosome 14 (optionally the additional mutation being a guanine).
183. The vertebrate, cell or population of clause 178 to 182, wherein the plurality comprises a human JH5 gene variant of SEQ ID NO: 1, wherein the variant comprises a cytosine at a position corresponding to position 106,330,045 on human chromosome 14; and optionally no further mutation from the sequence of SEQ ID NO: 1.
184. The vertebrate, cell or population of clause 178 to 183, wherein the plurality comprises a human JH5 gene variant of SEQ ID NO: 1, wherein the variant comprises an adenine at a position corresponding to position 106,330,044 on human chromosome 14; and optionally no further mutation from the sequence of SEQ ID NO: 1.
185. The vertebrate, cell or population of clause 184, wherein the variant comprises additionally a mutation at a position corresponding to (i) position 106,330,066 on human chromosome 14 (optionally the additional mutation being a guanine); and/or (ii) position 106,330,068 on human chromosome 14 (optionally the additional mutation being a thymine).
186. The vertebrate, cell or population of clause 178 to 185, wherein the plurality comprises a human JH5 gene variant of SEQ ID NO: 1, wherein the variant comprises a guanine at a position corresponding to position 106,330,066 on human chromosome 14; and optionally no further mutation from the sequence of SEQ ID NO: 1.
187. The vertebrate, cell or population of clause 186, wherein the variant comprises additionally a mutation at a position corresponding to (i) position 106,330,067 on human chromosome 14 (optionally the additional mutation being a guanine); and/or (ii) position 106,330,068 on human chromosome 14 (optionally the additional mutation being a thymine).
188. The vertebrate, cell or population of clause 178 to 185, wherein the plurality comprises a human JH5 gene variant of SEQ ID NO: 1, wherein the variant comprises a thymine at a position corresponding to position 106,330,068 on human chromosome 14; and optionally no further mutation from the sequence of SEQ ID NO: 1.
189. The vertebrate, cell or population of claim 188, wherein the variant comprises additionally a mutation at a position corresponding to (i) position 106,330,067 on human chromosome 14 (optionally the additional mutation being a guanine); and/or (ii) position 106,330,066 on human chromosome 14 (optionally the additional mutation being a guanine).
190. The vertebrate, cell or population of clause 178 to 189, wherein the plurality comprises a human JH5 gene variant of SEQ ID NO: 1, wherein the variant comprises a cytosine at a position corresponding to position 106,330,027 on human chromosome 14; and optionally no further mutation from the sequence of SEQ ID NO: 1.
191. The vertebrate, cell or population of clause 178 to 190, wherein the plurality comprises a human JH5 gene variant of SEQ ID NO: 1, wherein the variant comprises an adenine at a position corresponding to position 106,330,024 on human chromosome 14; and optionally no further mutation from the sequence of SEQ ID NO: 1.
192. The vertebrate, cell or population of clause 178 to 191, wherein the plurality comprises a human JH5 gene variant of SEQ ID NO: 1, wherein the variant comprises a thymine at a position corresponding to position 106,330,032 on human chromosome 14; and optionally no further mutation from the sequence of SEQ ID NO: 1.
193. The vertebrate, cell or population of clause 178 to 192, wherein the plurality comprises a human JH5 gene variant of SEQ ID NO: 1, wherein the variant comprises a thymine at a position corresponding to position 106,330,041 on human chromosome 14; and optionally no further mutation from the sequence of SEQ ID NO: 1.
194. The vertebrate, cell or population of clause 178 to 193, wherein the plurality comprises a human JH5 gene variant of SEQ ID NO: 1, wherein the variant comprises an adenine or thymine at a position corresponding to position 106,330,063 on human chromosome 14; and optionally no further mutation from the sequence of SEQ ID NO: 1.
195. The vertebrate, cell or population of clause 194, wherein the variant comprises additionally a mutation at a position corresponding to position 106,330,071 on human chromosome 14 (optionally the additional mutation being a guanine).
196. The vertebrate, cell or population of clause 178 to 195, wherein the plurality comprises a human JH5 gene variant of SEQ ID NO: 1, wherein the variant comprises a cytosine at a position corresponding to position 106,330,062 on human chromosome 14; and optionally no further mutation from the sequence of SEQ ID NO: 1.
197. The vertebrate, cell or population of clause 178 to 196, wherein the genome comprises SEQ ID NO:1; optionally in *cis* at the same Ig locus as one, two or more of the variants.
198. The vertebrate, cell or population of any one of clauses 147 to 151, 157 to 161 and 96 to 130, comprising a plurality of JH6 gene segments, wherein the plurality comprises a human JH6 gene variant of SEQ ID NO: 2, wherein the variant comprises a nucleotide mutation at one or more positions corresponding to positions
   106,329,411
   106,329,413
   106,329,414
   106,329,417
   106,329,419
   106,329,426
   106,329,434
   106,329,435, and
   106,329,468
   on human chromosome 14.
199. The vertebrate, cell or population of clause 198, comprising a plurality of JH6 gene segments, wherein the plurality comprises a human JH6 gene variant of SEQ ID NO: 2, wherein the variant comprises a guanine at a position corresponding to position 106,329,435 on human chromosome 14; and optionally no further mutation from the sequence of SEQ ID NO: 2.
200. The vertebrate, cell or population of clause 199, wherein the variant comprises additionally a mutation at a position corresponding to (i) position 106,329,468 on human chromosome 14 (optionally the additional mutation being a guanine); (ii) position 106,329,419 on human chromosome 14 (optionally the additional mutation being an adenine); (iii) position 106,329,434 on human chromosome 14 (optionally the additional mutation being a cytosine) and/or position 106,329,414 on human chromosome 14 (optionally the additional mutation being a guanine); (iv) position 106,329,426 on human chromosome 14 (optionally the additional mutation being an adenine); (v) position 106,329,413 on human chromosome 14 (optionally the additional mutation being an adenine); (vi) position 106,329,417 on human chromosome 14 (optionally the additional mutation being a thymine); (vii) position 106,329,411 on human chromosome 14 (optionally the additional mutation being a thymine); (viii) position 106,329,451 on human chromosome 14 (optionally the additional mutation being an adenine); (ix) position 106,329,452 on human chromosome 14 (optionally the additional mutation being a cytosine); and/or (x) position 106,329,453 on human chromosome 14 (optionally the additional mutation being a cytosine).
201. The vertebrate, cell or population of clause 200, wherein the variant comprises additionally mutations at positions corresponding to position 106,329,451 on human chromosome 14, the additional mutation being an adenine; position 106,329,452 on human chromosome 14, the additional mutation being a cytosine; and position 106,329,453 on human chromosome 14, the additional mutation being a cytosine.
202. The vertebrate, cell or population of any one of clauses 147 to 151, 157 to 161 and 163 to 201, comprising a plurality of JH6 gene segments,, wherein the plurality comprises a human JH6 gene variant of SEQ ID NO: 2, wherein the variant comprises a guanine at a position corresponding to position 106,329,468 on human chromosome 14; and optionally no further mutation from the sequence of SEQ ID NO: 2.
203. The vertebrate, cell or population of clause202, wherein the variant comprises additionally a mutation at a position corresponding to position 106,329,435 on human chromosome 14 (optionally the additional mutation being a guanine).
204. The vertebrate, cell or population of any one of clauses 147 to 151, 157 to 161 and 163 to 203, comprising a plurality of JH6 gene segments,, wherein the plurality comprises a human JH6 gene variant of SEQ ID NO: 2, wherein the variant comprises a thymine at a position corresponding to position 106,329,417 on human chromosome 14; and optionally no further mutation from the sequence of SEQ ID NO: 2.
205. The vertebrate, cell or population of clause 204, wherein the variant comprises additionally a mutation at a position corresponding to position 106,329,435 on human chromosome 14 (optionally the additional mutation being a guanine).
206. The vertebrate, cell or population of any one ofclauses 147 to 151, 157 to 161 and 163 to 205, comprising a plurality of JH6 gene segments,, wherein the plurality comprises a human JH6 gene variant of SEQ ID NO: 2, wherein the variant comprises a cytosine at a position corresponding to position 106,329,434 on human chromosome 14; and optionally no further mutation from the sequence of SEQ ID NO: 2.
207. The vertebrate, cell or population of clause206, wherein the variant comprises additionally a mutation at a position corresponding to (i) position 106,329,414 on human chromosome 14 (optionally the additional mutation being a guanine); and/or (ii) position 106,329,435 on human chromosome 14 (optionally the additional mutation being a guanine).
208. The vertebrate, cell or population of any one of clauses 147 to 151, 157 to 161 and 163 to 207, comprising a plurality of JH6 gene segments, wherein the plurality comprises a human JH6 gene variant of SEQ ID NO: 2, wherein the variant comprises a thymine at a position corresponding to position 106,329,411 on human chromosome 14; and optionally no further mutation from the sequence of SEQ ID NO: 2.
209. The vertebrate, cell or population of clause 208, wherein the variant comprises additionally a mutation at a position corresponding to position 106,329,435 on human chromosome 14 (optionally the additional mutation being a guanine).
210. The vertebrate, cell or population of any one of clauses 147 to 151, 157 to 161 and 163 to 209, comprising a plurality of JH6 gene segments, wherein the plurality comprises a human JH6 gene variant that is an antisense sequence of the variant of any one of claims 107 to 118.
211. The vertebrate, cell or population of any one of clauses198 to 210, wherein the genome comprises SEQ ID NO:2; optionally *cis* at the same Ig locus as one, two or more of the JH6 variants.
212. The vertebrate, cell or population of any one of clauses 147 to 151, 157 to 161 and 163 to 211, comprising a plurality of JH2 gene segments, wherein the plurality comprises a human JH2 gene variant of SEQ ID NO: 3, wherein the variant comprises a nucleotide mutation at one or more positions corresponding to positions
   106,331,455
   106,331,453, and
   106,331,409
   on human chromosome 14.
213. The vertebrate, cell or population of clause212, comprising said plurality of JH2 gene segments, wherein the plurality comprises a human JH2 gene variant of SEQ ID NO: 3, wherein the variant comprises a guanine at a position corresponding to position 106,331,455 on human chromosome 14; and optionally no further mutation from the sequence of SEQ ID NO: 3.
214. The vertebrate, cell or population of clause 213, wherein the variant comprises additionally a mutation at a position corresponding to (i) position 106,331,453 on human chromosome 14 (optionally the additional mutation being an adenine); and/or (ii) position 106,331,409 on human chromosome 14 (optionally the additional mutation being an adenine); (iii) position 106,329,434 on human chromosome 14 (optionally the additional mutation being an adenine).
215. The vertebrate, cell or population of any one of clauses 147 to 151, 157 to 161 and 163 to 214, comprising a plurality of JH2 gene segments, wherein the plurality comprises a human JH2 gene variant of SEQ ID NO: 3, wherein the variant comprises an adenine at a position corresponding to position 106,331,453 on human chromosome 14; and optionally no further mutation from the sequence of SEQ ID NO: 3.
216. The vertebrate, cell or population of clause215, wherein the variant comprises additionally a mutation at a position corresponding to position 106,331,409 on human chromosome 14 (optionally the additional mutation being an adenine).
217. The vertebrate, cell or population of any one of clauses 147 to 151, 157 to 161 and 163 to 216, comprising a plurality of JH2 gene segments, wherein the plurality comprises a human JH2 gene variant of SEQ ID NO: 3, wherein the variant comprises an adenine at a position corresponding to position 106,331,409 on human chromosome 14; and optionally no further mutation from the sequence of SEQ ID NO: 3.
218. The vertebrate, cell or population of any one of clauses 147 to 151, 157 to 161 and 163 to 217, comprising a plurality of JH2 gene segments, wherein the plurality comprises a human JH2 gene variant that is an antisense sequence of the variant of any one of clauses 188 to 193.
219. The vertebrate, cell or population of any one of clauses 212 to 218, wherein the genome comprises SEQ ID NO:3; optionally *cis* at the same Ig locus as one, two or more of the JH2 variants.
220. The vertebrate, cell or population of any one of clauses 147 to 151, 157 to 161 and 163 to 219, wherein the genome comprises two or more different JH gene segments selected from SEQ ID NOs: 1 to 3 and variants according to any one of clauses 178 to 219; optionally wherein said JH gene segments are *cis* at the same immunoglobulin Ig locus.
221. A non-human vertebrate (eg, a mouse or rat) or a non-human vertebrate cell (eg, an ES cell or a B-cell) according to clause 34, having a genome comprising at least 3 human JL gene segments of the same type (eg, Jκ1), wherein at least two of the human JL gene segments are variants that are not identical to each other.
222. A non-human vertebrate (eg, a mouse or rat) or a non-human vertebrate cell (eg, an ES cell or a B-cell) according to clause 35, having a genome comprising at least 2 different non-endogenous JL gene segments of the same type (eg, Jκ1) *cis* at the same Ig locus.
223. A non-human vertebrate (eg, a mouse or rat) or a non-human vertebrate cell (eg, an ES cell or a B-cell) according to clause36, having a genome comprising at least 2 different human JL gene segments of the same type (eg, Jκ1) *trans* at the same Ig locus; and optionally a third human JL gene segment of the same type, wherein the third JL is *cis* with one of said 2 different JL gene segments.
224. A population of non-human vertebrates (eg, mice or rats) comprising a repertoire of human JL gene segments according to clause37, wherein the plurality comprises at least 2 different human JL gene segments of the same type (eg, Jκ1), a first of said different JL gene segments is provided in the genome of a first vertebrate of the population, and a second of said different JL gene segments being provided in the genome of a second vertebrate of the population, wherein the genome of the first vertebrate does not comprise the second JL gene segment.
225. A non-human vertebrate (eg, a mouse or rat) or a non-human vertebrate cell (eg, an ES cell or a B-cell) according to clause38, having a genome comprising at least 2 different non-endogenous JL gene segments of the same type (eg, Jκ1), wherein the JL gene segments are derived from the genome sequence of different human individuals that are not genetically related over at least 3 generations.
226. A method of enhancing the human immunoglobulin gene diversity of a non-human vertebrate (eg, a mouse or rat) according to clause39, the method comprising providing the vertebrate with a genome comprising at least 3 human JL gene segments of the same type (eg, Jκ1), wherein at least two of the human JL gene segments are variants that are not identical to each other.
227. A method of enhancing the immunoglobulin gene diversity of a non-human vertebrate (eg, a mouse or rat) according to clause 40, the method comprising providing the vertebrate with a genome comprising at least 2 different non-endogenous JL gene segments of the same type (eg, Jκ1) *cis* at the same Ig locus.
228. A method of enhancing the immunoglobulin gene diversity of a non-human vertebrate (eg, a mouse or rat) according to clause 41, the method comprising providing the vertebrate with a genome comprising at least 2 different human JL gene segments of the same type(eg, Jκ1) *trans* at the same Ig locus; and optionally a third human JL gene segment of the same type, wherein the third JL is *cis* with one of said 2 different JL gene segments.
229. A method of providing an enhanced human immunoglobulin JL gene segment repertoire according toclause 42, the method comprising providing a population of non-human vertebrates (eg, a mouse or rat) comprising a repertoire of human JL gene segments, wherein the method comprises providing at least 2 different human JL gene segments of the same type (eg, Jκ1), wherein a first of said different JL gene segments is provided in the genome of a first vertebrate of the population, and a second of said different JL gene segments is provided in the genome of a second vertebrate of the population, wherein the genome of the first vertebrate does not comprise the second JL gene segment.
230. A method of enhancing the human immunoglobulin gene diversity of a non-human vertebrate (eg, a mouse or rat) according to clause 43, the method comprising providing the vertebrate with a genome comprising at least 2 different non-endogenous JL gene segments of the same type (eg, Jκ1), wherein the JL gene segments are derived from the genome sequence of different human individuals that are not genetically related over at least 3 generations.
231. The vertebrate or cell of clause221, 223 or 225, or the method of clause 226, 228, 229 or 230, wherein at least 2 or 3 of said different gene segments are provided *cis* at the same Ig locus in said genome.
232. The vertebrate or cell of clause 221, 222 or 223, or the method of clause 226, 227, 228 or 229, wherein the JL gene segments are derived from the genome sequence of different human individuals that are not genetically related over at least 3 generations.
233. The vertebrate or cell of clause 221, 222 or 223, or the method of clause 226, 227, 228 or 229, wherein the JL gene segments are derived from the genome sequence of two or more different human individuals; optionally wherein the different human individuals are from different human populations.
234. The vertebrate, cell or method of clause 233, wherein the individuals are not genetically related.
235. The vertebrate, cell or method of any one of clauses 221 to 234, wherein at least one of the different JL segments is a synthetic mutant of a human germline JL gene segment.
236. A method of enhancing the human immunoglobulin gene diversity of a non-human vertebrate (eg, a mouse or rat), the method comprising providing the vertebrate with a genome comprising at least 2 human JL gene segments of the same type (eg, Jκ1), wherein the JL gene segments are derived from the genome sequence of different human individuals that are not genetically related over at least 3 generations; optionally wherein at least 2 or 3 of said different gene segments are provided at the same IgL locus in said genome.
237. The vertebrate or cell of any one ofclause clauses 221 to 223 and 225, wherein the genome comprises a substantially complete functional repertoire of human Jκ and/or Jλ gene segment types supplemented with one, two or more human Jκ and/or Jλ gene segments respectively, wherein said substantially complete functional repertoire and the supplementary gene segments are not found together in the germline genome of a human individual.
238. The population of clause224, wherein the population comprises a substantially complete functional repertoire of human JL gene segment types supplemented with one, two or more human Jκ and/or Jλ gene segments respectively, wherein said substantially complete functional repertoire and the supplementary gene segments are not found together in the germline genome of a human individual.
239. A non-human vertebrate (eg, a mouse or rat) or a non-human cell (eg, an ES cell or a B-cell) having a genome comprising a substantially complete functional repertoire of human Jκ and/or Jλ gene segment types supplemented with one, two or more human Jκ and/or Jλ gene segments respectively, wherein said substantially complete functional repertoire and the supplementary gene segments are not found together in the germline genome of a human individual.
240. A population of non-human vertebrates (eg, mice or rats) comprising a substantially complete functional repertoire of human Jκ and/or Jλ gene segment types supplemented with one, two or more human Jκ and/or Jλ gene segments respectively, wherein said substantially complete functional repertoire and the supplementary gene segments are not found together in the germline genome of a human individual.
241. A non-human vertebrate or vertebrate cell according to clause 222, comprising a genome that comprises VL and JL gene repertoires comprising human gene segments, the JL gene repertoire comprising
   a plurality of human Jκ1 gene segments provided by at least 2 different human Jκ1 gene segments in *cis* at the same Ig locus in said genome;
   a plurality of human Jκ2 gene segments provided by at least 2 different human Jκ1 gene segments in *cis* at the same Ig locus in said genome;
   a plurality of human Jκ3 gene segments provided by at least 2 different human Jκ1 gene segments in *cis* at the same Ig locus in said genome;
   a plurality of human Jκ4 gene segments provided by at least 2 different human Jκ1 gene segments in *cis* at the same Ig locus in said genome;
   a plurality of human Jκ5 gene segments provided by at least 2 different human Jκ1 gene segments in *cis* at the same Ig locus in said genome;
   a plurality of human Jλ1 gene segments provided by at least 2 different human Jλ1 gene segments in *cis* at the same Ig locus in said genome;
   a plurality of human Jλ2 gene segments provided by at least 2 different human Jλ2 gene segments in *cis* at the same Ig locus in said genome;
   a plurality of human Jλ3 gene segments provided by at least 2 different human Jλ3 gene segments in *cis* at the same Ig locus in said genome;
   a plurality of human Jλ4 gene segments provided by at least 2 different human Jλ4 gene segments in *cis* at the same Ig locus in said genome;
   a plurality of human Jλ5 gene segments provided by at least 2 different human Jλ5 gene segments in *cis* at the same Ig locus in said genome;
   a plurality of human Jλ6 gene segments provided by at least 2 different human Jλ6 gene segments in *cis* at the same Ig locus in said genome; or
   a plurality of human Jλ7 gene segments provided by at least 2 different human Jλ7 gene segments in *cis* at the same Ig locus in said genome;
   optionally wherein the JL gene segments are derived from the genome sequence of two or more different human individuals.
242. A non-human vertebrate or vertebrate cell (optionally an ES cell or B-cell), according to clause 221, comprising a genome that comprises VL and JL gene repertoires comprising human gene segments, the JL gene repertoire comprising
   a plurality of human Jκ1 gene segments provided by at least 3 different human Jκ1 gene segments;
   a plurality of human Jκ2 gene segments provided by at least 3 different human Jκ1 gene segments;
   a plurality of human Jκ3 gene segments provided by at least 3 different human Jκ1 gene segments;
   a plurality of human Jκ4 gene segments provided by at least 3 different human Jκ1 gene segments;
   a plurality of human Jκ5 gene segments provided by at least 3 different human Jκ1 gene segments;
   a plurality of human Jλ1 gene segments provided by at least 3 different human Jλ1 gene segments;
   a plurality of human Jλ2 gene segments provided by at least 3 different human Jλ2 gene segments;
   a plurality of human Jλ3 gene segments provided by at least 3 different human Jλ3 gene segments;
   a plurality of human Jλ4 gene segments provided by at least 3 different human Jλ4 gene segments;
   a plurality of human Jλ5 gene segments provided by at least 3 different human Jλ5 gene segments;
   a plurality of human Jλ6 gene segments provided by at least 3 different human Jλ6 gene segments; or
   a plurality of human Jλ7 gene segments provided by at least 3 different human Jλ7 gene segments;
   optionally wherein the JL gene segments are derived from the genome sequence of two or three different human individuals;
   optionally wherein at least 2 or 3 of said different gene segments are provided in *cis* at the same Ig locus in said genome.
243. The vertebrate or cell of clause 241 or 242, wherein the different human individuals are from different human populations.
244. The vertebrate or cell of any one of clauses 241 to 243, wherein the individuals are not genetically related.
245. The vertebrate or cell of any one of clauses 241 to 244, wherein at least one of the different JL segments is a synthetic mutant of a human germline JL gene segment.
246. A non-human vertebrate or vertebrate cell (optionally an ES cell or B-cell) according to clause 225, comprising a genome comprising human VL and JL gene repertoires, the JL gene repertoire comprising
   a plurality of human Jκ1 gene segments provided by at least 2 different human Jκ1 gene segments, optionally in *cis* at the same Ig locus in said genome;
   a plurality of human Jκ2 gene segments provided by at least 2 different human Jκ1 gene segments, optionally in *cis* at the same Ig locus in said genome;
   a plurality of human Jκ3 gene segments provided by at least 2 different human Jκ1 gene segments, optionally in *cis* at the same Ig locus in said genome;
   a plurality of human Jκ4 gene segments provided by at least 2 different human Jκ1 gene segments, optionally in *cis* at the same Ig locus in said genome;
   a plurality of human Jκ5 gene segments provided by at least 2 different human Jκ1 gene segments, optionally in *cis* at the same Ig locus in said genome;
   a plurality of human Jλ1 gene segments provided by at least 2 different human Jλ1 gene segments, optionally in *cis* at the same Ig locus in said genome;
   a plurality of human Jλ2 gene segments provided by at least 2 different human Jλ2 gene segments, optionally in *cis* at the same Ig locus in said genome;
   a plurality of human Jλ3 gene segments provided by at least 2 different human Jλ3 gene segments, optionally in *cis* at the same Ig locus in said genome;
   a plurality of human Jλ4 gene segments provided by at least 2 different human Jλ4 gene segments, optionally in *cis* at the same Ig locus in said genome;
   a plurality of human Jλ5 gene segments provided by at least 2 different human Jλ5 gene segments, optionally in *cis* at the same Ig locus in said genome;
   a plurality of human Jλ6 gene segments provided by at least 2 different human Jλ6 gene segments, optionally in *cis* at the same Ig locus in said genome; or
   a plurality of human Jλ7 gene segments provided by at least 2 different human Jλ7 gene segments, optionally in *cis* at the same Ig locus in said genome;
   wherein the JL gene segments are derived from the genome sequence of different human individuals that are not genetically related over at least 3 generations.
247. The vertebrate or cell of clause 246, wherein the human individuals are from different human populations.
248. A method of producing an antibody heavy chain, the method comprising
   (a) providing an antigen-specific heavy chain variable domain; and
   (b) combining the variable domain with a human heavy chain constant region to produce an antibody heavy chain comprising (in N- to C-terminal direction) the variable domain and the constant region;
   wherein
   the human heavy chain constant region is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region.
249. The method of clause 248, wherein the variable domain is a human variable domain.
250. The method of any clause 248 or 249, wherein the variable domain has previously been selected from a non-human vertebrate that has been immunised with the antigen.
251. The method of any one ofclauses 248 to 250, comprising providing an expression vector comprising a nucleotide sequence encoding the constant region; inserting a nucleotide sequence encoding the variable domain into the vector 5' of the constant region sequence; inserting the vector into a host cell and expressing the heavy chain by the host cell; the method further comprising isolating a heavy chain (eg, as part of an antibody) comprising the variable domain and the human constant region.
252. The method of any one of clauses 248 to 251, further comprising obtaining a nucleotide sequence encoding the heavy chain.
253. An antibody comprising a human heavy chain, the heavy chain comprising a variable domain that is specific for an antigen and a constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region.
254. A polypeptide comprising (in N- to C- terminal direction) a leader sequence, a human variable domain that is specific for an antigen and a human constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region; wherein (i) the leader sequence is not the native human variable domain leader sequence; and/or (ii) the variable domain comprises mouse AID-pattern somatic mutations or mouse terminal deoxynucleotidyl transferase (TdT)- pattern junctional mutations.
255. A nucleotide sequence encoding (in 5' to 3' direction) a leader sequence and a human antibody heavy chain, the heavy chain comprising a variable domain that is specific for an antigen and a constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region; and the leader sequence being operable for expression of the heavy chain and wherein the leader sequence is not the native human variable domain leader sequence.
256. A nucleotide sequence encoding (in 5' to 3' direction) a promoter and a human antibody heavy chain, the heavy chain comprising a variable domain that is specific for an antigen and a constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region; and the promoter being operable for expression of the heavy chain and wherein the promoter is not the native human promoter.
257. The antibody, polypeptide or nucleotide sequence of any one of clauses 253 to 256, wherein the variable domain comprises mouse AID-pattern somatic mutations or mouse terminal deoxynucleotidyl transferase (TdT)- pattern junctional mutations.
258. A vector (eg, a CHO cell or HEK293 cell vector) comprising the nucleic acid of clause 255, 256 or 257; optionally wherein the vector is in a host cell (eg, a CHO cell or HEK293 cell).
259. A pharmaceutical composition comprising the antibody or polypeptide of any one of clauses 253, 254 and 257, together with a pharmaceutically-acceptable excipient, diluent or a medicament (eg, a further antigen-specific variable domain, antibody chain or antibody).
260. The antibody or polypeptide of any one of clauses253, 254 and 257 for use in treating and/or preventing a medical condition in a human patient.
261. Use of the antibody or polypeptide of any one of clauses 253, 254 and 257 for the manufacture of a medicament for treating and/or preventing a medical condition in a human patient.
262. The antibody, polypeptide or use of clause 260 or 261, wherein the human is a member of a human population selected from population numbers 1-14, wherein the populations are numbered as follows (population labels being according to 1000 Genomes Project nomenclature)
   1= ASW;
   2= CEU;
   3=CHB;
   4=CHS;
   5=CLM;
   6=FIN;
   7=GBR;
   8=IB5;
   9=JPT;
   10=LWK;
   11=MXL;
   12=PUR;
   13=TSI;
   14=YRI.
263. The antibody, polypeptide or use of clause 262, wherein the constant region is a
   (i) IGHAla constant region and the human population is selected from any population number 1-14;
   (ii) IGHA2a constant region and the human population is selected from any population number 1-14;
   (ill) IGHA2b constant region and the human population is selected from any population number 1-14;
   (iV) IGHG2a constant region and the human population is selected from any population number 1-9 and 11-13;
   (v) IGHG3a constant region and the human population is selected from any population number 1-14;
   (vi) IGHG3b constant region and the human population is selected from any population number 1-8 and 11-13;
   (vii) IGHG4a constant region and the human population is selected from any population number 1-9 and 11-13;
   (viii)IGHMa constant region and the human population is selected from any population number 1-14; or
   (ix) IGHMb constant region and the human population is selected from any population number 1-14;
   Wherein the populations are numbered as follows (population labels being according to 1000 Genomes Project nomenclature)
   1= ASW;
   2= CEU;
   3=CHB;
   4=CHS;
   5=CLM;
   6=FIN;
   7=GBR;
   8=IBS;
   9=JPT;
   10=LWK;
   11=MXL;
   12=PUR;
   13=TSI;
   14=YRI.
265. A vector (eg, a CHO cell or HEK293 cell vector) comprising a IGHG1ref, IGHG2ref, IGHG2a, IGHG3ref, IGHG3a, IGHG3b, IGHG4ref or IGHG4a constant region nucleotide sequence that is 3' of a cloning site for the insertion of a human antibody heavy chain variable domain nucleotide sequence, such that upon insertion of such a variable domain sequence the vector comprises (in 5' to 3' direction) a promoter, a leader sequence, the variable domain sequence and the constant region sequence so that the vector is capable of expressing a human antibody heavy chain when present in a host cell.
   A non-human vertebrate or vertebrate cell (optionally an ES cell or antibody-producing cell) comprising a genome having a superhuman immunoglobulin heavy chain human VH and/or D and/or J gene repertoire.
266. The vertebrate or cell of clause 265, wherein the genome comprises a transgenic immunoglobulin heavy chain locus comprising a plurality of human immunoglobulin VH gene segments, one or more human D gene segments and one or more human J gene segments, wherein the plurality of VH gene segments consists of more than the natural human repertoire of functional VH gene segments; optionally wherein the genome is homozygous for said transgenic heavy chain locus.
267. The vertebrate or cell ofclause 266, wherein VH gene repertoire consists of a plurality of VH gene segments derived from the genome sequence of a first human individual, supplemented with one or more different VH gene segments derived from the genome sequence of a second, different human individual; optionally wherein the individuals are not related; optionally wherein the D and J segments are derived from the genome sequence of the first human individual; and optionally wherein the VH gene segments from the genome sequence of the second individual are selected from the VH gene segments listed in Table 1.
268. The vertebrate or cell of clause 267, wherein the transgenic locus comprises more than 26 functional human VH gene segments; optionally wherein the locus comprises at least 27 or 28 functional human VH gene segments (eg, wherein the locus comprises the full functional VH repertoire of said first individual supplemented with one or more VH gene segments derived from the genome sequence of the second human individual and optionally with one or more VH gene segments derived from the genome sequence of a third human individual).
269. The vertebrate of cell of clause 267 or 268, wherein the transgenic locus comprises a first VH gene segment derived from the genome sequence of the first individual and a second VH gene segment derived from the genome sequence of the second individual, wherein the second VH gene segment is a polymorphic variant of the first VH gene segment; optionally wherein the locus comprises a further polymorphic variant of the first VH gene segment (eg, a variant derived from the genome sequence of a third human individual).
270. The vertebrate or cell of any one of clauses 265 to 269, wherein the genome comprises a (or said) transgenic immunoglobulin heavy chain locus comprising a plurality of human immunoglobulin VH gene segments, a plurality of human D gene segments and one or more human J gene segments, wherein the plurality of D gene segments consists of more than the natural human repertoire of functional D gene segments; optionally wherein the genome is homozygous for said transgenic heavy chain locus.
271. The vertebrate or cell of clause 270, wherein D gene repertoire consists of a plurality of D gene segments derived from the genome sequence of a (or said) first human individual, supplemented with one or more different D gene segments derived from the genome sequence of a (or said) second, different human individual; optionally wherein the individuals are not related; optionally wherein J segments are derived from the genome sequence of the first human individual; and optionally wherein the D gene segments from the genome sequence of the second individual are selected from the D gene segments listed in Table 2.
272. The vertebrate or cell of clause 271, wherein the transgenic locus comprises more than 26 functional human D gene segments; optionally wherein the locus comprises at least 27 or 28functional human D gene segments (eg, wherein the locus comprises the full functional D repertoire of said first individual supplemented with one or more D gene segments derived from the genome sequence of the second human individual and optionally with one or more D gene segments derived from the genome sequence of a third human individual).
273. The vertebrate of cell of clause 271 or 272, wherein the transgenic locus comprises a first D gene segment derived from the genome sequence of the first individual and a second D gene segment derived from the genome sequence of the second individual, wherein the second D gene segment is a polymorphic variant of the first D gene segment; optionally wherein the locus comprises a further polymorphic variant of the first D gene segment (eg, a variant derived from the genome sequence of a third human individual).
274. The vertebrate or cell of any one of clauses 265 to 273, wherein the genome comprises a (or said) transgenic immunoglobulin heavy chain locus comprising a plurality of human immunoglobulin VH gene segments, one or more human D gene segments and a plurality of human JH gene segments, wherein the plurality of J gene segments consists of more than the natural human repertoire of functional J gene segments; optionally wherein the genome is homozygous for said transgenic heavy chain locus.
275. The vertebrate or cell of clause 274, wherein the J gene repertoire consists of a plurality of J gene segments derived from the genome sequence of a (or said) first human individual, supplemented with one or more different J gene segments derived from the genome sequence of a (or said) second, different human individual; optionally wherein the individuals are not related; optionally wherein D segments are derived from the genome sequence of the first human individual; and optionally wherein the J gene segments from the genome sequence of the second individual are selected from the J gene segments listed in Table 3.
276. The vertebrate or cell of clause 275, wherein the transgenic locus comprises more than 26 functional human J gene segments; optionally wherein the locus comprises at least 27 or 28 functional human J gene segments (eg, wherein the locus comprises the full functional J repertoire of said first individual supplemented with one or more J gene segments derived from the genome sequence of the second human individual and optionally with one or more J gene segments derived from the genome sequence of a third human individual).
277. The vertebrate of cell of clause 275 or 276, wherein the transgenic locus comprises a first J gene segment derived from the genome sequence of the first individual and a second J gene segment derived from the genome sequence of the second individual, wherein the second J gene segment is a polymorphic variant of the first J gene segment; optionally wherein the locus comprises a further polymorphic variant of the first J gene segment (eg, a variant derived from the genome sequence of a third human individual).
278. A non-human vertebrate or vertebrate cell (optionally an ES cell or antibody-producing cell) comprising a genome having a superhuman immunoglobulin light chain human VL gene repertoire; optionally wherein the vertebrate or cell is according to any one of clauses 265 to 277.
279. The vertebrate or cell of clause 278, wherein the genome comprises
   (i) a transgenic immunoglobulin kappa light chain locus comprising a plurality of human immunoglobulin Vκ gene segments and one or more human J gene segments, wherein the plurality of Vκ gene segments consists of more than the natural human repertoire of functional Vκ gene segments; optionally wherein the genome is homozygous for said transgenic kappa light chain locus; and/or
   (ii) a transgenic immunoglobulin lambda light chain locus comprising a plurality of human immunoglobulin Vλ gene segments and one or more human J gene segments, wherein the plurality of Vλ gene segments consists of more than the natural human repertoire of functional Vλ gene segments; optionally wherein the genome is homozygous for said transgenic lambda light chain locus.
280. The vertebrate or cell of clause 279, wherein
   (i) the Vκ gene repertoire consists of a plurality of Vκ gene segments derived from the genome sequence of a first human individual, supplemented with one or more Vκ gene segments derived from the genome sequence of a second, different human individual; optionally wherein the individuals are not related; optionally wherein the J segments are derived from the genome sequence of the first human individual; and optionally wherein the Vκ gene segments from the genome sequence of the second individual are selected from the Vκ gene segments listed in Table 4; and
   (i) the Vλ gene repertoire consists of a plurality of Vλ gene segments derived from the genome sequence of a first human individual, supplemented with one or more Vλ gene segments derived from the genome sequence of a second, different human individual; optionally wherein the individuals are not related; optionally wherein the J segments are derived from the genome sequence of the first human individual; and optionally wherein the Vλ gene segments from the genome sequence of the second individual are selected from the Vλ gene segments listed in Table 5.
281. The vertebrate or cell of clause 280, wherein
   the kappa light transgenic locus comprises more than 26 functional human Vκ gene segments; optionally wherein the locus comprises at least 27 or 28 functional human Vκ gene segments (eg, wherein the locus comprises the full functional Vκ repertoire of said first individual supplemented with one or more Vκ gene segments derived from the genome sequence of the second human individual and optionally with one or more Vκ gene segments derived from the genome sequence of a third human individual); and
   the lambda light transgenic locus comprises more than 26 functional human Vλ gene segments; optionally wherein the locus comprises at least 27 or 28 functional human Vλ gene segments (eg, wherein the locus comprises the full functional Vλ repertoire of said first individual supplemented with one or more Vλ gene segments derived from the genome sequence of the second human individual and optionally with one or more Vλ gene segments derived from the genome sequence of a third human individual);
282. The vertebrate of cell of clause 280 or 281, wherein
   the kappa light transgenic locus comprises a first Vκ gene segment derived from the genome sequence of the first individual and a second Vκ gene segment derived from the genome sequence of the second individual, wherein the second Vκ gene segment is a polymorphic variant of the first Vκ gene segment; optionally wherein the locus comprises a further polymorphic variant of the first Vκ gene segment (eg, a variant derived from the genome sequence of a third human individual); and
   the lambda light transgenic locus comprises a first Vλ gene segment derived from the genome sequence of the first individual and a second Vλ gene segment derived from the genome sequence of the second individual, wherein the second Vλ gene segment is a polymorphic variant of the first Vλ gene segment; optionally wherein the locus comprises a further polymorphic variant of the first Vλ gene segment (eg, a variant derived from the genome sequence of a third human individual).
283. The vertebrate or cell of any one of clauses 278 to 282, wherein the genome comprises a (or said) transgenic immunoglobulin light chain locus comprising a plurality of human immunoglobulin VL gene segments and a plurality of human JL gene segments, wherein the plurality of J gene segments consists of more than the natural human repertoire of functional J gene segments; optionally wherein the genome is homozygous for said transgenic heavy chain locus.
284. The vertebrate or cell of clause 283, wherein
   (i) the Jκ gene repertoire consists of a plurality of Jκ gene segments derived from the genome sequence of a (or said) first human individual, supplemented with one or more Jκ gene segments derived from the genome sequence of a (or said) second, different human individual; optionally wherein the individuals are not related; optionally wherein the Vκ segments are derived from the genome sequence of the first human individual; optionally wherein the Jκ gene segments from the genome sequence of the second individual are selected from the Jκ gene segments listed in Table 6; and
   (ii) the Jκ gene repertoire consists of a plurality of Jλ gene segments derived from the genome sequence of a (or said) first human individual, supplemented with one or more Jλ gene segments derived from the genome sequence of a (or said) second, different human individual; optionally wherein the individuals are not related; optionally wherein the Vλ segments are derived from the genome sequence of the first human individual; optionally wherein the Jλ gene segments from the genome sequence of the second individual are selected from the Jλ gene segments listed in Table 7.
285. The vertebrate or cell of clause 284, wherein
   (i) the transgenic light chain locus comprises more than 26 functional human Jκ gene segments; optionally wherein the locus comprises at least 27 or 28functional human Jκ gene segments (eg, wherein the locus comprises the full functional Jκ repertoire of said first individual supplemented with one or more Jκ gene segments derived from the genome sequence of the second human individual and optionally with one or more Jκ gene segments derived from the genome sequence of a third human individual); and/or
   (i) the transgenic light chain locus comprises more than 26 functional human Jλ gene segments; optionally wherein the locus comprises at least 27 or 28 functional human Jλ gene segments (eg, wherein the locus comprises the full functional Jλ repertoire of said first individual supplemented with one or more Jλ gene segments derived from the genome sequence of the second human individual and optionally with one or more Jλ gene segments derived from the genome sequence of a third human individual).
286. The vertebrate or cell of clause 284 or 285, wherein
   (i) the kappa light transgenic locus comprises a first Jκ gene segment derived from the genome sequence of the first individual and a second Jκ gene segment derived from the genome sequence of the second individual, wherein the second Jκ gene segment is a polymorphic variant of the first Jκ gene segment; optionally wherein the locus comprises a further polymorphic variant of the first Jκ gene segment (eg, a variant derived from the genome sequence of a third human individual); and
   (ii) the lambda light transgenic locus comprises a first Jλ gene segment derived from the genome sequence of the first individual and a second Jλ gene segment derived from the genome sequence of the second individual, wherein the second Jκ gene segment is a polymorphic variant of the first Jλ gene segment; optionally wherein the locus comprises a further polymorphic variant of the first Jλ gene segment (eg, a variant derived from the genome sequence of a third human individual).
287. A library of antibody-producing transgenic cells whose genomes collectively encode a repertoire of antibodies, wherein
   (a) a first transgenic cell expresses a first antibody having a chain encoded by a first immunoglobulin gene, the gene comprising a first variable domain nucleotide sequence produced following recombination of a first human unrearranged immunoglobulin gene segment;
   (b) a second transgenic cell expresses a second antibody having a chain encoded by a second immunoglobulin gene, the second gene comprising a second variable domain nucleotide sequence produced following recombination of a second human unrearranged immunoglobulin gene segment, the first and second antibodies being non-identical;
   (c) the first and second gene segments are different and derived from the genome sequences of first and second human individuals respectively, wherein the individuals are different; and optionally not related;
   (d) wherein the cells are non-human vertebrate (eg, mouse or rat) cells.
288. The library of clause 23, wherein in step (c) the individuals or not related.
289. The vertebrate, cell or library of any one of clauses 265 to 288, wherein the first and second human individuals are members of first and second ethnic populations respectively, wherein the populations are different; optionally wherein the ethnic populations are selected from those identified in the 1000 genomes database.
290. The vertebrate, cell or library of clause 289, wherein the human immunoglobulin gene segment derived from the genome sequence of the second individual is low-frequency (optionally rare) within the second ethnic population.
291. The library of clause 289 or 290, wherein the first variable region nucleotide sequence is produced by recombination of the first human immunoglobulin gene segment with a first J gene segment and optionally a first D gene segment, wherein the first human immunoglobulin gene segment is a V gene segment and the V, D and J segments are derived from the first human population, optionally from the genome of one individual of the first human population.
292. The library of clause 291, wherein the second variable region nucleotide sequence is produced by recombination of the second human immunoglobulin gene segment with a second J gene segment and optionally a second D gene segment, wherein the second human immunoglobulin gene segment is a V gene segment derived from the second population and the D and/or J segments are derived from the first human population, optionally the D and J gene segments being from the genome of one individual of the first human population.
293. The library of clause292, wherein all of the D and J segments that have been recombined with the first and second V gene segments are D and J segments derived from the first human population, optionally the D and J gene segments being from the genome of one individual of the first human population.
294. The vertebrate, cell or library of any one of clauses289 to 293, wherein the first and second ethnic populations are selected from the group consisting of an ethnic population with European ancestry, an ethnic population with East Asian, an ethnic population with West African ancestry, an ethnic population with Americas ancestry and an ethnic population with South Asian ancestry.
295. The vertebrate, cell or library of clause 294, wherein the first and second ethnic populations are selected from the group consisting of an ethnic population with Northern European ancestry; or an ethnic population with Western European ancestry; or an ethnic population with Toscani ancestry; or an ethnic population with British ancestry; or an ethnic population with Icelandic ancestry; or an ethnic population with Finnish ancestry; or an ethnic population with Iberian ancestry; or an ethnic population with Japanese ancestry; or an ethnic population with Chinese ancestry; or an ethnic population Vietnamese ancestry; or an ethnic population with Yoruba ancestry; or an ethnic population with Luhya ancestry; or an ethnic population with Gambian ancestry; or an ethnic population with Malawian ancestry; or an ethnic population with Native American ancestry; or an ethnic population with Afro-Caribbean ancestry; or an ethnic population with Mexican ancestry; or an ethnic population with Puerto Rican ancestry; or an ethnic population with Columbian ancestry; or an ethnic population with Peruvian ancestry; or an ethnic population with Ahom ancestry; or an ethnic population with Kayadtha ancestry; or an ethnic population with Reddy ancestry; or an ethnic population with Maratha; or an ethnic population with Punjabi ancestry.
296. The library of clause287, wherein the second human immunoglobulin gene segment is a polymorphic variant of the first human immunoglobulin gene segment; optionally wherein the second gene segment is selected from the group consisting of a gene segment in any of Tables 1 to 7 and 9 to 14, eg, the second gene segment is a polymorphic variant of VH1-69.
297. The library of any one ofclauses 287 to 296, wherein the first and second human immunoglobulin gene segments are both (i) V_{H} gene segments; (ii) D segments; (iii) J segments (optionally both J_{H} segments, both J_{K} segments or both J_{λ} segments); (iv) constant regions (optionally both a gamma constant region, optionally both a C gamma-1 constant region); (v) CH1 regions; (vi) CH2 regions; or (vii) CH3 regions.
298. The library of any one of clauses 287 to 297, wherein the library is naive and optionally has a library size of from 10² to 10⁹ cells.
299. The library of any one of clauses 287 to 298, wherein the library has been selected against a predetermined antigen optionally has a library size of from 10² to 10⁹ cells.
300. The library of any one of clauses 287 to 299, wherein said first and second cells are progeny of first and second ancestor non-human vertebrate cells respectively, wherein the first ancestor cell comprises a genome comprising said first human immunoglobulin gene segment; and the second ancestor cell comprises a genome comprising said second human immunoglobulin gene segment.
301. A library of antibody-producing transgenic cells whose genomes collectively encode a repertoire of antibodies, wherein the library comprises the first and second ancestor cells recited in clause 300.
302. A library of hybridoma cells produced by fusion of the library of any one of clauses 265 to 301 with fusion partner cells; and optionally the hybridoma library has a size of from 10² to 10⁹ cells
303. An isolated antibody having
   (a) a heavy chain encoded by a nucleotide sequence produced following recombination in a transgenic non-human vertebrate cell of an unrearranged human immunoglobulin V gene segment with a human D and human J segment, optionally with affinity maturation in said cell, wherein one of the gene segments is derived from the genome of an individual from a first human ethnic population; and the other two gene segments are derived from the genome of an individual from a second, different, human ethnic population, and wherein the antibody comprises heavy chain constant regions of said non-human vertebrate (eg, rodent, mouse or rat heavy chain constant regions); and/or
   (b) a light chain encoded by a nucleotide sequence produced following recombination in a transgenic non-human vertebrate cell of an unrearranged human immunoglobulin V gene segment with a human J segment, optionally with affinity maturation in said cell, wherein one of the gene segments is derived from the genome of an individual from a first human ethnic population (optionally the same as the first population in (a)); and the other gene segment is derived from the genome of an individual from a second, different, human ethnic population (optionally the same as the second population in (a)), and wherein the antibody comprises light chain constant regions of said non-human vertebrate (eg, rodent, mouse or rat heavy light constant regions);
   (c) Optionally wherein each variable domain of the antibody is a human variable domain.
   (d) Optionally wherein the heavy chain constant regions are gamma-type constant regions.
304. An isolated nucleotide sequence encoding the antibody of clause303, optionally wherein the sequence is provided in an antibody expression vector, optionally in a host cell.
305. A method of producing a human antibody, the method comprising replacing the non-human vertebrate constant regions of the antibody of clause 303 with human antibody constant regions.
306. A pharmaceutical composition comprising an antibody according to clause 303, or an antibody produced according to the method of clause 305 and a diluent, excipient or carrier; optionally wherein the composition is provided in a container connected to an IV needle or syringe or in an IV bag.
307. An antibody-producing cell that expresses the second antibody recited in any one of clauses 287 to 302 or the isolated antibody of clause 303.
308. A non-human vertebrate or vertebrate cell (optionally an ES cell or antibody-producing cell) whose genome comprises a transgenic immunoglobulin locus (eg, a heavy chain locus or a light chain locus), said locus comprising immunoglobulin gene segments according to the first and second human immunoglobulin gene segments (optionally V segments) recited in any one of clauses 287 to 302 operably connected upstream of an immunoglobulin constant region; optionally wherein the genome is homozygous for said transgenic immunoglobulin locus;
   optionally wherein the immunoglobulin locus comprises more than the natural human complement of functional V gene segments; and/or
   optionally wherein the immunoglobulin locus comprises more than the natural human complement of functional D gene segments; and/or
   optionally wherein the immunoglobulin locus comprises more than the natural human complement of functional J gene segments.
309. A transgenic non-human vertebrate (eg, a mouse or rat) or vertebrate cell (optionally an ES cell or antibody-producing cell) whose genome comprises a transgenic immunoglobulin locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region;
   wherein the transgenic locus comprises one or more human immunoglobulin V gene segments, one or more human J gene segments and optionally one or more human D gene segments, a first (optionally a V segment) of said gene segments and a second (optionally a V segment) of said gene segments being different and derived from the genomes of first and second human individuals respectively, wherein the individuals are different; and optionally not related;
   optionally wherein the immunoglobulin locus comprises more than the natural human complement of functional V gene segments; and/or
   optionally wherein the immunoglobulin locus comprises more than the natural human complement of functional D gene segments; and/or
   optionally wherein the immunoglobulin locus comprises more than the natural human complement of functional J gene segments.
310. A transgenic non-human vertebrate (eg, a mouse or rat) or vertebrate cell (optionally an ES cell or antibody-producing cell) whose genome comprises first and second transgenic immunoglobulin loci, each locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region;
   wherein (i) the first transgenic locus comprises one or more human immunoglobulin V gene segments, one or more human J gene segments and optionally one or more human D gene segments, (ii) the second transgenic locus comprises one or more human immunoglobulin V gene segments, one or more human J gene segments and optionally one or more human D gene segments; and (iii) wherein a first (optionally a V) gene segment of said first locus and a second (optionally a V) gene segment of said second gene locus are different and derived from the genomes of first and second human individuals respectively, wherein the individuals are different; and optionally not related
   optionally wherein the first and second loci are on different chromosomes (optionally chromosomes with the same chromosome number) in said genome;
   optionally wherein each immunoglobulin locus comprises more than the natural human complement of functional V gene segments; and/or
   optionally wherein each immunoglobulin locus comprises more than the natural human complement of functional D gene segments; and/or
   optionally wherein each immunoglobulin locus comprises more than the natural human complement of functional J gene segments.
311. The non-human vertebrate or cell of clause 309 or 310, wherein the immunoglobulin gene segments are as recited in any one of clauses 23 to 37.
312. The non-human vertebrate or cell of clause308, 309, 310 or 311, wherein the genome comprises a third immunoglobulin gene segment (optionally a V segment), the third gene segment being derived from a human individual that is different from the individual from which the first (and optionally also the second) gene segment is derived; optionally wherein the first, second and third gene segments are polymorphic variants of a human immunoglobulin gene segment.
313. The non-human vertebrate or cell of any one of clauses 308 to 312, wherein the genome of the vertebrate or cell is homozygous for the first, second and optional third gene segment, wherein a copy of the first, second and optional third gene segments are provided together on the same chromosome operably connected upstream of a common non-human vertebrate constant region.
314. The non-human vertebrate or cell of any one of clauses 308 to 313, wherein each first, second and optional third gene segment is a V gene segment.
315. The library of any one of clauses 287 to 302, wherein the library is provided by a collection of non-human vertebrates (optionally a collection of rodents, mice or rats); optionally, wherein a first member of said collection produces said first antibody but not said second antibody, and a second member of the collection produces said second antibody (but optionally not said first antibody).
316. A repertoire of antibodies expressed from a library of cells according to any one of clauses 287 to 302 and 315.
317. A method of constructing a cell (eg, an ES cell) according to any one of clauses 265 to 286, the method comprising
   (a) identifying functional V and J (and optionally D) gene segments of the genome sequence of a (or said) first human individual;
   (b) identifying one or more functional V and/or D and/or J gene segments of the genome sequence of a (or said) second human individual, wherein these additional gene segments are not found in the genome sequence of the first individual;
   (c) and constructing a transgenic immunoglobulin locus in the cell, wherein the gene segments of (a) and (b) are provided in the locus operably connected upstream of a constant region.
318. The method of clause 317, wherein the cell comprises a heavy chain locus constructed according to steps (a) to (c) and/or a light chain locus (kappa and/or lambda loci) constructed according to steps (a) to (c).
319. The method of clause 317 or 318, wherein the cell is homozygous for the or each transgenic locus; optionally wherein antibody expression from loci endogenous to said cell has been inactivated.
320. The method of any one ofclauses317 to 319, wherein the gene segment(s) in step (b) are identified from an immunoglobulin gene database selected from the 1000 genomes, Ensembl, Genbank and IMGT databases.
321. The method of any one of clauses 317 to 320, wherein the first and second human individuals are members of first and second ethnic populations respectively, wherein the populations are different, optionally wherein the human immunoglobulin gene segment derived from the genome sequence of the second individual is low-frequency (optionally rare) within the second ethnic population.
322. A method of making a transgenic non-human vertebrate (eg, a mouse or rat), the method comprising
   (a) constructing an ES cell (eg, a mouse C57BL/6N, C57BL/6J, 129S5 or 129Sv strain ES cell) by carrying out the method of any one of claims 317 to 321;
   (b) injecting the ES cell into a donor non-human vertebrate blastocyst (eg, a mouse C57BL/6N, C57BL/6J, 129S5 or 129Sv strain blastocyst);
   (c) implanting the blastocyst into a foster non-human vertebrate mother (eg, a C57BL/6N, C57BL/6J, 129S5 or 129Sv strain mouse); and
   (d) obtaining a child from said mother, wherein the child genome comprises a transgenic immunoglobulin locus.
323. A transgenic non-human vertebrate (eg, a mouse or rat) made by the method of clause 322 or a progeny thereof.
324. A population of non-human vertebrates according to clause 323.
325. A method of isolating an antibody that binds a predetermined antigen (eg, a bacterial or viral pathogen antigen), the method comprising
   (a) providing a vertebrate (optionally a mouse or rat) according to any one of clauses 265 to 286, 289, 290, 294, 295, 308 to 314 and 323;
   (b) immunising (eg, using a standard prime-boost method) said vertebrate with said antigen (optionally wherein the antigen is an antigen of an infectious disease pathogen);
   (c) removing B lymphocytes from the vertebrate and selecting one or more B lymphocytes expressing antibodies that bind to the antigen;
   (d) optionally immortalising said selected B lymphocytes or progeny thereof, optionally by producing hybridomas therefrom; and
   (e) isolating an antibody (eg, and IgG-type antibody) expressed by the B lymphocytes; and
   (f) optionally producing a derivative or variant of the antibody.
326. The method of clause325, further comprising after step (e) the step of isolating from said B lymphocytes nucleic acid encoding said antibody that binds said antigen; optionally exchanging the heavy chain constant region nucleotide sequence of the antibody with a nucleotide sequence encoding a human or humanised heavy chain constant region and optionally affinity maturing the variable region of said antibody; and optionally inserting said nucleic acid into an expression vector and optionally a host.
327. A non-human vertebrate (eg, a mouse or rat) or cell (eg, a mouse cell or rat cell) comprising a genome that comprises a transgenic heavy chain immunoglobulin locus, wherein the locus comprises at least 42 (optionally at least 43, 44, 45, 46, 47, 48, 49, 50) functional human VH gene segments, one or more functional human D gene segments, one or more functional human JH gene segments operably connected upstream of a non-human vertebrate constant region (eg, a mouse constant region, eg, a Cmu and/or a C gamma).
328. The vertebrate or cell of clause 327, wherein the locus comprises at least 23 functional human D gene segments and/or at least 6 functional human JH gene segments, optionally at least a full human repertoire of functional VH, D and JH segments (optionally derived from the genome sequence of the same human individual).
329. A non-human vertebrate (eg, a mouse or rat) or cell (eg, a mouse cell or rat cell) comprising a genome that comprises a transgenic heavy chain immunoglobulin locus, wherein the locus comprises a full human repertoire of functional VH, D and JH segments derived from the genome sequence of the same human individual, supplemented with one or more additional functional human VH, D and/or JH gene segment that is not found in the genome sequence of said human individual.
330. The vertebrate or cell of clause 329, wherein each additional VH, D and JH segment is selected from the group consisting of (i) a gene segment derived from a second, different individual, optionally wherein the first and second individuals are members of different ethnic populations; (ii) a mutant of a human gene segment (optionally a human germline gene segment having up to 5, 10 or 15 mutations); and (iii) a hybrid human gene segment comprising a first and second nucleotide sequences derived from genome sequences of different human individuals or different polymorphic variants of a human immunoglobulin gene segment (eg, variants of a germline human VH, D or JH gene segment).
331. The vertebrate or cell of any one ofclauses 328 to 330, wherein the genome is homozygous for said heavy chain transgene and optionally endogenous immunoglobulin heavy chain expression has been inactivated.
332. A non-human vertebrate (eg, a mouse or rat) or cell (eg, a mouse cell or rat cell) comprising a genome that comprises a transgenic kappa light chain immunoglobulin locus, wherein the locus comprises at least 39 functional human Vk gene segments and one or more functional human Jk gene segments operably connected upstream of a non-human vertebrate constant region (eg, a mouse constant region).
333. The vertebrate or cell of clause 332, wherein the locus comprises at least a full human repertoire of functional Vk and Jk segments (optionally derived from the genome sequence of the same human individual).
334. A non-human vertebrate (eg, a mouse or rat) or cell (eg, a mouse cell or rat cell) comprising a genome that comprises a transgenic kappa light chain immunoglobulin locus, wherein the locus comprises a full human repertoire of functional Vk and Jk segments derived from the genome sequence of the same human individual, supplemented with one or more additional functional human Vk and/or Jk gene segment that is not found in the genome sequence of said human individual.
335. The vertebrate or cell of clause 334, wherein each additional Vk and Jk segment is selected from the group consisting of (i) a gene segment derived from a second, different individual, optionally wherein the first and second individuals are members of different ethnic populations; (ii) a mutant of a human gene segment (optionally a human germline gene segment having up to 5, 10 or 15 mutations); and (iii) a hybrid human gene segment comprising a first and second nucleotide sequences derived from: genome sequences of different human individuals or different polymorphic variants of a human immunoglobulin gene segment (eg, variants of a germline human Vk or Jk gene segment).
336. The vertebrate or cell of any one of clauses 332 to 335, wherein the genome is homozygous for said kappa light chain transgene and optionally endogenous immunoglobulin kappa light chain expression has been inactivated.
337. A non-human vertebrate (eg, a mouse or rat) or cell (eg, a mouse cell or rat cell) comprising a genome that comprises a transgenic lambda light chain immunoglobulin locus, wherein the locus comprises at least 32 functional human V lambda gene segments and one or more functional human J lambda gene segments operably connected upstream of a non-human vertebrate constant region (eg, a mouse constant region).
338. The vertebrate or cell of clause 337, wherein the locus comprises at least a full human repertoire of functional V lambda and J lambda segments (optionally derived from the genome sequence of the same human individual).
339. A non-human vertebrate (eg, a mouse or rat) or cell (eg, a mouse cell or rat cell) comprising a genome that comprises a transgenic lambda light chain immunoglobulin locus, wherein the locus comprises a full human repertoire of functional V lambda and J lambda segments derived from the genome sequence of the same human individual, supplemented with one or more additional functional human V lambda and/or J lambda gene segment that is not found in the genome sequence of said human individual.
340. The vertebrate or cell of clause 339, wherein each additional V lambda and J lambda segment is selected from the group consisting of (i) a gene segment derived from a second, different individual, optionally wherein the first and second individuals are members of different ethnic populations; (ii) a mutant of a human gene segment (optionally a human germline gene segment having up to 5, 10 or 15 mutations); and (iii) a hybrid human gene segment comprising a first and second nucleotide sequences derived from genome sequences of different human individuals or different polymorphic variants of a human immunoglobulin gene segment (eg, variants of a germline human V lambda or J lambda gene segment).
341. The vertebrate or cell of any one of clauses 337 to 340, wherein the genome is homozygous for said lambda light chain transgene and optionally endogenous immunoglobulin lambda light chain expression has been inactivated.
342. A transgenic immunoglobulin locus comprising a synthetic immunoglobulin gene haplotype, the haplotype comprising first and second human gene segments (each being a V, D or J), a switch region and a constant region, wherein
   a) the second gene segment is a polymorphic variant of the first gene segment; or
   b) the first and second gene segments are derived respectively from genome sequence of individuals from different, first and second, ethnic populations (eg, according to the 1000 Genomes database) and the second gene segment is not found in the first population (eg, according to the 1000 Genomes database), optionally wherein the second gene segment occurs at low-frequency (or is rare) in the second population; and
   Wherein the constant region, and optionally the switch region, are non-human vertebrate (eg, mouse or rat) constant and switch regions (eg, mouse or rat Cmu; mouse or rat Smu; mouse or rat C gamma; or mouse or rat S gamma).
343. The transgenic immunoglobulin locus of clause 342 wherein the transgene comprises a third human immunoglobulin gene segment (a V, D or J) which is (a) a polymorphic variant of the first and second gene segments; or (b) derived from a genome sequence of the second population (and optionally is a low-frequency or rare gene in that population) or is from a third human ethnic population, wherein the third gene segment is not found in the first population (eg, according to the 1000 Genomes database).
344. A non-human vertebrate or cell comprising the transgene of clause 342 or 343; optionally wherein the vertebrate or cell is homozygous for said transgene.
345. An isolated antibody for administration to a Chinese patient, the antibody comprising a human heavy chain, the heavy chain comprising a variable domain that is specific for an antigen and a constant region, wherein the constant region is a human constant region selected from a constant region (eg, an IGHG constant region) inTable 13 found in a Chinese population and with a cumulative frequency of at least 5%;, and wherein
   (i) the variable domain is derived from the recombination of said human gene segments in a non-human vertebrate (eg, in a mouse or a rat); or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
346. The antibody of clause 345 wherein the constant region is a IGHG1a, IGHG2a, IGHG3a, IGHG3b or IGHG4a constant region.
347. The antibody of clause345 or 346, wherein the variable domain is derived from the recombination of a human VH gene segment with a human D gene segment and a human JH gene segment, the VH gene segment being selected from a VH in Table 13 found in a Chinese population and with a cumulative frequency of at least 5%.
348. The antibody of clause 345, 346 or 347, wherein the variable domain is derived from the recombination of a human VH gene segment with a human D gene segment and a human JH gene segment, the D gene segment being selected from a D in Table 13 found in a Chinese population and with a cumulative frequency of at least 5%.
349. The antibody of clause 345, 346, 347 or 348 wherein the variable domain is derived from the recombination of a human VH gene segment with a human D gene segment and a human JH gene segment, the JH gene segment being selected from a JH in Table 13 found in a Chinese population and with a cumulative frequency of at least 5%.
350. An isolated VH domain identical to a variable domain as recited in any one of clauses 347 to 349, optionally fused at its C-terminus to a polypeptide (eg, an antibody Fc).
351. A pharmaceutical composition comprising the antibody or variable domain of any one of clauses 345 to 350 together with a pharmaceutically-acceptable excipient, diluent or a medicament (eg, a further antigen-specific variable domain, antibody chain or antibody).
352. An isolated antibody for administration to a Chinese patient, the antibody comprising a human heavy chain, the heavy chain comprising a variable domain that is specific for an antigen and a constant region, wherein the variable domain is derived from the recombination of a human VH gene segment with a human D gene segment and a human JH gene segment, the VH gene segment being selected from a VH in Table 13 found in a Chinese population and with a cumulative frequency of at least 5%; and wherein
   (i) the variable domain is derived from the recombination of said human gene segments in a non-human vertebrate (eg, in a mouse or a rat); or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
353. The antibody of clause352, wherein the variable domain is derived from the recombination of a human VH gene segment with a human D gene segment and a human JH gene segment, the D gene segment being selected from a D in Table 13 found in a Chinese population and with a cumulative frequency of at least 5%.
354. The antibody of clause352 or 353, wherein the variable domain is derived from the recombination of a human VH gene segment with a human D gene segment and a human JH gene segment, the JH gene segment being selected from a JH in Table 13 found in a Chinese population and with a cumulative frequency of at least 5%.
355. An isolated VH domain identical to a variable domain as recited in any one of clauses 352 to 354, optionally fused at its C-terminus to a polypeptide (eg, an antibody Fc).
356. A pharmaceutical composition comprising the antibody or variable domain of any one of clauses 352 to 355 together with a pharmaceutically-acceptable excipient, diluent or a medicament (eg, a further antigen-specific variable domain, antibody chain or antibody).
357. An antibody heavy chain or VH domain (eg, provided as part of an antibody) for therapy and/or prophylaxis of a disease or medical condition in a Chinese patient, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH in Table 13 found in a Chinese population and with a cumulative frequency of at least 5%;
   (b) Optional humanisation of the heavy chain by combining the variable domain of the heavy chain with a human constant region; or optional humanisation of the selected VH domain by combining with a human constant region.
358. The antibody heavy chain or VH domain ofclause357, wherein the human constant region is as recited in clause 345 or 346.
359. An antibody heavy chain or VH domain as recited in clause 357 or 358 for use in a medicament for therapy and/or prophylaxis of a disease or medical condition in a Chinese patient.
360. A method of treating and/or preventing a disease or medical condition in a Chinese patient, the method comprising administering to the patient a therapeutically or prophylactically-effective amount of the antibody heavy chain or VH domain as recited in clause 357 or 358.
361. An isolated antibody for administration to a patient of European, East Asian, West African, South Asian or Americas ancestry, the antibody comprising a human heavy chain, the heavy chain comprising a variable domain that is specific for an antigen and a constant region, wherein the constant region is a human constant region selected from a constant region (eg, an IGHG constant region) in Table 13 found in a population of European, East Asian, West African, South Asian or Americas ancestry respectively and with a cumulative frequency of at least 5%;, and wherein
   (i) the variable domain is derived from the recombination of said human gene segments in a non-human vertebrate (eg, in a mouse or a rat); or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
362. The antibody of claim 361 wherein the constant region is a IGHG1a, IGHG2a, IGHG3a, IGHG3b or IGHG4a constant region and the patient is of European ancestry.
363. The antibody of clause 361 or 362, wherein the variable domain is derived from the recombination of a human VH gene segment with a human D gene segment and a human JH gene segment, the VH gene segment being selected from a VH in Table 13 found in said population and with a cumulative frequency of at least 5%.
364. The antibody of clause361, 362 or 363, wherein the variable domain is derived from the recombination of a human VH gene segment with a human D gene segment and a human JH gene segment, the D gene segment being selected from a D in Table 13 found in said population and with a cumulative frequency of at least 5%.
365. The antibody ofclause 361, 362, 363 or 364 wherein the variable domain is derived from the recombination of a human VH gene segment with a human D gene segment and a human JH gene segment, the JH gene segment being selected from a JH in Table 13 found in said population and with a cumulative frequency of at least 5%.
366. An isolated VH domain identical to a variable domain as recited in any one of clauses 363 to 365, optionally fused at its C-terminus to a polypeptide (eg, an antibody Fc).
367. A pharmaceutical composition comprising the antibody or variable domain of any one of clauses361 to 366 together with a pharmaceutically-acceptable excipient, diluent or a medicament (eg, a further antigen-specific variable domain, antibody chain or antibody).
368. An isolated antibody for administration to a patient of European, East Asian, West African or Americas ancestry, the antibody comprising a human heavy chain, the heavy chain comprising a variable domain that is specific for an antigen and a constant region, wherein the variable domain is derived from the recombination of a human VH gene segment with a human D gene segment and a human JH gene segment, the VH gene segment being selected from a VH in Table 13 found in a population of European, East Asian, West African, South Asian or Americas ancestry respectively and with a cumulative frequency of at least 5%; and wherein
   (i) the variable domain is derived from the recombination of said human gene segments in a non-human vertebrate (eg, in a mouse or a rat); or (ii) the variable domain comprises non-human vertebrate (eg, mouse or rat) AID-pattern mutations and non-human vertebrate (eg, mouse or rat) terminal deoxynucleotidyl transferase (TdT)-pattern mutations.
369. The antibody of clause 368, wherein the variable domain is derived from the recombination of a human VH gene segment with a human D gene segment and a human JH gene segment, the D gene segment being selected from a D in Table 13 found in said population and with a cumulative frequency of at least 5%.
370. The antibody of clause368 or 369, wherein the variable domain is derived from the recombination of a human VH gene segment with a human D gene segment and a human JH gene segment, the JH gene segment being selected from a JH in Table 13 found in said population and with a cumulative frequency of at least 5%.
371. An isolated VH domain identical to a variable domain as recited in any one of clauses 368 to 370, optionally fused at its C-terminus to a polypeptide (eg, an antibody Fc).
372. A pharmaceutical composition comprising the antibody or variable domain of any one of clauses 368 to 371 together with a pharmaceutically-acceptable excipient, diluent or a medicament (eg, a further antigen-specific variable domain, antibody chain or antibody).
373. An antibody heavy chain or VH domain (eg, provided as part of an antibody) for therapy and/or prophylaxis of a disease or medical condition in a patient of European, East Asian, West African, South Asian or Americas ancestry, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH in Table 13 found in said population and with a cumulative frequency of at least 5%;
   (b) Optional humanisation of the heavy chain by combining the variable domain of the heavy chain with a human constant region; or optional humanisation of the selected VH domain by combining with a human constant region.
374. The antibody heavy chain or VH domain of clause 373, wherein the human constant region is as recited in clause361 or 362.
375. An antibody heavy chain or VH domain as recited in clause 373 or 374 for use in a medicament for therapy and/or prophylaxis of a disease or medical condition in a patient of said ancestry.
376. A method of treating and/or preventing a disease or medical condition in a patient of European, East Asian, West African, South Asian or Americas ancestry, the method comprising administering to the patient a therapeutically or prophylactically-effective amount of the antibody heavy chain or VH domain as recited in clause 373 or 374.

## Claims

1. A method for producing a heavy chain, VH domain or an antibody specific to a target antigen, the method comprising
immunizing a mouse or rat with the antigen, wherein the mouse or rat has a genome comprising an immunoglobulin heavy chain locus comprising human gene segment JH6*02, one or more VH gene segments and one or more D gene segments upstream of a constant region, wherein the gene segments in the heavy chain locus are operably linked to the constant region thereof so that the mouse or rat is capable of producing an antibody heavy chain produced by recombination of the human JH6*02 with a D segment and a VH segment, and
isolating the heavy chain, VH domain or an antibody specific to a target antigen or a cell producing the heavy chain, VH domain or an antibody, wherein the heavy chain, VH domain or an antibody comprises a HCDR3 that is derived from the recombination of human JH6*02 with a VH gene segment and a D gene segment.

2. A method according to claim 1, comprising removing B lymphocytes from the mouse or rat, selecting one or more B lymphocytes expressing antibodies that bind to the antigen, and isolating from said B lymphocytes nucleic acid encoding said antibody.

3. A method for producing a human heavy chain or antibody comprising carrying out the method of claim 1 or claim 2, wherein the constant region of the locus is a non-human vertebrate (eg, mouse or rat) constant region, and then replacing the non-human constant region of the isolated heavy chain or antibody with a human constant region.

4. A method according to any preceding claim, further comprising inserting nucleic acid encoding said heavy chain, VH domain or antibody into an expression vector and optionally a host cell.

5. A method according to claim 4, comprising expressing the heavy chain, VH domain or antibody from the host cell and providing an isolated heavy chain, VH domain or antibody.

6. A method according to any preceding claim, wherein
(i) the target antigen is an antigen of an infectious disease pathogen;
(ii) the target antigen is a receptor, and the antibody heavy chain specifically binds a receptor cleft; or
(iii) the target antigen is an enzyme, and the antibody heavy chain specifically binds the enzyme active site.

7. A mouse or a rat whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof so that the mouse is capable of producing an antibody heavy chain produced by recombination of the human JH6*02 with a D segment and a VH segment.

8. A mouse or rat according to claim 7, which has been immunised with a target antigen and wherein the variable domain of the heavy chain is the product of recombination between a VH, D and JH6*02 and wherein the HCDR3 length is at least 20 amino acids.

9. A mouse cell or a rat cell whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof for producing (eg, in a subsequent progeny cell) an antibody heavy chain produced by recombination of the human JH6*02 with a D segment and a VH segment.

10. A method, mouse, rat or cell according to any preceding claim, wherein the JH6*02 is the only JH6-type gene segment in the genome and/or wherein the JH6*02 is the closest JH gene segment to the constant region in the locus.

11. A method, mouse, rat or cell according to any preceding claim, wherein the locus comprises one, more or all human D gene segments D3-9; D4-17; D3-10; D2-2; D5-24; D6-19; D3-22; D6-13; D5-12; D1-26; D1-20; D5-18; D3-16; D2-21; D1-14; D7-27; D1-1; D6-25; D2-14; and D4-23.

12. A method, mouse, rat or cell according to any preceding claim, wherein the locus comprises one, more or all of IGHV gene segments selected from V3-21, V3-13, V3-7, V6-1, V1-8, V1-2, V7-4-1, V1-3, V1-18, V4-4, V3-9, V3-23, V3-11 and V3-20.

13. A method, mouse, rat or cell according to any preceding claim, wherein the antibody heavy chain has a HCDR3 length of at least 20 amino acids.

14. A method, mouse, rat or cell according to any preceding claim, wherein the antibody heavy chain is a product of the recombination of JH6*02 with a human VH gene segment recited in claim 13 and/or a D gene segment recited in claim 10.

15. Use of human JH6*02 for generating a HCDR3 of at least 20 amino acids in a non-human vertebrate whose genome comprises an immunoglobulin heavy chain locus comprising human gene segment JH6*02, one or more human VH gene segments and one or more human D gene segments upstream of a constant region; wherein the gene segments in the heavy chain locus are operably linked to the constant region thereof so that the vertebrate is capable of producing an antibody heavy chain produced by recombination of the human JH6*02 with a human D segment and a human VH segment and wherein the JH6*02 gene segment comprises the following nucleotide sequence: ATTACTA CTACTACTAC GGTATGGACG TCTGGGGCCA AGGGACCACG GTCACCGTCT CCTCAG.

16. A method of producing an antibody comprising a heavy chain, the heavy chain comprising a variable domain and a constant region, wherein the variable domain is specific for an antigen and is encoded by a variable region nucleotide sequence produced by recombination in a transgenic mouse of a human VH gene segment with a JH gene segment and a D gene segment; wherein
(a) the VH gene segment is a human IGHV3-23 (e.g., V3-23*04) gene segment;
(b) the JH gene segment is a human IGHJ4 (e.g., JH4*01) or IGHJ6 (e.g., JH6*02) gene segment; and
(c) the constant region is an IGHG4 constant region;
wherein the method comprises expressing the antibody from a CHO cell and obtaining the antibody.

17. An antibody comprising a heavy chain, the heavy chain comprising a variable domain that is specific for an antigen and a constant region, wherein the variable domain is encoded by a variable region nucleotide sequence produced by recombination in a transgenic mouse of a human VH gene segment with a J gene segment and a D gene segment; wherein
(a) the VH gene segment is a human IGHV3-23 (e.g., V3-23*04) gene segment;
(b) the J gene segment is a human IGHJ4 (e.g., JH4*01) or IGHJ6 (e.g., JH6*02) gene segment; and
(c) the constant region is an IGHG4 constant region.


SEQUENCE LISTING

<110>  Kymab Limited

<120>  Synthetically -Extended & Ethnically-Diverse Superhuman
       Immunoglobin Gene Repertoires

<130>  25077.372

<160>  445

<170>  PatentIn version 3.5

<210>  1
<211>  49
<212>  DNA
<213>  homo sapiens;

<400>  1
ttgaccaagc tggggacccc ggtcccttgg gaccagtggc agaggagtc                49

<210>  2
<211>  61
<212>  DNA
<213>  homo sapiens;

<400>  2
atgatgatga tgatgatgta cctgcagacc ccgtttccct ggtgccagtg gcagaggagt    60

c                                                                    61

<210>  3
<211>  52
<212>  DNA
<213>  homo sapiens;

<400>  3
atgaccatga agctagagac cccggcaccg tgggaccagt gacagaggag tc            52

<210>  4
<211>  61
<212>  DNA
<213>  homo sapiens;

<400>  4
atgatgatga tgatgccata cctgcagacc ccggttccct ggtgccagtg gcagaggagt    60

c                                                                    61

<210>  5
<211>  49
<212>  DNA
<213>  homo sapiens;

<400>  5
aactggttcg acccctgggg ccagggaacc ctggtcaccg tctcctcag                49

<210>  6

<211>  16
<212>  PRT
<213>  homo sapiens;

<400>  6

Asn Trp Phe Asp Pro Trp Gly Gln Gly Thr Leu Val Thr Val Ser Ser
1               5                   10                  15

<210>  7
<211>  61
<212>  DNA
<213>  homo sapiens;

<400>  7
tactactact actactacat ggacgtctgg ggcaaaggga ccacggtcac cgtctcctca      60

g                                                                      61

<210>  8
<211>  20
<212>  PRT
<213>  homo sapiens;

<400>  8

Tyr Tyr Tyr Tyr Tyr Tyr Met Asp Val Trp Gly Lys Gly Thr Thr Val
1               5                   10                  15

Thr Val Ser Ser
            20

<210>  9
<211>  52
<212>  DNA
<213>  homo sapiens;

<400>  9
tactggtact tcgatctctg gggccgtggc accctggtca ctgtctcctc ag              52

<210>  10
<211>  17
<212>  PRT
<213>  homo sapiens;

<400>  10

Tyr Trp Tyr Phe Asp Leu Trp Gly Arg Gly Thr Leu Val Thr Val Ser
1               5                   10                  15

Ser

<210>  11
<211>  296
<212>  DNA

<213>  homo sapiens;

<400>  11
caggtgcagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggacgg atcaacccta acagtggtgg cacaaactat     180

gcacagaagt ttcagggcag ggtcaccagt accagggaca cgtccatcag cacagcctac     240

atggagctga gcaggctgag atctgacgac acggtcgtgt attactgtgc gagaga         296


<210>  12
<211>  296
<212>  DNA
<213>  homo sapiens;

<400>  12
caggtccagc ttgtgcagtc tggggctgag gtgaagaagc ctggggcctc agtgaaggtt      60

tcctgcaagg cttctggata caccttcact agctatgcta tgcattgggt gcgccaggcc     120

cccggacaaa ggcttgagtg gatgggatgg atcaacgctg gcaatggtaa cacaaaatat     180

tcacagaagt tccagggcag agtcaccatt accagggaca catccgcgag cacagcctac     240

atggagctga gcagcctgag atctgaagac acggctgtgt attactgtgc gagaga         296


<210>  13
<211>  296
<212>  DNA
<213>  homo sapiens;

<400>  13
caggtgcagc tggtgcagtc tggggctgag gtgaagaagc ctggggcctc agtgaaggtc      60

tcctgcaagg cttctggata caccttcacc agttatgata tcaactgggt gcgacaggcc     120

actggacaag ggcttgagtg gatgggatgg atgaacccta acagtggtaa cacaggctat     180

gcacagaagt tccagggcag agtcaccatg accaggaaca cctccataag cacagcctac     240

atggagctga gcagcctgag atctgaggac acggccgtgt attactgtgc gagagg         296


<210>  14
<211>  296
<212>  DNA
<213>  homo sapiens;

<400>  14
caggtccagc tggtacagtc tggggctgag gtgaagaagc ctggggcctc agtgaaggtc      60

tcctgcaagg tttccggata caccctcact gaattatcca tgcactgggt gcgacaggct     120

cctggaaaag ggcttgagtg gatgggaggt tttgatcctg aagatggtga aacaatctac     180

gcacagaagt tccagggcag agtcaccatg accgaggaca catctacaga cacagcctac     240

atggagctga gcagcctgag atctgaggac acggccgtgt attactgtgc aacaga         296

<210>  15
<211>  296
<212>  DNA
<213>  homo sapiens;

<220>
<221>  misc_feature
<222>  (295)..(295)
<223>  n is a, c, g, or t

<400>  15
cagatgcagc tggtgcagtc tggggctgag gtgaagaaga ctgggtcctc agtgaaggtt      60

tcctgcaagg cttccggata caccttcacc taccgctacc tgcactgggt gcgacaggcc     120

cccggacaag cgcttgagtg gatgggatgg atcacacctt tcaatggtaa caccaactac     180

gcacagaaat tccaggacag agtcaccatt actagggaca ggtctatgag cacagcctac     240

atggagctga gcagcctgag atctgaggac acagccatgt attactgtgc aagana         296

<210>  16
<211>  296
<212>  DNA
<213>  homo sapiens;

<400>  16
caggtgcagc tggtgcagtc tggggctgag gtgaagaagc ctggggcctc agtgaaggtt      60

tcctgcaagg catctggata caccttcacc agctactata tgcactgggt gcgacaggcc     120

cctggacaag ggcttgagtg gatgggaata atcaacccta gtggtggtag cacaagctac     180

gcacagaagt tccagggcag agtcaccatg accagggaca cgtccacgag cacagtctac     240

atggagctga gcagcctgag atctgaggac acggccgtgt attactgtgc gagaga         296

<210>  17
<211>  296
<212>  DNA
<213>  homo sapiens;

<400>  17
caaatgcagc tggtgcagtc tgggcctgag gtgaagaagc ctgggacctc agtgaaggtc      60

tcctgcaagg cttctggatt cacctttact agctctgctg tgcagtgggt gcgacaggct     120

cgtggacaac gccttgagtg gataggatgg atcgtcgttg gcagtggtaa cacaaactac     180

gcacagaagt tccaggaaag agtcaccatt accagggaca tgtccacaag cacagcctac     240

atggagctga gcagcctgag atccgaggac acggccgtgt attactgtgc ggcaga         296

<210>  18
<211>  296
<212>  DNA
<213>  homo sapiens;

<400>  18
caggtgcagc tggtgcagtc tggggctgag gtgaagaagc ctgggtcctc ggtgaaggtc      60

tcctgcaagg cttctggagg caccttcagc agctatgcta tcagctgggt gcgacaggcc     120

cctggacaag ggcttgagtg gatgggaggg atcatcccta tctttggtac agcaaactac     180

gcacagaagt tccagggcag agtcacgatt accgcggacg aatccacgag cacagcctac     240

atggagctga gcagcctgag atctgaggac acggccgtgt attactgtgc gagaga         296


<210>  19
<211>  294
<212>  DNA
<213>  homo sapiens;

<400>  19
caggtccagc tggtgcagtc ttgggctgag gtgaggaagt ctggggcctc agtgaaagtc      60

tcctgtagtt tttctgggtt taccatcacc agctacggta tacattgggt gcaacagtcc     120

cctggacaag ggcttgagtg gatgggatgg atcaaccctg gcaatggtag cccaagctat     180

gccaagaagt ttcagggcag attcaccatg accagggaca tgtccacaac cacagcctac      240

acagacctga gcagcctgac atctgaggac atggctgtgt attactatgc aaga           294


<210>  20
<211>  294
<212>  DNA
<213>  homo sapiens;

<400>  20
gaggtccagc tggtacagtc tggggctgag gtgaagaagc ctggggctac agtgaaaatc      60

tcctgcaagg tttctggata caccttcacc gactactaca tgcactgggt gcaacaggcc     120

cctggaaaag ggcttgagtg gatgggactt gttgatcctg aagatggtga aacaatatac     180

gcagagaagt tccagggcag agtcaccata accgcggaca cgtctacaga cacagcctac     240

atggagctga gcagcctgag atctgaggac acggccgtgt attactgtgc aaca           294


<210>  21
<211>  302
<212>  DNA
<213>  homo sapiens;

<400>  21
cagatcacct tgaaggagtc tggtcctacg ctggtgaaac ccacacagac cctcacgctg      60

acctgcacct tctctgggtt ctcactcagc actagtggag tgggtgtggg ctggatccgt     120

cagcccccag gaaaggccct ggagtggctt gcactcattt attggaatga tgataagcgc     180

tacagcccat ctctgaagag caggctcacc atcaccaagg acacctccaa aaaccaggtg     240

gtccttacaa tgaccaacat ggaccctgtg gacacagcca catattactg tgcacacaga     300

cc                                                                 302

<210>  22
<211>  301
<212>  DNA
<213>  homo sapiens;

<400>  22
caggtcacct tgaaggagtc tggtcctgtg ctggtgaaac ccacagagac cctcacgctg      60

acctgcaccg tctctgggtt ctcactcagc aatgctagaa tgggtgtgag ctggatccgt     120

cagcccccag ggaaggccct ggagtggctt gcacacattt tttcgaatga cgaaaaatcc     180

tacagcacat ctctgaagag caggctcacc atctccaagg acacctccaa aagccaggtg     240

gtccttacca tgaccaacat ggaccctgtg gacacagcca catattactg tgcacggata     300

c                                                                 301

<210>  23
<211>  301
<212>  DNA
<213>  homo sapiens;

<400>  23
caggtcacct tgagggagtc tggtcctgcg ctggtgaaac ccacacagac cctcacactg      60

acctgcacct tctctgggtt ctcactcagc actagtggaa tgtgtgtgag ctggatccgt     120

cagcccccag ggaaggccct ggagtggctt gcactcattg attgggatga tgataaatac     180

tacagcacat ctctgaagac caggctcacc atctccaagg acacctccaa aaaccaggtg     240

gtccttacaa tgaccaacat ggaccctgtg gacacagcca cgtattactg tgcacggata     300

c                                                                 301

<210>  24
<211>  296
<212>  DNA
<213>  homo sapiens;

<400>  24
gaggtgcagc tggtggagtc tgggggaggc ttggtccagc ctggggggtc cctgagactc      60

tcctgtgcag cctctggatt cacctttagt agctattgga tgagctgggt ccgccaggct     120

ccagggaagg ggctggagtg ggtggccaac ataaagcaag atggaagtga gaaatactat     180

gtggactctg tgaagggccg attcaccatc tccagagaca acgccaagaa ctcactgtat     240

ctgcaaatga acagcctgag agccgaggac acggctgtgt attactgtgc gagaga        296

<210>  25
<211>  298
<212>  DNA
<213>  homo sapiens;

<400>  25
gaagtgcagc tggtggagtc tgggggaggc ttggtacagc ctggcaggtc cctgagactc      60

tcctgtgcag cctctggatt cacctttgat gattatgcca tgcactgggt ccggcaagct     120

ccagggaagg gcctggagtg ggtctcaggt attagttgga atagtggtag cataggctat     180

gcggactctg tgaagggccg attcaccatc tccagagaca acgccaagaa ctccctgtat     240

ctgcaaatga acagtctgag agctgaggac acggccttgt attactgtgc aaaagata      298


<210>  26
<211>  296
<212>  DNA
<213>  homo sapiens;

<400>  26
caggtgcagc tggtggagtc tgggggaggc ttggtcaagc ctggagggtc cctgagactc      60

tcctgtgcag cctctggatt caccttcagt gactactaca tgagctggat ccgccaggct     120

ccagggaagg ggctggagtg ggtttcatac attagtagta gtggtagtac catatactac     180

gcagactctg tgaagggccg attcaccatc tccagggaca acgccaagaa ctcactgtat     240

ctgcaaatga acagcctgag agccgaggac acggccgtgt attactgtgc gagaga        296


<210>  27
<211>  293
<212>  DNA
<213>  homo sapiens;

<400>  27
gaggtgcagc tggtggagtc tgggggaggc ttggtacagc ctggggggtc cctgagactc      60

tcctgtgcag cctctggatt caccttcagt agctacgaca tgcactgggt ccgccaagct     120

acaggaaaag gtctggagtg ggtctcagct attggtactg ctggtgacac atactatcca     180

ggctccgtga agggccgatt caccatctcc agagaaaatg ccaagaactc cttgtatctt     240

caaatgaaca gcctgagagc cggggacacg gctgtgtatt actgtgcaag aga           293


<210>  28
<211>  302
<212>  DNA
<213>  homo sapiens;

<400>  28
gaggtgcagc tggtggagtc tgggggaggc ttggtaaagc ctggggggtc ccttagactc      60

tcctgtgcag cctctggatt cactttcagt aacgcctgga tgagctgggt ccgccaggct     120

ccagggaagg ggctggagtg ggttggccgt attaaaagca aaactgatgg tgggacaaca     180

gactacgctg cacccgtgaa aggcagattc accatctcaa gagatgattc aaaaaacacg     240

ctgtatctgc aaatgaacag cctgaaaacc gaggacacag ccgtgtatta ctgtaccaca     300

ga                                                                    302

<210>  29
<211>  296
<212>  DNA
<213>  homo sapiens;

<400>  29
gaggtacaac tggtggagtc tgggggaggc ttggtacagc ctggggggtc cctgagactc      60

tcctgtgcag cctctggatt caccttcagt aacagtgaca tgaactgggc ccgcaaggct     120

ccaggaaagg ggctggagtg ggtatcgggt gttagttgga atggcagtag gacgcactat     180

gtggactccg tgaagcgccg attcatcatc tccagagaca attccaggaa ctccctgtat     240

ctgcaaaaga acagacggag agccgaggac atggctgtgt attactgtgt gagaaa        296


<210>  30
<211>  296
<212>  DNA
<213>  homo sapiens;

<400>  30
acagtgcagc tggtggagtc tgggggaggc ttggtagagc ctggggggtc cctgagactc      60

tcctgtgcag cctctggatt caccttcagt aacagtgaca tgaactgggt ccgccaggct     120

ccaggaaagg ggctggagtg ggtatcgggt gttagttgga atggcagtag gacgcactat     180

gcagactctg tgaagggccg attcatcatc tccagagaca attccaggaa cttcctgtat     240

cagcaaatga acagcctgag gcccgaggac atggctgtgt attactgtgt gagaaa        296


<210>  31
<211>  296
<212>  DNA
<213>  homo sapiens;

<400>  31
gaggtgcagc tggtggagtc tgggggaggt gtggtacggc ctggggggtc cctgagactc      60

tcctgtgcag cctctggatt cacctttgat gattatggca tgagctgggt ccgccaagct     120

ccagggaagg ggctggagtg ggtctctggt attaattgga atggtggtag cacaggttat     180

gcagactctg tgaagggccg attcaccatc tccagagaca acgccaagaa ctccctgtat     240

ctgcaaatga acagtctgag agccgaggac acggccttgt atcactgtgc gagaga        296


<210>  32
<211>  296
<212>  DNA
<213>  homo sapiens;

<400>  32
gaggtgcagc tggtggagtc tgggggaggc ctggtcaagc ctggggggtc cctgagactc      60

tcctgtgcag cctctggatt caccttcagt agctatagca tgaactgggt ccgccaggct     120

ccagggaagg ggctggagtg ggtctcatcc attagtagta gtagtagtta catatactac 180

gcagactcag tgaagggccg attcaccatc tccagagaca acgccaagaa ctcactgtat 240

ctgcaaatga acagcctgag agccgaggac acggctgtgt attactgtgc gagaga 296

<210> 33
<211> 296
<212> DNA
<213> homo sapiens;

<400> 33
gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtat attactgtgc gaaaga 296

<210> 34
<211> 296
<212> DNA
<213> homo sapiens;

<400> 34
caggtgcagc tggtggagtc tgggggaggc gtggtccagc ctgggaggtc cctgagactc 60

tcctgtgcag cctctggatt caccttcagt agctatgcta tgcactgggt ccgccaggct 120

ccaggcaagg ggctagagtg ggtggcagtt atatcatatg atggaagtaa taaatactac 180

gcagactccg tgaagggccg attcaccatc tccagagaca attccaagaa cacgctgtat 240

ctgcaaatga acagcctgag agctgaggac acggctgtgt attactgtgc gagaga 296

<210> 35
<211> 294
<212> DNA
<213> homo sapiens;

<400> 35
caggtgcagc tggtggagtc tgggggaggc gtggtccagc ctgggaggtc cctgagactc 60

tcctgtgcag cctctggatt caccttcagt agctatgcta tgcactgggt ccgccaggct 120

ccaggcaagg ggctggagtg ggtggcagtt atatcatatg atggaagcaa taaatactac 180

gcagactccg tgaagggccg attcaccatc tccagagaca attccaagaa cacgctgtat 240

ctgcaaatga acagcctgag agctgaggac acggctgtgt attactgtgc gaga 294

<210> 36
<211> 296
<212> DNA
<213> homo sapiens;

<400>  36
caggtgcagc tggtggagtc tgggggaggc gtggtccagc ctgggaggtc cctgagactc      60

tcctgtgcag cgtctggatt caccttcagt agctatggca tgcactgggt ccgccaggct     120

ccaggcaagg ggctggagtg ggtggcagtt atatggtatg atggaagtaa taaatactat     180

gcagactccg tgaagggccg attcaccatc tccagagaca attccaagaa cacgctgtat     240

ctgcaaatga acagcctgag agccgaggac acggctgtgt attactgtgc gagaga        296


<210>  37
<211>  296
<212>  DNA
<213>  homo sapiens;

<400>  37
gaggtgcagc tggtggagtc tgggggaggc ttggtacagc ctgggggatc cctgagactc      60

tcctgtgcag cctctggatt caccttcagt aacagtgaca tgaactgggt ccatcaggct     120

ccaggaaagg ggctggagtg ggtatcgggt gttagttgga atggcagtag gacgcactat     180

gcagactctg tgaagggccg attcatcatc tccagagaca attccaggaa caccctgtat     240

ctgcaaacga atagcctgag ggccgaggac acggctgtgt attactgtgt gagaaa        296


<210>  38
<211>  292
<212>  DNA
<213>  homo sapiens;

<400>  38
gaggtgcagc tggtggagtc tgggggaggc ttggtacagc ctagggggtc cctgagactc      60

tcctgtgcag cctctggatt caccgtcagt agcaatgaga tgagctggat ccgccaggct     120

ccagggaagg ggctggagtg ggtctcatcc attagtggtg gtagcacata ctacgcagac     180

tccaggaagg gcagattcac catctccaga gacaattcca agaacacgct gtatcttcaa     240

atgaacaacc tgagagctga gggcacggcc gcgtattact gtgccagata ta            292


<210>  39
<211>  298
<212>  DNA
<213>  homo sapiens;

<400>  39
gaagtgcagc tggtggagtc tgggggagtc gtggtacagc ctggggggtc cctgagactc      60

tcctgtgcag cctctggatt cacctttgat gattatacca tgcactgggt ccgtcaagct     120

ccggggaagg gtctggagtg ggtctctctt attagttggg atggtggtag cacatactat     180

gcagactctg tgaagggccg attcaccatc tccagagaca acagcaaaaa ctccctgtat     240

ctgcaaatga acagtctgag aactgaggac accgccttgt attactgtgc aaaagata      298

<210>  40
<211>  291
<212>  DNA
<213>  homo sapiens;

<400>  40
gaggatcagc tggtggagtc tgggggaggc ttggtacagc ctggggggtc cctgcgaccc      60

tcctgtgcag cctctggatt cgccttcagt agctatgctc tgcactgggt tcgccgggct      120

ccagggaagg gtctggagtg ggtatcagct attggtactg gtggtgatac atactatgca      180

gactccgtga tgggccgatt caccatctcc agagacaacg ccaagaagtc cttgtatctt      240

catatgaaca gcctgatagc tgaggacatg gctgtgtatt attgtgcaag a      291


<210>  41
<211>  296
<212>  DNA
<213>  homo sapiens;

<400>  41
gaggtgcagc tggtggagtc tgggggaggc ttggtacagc ctggggggtc cctgagactc      60

tcctgtgcag cctctggatt caccttcagt agctatagca tgaactgggt ccgccaggct      120

ccagggaagg ggctggagtg ggtttcatac attagtagta gtagtagtac catatactac      180

gcagactctg tgaagggccg attcaccatc tccagagaca atgccaagaa ctcactgtat      240

ctgcaaatga acagcctgag agccgaggac acggctgtgt attactgtgc gagaga      296


<210>  42
<211>  302
<212>  DNA
<213>  homo sapiens;

<400>  42
gaggtgcagc tggtggagtc tgggggaggc ttggtacagc cagggcggtc cctgagactc      60

tcctgtacag cttctggatt cacctttggt gattatgcta tgagctggtt ccgccaggct      120

ccagggaagg ggctggagtg ggtaggtttc attagaagca aagcttatgg tgggacaaca      180

gaatacaccg cgtctgtgaa aggcagattc accatctcaa gagatggttc caaaagcatc      240

gcctatctgc aaatgaacag cctgaaaacc gaggacacag ccgtgtatta ctgtactaga      300

ga      302


<210>  43
<211>  293
<212>  DNA
<213>  homo sapiens;

<400>  43
gaggtgcagc tggtggagtc tggaggaggc ttgatccagc ctggggggtc cctgagactc      60

tcctgtgcag cctctgggtt caccgtcagt agcaactaca tgagctgggt ccgccaggct      120

ccagggaagg ggctggagtg ggtctcagtt atttatagcg gtggtagcac atactacgca 180

gactccgtga agggccgatt caccatctcc agagacaatt ccaagaacac gctgtatctt 240

caaatgaaca gcctgagagc cgaggacacg gccgtgtatt actgtgcgag aga 293

<210> 44
<211> 296
<212> DNA
<213> homo sapiens;

<400> 44
gaggtgcagc tggtggagtc tgggggaggc ttggtccagc ctggagggtc cctgagactc 60

tcctgtgcag cctctggatt caccttcagt agctatgcta tgcactgggt ccgccaggct 120

ccagggaagg gactggaata tgtttcagct attagtagta atgggggtag cacatattat 180

gcaaactctg tgaagggcag attcaccatc tccagagaca attccaagaa cacgctgtat 240

cttcaaatgg gcagcctgag agctgaggac atggctgtgt attactgtgc gagaga 296

<210> 45
<211> 293
<212> DNA
<213> homo sapiens;

<400> 45
gaggtgcagc tggtggagtc tgggggaggc ttggtccagc ctggagggtc cctgagactc 60

tcctgtgcag cctctggatt caccgtcagt agcaactaca tgagctgggt ccgccaggct 120

ccagggaagg ggctggagtg ggtctcagtt atttatagcg gtggtagcac atactacgca 180

gactccgtga agggcagatt caccatctcc agagacaatt ccaagaacac gctgtatctt 240

caaatgaaca gcctgagagc cgaggacacg gctgtgtatt actgtgcgag aga 293

<210> 46
<211> 302
<212> DNA
<213> homo sapiens;

<400> 46
gaggtgcagc tggtggagtc tgggggaggc ttggtccagc ctggagggtc cctgagactc 60

tcctgtgcag cctctggatt caccttcagt gaccactaca tggactgggt ccgccaggct 120

ccagggaagg ggctggagtg ggttggccgt actagaaaca aagctaacag ttacaccaca 180

gaatacgccg cgtctgtgaa aggcagattc accatctcaa gagatgattc aaagaactca 240

ctgtatctgc aaatgaacag cctgaaaacc gaggacacgg ccgtgtatta ctgtgctaga 300

ga 302

<210> 47
<211> 302
<212> DNA

<213>  homo sapiens;

<400>  47
gaggtgcagc tggtggagtc tgggggaggc ttggtccagc ctggggggtc cctgaaactc      60

tcctgtgcag cctctgggtt caccttcagt ggctctgcta tgcactgggt ccgccaggct     120

tccgggaaag ggctggagtg ggttggccgt attagaagca aagctaacag ttacgcgaca     180

gcatatgctg cgtcggtgaa aggcaggttc accatctcca gagatgattc aaagaacacg     240

gcgtatctgc aaatgaacag cctgaaaacc gaggacacgg ccgtgtatta ctgtactaga     300

ca                                                                    302


<210>  48
<211>  296
<212>  DNA
<213>  homo sapiens;

<400>  48
gaggtgcagc tggtggagtc cgggggaggc ttagttcagc ctggggggtc cctgagactc      60

tcctgtgcag cctctggatt caccttcagt agctactgga tgcactgggt ccgccaagct     120

ccagggaagg ggctggtgtg ggtctcacgt attaatagtg atgggagtag cacaagctac     180

gcggactccg tgaagggccg attcaccatc tccagagaca acgccaagaa cacgctgtat     240

ctgcaaatga acagtctgag agccgaggac acggctgtgt attactgtgc aagaga         296


<210>  49
<211>  288
<212>  DNA
<213>  homo sapiens;

<400>  49
gaggtgcagc tggtggagtc tcggggagtc ttggtacagc ctggggggtc cctgagactc      60

tcctgtgcag cctctggatt caccgtcagt agcaatgaga tgagctgggt ccgccaggct     120

ccagggaagg gtctggagtg ggtctcatcc attagtggtg gtagcacata ctacgcagac     180

tccaggaagg gcagattcac catctccaga gacaattcca agaacacgct gcatcttcaa     240

atgaacagcc tgagagctga ggacacggct gtgtattact gtaagaaa                  288


<210>  50
<211>  293
<212>  DNA
<213>  homo sapiens;

<400>  50
gaggtgcagc tggtggagtc tgggggaggc ttggtaaagc ctggggggtc cctgagactc      60

tcctgtgcag cctctggatt caccttcagt gactactaca tgaactgggt ccgccaggct     120

ccagggaagg ggctggagtg ggtctcatcc attagtagta gtagtaccat atactacgca     180

gactctgtga agggccgatt caccatctcc agagacaacg ccaagaactc actgtatctg     240

caaatgaaca gcctgagagc cgaggacacg gctgtgtatt actgtgcgag aga 293

<210> 51
<211> 296
<212> DNA
<213> homo sapiens;

<400> 51
caggtgcagc tgcaggagtc gggcccagga ctggtgaagc ctccggggac cctgtccctc 60

acctgcgctg tctctggtgg ctccatcagc agtagtaact ggtggagttg ggtccgccag 120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attgctgtgc gagaga 296

<210> 52
<211> 296
<212> DNA
<213> homo sapiens;

<400> 52
caggtgcagc tgcaggagtc gggcccagga ctggtgaagc cttcggacac cctgtccctc 60

acctgcgctg tctctggtta ctccatcagc agtagtaact ggtggggctg gatccggcag 120

cccccaggga agggactgga gtggattggg tacatctatt atagtgggag cacctactac 180

aacccgtccc tcaagagtcg agtcaccatg tcagtagaca cgtccaagaa ccagttctcc 240

ctgaagctga gctctgtgac cgccgtggac acggccgtgt attactgtgc gagaaa 296

<210> 53
<211> 299
<212> DNA
<213> homo sapiens;

<400> 53
cagctgcagc tgcaggagtc cggctcagga ctggtgaagc cttcacagac cctgtccctc 60

acctgcgctg tctctggtgg ctccatcagc agtggtggtt actcctggag ctggatccgg 120

cagccaccag ggaagggcct ggagtggatt gggtacatct atcatagtgg gagcacctac 180

tacaacccgt ccctcaagag tcgagtcacc atatcagtag acaggtccaa gaaccagttc 240

tccctgaagc tgagctctgt gaccgccgcg gacacggccg tgtattactg tgccagaga 299

<210> 54
<211> 299
<212> DNA
<213> homo sapiens;

<400> 54
caggtgcagc tgcaggagtc gggcccagga ctggtgaagc cttcacagac cctgtccctc 60

acctgcactg tctctggtgg ctccatcagc agtggtgatt actactggag ttggatccgc 120

cagcccccag ggaagggcct ggagtggatt gggtacatct attacagtgg gagcacctac 180

tacaacccgt ccctcaagag tcgagttacc atatcagtag acacgtccaa gaaccagttc 240

tccctgaagc tgagctctgt gactgccgca gacacggccg tgtattactg tgccagaga 299

<210> 55
<211> 299
<212> DNA
<213> homo sapiens;

<400> 55
caggtgcagc tgcaggagtc gggcccagga ctggtgaagc cttcacagac cctgtccctc 60

acctgcactg tctctggtgg ctccatcagc agtggtggtt actactggag ctggatccgc 120

cagcacccag ggaagggcct ggagtggatt gggtacatct attacagtgg gagcacctac 180

tacaacccgt ccctcaagag tctagttacc atatcagtag acacgtctaa gaaccagttc 240

tccctgaagc tgagctctgt gactgccgcg gacacggccg tgtattactg tgcgagaga 299

<210> 56
<211> 293
<212> DNA
<213> homo sapiens;

<400> 56
caggtgcagc tacagcagtg gggcgcagga ctgttgaagc cttcggagac cctgtccctc 60

acctgcgctg tctatggtgg gtccttcagt ggttactact ggagctggat ccgccagccc 120

ccagggaagg ggctggagtg gattggggaa atcaatcata gtggaagcac caactacaac 180

ccgtccctca agagtcgagt caccatatca gtagacacgt ccaagaacca gttctccctg 240

aagctgagct ctgtgaccgc cgcggacacg gctgtgtatt actgtgcgag agg 293

<210> 57
<211> 299
<212> DNA
<213> homo sapiens;

<400> 57
cagctgcagc tgcaggagtc gggcccagga ctggtgaagc cttcggagac cctgtccctc 60

acctgcactg tctctggtgg ctccatcagc agtagtagtt actactgggg ctggatccgc 120

cagcccccag ggaaggggct ggagtggatt gggagtatct attatagtgg gagcacctac 180

tacaacccgt ccctcaagag tcgagtcacc atatccgtag acacgtccaa gaaccagttc 240

tccctgaagc tgagctctgt gaccgccgca gacacggctg tgtattactg tgcgagaca 299

<210> 58
<211> 296
<212> DNA

<213>  homo sapiens;

<400>  58
caggtgcagc tgcaggagtc gggcccagga ctggtgaagc cttcggagac cctgtccctc      60

atctgcgctg tctctggtga ctccatcagc agtggtaact ggtgaatctg ggtccgccag     120

cccccaggga aggggctgga gtggattggg gaaatccatc atagtgggag cacctactac     180

aacccgtccc tcaagagtcg aatcaccatg tccgtagaca cgtccaagaa ccagttctac     240

ctgaagctga gctctgtgac cgccgcggac acggccgtgt attactgtgc gagata         296


<210>  59
<211>  293
<212>  DNA
<213>  homo sapiens;

<400>  59
caggtgcagc tgcaggagtc gggcccagga ctggtgaagc cttcggagac cctgtccctc      60

acctgcactg tctctggtgg ctccatcagt agttactact ggagctggat ccggcagccc     120

ccagggaagg gactggagtg gattgggtat atctattaca gtgggagcac caactacaac     180

ccctccctca agagtcgagt caccatatca gtagacacgt ccaagaacca gttctccctg     240

aagctgagct ctgtgaccgc tgcggacacg gccgtgtatt actgtgcgag aga            293


<210>  60
<211>  299
<212>  DNA
<213>  homo sapiens;

<400>  60
caggtgcagc tgcaggagtc gggcccagga ctggtgaagc cttcggagac cctgtccctc      60

acctgcactg tctctggtgg ctccgtcagc agtggtagtt actactggag ctggatccgg     120

cagcccccag ggaagggact ggagtggatt gggtatatct attacagtgg gagcaccaac     180

tacaacccct ccctcaagag tcgagtcacc atatcagtag acacgtccaa gaaccagttc     240

tccctgaagc tgagctctgt gaccgctgcg gacacggccg tgtattactg tgcgagaga      299


<210>  61
<211>  294
<212>  DNA
<213>  homo sapiens;

<400>  61
caggtgcagc tgcaggagtc gggcccagga ctggtgaagc cttcggagac cctgtccctc      60

acctgcgctg tctctggtta ctccatcagc agtggttact actggggctg gatccggcag     120

cccccaggga aggggctgga gtggattggg agtatctatc atagtgggag cacctactac     180

aacccgtccc tcaagagtcg agtcaccata tcagtagaca cgtccaagaa ccagttctcc     240

ctgaagctga gctctgtgac cgccgcagac acggccgtgt attactgtgc gaga           294

<210>  62
<211>  296
<212>  DNA
<213>  homo sapiens;

<400>  62
gaggtgcagc tggtgcagtc tggagcagag gtgaaaaagc ccggggagtc tctgaagatc      60

tcctgtaagg gttctggata cagctttacc agctactgga tcggctgggt gcgccagatg     120

cccgggaaag gcctggagtg gatggggatc atctatcctg gtgactctga taccagatac     180

agcccgtcct tccaaggcca ggtcaccatc tcagccgaca agtccatcag caccgcctac     240

ctgcagtgga gcagcctgaa ggcctcggac accgccatgt attactgtgc gagaca         296


<210>  63
<211>  294
<212>  DNA
<213>  homo sapiens;

<400>  63
gaagtgcagc tggtgcagtc tggagcagag gtgaaaaagc ccggggagtc tctgaggatc      60

tcctgtaagg gttctggata cagctttacc agctactgga tcagctgggt gcgccagatg     120

cccgggaaag gcctggagtg gatggggagg attgatccta gtgactctta taccaactac     180

agcccgtcct tccaaggcca cgtcaccatc tcagctgaca agtccatcag cactgcctac     240

ctgcagtgga gcagcctgaa ggcctcggac accgccatgt attactgtgc gaga           294


<210>  64
<211>  305
<212>  DNA
<213>  homo sapiens;

<400>  64
caggtacagc tgcagcagtc aggtccagga ctggtgaagc cctcgcagac cctctcactc      60

acctgtgcca tctccgggga cagtgtctct agcaacagtg ctgcttggaa ctggatcagg     120

cagtccccat cgagaggcct tgagtggctg ggaaggacat actacaggtc caagtggtat     180

aatgattatg cagtatctgt gaaaagtcga ataaccatca acccagacac atccaagaac     240

cagttctccc tgcagctgaa ctctgtgact cccgaggaca cggctgtgta ttactgtgca     300

agaga                                                                 305


<210>  65
<211>  294
<212>  DNA
<213>  homo sapiens;

<400>  65
caggtgcagc tggtgcaatc tgggtctgag ttgaagaagc ctggggcctc agtgaaggtt      60

tcctgcaagg cttctggata caccttcact agctatgcta tgaattgggt gcgacaggcc 120

cctggacaag ggcttgagtg gatgggatgg atcaacacca acactgggaa cccaacgtat 180

gcccagggct tcacaggacg gtttgtcttc tccttggaca cctctgtcag cacggcatat 240

ctgcagatct gcagcctaaa ggctgaggac actgccgtgt attactgtgc gaga 294

<210> 66
<211> 296
<212> DNA
<213> homo sapiens;

<400> 66
caggtgcagc tggtgcagtc tggccatgag gtgaagcagc ctggggcctc agtgaaggtc 60

tcctgcaagg cttctggtta cagtttcacc acctatggta tgaattgggt gccacaggcc 120

cctggacaag ggcttgagtg gatgggatgg ttcaacacct acactgggaa cccaacatat 180

gcccagggct tcacaggacg gtttgtcttc tccatggaca cctctgccag cacagcatac 240

ctgcagatca gcagcctaaa ggctgaggac atggccatgt attactgtgc gagata 296

<210> 67
<211> 17
<212> DNA
<213> homo sapiens;

<400> 67
ggtacaactg gaacgac 17

<210> 68
<211> 17
<212> DNA
<213> homo sapiens;

<400> 68
ggtataactg gaactac 17

<210> 69
<211> 17
<212> DNA
<213> homo sapiens;

<400> 69
ggtataaccg gaaccac 17

<210> 70
<211> 17
<212> DNA
<213> homo sapiens;

<400> 70
ggtataactg gaacgac 17

<210> 71

<211>  20
<212>  DNA
<213>  homo sapiens;

<400>  71
ggtatagtgg gagctactac                                                    20


<210>  72
<211>  31
<212>  DNA
<213>  homo sapiens;

<400>  72
aggatattgt agtagtacca gctgctatgc c                                       31


<210>  73
<211>  31
<212>  DNA
<213>  homo sapiens;

<400>  73
aggatattgt actaatggtg tatgctatac c                                       31


<210>  74
<211>  31
<212>  DNA
<213>  homo sapiens;

<400>  74
aggatattgt agtggtggta gctgctactc c                                       31


<210>  75
<211>  28
<212>  DNA
<213>  homo sapiens;

<400>  75
agcatattgt ggtggtgatt gctattcc                                           28


<210>  76
<211>  31
<212>  DNA
<213>  homo sapiens;

<400>  76
gtattacgat ttttggagtg gttattatac c                                       31


<210>  77
<211>  31
<212>  DNA
<213>  homo sapiens;

<400>  77
gtattacgat attttgactg gttattataa c                                       31


<210>  78

<211>  31
<212>  DNA
<213>  homo sapiens;

<400>  78
gtattactat ggttcgggga gttattataa c                                    31


<210>  79
<211>  37
<212>  DNA
<213>  homo sapiens;

<400>  79
gtattatgat tacgtttggg ggagttatgc ttatacc                              37


<210>  80
<211>  31
<212>  DNA
<213>  homo sapiens;

<400>  80
gtattactat gatagtagtg gttattacta c                                    31


<210>  81
<211>  16
<212>  DNA
<213>  homo sapiens;

<400>  81
tgactacagt aactac                                                     16


<210>  82
<211>  16
<212>  DNA
<213>  homo sapiens;

<400>  82
tgactacagt aactac                                                     16


<210>  83
<211>  16
<212>  DNA
<213>  homo sapiens;

<400>  83
tgactacggt gactac                                                     16


<210>  84
<211>  19
<212>  DNA
<213>  homo sapiens;

<400>  84
tgactacggt ggtaactcc                                                  19


<210>  85

<211>  20
<212>  DNA
<213>  homo sapiens;

<400>  85
gtggatacag ctatggttac                                                    20


<210>  86
<211>  23
<212>  DNA
<213>  homo sapiens;

<400>  86
gtggatatag tggctacgat tac                                                23


<210>  87
<211>  20
<212>  DNA
<213>  homo sapiens;

<400>  87
gtggatacag ctatggttac                                                    20


<210>  88
<211>  20
<212>  DNA
<213>  homo sapiens;

<400>  88
gtagagatgg ctacaattac                                                    20


<210>  89
<211>  18
<212>  DNA
<213>  homo sapiens;

<400>  89
gagtatagca gctcgtcc                                                      18


<210>  90
<211>  21
<212>  DNA
<213>  homo sapiens;

<400>  90
gggtatagca gcagctggta c                                                  21


<210>  91
<211>  21
<212>  DNA
<213>  homo sapiens;

<400>  91
gggtatagca gtggctggta c                                                  21


<210>  92

<211>  18
<212>  DNA
<213>  homo sapiens;

<400>  92
gggtatagca gcggctac                                                    18


<210>  93
<211>  11
<212>  DNA
<213>  homo sapiens;

<400>  93
ctaactgggg a                                                           11


<210>  94
<211>  52
<212>  DNA
<213>  homo sapiens;

<400>  94
gctgaatact tccagcactg gggccagggc accctggtca ccgtctcctc ag              52


<210>  95
<211>  53
<212>  DNA
<213>  homo sapiens;

<400>  95
ctactggtac ttcgatctct ggggccgtgg caccctggtc actgtctcct cag             53


<210>  96
<211>  50
<212>  DNA
<213>  homo sapiens;

<400>  96
tgatgctttt gatgtctggg gccaagggac aatggtcacc gtctcttcag                 50


<210>  97
<211>  48
<212>  DNA
<213>  homo sapiens;

<400>  97
actactttga ctactggggc caaggaaccc tggtcaccgt ctcctcag                   48


<210>  98
<211>  51
<212>  DNA
<213>  homo sapiens;

<400>  98
acaactggtt cgactcctgg ggccaaggaa ccctggtcac cgtctcctca g               51


<210>  99

<211>  63
<212>  DNA
<213>  homo sapiens;

<400>  99
attactacta ctactacggt atggacgtct gggggcaagg gaccacggtc accgtctcct      60

cag                                                                    63


<210>  100
<211>  287
<212>  DNA
<213>  homo sapiens;

<400>  100
gacatccaga tgacccagtc tccttccacc ctgtctgcat ctgtaggaga cagagtcacc      60

atcacttgcc gggccagtca gagtattagt agctggttgg cctggtatca gcagaaacca     120

gggaaagccc ctaagctcct gatctatgat gcctccagtt tggaaagtgg ggtcccatca     180

aggttcagcg gcagtggatc tgggacagaa ttcactctca ccatcagcag cctgcagcct     240

gatgattttg caacttatta ctgccaacag tataatagtt attctcc                   287


<210>  101
<211>  287
<212>  DNA
<213>  homo sapiens;

<400>  101
gccatccaga tgacccagtc tccatcctcc ctgtctgcat ctgtaggaga cagagtcacc      60

atcacttgcc gggcaagtca gggcattaga aatgatttag gctggtatca gcagaaacca     120

gggaaagccc ctaagctcct gatctatgct gcatccagtt tacaaagtgg ggtcccatca     180

aggttcagcg gcagtggatc tggcacagat ttcactctca ccatcagcag cctgcagcct     240

gaagattttg caacttatta ctgtctacaa gattacaatt accctcc                   287


<210>  102
<211>  287
<212>  DNA
<213>  homo sapiens;

<400>  102
gccatccgga tgacccagtc tccatcctca ttctctgcat ctacaggaga cagagtcacc      60

atcacttgtc gggcgagtca gggtattagc agttatttag cctggtatca gcaaaaacca     120

gggaaagccc ctaagctcct gatctatgct gcatccactt tgcaaagtgg ggtcccatca     180

aggttcagcg gcagtggatc tgggacagat ttcactctca ccatcagctg cctgcagtct     240

gaagattttg caacttatta ctgtcaacag tattatagtt accctcc                   287


<210>  103
<211>  287

<212>  DNA
<213>  homo sapiens;

<400>  103
gtcatctgga tgacccagtc tccatcctta ctctctgcat ctacaggaga cagagtcacc      60

atcagttgtc ggatgagtca gggcattagc agttatttag cctggtatca gcaaaaacca     120

gggaaagccc ctgagctcct gatctatgct gcatccactt tgcaaagtgg ggtcccatca     180

aggttcagtg gcagtggatc tgggacagat ttcactctca ccatcagttg cctgcagtct     240

gaagattttg caacttatta ctgtcaacag tattatagtt tccctcc                   287


<210>  104
<211>  287
<212>  DNA
<213>  homo sapiens;

<400>  104
gacatccagt tgacccagtc tccatccttc ctgtctgcat ctgtaggaga cagagtcacc      60

atcacttgcc gggccagtca gggcattagc agttatttag cctggtatca gcaaaaacca     120

gggaaagccc ctaagctcct gatctatgct gcatccactt tgcaaagtgg ggtcccatca     180

aggttcagcg gcagtggatc tgggacagaa ttcactctca caatcagcag cctgcagcct     240

gaagattttg caacttatta ctgtcaacag cttaatagtt accctcc                   287


<210>  105
<211>  287
<212>  DNA
<213>  homo sapiens;

<400>  105
gacatccaga tgacccagtc tccatcttcc gtgtctgcat ctgtaggaga cagagtcacc      60

atcacttgtc gggcgagtca gggtattagc agctggttag cctggtatca gcagaaacca     120

gggaaagccc ctaagctcct gatctatgct gcatccagtt tgcaaagtgg ggtcccatca     180

aggttcagcg gcagtggatc tgggacagat ttcactctca ccatcagcag cctgcagcct     240

gaagattttg caacttacta ttgtcaacag gctaacagtt tccctcc                   287


<210>  106
<211>  287
<212>  DNA
<213>  homo sapiens;

<400>  106
gccatccagt tgacccagtc tccatcctcc ctgtctgcat ctgtaggaga cagagtcacc      60

atcacttgcc gggcaagtca gggcattagc agtgctttag cctgatatca gcagaaacca     120

gggaaagctc ctaagctcct gatctatgat gcctccagtt tggaaagtgg ggtcccatca     180

aggttcagcg gcagtggatc tgggacagat ttcactctca ccatcagcag cctgcagcct     240

gaagattttg caacttatta ctgtcaacag tttaataatt accctca 287

<210> 107
<211> 287
<212> DNA
<213> homo sapiens;

<400> 107
gacatccaga tgacccagtc tccatcctca ctgtctgcat ctgtaggaga cagagtcacc 60

atcacttgtc gggcgagtca gggcattagc aattatttag cctggtttca gcagaaacca 120

gggaaagccc ctaagtccct gatctatgct gcatccagtt tgcaaagtgg ggtcccatca 180

aggttcagcg gcagtggatc tgggacagat ttcactctca ccatcagcag cctgcagcct 240

gaagattttg caacttatta ctgccaacag tataatagtt accctcc 287

<210> 108
<211> 287
<212> DNA
<213> homo sapiens;

<400> 108
gacatccaga tgacccagtc tccatcctca ctgtctgcat ctgtaggaga cagagtcacc 60

atcacttgtc gggcgagtca gggtattagc agctggttag cctggtatca gcagaaacca 120

gagaaagccc ctaagtccct gatctatgct gcatccagtt tgcaaagtgg ggtcccatca 180

aggttcagcg gcagtggatc tgggacagat ttcactctca ccatcagcag cctgcagcct 240

gaagattttg caacttatta ctgccaacag tataatagtt accctcc 287

<210> 109
<211> 287
<212> DNA
<213> homo sapiens;

<400> 109
gacatccaga tgacccagtc tccatcctcc ctgtctgcat ctgtaggaga cagagtcacc 60

atcacttgcc gggcaagtca gggcattaga aatgatttag gctggtatca gcagaaacca 120

gggaaagccc ctaagcgcct gatctatgct gcatccagtt tgcaaagtgg ggtcccatca 180

aggttcagcg gcagtggatc tgggacagaa ttcactctca caatcagcag cctgcagcct 240

gaagattttg caacttatta ctgtctacag cataatagtt accctcc 287

<210> 110
<211> 287
<212> DNA
<213> homo sapiens;

<400> 110
aacatccaga tgacccagtc tccatctgcc atgtctgcat ctgtaggaga cagagtcacc 60

atcacttgtc gggcgaggca gggcattagc aattatttag cctggtttca gcagaaacca 120

gggaaagtcc ctaagcacct gatctatgct gcatccagtt tgcaaagtgg ggtcccatca 180

aggttcagcg gcagtggatc tgggacagaa ttcactctca caatcagcag cctgcagcct 240

gaagattttg caacttatta ctgtctacag cataatagtt accctcc 287

<210> 111
<211> 287
<212> DNA
<213> homo sapiens;

<400> 111
gacatccaga tgacccagtc tccatcctcc ctgtctgcat ctgtaggaga cagagtcacc 60

atcacttgcc gggcgagtca gggcattagc aattatttag cctggtatca gcagaaacca 120

gggaaagttc ctaagctcct gatctatgct gcatccactt tgcaatcagg ggtcccatct 180

cggttcagtg gcagtggatc tgggacagat ttcactctca ccatcagcag cctgcagcct 240

gaagatgttg caacttatta ctgtcaaaag tataacagtg cccctcc 287

<210> 112
<211> 287
<212> DNA
<213> homo sapiens;

<400> 112
gacatccaga tgacccagtc tccatcctcc ctgtctgcat ctgtaggaga cagagtcacc 60

atcacttgcc aggcgagtca ggacattagc aactatttaa attggtatca gcagaaacca 120

gggaaagccc ctaagctcct gatctacgat gcatccaatt tggaaacagg ggtcccatca 180

aggttcagtg gaagtggatc tgggacagat tttactttca ccatcagcag cctgcagcct 240

gaagatattg caacatatta ctgtcaacag tatgataatc tccctcc 287

<210> 113
<211> 287
<212> DNA
<213> homo sapiens;

<400> 113
gacatccaga tgacccagtc tccatcctcc ctgtctgcat ctgtaggaga cagagtcacc 60

atcacttgcc aggcgagtca ggacattagc aactatttaa attggtatca gcagaaacca 120

gggaaagccc ctaagctcct gatctacgat gcatccaatt tggaaacagg ggtcccatca 180

aggttcagtg gaagtggatc tgggacagat tttactttca ccatcagcag cctgcagcct 240

gaagatattg caacatatta ctgtcaacag tatgataatc tccctcc 287

<210> 114
<211> 287
<212> DNA
<213> homo sapiens;

<400>  114
gacatccagt tgacccagtc tccatcctcc ctgtctgcat ctgtaggaga cagagtcacc      60

atcacttgcc gggtgagtca gggcattagc agttatttaa attggtatcg gcagaaacca     120

gggaaagttc ctaagctcct gatctatagt gcatccaatt tgcaatctgg agtcccatct     180

cggttcagtg gcagtggatc tgggacagat ttcactctca ctatcagcag cctgcagcct     240

gaagatgttg caacttatta cggtcaacgg acttacaatg cccctcc                   287


<210>  115
<211>  287
<212>  DNA
<213>  homo sapiens;

<400>  115
gacatccagt tgacccagtc tccatcctcc ctgtctgcat ctgtaggaga cagagtcacc      60

atcacttgcc gggtgagtca gggcattagc agttatttaa attggtatcg gcagaaacca     120

gggaaagttc ctaagctcct gatctatagt gcatccaatt tgcaatctgg agtcccatct     180

cggttcagtg gcagtggatc tgggacagat ttcactctca ctatcagcag cctgcagcct     240

gaagatgttg caacttatta cggtcaacgg acttacaatg cccctcc                   287


<210>  116
<211>  287
<212>  DNA
<213>  homo sapiens;

<400>  116
gacatccaga tgacccagtc tccatcctcc ctgtctgcat ctgtaggaga cagagtcacc      60

atcacttgcc gggcaagtca gagcattagc agctatttaa attggtatca gcagaaacca     120

gggaaagccc ctaagctcct gatctatgct gcatccagtt tgcaaagtgg ggtcccatca     180

aggttcagtg gcagtggatc tgggacagat ttcactctca ccatcagcag tctgcaacct     240

gaagattttg caacttacta ctgtcaacag agttacagta cccctcc                   287


<210>  117
<211>  287
<212>  DNA
<213>  homo sapiens;

<400>  117
gacatccaga tgacccagtc tccatcctcc ctgtctgcat ctgtaggaga cagagtcacc      60

atcacttgcc gggcaagtca gagcattagc agctatttaa attggtatca gcagaaacca     120

gggaaagccc ctaagctcct gatctatgct gcatccagtt tgcaaagtgg ggtcccatca     180

aggttcagtg gcagtggatc tgggacagat ttcactctca ccatcagcag tctgcaacct     240

gaagattttg caacttacta ctgtcaacag agttacagta cccctcc                   287

<210>  118
<211>  287
<212>  DNA
<213>  homo sapiens;

<400>  118
gacatccaga tgatccagtc tccatctttc ctgtctgcat ctgtaggaga cagagtcagt      60

atcatttgct gggcaagtga gggcattagc agtaatttag cctggtatct gcagaaacca     120

gggaaatccc ctaagctctt cctctatgat gcaaaagatt tgcaccctgg ggtctcatcg     180

aggttcagtg gcaggggatc tgggacggat ttcactctca ccatcatcag cctgaagcct     240

gaagattttg cagcttatta ctgtaaacag gacttcagtt accctcc                   287


<210>  119
<211>  287
<212>  DNA
<213>  homo sapiens;

<400>  119
gccatccgga tgacccagtc tccattctcc ctgtctgcat ctgtaggaga cagagtcacc      60

atcacttgct gggccagtca gggcattagc agttatttag cctggtatca gcaaaaacca     120

gcaaaagccc ctaagctctt catctattat gcatccagtt tgcaaagtgg ggtcccatca     180

aggttcagcg gcagtggatc tgggacggat tacactctca ccatcagcag cctgcagcct     240

gaagattttg caacttatta ctgtcaacag tattatagta cccctcc                   287


<210>  120
<211>  302
<212>  DNA
<213>  homo sapiens;

<400>  120
gatattgtga tgacccagac tccactctcc tcacctgtca cccttggaca gccggcctcc      60

atctcctgca ggtctagtca aagcctcgta cacagtgatg gaaacaccta cttgagttgg     120

cttcagcaga ggccaggcca gcctccaaga ctcctaattt ataagatttc taaccggttc     180

tctggggtcc cagacagatt cagtggcagt ggggcaggga cagatttcac actgaaaatc     240

agcagggtgg aagctgagga tgtcggggtt tattactgca tgcaagctac acaatttcct     300

ca                                                                    302


<210>  121
<211>  302
<212>  DNA
<213>  homo sapiens;

<400>  121
gatattgtga tgacccagac tccactctcc tcgcctgtca cccttggaca gccggcctcc      60

atctccttca ggtctagtca aagcctcgta cacagtgatg gaaacaccta cttgagttgg     120

cttcagcaga ggccaggcca gcctccaaga ctcctaattt ataaggtttc taaccggttc 180

tctggggtcc cagacagatt cagtggcagt ggggcaggga cagatttcac actgaaaatc 240

agcagggtgg aagctgagga tgtcggggtt tattactgca cgcaagctac acaatttcct 300

ca 302

<210> 122
<211> 302
<212> DNA
<213> homo sapiens;

<400> 122
gatattgtga tgactcagtc tccactctcc ctgcccgtca cccctggaga gccggcctcc 60

atctcctgca ggtctagtca gagcctcctg catagtaatg gatacaacta tttggattgg 120

tacctgcaga agccagggca gtctccacag ctcctgatct atttgggttc taatcgggcc 180

tccggggtcc ctgacaggtt cagtggcagt ggatcaggca cagattttac actgaaaatc 240

agcagagtgg aggctgagga tgttggggtt tattactgca tgcaagctct acaaactcct 300

cc 302

<210> 123
<211> 302
<212> DNA
<213> homo sapiens;

<400> 123
gatattgtga tgactcagtc tccactctcc ctgcccgtca cccctggaga gccggcctcc 60

atctcctgca ggtctagtca gagcctcctg catagtaatg gatacaacta tttggattgg 120

tacctgcaga agccagggca gtctccacag ctcctgatct atttgggttc taatcgggcc 180

tccggggtcc ctgacaggtt cagtggcagt ggatcaggca cagattttac actgaaaatc 240

agcagagtgg aggctgagga tgttggggtt tattactgca tgcaagctct acaaactcct 300

cc 302

<210> 124
<211> 302
<212> DNA
<213> homo sapiens;

<400> 124
gatattgtga tgacccagac tccactctct ctgtccgtca cccctggaca gccggcctcc 60

atctcctgca agtctagtca gagcctcctg catagtgatg gaaagaccta tttgtattgg 120

tacctgcaga agccaggcca gtctccacag ctcctgatct atgaagtttc cagccggttc 180

tctggagtgc cagataggtt cagtggcagc gggtcaggga cagatttcac actgaaaatc 240

agccgggtgg aggctgagga tgttggggtt tattactgaa tgcaaggtat acaccttcct 300

cc                                                                302

<210>  125
<211>  302
<212>  DNA
<213>  homo sapiens;

<400>  125
gatattgtga tgacccagac tccactctct ctgtccgtca cccctggaca gccggcctcc      60

atctcctgca agtctagtca gagcctcctg catagtgatg gaaagaccta tttgtattgg     120

tacctgcaga agccaggcca gcctccacag ctcctgatct atgaagtttc caaccggttc     180

tctggagtgc cagataggtt cagtggcagc gggtcaggga cagatttcac actgaaaatc     240

agccgggtgg aggctgagga tgttggggtt tattactgca tgcaaagtat acagcttcct     300

cc                                                                302

<210>  126
<211>  302
<212>  DNA
<213>  homo sapiens;

<400>  126
gatgttgtga tgactcagtc tccactctcc ctgcccgtca cccttggaca gccggcctcc      60

atctcctgca ggtctagtca aagcctcgta tacagtgatg gaaacaccta cttgaattgg     120

tttcagcaga ggccaggcca atctccaagg cgcctaattt ataaggtttc taaccgggac     180

tctggggtcc cagacagatt cagcggcagt gggtcaggca ctgatttcac actgaaaatc     240

agcagggtgg aggctgagga tgttggggtt tattactgca tgcaaggtac acactggcct     300

cc                                                                302

<210>  127
<211>  302
<212>  DNA
<213>  homo sapiens;

<400>  127
gatgttgtga tgactcagtc tccactctcc ctgcccgtca cccttggaca gccggcctcc      60

atctcctgca ggtctagtca aagcctcgta tacagtgatg gaaacaccta cttgaattgg     120

tttcagcaga ggccaggcca atctccaagg cgcctaattt ataaggtttc taactgggac     180

tctggggtcc cagacagatt cagcggcagt gggtcaggca ctgatttcac actgaaaatc     240

agcagggtgg aggctgagga tgttggggtt tattactgca tgcaaggtac acactggcct     300

cc                                                                302

<210>  128
<211>  305

<212>  DNA
<213>  homo sapiens;

<400>  128
gatattgtga tgacccagac tccactctcc ctgcccgtca cccctggaga gccggcctcc      60

atctcctgca ggtctagtca gagcctcttg gatagtgatg atggaaacac ctatttggac     120

tggtacctgc agaagccagg gcagtctcca cagctcctga tctatacgct ttcctatcgg     180

gcctctggag tcccagacag gttcagtggc agtgggtcag gcactgattt cacactgaaa     240

atcagcaggg tggaggctga ggatgttgga gtttattact gcatgcaacg tatagagttt     300

ccttc                                                                 305


<210>  129
<211>  305
<212>  DNA
<213>  homo sapiens;

<400>  129
gatattgtga tgacccagac tccactctcc ctgcccgtca cccctggaga gccggcctcc      60

atctcctgca ggtctagtca gagcctcttg gatagtgatg atggaaacac ctatttggac     120

tggtacctgc agaagccagg gcagtctcca cagctcctga tctatacgct ttcctatcgg     180

gcctctggag tcccagacag gttcagtggc agtgggtcag gcactgattt cacactgaaa     240

atcagcaggg tggaggctga ggatgttgga gtttattact gcatgcaacg tatagagttt     300

ccttc                                                                 305


<210>  130
<211>  290
<212>  DNA
<213>  homo sapiens;

<400>  130
gaaattgtaa tgacacagtc tccacccacc ctgtctttgt ctccagggga aagagtcacc      60

ctctcctgca gggccagtca gagtgttagc agcagctact taacctggta tcagcagaaa     120

cctggccagg cgcccaggct cctcatctat ggtgcatcca ccagggccac tagcatccca     180

gccaggttca gtggcagtgg gtctgggaca gacttcactc tcaccatcag cagcctgcag     240

cctgaagatt ttgcagttta ttactgtcag caggatcata acttacctcc                290


<210>  131
<211>  290
<212>  DNA
<213>  homo sapiens;

<400>  131
gaaattgtaa tgacacagtc tccacccacc ctgtctttgt ctccagggga aagagtcacc      60

ctctcctgca gggccagtca gagtgttagc agcagctact taacctggta tcagcagaaa     120

cctggccagg cgcccaggct cctcatctat ggtgcatcca ccagggccac tagcatccca 180

gccaggttca gtggcagtgg gtctgggaga gacttcactc tcaccatcag cagcctgcag 240

cctgaagatt ttgcagttta ttactgtcag caggatcata acttacctcc 290

<210> 132
<211> 290
<212> DNA
<213> homo sapiens;

<400> 132
gaaattgtaa tgacacagtc tccagccacc ctgtctttgt ctccagggga aagagccacc 60

ctctcctgca gggccagtca gagtgttagc agcagctact tatcctggta ccagcagaaa 120

cctgggcagg ctcccaggct cctcatctat ggtgcatcca ccagggccac tggcatccca 180

gccaggttca gtggcagtgg gtctgggaca gacttcactc tcaccatcag cagcctgcag 240

cctgaagatt ttgcagttta ttactgtcag caggattata acttacctcc 290

<210> 133
<211> 287
<212> DNA
<213> homo sapiens;

<400> 133
gaaattgtgt tgacacagtc tccagccacc ctgtctttgt ctccagggga aagagccacc 60

ctctcctgca gggccagtca gagtgttagc agctacttag cctggtacca acagaaacct 120

ggccaggctc ccaggctcct catctatgat gcatccaaca gggccactgg catcccagcc 180

aggttcagtg gcagtgggtc tgggacagac ttcactctca ccatcagcag cctagagcct 240

gaagattttg cagtttatta ctgtcagcag cgtagcaact ggcctcc 287

<210> 134
<211> 287
<212> DNA
<213> homo sapiens;

<400> 134
gaaattgtgt tgacacagtc tccagccacc ctgtctttgt ctccagggga aagagccacc 60

ctctcctgca gggccagtca gggtgttagc agctacttag cctggtacca gcagaaacct 120

ggccaggctc ccaggctcct catctatgat gcatccaaca gggccactgg catcccagcc 180

aggttcagtg gcagtgggcc tgggacagac ttcactctca ccatcagcag cctagagcct 240

gaagattttg cagtttatta ctgtcagcag cgtagcaact ggcatcc 287

<210> 135
<211> 287
<212> DNA
<213> homo sapiens;

<400>  135
gaaatagtga tgacgcagtc tccagccacc ctgtctgtgt ctccagggga aagagccacc      60

ctctcctgca gggccagtca gagtgttagc agcaacttag cctggtacca gcagaaacct     120

ggccaggctc ccaggctcct catctatggt gcatccacca gggccactgg tatcccagcc     180

aggttcagtg gcagtgggtc tgggacagag ttcactctca ccatcagcag cctgcagtct     240

gaagattttg cagtttatta ctgtcagcag tataataact ggcctcc                   287


<210>  136
<211>  287
<212>  DNA
<213>  homo sapiens;

<400>  136
gaaatagtga tgacgcagtc tccagccacc ctgtctgtgt ctccagggga aagagccacc      60

ctctcctgca gggccagtca gagtgttagc agcaacttag cctggtacca gcagaaacct     120

ggccaggctc ccaggctcct catctatggt gcatccacca gggccactgg catcccagcc     180

aggttcagtg gcagtgggtc tgggacagag ttcactctca ccatcagcag cctgcagtct     240

gaagattttg cagtttatta ctgtcagcag tataataact ggcctcc                   287


<210>  137
<211>  290
<212>  DNA
<213>  homo sapiens;

<400>  137
gaaattgtgt tgacgcagtc tccaggcacc ctgtctttgt ctccagggga aagagccacc      60

ctctcctgca gggccagtca gagtgttagc agcagctact tagcctggta ccagcagaaa     120

cctggccagg ctcccaggct cctcatctat ggtgcatcca gcagggccac tggcatccca     180

gacaggttca gtggcagtgg gtctgggaca gacttcactc tcaccatcag cagactggag     240

cctgaagatt ttgcagtgta ttactgtcag cagtatggta gctcacctcc                290


<210>  138
<211>  290
<212>  DNA
<213>  homo sapiens;

<400>  138
gaaattgtgt tgacgcagtc tccagccacc ctgtctttgt ctccagggga aagagccacc      60

ctctcctgcg gggccagtca gagtgttagc agcagctact tagcctggta ccagcagaaa     120

cctggcctgg cgcccaggct cctcatctat gatgcatcca gcagggccac tggcatccca     180

gacaggttca gtggcagtgg gtctgggaca gacttcactc tcaccatcag cagactggag     240

cctgaagatt ttgcagtgta ttactgtcag cagtatggta gctcacctcc                290

<210>  139
<211>  305
<212>  DNA
<213>  homo sapiens;

<400>  139
gacatcgtga tgacccagtc tccagactcc ctggctgtgt ctctgggcga gagggccacc      60

atcaactgca agtccagcca gagtgtttta tacagctcca acaataagaa ctacttagct     120

tggtaccagc agaaaccagg acagcctcct aagctgctca tttactgggc atctacccgg     180

gaatccgggg tccctgaccg attcagtggc agcgggtctg ggacagattt cactctcacc     240

atcagcagcc tgcaggctga agatgtggca gtttattact gtcagcaata ttatagtact     300

cctcc                                                                 305


<210>  140
<211>  287
<212>  DNA
<213>  homo sapiens;

<400>  140
gaaacgacac tcacgcagtc tccagcattc atgtcagcga ctccaggaga caaagtcaac      60

atctcctgca aagccagcca agacattgat gatgatatga actggtacca acagaaacca     120

ggagaagctg ctattttcat tattcaagaa gctactactc tcgttcctgg aatcccacct     180

cgattcagtg gcagcgggta tggaacagat tttaccctca caattaataa catagaatct     240

gaggatgctg catattactt ctgtctacaa catgataatt tccctct                   287


<210>  141
<211>  287
<212>  DNA
<213>  homo sapiens;

<400>  141
gaaattgtgc tgactcagtc tccagacttt cagtctgtga ctccaaagga gaaagtcacc      60

atcacctgcc gggccagtca gagcattggt agtagcttac actggtacca gcagaaacca     120

gatcagtctc caaagctcct catcaagtat gcttcccagt ccttctcagg ggtcccctcg     180

aggttcagtg gcagtggatc tgggacagat ttcaccctca ccatcaatag cctggaagct     240

gaagatgctg caacgtatta ctgtcatcag agtagtagtt tacctca                   287


<210>  142
<211>  287
<212>  DNA
<213>  homo sapiens;

<400>  142
gaaattgtgc tgactcagtc tccagacttt cagtctgtga ctccaaagga gaaagtcacc      60

atcacctgcc gggccagtca gagcattggt agtagcttac actggtacca gcagaaacca     120

gatcagtctc caaagctcct catcaagtat gcttcccagt ccttctcagg ggtcccctcg 180

aggttcagtg gcagtggatc tgggacagat ttcaccctca ccatcaatag cctggaagct 240

gaagatgctg caacgtatta ctgtcatcag agtagtagtt tacctca 287

<210> 143
<211> 287
<212> DNA
<213> homo sapiens;

<400> 143
gatgttgtga tgacacagtc tccagctttc ctctctgtga ctccagggga gaaagtcacc 60

atcacctgcc aggccagtga aggcattggc aactacttat actggtacca gcagaaacca 120

gatcaagccc caaagctcct catcaagtat gcttcccagt ccatctcagg ggtcccctcg 180

aggttcagtg gcagtggatc tgggacagat ttcaccttta ccatcagtag cctggaagct 240

gaagatgctg caacatatta ctgtcagcag ggcaataagc accctca 287

<210> 144
<211> 296
<212> DNA
<213> homo sapiens;

<400> 144
cagtctgtgc tgactcagcc accctcggtg tctgaagccc ccaggcagag ggtcaccatc 60

tcctgttctg gaagcagctc caacatcgga aataatgctg taaactggta ccagcagctc 120

ccaggaaagg ctcccaaact cctcatctat tatgatgatc tgctgccctc aggggtctct 180

gaccgattct ctggctccaa gtctggcacc tcagcctccc tggccatcag tgggctccag 240

tctgaggatg aggctgatta ttactgtgca gcatgggatg acagcctgaa tggtcc 296

<210> 145
<211> 299
<212> DNA
<213> homo sapiens;

<400> 145
cagtctgtgc tgacgcagcc gccctcagtg tctggggccc cagggcagag ggtcaccatc 60

tcctgcactg ggagcagctc caacatcggg gcaggttatg atgtacactg gtaccagcag 120

cttccaggaa cagcccccaa actcctcatc tatggtaaca gcaatcggcc ctcaggggtc 180

cctgaccgat tctctggctc caagtctggc acctcagcct ccctggccat cactgggctc 240

caggctgagg atgaggctga ttattactgc cagtcctatg acagcagcct gagtggttc 299

<210> 146
<211> 296
<212> DNA
<213> homo sapiens;

<400>  146
cagtctgtgt tgacgcagcc gccttcagtg tctgcggccc caggacagaa ggtcaccatc      60

tcctgctctg gaagcagctc cgacatgggg aattatgcgg tatcctggta ccagcagctc     120

ccaggaacag cccccaaact cctcatctat gaaaataata agcgaccctc agggattcct     180

gaccgattct ctggctccaa gtctggcacc tcagccaccc tgggcatcac tggcctctgg     240

cctgaggacg aggccgatta ttactgctta gcatgggata ccagcccgag agcttg         296


<210>  147
<211>  296
<212>  DNA
<213>  homo sapiens;

<400>  147
cagtctgtgc tgactcagcc accctcagcg tctgggaccc ccgggcagag ggtcaccatc      60

tcttgttctg gaagcagctc caacatcgga agtaatactg taaactggta ccagcagctc     120

ccaggaacgg cccccaaact cctcatctat agtaataatc agcggccctc aggggtccct     180

gaccgattct ctggctccaa gtctggcacc tcagcctccc tggccatcag tgggctccag     240

tctgaggatg aggctgatta ttactgtgca gcatgggatg acagcctgaa tggtcc         296


<210>  148
<211>  296
<212>  DNA
<213>  homo sapiens;

<400>  148
cagtctgtgc tgactcagcc accctcagcg tctgggaccc ccgggcagag ggtcaccatc      60

tcttgttctg gaagcagctc caacatcgga agtaattatg tatactggta ccagcagctc     120

ccaggaacgg cccccaaact cctcatctat aggaataatc agcggccctc aggggtccct     180

gaccgattct ctggctccaa gtctggcacc tcagcctccc tggccatcag tgggctccgg     240

tccgaggatg aggctgatta ttactgtgca gcatgggatg acagcctgag tggtcc         296


<210>  149
<211>  299
<212>  DNA
<213>  homo sapiens;

<400>  149
cagtctgtgc tgacgcagcc gccctcagtg tctggggccc cagggcagag ggtcaccatc      60

tcctgcactg ggagcagctc caacattggg gcgggttatg ttgtacattg gtaccagcag     120

cttccaggaa cagcccccaa actcctcatc tatggtaaca gcaatcggcc ctcaggggtc     180

cctgaccaat tctctggctc caagtctggc acctcagcct ccctggccat cactggactc     240

cagtctgagg atgaggctga ttattactgc aaagcatggg ataacagcct gaatgctca      299

<210>  150
<211>  296
<212>  DNA
<213>  homo sapiens;

<400>  150
cagtctgtgt tgacgcagcc gccctcagtg tctgcggccc caggacagaa ggtcaccatc      60

tcctgctctg gaagcagctc caacattggg aataattatg tatcctggta ccagcagctc     120

ccaggaacag cccccaaact cctcatttat gacaataata agcgaccctc agggattcct     180

gaccgattct ctggctccaa gtctggcacg tcagccaccc tgggcatcac cggactccag     240

actggggacg aggccgatta ttactgcgga acatgggata gcagcctgag tgctgg         296


<210>  151
<211>  297
<212>  DNA
<213>  homo sapiens;

<400>  151
cagtctgccc tgactcagcc tccctccgcg tccgggtctc ctggacagtc agtcaccatc      60

tcctgcactg gaaccagcag tgacgttggt ggttataact atgtctcctg gtaccaacag     120

cacccaggca aagcccccaa actcatgatt tatgaggtca gtaagcggcc ctcaggggtc     180

cctgatcgct tctctggctc caagtctggc aacacggcct ccctgaccgt ctctgggctc     240

caggctgagg atgaggctga ttattactgc agctcatatg caggcagcaa caatttc        297


<210>  152
<211>  297
<212>  DNA
<213>  homo sapiens;

<400>  152
cagtctgccc tgactcagcc tcgctcagtg tccgggtctc ctggacagtc agtcaccatc      60

tcctgcactg gaaccagcag tgatgttggt ggttataact atgtctcctg gtaccaacag     120

cacccaggca aagcccccaa actcatgatt tatgatgtca gtaagcggcc ctcaggggtc     180

cctgatcgct tctctggctc caagtctggc aacacggcct ccctgaccat ctctgggctc     240

caggctgagg atgaggctga ttattactgc tgctcatatg caggcagcta cactttc        297


<210>  153
<211>  297
<212>  DNA
<213>  homo sapiens;

<400>  153
cagtctgccc tgactcagcc tgcctccgtg tctgggtctc ctggacagtc gatcaccatc      60

tcctgcactg gaaccagcag tgacgttggt ggttataact atgtctcctg gtaccaacag     120

cacccaggca aagcccccaa actcatgatt tatgaggtca gtaatcggcc ctcaggggtt     180

tctaatcgct tctctggctc caagtctggc aacacggcct ccctgaccat ctctgggctc 240

caggctgagg acgaggctga ttattactgc agctcatata caagcagcag cactctc 297

<210> 154
<211> 297
<212> DNA
<213> homo sapiens;

<400> 154
cagtctgccc tgactcagcc tccctccgtg tccgggtctc ctggacagtc agtcaccatc 60

tcctgcactg gaaccagcag tgacgttggt agttataacc gtgtctcctg gtaccagcag 120

cccccaggca cagcccccaa actcatgatt tatgaggtca gtaatcggcc ctcaggggtc 180

cctgatcgct tctctgggtc caagtctggc aacacggcct ccctgaccat ctctgggctc 240

caggctgagg acgaggctga ttattactgc agcttatata caagcagcag cactttc 297

<210> 155
<211> 298
<212> DNA
<213> homo sapiens;

<400> 155
cagtctgccc tgactcagcc tgcctccgtg tctgggtctc ctggacagtc gatcaccatc 60

tcctgcactg gaaccagcag tgatgttggg agttataacc ttgtctcctg gtaccaacag 120

cacccaggca aagcccccaa actcatgatt tatgagggca gtaagcggcc ctcaggggtt 180

tctaatcgct tctctggctc caagtctggc aacacggcct ccctgacaat ctctgggctc 240

caggctgagg acgaggctga ttattactgc tgctcatatg caggtagtag cactttac 298

<210> 156
<211> 297
<212> DNA
<213> homo sapiens;

<400> 156
caatctgccc tgactcagcc tccttttgtg tccggggctc ctggacagtc ggtcaccatc 60

tcctgcactg gaaccagcag tgacgttggg gattatgatc atgtcttctg gtaccaaaag 120

cgtctcagca ctacctccag actcctgatt tacaatgtca atactcggcc ttcagggatc 180

tctgacctct tctcaggctc caagtctggc aacatggctt ccctgaccat ctctgggctc 240

aagtccgagg ttgaggctaa ttatcactgc agcttatatt caagtagtta cactttc 297

<210> 157
<211> 285
<212> DNA
<213> homo sapiens;

<400> 157
tcctatgagc tgactcagcc accctcagtg tccgtgtccc caggacagac agccagcatc 60

acctgctctg gagataaatt gggggataaa tatgcttgct ggtatcagca gaagccaggc 120

cagtcccctg tgctggtcat ctatcaagat agcaagcggc cctcagggat ccctgagcga 180

ttctctggct ccaactctgg gaacacagcc actctgacca tcagcgggac ccaggctatg 240

gatgaggctg actattactg tcaggcgtgg gacagcagca ctgca 285

<210> 158
<211> 290
<212> DNA
<213> homo sapiens;

<400> 158
tcctatgagc tgacacagcc accctcggtg tcagtgtccc caggacaaac ggccaggatc 60

acctgctctg gagatgcatt gccaaaaaaa tatgcttatt ggtaccagca gaagtcaggc 120

caggcccctg tgctggtcat ctatgaggac agcaaacgac cctccgggat ccctgagaga 180

ttctctggct ccagctcagg gacaatggcc accttgacta tcagtggggc ccaggtggag 240

gatgaagctg actactactg ttactcaaca gacagcagtg gtaatcatag 290

<210> 159
<211> 290
<212> DNA
<213> homo sapiens;

<400> 159
tcctatgagc tgactcagcc acactcagtg tcagtggcca cagcacagat ggccaggatc 60

acctgtgggg gaaacaacat tggaagtaaa gctgtgcact ggtaccagca aaagccaggc 120

caggaccctg tgctggtcat ctatagcgat agcaaccggc cctcagggat ccctgagcga 180

ttctctggct ccaacccagg gaacaccacc accctaacca tcagcaggat cgaggctggg 240

gatgaggctg actattactg tcaggtgtgg gacagtagta gtgatcatcc 290

<210> 160
<211> 290
<212> DNA
<213> homo sapiens;

<400> 160
tcctatgagc tgacacagcc accctcggtg tcagtgtccc taggacagat ggccaggatc 60

acctgctctg gagaagcatt gccaaaaaaa tatgcttatt ggtaccagca gaagccaggc 120

cagttccctg tgctggtgat atataaagac agcgagaggc cctcagggat ccctgagcga 180

ttctctggct ccagctcagg gacaatagtc acattgacca tcagtggagt ccaggcagaa 240

gacgaggctg actattactg tctatcagca gacagcagtg gtacttatcc 290

<210> 161
<211> 290

<212>  DNA
<213>  homo sapiens;

<400>  161
tcttctgagc tgactcagga ccctgctgtg tctgtggcct tgggacagac agtcaggatc      60

acatgccaag gagacagcct cagaagctat tatgcaagct ggtaccagca gaagccagga     120

caggcccctg tacttgtcat ctatggtaaa aacaaccggc cctcagggat cccagaccga     180

ttctctggct ccagctcagg aaacacagct tccttgacca tcactggggc tcaggcggaa     240

gatgaggctg actattactg taactcccgg gacagcagtg gtaaccatct                290


<210>  162
<211>  290
<212>  DNA
<213>  homo sapiens;

<400>  162
tcctatgtgc tgactcagcc accctcagtg tcagtggccc caggaaagac ggccaggatt      60

acctgtgggg gaaacaacat tggaagtaaa agtgtgcact ggtaccagca gaagccaggc     120

caggcccctg tgctggtcat ctattatgat agcgaccggc cctcagggat ccctgagcga     180

ttctctggct ccaactctgg gaacacggcc accctgacca tcagcagggt cgaagccggg     240

gatgaggccg actattactg tcaggtgtgg gacagtagta gtgatcatcc                290


<210>  163
<211>  284
<212>  DNA
<213>  homo sapiens;

<400>  163
tcctatgagc tgacacagct accctcggtg tcagtgtccc caggacagac agccaggatc      60

acctgctctg gagatgtact gggggaaaat tatgctgact ggtaccagca gaagccaggc     120

caggcccctg agttggtgat atacgaagat agtgagcggt accctggaat ccctgaacga     180

ttctctgggt ccacctcagg gaacacgacc accctgacca tcagcagggt cctgaccgaa     240

gacgaggctg actattactg tttgtctggg gatgaggaca atcc                      284


<210>  164
<211>  290
<212>  DNA
<213>  homo sapiens;

<400>  164
tcctatgagc tgatgcagcc accctcggtg tcagtgtccc caggacagac ggccaggatc      60

acctgctctg gagatgcatt gccaaagcaa tatgcttatt ggtaccagca gaagccaggc     120

caggcccctg tgctggtgat atataaagac agtgagaggc cctcagggat ccctgagcga     180

ttctctggct ccagctcagg gacaacagtc acgttgacca tcagtggagt ccaggcagaa     240

gatgaggctg actattactg tcaatcagca gacagcagtg gtacttatcc          290

<210>  165
<211>  284
<212>  DNA
<213>  homo sapiens;

<400>  165
tcctatgagc tgacacagcc atcctcagtg tcagtgtctc cgggacagac agccaggatc     60

acctgctcag gagatgtact ggcaaaaaaa tatgctcggt ggttccagca gaagccaggc    120

caggcccctg tgctggtgat ttataaagac agtgagcggc cctcagggat ccctgagcga    180

ttctccggct ccagctcagg gaccacagtc accttgacca tcagcggggc ccaggttgag    240

gatgaggctg actattactg ttactctgcg gctgacaaca atct                     284

<210>  166
<211>  284
<212>  DNA
<213>  homo sapiens;

<400>  166
tcctctgggc caactcaggt gcctgcagtg tctgtggcct tgggacaaat ggccaggatc     60

acctgccagg gagacagcat ggaaggctct tatgaacact ggtaccagca gaagccaggc    120

caggcccccg tgctggtcat ctatgatagc agtgaccggc cctcaaggat ccctgagcga    180

ttctctggct ccaaatcagg caacacaacc accctgacca tcactggggc ccaggctgag    240

gatgaggctg attattacta tcagttgata gacaaccatg ctac                     284

<210>  167
<211>  314
<212>  DNA
<213>  homo sapiens;

<400>  167
ctgcctgtgc tgactcagcc cccgtctgca tctgccttgc tgggagcctc gatcaagctc     60

acctgcaccc taagcagtga gcacagcacc tacaccatcg aatggtatca acagagacca    120

gggaggtccc cccagtatat aatgaaggtt aagagtgatg gcagccacag caagggggac    180

gggatccccg atcgcttcat gggctccagt tctggggctg accgctacct caccttctcc    240

aacctccagt ctgacgatga ggctgagtat cactgtggag agagccacac gattgatggc    300

caagtcggtt gagc                                                      314

<210>  168
<211>  297
<212>  DNA
<213>  homo sapiens;

<400>  168
cagcctgtgc tgactcaatc atcctctgcc tctgcttccc tgggatcctc ggtcaagctc     60

acctgcactc tgagcagtgg gcacagtagc tacatcatcg catggcatca gcagcagcca 120

gggaaggccc ctcggtactt gatgaagctt gaaggtagtg gaagctacaa caaggggagc 180

ggagttcctg atcgcttctc aggctccagc tctggggctg accgctacct caccatctcc 240

aacctccagt tagaggatga ggctgattat tactgtgaga cctgggacag taacact 297

<210> 169
<211> 299
<212> DNA
<213> homo sapiens;

<400> 169
cagcttgtgc tgactcaatc gccctctgcc tctgcctccc tgggagcctc ggtcaagctc 60

acctgcactc tgagcagtgg gcacagcagc tacgccatcg catggcatca gcagcagcca 120

gagaagggcc ctcggtactt gatgaagctt aacagtgatg gcagccacag caagggggac 180

gggatccctg atcgcttctc aggctccagc tctggggctg agcgctacct caccatctcc 240

agcctccagt ctgaggatga ggctgactat tactgtcaga cctggggcac tggcattca 299

<210> 170
<211> 312
<212> DNA
<213> homo sapiens;

<400> 170
cagcctgtgc tgactcagcc accttcctcc tccgcatctc ctggagaatc cgccagactc 60

acctgcacct tgcccagtga catcaatgtt ggtagctaca acatatactg gtaccagcag 120

aagccaggga gccctcccag gtatctcctg tactactact cagactcaga taagggccag 180

ggctctggag tccccagccg cttctctgga tccaaagatg cttcagccaa tacagggatt 240

ttactcatct ccgggctcca gtctgaggat gaggctgact attactgtat gatttggcca 300

agcaatgctt ct 312

<210> 171
<211> 312
<212> DNA
<213> homo sapiens;

<400> 171
caggctgtgc tgactcagcc ggcttccctc tctgcatctc ctggagcatc agccagtctc 60

acctgcacct tgcgcagtgg catcaatgtt ggtacctaca ggatatactg gtaccagcag 120

aagccaggga gtcctcccca gtatctcctg aggtacaaat cagactcaga taagcagcag 180

ggctctggag tccccagccg cttctctgga tccaaagatg cttcggccaa tgcagggatt 240

ttactcatct ctgggctcca gtctgaggat gaggctgact attactgtat gatttggcac 300

agcagcgctt ct 312

<210>  172
<211>  312
<212>  DNA
<213>  homo sapiens;

<400>  172
cagcctgtgc tgactcagcc aacttccctc tcagcatctc ctggagcatc agccagactc      60

acctgcacct tgcgcagtgg catcaatctt ggtagctaca ggatattctg gtaccagcag     120

aagccagaga gccctccccg gtatctcctg agctactact cagactcaag taagcatcag     180

ggctctggag tccccagccg cttctctgga tccaaagatg cttcgagcaa tgcagggatt     240

ttagtcatct ctgggctcca gtctgaggat gaggctgact attactgtat gatttggcac     300

agcagtgctt ct                                                         312


<210>  173
<211>  317
<212>  DNA
<213>  homo sapiens;

<400>  173
cagcctgtgc tgactcagcc atcttcccat tctgcatctt ctggagcatc agtcagactc      60

acctgcatgc tgagcagtgg cttcagtgtt ggggacttct ggataaggtg gtaccaacaa     120

aagccaggga accctccccg gtatctcctg tactaccact cagactccaa taagggccaa     180

ggctctggag ttcccagccg cttctctgga tccaacgatg catcagccaa tgcagggatt     240

ctgcgtatct ctgggctcca gcctgaggat gaggctgact attactgtgg tacatggcac     300

agcaactcta agactca                                                    317


<210>  174
<211>  296
<212>  DNA
<213>  homo sapiens;

<400>  174
aattttatgc tgactcagcc ccactctgtg tcggagtctc cggggaagac ggtaaccatc      60

tcctgcaccc gcagcagtgg cagcattgcc agcaactatg tgcagtggta ccagcagcgc     120

ccgggcagtt cccccaccac tgtgatctat gaggataacc aaagaccctc tggggtccct     180

gatcggttct ctggctccat cgacagctcc tccaactctg cctccctcac catctctgga     240

ctgaagactg aggacgaggc tgactactac tgtcagtctt atgatagcag caatca         296


<210>  175
<211>  294
<212>  DNA
<213>  homo sapiens;

<400>  175

cagactgtgg tgactcagga gccctcactg actgtgtccc caggagggac agtcactctc 60

acctgtgctt ccagcactgg agcagtcacc agtggttact atccaaactg gttccagcag 120

aaacctggac aagcacccag ggcactgatt tatagtacaa gcaacaaaca ctcctggacc 180

cctgcccggt tctcaggctc cctccttggg ggcaaagctg ccctgacact gtcaggtgtg 240

cagcctgagg acgaggctga gtattactgc ctgctctact atggtggtgc tcag 294

<210> 176
<211> 294
<212> DNA
<213> homo sapiens;

<400> 176
caggctgtgg tgactcagga gccctcactg actgtgtccc caggagggac agtcactctc 60

acctgtggct ccagcactgg agctgtcacc agtggtcatt atccctactg gttccagcag 120

aagcctggcc aagcccccag gacactgatt tatgatacaa gcaacaaaca ctcctggaca 180

cctgcccggt tctcaggctc cctccttggg ggcaaagctg ccctgaccct ttcgggtgcg 240

cagcctgagg atgaggctga gtattactgc ttgctctcct atagtggtgc tcgg 294

<210> 177
<211> 296
<212> DNA
<213> homo sapiens;

<400> 177
cagactgtgg tgacccagga gccatcgttc tcagtgtccc ctggagggac agtcacactc 60

acttgtggct tgagctctgg ctcagtctct actagttact accccagctg gtaccagcag 120

accccaggcc aggctccacg cacgctcatc tacagcacaa acactcgctc ttctggggtc 180

cctgatcgct tctctggctc catccttggg aacaaagctg ccctcaccat cacgggggcc 240

caggcagatg atgaatctga ttattactgt gtgctgtata tgggtagtgg catttc 296

<210> 178
<211> 317
<212> DNA
<213> homo sapiens;

<400> 178
cagcctgtgc tgactcagcc accttctgca tcagcctccc tgggagcctc ggtcacactc 60

acctgcaccc tgagcagcgg ctacagtaat tataaagtgg actggtacca gcagagacca 120

gggaagggcc cccggtttgt gatgcgagtg ggcactggtg ggattgtggg atccaagggg 180

gatggcatcc ctgatcgctt ctcagtcttg ggctcaggcc tgaatcggta cctgaccatc 240

aagaacatcc aggaagagga tgagagtgac taccactgtg gggcagacca tggcagtggg 300

agcaacttcg tgtaacc 317

<210>  179
<211>  296
<212>  DNA
<213>  homo sapiens;

<400>  179
caggcagggc tgactcagcc accctcggtg tccaagggct tgagacagac cgccacactc      60

acctgcactg ggaacagcaa caatgttggc aaccaaggag cagcttggct gcagcagcac     120

cagggccacc ctcccaaact cctatcctac aggaataaca accggccctc agggatctca     180

gagagattat ctgcatccag gtcaggaaac acagcctccc tgaccattac tggactccag     240

cctgaggacg aggctgacta ttactgctca gcatgggaca gcagcctcag tgctca         296


<210>  180
<211>  312
<212>  DNA
<213>  homo sapiens;

<400>  180
cggcccgtgc tgactcagcc gccctctctg tctgcatccc cgggagcaac agccagactc      60

ccctgcaccc tgagcagtga cctcagtgtt ggtggtaaaa acatgttctg gtaccagcag     120

aagccaggga gctctcccag gttattcctg tatcactact cagactcaga caagcagctg     180

ggacctgggg tccccagtcg agtctctggc tccaaggaga cctcaagtaa cacagcgttt     240

ttgctcatct ctgggctcca gcctgaggac gaggccgatt attactgcca ggtgtacgaa     300

agtagtgcta at                                                         312


<210>  181
<211>  38
<212>  DNA
<213>  homo sapiens;

<400>  181
gtggacgttc ggccaaggga ccaaggtgga aatcaaac                              38


<210>  182
<211>  39
<212>  DNA
<213>  homo sapiens;

<400>  182
tgtacacttt tggccagggg accaagctgg agatcaaac                             39


<210>  183
<211>  38
<212>  DNA
<213>  homo sapiens;

<400>  183
attcactttc ggccctggga ccaaagtgga tatcaaac                              38

<210>  184
<211>  38
<212>  DNA
<213>  homo sapiens;

<400>  184
gctcactttc ggcggaggga ccaaggtgga gatcaaac                              38


<210>  185
<211>  38
<212>  DNA
<213>  homo sapiens;

<400>  185
gatcaccttc ggccaaggga cacgactgga gattaaac                              38


<210>  186
<211>  38
<212>  DNA
<213>  homo sapiens;

<400>  186
ttatgtcttc ggaactggga ccaaggtcac cgtcctag                              38


<210>  187
<211>  38
<212>  DNA
<213>  homo sapiens;

<400>  187
tgtggtattc ggcggaggga ccaagctgac cgtcctag                              38


<210>  188
<211>  38
<212>  DNA
<213>  homo sapiens;

<400>  188
tgtggtattc ggcggaggga ccaagctgac cgtcctag                              38


<210>  189
<211>  38
<212>  DNA
<213>  homo sapiens;

<400>  189
ttttgtattt ggtggaggaa cccagctgat cattttag                              38


<210>  190
<211>  38
<212>  DNA
<213>  homo sapiens;

<400>  190
ctgggtgttt ggtgagggga ccgagctgac cgtcctag                              38

<210>  191
<211>  38
<212>  DNA
<213>  homo sapiens;

<400>  191
taatgtgttc ggcagtggca ccaaggtgac cgtcctcg                              38


<210>  192
<211>  38
<212>  DNA
<213>  homo sapiens;

<400>  192
tgctgtgttc ggaggaggca cccagctgac cgtcctcg                              38


<210>  193
<211>  302
<212>  DNA
<213>  homo sapiens;

<400>  193
gagattgtga tgacccagac tccactctcc ttgtctatca cccctggaga gcaggcctcc      60

atctcctgca ggtctagtca gagcctcctg catagtgatg gatacaccta tttgtattgg     120

tttctgcaga aagccaggcc agtctccaca ctcctgatct atgaagtttc caaccggttc     180

tctggagtgc cagataggtt cagtggcagc gggtcaggga cagatttcac actgaaaatc     240

agccgggtgg aggctgagga ttttggagtt tattactgca tgcaagatgc acaagatcct     300

cc                                                                    302


<210>  194
<211>  296
<212>  DNA
<213>  homo sapiens;

<400>  194
cagtctgtgc tgactcagcc accctcggtg tctgaagccc ccaggcagag ggtcaccatc      60

tcctgttctg gaagcagctc caacatcgga aataatgctg taaactggta ccagcagctc     120

ccaggaaagg ctcccaaact cctcatctat tatgatgatc tgctgccctc aggggtctct     180

gaccgattct ctggctccaa gtctggcacc tcagcctccc tggccatcag tgggctccag     240

tctgaggatg aggctgatta ttactgtgca gcatgggatg acagcctgaa tggtcc         296


<210>  195
<211>  296
<212>  DNA
<213>  homo sapiens;

<400>  195
cagtctgtgc tgactcagcc accctcagcg tctgggaccc ccgggcagag ggtcaccatc      60

tcttgttctg gaagcagctc caacatcgga agtaattatg tatactggta ccagcagctc 120

ccaggaacgg cccccaaact cctcatctat aggaataatc agcggccctc aggggtccct 180

gaccgattct ctggctccaa gtctggcacc tcagcctccc tggccatcag tgggctccgg 240

tccgaggatg aggctgatta ttactgtgca gcatgggatg acagcctgag tggtcc 296

<210> 196
<211> 299
<212> DNA
<213> homo sapiens;

<400> 196
cagtctgtgc tgacgcagcc gccctcagtg tctggggccc cagggcagag ggtcaccatc 60

tcctgcactg ggagcagctc caacattggg gcgggttatg ttgtacattg gtaccagcag 120

cttccaggaa cagcccccaa actcctcatc tatggtaaca gcaatcggcc ctcaggggtc 180

cctgaccaat tctctggctc caagtctggc acctcagcct ccctggccat cactggactc 240

cagtctgagg atgaggctga ttattactgc aaagcatggg ataacagcct gaatgctca 299

<210> 197
<211> 296
<212> DNA
<213> homo sapiens;

<400> 197
cagtctgtgt tgacgcagcc gccctcagtg tctgcggccc caggacagaa ggtcaccatc 60

tcctgctctg gaagcagctc caacattggg aataattatg tatcctggta ccagcagctc 120

ccaggaacag cccccaaact cctcatttat gacaataata agcgaccctc agggattcct 180

gaccgattct ctggctccaa gtctggcacg tcagccaccc tgggcatcac cggactccag 240

actggggacg aggccgatta ttactgcgga acatgggata gcagcctgag tgctgg 296

<210> 198
<211> 296
<212> DNA
<213> homo sapiens;

<400> 198
caggcagggc tgactcagcc accctcggtg tccaagggct tgagacagac cgccacactc 60

acctgcactg ggaacagcaa caatgttggc aaccaaggag cagcttggct gcagcagcac 120

cagggccacc ctcccaaact cctatcctac aggaataaca accggccctc agggatctca 180

gagagattat ctgcatccag gtcaggaaac acagcctccc tgaccattac tggactccag 240

cctgaggacg aggctgacta ttactgctca gcatgggaca gcagcctcag tgctca 296

<210> 199
<211> 312
<212> DNA

<213>  homo sapiens;

<400>  199
cggcccgtgc tgactcagcc gccctctctg tctgcatccc cgggagcaac agccagactc      60

ccctgcaccc tgagcagtga cctcagtgtt ggtggtaaaa acatgttctg gtaccagcag     120

aagccaggga gctctcccag gttattcctg tatcactact cagactcaga caagcagctg     180

ggacctgggg tccccagtcg agtctctggc tccaaggaga cctcaagtaa cacagcgttt     240

ttgctcatct ctgggctcca gcctgaggac gaggccgatt attactgcca ggtgtacgaa     300

agtagtgcta at                                                         312


<210>  200
<211>  297
<212>  DNA
<213>  homo sapiens;

<400>  200
cagtctgccc tgactcagcc tgcctccgtg tctgggtctc ctggacagtc gatcaccatc      60

tcctgcactg gaaccagcag tgacgttggt ggttataact atgtctcctg gtaccaacag     120

cacccaggca aagcccccaa actcatgatt tatgaggtca gtaatcggcc ctcaggggtt     180

tctaatcgct tctctggctc caagtctggc aacacggcct ccctgaccat ctctgggctc     240

caggctgagg acgaggctga ttattactgc agctcatata caagcagcag cactctc        297


<210>  201
<211>  297
<212>  DNA
<213>  homo sapiens;

<400>  201
cagtctgccc tgactcagcc tccctccgtg tccgggtctc ctggacagtc agtcaccatc      60

tcctgcactg gaaccagcag tgacgttggt agttataacc gtgtctcctg gtaccagcag     120

cccccaggca cagcccccaa actcatgatt tatgaggtca gtaatcggcc ctcaggggtc     180

cctgatcgct tctctgggtc caagtctggc aacacggcct ccctgaccat ctctgggctc     240

caggctgagg acgaggctga ttattactgc agcttatata caagcagcag cactttc        297


<210>  202
<211>  298
<212>  DNA
<213>  homo sapiens;

<400>  202
cagtctgccc tgactcagcc tgcctccgtg tctgggtctc ctggacagtc gatcaccatc      60

tcctgcactg gaaccagcag tgatgttggg agttataacc ttgtctcctg gtaccaacag     120

cacccaggca aagcccccaa actcatgatt tatgagggca gtaagcggcc ctcaggggtt     180

tctaatcgct tctctggctc caagtctggc aacacggcct ccctgacaat ctctgggctc     240

caggctgagg acgaggctga ttattactgc tgctcatatg caggtagtag cactttac 298

<210> 203
<211> 297
<212> DNA
<213> homo sapiens;

<400> 203
cagtctgccc tgactcagcc tccctccgcg tccgggtctc ctggacagtc agtcaccatc 60

tcctgcactg gaaccagcag tgacgttggt ggttataact atgtctcctg gtaccaacag 120

cacccaggca aagcccccaa actcatgatt tatgaggtca gtaagcggcc ctcaggggtc 180

cctgatcgct tctctggctc caagtctggc aacacggcct ccctgaccgt ctctgggctc 240

caggctgagg atgaggctga ttattactgc agctcatatg caggcagcaa caatttc 297

<210> 204
<211> 285
<212> DNA
<213> homo sapiens;

<400> 204
tcctatgagc tgactcagcc accctcagtg tccgtgtccc caggacagac agccagcatc 60

acctgctctg gagataaatt gggggataaa tatgcttgct ggtatcagca gaagccaggc 120

cagtcccctg tgctggtcat ctatcaagat agcaagcggc cctcagggat ccctgagcga 180

ttctctggct ccaactctgg gaacacagcc actctgacca tcagcgggac ccaggctatg 240

gatgaggctg actattactg tcaggcgtgg gacagcagca ctgca 285

<210> 205
<211> 290
<212> DNA
<213> homo sapiens;

<400> 205
tcctatgagc tgactcagcc acactcagtg tcagtggcca cagcacagat ggccaggatc 60

acctgtgggg gaaacaacat tggaagtaaa gctgtgcact ggtaccagca aaagccaggc 120

caggaccctg tgctggtcat ctatagcgat agcaaccggc cctcagggat ccctgagcga 180

ttctctggct ccaacccagg gaacaccacc accctaacca tcagcaggat cgaggctggg 240

gatgaggctg actattactg tcaggtgtgg gacagtagta gtgatcatcc 290

<210> 206
<211> 290
<212> DNA
<213> homo sapiens;

<400> 206
tcttctgagc tgactcagga ccctgctgtg tctgtggcct tgggacagac agtcaggatc 60

acatgccaag gagacagcct cagaagctat tatgcaagct ggtaccagca gaagccagga 120

caggcccctg tacttgtcat ctatggtaaa aacaaccggc cctcagggat cccagaccga 180

ttctctggct ccagctcagg aaacacagct tccttgacca tcactggggc tcaggcggaa 240

gatgaggctg actattactg taactcccgg gacagcagtg gtaaccatct 290

<210> 207
<211> 290
<212> DNA
<213> homo sapiens;

<400> 207
tcctatgtgc tgactcagcc accctcagtg tcagtggccc caggaaagac ggccaggatt 60

acctgtgggg gaaacaacat tggaagtaaa agtgtgcact ggtaccagca gaagccaggc 120

caggcccctg tgctggtcat ctattatgat agcgaccggc cctcagggat ccctgagcga 180

ttctctggct ccaactctgg gaacacggcc accctgacca tcagcagggt cgaagccggg 240

gatgaggccg actattactg tcaggtgtgg gacagtagta gtgatcatcc 290

<210> 208
<211> 284
<212> DNA
<213> homo sapiens;

<400> 208
tcctatgagc tgacacagct accctcggtg tcagtgtccc caggacagac agccaggatc 60

acctgctctg gagatgtact gggggaaaat tatgctgact ggtaccagca gaagccaggc 120

caggcccctg agttggtgat atacgaagat agtgagcggt accctggaat ccctgaacga 180

ttctctgggt ccacctcagg gaacacgacc accctgacca tcagcagggt cctgaccgaa 240

gacgaggctg actattactg tttgtctggg gatgaggaca atcc 284

<210> 209
<211> 290
<212> DNA
<213> homo sapiens;

<400> 209
tcctatgagc tgatgcagcc accctcggtg tcagtgtccc caggacagac ggccaggatc 60

acctgctctg gagatgcatt gccaaagcaa tatgcttatt ggtaccagca gaagccaggc 120

caggcccctg tgctggtgat atataaagac agtgagaggc cctcagggat ccctgagcga 180

ttctctggct ccagctcagg gacaacagtc acgttgacca tcagtggagt ccaggcagaa 240

gatgaggctg actattactg tcaatcagca gacagcagtg gtacttatcc 290

<210> 210
<211> 297
<212> DNA

<213>  homo sapiens;

<400>  210
cagcctgtgc tgactcaatc atcctctgcc tctgcttccc tgggatcctc ggtcaagctc      60

acctgcactc tgagcagtgg gcacagtagc tacatcatcg catggcatca gcagcagcca     120

gggaaggccc ctcggtactt gatgaagctt gaaggtagtg gaagctacaa caaggggagc     180

ggagttcctg atcgcttctc aggctccagc tctggggctg accgctacct caccatctcc     240

aacctccagt tagaggatga ggctgattat tactgtgaga cctgggacag taacact        297


<210>  211
<211>  312
<212>  DNA
<213>  homo sapiens;

<400>  211
cagcctgtgc tgactcagcc accttcctcc tccgcatctc ctggagaatc cgccagactc      60

acctgcacct tgcccagtga catcaatgtt ggtagctaca acatatactg gtaccagcag     120

aagccaggga gccctcccag gtatctcctg tactactact cagactcaga taagggccag     180

ggctctggag tccccagccg cttctctgga tccaaagatg cttcagccaa tacagggatt     240

ttactcatct ccgggctcca gtctgaggat gaggctgact attactgtat gatttggcca     300

agcaatgctt ct                                                         312


<210>  212
<211>  312
<212>  DNA
<213>  homo sapiens;

<400>  212
caggctgtgc tgactcagcc ggcttccctc tctgcatctc ctggagcatc agccagtctc      60

acctgcacct tgcgcagtgg catcaatgtt ggtacctaca ggatatactg gtaccagcag     120

aagccaggga gtcctcccca gtatctcctg aggtacaaat cagactcaga taagcagcag     180

ggctctggag tccccagccg cttctctgga tccaaagatg cttcggccaa tgcagggatt     240

ttactcatct ctgggctcca gtctgaggat gaggctgact attactgtat gatttggcac     300

agcagcgctt ct                                                         312


<210>  213
<211>  312
<212>  DNA
<213>  homo sapiens;

<400>  213
cagcctgtgc tgactcagcc aacttccctc tcagcatctc ctggagcatc agccagactc      60

acctgcacct tgcgcagtgg catcaatctt ggtagctaca ggatattctg gtaccagcag     120

aagccagaga gccctccccg gtatctcctg agctactact cagactcaag taagcatcag     180

ggctctggag tccccagccg cttctctgga tccaaagatg cttcgagcaa tgcagggatt 240

ttagtcatct ctgggctcca gtctgaggat gaggctgact attactgtat gatttggcac 300

agcagtgctt ct 312

<210> 214
<211> 296
<212> DNA
<213> homo sapiens;

<400> 214
aattttatgc tgactcagcc ccactctgtg tcggagtctc cggggaagac ggtaaccatc 60

tcctgcaccc gcagcagtgg cagcattgcc agcaactatg tgcagtggta ccagcagcgc 120

ccgggcagtt cccccaccac tgtgatctat gaggataacc aaagaccctc tggggtccct 180

gatcggttct ctggctccat cgacagctcc tccaactctg cctccctcac catctctgga 240

ctgaagactg aggacgaggc tgactactac tgtcagtctt atgatagcag caatca 296

<210> 215
<211> 294
<212> DNA
<213> homo sapiens;

<400> 215
caggctgtgg tgactcagga gccctcactg actgtgtccc caggagggac agtcactctc 60

acctgtggct ccagcactgg agctgtcacc agtggtcatt atccctactg gttccagcag 120

aagcctggcc aagcccccag gacactgatt tatgatacaa gcaacaaaca ctcctggaca 180

cctgcccggt tctcaggctc cctccttggg ggcaaagctg ccctgaccct ttcgggtgcg 240

cagcctgagg atgaggctga gtattactgc ttgctctcct atagtggtgc tcgg 294

<210> 216
<211> 296
<212> DNA
<213> homo sapiens;

<400> 216
cagactgtgg tgacccagga gccatcgttc tcagtgtccc ctggagggac agtcacactc 60

acttgtggct tgagctctgg ctcagtctct actagttact accccagctg gtaccagcag 120

accccaggcc aggctccacg cacgctcatc tacagcacaa acactcgctc ttctggggtc 180

cctgatcgct tctctggctc catccttggg aacaaagctg ccctcaccat cacgggggcc 240

caggcagatg atgaatctga ttattactgt gtgctgtata tgggtagtgg catttc 296

<210> 217
<211> 1134
<212> DNA
<213> homo sapiens;

<220>
<221>  misc_feature
<222>  (1)..(1)
<223>  n is a, c, g, or t

<400>  217
ncttccacca agggcccatc ggtcttcccc ctggcgccct gctccaggag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacacctgca acgtgaatca caagcccagc aacaccaagg tggacaagag agttgagctc     300

aaaaccccac ttggtgacac aactcacaca tgcccacggt gcccagagcc caaatcttgt     360

gacacacctc ccccgtgccc acggtgccca gagcccaaat cttgtgacac acctccccca     420

tgcccacggt gcccagagcc caaatcttgt gacacacctc ccccgtgccc aaggtgccca     480

gcacctgaac tcctgggagg accgtcagtc ttcctcttcc ccccaaaacc caaggatacc     540

cttatgattt cccggacccc tgaggtcacg tgcgtggtgg tggacgtgag ccacgaagac     600

cccgaggtcc agttcaagtg gtacgtggac ggcgtggagg tgcataatgc caagacaaag     660

ccgcgggagg agcagtacaa cagcacgttc cgtgtggtca gcgtcctcac cgtcctgcac     720

caggactggc tgaacggcaa ggagtacaag tgcaaggtct ccaacaaagc cctcccagcc     780

cccatcgaga aaaccatctc caaaaccaaa ggacagcccc gagaaccaca ggtgtacacc     840

ctgcccccat cccgggagga gatgaccaag aaccaggtca gcctgacctg cctggtcaaa     900

ggcttctacc ccagcgacat cgccgtggag tgggagagca gcgggcagcc ggagaacaac     960

tacaacacca cgcctcccat gctggactcc gacggctcct tcttcctcta cagcaagctc    1020

accgtggaca agagcaggtg gcagcagggg aacatcttct catgctccgt gatgcatgag    1080

gctctgcaca accgcttcac gcagaagagc ctctccctgt ctccgggtaa atga          1134


<210>  218
<211>  1023
<212>  DNA
<213>  homo sapiens;

<400>  218
   gcatccccga ccagccccaa ggtcttcccg ctgagcctcg acagcacccc ccaagatggg      60
   aacgtggtcg tcgcatgcct ggtccagggc ttcttccccc aggagccact cagtgtgacc     120
   tggagcgaaa gcggacagaa cgtgaccgcc agaaacttcc cacctagcca ggatgcctcc     180
   ggggacctgt acaccacgag cagccagctg accctgccgg ccacacagtg cccagacggc     240
   aagtccgtga catgccacgt gaagcactac acgaattcca gccaggatgt gactgtgccc     300
   tgccgagttc ccccacctcc cccatgctgc cacccccgac tgtcgctgca ccgaccggcc 360
   ctcgaggacc tgctcttagg ttcagaagcg aacctcacgt gcacactgac cggcctgaga 420
   gatgcctctg gtgccacctt cacctggacg ccctcaagtg ggaagagcgc tgttcaagga 480
   ccacctgagc gtgacctctg tggctgctac agcgtgtcca gtgtcctgcc tggctgtgcc 540
   cagccatgga accatgggga gaccttcacc tgcactgctg cccaccccga gttgaagacc 600
   ccactaaccg ccaacatcac aaaatccgga aacacattcc ggcccgaggt ccacctgctg 660
   ccgccgccgt cggaggagct ggccctgaac gagctggtga cgctgacgtg cctggcacgt 720
   ggcttcagcc ccaaggatgt gctggttcgc tggctgcagg ggtcacagga gctgccccgc 780
   gagaagtacc tgacttgggc atcccggcag gagcccagcc agggcaccac cacctacgct 840
   gtaaccagca tactgcgcgt ggcagctgag gactggaaga agggggagac cttctcctgc 900
   atggtgggcc acgaggccct gccgctggcc ttcacacaga agaccatcga ccgcatggcg 960
   ggtaaaccca cccacatcaa tgtgtctgtt gtcatggcgg aggcggatgg cacctgctac 1020
   tga 1023

<210> 219
<211> 981
<212> DNA
<213> homo sapiens;

<400> 219
gcctccacca agggcccatc ggtcttcccc ctggcgccct gctccaggag cacctccgag 60

agcacagcgg ccctgggctg cctggtcaag gactacttcc ccgaaccggt gacggtgtcg 120

tggaactcag gcgctctgac cagcggcgtg cacaccttcc cggctgtcct acagtcctca 180

ggactctact ccctcagcag cgtggtgacc gtgccctcca gcaacttcgg cacccagacc 240

tacacctgca acgtagatca caagcccagc aacaccaagg tggacaagac agttgagcgc 300

aaatgttgtg tcgagtgccc accgtgccca gcaccacctg tggcaggacc gtcagtcttc 360

ctcttccccc caaaacccaa ggacaccctc atgatctccc ggacccctga ggtcacgtgc 420

gtggtggtgg acgtgagcca cgaagacccc gaggtccagt tcaactggta cgtggacggc 480

gtggaggtgc ataatgccaa gacaaagcca cgggaggagc agttcaacag cacgttccgt 540

gtggtcagcg tcctcaccgt cgtgcaccag gactggctga acggcaagga gtacaagtgc 600

aaggtctcca acaaaggcct cccagccccc atcgagaaaa ccatctccaa aaccaaaggg 660

cagccccgag aaccacaggt gtacaccctg cccccatccc gggaggagat gaccaagaac 720

caggtcagcc tgacctgcct ggtcaaaggc ttctacccca gcgacatctc cgtggagtgg 780

gagagcaatg ggcagccgga gaacaactac aagaccacac ctcccatgct ggactccgac 840

ggctccttct tcctctacag caagctcacc gtggacaaga gcaggtggca gcaggggaac 900

gtcttctcat gctccgtgat gcatgaggct ctgcacaacc actacacaca gaagagcctc 960

tccctgtctc cgggtaaatg a 981

<210> 220
<211> 1287
<212> DNA
<213> homo sapiens;

<400> 220
gcctccacac agagcccatc cgtcttcccc ttgacccgct gctgcaaaaa cattccctcc 60

aatgccacct ccgtgactct gggctgcctg gccacgggct acttcccgga gccggtgatg 120

gtgacctggg acacaggctc cctcaacggg acaactatga ccttaccagc caccaccctc 180

acgctctctg gtcactatgc caccatcagc ttgctgaccg tctcgggtgc gtgggccaag 240

cagatgttca cctgccgtgt ggcacacact ccatcgtcca cagactgggt cgacaacaaa 300

accttcagcg tctgctccag ggacttcacc ccgcccaccg tgaagatctt acagtcgtcc 360

tgcgacggcg gcgggcactt ccccccgacc atccagctcc tgtgcctcgt ctctgggtac 420

accccaggga ctatcaacat cacctggctg gaggacgggc aggtcatgga cgtggacttg 480

tccaccgcct ctaccacgca ggagggtgag ctggcctcca cacaaagcga gctcaccctc 540

agccagaagc actggctgtc agaccgcacc tacacctgcc aggtcaccta tcaaggtcac 600

acctttgagg acagcaccaa gaagtgtgca gattccaacc cgagaggggt gagcgcctac 660

ctaagccggc ccagcccgtt cgacctgttc atccgcaagt cgcccacgat cacctgtctg 720

gtggtggacc tggcacccag caaggggacc gtgaacctga cctggtcccg ggccagtggg 780

aagcctgtga accactccac cagaaaggag gagaagcagc gcaatggcac gttaaccgtc 840

acgtccaccc tgccggtggg cacccgagac tggatcgagg gggagaccta ccagtgcagg 900

gtgacccacc cccacctgcc cagggccctc atgcggtcca cgaccaagac cagcggcccg 960

cgtgctgccc cggaagtcta tgcgtttgcg acgccggagt ggccggggag ccgggacaag 1020

cgcaccctcg cctgcctgat ccagaacttc atgcctgagg acatctcggt gcagtggctg 1080

cacaacgagg tgcagctccc ggacgcccgg cacagcacga cgcagccccg caagaccaag 1140

ggctccggct tcttcgtctt cagccgcctg gaggtgacca gggccgaatg ggagcagaaa 1200

gatgagttca tctgccgtgc agtccatgag gcagcaagcc cctcacagac cgtccagcga 1260

gcggtgtctg taaatcccgg taaatga 1287

<210> 221
<211> 30
<212> DNA
<213> homo sapiens;

<400> 221
tccggcttct tcgtcttcag ccgcctggag 30

<210>  222
<211>  1062
<212>  DNA
<213>  homo sapiens;

<400>  222
gcatccccga ccagccccaa ggtcttcccg ctgagcctct gcagcaccca gccagatggg      60

aacgtggtca tcgcctgcct ggtccagggc ttcttccccc aggagccact cagtgtgacc     120

tggagcgaaa gcggacaggg cgtgaccgcc agaaacttcc cacccagcca ggatgcctcc     180

ggggacctgt acaccacgag cagccagctg accctgccgg ccacacagtg cctagccggc     240

aagtccgtga catgccacgt gaagcactac acgaatccca gccaggatgt gactgtgccc     300

tgcccagttc cctcaactcc acctacccca tctccctcaa ctccacctac cccatctccc     360

tcatgctgcc acccccgact gtcactgcac cgaccggccc tcgaggacct gctcttaggt     420

tcagaagcga acctcacgtg cacactgacc ggcctgagag atgcctcagg tgtcaccttc     480

acctggacgc cctcaagtgg gaagagcgct gttcaaggac cacctgagcg tgacctctgt     540

ggctgctaca gcgtgtccag tgtcctgccg ggctgtgccg agccatggaa ccatgggaag     600

accttcactt gcactgctgc ctaccccgag tccaagaccc cgctaaccgc caccctctca     660

aaatccggaa acacattccg gcccgaggtc cacctgctgc cgccgccgtc ggaggagctg     720

gccctgaacg agctggtgac gctgacgtgc ctggcacgcg gcttcagccc caaggatgtg     780

ctggttcgct ggctgcaggg gtcacaggag ctgccccgcg agaagtacct gacttgggca     840

tcccggcagg agcccagcca gggcaccacc accttcgctg tgaccagcat actgcgcgtg     900

gcagccgagg actggaagaa gggggacacc ttctcctgca tggtgggcca cgaggccctg     960

ccgctggcct tcacacagaa gaccatcgac cgcttggcgg gtaaacccac ccatgtcaat    1020

gtgtctgttg tcatggcgga ggtggacggc acctgctact ga                       1062


<210>  223
<211>  888
<212>  DNA
<213>  homo sapiens;

<400>  223
gcctccacca agggcccatc ggtcttcccc ctggcaccct cctccaagag cacctctggg      60

ggcacagcag ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggagta caagtgcaag gtctccaaca aagccctccc agcccccatc 540

gagaaaacca tctccaaagc caaagggcag ccccgagaac cacaggtgta caccctgccc 600

ccatcccggg atgagctgac caagaaccag gtcagcctga cctgcctggt caaaggcttc 660

tatcccagcg acatcgccgt ggagtgggag agcaatgggc agccggagaa caactacaag 720

accacgcctc ccgtgctgga ctccgacggc tccttcttcc tctacagcaa gctcaccgtg 780

gacaagagca ggtggcagca ggggaacgtc ttctcatgct ccgtgatgca tgaggctctg 840

cacaaccact acacacagaa gagcctctcc ctgtctccgg gtaaatga 888

<210> 224
<211> 993
<212> DNA
<213> homo sapiens;

<400> 224
gcctccacca agggcccatc ggtcttcccc ctggcaccct cctccaagag cacctctggg 60

ggcacagcag ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacaca 960

cagaagagcc tctccctgtc tccgggtaaa tga 993

<210> 225
<211> 1362
<212> DNA

<213>  homo sapiens;

<400>  225
gggagtgcat ccgccccaac ccttttcccc ctcgtctcct gtgagaattc cccgtcggat      60

acgagcagcg tggccgttgg ctgcctcgca caggacttcc ttcccgactc catcactttc     120

tcctggaaat acaagaacaa ctctgacatc agcagcaccc ggggcttccc atcagtcctg     180

agagggggca agtacgcagc cacctcacag gtgctgctgc cttccaagga cgtcatgcag     240

ggcacagacg aacacgtggt gtgcaaagtc cagcacccca acggcaacaa agaaaagaac     300

gtgcctcttc cagtgattgc cgagctgcct cccaaagtga gcgtcttcgt cccaccccgc     360

gacggcttct tcggcaaccc ccgcaagtcc aagctcatct gccaggccac gggtttcagt     420

ccccggcaga ttcaggtgtc ctggctgcgc gaggggaagc aggtggggtc tggcgtcacc     480

acggaccagg tgcaggctga ggccaaagag tctgggccca cgacctacaa ggtgaccagc     540

acactgacca tcaaagagag cgactggctc agccagagca tgttcacctg ccgcgtggat     600

cacaggggcc tgaccttcca gcagaatgcg tcctccatgt gtggccccga tcaagacaca     660

gccatccggg tcttcgccat ccccccatcc tttgccagca tcttcctcac caagtccacc     720

aagttgacct gcctggtcac agacctgacc acctatgaca gcgtgaccat ctcctggacc     780

cgccagaatg gcgaagctgt gaaaacccac accaacatct ccgagagcca ccccaatgcc     840

actttcagcg ccgtgggtga ggccagcatc tgcgaggatg actggaattc cggggagagg     900

ttcacgtgca ccgtgaccca cacagacctg ccctcgccac tgaagcagac catctcccgg     960

cccaaggggg tggccctgca caggcccgat gtctacttgc tgccaccagc ccgggagcag    1020

ctgaacctgc gggagtcggc caccatcacg tgcctggtga cgggcttctc tcccgcggac    1080

gtcttcgtgc agtggatgca gagggggcag cccttgtccc cggagaagta tgtgaccagc    1140

gccccaatgc ctgagcccca ggccccaggc cggtacttcg cccacagcat cctgaccgtg    1200

tccgaagagg aatggaacac gggggagacc tacacctgcg tggtggccca tgaggccctg    1260

cccaacaggg tcaccgagag gaccgtggac aagtccaccg gtaaacccac cctgtacaac    1320

gtgtccctgg tcatgtccga cacagctggc acctgctact ga                        1362

<210>  226
<211>  1200
<212>  DNA
<213>  homo sapiens;

<400>  226
gcctccacca agggcccatc ggtcttcccc ctggcaccct cctccaagag cacctctggg      60

ggcacagcag ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacaca 960

cagaagagcc tctccctgtc tccggagctg caactggagg agagctgtgc ggaggcgcag 1020

gacggggagc tggacgggct gtggacgacc atcaccatct tcatcacact cttcctgtta 1080

agcgtgtgct acagtgccac cgtcaccttc ttcaaggtga agtggatctt ctcctcggtg 1140

gtggacctga agcagaccat catccccgac tacaggaaca tgatcggaca gggggcctag 1200

<210> 227
<211> 1293
<212> DNA
<213> homo sapiens;

<220>
<221> misc_feature
<222> (1)..(1)
<223> n is a, c, g, or t

<400> 227
ncacccacca aggctccgga tgtgttcccc atcatatcag ggtgcagaca cccaaaggat 60

aacagccctg tggtcctggc atgcttgata actgggtacc acccaacgtc cgtgactgtc 120

acctggtaca tggggacaca gagccagccc cagagaacct tccctgagat acaaagacgg 180

gacagctact acatgacaag cagccagctc tccacccccc tccagcagtg gcgccaaggc 240

gagtacaaat gcgtggtcca gcacaccgcc agcaagagta agaaggagat cttccgctgg 300

ccagagtctc caaaggcaca ggcctcctca gtgcccactg cacaacccca agcagagggc 360

agcctcgcca aggcaaccac agccccagcc accacccgta acacaggaag aggaggagaa 420

gagaagaaga aggagaagga gaaagaggaa caagaagaga gagagacaaa gacaccagag 480

tgtccgagcc acacccagcc tcttggcgtc tacctgctaa cccctgcagt gcaggacctg 540

tggctccggg acaaagccac cttcacctgc ttcgtggtgg gcagtgacct gaaggatgct 600

cacctgacct gggaggtggc cgggaaggtc cccacagggg gcgtggagga agggctgctg 660

gagcggcaca gcaacggctc ccagagccag cacagccgtc tgaccctgcc caggtccttg 720

tggaacgcgg ggacctccgt cacctgcaca ctgaaccatc ccagcctccc accccagagg 780

ttgatggcgc tgagagaacc cgctgcgcag gcacccgtca agctttccct gaacctgctg 840

gcctcgtctg accctcccga ggcggcctcg tggctcctgt gtgaggtgtc tggcttctcg 900

ccccccaaca tcctcctgat gtggctggag gaccagcgtg aggtgaacac ttctgggttt 960

gcccccgcac gcccccctcc acagcccggg agcaccacgt tctgggcctg gagtgtgctg 1020

cgtgtcccag ccccgcccag ccctcagcca gccacctaca cgtgtgtggt cagccacgag 1080

gactcccgga ctctgctcaa cgccagccgg agcctagaag tcagctacct ggccatgacc 1140

cccctgatcc ctcagagcaa ggatgagaac agcgatgact acacgacctt tgatgatgtg 1200

ggcagcctgt ggaccgccct gtccacgttt gtggccctct tcatcctcac cctcctctac 1260

agcggcattg tcactttcat caaggtgaag tag 1293

<210> 228
<211> 984
<212> DNA
<213> homo sapiens;

<400> 228
gcttccacca agggcccatc ggtcttcccc ctggcgccct gctccaggag cacctccgag 60

agcacagccg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacgaagacc 240

tacacctgca acgtagatca caagcccagc aacaccaagg tggacaagag agttgagtcc 300

aaatatggtc ccccatgccc atcatgccca gcacctgagt tcctgggggg accatcagtc 360

ttcctgttcc ccccaaaacc caaggacact ctcatgatct cccggacccc tgaggtcacg 420

tgcgtggtgg tggacgtgag ccaggaagac cccgaggtcc agttcaactg gtacgtggat 480

ggcgtggagg tgcataatgc caagacaaag ccgcgggagg agcagttcaa cagcacgtac 540

cgtgtggtca gcgtcctcac cgtcctgcac caggactggc tgaacggcaa ggagtacaag 600

tgcaaggtct ccaacaaagg cctcccgtcc tccatcgaga aaaccatctc caaagccaaa 660

gggcagcccc gagagccaca ggtgtacacc ctgcccccat cccaggagga gatgaccaag 720

aaccaggtca gcctgacctg cctggtcaaa ggcttctacc ccagcgacat cgccgtggag 780

tgggagagca atgggcagcc ggagaacaac tacaagacca cgcctcccgt gctggactcc 840

gacggctcct tcttcctcta cagcaggctc accgtggaca agagcaggtg gcaggagggg 900

aatgtcttct catgctccgt gatgcatgag gctctgcaca accactacac acagaagagc 960

ctctccctgt ctctgggtaa atga 984

<210> 229
<211> 228
<212> DNA
<213> homo sapiens;

<400> 229
tccggcttct tcgtcttcag ccgcctggag gtgaccaggg ccgaatggga gcagaaagat 60

gagttcatct gccgtgcagt ccatgaggca gcaagcccct cacagaccgt ccagcgagcg 120

gtgtctgtaa atcccgagct ggacgtgtgc gtggaggagg ccgagggcga ggcgccgtgg 180

acgtggaccg gcctctgcat cttcgccgca ctcttcctgc tcagcgtg 228

<210> 230
<211> 324
<212> DNA
<213> homo sapiens;

<220>
<221> misc_feature
<222> (1)..(1)
<223> n is a, c, g, or t

<400> 230
ngaactgtgg ctgcaccatc tgtcttcatc ttcccgccat ctgatgagca gttgaaatct 60

ggaactgcct ctgttgtgtg cctgctgaat aacttctatc ccagagaggc caaagtacag 120

tggaaggtgg ataacgccct ccaatcgggt aactcccagg agagtgtcac agagcaggac 180

agcaaggaca gcacctacag cctcagcagc accctgacgc tgagcaaagc agactacgag 240

aaacacaaag tctacgcctg cgaagtcacc catcagggcc tgagctcgcc cgtcacaaag 300

agcttcaaca ggggagagtg ttag 324

<210> 231
<211> 321
<212> DNA
<213> homo sapiens;

<220>
<221> misc_feature
<222> (1)..(1)
<223> n is a, c, g, or t

<400> 231
ngtcagccca aggctgcccc ctcggtcact ctgttcccgc cctcctctga ggagcttcaa 60

gccaacaagg ccacactggt gtgtctcata agtgacttct acccgggagc cgtgacagtg 120

gcctggaagg cagatagcag ccccgtcaag gcgggagtgg agaccaccac accctccaaa 180

caaagcaaca acaagtacgc ggccagcagc tatctgagcc tgacgcctga gcagtggaag 240

tcccacagaa gctacagctg ccaggtcacg catgaaggga gcaccgtgga gaagacagtg 300

gcccctacag aatgttcata g 321

<210> 232
<211> 321
<212> DNA
<213> homo sapiens;

<220>
<221> misc_feature
<222> (1)..(1)
<223> n is a, c, g, or t

<400> 232
ngtcagccca aggccaaccc cactgtcact ctgttcccgc cctcctctga ggagctccaa 60

gccaacaagg ccacactagt gtgtctgatc agtgacttct acccgggagc tgtgacagtg 120

gcctggaagg cagatggcag ccccgtcaag gcgggagtgg agaccaccaa accctccaaa 180

cagagcaaca acaagtacgc ggccagcagc tacctgagcc tgacgcccga gcagtggaag 240

tcccacagaa gctacagctg ccaggtcacg catgaaggga gcaccgtgga gaagacagtg 300

gcccctacag aatgttcata g 321

<210> 233
<211> 321
<212> DNA
<213> homo sapiens;

<220>
<221> misc_feature
<222> (1)..(1)
<223> n is a, c, g, or t

<400> 233
ngtcagccca aggctgcccc ctcggtcact ctgttcccgc cctcctctga ggagcttcaa 60

gccaacaagg ccacactggt gtgtctcata agtgacttct acccgggagc cgtgacagtg 120

gcctggaagg cagatagcag ccccgtcaag gcgggagtgg agaccaccac accctccaaa 180

caaagcaaca acaagtacgc ggccagcagc tacctgagcc tgacgcctga gcagtggaag 240

tcccacagaa gctacagctg ccaggtcacg catgaaggga gcaccgtgga gaagacagtg 300

gcccctacag aatgttcata g 321

<210> 234
<211> 321
<212> DNA
<213> homo sapiens;

<220>
<221>  misc_feature
<222>  (1)..(1)
<223>  n is a, c, g, or t

<400>  234
ngtcagccca aggctgcccc ctcggtcact ctgttcccac cctcctctga ggagcttcaa      60

gccaacaagg ccacactggt gtgtctcgta agtgacttct acccgggagc cgtgacagtg     120

gcctggaagg cagatggcag ccccgtcaag gtgggagtgg agaccaccaa accctccaaa     180

caaagcaaca acaagtatgc ggccagcagc tacctgagcc tgacgcccga gcagtggaag     240

tcccacagaa gctacagctg ccgggtcacg catgaaggga gcaccgtgga gaagacagtg     300

gcccctgcag aatgctctta g                                               321


<210>  235
<211>  61
<212>  DNA
<213>  homo sapiens;

<400>  235
tactactact actacggtat ggacgtctgg ggccaaggga ccacggtcac cgtctcctca      60

g                                                                      61


<210>  236
<211>  20
<212>  PRT
<213>  homo sapiens;

<400>  236

Tyr Tyr Tyr Tyr Tyr Gly Met Asp Val Trp Gly Gln Gly Thr Thr Val
1               5                   10                  15


Thr Val Ser Ser
            20


<210>  237
<211>  101
<212>  DNA
<213>  homo sapiens;

<400>  237
ggtttttgtg gggtgaggat ggacattctg ccattgtgat tactactact actacggtat      60

ggacgtctgg ggccaaggga ccacggtcac cgtctcctca g                         101


<210>  238
<211>  38
<212>  DNA
<213>  homo sapiens;

<400>  238

ggtttttgtg gggtgaggat ggacattctg ccattgtg                                38

<210>  239
<211>  52
<212>  DNA
<213>  homo sapiens;

<400>  239
gctgaatact tccagcactg gggccagggc accctggtca ccgtctcctc ag                52

<210>  240
<211>  52
<212>  DNA
<213>  homo sapiens;

<400>  240
tactggtact tcgatctctg gggccgtggc accctggtca ctgtctcctc ag               52

<210>  241
<211>  49
<212>  DNA
<213>  homo sapiens;

<400>  241
gatgcttttg atatctgggg ccaagggaca atggtcaccg tctcttcag                   49

<210>  242
<211>  46
<212>  DNA
<213>  homo sapiens;

<400>  242
tactttgact actggggcca gggaaccctg gtcaccgtct cctcag                      46

<210>  243
<211>  49
<212>  DNA
<213>  homo sapiens;

<400>  243
aactggttcg acccctgggg ccagggaacc ctggtcaccg tctcctcag                   49

<210>  244
<211>  61
<212>  DNA
<213>  homo sapiens;

<400>  244
tactactact actactacat ggacgtctgg ggcaaaggga ccacggtcac cgtctcctca       60

g                                                                       61

<210>  245
<211>  494
<212>  DNA
<213>  homo sapiens;

<400> 245
gcatcaccca aaaaccacac ccctccttgg gagaatcccc tagatcacag ctcctcacca 60

tggactggac ctggagcatc cttttcttgg tggcagcagc aacaggtaac ggactcccca 120

gtcccagggc tgagagagaa accaggccag tcatgtgaga cttcacccac tcctgtgtcc 180

tctccacagg tgcccactcc caggttcagc tggtgcagtc tggagctgag gtgaagaagc 240

ctggggcctc agtgaaggtc tcctgcaagg cttctggtta cacctttacc agctatggta 300

tcagctgggt gcgacaggcc cctggacaag ggcttgagtg gatgggatgg atcagcgctt 360

acaatggtaa cacaaactat gcacagaagc tccagggcag agtcaccatg accacagaca 420

catccacgag cacagcctac atggagctga ggagcctgag atctgacgac acggccgtgt 480

attactgtgc gaga 494


<210> 246
<211> 500
<212> DNA
<213> homo sapiens;

<400> 246
gagagcatca cccagcaacc acatctgtcc tctagagaat cccctgagag ctccgttcct 60

caccatggac tggacctgga ggatcctctt cttggtggca gcagccacag gtaagaggct 120

ccctagtccc agtgatgaga aagagattga gtccagtcca gggagatctc atccacttct 180

gtgttctctc cacaggagcc cactcccagg tgcagctggt gcagtctggg gctgaggtga 240

agaagcctgg ggcctcagtg aaggtctcct gcaaggcttc tggatacacc ttcaccggct 300

actatatgca ctgggtgcga caggcccctg gacaagggct tgagtggatg ggatggatca 360

accctaacag tggtggcaca aactatgcac agaagtttca gggcagggtc accatgacca 420

gggacacgtc catcagcaca gcctacatgg agctgagcag gctgagatct gacgacacgg 480

ccgtgtatta ctgtgcgaga 500


<210> 247
<211> 496
<212> DNA
<213> homo sapiens;

<400> 247
accatcacac aacagccaca tccctcccct acagaagccc ccagagcgca gcacctcacc 60

atggactgca cctggaggat cctcttcttg gtggcagcag ctacaggcaa gagaatcctg 120

agttccaggg ctgatgaggg gactgggtcc agttaagtgg tgtctcatcc actcctctgt 180

cctctccaca ggcacccacg cccaggtcca gctggtacag tctggggctg aggtgaagaa 240

gcctggggcc tcagtgaagg tctcctgcaa ggtttccgga tacaccctca ctgaattatc 300

catgcactgg gtgcgacagg ctcctggaaa agggcttgag tggatgggag gttttgatcc 360

tgaagatggt gaaacaatct acgcacagaa gttccagggc agagtcacca tgaccgagga 420

cacatctaca gacacagcct acatggagct gagcagcctg agatctgagg acacggccgt 480

gtattactgt gcaaca 496

<210> 248
<211> 478
<212> DNA
<213> homo sapiens;

<400> 248
ccacatccct cctcagaagc ccccagagca caactcctca ccatggactg gacctggagg 60

atcctctttt tggtggcagc agccacaggt aaggggctgc caaatcccag tgaggaggaa 120

gggatcgaag ccagtcaagg gggcttccat ccactcctgt gtcttctcta caggtgtcca 180

ctcccaggtt cagctggtgc agtctggggc tgaggtgaag aagcctgggg cctcagtgaa 240

ggtttcctgc aaggcttctg gatacacctt cactagctat gctatgcatt gggtgcgcca 300

ggcccccgga caaaggcttg agtggatggg atggagcaac gctggcaatg gtaacacaaa 360

atattcacag gagttccagg gcagagtcac cattaccagg gacacatccg cgagcacagc 420

ctacatggag ctgagcagcc tgagatctga ggacatggct gtgtattact gtgcgaga 478

<210> 249
<211> 494
<212> DNA
<213> homo sapiens;

<400> 249
atcacccaac aaccacatcc ctcctctaga gaatcccctg aaagcacagc tcctcaccat 60

ggactggacc tggagaatcc tcttcttggt ggcagcagcc acaggtaagg ggctcccaag 120

tcccagtgat gaggagggga ttgagtccag tcaaggtggc ttttatccac tcctgtgtcc 180

cctccacaga tgcctactcc cagatgcagc tggtgcagtc tggggctgag gtgaagaaga 240

ctgggtcctc agtgaaggtt tcctgcaagg cttccggata caccttcacc taccgctacc 300

tgcactgggt gcgacaggcc cccggacaag cgcttgagtg gatgggatgg atcacacctt 360

tcaatggtaa caccaactac gcacagaaat tccaggacag agtcaccatt accagggaca 420

ggtctatgag cacagcctac atggagctga gcagcctgag atctgaggac acagccatgt 480

attactgtgc aaga 494

<210> 250
<211> 740
<212> DNA
<213> homo sapiens;

<400> 250
atctgtgggg acttgttctt cagtgaaagg atcctgtccg caaacagaaa tggagcagga 60

catgcatttc ttcaagcagg attagggctt ggaccatcag catcccactc ctgtgtggca 120

gatgggacat ctatcttctt tctcaacctc gatcaggctt tgaggtatga aataatctgt 180

ctcatgaata tgcaaataac cttagatcta ctgaggtaaa tatggataca tctgggccct 240

gaaagcatca tccaacaacc acatcccttc tctacagaag cctctgagag gaaagttctt 300

caccatggac tggacctgga gggtcttctg cttgctggct gtagctccag gtaaagggcc 360

aactggttcc agggctgagg aaggattttt ttccagttta gaggactgtc attctctact 420

gtgtcctctc cgcaggtgct cactcccagg tgcagctggt gcagtctggg gctgaggtga 480

agaagcctgg ggcctcagtg aaggtttcct gcaaggcatc tggatacacc ttcaccagct 540

actatatgca ctgggtgcga caggcccctg gacaagggct tgagtggatg ggaataatca 600

accctagtgg tggtagcaca agctacgcac agaagttcca gggcagagtc accatgacca 660

gggacacgtc cacgagcaca gtctacatgg agctgagcag cctgagatct gaggacacgg 720

ccgtgtatta ctgtgcgaga 740

<210> 251
<211> 497
<212> DNA
<213> homo sapiens;

<400> 251
agcatcatcc agaaaccaca tccctccgct agagaagccc ctgacggcac agttcctcac 60

tatggactgg atttggaggg tcctcttctt ggtgggagca gcgacaggca aggagatgcc 120

aagtcccagt gatgaggagg ggattgagtc cagtcaaggt ggctttcatc cactcctgtg 180

ttctctccac aggtgcccac tcccaaatgc agctggtgca gtctgggcct gaggtgaaga 240

agcctgggac ctcagtgaag gtctcctgca aggcttctgg attcaccttt actagctctg 300

ctatgcagtg ggtgcgacag gctcgtggac aacgccttga gtggatagga tggatcgtcg 360

ttggcagtgg taacacaaac tacgcacaga agttccagga aagagtcacc attaccaggg 420

acatgtccac aagcacagcc tacatggagc tgagcagcct gagatccgag gacacggccg 480

tgtattactg tgcggca 497

<210> 252
<211> 498
<212> DNA
<213> homo sapiens;

<400> 252
agcatcacat aacaaccaca ttcctcctct gaagaagccc ctgggagcac agctcatcac 60

catggactgg acctggaggt tcctctttgt ggtggcagca gctacaggta aggggcttcc 120

tagtcctaag gctgaggaag ggatcctggt ttagttaaag aggattttat tcacccctgt 180

gtcctctcca caggtgtcca gtcccaggtg cagctggtgc agtctggggc tgaggtgaag 240

aagcctgggt cctcggtgaa ggtctcctgc aaggcttctg gaggcacctt cagcagctat 300

gctatcagct gggtgcgaca ggcccctgga caagggcttg agtggatggg agggatcatc 360

cctatctttg gtacagcaaa ctacgcacag aagttccagg gcagagtcac gattaccgcg 420

gacaaatcca cgagcacagc ctacatggag ctgagcagcc tgagatctga ggacacggcc 480

gtgtattact gtgcgaga 498

<210> 253
<211> 499
<212> DNA
<213> homo sapiens;

<400> 253
gagcatcact caacaaccac atctgtcctc tagagaaaac cctgtgagca cagctcctca 60

ccatggactg gacctggagg atcctcttct tggtggcagc agctacaagt aaggggcttc 120

ctagtctcaa agctgaggaa cggatcctgg ttcagtcaaa gaggatttta ttctctcctg 180

tgttctctcc acaggtgccc actcccaggt gcagctggtg cagtctgggg ctgaggtgaa 240

gaagcctggg gcctcagtga aggtctcctg caaggcttct ggatacacct tcaccagtta 300

tgatatcaac tgggtgcgac aggccactgg acaagggctt gagtggatgg gatggatgaa 360

ccctaacagt ggtaacacag gctatgcaca gaagttccag ggcagagtca ccatgaccag 420

gaacacctcc ataagcacag cctacatgga gctgagcagc ctgagatctg aggacacggc 480

cgtgtattac tgtgcgaga 499

<210> 254
<211> 467
<212> DNA
<213> homo sapiens;

<400> 254
gctcagtgac tcctgtgccc caccatggac acactttgct acacactcct gctgctgacc 60

accccttcct gtgagtgctg tggtcaggga cttcctcaga agtgaaacat cagttgtctc 120

ctttgtgggc ttcatcttct tatgtcttct ccacaggggt cttgtcccag gtcaccttga 180

aggagtctgg tcctgtgctg gtgaaaccca cagagaccct cacgctgacc tgcaccgtct 240

ctgggttctc actcagcaat gctagaatgg gtgtgagctg gatccgtcag cccccaggga 300

aggccctgga gtggcttgca cacatttttt cgaatgacga aaaatcctac agcacatctc 360

tgaagagcag gctcaccatc tccaaggaca cctccaaaag ccaggtggtc cttaccatga 420

ccaacatgga ccctgtggac acagccacat attactgtgc acggata 467

<210> 255
<211> 463

<212>  DNA
<213>  homo sapiens;

<400>  255
agtgactcct gtgccccacc atggacacac tttgctccac gctcctgctg ctgaccatcc      60

cttcatgtga gtgctgtggt cagggactcc ttcacgggtg aaacatcagt tttcttgttt     120

gtgggcttca tcttcttatg ctttctccac aggggtcttg tcccagatca ccttgaagga     180

gtctggtcct acgctggtga aacccacaca gaccctcacg ctgacctgca ccttctctgg     240

gttctcactc agcactagtg gagtgggtgt gggctggatc cgtcagcccc caggaaaggc     300

cctggagtgg cttgcactca tttattggaa tgatgataag cgctacagcc catctctgaa     360

gagcaggctc accatcacca aggacacctc caaaaaccag gtggtcctta caatgaccaa     420

catggaccct gtggacacag ccacatatta ctgtgcacac aga                       463


<210>  256
<211>  519
<212>  DNA
<213>  homo sapiens;

<400>  256
atctccacca gctccaccct cccctgggtt caaaagacga ggacagggcc tcgctcagtg      60

aatcctgctc tccaccatgg acatactttg ttccacgctc ctgctactga ctgtcccgtc     120

ctgtgagtgc tgtggtcagg tagtacttca gaagcaaaaa atctattctc tcctttgtgg     180

gcttcatctt cttatgtctt ctccacaggg gtcttatccc aggtcacctt gagggagtct     240

ggtcctgcgc tggtgaaacc cacacagacc ctcacactga cctgcacctt ctctgggttc     300

tcactcagca ctagtggaat gtgtgtgagc tggatccgtc agcccccagg gaaggccctg     360

gagtggcttg cactcattga ttgggatgat gataaatact acagcacatc tctgaagacc     420

aggctcacca tctccaagga cacctccaaa aaccaggtgg tccttacaat gaccaacatg     480

gaccctgtgg acacagccac gtattattgt gcacggata                            519


<210>  257
<211>  568
<212>  DNA
<213>  homo sapiens;

<400>  257
ctccctctgc tgataaaaac cagccgagcc cagaccctgc agctctggga gaagagcccc      60

agccccagaa ttcccaggag tttccattcg gtgatcagca ctgaacacag aggactcacc     120

atggagtttg ggctgagctg ggttttcctt gttgctatta taaaaggtga tttatggaga     180

actagagaca ttgagtggac gtgagtgaga taagcagtga atatatgtgg cagtttctga     240

ctaggttgtc tctgtgtttg caggtgtcca gtgtcaggtg cagctggtgg agtctggggg     300

aggcttggtc aagcctggag ggtccctgag actctcctgt gcagcctctg gattcacctt     360

cagtgactac tacatgagct ggatccgcca ggctccaggg aaggggctgg agtgggtttc 420

atacattagt agtagtggta gtaccatata ctacgcagac tctgtgaagg gccgattcac 480

catctccagg gacaacgcca agaactcact gtatctgcaa atgaacagcc tgagagccga 540

ggacacggcc gtgtattact gtgcgaga 568

<210> 258
<211> 531
<212> DNA
<213> homo sapiens;

<400> 258
agctctggga gtggagcccc agccttggga ttcccaagtg tttgtattca gtgatcagga 60

ctgaacacac aggactcacc atggagttgg ggctgagctg ggttttcctt gttgctatat 120

tagaaggtga ttcatggaga actagagata ttgagtgtga atgggcatga atgagagaaa 180

cagtgggtat gtgtggcaat ttctgacttt tgtgtctctg tgtttgcagg tgtccagtgt 240

gaggtgcagc tggtggagtc tgggggaggc ttggtacagc ctggggggtc cctgagactc 300

tcctgtgcag cctctggatt caccttcagt agctacgaca tgcactgggt ccgccaagct 360

acaggaaaag gtctggagtg ggtctcagct attggtactg ctggtgacac atactatcca 420

ggctccgtga agggccgatt caccatctcc agagaaaatg ccaagaactc cttgtatctt 480

caaatgaaca gcctgagagc cggggacacg gctgtgtatt actgtgcaag a 531

<210> 259
<211> 540
<212> DNA
<213> homo sapiens;

<400> 259
agctctggga gaggagcccc agccttggga ttcccaagtg ttttcattca gtgatcagga 60

ctgaacacag aggactcacc atggagtttg ggctgagctg gattttcctt gctgctattt 120

taaaaggtga tttatggaga actagagaga ttaagtgtga gtggacgtga gtgagagaaa 180

cagtggatat gtgtggcagt ttctgatctt agtgtctctg tgtttgcagg tgtccagtgt 240

gaggtgcagc tggtggagtc tgggggaggc ttggtaaagc ctggggggtc ccttagactc 300

tcctgtgcag cctctggatt cactttcagt aacgcctgga tgagctgggt ccgccaggct 360

ccagggaagg ggctggagtg ggttggccgt attaaaagca aaactgatgg tgggacaaca 420

gactacgctg cacccgtgaa aggcagattc accatctcaa gagatgattc aaaaaacacg 480

ctgtatctgc aaatgaacag cctgaaaacc gaggacacag ccgtgtatta ctgtaccaca 540

<210> 260
<211> 526
<212> DNA

<213>  homo sapiens;

<400>  260
agccctggga gagaagcccc agccctggga ttctcaggtg tttctattgg gtcaacagca      60

ataaacaaat taccatggaa tttgggctga gctgggtttt tcttgctggt attttaaaag     120

gtgattcatg gagaactaag gatattgagt gagtggacat gagtgagaga aacagtggat     180

atgtgtggca gtttctgacc agggtgtctc tgtgtttgca ggtgtccagt gtgaggtgca     240

gctggtggag tctgggggag gcttggtaca gcctgggggg tccctgagac tctcctgtgc     300

agcctctgga ttcaccttca gtaacagtga catgaactgg gcccgcaagg ctccaggaaa     360

ggggctggag tgggtatcgg gtgttagttg gaatggcagt aggacgcact atgtggactc     420

cgtgaagcgc cgattcatca tctccagaga caattccagg aactccctgt atctgcaaaa     480

gaacagacgg agagccgagg acatggctgt gtattactgt gtgaga                    526


<210>  261
<211>  515
<212>  DNA
<213>  homo sapiens;

<400>  261
ccagccctga gattcccacg tgtttccatt cagtgatcag cactgaacac agaggactcg      60

ccatggagtt tgggctgagc tgggttttcc ttgttgctat tttaaaaggt gattcatgga     120

tcaatagaga tgttgagtgt gagtgaacac gagtgagaga aacagtggat ttgtgtggca     180

gtttctgacc aggtgtctct gtgtttgcag gtgtccagtg tgaggtgcag ctggtggagt     240

ctgggggagg tgtggtacgg cctggggggt ccctgagact ctcctgtgca gcctctggat     300

tcacctttga tgattatggc atgagctggg tccgccaagc tccagggaag gggctggagt     360

gggtctctgg tattaattgg aatggtggta gcacaggtta tgcagactct gtgaagggcc     420

gattcaccat ctccagagac aacgccaaga actccctgta tctgcaaatg aacagtctga     480

gagccgagga cacggccttg tatcactgtg cgaga                                515


<210>  262
<211>  531
<212>  DNA
<213>  homo sapiens;

<400>  262
agctctgaga gaggagcctt agccctggat tccaaggcct atccacttgg tgatcagcac      60

tgagcaccga ggattcacca tggaactggg gctccgctgg gttttccttg ttgctatttt     120

agaaggtgaa tcatggaaaa gtagagagat ttagtgtgtg tggatatgag tgagagaaac     180

ggtggatgtg tgtgacagtt tctgaccaat gtctctctgt ttgcaggtgt ccagtgtgag     240

gtgcagctgg tggagtctgg gggaggcctg gtcaagcctg gggggtccct gagactctcc     300

tgtgcagcct ctggattcac cttcagtagc tatagcatga actgggtccg ccaggctcca 360

gggaaggggc tggagtgggt ctcatccatt agtagtagta gtagttacat atactacgca 420

gactcagtga agggccgatt caccatctcc agagacaacg ccaagaactc actgtatctg 480

caaatgaaca gcctgagagc cgaggacacg gctgtgtatt actgtgcgag a 531

<210> 263
<211> 533
<212> DNA
<213> homo sapiens;

<400> 263
agctctgaga gaggagccca gccctgggat tttcaggtgt tttcatttgg tgatcaggac 60

tgaacagaga gaactcacca tggagtttgg gctgagctgg ctttttcttg tggctatttt 120

aaaaggtaat tcatggagaa atagaaaaat tgagtgtgaa tggataagag tgagagaaac 180

agtggatacg tgtggcagtt tctgaccagg gtttcttttt gtttgcaggt gtccagtgtg 240

aggtgcagct gttggagtct gggggaggct tggtacagcc tggggggtcc ctgagactct 300

cctgtgcagc ctctggattc acctttagca gctatgccat gagctgggtc cgccaggctc 360

cagggaaggg gctggagtgg gtctcagcta ttagtggtag tggtggtagc acatactacg 420

cagactccgt gaagggccgg ttcaccatct ccagagacaa ttccaagaac acgctgtatc 480

tgcaaatgaa cagcctgaga gccgaggaca cggccgtata ttactgtgcg aaa 533

<210> 264
<211> 532
<212> DNA
<213> homo sapiens;

<400> 264
cagctctggg agaggagccc agcactagaa gtcggcggtg tttccattcg gtgatcagca 60

ctgaacacag aggactcacc atggagtttg ggctgagctg ggttttcctc gttgctcttt 120

taagaggtga ttcatggaga aatagagaga ctgagtgtga gtgaacatga gtgagaaaaa 180

ctggatttgt gtggcatttt ctgataacgg tgtccttctg tttgcaggtg tccagtgtca 240

ggtgcagctg gtggagtctg ggggaggcgt ggtccagcct gggaggtccc tgagactctc 300

ctgtgcagcc tctggattca ccttcagtag ctatggcatg cactgggtcc gccaggctcc 360

aggcaagggg ctggagtggg tggcagttat atcatatgat ggaagtaata aatactatgc 420

agactccgtg aagggccgat tcaccatctc cagagacaat tccaagaaca cgctgtatct 480

gcaaatgaac agcctgagag ctgaggacac ggctgtgtat tactgtgcga ga 532

<210> 265
<211> 532
<212> DNA
<213> homo sapiens;

<400>  265
cagctctggg agaggagccc agcactagaa gtcggcggtg tttccattcg gtgatcagca      60

ctgaacacag aggactcacc atggagtttg ggctgagctg ggttttcctc gttgctcttt     120

taagaggtga ttcatggaga aatagagaga ctgagtgtga gtgaacatga gtgagaaaaa     180

ctggatttgt gtggcatttt ctgataacgg tgtccttctg tttgcaggtg tccagtgtca     240

ggtgcagctg gtggagtctg ggggaggcgt ggtccagcct gggaggtccc tgagactctc     300

ctgtgcagcg tctggattca ccttcagtag ctatggcatg cactgggtcc gccaggctcc     360

aggcaagggg ctggagtggg tggcagttat atggtatgat ggaagtaata aatactatgc     420

agactccgtg aagggccgat tcaccatctc cagagacaat tccaagaaca cgctgtatct     480

gcaaatgaac agcctgagag ccgaggacac ggctgtgtat tactgtgcga ga             532


<210>  266
<211>  467
<212>  DNA
<213>  homo sapiens;

<400>  266
aacaaacaaa ttaccatgga atttgggctg agctgggttt ttcttgctgc tattttaaaa      60

ggtgattcat gaagaactaa ggatattgag tgagtggaca tgagtgagag aaacagtgga     120

tttgtgtggc agtttctgac cagggtgtct ctgtgtttgc aggtgtccag tgtgaggtgc     180

agctggtgga gtctggggga ggcttggtac agcctggggg atccctgaga ctctcctgtg     240

cagcctctgg attcaccttc agtaacagtg acatgaactg ggtccatcag gctccaggaa     300

aggggctgga gtgggtatcg ggtgttagtt ggaatggcag taggacgcac tatgcagact     360

ctgtgaaggg ccgattcatc atctccagag acaattccag gaacaccctg tatctgcaaa     420

cgaatagcct gagggccgag gacacggctg tgtattactg tgtgaga                   467


<210>  267
<211>  529
<212>  DNA
<213>  homo sapiens;

<400>  267
   ctctgggagt ggagccccag ccttgggatt cccaggtgtt tcccttcagt gatcaggact      60
   gaacacacac aactcatcat gcagtttgtg ctgagctggg ttttccttgt tggtatttta     120
   aaaggtgatt catggagaac tacagatgtt gagtgtgagt ggacatgagt gagcaaaaca     180
   gtgggtttgt gtggcagttt ctgaccttgg tgtctctgtg tttgcaggtg tccagtgtga     240
   ggtgcagctg gtggagtctg ggggaggctt ggtacagcct agggggtccc tgagactctc     300
   ctgtgcagcc tctggattca ccgtcagtag caatgagatg agctggatcc gccaggctcc     360
   agggaagggg ctggagtggg tctcatccat tagtggtggt agcacatact acgcagactc     420
   caggaagggc agattcacca tctccagaga caattccaag aacacgctgt atcttcaaat 480
   gaacaacctg agagctgagg gcacggccgt gtattactgt gccagatat 529

<210> 268
<211> 537
<212> DNA
<213> homo sapiens;

<400> 268
agctctggga gaggagcccc agccctgaga ttcccaggtg tttccattcg gtgatcagca 60

ctgaacacag agaacgcacc atggagtttg gactgagctg ggttttcctt gttgctattt 120

taaaaggtga ttcatggata aatagagatg ttgagtgtga gtgaacatga gtgagagaaa 180

cagtggatat gtgtggcagt gtctgaccag ggtgtctctg tgtttgcagg tgtccagtgt 240

gaagtgcagc tggtggagtc tgggggagtc gtggtacagc ctggggggtc cctgagactc 300

tcctgtgcag cctctggatt cacctttgat gattatacca tgcactgggt ccgtcaagct 360

ccggggaagg gtctggagtg ggtctctctt attagttggg atggtggtag cacatactat 420

gcagactctg tgaagggccg attcaccatc tccagagaca acagcaaaaa ctccctgtat 480

ctgcaaatga acagtctgag aactgaggac accgccttgt attactgtgc aaaagat 537

<210> 269
<211> 533
<212> DNA
<213> homo sapiens;

<400> 269
agctctcaga gaggtgcctt agccctggat tccaaggcat ttccacttgg tgatcagcac 60

tgaacacaga ggactcacca tggagttggg gctgtgctgg gttttccttg ttgctatttt 120

agaaggtgat tcatggaaaa ctagagagat ttagtgtgtg tggatatgag tgagagaaac 180

agtggatatg tgtggcagtt tctgaccttg gtgtctcttt gtttgcaggt gtccagtgtg 240

aggtgcagct ggtggagtct gggggaggct tggtacagcc tggggggtcc ctgagactct 300

cctgtgcagc ctctggattc accttcagta gctatagcat gaactgggtc cgccaggctc 360

cagggaaggg gctggagtgg gtttcataca ttagtagtag tagtagtacc atatactacg 420

cagactctgt gaagggccga ttcaccatct ccagagacaa tgccaagaac tcactgtatc 480

tgcaaatgaa cagcctgaga gacgaggaca cggctgtgta ttactgtgcg aga 533

<210> 270
<211> 540
<212> DNA
<213> homo sapiens;

<400> 270
agctctggga gaggagcccc agccgtgaga ttcccaggag tttccacttg gtgatcagca 60

ctgaacacag accaccaacc atggagtttg ggcttagctg ggttttcctt gttgctattt 120

taaaaggtaa ttcatggtgt actagagata ctgagtgtga ggggacatga gtggtagaaa 180

cagtggatat gtgtggcagt ttctgacctt ggtgtttctg tgtttgcagg tgtccaatgt 240

gaggtgcagc tggtggagtc tgggggaggc ttggtacagc cagggcggtc cctgagactc 300

tcctgtacag cttctggatt cacctttggt gattatgcta tgagctggtt ccgccaggct 360

ccagggaagg ggctggagtg ggtaggtttc attagaagca aagcttatgg tgggacaaca 420

gaatacgccg cgtctgtgaa aggcagattc accatctcaa gagatgattc caaaagcatc 480

gcctatctgc aaatgaacag cctgaaaacc gaggacacag ccgtgtatta ctgtactaga 540

<210> 271
<211> 670
<212> DNA
<213> homo sapiens;

<400> 271
aataccaatc tcccccagga cacttcatct gcacggagcc cggcctctcc tcagatgtcc 60

caccccagag cttgctatat agtcggggac atccaaatag ggccctccct ctgctgatga 120

aaaccagccc agctgaccct gcagctctgg gagaggagcc cagcactggg attccgaggt 180

gtttccattc ggtgatcagc actgaacaca gaggactcac catggagttt tggctgagct 240

gggttttcct tgttgctatt ttaaaaggtg attcatggag aactagagat attgagtgtg 300

agtgaacacg agtgagagaa acagtggata tgtgtggcag tttctaacca atgtctctgt 360

gtttgcaggt gtccagtgtg aggtgcagct ggtggagtct ggaggaggct tgatccagcc 420

tggggggtcc ctgagactct cctgtgcagc ctctgggttc accgtcagta gcaactacat 480

gagctgggtc cgccaggctc cagggaaggg gctggagtgg gtctcagtta tttatagcgg 540

tggtagcaca tactacgcag actccgtgaa gggccgattc accatctcca gagacaattc 600

caagaacacg ctgtatcttc aaatgaacag cctgagagcc gaggacacgg ccgtgtatta 660

ctgtgcgaga 670

<210> 272
<211> 534
<212> DNA
<213> homo sapiens;

<400> 272
agctctggga gaggagcccc cgccctggga ttcccaggtg ttttcatttg gtgatcagca 60

ctgaacacag aagagtcatg atggagtttg ggctgagctg ggttttcctt gttgctattt 120

ttaaaggtga ttcatgagga aatagagata ttgagtgtga gtggacatga gtgagagaaa 180

cagtggattt gtgtggcagt ttctgacctt ggtgtctctg tgtttgcagg tgtccagtgt 240

gaggtgcagc tggtggagtc tggggaaggc ttggtccagc ctggggggtc cctgagactc 300

tcctgtgcag cctctggatt caccttcagt agctatgcta tgcactgggt ccgccaggct 360

ccagggaagg gactggaata tgtttcagct attagtagta atgggggtag cacatattat 420

gcagactctg tgaagggcag attcaccatc tccagagaca attccaagaa cacgctgtat 480

cttcaaatgg gcagcctgag agctgaggac atggctgtgt attactgtgc gaga 534

<210> 273
<211> 528
<212> DNA
<213> homo sapiens;

<400> 273
agctctggga gaggagccca gcactgggat tccgaggtgt ttccattcag tgatctgcac 60

tgaacacaga ggactcgcca tggagtttgg gctgagctgg gttttccttg ttgctatttt 120

aaaaggtgat tcatggagaa ctagagatat tgagtgtgag tgaacacgag tgagagaaac 180

agtggatatg tgtggcagtt tctaaccaat gtctctgtgt ttgcaggtgt ccagtgtgag 240

gtgcagctgg tggagtctgg aggaggcttg atccagcctg gggggtccct gagactctcc 300

tgtgcagcct ctgggttcac cgtcagtagc aactacatga gctgggtccg ccaggctcca 360

gggaaggggc tggagtgggt ctcagttatt tatagctgtg gtagcacata ctacgcagac 420

tccgtgaagg gccgattcac catctccaga gacaattcca agaacacgct gtatcttcaa 480

atgaacagcc tgagagctga ggacacggct gtgtattact gtgcgaga 528

<210> 274
<211> 533
<212> DNA
<213> homo sapiens;

<400> 274
aggtctcaga gaggagcctt agccctggac tccaaggcct ttccacttgg tgatcagcac 60

tgagcacaga ggactcacca tggaattggg gctgagctgg gttttccttg ttgctatttt 120

agaaggtgat tcatggaaaa ctaggaagat tgagtgtgtg tggatatgag tgtgagaaac 180

agtggatttg tgtggcagtt tctgaccttg gtgtctcttt gtttgcaggt gtccagtgtg 240

aggtgcagct ggtggagtct gggggaggct tggtccagcc tggggggtcc ctgagactct 300

cctgtgcagc ctctggattc acctttagta gctattggat gagctgggtc cgccaggctc 360

cagggaaggg gctggagtgg gtggccaaca taaagcaaga tggaagtgag aaatactatg 420

tggactctgt gaagggccga ttcaccatct ccagagacaa cgccaagaac tcactgtatc 480

tgcaaatgaa cagcctgaga gccgaggaca cggctgtgta ttactgtgcg aga 533

<210> 275
<211> 540

<212>  DNA
<213>  homo sapiens;

<400>  275
agctctgaga gcggagcccc agccccagaa ttcccaggtg ttttcatttg gtgatcagca      60

ctgaacacag aggactcacc atggagtttg ggctgagctg ggttttcctt gttgttattt     120

tacaaggtga tttatggaga actagagatg ttaagtgtga gtggacgtga gtgagagaaa     180

cagtggattt gtgtgacagt ttctgaccag ggtgtctctg tgtttgcagg tgtccagtgt     240

gaggtgcagc tggtggagtc tgggggaggc ttggtccagc ctggagggtc cctgagactc     300

tcctgtgcag cctctggatt caccttcagt gaccactaca tggactgggt ccgccaggct     360

ccagggaagg ggctggagtg ggttggccgt actagaaaca aagctaacag ttacaccaca     420

gaatacgccg cgtctgtgaa aggcagattc accatctcaa gagatgattc aaagaactca     480

ctgtatctgc aaatgaacag cctgaaaacc gaggacacgg ccgtgtatta ctgtgctaga     540


<210>  276
<211>  540
<212>  DNA
<213>  homo sapiens;

<400>  276
   agctctggga gaggagctcc agccttggga ttcccagctg tctccactcg gtgatcggca      60
   ctgaatacag gagactcacc atggagtttg ggctgagctg ggttttcctt gttgctattt     120
   taaaaggtga ttcatgggga actagagata ctgagtgtga gtggacatga gtgagagaaa     180
   cagtggacgt gtgtggcact ttctgaccag ggtgtctctg tgtttgcagg tgtccagtgt     240
   gaggtgcagc tggtggagtc cgggggaggc ttggtccagc ctggggggtc cctgaaactc     300
   tcctgtgcag cctctgggtt caccttcagt ggctctgcta tgcactgggt ccgccaggct     360
   tccgggaaag ggctggagtg ggttggccgt attagaagca aagctaacag ttacgcgaca     420
   gcatatgctg cgtcggtgaa aggcaggttc accatctcca gagatgattc aaagaacacg     480
   gcgtatctgc aaatgaacag cctgaaaacc gaggacacgg ccgtgtatta ctgtactaga     540


<210>  277
<211>  690
<212>  DNA
<213>  homo sapiens;

<400>  277
aatttctcaa atcccattgt tgtcacccat cttcctcagg acactttcat ctgccctggg      60

tcctgctctt tcttcaggtg tctcacccca gagcttgata tatagtagga gacatgcaaa     120

tagggccctc actctgctga agaaaaccag ccctgcagct ctgggagagg agccccagcc     180

ctgggattcc cagctgtttc tgcttgctga tcaggactgc acacagagaa ctcaccatgg     240

agtttgggct gagctgggtt ttccttgttg ctattttaaa aggtgattca tggagaactg     300

gagatatgga gtgtgaatgg acatgagtga gataagcagt ggatgtgtgt ggcagtttct 360

gaccagggtg tctctgtgtt tgcaggtgtc cagtgtgagg tgcagctggt ggagtccggg 420

ggaggcttag ttcagcctgg ggggtccctg agactctcct gtgcagcctc tggattcacc 480

ttcagtagct actggatgca ctgggtccgc caagctccag ggaaggggct ggtgtgggtc 540

tcacgtatta atagtgatgg gagtagcaca agctacgcgg actccgtgaa gggccgattc 600

accatctcca gagacaacgc caagaacacg ctgtatctgc aaatgaacag tctgagagcc 660

gaggacacgg ctgtgtatta ctgtgcaaga 690

<210> 278
<211> 525
<212> DNA
<213> homo sapiens;

<400> 278
agctctggga gaggagcccc agccctgaga ttcccaggtg tttccattca gtgatcagca 60

ctgaacacag aggactcacc atggagttgg gactgagctg gattttcctt ttggctattt 120

taaaaggtga ttcatggaga aatagagaga ttgagtgtga gtggacatga gtggatttgt 180

gtggcagttt ctgaccttgg tgtctctgtg tttgcaggtg tccagtgtga agtgcagctg 240

gtggagtctg ggggaggctt ggtacagcct ggcaggtccc tgagactctc ctgtgcagcc 300

tctggattca cctttgatga ttatgccatg cactgggtcc ggcaagctcc agggaagggc 360

ctggagtggg tctcaggtat tagttggaat agtggtagca taggctatgc ggactctgtg 420

aagggccgat tcaccatctc cagagacaac gccaagaact ccctgtatct gcaaatgaac 480

agtctgagag ctgaggacac ggccttgtat tactgtgcaa aagat 525

<210> 279
<211> 505
<212> DNA
<213> homo sapiens;

<400> 279
atttccttaa attcagggtc ctgctcacat gggaaatact ttctgagagt cctggacctc 60

ctgtgcaaga acatgaaaca cctgtggttc ttcctcctgc tggtggcagc tcccagatgt 120

gagtgtctca aggctgcaga catggagata tgggaggtgc ctctgagccc agggctcact 180

gtgggtctct ctgttcacag tggtcctgtc ccaggtgcag ctgcaggagt cgggcccagg 240

actggtgaag ccttcggaca ccctgtccct cacctgcgct gtctctggtt actccatcag 300

cagtagtaac tggtggggct ggatccggca gcccccaggg aagggactgg agtggattgg 360

gtacatctat tatagtggga gcacctacta caacccgtcc ctcaagagtc gagtcaccat 420

gtcagtagac acgtccaaga accagttctc cctgaagctg agctctgtga ccgccgtgga 480

cacggccgtg tattactgtg cgaga                                          505

<210>  280
<211>  508
<212>  DNA
<213>  homo sapiens;

<400>  280
atttccttaa attcagggtc ctgctcacat gggaaatact ttctgagagt cctggacctc      60

ctgtgcaaga acatgaaaca cctgtggttc ttcctcctgc tggtggcagc tcccagatgt     120

gagtgtctca aggctgcaga catggagata tgggaggtgc ctctgatccc agggctcact     180

gtgtgtctct ctgttcacag gggtcctgcc ccaggtgcag ctgcaggagt cgggcccagg     240

actggtgaag ccttcacaga ccctgtccct cacctgtact gtctctggtg gctccatcag     300

cagtggtggt tactactgga gctggatccg ccagcaccca gggaagggcc tggagtggat     360

tgggtacatc tattacagtg ggagcaccta ctacaacccg tccctcaaga gtcgagttac     420

catatcagta gacacgtcta agaaccagtt ctccctgaag ctgagctctg tgactgccgc     480

ggacacggcc gtgtattact gtgcgaga                                       508

<210>  281
<211>  483
<212>  DNA
<213>  homo sapiens;

<400>  281
cagctcacat gggaagtgct ttctgagagt catggacctc ctgcacaaga acatgaaaca      60

cctgtggttc ttcctcctcc tggtggcagc tcccagatgt gagtgtctca ggaatgcgga     120

tatgaagata tgagatgctg cctctgatcc cagggctcac tgtgggtttc tctgttcaca     180

ggggtcctgt cccaggtgca gctacagcag tggggcgcag gactgttgaa gccttcggag     240

accctgtccc tcacctgcgc tgtctatggt gggtccttca gtggttacta ctggagctgg     300

atccgccagc ccccagggaa ggggctggag tggattgggg aaatcaatca tagtggaagc     360

accaactaca acccgtccct caagagtcga gtcaccatat cagtagacac gtccaagaac     420

cagttctccc tgaagctgag ctctgtgacc gccgcggaca cggctgtgta ttactgtgcg     480

aga                                                                  483

<210>  282
<211>  508
<212>  DNA
<213>  homo sapiens;

<400>  282
atttccttaa attcaggtcc aactcataag ggaaatgctt tctgagagtc atggatctca      60

tgtgcaagaa aatgaagcac ctgtggttct tcctcctgct ggtggcggct cccagatgtg     120

agtgtttcta ggatgcagac atggagatat gggaggctgc ctctgatccc agggctcact 180

gtgggttttt ctgttcacag gggtcctgtc ccagctgcag ctgcaggagt cgggcccagg 240

actggtgaag ccttcggaga ccctgtccct cacctgcact gtctctggtg gctccatcag 300

cagtagtagt tactactggg gctggatccg ccagccccca gggaaggggc tggagtggat 360

tgggagtatc tattatagtg ggagcaccta ctacaacccg tccctcaaga gtcgagtcac 420

catatccgta gacacgtcca agaaccagtt ctccctgaag ctgagctctg tgaccgccgc 480

agacacggct gtgtattact gtgcgaga 508

<210> 283
<211> 494
<212> DNA
<213> homo sapiens;

<400> 283
aaattcaggg tccagctcac atgggaaata ctttctgaga ctcatggacc tcctgcacaa 60

gaacatgaaa cacctgtggt tcttcctcct gctggtggca gctcccagat gtgagtgtct 120

caaggctgca gacatgggga tatgggaggt gcctctgatc ccagggctca ctgtgggtct 180

ctctgttcac aggggtcctg tcccaggtgc agctgcagga gtcgggccca ggactggtga 240

agccttcgga gaccctgtcc ctcacctgca ctgtctctgg tggctccatc agtagttact 300

actggagctg gatccggcag cccgccggga agggactgga gtggattggg cgtatctata 360

ccagtgggag caccaactac aacccctccc tcaagagtcg agtcaccatg tcagtagaca 420

cgtccaagaa ccagttctcc ctgaagctga gctctgtgac cgccgcggac acggccgtgt 480

attactgtgc gaga 494

<210> 284
<211> 2025
<212> DNA
<213> homo sapiens;

<400> 284
ttttcacctc tccatacaaa ggcaccaccc acatgcaaat cctcacttaa gcacccacag 60

gaaaccacca cacatttcct taaattcagg ttccagctca catgggaaat actttctgag 120

agtcctggac ctcctgtgca agaacatgaa acatctgtgg ttcttccttc tcctggtggc 180

agctcccaga tgtgagtatc tcagggatcc agacatgggg atatgggagg tgcctctgat 240

cccagggctc actgtgggtc tctctgttca caggggtcct gtcccaggtg cagctgcagg 300

agtcgggccc aggactggtg aagccttcgg agaccctgtc cctcacctgc actgtctctg 360

gtggctccat cagtagttac tactggagct ggatccggca gcccccaggg aagggactgg 420

agtggattgg gtatatctat tacagtggga gcaccaacta caacccctcc ctcaagagtc 480

gagtcaccat atcagtagac acgtccaaga accagttctc cctgaagctg agctctgtga 540

ccgctgcgga cacggccgtg tattactgtg cgagagacac agtgagggga ggtgagtgtg 600

agcccagaca aaaacctccg tgcagggagg cggaggggac cggcgcaggt gctgctcagc 660

gccagcaggg ggcgcgcggg gcccacagag caggaggccc ggtcaggagc aggtgcaggg 720

agggcggggc ttcctcatct gctcagtggt ctccctcctc gccagcacct cagctgtccc 780

caggggtcct ctttctttat tatctgtggt tctgcttcct cacattcttg tgccaagaaa 840

gaaatgagga agacaaattt tcgtctgtag ttgaagtttc accaattact aggaactttc 900

ctagaagttc ctgcatggcc cattatagct tacagattaa atatatatca agcttctcat 960

ctcttgattt gtgtcatcaa ctgaattgtg ccctctttga aattcatatg cagaaacctt 1020

aaattcaatt gatgtatatt ggaattttaa tgaaataatt aaggttaaat gtggtcataa 1080

gtgtaagact ctaattcaac agacgtgtcg tctttataag aagaggaaga gacaccagag 1140

acctctcact tttcacgtgc aggcagagaa gaggccatgt ggagacgtaa tgcactagaa 1200

ggtggcccag tgcaagccag gaagaagcct caccaagaac caaccctgcc agaacattga 1260

tcttcaacat tcagactgca gaattttaag aaaatcaata tttgttgttt aagccaccca 1320

ctcctgttgt cttcttatga agatccagac agactaatac cacataactc tgttagcgct 1380

gtcccctgga tgcagaatca gcccgctggg gctgggcaca tctctcagat ttccacataa 1440

agtaggcaaa aaatagtagt tctgatataa aaatttgtca tgtccctgtt ggccaatttc 1500

tgggcaaggt cttttaaaga agccctgggg gctttgtcac aaaagttgcc ttttatcatt 1560

tattaggaca taactgatga acaatgagta ccagttggat ggagactgac cactgaccat 1620

cttctgctgt ctcctaagta tgccacagaa aaccacacca acattactct atgtcttcaa 1680

ctttctaaat ttgcactgat tggtatttaa ggcaggccca gcgttgaata actcctttag 1740

tttttgcttc tctgggaaag gtcttatcta tcctggcctt ggtcttcaag tttcagcaat 1800

tctgggaagc caaggacgcc tctatctcct cctccatgct ctgcaactca cctgagaaca 1860

gctttctcat tggaatgtct tctgtttaag gaataagagt ccctgtttca ggcttgggtg 1920

cctgagtaca cctactggat ccagcccagg attggagaaa ctttccagaa cacatcacct 1980

gagaaatgac cagtcacact gttacacttt cacaatttcc gcttc 2025

<210> 285
<211> 537
<212> DNA
<213> homo sapiens;

<400> 285
acttaagcac ccacaggaaa ccaccacaca tttccttaaa ttcaggttcc agctcacatg 60

ggaaatactt tctgagagtc ctggacctcc tgtgcaagaa catgaaacac ctgtggttct 120

tcctcctcct ggtggcagct cccagatgtg agtgtctcag ggatccagac atgggggtat 180

gggaggtgcc tctgatccca gggctcactg tgggtctctc tgttcacagg ggtcctgtcc 240

caggtgcagc tgcaggagtc gggcccagga ctggtgaagc cttcggagac cctgtccctc 300

acctgcactg tctctggtgg ctccgtcagc agtggtggtt actactggag ctggatccgg 360

cagcccccag ggaagggact ggagtggatt gggtatatct attacagtgg gagcaccaac 420

tacaacccct ccctcaagag tcgagtcacc atatcagtag acacgtccaa gaaccagttc 480

tccctgaagc tgagctctgt gaccgctgcg gacacggccg tgtattactg tgcgaga 537

<210> 286
<211> 493
<212> DNA
<213> homo sapiens;

<400> 286
tgagtctccc tcactgccca gctgggatct cagggcttca ttttctgtcc tccaccatca 60

tggggtcaac cgccatcctc gccctcctcc tggctgttct ccaaggtcag tcctgccgag 120

ggcttgaggt cacagaggag aacgggtgga aaggagcccc tgattcaaat tttgtgtctc 180

ccccacagga gtctgtgccg aggtgcagct ggtgcagtct ggagcagagg tgaaaaagcc 240

cggggagtct ctgaagatct cctgtaaggg ttctggatac agctttacca gctactggat 300

cggctgggtg cgccagatgc ccgggaaagg cctggagtgg atggggatca tctatcctgg 360

tgactctgat accagataca gcccgtcctt ccaaggccag gtcaccatct cagccgacaa 420

gtccatcagc accgcctacc tgcagtggag cagcctgaag gcctcggaca ccgccatgta 480

ttactgtgcg aga 493

<210> 287
<211> 498
<212> DNA
<213> homo sapiens;

<400> 287
gcagagcctg ctgaattctg gctgaccagg gcagtcacca gagctccaga caatgtctgt 60

ctccttcctc atcttcctgc ccgtgctggg cctcccatgg ggtcagtgtc agggagatgc 120

cgtattcaca gcagcattca cagactgagg ggtgtttcac tttgctgttt ccttttgtct 180

ccaggtgtcc tgtcacaggt acagctgcag cagtcaggtc caggactggt gaagccctcg 240

cagaccctct cactcacctg tgccatctcc ggggacagtg tctctagcaa cagtgctgct 300

tggaactgga tcaggcagtc cccatcgaga ggccttgagt ggctgggaag gacatactac 360

aggtccaagt ggtataatga ttatgcagta tctgtgaaaa gtcgaataac catcaaccca 420

gacacatcca agaaccagtt ctccctgcag ctgaactctg tgactcccga ggacacggct 480

gtgtattact gtgcaaga 498

<210>  288
<211>  489
<212>  DNA
<213>  homo sapiens;

<400>  288
acccaacaac aacatccctc cttgggagaa tcccctagag cacagctcct caccatggac      60

tggacctgga gcatcctctt cttggtggca gcagcaacag gtaaggggct ccccagtctc      120

ggggttgagg cagaaaccag gccactcaag tgaggcttta cccacccctg tgtcctctcc      180

acaggtacct actcccaggt gcagctggtg cagtctggcc atgaggtgaa gcagcctggg      240

gcctcagtga aggtctcctg caaggcttct ggttacagtt tcaccaccta tggtatgaat      300

tgggtgccac aggcccctgg acaagggctt gagtggatgg gatggttcaa cacctacact      360

gggaacccaa catatgccca gggcttcaca ggacggtttg tcttctccat ggacacctct      420

gccagcacag catacctgca gatcagcagc ctaaaggctg aggacatggc catgtattac      480

tgtgcgaga                                                              489


<210>  289
<211>  38
<212>  DNA
<213>  homo sapiens;

<400>  289
gtacactttt ggccagggga ccaagctgga gatcaaac                              38


<210>  290
<211>  38
<212>  DNA
<213>  homo sapiens;

<400>  290
attcactttc ggccctggga ccaaagtgga tatcaaac                              38


<210>  291
<211>  37
<212>  DNA
<213>  homo sapiens;

<400>  291
ctcactttcg gcggagggac caaggtggag atcaaac                               37


<210>  292
<211>  38
<212>  DNA
<213>  homo sapiens;

<400>  292
gatcaccttc ggccaaggga cacgactgga gattaaac                              38


<210>  293

<211>  503
<212>  DNA
<213>  homo sapiens;

<400>  293
aggaatcaga cccagtcagg acacagcatg gacatgagag tcctcgctca gctcctgggg      60

ctcctgctgc tctgtttccc aggtaaggat ggagaacact agcagtttac tcagcccagg     120

gtgctcagta ctgctttact attcagggaa attctcttac aacatgatta attgtgtgga     180

catttgtttt tatgtttcca atctcaggtg ccagatgtga catccagatg acccagtctc     240

catcctcact gtctgcatct gtaggagaca gagtcaccat cacttgtcgg gcgagtcagg     300

gcattagcaa ttatttagcc tggtttcagc agaaaccagg gaaagcccct aagtccctga     360

tctatgctgc atccagtttg caaagtgggg tcccatcaaa gttcagcggc agtggatctg     420

ggacagattt cactctcacc atcagcagcc tgcagcctga agattttgca acttattact     480

gccaacagta taatagttac cct                                             503

<210>  294
<211>  503
<212>  DNA
<213>  homo sapiens;

<400>  294
aggaatcagt cccactcagg acacagcatg gacatgaggg tccccgctca gctcctgggg      60

ctcctgctgc tctggttccc aggtaaggat ggagaacact agcagtttac tcagcccaga     120

gtgctcagta ctgctttact gttcagggaa attctcttac aacatgatta attgtgtgga     180

catttgtttt tatgtttcca atctcaggtg ccaggtgtga catccagatg acccagtctc     240

catcctccct gtctgcatct gtaggagaca gagtcaccat cacttgccgg gcaagtcagg     300

gcattagaaa tgatttaggc tggtatcagc agaaaccagg gaaagcccct aagcgcctga     360

tctatgctgc atccagtttg caaagtgggg tcccatcaag gttcagcggc agtggatctg     420

ggacagaatt cactctcaca atcagcagcc tgcagcctga agattttgca acttattact     480

gtctacagca taatagttac cct                                             503

<210>  295
<211>  657
<212>  DNA
<213>  homo sapiens;

<400>  295
gggacacctg gggacactga gctggtgctg agttactgag atgagccagc tctgcagctg      60

tgcccagcct gccccatccc ctgctcattt gcatgttccc agagcacaac ctcctgccct     120

gaagccttat taataggctg gtcacacttt gtgcaggagt cagacccagt caggacacag     180

catggacatg agggtccccg ctcagctcct ggggctcctg ctgctctggc tcccaggtaa     240

ggaaggagaa cactaggaat ttactcagcc cagtgtgctc agtactgcct ggttattcag 300

ggaagtcttc ctataatatg atcaatagta tgaatatttg tgtttctatt tccaatctca 360

ggtgccaaat gtgacatcca gatgacccag tctccttcca ccctgtctgc atctgtagga 420

gacagagtca ccatcacttg ccgggccagt cagagtatta gtagctggtt ggcctggtat 480

cagcagaaac cagggaaagc ccctaagctc ctgatctata aggcgtctag tttagaaagt 540

ggggtcccat caaggttcag cggcagtgga tctgggacag aattcactct caccatcagc 600

agcctgcagc ctgatgattt tgcaacttat tactgccaac agtataatag ttattct 657

<210> 296
<211> 506
<212> DNA
<213> homo sapiens;

<400> 296
gcaggagtca gacccactca ggacacagca tggacatgag ggtccccgct cagctcctgg 60

ggctcctgct gctctggctc ccaggtaagg atggagaaca ctggcagttt actcagccca 120

gggtgctcag cacagcctgg ctattcaggg aaattctctt actacatgat taattgtgtg 180

gaccatttgt ttttgtgttt ccaatctcag gtgccagatg tgccatccag atgacccagt 240

ctccatcctc cctgtctgca tctgtaggag acagagtcac catcacttgc cgggcaagtc 300

agggcattag aaatgattta ggctggtatc agcagaaacc agggaaagcc cctaagctcc 360

tgatctatgc tgcatccagt ttacaaagtg gggtcccatc aaggttcagc ggcagtggat 420

ctggcacaga tttcactctc accatcagca gcctgcagcc tgaagatttt gcaacttatt 480

actgtctaca agattacaat taccct 506

<210> 297
<211> 523
<212> DNA
<213> homo sapiens;

<400> 297
aggctggaca cacttcatgc aggagtcaga ccctgtcagg acacagcata gacatgaggg 60

tccccgctca gctcctgggg ctcctgctgc tctggctccc aggtaaggaa ggagaacact 120

aggaatttac tcagcccagt gtgcttggta cagcctggcc cttcagggaa gttctcttac 180

aacatgatta attgtatgga catttgtttt tatgtttcca atctcaggtg ccagatgtgc 240

catccggatg acccagtctc catcctcatt ctctgcatct acaggagaca gagtcaccat 300

cacttgtcgg gcgagtcagg gtattagcag ttatttagcc tggtatcagc aaaaaccagg 360

gaaagcccct aagctcctga tctatgctgc atccactttg caaagtgggg tcccatcaag 420

gttcagcggc agtggatctg ggacagattt cactctcacc atcagctgcc tgcagtctga 480

agattttgca acttattact gtcaacagta ttatagttac cct 523

<210>  298
<211>  534
<212>  DNA
<213>  homo sapiens;

<400>  298
agacttctta ataggctggt cacacctgtg caggagtcag tcccagtcag gacacagcat      60

ggacatgagg gtccccgctc agctcctggg gctcctgctg ctctggctcc caggtaagga     120

aggagaacac taggaattta ctcagcccag tgtgttccgt acagcctggc tcttgaggga     180

agttctctta caacatgatt aattctatgg acatttgtgt ttatatttcc aatctcaggt     240

gccagatgtg acatccagtt gacccagtct ccatccttcc tgtctgcatc tgtaggagac     300

agagtcacca tcacttgccg ggccagtcag ggcattagca gttatttagc ctggtatcag     360

caaaaaccag ggaaagcccc taagctcctg atctatgctg catccacttt gcaaagtggg     420

gtcccatcaa ggttcagcgg cagtggatct gggacagaat tcactctcac aatcagcagc     480

ctgcagcctg aagattttgc aacttattac tgtcaacagc ttaatagtta ccct           534


<210>  299
<211>  656
<212>  DNA
<213>  homo sapiens;

<400>  299
gggacacctg gggacactga gctggtgctg agttactgag atgagccagc cctgcagctg      60

cgcccagcct gccccatccc ctgctcattt gcatgttccc agagcacagt ctcctgacct     120

gaagacttat taacaggctg atcacaccct gtgcaggagt cagacccagt caggacacag     180

catggacatg agggtccccg ctcagctcct ggggctcctg ctgctctggt tcccaggtaa     240

gaaaggagaa cactaggatt atactcggtc agtgtgctga gtactgcttt actattcagg     300

gaacttctct tacagcatga ttaattgtgt ggacatttgt ttttatgttt ccaatctcag     360

gttccagatg cgacatccag atgacccagt ctccatcttc tgtgtctgca tctgtaggag     420

acagagtcac catcacttgt cgggcgagtc agggtattag cagctggtta gcctggtatc     480

agcagaaacc agggaaagcc cctaagctcc tgatctatgc tgcatccagt ttgcaaagtg     540

gggtcccatc aaggttcagc ggcagtggat ctgggacaga tttcactctc actatcagca     600

gcctgcagcc tgaagatttt gcaacttact attgtcaaca ggctaacagt ttccct         656


<210>  300
<211>  503
<212>  DNA
<213>  homo sapiens;

<400>  300
aggaatcaga cccagtcagg acacagcatg gacatgaggg tcctcgctca gctcctgggg      60

ctcctgctgc tctgtttccc aggtaaggat ggagaacact agcagtttac tcagcccagg 120

gtgctcagta ctgctttact attcagggaa attctcttac aacatgatta attgtgtgga 180

catttgtttt tatgtttcca atctcaggtg ccagatgtga catccagatg acccagtctc 240

catcctcact gtctgcatct gtaggagaca gagtcaccat cacttgtcgg gcgagtcagg 300

gtattagcag ctggttagcc tggtatcagc agaaaccaga gaaagcccct aagtccctga 360

tctatgctgc atccagtttg caaagtgggg tcccatcaag gttcagcggc agtggatctg 420

ggacagattt cactctcacc atcagcagcc tgcagcctga agattttgca acttattact 480

gccaacagta taatagttac cct 503

<210> 301
<211> 657
<212> DNA
<213> homo sapiens;

<400> 301
gggacacctg gggacactga gctgctgctg agttactgag atgagccagc cctgcagctg 60

cgcccagcct gccccatccc ctgctcattt gcatgttccc agagcatagc ctcctgccct 120

gaagccttat taataggctg gacacacttc atggaggaat cagtcccact caggacacag 180

catggacatg agggtccctg ctcagctcct ggggctcctg ctgctctggt tcccaggtaa 240

ggatggagaa cactaacagt ttactcagcc cagagtgctc agtactgctt tactgttcag 300

ggaaattctc ttacaacatg attaattgtg tggacatttg tttttatgtt tccaatctca 360

ggtgccagat gtaacatcca gatgacccag tctccatctg ccatgtctgc atctgtagga 420

gacagagtca ccatcacttg tcgggcgagg cagggcatta gcaattattt agcctggttt 480

cagcagaaac cagggaaagt ccctaagcac ctgatctatg ctgcatccag tttgcaaagt 540

ggggtcccat caaggttcag cggcagtgga tctgggacag aattcactct cacaatcagc 600

agcctgcagc ctgaagattt tgcaacttat tactgtctac agcataatag ttaccct 657

<210> 302
<211> 487
<212> DNA
<213> homo sapiens;

<400> 302
agtcccagtc aggacacagc atggacatga gggtccccgc tcagctcctg gggctcctgc 60

tgctctggct cccaggtaag gaaggagaac actaggaatt ttcttagccc actgtgctct 120

ggcacttctg ggaagttctc ttataccatg attcatggtg tggatatttg tttttatgtt 180

tccaatctca ggtgtcagat ttgacatcca gatgatccag tctccatctt tcctgtctgc 240

atctgtagga gacagagtca gtatcatttg ctgggcaagt gagggcatta gcagtaattt 300

agcctggtat ctgcagaaac cagggaaatc ccctaagctc ttcctctatg atgcaaaaga 360

tttgcaccct ggggtctcat cgaggttcag tggcagggga tctgggacgg atttcactct 420

caccatcatc agcctgaagc ctgaagattt tgcagcttat tactgtaaac aggacttcag 480

ttaccct 487

<210> 303
<211> 657
<212> DNA
<213> homo sapiens;

<400> 303
gggacacctg gggacactga gctggtgctg agttactgag atgaaccagc cctgcagctg 60

tgcccagcct gccttgcccc ctgctaattt gcatgttccc agagcacatc ctcctaccct 120

gaagacttat taatgcgctg gtcacacttc atgcaggagt cagacccagt caggacacag 180

catggacatg agggtgcccg ctcagcgcct ggggctcctg ctgctctggt tcccaggtaa 240

ggaaggagaa ccctagcagt ttactcagcc cagtgtgttc cgtacagcct ggctcttgag 300

ggaagttctc ttacaacatg attaattgta tggacatttg tgtttatatt tccaatctca 360

ggtgccagat gtgccatccg gatgacccag tctccattct ccctgtctgc atctgtagga 420

gacagagtca ccatcacttg ctgggccagt cagggcatta gcagttattt agcctggtat 480

cagcaaaaac cagcaaaagc ccctaagctc ttcatctatt atgcatccag tttgcaaagt 540

ggggtcccat caaggttcag cggcagtgga tctgggacgg attacactct caccatcagc 600

agcctgcagc ctgaagattt tgcaacttat tactgtcaac agtattatag taccccct 657

<210> 304
<211> 656
<212> DNA
<213> homo sapiens;

<400> 304
gggacacctg gggacactga gctggtgctg agttactgag atgagccagc tctgcagctg 60

tgcccagtca gccccatccc ctgctcattt gcatgttccc agagcacaac ctcctgcact 120

gaagccttat taataggctg gccacacttc atgcaggagt cagacccagt caggacacag 180

catggacatg agggtccccg ctcagctcct ggggctcctg ctgctctggc tcccaggtaa 240

ggaaggagaa cactatgaat ttactcagcc aatgtgctca gtacagcctg gcccttcagg 300

gaaattctct tactacatga ttaattgtat ggatatttgt ttttatgttt ccaatctcag 360

gtgccagatg tgtcatctgg atgacccagt ctccatcctt actctctgca tctacaggag 420

acagagtcac catcagttgt cggatgagtc agggcattag cagttattta gcctggtatc 480

agcaaaaacc agggaaagcc cctgagctcc tgatctatgc tgcatccact ttgcaaagtg 540

gggtcccatc aaggttcagt ggcagtggat ctgggacaga tttcactctc accatcagtt 600

gcctgcagtc tgaagatttt gcaacttatt actgtcaaca gtattatagt ttccct 656

<210> 305
<211> 833
<212> DNA
<213> homo sapiens;

<400> 305
aattaggact cctcaggtca ccttctcaca atgaggctcc ttgctcagct tctggggctg 60

ctaatgctct gggtccctgg tgaggacaga agagagatga gggaggagaa tggggtggga 120

gggtgaactc tgggggcccc attgcctccc atgtgtgttc tgtcctcatg ttagatgtgt 180

acgtcttgta ctccaggatg gggcttgtaa cttttatatc tgcgtgagta aggcatgtga 240

ggtttagatc tgtaagaatg aggaagattc cagaaggaac aaagaccagt gctccggtga 300

agactctaac agagaaagag ggaatggtag aggaaacttc tagcactcaa agcactctgc 360

tgtgctttga aaatatgttt ttattttgaa attatatatt actagggtct gaatcaaatt 420

ataaaaattg atttagcctg aaataaataa cagaagaaaa attattttaa aattgtgctt 480

aaagtttcta cataaccttg cacttctctc tcattatttc aggatccagt ggggatattg 540

tgatgaccca gactccactc tcctcacctg tcacccttgg acagccggcc tccatctcct 600

gcaggtctag tcaaagcctc gtacacagtg atggaaacac ctacttgagt tggcttcagc 660

agaggccagg ccagcctcca agactcctaa tttataagat ttctaaccgg ttctctgggg 720

tcccagacag attcagtggc agtggggcag ggacagattt cacactgaaa atcagcaggg 780

tggaagctga ggatgtcggg gtttattact gcatgcaagc tacacaattt cct 833

<210> 306
<211> 816
<212> DNA
<213> homo sapiens;

<400> 306
gatcaggact cctcagttca ccttctcaca atgaggctcc ctgctcagct cctggggctg 60

ctaatgctct gggtcccagg taagggtaga agggagatga gggaggagaa tggcatggaa 120

cggtgagttc tggggcccca ctgcctctaa caacagtgat ctctgggggt ctcactacac 180

tcctatgtgt gttcctttcc tgtattggac atgcacatgt tgtcctccag agtggggcat 240

gtgatgatca gatctgtgag agtgaggaag attcaagcag aaacaaggat ctgtgctctg 300

gggaagactg acacagaaag gggatggtgt ggggtcttct ggagacccct ttgagccttg 360

gatcccttga gttccatttt gaaactgtgt atttttgaaa tatgaacaaa tacatatata 420

gcctgaaata aacaacaaat caaaatttat gaaaattaca cataaacttt atacataacc 480

ttgctcttct ttctatttat ttcaggatcc agtggggatg ttgtgatgac tcagtctcca 540

ctctccctgc ccgtcaccct tggacagccg gcctccatct cctgcaggtc tagtcaaagc 600

ctcgtataca gtgatggaaa cacctacttg aattggtttc agcagaggcc aggccaatct 660

ccaaggcgcc taatttataa ggtttctaac cgggactctg gggtcccaga cagattcagc 720

ggcagtgggt caggcactga tttcacactg aaaatcagca gggtggaggc tgaggatgtt 780

ggggtttatt actgcatgca aggtacacac tggcct 816

<210> 307
<211> 833
<212> DNA
<213> homo sapiens;

<400> 307
aattaggact cctcaggtca ccttctcaca atgaggctcc ttgctcagct tctggggctg 60

ctaatgctct gggtccctgg tgaggacaga agagagatga gggaggagaa tggggtggga 120

gggtgaactc tgggggcccc attgcctccc atgtgtgttc tgtcctcatg ttagatgtgt 180

acgtcttgta ctccaggatg gggcttgtaa cttttatatc tgcgtgagta aggcatgtga 240

ggtttagatc tgtaagaatg aggaagattc cagaaggaac aaagaccagt gctccggtga 300

agactctaac agagaaagag ggaatggtag aggaaacttc tagcactcaa agcactctgc 360

tgtgctttga aaatatgttt ttattttgaa attatatatt actagggtct gaatcaaatt 420

ataaaaattg atttagcctg aaataaataa cagaagaaaa attattttaa aattgtgctt 480

aaagtttcta cataaccttg cacttctctc tcattatttc aggatccagt ggggatattg 540

tgatgaccca gactccactc tcctcgcctg tcacccttgg acagccggcc tccatctcct 600

tcaggtctag tcaaagcctc gtacacagtg atggaaacac ctacttgagt tggcttcagc 660

agaggccagg ccagcctcca agactcctaa tttataaggt ttctaaccgg ttctctgggg 720

tcccagacag attcagtggc agtggggcag ggacagattt cacactgaaa atcagcaggg 780

tggaagctga ggatgtcggg gtttattact gcacgcaagc tacacaattt cct 833

<210> 308
<211> 781
<212> DNA
<213> homo sapiens;

<400> 308
gatcaggact cctcagttca ccttctcact atgaggctcc ctgctcagct cttggggctg 60

ctaatgctct gggtccctgg taaggacaga aggagatgag ggaggagaat ggggtgggaa 120

ggtaagcctg gggaccccac tgccttccat gtgtgttctg ccctgcccat gtgttagatg 180

tacaggtctt gttctccagg atggggaatg tgaggtttaa atctgtgaga gtgaggacga 240

ttcaaaaaga agcaaggacc tgtgtgctct ggtgaatatc gtcacacaga gaaagggagg 300

tggtgtaggt gacttctaga atcccctttg cagcttgcaa atttggaata tgtttagtgt 360

ataaatacaa acaacaaaaa attatatagc ctgaaataaa aaatgaaaat ttatgataaa 420

tgacacatga tatttgtaca tatccttcca cttctttcta tctattttag gatccagtgc 480

agagattgtg atgacccaga ctccactctc cttgtctatc acccctggag agcaggcctc 540

catgtcctgc aggtctagtc agagcctcct gcatagtgat ggatacacct atttgtattg 600

gtttctgcag aaagccaggc cagtctccac gctcctgatc tatgaagttt ccaaccggtt 660

ctctggagtg ccagataggt tcagtggcag cgggtcaggg acagatttca cactgaaaat 720

cagccgggtg gaggctgagg attttggagt ttattactgc atgcaagatg cacaagatcc 780

t 781

<210> 309
<211> 758
<212> DNA
<213> homo sapiens;

<400> 309
gatcaggact tctcagttca tcttctcacc atgaggctcc ctgctcagct cctggggctg 60

ctaatgctct ggatacctgg taaggatgga aggagatgag ggaggaggag ggggtgggaa 120

gctgagctct ggcggcccca ctgattcccg tgtttattct aaccatgtgt taaaggaata 180

tggcctatgc tccagggaga ggaattcata ttttgccctg atgatgattt gaaaactcct 240

aaaagcagtg ctctgaataa tatcttgaga aatgaaagaa ctcttgtgcc tatttaataa 300

agggttcatt taaagagttt gtttttatga tatgaataca aatttgtaaa aataaaagat 360

tagccataaa tcaataccat aaggcaaatc tcaaaagttg ttcattatgc tttcacataa 420

ccttgcactt ctctctcata atttcaggat ccagtgcaga tattgtgatg acccagactc 480

cactctctct gtccgtcacc cctggacagc cggcctccat ctcctgcaag tctagtcaga 540

gcctcctgca tagtgatgga aagacctatt tgtattggta cctgcagaag ccaggccagc 600

ctccacagct cctgatctat gaagtttcca accggttctc tggagtgcca gataggttca 660

gtggcagcgg gtcagggaca gatttcacac tgaaaatcag ccgggtggag gctgaggatg 720

ttggggttta ttactgcatg caaagtatac agcttcct 758

<210> 310
<211> 821
<212> DNA
<213> homo sapiens;

<400> 310
tgactgatca ggactcctca gttcaccttc tcacaatgag gctccctgct cagctcctgg 60

ggctgctaat gctctgggtc ccaggtaagg gtagaaggga gatgagggag gagaatggca 120

tggaacggtg agttctgggg ccccactgcc tctaacaaca gtgatctctg ggggtctcac 180

tacactccta tgtgtgttcc tttcctgtat tggacatgca catgttgtcc tccagaatgg 240

ggcatgtgat gatcagatct gtgagagtca ggaagattca agaagaaaca aggatctgtg 300

ctctggggaa gactgacaca gaaaggggat ggtgtggggt cttctggaga cccctttgag 360

ccttggatcc cttgagttcc attttgaaac tgtatatttt tgaaatatga acaaatacat 420

atatagcctg agataaacaa caaatcaaaa tttatgaaaa ttacacataa actttataca 480

taaccttgct cttctttcta tttatttcag gatccagtgg ggatgttgtg atgactcagt 540

ctccactctc cctgcccgtc acccttggac agccggcctc catctcctgc aggtctagtc 600

aaagcctcgt atacagtgat ggaaacacct acttgaattg gtttcagcag aggccaggcc 660

aatctccaag gcgcctaatt tataaggttt ctaactggga ctctggggtc ccagacagat 720

tcagcggcag tgggtcaggc actgatttca cactgaaaat cagcagggtg gaggctgagg 780

atgttggggt ttattactgc atgcaaggta cacactggcc t 821

<210> 311
<211> 561
<212> DNA
<213> homo sapiens;

<400> 311
gtcagagccc tggggaggaa ctgctcagtt aggacccaga gggaaccatg gaagccccag 60

ctcagcttct cttcctcctg ctactctggc tcccaggtga ggggaacatg aggtggtttt 120

gcacattagt gaaaactctt gccacctctg ctcagcaaga aatataatta aaattcaaag 180

tatatcaaca attttggctc tactcaaaga cagttggttt gatcttgatt acatgagtgc 240

atttctgttt tatttccaat ttcagatacc accggagaaa ttgtgttgac acagtctcca 300

gccaccctgt ctttgtctcc aggggaaaga gccaccctct cctgcagggc cagtcagagt 360

gttagcagct acttagcctg gtaccaacag aaacctggcc aggctcccag gctcctcatc 420

tatgatgcat ccaacagggc cactggcatc ccagccaggt tcagtggcag tgggtctggg 480

acagacttca ctctcaccat cagcagccta gagcctgaag attttgcagt ttattactgt 540

cagcagcgta gcaactggcc t 561

<210> 312
<211> 587
<212> DNA
<213> homo sapiens;

<400> 312
cctgggtcag agctctggag aagagctgct cagttaggac ccagagggaa ccatggaaac 60

cccagcgcag cttctcttcc tcctgctact ctggctccca ggtgagggga acatgggatg 120

gttttgcatg tcagtgaaaa ccctctcaag tcctgttacc tggcaactct gctcagtcaa 180

tacaataatt aaagctcaat ataaagcaat aattctggct cttctgggaa gacaatgggt 240

ttgatttaga ttacatgggt gacttttctg ttttatttcc aatctcagat accaccggag 300

aaattgtgtt gacgcagtct ccaggcaccc tgtctttgtc tccaggggaa agagccaccc 360

tctcctgcag ggccagtcag agtgttagca gcagctactt agcctggtac cagcagaaac 420

ctggccaggc tcccaggctc ctcatctatg gtgcatccag cagggccact ggcatcccag 480

acaggttcag tggcagtggg tctgggacag acttcactct caccatcagc agactggagc 540

ctgaagattt tgcagtgtat tactgtcagc agtatggtag ctcacct 587

<210> 313
<211> 614
<212> DNA
<213> homo sapiens;

<400> 313
gcatgtccct cccagctgcc ctaccttcca gagcccatat caatgcctgg gtcagagccc 60

tgggaaggaa ctgctcagtt aggacccaga cggaaccatg gaagccccag ctcagcttct 120

cttcctcctg ctactctggc tcccaggtga ggggaacatg aggtggtttt gcacatcagt 180

gaaaactcct gccacctctg ctcagcaaga aatataatta aaattcaatg tagatcaaca 240

attttggctc tactcaaaga cagctggttt gatctagatt acatgagtgc atttctgttt 300

tatttccaat cttggatacc accagagaaa ttgtaatgac acagtctcca cccaccctgt 360

ctttgtctcc aggggaaaga gtcaccctct cctgcagggc cagtcagagt gttagcagca 420

gctacttaac ctggtatcag cagaaacctg gccaggcgcc caggctcctc atctatggtg 480

catccaccag ggccactagc atcccagcca ggttcagtgg cagtgggtct gggacagact 540

tcactctcac catcagcagc ctgcagcctg aagattttgc agtttattac tgtcagcagg 600

attataactt acct 614

<210> 314
<211> 611
<212> DNA
<213> homo sapiens;

<400> 314
gcatgtccct cccagctgcc ctaccttcca gagcccatat caatgcctgg gtcagagctc 60

tggggaggaa ctgctcagtt aggacccaga cggaaccatg gaagccccag cgcagcttct 120

cttcctcctg ctactctggc tcccaggtga ggggaatatg aggtgtcttt gcacatcagt 180

gaaaactcct gccacctctg ctcagcaaga aatataatta aaattcaaaa tagatcaaca 240

attttggctc tactcaaaga cagtgggttt gattttgatt acatgagtgc atttctgttt 300

tatttccaat ttcagatacc accggagaaa ttgtgttgac acagtctcca gccaccctgt 360

ctttgtctcc aggggaaaga gccaccctct cctgcagggc cagtcagggt gttagcagct 420

acttagcctg gtaccagcag aaacctggcc aggctcccag gctcctcatc tatgatgcat 480

ccaacagggc cactggcatc ccagccaggt tcagtggcag tgggcctggg acagacttca 540

ctctcaccat cagcagccta gagcctgaag attttgcagt ttattactgt cagcagcgta 600

gcaactggca t 611

<210> 315
<211> 563
<212> DNA
<213> homo sapiens;

<400> 315
gggtcagagc tctggggagg aactgctcag ttaggaccca gacggaacca tggaagcccc 60

agcgcagctt ctcttcctcc tgctactctg gctcccaggt gaggggaata tgaggtggtt 120

ttgcacatca gtgaaaactc ctgccacctc tgctcagcaa gaaatataat taaaattcaa 180

tgtagatcaa caattttggc tctacttaaa gacagtgggt ttgattttga ttacatgagt 240

gcatttctgt tttatttcca atttcagata ccactggaga aatagtgatg acgcagtctc 300

cagccaccct gtctgtgtct ccaggggaaa gagccaccct ctcctgcagg gccagtcaga 360

gtgttagcag caacttagcc tggtaccagc agaaacctgg ccaggctccc aggctcctca 420

tctatggtgc atccaccagg gccactggca tcccagccag gttcagtggc agtgggtctg 480

ggacagagtt cactctcacc atcagcagcc tgcagtctga agattttgca gtttattact 540

gtcagcagta taataactgg cct 563

<210> 316
<211> 632
<212> DNA
<213> homo sapiens;

<400> 316
gcatgtccct cccagccgcc ctgcagtcca gagcccatat caatgcctgg gtcagagctc 60

tggggaggaa ctgctcagtt aggacccaga gggaaccatg gaaaccccag cgcagcttct 120

cttcctcctg ctactctggc tcccaggtga ggggaacatg ggatggtttt gcatgtcagt 180

gaaaaccctc tcaagtcctg ttacctggca actctgctga atcaatacaa taattaaagc 240

tcaatataaa gcaataattc tggctcttct gggaagacag tgggtttgat ttagattaca 300

tgggtgactt ttctatttta tttccaatct cagataccac cggagaaatt gtgttgacgc 360

agtctccagc caccctgtct ttgtctccag gggaaagagc caccctctcc tgcgggccca 420

gtcagagtgt tagcagcagc tacttagcct ggtaccagca gaaacctggc ctggcgccca 480

ggctcctcat ctatgatgca tccagcaggg ccactggcat cccagacagg ttcagtggca 540

gtgggtctgg gacagacttc actctcacca tcagcagact ggagcctgaa gattttgcag 600

tgtattactg tcagcagtat ggtagctcac ct 632

<210>  317
<211>  757
<212>  DNA
<213>  homo sapiens;

<400>  317
tttggctctt gatttacatt gggtactttc acaacccact gctcatgaaa tttgcttttg      60

tactcactgg ttgtttttgc ataggcccct ccaggccacg accagctgtt tggattttat     120

aaacgggccg tttgcattgt gaactgagct acaacaggca ggcaggggca gcaagatggt     180

gttgcagacc caggtcttca tttctctgtt gctctggatc tctggtgagg aattaaaaag     240

tgccacagtc ttttcagagt aatatctgtg tagaaataaa aaaaattaag atatagttgg     300

aaataatgac tatttccaat atggatccaa ttatctgctg acttataata ctactagaaa     360

gcaaatttaa atgacatatt tcaattatat ctgagacagc gtgtataagt ttatgtataa     420

tcattgtcca ttactgacta caggtgccta cggggacatc gtgatgaccc agtctccaga     480

ctccctggct gtgtctctgg gcgagagggc caccatcaac tgcaagtcca gccagagtgt     540

tttatacagc tccaacaata agaactactt agcttggtac cagcagaaac caggacagcc     600

tcctaagctg ctcatttact gggcatctac ccgggaatcc ggggtccctg accgattcag     660

tggcagcggg tctgggacag atttcactct caccatcagc agcctgcagg ctgaagatgt     720

ggcagtttat tactgtcagc aatattatag tactcct                              757


<210>  318
<211>  553
<212>  DNA
<213>  homo sapiens;

<400>  318
ataaaatctg tgctgtcaaa ctgattagga actgactacc acctgcaggt cagggccaag      60

gttatggggt cccaggttca cctcctcagc ttcctcctcc tttggatctc tggtaagaga     120

aacacttcct ctcctctgtg ccaccaagtc ccctgcatat ccacaaaaat aatatatttt     180

cataaggaat tgattttcct cattctctgc aaatatgatg catttgattt atgtttttta     240

ctttgctcca taatcagata ccagggcaga aacgacactc acgcagtctc cagcattcat     300

gtcagcgact ccaggagaca aagtcaacat ctcctgcaaa gccagccaag acattgatga     360

tgatatgaac tggtaccaac agaaaccagg agaagctgct attttcatta ttcaagaagc     420

tactactctc gttcctggaa tcccacctcg attcagtggc agcgggtatg gaacagattt     480

taccctcaca attaataaca tagaatctga ggatgctgca tattacttct gtctacaaca     540

tgataatttc cct                                                        553


<210>  319

<211>  616
<212>  DNA
<213>  homo sapiens;

<400>  319
atcttaaaag aggttctttc tctgggatgt ggcatgagca aaactgacaa gtcaaggcag      60

gaagatgttg ccatcacaac tcattgggtt tctgctgctc tgggttccag gtgagaatat     120

ttccacaaac ctaggcggag atattctttc aatctgtaat ttctttcatt ggggactctg     180

caataggtga tttttggctt gattttaaaa tcctaatttt aaaaatgtaa tgcatattct     240

ttcttcatgt ctagcaagat taaaggtgat tttcatacac agatatttat gttgtactga     300

tgtttgctgt atattttcag cctccagggg tgaaattgtg ctgactcagt ctccagactt     360

tcagtctgtg actccaaagg agaaagtcac catcacctgc cgggccagtc agagcattgg     420

tagtagctta cactggtacc agcagaaacc agatcagtct ccaaagctcc tcatcaagta     480

tgcttcccag tccttctcag gggtcccctc gaggttcagt ggcagtggat ctgggacaga     540

tttcaccctc accatcaata gcctggaagc tgaagatgct gcaacgtatt actgtcatca     600

gagtagtagt ttacct                                                     616


<210>  320
<211>  619
<212>  DNA
<213>  homo sapiens;

<400>  320
ggtatcttaa aagaggttct ttctctggga tgtggcatga gcaaaactga caagtcaagg      60

caggaagatg tcgccatcac aactcattgg gtttctgctg ctctgggttc caggtgagaa     120

tatttccaca aacctaggcg gagatattct ttcaatctgt aatttctttc attggggact     180

ctgcaatagg tgatttttgg cttgatttta aaatcctaat tttaaaaatg taatgcatat     240

tctttcttca tgtctagcaa gattaaaggt gattttcata cacagatatt tatgttgtac     300

tgatgtttgc tgtatatttt cagcctccag gggtgaaatt gtgctgactc agtctccaga     360

ctttcagtct gtgactccaa aggagaaagt caccatcacc tgccgggcca gtcagagcat     420

tggtagtagc ttacactggt accagcagaa accagatcag tctccaaagc tcctcatcaa     480

gtatgcttcc cagtccatct caggggtccc ctcgaggttc agtggcagtg gatctgggac     540

agatttcacc ctcaccatca atagcctgga agctgaagat gctgcagcgt attactgtca     600

tcagagtagt agtttacct                                                  619


<210>  321
<211>  577
<212>  DNA
<213>  homo sapiens;

<400>  321

agcaaaactg aagtcaaaac actgagatgg tgtccccgtt gcaattcctg cggcttctgc 60

tcctctgggt tccaggtgag aatatttaga aaaagctaaa actaattctt tgaaccatta 120

attttcttaa ttaggaacct ggcaccatat ggaacttggc ttgtttttaa atgtgatttt 180

tttttaagta atgcgtattc tttcatcttg tgctactaga ttagtggtga tttcattaag 240

cagatgctta tattgtgcta atgtttgctg tatggtttca gcctccaggg gtgatgttgt 300

gatgacacag tctccagctt tcctctctgt gactccaggg gagaaagtca ccatcacctg 360

ccaggccagt gaaggcattg gcaactactt atactggtac cagcagaaac cagatcaagc 420

cccaaagctc ctcatcaagt atgcttccca gtccatctca ggggtcccct cgaggttcag 480

tggcagtgga tctgggacag atttcacctt taccatcagt agcctggaag ctgaagatgc 540

tgcaacatat tactgtcagc agggcaataa gcaccct 577

<210> 322
<211> 127
<212> DNA
<213> homo sapiens;

<400> 322
cccagcaggc tcctgctcca gcccagcccc cagagagcag accccaggtg ctggccccgg 60

gggttttggt ctgagcctca gtcactgtgt tatgtcttcg gaactgggac caaggtcacc 120

gtcctag 127

<210> 323
<211> 175
<212> DNA
<213> homo sapiens;

<400> 323
gtgtgggggc catgtggact ccctcatgag cagatgccac cagggccact ggccccagct 60

tcctccttca cagctgcagt gggggctggg gctggggcat cccagggagg gtttttgtat 120

gagcctgtgt cacagtgtgt ggtattcggc ggagggacca agctgaccgt cctag 175

<210> 324
<211> 176
<212> DNA
<213> homo sapiens;

<400> 324
gtgtgggggc catgtggact ccctcatgag cagatgccac caggaccact ggccccagct 60

tcctccttca cagctgcagt gggggctggg gctaggggca tcccagggag ggtttttgta 120

tgagcctgtg tcacagtgtt gggtgttcgg cggagggacc aagctgaccg tcctag 176

<210> 325
<211> 72
<212> DNA

<213>  homo sapiens;

<400>  325
cagagagggt ttttgtatga gcctgtgtca cagcactggg tgtttggtga ggggacggag      60

ctgaccgtcc ta                                                          72


<210>  326
<211>  70
<212>  DNA
<213>  homo sapiens;

<400>  326
ggagggtttg tgtgcagggt tatatcacag tgtaatgtgt tcggcagtgg caccaaggtg      60

accgtcctcg                                                             70


<210>  327
<211>  46
<212>  DNA
<213>  homo sapiens;

<400>  327
tcactgtgtg ctgtgttcgg aggaggcacc cagctgaccg ccctcg                     46


<210>  328
<211>  477
<212>  DNA
<213>  homo sapiens;

<400>  328
gggaatctgc accatgccct gggctctgct cctcctgacc ctcctcactc actctgcagg      60

tgagagtgga ccttacccag ggatctgcac ccacctctgc tccagcttct ccactccctg     120

gctcagtgga ctctgatcct gctctcacat tcctttctgt cccctctaca gtgtcagtgg     180

tccaggcagg gctgactcag ccaccctcgg tgtccaaggg cttgagacag accgccacac     240

tcacctgcac tgggaacagc aacattgttg gcaaccaagg agcagcttgg ctgcagcagc     300

accagggcca ccctcccaaa ctcctatcct acaggaataa caaccggccc tcagggatct     360

cagagagatt ctctgcatcc aggtcaggaa acacagcctc cctgaccatt actggactcc     420

agcctgagga cgaggctgac tattactgct cagcattgga cagcagcctc agtgctc        477


<210>  329
<211>  544
<212>  DNA
<213>  homo sapiens;

<400>  329
gctgtgtcca ctatggccct gactcctctc ctcctcctgc tcctctctca ctgcacaggt      60

agggacaggg ctcagagccc agggtggtcc ccagcctgat ctgtccctca tggctcagat     120

ccctcagcag ctgcgccctg accctgctcc tcactgtgct gtgtctgtgt ctgcaggttc     180

cctctcccgg cccgtgctga ctcagccgcc ctctctgtct gcatccccgg gagcaacagc 240

cagactcccc tgcaccctga gcagtgacct cagtgttggt ggtaaaaaca tgttctggta 300

ccagcagaag ccagggagct ctcccaggtt attcctgtat cactactcag actcagacaa 360

gcagctggga cctggggtcc ccagtcgagt ctctggctcc aaggagacct caagtaacac 420

agcgtttttg ctcatctctg ggctccagcc tgaggacgag gccgattatt actgccaggt 480

gtacgaaagt agtgctaatc acagtgagac agatgaggaa gtcggacaaa aaccaaggtt 540

ttaa 544

<210> 330
<211> 507
<212> DNA
<213> homo sapiens;

<400> 330
gctgcgggta gagaagacag gactcaggac aatctccagc atggcctggt cccctctctt 60

cctcaccctc atcactcact gtgcaggtga caggatgggg accaagagag aggccctggg 120

aagcccatgc gaccctgctt tctcctcttg tctccttttg tctcttgtca atcaccatgt 180

ctgtgtctct ctcacttcca gggtcctggg cccagtctgt gctgactcag ccaccctcgg 240

tgtctgaagc ccccaggcag agggtcacca tctcctgttc tggaagcagc tccaacatcg 300

gaaataatgc tgtaaactgg taccagcagc tcccaggaaa ggctcccaaa ctcctcatct 360

attatgatga tctgctgccc tcaggggtct ctgaccgatt ctctggctcc aagtctggca 420

cctcagcctc cctggccatc agtgggctcc agtctgagga tgaggctgat tattactgtg 480

cagcatggga tgacagcctg aatggtc 507

<210> 331
<211> 517
<212> DNA
<213> homo sapiens;

<400> 331
gctctgcttc agctgtgggc acaagaggca gcactcagga caatctccag catggcctgg 60

tctcctctcc tcctcactct cctcgctcac tgcacaggtg actggataca ggtccagggg 120

aggggccctg ggaagcctat ggattcttgc tttctcctgt tgtctctaga agccgaataa 180

tgatgcctgt gtctctccca cttccagggt cctgggccca gtctgtgctg acgcagccgc 240

cctcagtgtc tggggcccca gggcagaggg tcaccatctc ctgcactggg agcagctcca 300

acatcggggc aggttatgat gtacactggt accagcagct tccaggaaca gcccccaaac 360

tcctcatcta tggtaacagc aatcggccct caggggtccc tgaccgattc tctggctcca 420

agtctggcac ctcagcctcc ctggccatca ctgggctcca ggctgaggat gaggctgatt 480

attactgcca gtcctatgac agcagcctga gtggttc 517

<210>  332
<211>  581
<212>  DNA
<213>  homo sapiens;

<400>  332
ctgatttgca tggatggact ctccccctct cagagtatga agagagggag agatctgggg      60

gaagctcagc ttcagctgtg ggtagagaag acaggactca ggacaatctc cagcatggcc     120

agcttccctc tcctcctcac cctcctcact cactgtgcag gtgacaggat ggggaccaag     180

aaaggggccc tgggaagccc atggggccct gctttctcct cttgtctcct tttgtctctt     240

gtcaatcacc atgtctgtgt ctctctcact tccagggtcc tgggcccagt ctgtgctgac     300

tcagccaccc tcagcgtctg ggacccccgg gcagagggtc accatctctt gttctggaag     360

cagctccaac atcggaagta atactgtaaa ctggtaccag cagctcccag gaacggcccc     420

caaactcctc atctatagta ataatcagcg gccctcaggg gtccctgacc gattctctgg     480

ctccaagtct ggcacctcag cctccctggc catcagtggg ctccagtctg aggatgaggc     540

tgattattac tgtgcagcat gggatgacag cctgaatggt c                         581


<210>  333
<211>  522
<212>  DNA
<213>  homo sapiens;

<400>  333
ggggaagctc agcttcagct gtggtagaga agacaggatt caggacaatc tccagcatgg      60

ccggcttccc tctcctcctc accctcctca ctcactgtgc aggtgacagg atggggacca     120

agagaggggc cctgggaagc ccatggggcc ctgctttctc ctcttgtctc ctttcgtctc     180

ttgtcaatca ccatgtctgt gtctctctca cttccagggt cctgggccca gtctgtgctg     240

actcagccac cctcagcgtc tgggaccccc gggcagaggg tcaccatctc ttgttctgga     300

agcagctcca acatcggaag taattatgta tactggtacc agcagctccc aggaacggcc     360

cccaaactcc tcatctatag taataatcag cggccctcag gggtccctga ccgattctct     420

ggctccaagt ctggcacctc agcctccctg gccatcagtg ggctccggtc cgaggatgag     480

gctgattatt actgtgcagc atgggatgac agcctgagtg gt                        522


<210>  334
<211>  515
<212>  DNA
<213>  homo sapiens;

<400>  334
gctctgcttc agctgtgggc acaggaggca gcactcagga caatctccag catggcctgg      60

tcttctctcc tcctcactct cctcgctcac tgcacaggtg actggatgca gatcgagggg     120

agggtccctg ggaagcctat ggattcttgc tttctcctct tgtctctaga agcagaatca 180

tgatgcctgt gtctctccca cttccagggt cctgggccca gtctgtgctg acgcagccgc 240

cctcagtgtc tggggcccca gggcagaggg tcaccatctc ctgcactggg agcagctcca 300

acattggggc gggttatgtt gtacattggt accagcagct tccaggaaca gcccccaaac 360

tcctcatcta tggtaacagc aatcggccct caggggtccc tgaccaattc tctggctcca 420

agtctggcac ctcagcctcc ctggccatca ctggactcca gtctgaggat gaggctgatt 480

attactgcaa agcatgggat aacagcctga atgct 515

<210> 335
<211> 509
<212> DNA
<213> homo sapiens;

<400> 335
tgagcgcaga aggcaggact cgggacaatc ttcatcatga cctgctcccc tctcctcctc 60

acccttctca ttcactgcac aggtgcccag acacagggtc aggggagggg tccaggaagc 120

ccatgaggcc ctgctttctc cttctctctc tagaccaaga atcaccgtgt ctgtgtctct 180

cctgcttcca gggtcctggg cccagtctgt gttgacgcag ccgccctcag tgtctgcggc 240

cccaggacag aaggtcacca tctcctgctc tggaagcagc tccaacattg ggaataatta 300

tgtatcctgg taccagcagc tcccaggaac agcccccaaa ctcctcattt atgacaataa 360

taagcgaccc tcagggattc ctgaccgatt ctctggctcc aagtctggca cgtcagccac 420

cctgggcatc accggactcc agactgggga cgaggccgat tattactgcg gaacatggga 480

tagcagcctg agtgctggca cagtgctcc 509

<210> 336
<211> 517
<212> DNA
<213> homo sapiens;

<400> 336
tgctggggtc tcaggaggca gcactctcgg gacgtctcca ccatggcctg ggctctgctc 60

ctcctcagcc tcctcactca gggcacaggt gacacctcca gggaaagggt cacaggggtc 120

tctgggctga tccttggtct cctgctcctc aggctcacct gggcccagca ctgactcact 180

agagtgtgtt tctccctctt tccaggatcc tgggctcagt ctgccctgac tcagcctcgc 240

tcagtgtccg ggtctcctgg acagtcagtc accatctcct gcactggaac cagcagtgat 300

gttggtggtt ataactatgt ctcctggtac caacagcacc caggcaaagc ccccaaactc 360

atgatttatg atgtcagtaa gcggccctca ggggtccctg atcgcttctc tggctccaag 420

tctggcaaca cggcctccct gaccatctct gggctccagg ctgaggatga ggctgattat 480

tactgctgct catatgcagg cagctacact ttccaca 517

<210> 337
<211> 519
<212> DNA
<213> homo sapiens;

<400> 337
gctggggtct caggaggcag cgctctcagg acatctccac catggcctgg gctctgctgc 60

tcctcaccct cctcactcag ggcacaggtg acgcctccag ggaaggggct tcagggacct 120

ctgggctgat ccttggtctc ctgctcctca ggctcaccgg ggcccagcac tgactcactg 180

gcatgtgttt ctccctcttt ccagggtcct gggcccagtc tgccctgact cagcctgcct 240

ccgtgtctgg gtctcctgga cagtcgatca ccatctcctg cactggaacc agcagtgacg 300

ttggtggtta taactatgtc tcctggtacc aacagcaccc aggcaaagcc cccaaactca 360

tgatttatga ggtcagtaat cggccctcag gggtttctaa tcgcttctct ggctccaagt 420

ctggcaacac ggcctccctg accatctctg ggctccaggc tgaggacgag gctgattatt 480

actgcagctc atatacaagc agcagcactc tccacagtg 519

<210> 338
<211> 490
<212> DNA
<213> homo sapiens;

<400> 338
gaatatctcc accatggcct gggctctgct cctcctcacc ctcctcactc agggcacagg 60

tgaggcctcc agggaagggg cttcggggac ctctgggctg atccttaact cctgctcctc 120

aggctcacct gggcccagca ctgacttact aaaatgtgtt tcttcctttt tccaggatcc 180

tgggctcagt ctgccctgac tcagcctccc tccgtgtccg ggtctcctgg acagtcagtc 240

accatctcct gcactggaac cagcagtgac gttggtagtt ataaccgtgt ctcctggtac 300

cagcagcccc caggcacagc ccccaaactc atgatttatg aggtcagtaa tcggccctca 360

ggggtccctg atcgcttctc tgggtccaag tctggcaaca cggcctccct gaccatctct 420

gggctccagg ctgaggacga ggctgattat tactgcagct tatatacaag cagcagcact 480

ttccacagag 490

<210> 339
<211> 619
<212> DNA
<213> homo sapiens;

<400> 339
tctctgagcc caggcccacg tgagggtggg gtgaggagag gagcccagga tgctgatttt 60

catggaggcc ccgccctcct ctgaggcaaa ggggataaga cagggctggg gcagggccag 120

tgctggggtc acaagaggca gcgctctcgg gacgtctcca ccatggcctg ggctctgctg 180

ctcctcactc tcctcactca ggacacaggt gacgcctcca gggaaggggt cttggggacc 240

tctgggctga tccttggtct cctgctcctc aggctcaccg gggcccagca ctgactcact 300

ggcatgtgtt tctccctctt tccagggtcc tgggcccagt ctgccctgac tcagcctgcc 360

tccgtgtctg ggtctcctgg acagtcgatc accatctcct gcactggaac cagcagtgat 420

gttgggagtt ataaccttgt ctcctggtac caacagcacc caggcaaagc ccccaaactc 480

atgatttatg agggcagtaa gcggccctca ggggtttcta atcgcttctc tggctccaag 540

tctggcaaca cggcctccct gacaatctct gggctccagg ctgaggacga ggctgattat 600

tactgctgct catatgcag 619

<210> 340
<211> 520
<212> DNA
<213> homo sapiens;

<400> 340
ggctagaggc aggcccggtg ctggggtctc aaggcagcgc tctcgggaca tctccaccat 60

ggcctgggct ctgctcctcc tcaccctcct cactcagggc acaggtgaca cctccaggga 120

aatggccttg gggacctctg agctaatgct tggtcttctg ctcctgctcc tcagggtcac 180

tggacccagt actgacccag tagagtgtgt ttctccctct ttccagggtc ctgggcccaa 240

tctgccctga ctcagcctcc ttttgtgtcc ggggctcctg gacagtcggt caccatctcc 300

tgcactggaa ccagcagtga cgttggggat tatgatcatg tcttctggta ccaaaagcgt 360

ctcagcacta cctccagact cctgatttac aatgtcaata ctcggccttc agggatctct 420

gacctcttct caggctccaa gtctggcaac atggcttccc tgaccatctc tgggctcaag 480

tccgaggttg aggctaatta tcactgcagc ttatattcaa 520

<210> 341
<211> 635
<212> DNA
<213> homo sapiens;

<400> 341
tctctaagcc caggcccaag tgagggtggg gtgagaagag gagctcagga tgcagatttg 60

catggaggtc ccgcccttct ctgaggcaga gggataagac agggctgggg gcaggcccag 120

tgctggggtc tcaggaggca gcgctctcag gacgtcacca ccatggcctg ggctctgctc 180

ctcctcaccc tcctcactca gggcacaggt gatgcctcca gggaaggggc cacaggaccc 240

tctgggctga tccttggtct cctgctcctc aggctcacct gggcccagca ctgactcact 300

agactgtgtt tctccctttc cagggtcctg ggcccagtct gccctgactc agcctccctc 360

cgcgtccggg tctcctggac agtcagtcac catctcctgc actggaacca gcagtgacgt 420

tggtggttat aactatgtct cctggtacca acagcaccca ggcaaagccc ccaaactcat 480

gatttatgag gtcagtaagc ggccctcagg ggtccctgat cgcttctctg gctccaagtc 540

tggcaacacg gcctccctga ccgtctctgg gctccaggct gaggatgagg ctgattatta 600

ctgcagctca tatgcaggca gcaacaattt ccaca 635

<210> 342
<211> 691
<212> DNA
<213> homo sapiens;

<400> 342
aagaacctgc ccagcctggg cctcaggaag cagcatcgga ggtgcctcag ccatggcatg 60

gatccctctc ttcctcggcg tccttgctta ctgcacaggt gctgccccta gggtcctagc 120

cactggtcca gtcccagggc tctgggtcca gcctggccct gactctgagc tcagcagggc 180

ccccgcctgt ggtgggcagg atgctcatga ccctgctgca ggtggatggg ctcggcgggg 240

ctgaaatccc cccacacagt gctcatgtgc tcacactgcc ttagggctct ttcatccctg 300

gatctgtgtc caggccaggc acgtgggaag atttacttgg agttcagctc ctcagtttca 360

agccttttct ctcccgtttt ctctcctgta ggatccgtgg cctcctatga gctgactcag 420

ccaccctcag tgtccgtgtc cccaggacag acagccagca tcacctgctc tggagataaa 480

ttgggggata aatatgcttg ctggtatcag cagaagccag gccagtcccc tgtgctggtc 540

atctatcaag atagcaagcg gccctcaggg atccctgagc gattctctgg ctccaactct 600

gggaacacag ccactctgac catcagcggg acccaggcta tggatgaggc tgactattac 660

tgtcaggcgt gggacagcag cactgcacac a 691

<210> 343
<211> 539
<212> DNA
<213> homo sapiens;

<400> 343
gtgggctcag gaggcagagc tctgggaatc tcaccatggc ctggacccct ctcctgctcc 60

ccctcctcac tttctgcaca ggtgcttctc ccaggccctg ccccaggctc agtgcccata 120

gaccccaagt tggccctgcc ctgaaccctg tgcaaagccc agacacagtc ttagggtagg 180

acccctggga atgggctctt gatcttcaag ccccctctcc tgttttcctt gcagtctctg 240

aggcctccta tgagctgaca cagccaccct cggtgtcagt gtccccagga caaacggcca 300

ggatcacctg ctctggagat gcattgccaa aaaaatatgc ttattggtac cagcagaagt 360

caggccaggc ccctgtgctg gtcatctatg aggacagcaa acgaccctcc gggatccctg 420

agagattctc tggctccagc tcagggacaa tggccacctt gactatcagt ggggcccagg 480

tggaggatga agctgactac tactgttact caacagacag cagtggtaat catagcaca 539

<210> 344
<211> 763
<212> DNA
<213> homo sapiens;

<400> 344
gcctcagcca tggcctggac ccctctcctc ctcagcctcc tcgctcactg cacaggtgct 60

ctgcccaggg tatcaccaac ctgcccatcc ccagggctct gggtccagtg tggccatgac 120

tatgagctca ggagggccct gcctgtggtg ggcaggatgc tcatgaccct gctgcagggt 180

gagggactgg cggagctgaa gtcccctcaa actctgctca gaggcttgtg agagcctgag 240

gggctgcacc tgccaggaga gagtactggg ttttcagttc aaaggctcca tgcagaggga 300

aagtccatgg gccactgggg ctagggctga ttgcagggga taccctgagg gttcacagac 360

tctctgaagc ttttccagga cagcagggca ggggatttca tacggatctt ttacctaaaa 420

gccatcctct cctttttttt tttttttaat ctttgcaggc tctgcgacct cctatgagct 480

gactcagcca cactcagtgt cagtggccac agcacagatg gccaggatca cctgtggggg 540

aaacaacatt ggaagtaaag ctgtgcactg gtaccagcaa aagccaggcc aggaccctgt 600

gctggtcatc tatagcgata gcaaccggcc ctcagggatc cctgagcgat tctctggctc 660

caacccaggg aacaccgcca ccctaaccat cagcaggatc gaggctgggg atgaggctga 720

ctattactgt caggtgtggg acagtagtag tgatcatccc acg 763

<210> 345
<211> 529
<212> DNA
<213> homo sapiens;

<400> 345
tctgtgggtc caggaggcac agctctggga atctcaccat ggcctggatc cctctcctgc 60

tccccctcct cactctctgc acaggtgctg accccaggcc cttccccagg ctcagtcccc 120

acagattcca agttgagcct gacctgaatc ctgagcaaag cccagacaca gcctctgggt 180

gggactcctg gaaatgggtc ctttgtcttc aagccccctc tcttgttctt ccttgcaggc 240

tctgaggcct cctatgagct gacacagcca ccctcggtgt cagtgtccct aggacagatg 300

gccaggatca cctgctctgg agaagcattg ccaaaaaaat atgcttattg gtaccagcag 360

aagccaggcc agttccctgt gctggtgata tataaagaca gcgagaggcc ctcagggatc 420

cctgagcgat tctctggctc cagctcaggg acaatagtca cattgaccat cagtggagtc 480

caggcagaag acgaggctga ctattactgt ctatcagcag acagcagtg 529

<210> 346
<211> 523

<212>  DNA
<213>  homo sapiens;

<400>  346
agctgtgggc tcagaagcag agttctgggg tgtctccacc atggcctgga cccctctctg      60

gctcactctc ctcactcttt gcataggtgc tgcctcccag ggctcaaccc catattatca     120

tgctagctgt gccaacctgg ccctgagctt cggctcaaca cagggagtag tgtagggtgt     180

gggactctag gcgtgaaacc cttatcctca cctcttctgt cctcttttgc aggttctgtg     240

gtttcttctg agctgactca ggaccctgct gtgtctgtgg ccttgggaca gacagtcagg     300

atcacatgcc aaggagacag cctcagaagc tattatgcaa gctggtacca gcagaagcca     360

ggacaggccc ctgtacttgt catctatggt aaaaacaacc ggccctcagg gatcccagac     420

cgattctctg gctccagctc aggaaacaca gcttccttga ccatcactgg ggctcaggcg     480

gaagatgagg ctgactatta ctgtaactcc cgggacagca gtg                       523


<210>  347
<211>  1515
<212>  DNA
<213>  homo sapiens;

<400>  347
gcacagagga gctgtgccct ggaatggggc ctgtacctgt ccaaggcttg tgccgtcccc      60

tgtgggagat gagaagcgtc cctgcattgg gctcttgggg acccgtcttg gacatgagtg     120

agaatgaaga gggtccctgc attgggctct ggcatgtgac tttaaatgga tttaggcctg     180

taccagacat ctcatgtctg acataaaata tttacaatca ggacattact agagaagcag     240

aaaaaagcta accacctccc tcctgagcca ggatggaatg aaggagggga ctgtggaccc     300

cagataattc ccctgtcacc actgtgactc taacaacctc ttaaatcacg gccaacatct     360

atcccatagg aaggtcttta tatcccctag aaaatacaga ggaagtcagc tctgagcttt     420

tccacgacca acccagccaa ggagcaaggc tgggcacaac ctgggtaaag atgtgagccc     480

agaccatggg accagtgggt gaaggaaaat cgcatgggct gagggggtgg gtaagcaggg     540

gccagccctc ctctctctgt ttcctttggg gctgagtcct tctctggaaa ccacagatct     600

cctccagcag cagcctctga ctctgctgat ttgcatcatg ggccgctctc tccagcaagg     660

ggataagaga ggcctgggag gaacctgctc agtctgggcc taaggaagca gcactggtgg     720

tgcctcagcc atggcctgga ccgttctcct cctcggcctc ctctctcact gcacaggtga     780

tccccccagg gtctcaccaa cctgcccagc ccaagggttc tgggtccagc gtgtccttga     840

ttctgagctc aggagggccc ttcctgtggt gggcaggatg ctcatgaccc tgctgcaggg     900

tgggaggctg gtggggctga actcccccca aactgtgctc aaaggcttgt gagagcctga     960

gggactgcac ctgccaggag agagtagtga gttttcagtt caaagtctcc atacaacagg    1020

aaagtcatgg gccactgggg ctggggctga ttgcagggga taccctgagg gttcacagac 1080

tctctggagc ttgtctggga cagcagggca agggatttca taagaagcat ctttcacctg 1140

caagccaacc tctctcttat ttatttattt atttatttat ttatttattt atttattttt 1200

atctttgcag gctctgtgac ctcctatgtg ctgactcagc caccctcggt gtcagtggcc 1260

ccaggacaga cggccaggat tacctgtggg ggaaacaaca ttggaagtaa aagtgtgcac 1320

tggtaccagc agaagccagg ccaggcccct gtgctggtcg tctatgatga tagcgaccgg 1380

ccctcaggga tccctgagcg attctctggc tccaactctg ggaacacggc caccctgacc 1440

atcagcaggg tcgaagccgg ggatgaggcc gactattact gtcaggtgtg ggatagtagt 1500

agtgatcatc ccacg 1515

<210> 348
<211> 558
<212> DNA
<213> homo sapiens;

<400> 348
aagagaggcc tgggaagccc agctgtgctg tgggctcagg aggcagagct gtgggtgtct 60

caccatggca tgggccacac tcctgctccc actcctcaac ctctacacag gtgctgcccc 120

cagaccctgc cccaggctca gccctcctaa gcccctggtc ttaccctgaa ccctgagctc 180

agcccaggca tagcctcagg gcgatactac tggaatgggt ttgttatctt caagccccct 240

ctcttgtcct ctcttgcagg ctctgttgcc tcctatgagc tgacacagct accctcggtg 300

tcagtgtccc caggacagac agccaggatc acctgctctg gagatgtact gggggaaaat 360

tatgctgact ggtaccagca gaagccaggc caggcccctg agttggtgat atacgaagat 420

agtgagcggt accctggaat ccctgaacga ttctctgggt ccacctcagg gaacacgacc 480

accctgacca tcagcagggt cctgaccgaa gacgaggctg actattactg tttgtctggg 540

gatgaggaca atccctca 558

<210> 349
<211> 546
<212> DNA
<213> homo sapiens;

<400> 349
gctgtgctgt gggtccagga ggcagaactc tgggtgtctc accatggcct ggatccctct 60

acttctcccc ctcttcactc tctgcacagg tgctgtcccc aggccctgct ccaggccctg 120

ctccagtctt attccccaca gatcccaagt tgagcctgcc ctgaatcccg agcaaagccc 180

agacgcagcc tctgggtgcg actcctggga atgggtcctt tgtcttcaag ccccctctct 240

tgttcttcct tgcaggctct gaggcctcct atgagctgac acagccaccc tcggtgtcag 300

tgtccccagg acagacggcc aggatcacct gctctggaga tgcattgcca aagcaatatg 360

cttattggta ccagcagaag ccaggccagg cccctgtgct ggtgatatat aaagacagtg 420

agaggccctc agggatccct gagcgattct ctggctccag ctcagggaca acagtcacgt 480

tgaccatcag tggagtccag gcagaagatg aggctgacta ttactgtcaa tcagcagaca 540

gcagtg 546

<210> 350
<211> 519
<212> DNA
<213> homo sapiens;

<400> 350
gctgtaggct caggaggcag agctctgaat gtctcaccat ggcctggatc cctctcctgc 60

tccccctcct cattctctgc acaggtgctg cccctaggct cagtctccac agaccccaag 120

ttgagcctga cctgaatcct gagcaaagcc ctgccactgc ctctgggggg gattcctggc 180

aatgcgtcct ttgtcctcaa gccccctctc ctgtcttttc ttgcagtctc tgtggcctcc 240

tatgagctga cacagccatc ctcagtgtca gtgtctccgg gacagacagc caggatcacc 300

tgctcaggag atgtactggc aaaaaaatat gctcggtggt tccagcagaa gccaggccag 360

gcccctgtgc tggtgattta taaagacagt gagcggccct cagggatccc tgagcgattc 420

tccggctcca gctcagggac cacagtcacc ttgaccatca gcggggccca ggttgaggat 480

gaggctgact attactgtta ctctgcggct gacaacaat 519

<210> 351
<211> 504
<212> DNA
<213> homo sapiens;

<400> 351
gctgtggact cagaggcaga gctctggggc atttccatta tggcctggac ccctcccctg 60

ctcgtcctca ctctctgcac aggtgctgcc tcccagggct cagcccccag tgggatcaag 120

atcagcctgg ccctgacctt caactcaaca tagggagtga tgcagggtgt ggggttctgg 180

gaatgaggcc ctcatcctca gactcacctc tcctgtcctc tcttgtgggc tccgttattt 240

cctctgggcc aactcaggtg cctgcagtgt ctgtggcctt gggacaaatg gccaggatca 300

cctgccaggg agacagcatg gaaggctctt atgaacactg gtaccagcag aagccaggcc 360

aggcccccgt gctggtcatc tatgatagca gtgaccggcc ctcaaggatc cctgagcgat 420

tctctggctc caaatcaggc aacacaacca ccctgaccat cactggggcc caggctgagg 480

atgaggctga ttattactat cagt 504

<210> 352
<211> 747
<212> DNA

<213>  homo sapiens;

<400>  352
cttgactctg ctgatttgca tcacaggctg ctctcttcag caaggggata agagagggct      60

ggaaggaacc tgcccagcct gggcctcagg aagcagcatc gggggtgccg cagccatggc     120

ctggaccgct ctccttctga gcctccttgc tcactttaca ggtgctgccc ccagtgtccc     180

agccacctac ccagctccaa ggctctgggt ccagcctggc ctgacagtga tctcagcagg     240

gccctgcctg tggtgtgcag gatgctcatg atcctgctgc agggggaggg gctgctggag     300

gtgaaatccc cccacactgt tcttctgtgc tcatggtccc ctgaggacac ttctattcct     360

gaaactcagg ccaggcaggt gggaaggcat tgttgggttg agcctctcag tttcaagtct     420

attctattct ctcccctttt cttgcaggtt ctgtggcctc ctatgagctg actcagccac     480

tctcagtgtc agtggccctg ggacagacgg ccaggattac ctgtggggga aacaacattg     540

gaagtaaaaa tgtgcactgg taccagcaga agccaggcca ggcccctgtg ctggtcatct     600

atagggatag caaccggccc tctgggatcc ctgagcgatt ctctggctcc aactcgggga     660

acacggccac cctgaccatc agcagagccc aagccgggga tgaggctgac tattactgtc     720

aggtgtggga cagcagcact gcacaca                                         747


<210>  353
<211>  529
<212>  DNA
<213>  homo sapiens;

<400>  353
   ctcgaataga gctcttggaa gtccctccaa ccatggcctg ggtctccttc tacctactgc      60
   ccttcatttt ctccacaggt cagaacatcc cagggaattc agggaaatgt tttcactgct     120
   attttcccat gagcaccagt cctcaggggc attctttcca gttcttctgt gcattcagca     180
   tcattcatga cattctgttt acaggtctct gtgctctgcc tgtgctgact cagcccccgt     240
   ctgcatctgc cttgctggga gcctcgatca agctcacctg caccctaagc agtgagcaca     300
   gcacctacac catcgaatgg tatcaacaga gaccagggag gtccccccag tatataatga     360
   aggttaagag tgatggcagc cacagcaagg gggacgggat ccccgatcgc ttcatgggct     420
   ccagttctgg ggctgaccgc tacctcacct tctccaacct ccagtctgac gatgaggctg     480
   agtatcactg tggagagagc cacacgattg atggccaagt cggttgagc                 529


<210>  354
<211>  483
<212>  DNA
<213>  homo sapiens;

<400>  354
atggcctgga ccccactcct cctcctcttc cctctcctcc tccactgcac aggtcaggag      60

gaccctcagc atcctcatgc cccagctcac tgacaccatc tcccaaactc ataccagaaa 120

tgttgtttgc tcttgtcctt ccttcaggcc ataatgagcg tctctgtttt cagggtctct 180

ctcccagcct gtgctgactc aatcatcctc tgcctctgct tccctgggat cctcggtcaa 240

gctcacctgc actctgagca gtgggcacag tagctacatc atcgcatggc atcagcagca 300

gccagggaag gcccctcggt acttgatgaa gcttgaaggt agtggaagct acaacaaggg 360

gagcggagtt cctgatcgct tctcaggctc cagctctggg gctgaccgct acctcaccat 420

ctccaacctc cagtttgagg atgaggctga ttattactgt gagacctggg acagtaacac 480

tca 483

<210> 355
<211> 539
<212> DNA
<213> homo sapiens;

<400> 355
agggtgggta agaaatacct gcaactgtca gcctcagcag agctctgggg agtctgcacc 60

atggcttgga ccccactcct cttcctcacc ctcctcctcc actgcacagg tcaggatggc 120

cctcagcacc ctgacctcca gctcactgat accacctccc aaacttatgc caggaatgtc 180

cttccctctt ttcttgactc cagccggtaa tgggtgtctg tgttttcagg gtctctctcc 240

cagcttgtgc tgactcaatc gccctctgcc tctgcctccc tgggagcctc ggtcaagctc 300

acctgcactc tgagcagtgg gcacagcagc tacgccatcg catggcatca gcagcagcca 360

gagaagggcc ctcggtactt gatgaagctt aacagtgatg gcagccacag caagggggac 420

gggatccctg atcgcttctc aggctccagc tctggggctg agcgctacct caccatctcc 480

agcctccagt ctgaggatga ggctgactat tactgtcaga cctggggcac tggcattca 539

<210> 356
<211> 496
<212> DNA
<213> homo sapiens;

<400> 356
ccaccatggc ctggactcct cttcttctct tgctcctctc tcactgcaca ggtagggaca 60

ggcctcagag atcagggcca gccacccaac ctgattctgg ctcttctggt aaagatccct 120

gaaaaacctc accctgaacc ctgcccatca accatgagtg tctgtgtttg caggttccct 180

ctcccagcct gtgctgactc agccaccttc ctcctccgca tctcctggag aatccgccag 240

actcacctgc accttgccca gtgacatcaa tgttggtagc tacaacatat actggtacca 300

gcagaagcca gggagccctc ccaggtatct cctgtactac tactcagact cagataaggg 360

ccagggctct ggagtcccca gccgcttctc tggatccaaa gatgcttcag ccaatacagg 420

gattttactc atctccgggc tccagtctga ggatgaggct gactattact gtatgatttg 480

gccaagcaat gcttct                                                    496


<210>  357
<211>  520
<212>  DNA
<213>  homo sapiens;

<400>  357
actgcggggg taagaggttg tgtccaccat ggcctggact cctctcctcc tcctgttcct      60

ctctcactgc acaggtagga atagacttca gagaccaggg tcagccaccc agcctgattc     120

tgactcttct ggcaaagatc cctgaaaaac tttaccctgg tttctgcctt agcacccatt     180

aatgtctgtg tttccaggtt ccctctcgca ggctgtgctg actcagccgt cttccctctc     240

tgcatctcct ggagcatcag ccagtctcac ctgcaccttg cgcagtggca tcaatgttgg     300

tacctacagg atatactggt accagcagaa gccagggagt cctccccagt atctcctgag     360

gtacaaatca gactcagata agcagcaggg ctctggagtc cccagccgct tctctggatc     420

caaagatgct tcggccaatg cagggatttt actcatctct gggctccagt ctgaggatga     480

ggctgactat tactgtatga tttggcacag cagcgcttct                           520


<210>  358
<211>  493
<212>  DNA
<213>  homo sapiens;

<400>  358
atggcctgga atcctctcct cctcctgttc ctctctcact gcacaggtag gaaaaggcct      60

cagagaccag ggtcagccac acagcctgat tctgactctt gtgtcaaaga tcactaaaaa     120

aaatattacc ttggtttctg tcttaaagcc tatatatgcc tgtgttccag gttccctctc     180

gcagcctgtg ctgactcagc caacttccct ctcagcatct cctggagcat cagccagact     240

cacctgcacc ttgcgcagtg gcatcaatct tggtagctac aggatattct ggtaccagca     300

gaagccagag agccctcccc ggtatctcct gagctactac tcagactcaa gtaagcatca     360

gggctctgga gtccccagcc gcttctctgg atccaaagat gcttcgagca atgcagggat     420

tttagtcatc tctgggctcc agtctgagga tgaggctgac tattactgta tgatttggca     480

cagcagtgct tct                                                        493


<210>  359
<211>  500
<212>  DNA
<213>  homo sapiens;

<400>  359
ccaccatggc ctggactctt ctccttctcg tgctcctctc tcactgcaca ggtagggaaa      60

gtccttataa actgagtctc agtgtccaac ctacaccatc ccctgtggct cagacctaca     120

agaagcttta ccctgggaac tgccttatca cccatgatgt ctgtgttttc aggttccctc 180

tcccagcctg tgctgactca gccatcttcc cattctgcat cttctggagc atcagtcaga 240

ctcacctgca tgctgagcag tggcttcagt gttggggact tctggataag gtggtaccaa 300

caaaagccag ggaaccctcc ccggtatctc ctgtactacc actcagactc caataagggc 360

caaggctctg gagttcccag ccgcttctct ggatccaacg atgcatcagc caatgcaggg 420

attctgcgta tctctgggct ccagcctgag gatgaggctg actattactg tggtacatgg 480

cacagcaact ctaagactca 500

<210> 360
<211> 574
<212> DNA
<213> homo sapiens;

<400> 360
tctgaggata cgcgtgacag ataagaaggg ctggtgggat cagtcctggt ggtagctcag 60

gaagcagagc ctggagcatc tccactatgg cctgggctcc actacttctc accctcctcg 120

ctcactgcac aggtggctgc ctgcaaggaa ttcagggagc gttcctggat gtcacctggg 180

ctgatgatct gttcctcctg cctgggaacc agtcttcatc tctcccgact gatctctgtg 240

ttgctctctt cttgcaggtt cttgggccaa ttttatgctg actcagcccc actctgtgtc 300

ggagtctccg gggaagacgg taaccatctc ctgcacccgc agcagtggca gcattgccag 360

caactatgtg cagtggtacc agcagcgccc gggcagttcc cccaccactg tgatctatga 420

ggataaccaa agaccctctg gggtccctga tcggttctct ggctccatcg acagctcctc 480

caactctgcc tccctcacca tctctggact gaagactgag gacgaggctg actactactg 540

tcagtcttat gatagcagca atcacacagt gctc 574

<210> 361
<211> 472
<212> DNA
<213> homo sapiens;

<400> 361
tctggcgcca ggggtccctt ccaatatcag caccatggcc tggactcctc tctttctgtt 60

cctcctcact tgctgcccag gttaagagag atttcaaata ccagcctttg gagggatcct 120

tctgtctgcc cttctaattt ctaacatgtg tctgtttttt gtttcagggt ccaattctca 180

gactgtggtg actcaggagc cctcactgac tgtgtcccca ggagggacag tcactctcac 240

ctgtgcttcc agcactggag cagtcaccag tggttactat ccaaactggt tccagcagaa 300

acctggacaa gcacccaggg cactgattta tagtacaagc aacaaacact cctggacccc 360

tgcccggttc tcaggctccc tccttggggg caaagctgcc ctgacactgt caggtgtgca 420

gcctgaggac gaggctgagt attactgcct gctctactat ggtggtgctc ag          472

<210>  362
<211>  473
<212>  DNA
<213>  homo sapiens;

<400>  362
tctggcacca ggggtccctt ccaatatcag caccatggcc tggactcctc tctttctgtt    60

cctcctcact tgctgcccag gttaagagag atttcaaata ccagcctttg gagggatccc   120

tttttctccc tttctaattc ctaatatatg tctgtttttt ttgtttcagg gtccaattcc   180

caggctgtgg tgactcagga gccctcactg actgtgtccc caggagggac agtcactctc   240

acctgtggct ccagcactgg agctgtcacc agtggtcatt atccctactg gttccagcag   300

aagcctggcc aagcccccag gacactgatt tatgatacaa gcaacaaaca ctcctggaca   360

cctgcccggt tctcaggctc cctccttggg ggcaaagctg ccctgaccct tttgggtgcg   420

cagcctgagg atgaggctga gtattactgc ttgctctcct atagtggtgc tcg          473

<210>  363
<211>  513
<212>  DNA
<213>  homo sapiens;

<400>  363
gaggaaaaca aaccccagct gggaagcctg agaacactta gccttcatga gtgtccccac    60

catggcctgg atgatgcttc tcctcggact ccttgcttat ggatcaggtc aggggaaggg   120

actctatccc tgggggacca cagaaaacag ggtccaggtt actctcatcc tcatgatcat   180

aactgtgtct ctcctgttcg ttttaggagt ggattctcag actgtggtga cccaggagcc   240

atcgttctca gtgtcccctg gagggacagt cacactcact tgtggcttga gctctggctc   300

agtctctact agttactacc ccagctggta ccagcagacc ccaggccagg ctccacgcac   360

gctcatctac agcacaaaca ctcgctcttc tggggtccct gatcgcttct ctggctccat   420

ccttgggaac aaagctgccc tcaccatcac gggggcccag gcagatgatg aatctgatta   480

ttactgtgtg ctgtatatgg gtagtggcat ttc                                513

<210>  364
<211>  546
<212>  DNA
<213>  homo sapiens;

<400>  364
gagagactga agaacccagc attgcagcag ctccaccatg gcctgggctc ctctgctcct    60

caccctcctc agtctcctca caggtcaggg tgggcagtgg gctgggcccc caaaggaccc   120

cccacctccc agcctccatc tccccatccc tgctcttcct cctccaacag ctcatcagcc   180

acccaccaac aggagccctc atgggtgtct gtgtttccag ggtccctctc ccagcctgtg 240

ctgactcagc caccttctgc atcagcctcc ctgggagcct cggtcacact cacctgcacc 300

ctgagcagcg gctacagtaa ttataaagtg gactggtacc agcagagacc agggaagggc 360

ccccggtttg tgatgcgagt gggcactggt gggattgtgg gatccaaggg ggatggcatc 420

cctgatcgct tctcagtctt gggctcaggc ctgaatcggt acctgaccat caagaacatc 480

caggaagaag atgagagtga ctaccactgt ggggcagacc atggcagtgg gagcaacttc 540

gtgtaa 546

<210> 365
<211> 6729
<212> DNA
<213> homo sapiens;

<400> 365
gcctccacca agggcccatc ggtcttcccc ctggcaccct cctccaagag cacctctggg 60

ggcacagcag ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttggtgag 300

aggccagcac agggagggag ggtgtctgct ggaagccagg ctcagcgctc ctgcctggac 360

gcatcccggc tatgcagccc cagtccaggg cagcaaggca ggccccgtct gcctcttcac 420

ccggaggcct ctgcccgccc cactcatgct cagggagagg gtcttctggc tttttcccca 480

ggctctgggc aggcacaggc taggtgcccc taacccaggc cctgcacaca aaggggcagg 540

tgctgggctc agacctgcca agagccatat ccgggaggac cctgcccctg acctaagccc 600

accccaaagg ccaaactctc cactccctca gctcggacac cttctctcct cccagattcc 660

agtaactccc aatcttctct ctgcagagcc caaatcttgt gacaaaactc acacatgccc 720

accgtgccca ggtaagccag cccaggcctc gccctccagc tcaaggcggg acaggtgccc 780

tagagtagcc tgcatccagg gacaggcccc agccgggtgc tgacacgtcc acctccatct 840

cttcctcagc acctgaactc ctggggggac cgtcagtctt cctcttcccc ccaaaaccca 900

aggacaccct catgatctcc cggacccctg aggtcacatg cgtggtggtg gacgtgagcc 960

acgaagaccc tgaggtcaag ttcaactggt acgtggacgg cgtggaggtg cataatgcca 1020

agacaaagcc gcgggaggag cagtacaaca gcacgtaccg tgtggtcagc gtcctcaccg 1080

tcctgcacca ggactggctg aatggcaagg agtacaagtg caaggtctcc aacaaagccc 1140

tcccagcccc catcgagaaa accatctcca aagccaaagg tgggacccgt ggggtgcgag 1200

ggccacatgg acagaggccg gctcggccca ccctctgccc tgagagtgac cgctgtacca 1260

acctctgtcc ctacagggca gccccgagaa ccacaggtgt acaccctgcc cccatcccgg 1320

gatgagctga ccaagaacca ggtcagcctg acctgcctgg tcaaaggctt ctatcccagc 1380

gacatcgccg tggagtggga gagcaatggg cagccggaga acaactacaa gaccacgcct 1440

cccgtgctgg actccgacgg ctccttcttc ctctacagca agctcaccgt ggacaagagc 1500

aggtggcagc aggggaacgt cttctcatgc tccgtgatgc atgaggctct gcacaaccac 1560

tacacacaga agagcctctc cctgtctccg ggtaaatgag tgccacggcc ggcaagcccc 1620

cgctccccag gctctcgggg tcgcgcgagg atgcttggca cgtaccccgt gtacatactt 1680

cccaggcacc cagcatggaa ataaagcacc cagcgcttcc ctgggcccct gcgagactgt 1740

gatggttctt tccacgggtc aggccgagtc tgaggcctga gtggcatgag ggaggcagag 1800

tgggtcccac tgtccccaca ctggcccagg ctgtgcaggt gtgcctgggc cgcctagggt 1860

ggggctcagc caggggctgc cctcggcagg gtgggggatt tgccagcgtg gccctccctc 1920

cagcagcagc tgccctgggc tgggccacga gaagccctag gagcccctgg ggacagacac 1980

acagcccctg cctctgtagg agactgtcct gttctgtgag cgccctgtcc tccgacccgc 2040

atgcccactc gggggcatgc ctagtccatg tgcgtaggga caggccctcc ctcacccatc 2100

tacccccacg gcactaaccc ctggcagccc tgcccagcct cgcacccgca tggggacaca 2160

accgactccg ggacatgcca ctctcggccc ctgtggagag actggtccag atgcccacac 2220

acacactcag cccagacccg ttcaacaaac cccgcactga ggttggccgg ccacacggcc 2280

accacacaca cacgtgcacg cctcacacac ggagcctcac ccgggcgaac cgcacagcac 2340

ccagaccaga gcaaggtcct cgcacacgtg aacactcctc ggacacaggc ccccacgagc 2400

cccacgcggc acctcaaggc ccacgagccg ctcggcagct tctccacatg ctgacctgct 2460

cagacaaacc cagccctcct ctcacaaggt gcccctgcag ccgccacaca cacacagggg 2520

atcacacacc acgtcacgtc cctggccctg gcccacttcc cagtgccgcc cttccctgca 2580

gctggggtca catgaggtgt gggcttcacc atcctcctgc cctctgggcc tcagggaggg 2640

acacggagaa cggggagcgg gtcctgctga gggccaggtc gctatctagg gccgggtgtc 2700

tggctgagcc ccggggccaa agctggtgcc cagggcgggc agctgtgggg agctgacctc 2760

aggacattgt tggcccatcc cggccgggcc ctacatcctg ggtcctgcca cagagggaat 2820

cacccccaga ggcccaagcc cagggggaca cagcactgac cacccccttc ctgtccagag 2880

ctgcaactgg aggagagctg tgcggaggcg caggacgggg agctggacgg gctgtggacg 2940

accatcacca tcttcatcac actcttcctg ttaagcgtgt gctacagtgc caccgtcacc 3000

ttcttcaagg tcggccgcac gttgtcccca gctgtccttg acattgtccc ccatgctgtc 3060

acaaactgtc tctgacactg tcccacaggc tgtccccacc tgtccctgac gctgtccccc 3120

atgctctcac aaactgtccc tgacattgtc cccaatgctg cccccacctg tccaacagtg 3180

tcccccaggc tctccccaca tgtccccgac actgtccccc atgctgtccc catctgtccc 3240

caacactgtc ccccaccctg tccccctttg tccccaacac tgtcccccac agtttccacc 3300

tgtccctgac actgtccccc atgctttccc cacctgtccc tgacaccatc ccccactctg 3360

tcccctatag ttcctggccc tgtcccccac gctgtcccct acagtacctg gcactgtccc 3420

ccatgctgtc ccctcctgta tgaaaccctg tcccacatgc tgtccccacc tgtccgtgac 3480

aatatccccc acactgtccc cacctgtccc cgacactctc ctccacgttg ttcttaccta 3540

aacccgacac tttcctccat gctgtcccca cccatctccg acactgtacc ccacgttgtc 3600

cccacctgtc ctcaacactg tcccccatgc tgtccccacc tgtccccaac actctcctcc 3660

atgctgtccc cacctgtccc tgatattgtc ccccatgcag tctccacctg tccccaatgc 3720

tgtcccccag gctgtaccta ccagtacaac actgtccccc atgctgtccc cacctgtccc 3780

tgacactgtc ccccacgctg tcccctcctg tccccgacac tgtcccccac actgtcccca 3840

cctgtcccca acactatcct ccatgctgtc ccctcctgtc cccacctgtc ccctacactg 3900

tcccccatgc tgtccccacc agtccccaaa actttcctcc acactgtccc cacctgtccc 3960

caacactgtc ccccacgcta tcccccctgt ccccgacaat gtccccactg tttcctcctg 4020

ttccctccta tccctgacac tgtccgccat gctgtcccca cctgtccctg acactgtctc 4080

ccactctgtc ccctataatc cctgacactg tcccccacgc cgtcccctcc cgtatgcacc 4140

actgtccccc aagctgtccc cacctgtcct caacacagtc ccccatgctg tccccacctg 4200

tccccaacac tctcctccat gtccccacct gtccctgata ttgtccccca tgcagtcccc 4260

acctgtcccc gatgctgtcc cccgggctgt acctaccagt ccaacactgt cccccacact 4320

ctccccacct gtccctgata ctgtccccca tgctgtcccc acctgtcccg gacactgttc 4380

tccacgctct cccctcctgt ccctgacact gtcccccaca ctgtccccac ctgtccccaa 4440

cactatcctc catcctgtcc caacctgtct cctacactgt cccccatgct gtccccacca 4500

gtccccaaca ctgtcctcca tgctgtcccc catgtcccca acactgtccc ccatgctatc 4560

tcccctgtcc ctgacaatgt ccccactgtt tcctgtcccc tcctatccct gacactgtcc 4620

cccatgctgt ccccacctgt cccccacatg gtctccaccg gtccctgaca ctgtctccca 4680

ctctgtcccc tataatccct gacactgtcc cccacaccgt cccctcctgt atgcaccact 4740

gtcccccatg ctgtccccac ctgtccctga tgctgtcctc cacacagtcc ccacctctcc 4800

ctgacactgt ccccatctct ccccaacact ctcctccatg ctgtccttaa ctgtccccaa 4860

cactcttcca cactctgtct ccacctgtcc ctgacactgt cccccacact gtcctcacct 4920

gtgtctgaca ctgtccccca cgctgtcccc acctgtccct gacgctgtct tctgtgctgt 4980

ccacatgctg ttggtgccct ggctctgctc tctatcacca agcctcagag caggcagtgg 5040

tgaggccatg gcacctgggt ggcatgaggg gccggatggg cctcaggggc agggctgtgg 5100

cctgcgtgga ctgacgggtg ggtgggcctt gggggcagag aggtggcctc agtgccctga 5160

ggggtgggtg gggctcgggg gcagggctgt ggcctcgctc acccctgtgc tgtgccttgc 5220

ctacaggtga agtggatctt ctcctcggtg gtggacctga agcagaccat catccccgac 5280

tacaggaaca tgatcggaca gggggcctag ggccaccctc tgcggggtgt ccagggccgc 5340

ccagacccca cacaccagcc atgggccatg ctcagccacc acccaggcca cacctgcccc 5400

cgacctcacc gccctcaacc ccatgactct ctggcctcgc agttgccctc tgaccctgac 5460

acacctgaca cgcccccctt ccagaccctg tgcatagcag gtctacccca gacctccgct 5520

gcttggtgca tgcagggcac tgggggccag gtgtcccctc agcaggacgt ccttgccctc 5580

cggaccacaa ggtgctcaca caaaaggagg cagtgaccgg tatcccaggc ccccacccag 5640

gcaggacctc gccctggagc caaccccgtc cacgccagcc tcctgaacac aggcgtggtt 5700

tccagatggt gagtgggagc gtcagccgcc aaggtaggga agccacagca ccatcaggcc 5760

ctgttgggga ggcttccgag agctgcgaag gctcactcag acggccttcc tcccagcccg 5820

cagccagcca gcctccattc cgggcactcc cgtgaactcc tgacatgagg aatgaggttg 5880

ttctgatttc aagcaaagaa cgctgctctc tggctcctgg gaacagtctc agtgccagca 5940

ccaccccttg gctgcctgcc cacactgctg gattctcggg tggaactgga cccgcaggga 6000

cagccagccc cagagtccgc actggggaga gaaggggcca ggcccaggac actgccacct 6060

cccacccact ccagtccacc gagatcactc agagaagagc ctgggccatg tggccgctgc 6120

aggagcccca cagtgcaagg gtgaggatag cccaaggaag ggctgggcat ctgcccagac 6180

aggcctccca gagaaggctg gtgaccaggt cccaggcggg caagactcag ccttggtggg 6240

gcctgaggac agaggaggcc caggagcatc ggggagagag gtggagggac accgggagag 6300

ccaggagcgt ggacacagcc agaactcatc acagaggctg gcgtccagcc ccgggtcacg 6360

tgcagcagga acaagcagcc actctggggg caccaggtgg agaggcaaga cgacaaagag 6420

ggtgcccgtg ttcttgcgaa agcagggctg ctggccacga gtgctggaca gaggccccca 6480

cgctctgctg cccccatcac gccgttccgt gactgtcacg cagaatctgc agacaggaag 6540

ggagactcga gcgggagtgc ggccagcgcc tgcctcggcc gtcagggagg actcctgggc 6600

tcactcgaag gaggtgccac catttcagct ttggtagctt ttcttcttct tttaaatttt 6660

ctaaagctca ttaattgtct ttgatgtttc ttttgtgatg acaataaaat atccttttta 6720

agtcttgta 6729

<210> 366
<211> 888
<212> DNA
<213> homo sapiens;

<400>  366
gcctccacca agggcccatc ggtcttcccc ctggcaccct cctccaagag cacctctggg      60

ggcacagcag ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggagta caagtgcaag gtctccaaca aagccctccc agcccccatc     540

gagaaaacca tctccaaagc caaagggcag ccccgagaac cacaggtgta caccctgccc     600

ccatcccggg atgagctgac caagaaccag gtcagcctga cctgcctggt caaaggcttc     660

tatcccagcg acatcgccgt ggagtgggag agcaatgggc agccggagaa caactacaag     720

accacgcctc ccgtgctgga ctccgacggc tccttcttcc tctacagcaa gctcaccgtg     780

gacaagagca ggtggcagca ggggaacgtc ttctcatgct ccgtgatgca tgaggctctg     840

cacaaccact acacacagaa gagcctctcc ctgtctccgg gtaaatga                  888


<210>  367
<211>  993
<212>  DNA
<213>  homo sapiens;

<400>  367
gcctccacca agggcccatc ggtcttcccc ctggcaccct cctccaagag cacctctggg      60

ggcacagcag ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacaca 960

cagaagagcc tctccctgtc tccgggtaaa tga 993

<210> 368
<211> 1200
<212> DNA
<213> homo sapiens;

<400> 368
gcctccacca agggcccatc ggtcttcccc ctggcaccct cctccaagag cacctctggg 60

ggcacagcag ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacaca 960

cagaagagcc tctccctgtc tccggagctg caactggagg agagctgtgc ggaggcgcag 1020

gacggggagc tggacgggct gtggacgacc atcaccatct tcatcacact cttcctgtta 1080

agcgtgtgct acagtgccac cgtcaccttc ttcaaggtga agtggatctt ctcctcggtg 1140

gtggacctga agcagaccat catccccgac tacaggaaca tgatcggaca gggggcctag 1200

<210> 369
<211> 1739
<212> DNA
<213> homo sapiens;

<400> 369
gcctccacca agggcccatc ggtcttcccc ctggcgccct gctccaggag cacctccgag 60

agcacagcgg ccctgggctg cctggtcaag gactacttcc ccgaaccggt gacggtgtcg 120

tggaactcag gcgctctgac cagcggcgtg cacaccttcc cggctgtcct acagtcctca 180

ggactctact ccctcagcag cgtggtgacc gtgccctcca gcaacttcgg cacccagacc 240

tacacctgca acgtagatca caagcccagc aacaccaagg tggacaagac agttggtgag 300

aggccagctc agggagggag ggtgtctgct ggaagccagg ctcagccctc ctgcctggac 360

gcaccccggc tgtgcagccc cagcccaggg cagcaaggca ggccccatct gtctcctcac 420

ccggaggcct ctgcccgccc cactcatgct cagggagagg gtcttctggc tttttccacc 480

aggctccagg caggcacagg ctgggtgccc ctaccccagg cccttcacac acaggggcag 540

gtgcttggct cagacctgcc aaaagccata tccgggagga ccctgcccct gacctaagcc 600

gaccccaaag gccaaactgt ccactccctc agctcggaca ccttctctcc tcccagatcc 660

gagtaactcc caatcttctc tctgcagagc gcaaatgttg tgtcgagtgc ccaccgtgcc 720

caggtaagcc agcccaggcc tcgccctcca gctcaaggcg ggacaggtgc cctagagtag 780

cctgcatcca gggacagacc ccagctgggt gctgacacgt ccacctccat ctcttcctca 840

gcaccacctg tggcaggacc gtcagtcttc ctcttccccc caaaacccaa ggacaccctc 900

atgatctccc ggacccctga ggtcacgtgc gtggtggtgg acgtgagcca cgaagacccc 960

gaggtccagt tcaactggta cgtggacggc gtggaggtgc ataatgccaa gacaaagcca 1020

cgggaggagc agttcaacag cacgttccgt gtggtcagcg tcctcaccgt cgtgcaccag 1080

gactggctga acggcaagga gtacaagtgc aaggtctcca acaaaggcct cccagccccc 1140

atcgagaaaa ccatctccaa aaccaaaggt gggacccgcg gggtatgagg gccacatgga 1200

cagaggccgg ctcggcccac cctctgccct gggagtgacc gctgtgccaa cctctgtccc 1260

tacagggcag ccccgagaac cacaggtgta caccctgccc ccatcccggg aggagatgac 1320

caagaaccag gtcagcctga cctgcctggt caaaggcttc taccccagcg acatctccgt 1380

ggagtgggag agcaatgggc agccggagaa caactacaag accacacctc ccatgctgga 1440

ctccgacggc tccttcttcc tctacagcaa gctcaccgtg gacaagagca ggtggcagca 1500

ggggaacgtc ttctcatgct ccgtgatgca tgaggctctg cacaaccact acacacagaa 1560

gagcctctcc ctgtctccgg gtaaatgagt gccacggccg gcaagccccc gctccccagg 1620

ctctcggggt cgcgcgagga tgcttggcac gtaccccgtc tacatacttc ccgggcaccc 1680

agcatggaaa taaagcaccc agcgctgccc tgggcccctg cgagactgtg atggttctt 1739

<210> 370
<211> 981
<212> DNA

<213>  homo sapiens;

<400>  370
gcctccacca agggcccatc ggtcttcccc ctggcgccct gctccaggag cacctccgag      60

agcacagcgg ccctgggctg cctggtcaag gactacttcc ccgaaccggt gacggtgtcg     120

tggaactcag gcgctctgac cagcggcgtg cacaccttcc cggctgtcct acagtcctca     180

ggactctact ccctcagcag cgtggtgacc gtgccctcca gcaacttcgg cacccagacc     240

tacacctgca acgtagatca caagcccagc aacaccaagg tggacaagac agttgagcgc     300

aaatgttgtg tcgagtgccc accgtgccca gcaccacctg tggcaggacc gtcagtcttc     360

ctcttccccc caaaacccaa ggacaccctc atgatctccc ggacccctga ggtcacgtgc     420

gtggtggtgg acgtgagcca cgaagacccc gaggtccagt tcaactggta cgtggacggc     480

gtggaggtgc ataatgccaa gacaaagcca cgggaggagc agttcaacag cacgttccgt     540

gtggtcagcg tcctcaccgt cgtgcaccag gactggctga acggcaagga gtacaagtgc     600

aaggtctcca acaaaggcct cccagccccc atcgagaaaa ccatctccaa aaccaaaggg     660

cagccccgag aaccacaggt gtacaccctg cccccatccc gggaggagat gaccaagaac     720

caggtcagcc tgacctgcct ggtcaaaggc ttctacccca gcgacatctc cgtggagtgg     780

gagagcaatg ggcagccgga gaacaactac aagaccacac ctcccatgct ggactccgac     840

ggctccttct tcctctacag caagctcacc gtggacaaga gcaggtggca gcaggggaac     900

gtcttctcat gctccgtgat gcatgaggct ctgcacaacc actacacaca gaagagcctc     960

tccctgtctc cgggtaaatg a                                               981


<210>  371
<211>  981
<212>  DNA
<213>  homo sapiens;

<400>  371
gcctccacca agggcccatc ggtcttcccc ctggcgccct gctccaggag cacctccgag      60

agcacagcgg ccctgggctg cctggtcaag gactacttcc ccgaaccggt gacggtgtcg     120

tggaactcag gcgctctgac cagcggcgtg cacaccttcc cggctgtcct acagtcctca     180

ggactctact ccctcagcag cgtggtgacc gtgacctcca gcaacttcgg cacccagacc     240

tacacctgca acgtagatca caagcccagc aacaccaagg tggacaagac agttgagcgc     300

aaatgttgtg tcgagtgccc accgtgccca gcaccacctg tggcaggacc gtcagtcttc     360

ctcttccccc caaaacccaa ggacaccctc atgatctccc ggacccctga ggtcacgtgc     420

gtggtggtgg acgtgagcca cgaagacccc gaggtccagt tcaactggta cgtggacggc     480

atggaggtgc ataatgccaa gacaaagcca cgggaggagc agttcaacag cacgttccgt     540

gtggtcagcg tcctcaccgt cgtgcaccag gactggctga acggcaagga gtacaagtgc     600

aaggtctcca acaaaggcct cccagccccc atcgagaaaa ccatctccaa aaccaaaggg 660

cagccccgag aaccacaggt gtacaccctg cccccatccc gggaggagat gaccaagaac 720

caggtcagcc tgacctgcct ggtcaaaggc ttctacccca gcgacatctc cgtggagtgg 780

gagagcaatg ggcagccgga gaacaactac aagaccacac ctcccatgct ggactccgac 840

ggctccttct tcctctacag caagctcacc gtggacaaga gcaggtggca gcaggggaac 900

gtcttctcat gctccgtgat gcatgaggct ctgcacaacc actacacaca gaagagcctc 960

tccctgtctc cgggtaaatg a 981

<210> 372
<211> 1739
<212> DNA
<213> homo sapiens;

<400> 372
gcctccacca agggcccatc ggtcttcccc ctggcgccct gctccaggag cacctccgag 60

agcacagcgg ccctgggctg cctggtcaag gactacttcc ccgaaccggt gacggtgtcg 120

tggaactcag gcgctctgac cagcggcgtg cacaccttcc cggctgtcct acagtcctca 180

ggactctact ccctcagcag cgtggtgacc gtgacctcca gcaacttcgg cacccagacc 240

tacacctgca acgtagatca caagcccagc aacaccaagg tggacaagac agttggtgag 300

aggccagctc agggagggag ggtgtctgct ggaagccagg ctcagccctc ctgcctggac 360

gcaccccggc tgtgcagccc cagcccaggg cagcaaggca ggccccatct gtctcctcac 420

ccggaggcct ctgcccgccc cactcatgct cagggagagg gtcttctggc tttttccacc 480

aggctccagg caggcacagg ctgggtgccc ctaccccagg cccttcacac acaggggcag 540

gtgcttggct cagacctgcc aaaagccata tccgggagga ccctgcccct gacctaagcc 600

gaccccaaag gccaaactgt ccactccctc agctcggaca ccttctctcc tcccagatcc 660

gagtaactcc caatcttctc tctgcagagc gcaaatgttg tgtcgagtgc ccaccgtgcc 720

caggtaagcc agcccaggcc tcgccctcca gctcaaggcg ggacaggtgc cctagagtag 780

cctgcatcca gggacagacc ccagctgggt gctgacacgt ccacctccat ctcttcctca 840

gcaccacctg tggcaggacc gtcagtcttc ctcttccccc caaaacccaa ggacaccctc 900

atgatctccc ggacccctga ggtcacgtgc gtggtggtgg acgtgagcca cgaagacccc 960

gaggtccagt tcaactggta cgtggacggc atggaggtgc ataatgccaa gacaaagcca 1020

cgggaggagc agttcaacag cacgttccgt gtggtcagcg tcctcaccgt cgtgcaccag 1080

gactggctga acggcaagga gtacaagtgc aaggtctcca acaaaggcct cccagccccc 1140

atcgagaaaa ccatctccaa aaccaaaggt gggacccgcg gggtatgagg gccacatgga 1200

cagaggccgg ctcggcccac cctctgccct gggagtgacc gctgtgccaa cctctgtccc 1260

tacagggcag ccccgagaac cacaggtgta caccctgccc ccatcccggg aggagatgac 1320

caagaaccag gtcagcctga cctgcctggt caaaggcttc taccccagcg acatctccgt 1380

ggagtgggag agcaatgggc agccggagaa caactacaag accacacctc ccatgctgga 1440

ctccgacggc tccttcttcc tctacagcaa gctcaccgtg gacaagagca ggtggcagca 1500

ggggaacgtc ttctcatgct ccgtgatgca tgaggctctg cacaaccact acacacagaa 1560

gagcctctcc ctgtctccgg gtaaatgagt gccacggccg gcaagccccc gctccccagg 1620

ctctcggggt cgcgcgagga tgcttggcac gtaccccgtc tacatacttc ccgggcaccc 1680

agcatggaaa taaagcaccc agcgctgccc tgggcccctg cgagactgtg atggttctt 1739

<210> 373
<211> 2304
<212> DNA
<213> homo sapiens;

<400> 373
cttccaccaa gggcccatcg gtcttccccc tggcgccctg ctccaggagc acctctgggg 60

gcacagcggc cctgggctgc ctggtcaagg actacttccc agaaccggtg acggtgtcgt 120

ggaactcagg cgccctgacc agcggcgtgc acaccttccc ggctgtccta cagtcctcag 180

gactctactc cctcagcagc gtggtgaccg tgccctccag cagcttgggc acccagacct 240

acacctgcaa cgtgaatcac aagcccagca acaccaaggt ggacaagaga gttggtgaga 300

ggccagcgca gggagggagg gtgtctgctg gaagccaggc tcagccctcc tgcctggacg 360

catcccggct gtgcagtccc agcccagggc accaaggcag gccccgtctg actcctcacc 420

cggaggcctc tgcccgcccc actcatgctc agggagaggg tcttctggct ttttccacca 480

ggctccgggc aggcacaggc tggatgcccc taccccaggc ccttcacaca caggggcagg 540

tgctgcgctc agagctgcca agagccatat ccaggaggac cctgcccctg acctaagccc 600

accccaaagg ccaaactctc tactcactca gctcagatac cttctctctt cccagatctg 660

agtaactccc aatcttctct ctgcagagct caaaacccca cttggtgaca caactcacac 720

atgcccacgg tgcccaggta agccagccca ggcctcgccc tccagctcaa ggcgggacaa 780

gagccctaga gtggcctgag tccagggaca ggccccagca gggtgctgac gcatccacct 840

ccatcccaga tccccgtaac tcccaatctt ctctctgcag agcccaaatc ttgtgacaca 900

cctcccccgt gcccacggtg cccaggtaag ccagcccagg cctcgccctc cagctcaagg 960

caggacaaga gccctagagt ggcctgagtc cagggacagg ccccagcagg gtgctgacgc 1020

gtccacctcc atcccagatc cccgtaactc ccaatcttct ctctgcagag cccaaatctt 1080

gtgacacacc tcccccatgc ccacggtgcc caggtaagcc agcccaggcc tcgccctcca 1140

gctcaaggcg ggacaagagc cctagagtgg cctgagtcca gggacaggcc ccagcagggt 1200

gctgacgcat ccacctccat cccagatccc cgtaactccc aatcttctct ctgcagagcc 1260

caaatcttgt gacacacctc ccccgtgccc aaggtgccca ggtaagccag cccaggcctc 1320

gccctccagc tcaaggcagg acaggtgccc tagagtggcc tgcatccagg gacaggtccc 1380

agtcgggtgc tgacacatct gcctccatct cttcctcagc acctgaactc ctgggaggac 1440

cgtcagtctt cctcttcccc ccaaaaccca aggataccct tatgatttcc cggacccctg 1500

aggtcacgtg cgtggtggtg gacgtgagcc acgaagaccc cgaggtccag ttcaagtggt 1560

acgtggacgg cgtggaggtg cataatgcca agacaaagcc gcgggaggag cagtacaaca 1620

gcacgttccg tgtggtcagc gtcctcaccg tcctgcacca ggactggctg aacggcaagg 1680

agtacaagtg caaggtctcc aacaaagccc tcccagcccc catcgagaaa accatctcca 1740

aaaccaaagg tgggacccgc ggggtatgag ggccacatgg acagaggcca gcttgaccca 1800

ccctctgccc tgggagtgac cgctgtgcca acctctgtcc ctacaggaca gccccgagaa 1860

ccacaggtgt acaccctgcc cccatcccgg gaggagatga ccaagaacca ggtcagcctg 1920

acctgcctgg tcaaaggctt ctaccccagc gacatcgccg tggagtggga gagcagcggg 1980

cagccggaga acaactacaa caccacgcct cccatgctgg actccgacgg ctccttcttc 2040

ctctacagca agctcaccgt ggacaagagc aggtggcagc aggggaacat cttctcatgc 2100

tccgtgatgc atgaggctct gcacaaccgc ttcacgcaga agagcctctc cctgtctccg 2160

ggtaaatgag tgcgacggcc ggcaagcccc cgctccccgg gctctcgggg tcgcgcgagg 2220

atgcttggca cgtaccccgt gtacatactt cccgggcacc cagcatggaa ataaagcacc 2280

cagcgctgcc ctgggcccct gcga 2304

<210> 374
<211> 1134
<212> DNA
<213> homo sapiens;

<220>
<221> misc_feature
<222> (1)..(1)
<223> n is a, c, g, or t

<400> 374
ncttccacca agggcccatc ggtcttcccc ctggcgccct gctccaggag cacctctggg 60

ggcacagcgg ccctgggctg cctggtcaag gactacttcc cagaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacacctgca acgtgaatca caagcccagc aacaccaagg tggacaagag agttgagctc 300

aaaaccccac ttggtgacac aactcacaca tgcccacggt gcccagagcc caaatcttgt 360

gacacacctc ccccgtgccc acggtgccca gagcccaaat cttgtgacac acctccccca 420

tgcccacggt gcccagagcc caaatcttgt gacacacctc ccccgtgccc aaggtgccca 480

gcacctgaac tcctgggagg accgtcagtc ttcctcttcc ccccaaaacc caaggatacc 540

cttatgattt cccggacccc tgaggtcacg tgcgtggtgg tggacgtgag ccacgaagac 600

cccgaggtcc agttcaagtg gtacgtggac ggcgtggagg tgcataatgc caagacaaag 660

ccgcgggagg agcagtacaa cagcacgttc cgtgtggtca gcgtcctcac cgtcctgcac 720

caggactggc tgaacggcaa ggagtacaag tgcaaggtct ccaacaaagc cctcccagcc 780

cccatcgaga aaaccatctc caaaaccaaa ggacagcccc gagaaccaca ggtgtacacc 840

ctgcccccat cccgggagga gatgaccaag aaccaggtca gcctgacctg cctggtcaaa 900

ggcttctacc ccagcgacat cgccgtggag tgggagagca gcgggcagcc ggagaacaac 960

tacaacacca cgcctcccat gctggactcc gacggctcct tcttcctcta cagcaagctc 1020

accgtggaca agagcaggtg gcagcagggg aacatcttct catgctccgt gatgcatgag 1080

gctctgcaca accgcttcac gcagaagagc ctctccctgt ctccgggtaa atga 1134

<210> 375
<211> 1134
<212> DNA
<213> homo sapiens;

<220>
<221> misc_feature
<222> (1)..(1)
<223> n is a, c, g, or t

<400> 375
ncttccacca agggcccatc ggtcttcccc ctggcgccct gctccaggag cacctctggg 60

ggcacagcgg ccctgggctg cctggtcaag gactacttcc cagaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacacctgca acgtgaatca caagcccagc aacaccaagg tggacaagag agttgagctc 300

aaaaccccac ttggtgacac aactcacaca tgcccacggt gcccagagcc caaatcttgt 360

gacacacctc ccccgtgccc acggtgccca gagcccaaat cttgtgacac acctccccca 420

tgcccacggt gcccagagcc caaatcttgt gacacacctc ccccgtgccc aaggtgccca 480

gcacctgaac tcctgggagg accgtcagtc ttcctcttcc ccccaaaacc caaggatacc 540

cttatgattt cccggacccc tgaggtcacg tgcgtggtgg tggacgtgag ccacgaagac 600

cccgaggtcc agttcaagtg gtacgtggac ggcgtggagg tgcataatgc caagacaaag 660

ctgcgggagg agcagtacaa cagcacgttc cgtgtggtca gcgtcctcac cgtcctgcac 720

caggactggc tgaacggcaa ggagtacaag tgcaaggtct ccaacaaagc cctcccagcc 780

cccatcgaga aaaccatctc caaaaccaaa ggacagcccc gagaaccaca ggtgtacacc 840

ctgcccccat cccgggagga gatgaccaag aaccaggtca gcctgacctg cctggtcaaa 900

ggcttctacc ccagcgacat cgccgtggag tgggagagca gcgggcagcc ggagaacaac 960

tacaacacca cgcctcccat gctggactcc gacggctcct tcttcctcta cagcaagctc 1020

accgtggaca agagcaggtg gcagcagggg aacatcttct catgctccgt gatgcatgag 1080

gctctgcaca accgctacac gcagaagagc ctctccctgt ctccgggtaa atga 1134

<210> 376
<211> 2304
<212> DNA
<213> homo sapiens;

<400> 376
cttccaccaa gggcccatcg gtcttccccc tggcgccctg ctccaggagc acctctgggg 60

gcacagcggc cctgggctgc ctggtcaagg actacttccc agaaccggtg acggtgtcgt 120

ggaactcagg cgccctgacc agcggcgtgc acaccttccc ggctgtccta cagtcctcag 180

gactctactc cctcagcagc gtggtgaccg tgccctccag cagcttgggc acccagacct 240

acacctgcaa cgtgaatcac aagcccagca acaccaaggt ggacaagaga gttggtgaga 300

ggccagcgca gggagggagg gtgtctgctg gaagccaggc tcagccctcc tgcctggacg 360

catcccggct gtgcagtccc agcccagggc accaaggcag gccccgtctg actcctcacc 420

cggaggcctc tgcccgcccc actcatgctc agggagaggg tcttctggct ttttccacca 480

ggctccgggc aggcacaggc tggatgcccc taccccaggc ccttcacaca caggggcagg 540

tgctgcgctc agagctgcca agagccatat ccaggaggac cctgcccctg acctaagccc 600

accccaaagg ccaaactctc tactcactca gctcagatac cttctctctt cccagatctg 660

agtaactccc aatcttctct ctgcagagct caaaacccca cttggtgaca caactcacac 720

atgcccacgg tgcccaggta agccagccca ggcctcgccc tccagctcaa ggcgggacaa 780

gagccctaga gtggcctgag tccagggaca ggccccagca gggtgctgac gcatccacct 840

ccatcccaga tccccgtaac tcccaatctt ctctctgcag agcccaaatc ttgtgacaca 900

cctcccccgt gcccacggtg cccaggtaag ccagcccagg cctcgccctc cagctcaagg 960

caggacaaga gccctagagt ggcctgagtc cagggacagg ccccagcagg gtgctgacgc 1020

gtccacctcc atcccagatc cccgtaactc ccaatcttct ctctgcagag cccaaatctt 1080

gtgacacacc tcccccatgc ccacggtgcc caggtaagcc agcccaggcc tcgccctcca 1140

gctcaaggcg ggacaagagc cctagagtgg cctgagtcca gggacaggcc ccagcagggt 1200

gctgacgcat ccacctccat cccagatccc cgtaactccc aatcttctct ctgcagagcc 1260

caaatcttgt gacacacctc ccccgtgccc aaggtgccca ggtaagccag cccaggcctc 1320

gccctccagc tcaaggcagg acaggtgccc tagagtggcc tgcatccagg gacaggtccc 1380

agtcgggtgc tgacacatct gcctccatct cttcctcagc acctgaactc ctgggaggac 1440

cgtcagtctt cctcttcccc ccaaaaccca aggataccct tatgatttcc cggacccctg 1500

aggtcacgtg cgtggtggtg gacgtgagcc acgaagaccc cgaggtccag ttcaagtggt 1560

acgtggacgg cgtggaggtg cataatgcca agacaaagct gcgggaggag cagtacaaca 1620

gcacgttccg tgtggtcagc gtcctcaccg tcctgcacca ggactggctg aacggcaagg 1680

agtacaagtg caaggtctcc aacaaagccc tcccagcccc catcgagaaa accatctcca 1740

aaaccaaagg tgggacccgc ggggtatgag ggccacatgg acagaggcca gcttgaccca 1800

ccctctgccc tgggagtgac cgctgtgcca acctctgtcc ctacaggaca gccccgagaa 1860

ccacaggtgt acaccctgcc cccatcccgg gaggagatga ccaagaacca ggtcagcctg 1920

acctgcctgg tcaaaggctt ctaccccagc gacatcgccg tggagtggga gagcagcggg 1980

cagccggaga acaactacaa caccacgcct cccatgctgg actccgacgg ctccttcttc 2040

ctctacagca agctcaccgt ggacaagagc aggtggcagc aggggaacat cttctcatgc 2100

tccgtgatgc atgaggctct gcacaaccgc tacacgcaga agagcctctc cctgtctccg 2160

ggtaaatgag tgcgacggcc ggcaagcccc cgctccccgg gctctcgggg tcgcgcgagg 2220

atgcttggca cgtaccccgt gtacatactt cccgggcacc cagcatggaa ataaagcacc 2280

cagcgctgcc ctgggcccct gcga 2304

<210> 377
<211> 1134
<212> DNA
<213> homo sapiens;

<220>
<221> misc_feature
<222> (1)..(1)
<223> n is a, c, g, or t

<400> 377
ncttccacca agggcccatc ggtcttcccc ctggcgccct gctccaggag cacctctggg 60

ggcacagcgg ccctgggctg cctggtcaag gactacttcc cagaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacacctgca acgtgaatca caagcccagc aacaccaagg tggacaagag agttgagctc 300

aaaaccccac ttggtgacac aactcacaca tgcccacggt gcccagagcc caaatcttgt 360

gacacacctc ccccgtgccc acggtgccca gagcccaaat cttgtgacac acctccccca 420

tgcccacggt gcccagagcc caaatcttgt gacacacctc ccccgtgccc aaggtgccca 480

gcacctgaac tcctgggagg accgtcagtc ttcctcttcc ccccaaaacc caaggatacc 540

cttatgattt cccggacccc tgaggtcacg tgcgtggtgg tggacgtgag ccacgaagac 600

cccgaggtcc agttcaagtg gtacgtggac ggcgtggagg tgcataatgc caagacaaag 660

ccgcgggagg agcagttcaa cagcacgttc cgtgtggtca gcgtcctcac cgtcctgcac 720

caggactggc tgaacggcaa ggagtacaag tgcaaggtct ccaacaaagc cctcccagcc 780

cccatcgaga aaaccatctc caaaaccaaa ggacagcccc gagaaccaca ggtgtacacc 840

ctgcccccat cccgggagga gatgaccaag aaccaggtca gcctgacctg cctggtcaaa 900

ggcttctacc ccagcgacat cgccgtggag tgggagagca gcgggcagcc ggagaacaac 960

tacaacacca cgcctcccat gctggactcc gacggctcct tcttcctcta cagcaagctc 1020

accgtggaca agagcaggtg gcagcagggg aacatcttct catgctccgt gatgcatgag 1080

gctctgcaca accgcttcac gcagaagagc ctctccctgt ctccgggtaa atga 1134

<210> 378
<211> 2304
<212> DNA
<213> homo sapiens;

<400> 378
cttccaccaa gggcccatcg gtcttccccc tggcgccctg ctccaggagc acctctgggg 60

gcacagcggc cctgggctgc ctggtcaagg actacttccc agaaccggtg acggtgtcgt 120

ggaactcagg cgccctgacc agcggcgtgc acaccttccc ggctgtccta cagtcctcag 180

gactctactc cctcagcagc gtggtgaccg tgccctccag cagcttgggc acccagacct 240

acacctgcaa cgtgaatcac aagcccagca acaccaaggt ggacaagaga gttggtgaga 300

ggccagcgca gggagggagg gtgtctgctg gaagccaggc tcagccctcc tgcctggacg 360

catcccggct gtgcagtccc agcccagggc accaaggcag gccccgtctg actcctcacc 420

cggaggcctc tgcccgcccc actcatgctc agggagaggg tcttctggct ttttccacca 480

ggctccgggc aggcacaggc tggatgcccc taccccaggc ccttcacaca caggggcagg 540

tgctgcgctc agagctgcca agagccatat ccaggaggac cctgcccctg acctaagccc 600

accccaaagg ccaaactctc tactcactca gctcagatac cttctctctt cccagatctg 660

agtaactccc aatcttctct ctgcagagct caaaacccca cttggtgaca caactcacac 720

atgcccacgg tgcccaggta agccagccca ggcctcgccc tccagctcaa ggcgggacaa 780

gagccctaga gtggcctgag tccagggaca ggccccagca gggtgctgac gcatccacct 840

ccatcccaga tccccgtaac tcccaatctt ctctctgcag agcccaaatc ttgtgacaca 900

cctcccccgt gcccacggtg cccaggtaag ccagcccagg cctcgccctc cagctcaagg 960

caggacaaga gccctagagt ggcctgagtc cagggacagg ccccagcagg gtgctgacgc 1020

gtccacctcc atcccagatc cccgtaactc ccaatcttct ctctgcagag cccaaatctt 1080

gtgacacacc tcccccatgc ccacggtgcc caggtaagcc agcccaggcc tcgccctcca 1140

gctcaaggcg ggacaagagc cctagagtgg cctgagtcca gggacaggcc ccagcagggt 1200

gctgacgcat ccacctccat cccagatccc cgtaactccc aatcttctct ctgcagagcc 1260

caaatcttgt gacacacctc ccccgtgccc aaggtgccca ggtaagccag cccaggcctc 1320

gccctccagc tcaaggcagg acaggtgccc tagagtggcc tgcatccagg gacaggtccc 1380

agtcgggtgc tgacacatct gcctccatct cttcctcagc acctgaactc ctgggaggac 1440

cgtcagtctt cctcttcccc ccaaaaccca aggataccct tatgatttcc cggacccctg 1500

aggtcacgtg cgtggtggtg gacgtgagcc acgaagaccc cgaggtccag ttcaagtggt 1560

acgtggacgg cgtggaggtg cataatgcca agacaaagcc gcgggaggag cagttcaaca 1620

gcacgttccg tgtggtcagc gtcctcaccg tcctgcacca ggactggctg aacggcaagg 1680

agtacaagtg caaggtctcc aacaaagccc tcccagcccc catcgagaaa accatctcca 1740

aaaccaaagg tgggacccgc ggggtatgag ggccacatgg acagaggcca gcttgaccca 1800

ccctctgccc tgggagtgac cgctgtgcca acctctgtcc ctacaggaca gccccgagaa 1860

ccacaggtgt acaccctgcc cccatcccgg gaggagatga ccaagaacca ggtcagcctg 1920

acctgcctgg tcaaaggctt ctaccccagc gacatcgccg tggagtggga gagcagcggg 1980

cagccggaga acaactacaa caccacgcct cccatgctgg actccgacgg ctccttcttc 2040

ctctacagca agctcaccgt ggacaagagc aggtggcagc aggggaacat cttctcatgc 2100

tccgtgatgc atgaggctct gcacaaccgc ttcacgcaga agagcctctc cctgtctccg 2160

ggtaaatgag tgcgacggcc ggcaagcccc cgctccccgg gctctcgggg tcgcgcgagg 2220

atgcttggca cgtaccccgt gtacatactt cccgggcacc cagcatggaa ataaagcacc 2280

cagcgctgcc ctgggcccct gcga 2304

<210> 379
<211> 1717
<212> DNA
<213> homo sapiens;

<400> 379
gcttccacca agggcccatc ggtcttcccc ctggcgccct gctccaggag cacctccgag 60

agcacagccg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacgaagacc 240

tacacctgca acgtagatca caagcccagc aacaccaagg tggacaagag agttggtgag 300

aggccagcac agggagggag ggtgtctgct ggaagccagg ctcagccctc ctgcctggac 360

gcaccccggc tgtgcagccc cagcccaggg cagcaaggca ggccccatct gtctcctcac 420

ccggaggcct ctgaccaccc cactcatgct cagggagagg gtcttctgga tttttccacc 480

aggctccggg cagccacagg ctggatgccc ctaccccagg ccctgcgcat acaggggcag 540

gtgctgcgct cagacctgcc aagagccata tccgggagga ccctgcccct gacctaagcc 600

caccccaaag gccaaactct ccactccctc agctcagaca ccttctctcc tcccagatct 660

gagtaactcc caatcttctc tctgcagagt ccaaatatgg tcccccatgc ccatcatgcc 720

caggtaagcc aacccaggcc tcgccctcca gctcaaggcg ggacaggtgc cctagagtag 780

cctgcatcca gggacaggcc ccagccgggt gctgacgcat ccacctccat ctcttcctca 840

gcacctgagt tcctgggggg accatcagtc ttcctgttcc ccccaaaacc caaggacact 900

ctcatgatct cccggacccc tgaggtcacg tgcgtggtgg tggacgtgag ccaggaagac 960

cccgaggtcc agttcaactg gtacgtggat ggcgtggagg tgcataatgc caagacaaag 1020

ccgcgggagg agcagttcaa cagcacgtac cgtgtggtca gcgtcctcac cgtcctgcac 1080

caggactggc tgaacggcaa ggagtacaag tgcaaggtct ccaacaaagg cctcccgtcc 1140

tccatcgaga aaaccatctc caaagccaaa ggtgggaccc acggggtgcg agggccacat 1200

ggacagaggt cagctcggcc caccctctgc cctgggagtg accgctgtgc caacctctgt 1260

ccctacaggg cagccccgag agccacaggt gtacaccctg cccccatccc aggaggagat 1320

gaccaagaac caggtcagcc tgacctgcct ggtcaaaggc ttctacccca gcgacatcgc 1380

cgtggagtgg gagagcaatg ggcagccgga gaacaactac aagaccacgc ctcccgtgct 1440

ggactccgac ggctccttct tcctctacag caggctcacc gtggacaaga gcaggtggca 1500

ggaggggaat gtcttctcat gctccgtgat gcatgaggct ctgcacaacc actacacaca 1560

gaagagcctc tccctgtctc tgggtaaatg agtgccaggg ccggcaagcc cccgctcccc 1620

gggctctcgg ggtcgcgcga ggatgcttgg cacgtacccc gtgtacatac ttcccgggcg 1680

cccagcatgg aaataaagca cccagcgctg ccctggg 1717

<210> 380
<211> 984
<212> DNA
<213> homo sapiens;

<400> 380
gcttccacca agggcccatc ggtcttcccc ctggcgccct gctccaggag cacctccgag 60

agcacagccg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacgaagacc 240

tacacctgca acgtagatca caagcccagc aacaccaagg tggacaagag agttgagtcc 300

aaatatggtc ccccatgccc atcatgccca gcacctgagt tcctgggggg accatcagtc 360

ttcctgttcc ccccaaaacc caaggacact ctcatgatct cccggacccc tgaggtcacg 420

tgcgtggtgg tggacgtgag ccaggaagac cccgaggtcc agttcaactg gtacgtggat 480

ggcgtggagg tgcataatgc caagacaaag ccgcgggagg agcagttcaa cagcacgtac 540

cgtgtggtca gcgtcctcac cgtcctgcac caggactggc tgaacggcaa ggagtacaag 600

tgcaaggtct ccaacaaagg cctcccgtcc tccatcgaga aaaccatctc caaagccaaa 660

gggcagcccc gagagccaca ggtgtacacc ctgcccccat cccaggagga gatgaccaag 720

aaccaggtca gcctgacctg cctggtcaaa ggcttctacc ccagcgacat cgccgtggag 780

tgggagagca atgggcagcc ggagaacaac tacaagacca cgcctcccgt gctggactcc 840

gacggctcct tcttcctcta cagcaggctc accgtggaca agagcaggtg gcaggagggg 900

aatgtcttct catgctccgt gatgcatgag gctctgcaca accactacac acagaagagc 960

ctctccctgt ctctgggtaa atga 984

<210> 381
<211> 984
<212> DNA
<213> homo sapiens;

<400> 381
gcttccacca agggcccatc ggtcttcccc ctggcgccct gctccaggag cacctccgag 60

agcacagccg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacgaagacc 240

tacacctgca acgtagatca caagcccagc aacaccaagg tggacaagag agttgagtcc 300

aaatatggtc ccccatgccc atcatgccca gcacctgagt tcctgggggg accatcagtc 360

ttcctgttcc ccccaaaacc caaggacact ctcatgatct cccggacccc tgaggtcacg 420

tgcgtggtgg tggacgtgag ccaggaagac cccgaggtcc agttcaactg gtacgtggat 480

ggcgtggagg tgcataatgc caagacaaag ccgcgggagg agcagttcaa cagcacgtac 540

cgtgtggtca gcgtcctcac cgtcgtgcac caggactggc tgaacggcaa ggagtacaag 600

tgcaaggtct ccaacaaagg cctcccgtcc tccatcgaga aaaccatctc caaagccaaa 660

gggcagcccc gagagccaca ggtgtacacc ctgcccccat cccaggagga gatgaccaag 720

aaccaggtca gcctgacctg cctggtcaaa ggcttctacc ccagcgacat cgccgtggag 780

tgggagagca atgggcagcc ggagaacaac tacaagacca cgcctcccgt gctggactcc 840

gacggctcct tcttcctcta cagcaggctc accgtggaca agagcaggtg gcaggagggg 900

aatgtcttct catgctccgt gatgcatgag gctctgcaca accactacac acagaagagc 960

ctctccctgt ctctgggtaa atga 984

<210> 382
<211> 1717
<212> DNA
<213> homo sapiens;

<400> 382
gcttccacca agggcccatc ggtcttcccc ctggcgccct gctccaggag cacctccgag 60

agcacagccg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacgaagacc 240

tacacctgca acgtagatca caagcccagc aacaccaagg tggacaagag agttggtgag 300

aggccagcac agggagggag ggtgtctgct ggaagccagg ctcagccctc ctgcctggac 360

gcaccccggc tgtgcagccc cagcccaggg cagcaaggca ggccccatct gtctcctcac 420

ccggaggcct ctgaccaccc cactcatgct cagggagagg gtcttctgga tttttccacc 480

aggctccggg cagccacagg ctggatgccc ctaccccagg ccctgcgcat acaggggcag 540

gtgctgcgct cagacctgcc aagagccata tccgggagga ccctgcccct gacctaagcc 600

caccccaaag gccaaactct ccactccctc agctcagaca ccttctctcc tcccagatct 660

gagtaactcc caatcttctc tctgcagagt ccaaatatgg tcccccatgc ccatcatgcc 720

caggtaagcc aacccaggcc tcgccctcca gctcaaggcg ggacaggtgc cctagagtag 780

cctgcatcca gggacaggcc ccagccgggt gctgacgcat ccacctccat ctcttcctca 840

gcacctgagt tcctgggggg accatcagtc ttcctgttcc ccccaaaacc caaggacact 900

ctcatgatct cccggacccc tgaggtcacg tgcgtggtgg tggacgtgag ccaggaagac 960

cccgaggtcc agttcaactg gtacgtggat ggcgtggagg tgcataatgc caagacaaag 1020

ccgcgggagg agcagttcaa cagcacgtac cgtgtggtca gcgtcctcac cgtcgtgcac 1080

caggactggc tgaacggcaa ggagtacaag tgcaaggtct ccaacaaagg cctcccgtcc 1140

tccatcgaga aaaccatctc caaagccaaa ggtgggaccc acggggtgcg agggccacat 1200

ggacagaggt cagctcggcc caccctctgc cctgggagtg accgctgtgc caacctctgt 1260

ccctacaggg cagccccgag agccacaggt gtacaccctg cccccatccc aggaggagat 1320

gaccaagaac caggtcagcc tgacctgcct ggtcaaaggc ttctacccca gcgacatcgc 1380

cgtggagtgg gagagcaatg ggcagccgga gaacaactac aagaccacgc ctcccgtgct 1440

ggactccgac ggctccttct tcctctacag caggctcacc gtggacaaga gcaggtggca 1500

ggaggggaat gtcttctcat gctccgtgat gcatgaggct ctgcacaacc actacacaca 1560

gaagagcctc tccctgtctc tgggtaaatg agtgccaggg ccggcaagcc cccgctcccc 1620

gggctctcgg ggtcgcgcga ggatgcttgg cacgtacccc gtgtacatac ttcccgggcg 1680

cccagcatgg aaataaagca cccagcgctg ccctggg 1717

<210> 383
<211> 1546
<212> DNA
<213> homo sapiens;

<400> 383
gcatccccga ccagccccaa ggtcttcccg ctgagcctct gcagcaccca gccagatggg 60

aacgtggtca tcgcctgcct ggtccagggc ttcttccccc aggagccact cagtgtgacc 120

tggagcgaaa gcggacaggg cgtgaccgcc agaaacttcc cacccagcca ggatgcctcc 180

ggggacctgt acaccacgag cagccagctg accctgccgg ccacacagtg cctagccggc 240

aagtccgtga catgccacgt gaagcactac acgaatccca gccaggatgt gactgtgccc 300

tgcccaggtc agagggcagg ctggggagtg gggcggggcc accccgtcgt gccctgacac 360

tgcgcctgca cccgtgttcc ccacagggag ccgccccttc actcacacca gagtggaccg 420

cgggccgagc cccaggaggt ggtggtggac aggccaggag gggcgaggcg ggggcatggg 480

gaagtatgtg ctgaccagct caggccatct ctccactcca gttccctcaa ctccacctac 540

cccatctccc tcaactccac ctaccccatc tccctcatgc tgccaccccc gactgtcact 600

gcaccgaccg gccctcgagg acctgctctt aggttcagaa gcgaacctca cgtgcacact 660

gaccggcctg agagatgcct caggtgtcac cttcacctgg acgccctcaa gtgggaagag 720

cgctgttcaa ggaccacctg agcgtgacct ctgtggctgc tacagcgtgt ccagtgtcct 780

gccgggctgt gccgagccat ggaaccatgg gaagaccttc acttgcactg ctgcctaccc 840

cgagtccaag accccgctaa ccgccaccct ctcaaaatcc ggtgggtcca gaccctgctc 900

ggggccctgc tcagtgctct ggtttgcaaa gcatattcct ggcctgcctc ctccctccca 960

atcctgggct ccagtgctca tgccaagtac agagggaaac tgaggcaggc tgaggggcca 1020

ggacacagcc cagggtgccc accagagcag aggggctctc tcatcccctg cccagccccc 1080

tgacctggct ctctaccctc caggaaacac attccggccc gaggtccacc tgctgccgcc 1140

gccgtcggag gagctggccc tgaacgagct ggtgacgctg acgtgcctgg cacgcggctt 1200

cagccccaag gatgtgctgg ttcgctggct gcaggggtca caggagctgc cccgcgagaa 1260

gtacctgact tgggcatccc ggcaggagcc cagccagggc accaccacct tcgctgtgac 1320

cagcatactg cgcgtggcag ccgaggactg gaagaagggg gacaccttct cctgcatggt 1380

gggccacgag gccctgccgc tggccttcac acagaagacc atcgaccgct tggcgggtaa 1440

acccacccat gtcaatgtgt ctgttgtcat ggcggaggtg gacggcacct gctactgagc 1500

cgcccgcctg tccccacccc tgaataaact ccatgctccc ccaagc 1546

<210> 384
<211> 1062
<212> DNA
<213> homo sapiens;

<400> 384
gcatccccga ccagccccaa ggtcttcccg ctgagcctct gcagcaccca gccagatggg 60

aacgtggtca tcgcctgcct ggtccagggc ttcttccccc aggagccact cagtgtgacc 120

tggagcgaaa gcggacaggg cgtgaccgcc agaaacttcc cacccagcca ggatgcctcc 180

ggggacctgt acaccacgag cagccagctg accctgccgg ccacacagtg cctagccggc 240

aagtccgtga catgccacgt gaagcactac acgaatccca gccaggatgt gactgtgccc 300

tgcccagttc cctcaactcc acctacccca tctccctcaa ctccacctac cccatctccc 360

tcatgctgcc acccccgact gtcactgcac cgaccggccc tcgaggacct gctcttaggt 420

tcagaagcga acctcacgtg cacactgacc ggcctgagag atgcctcagg tgtcaccttc 480

acctggacgc cctcaagtgg gaagagcgct gttcaaggac cacctgagcg tgacctctgt 540

ggctgctaca gcgtgtccag tgtcctgccg ggctgtgccg agccatggaa ccatgggaag 600

accttcactt gcactgctgc ctaccccgag tccaagaccc cgctaaccgc caccctctca 660

aaatccggaa acacattccg gcccgaggtc cacctgctgc cgccgccgtc ggaggagctg 720

gccctgaacg agctggtgac gctgacgtgc ctggcacgcg gcttcagccc caaggatgtg 780

ctggttcgct ggctgcaggg gtcacaggag ctgccccgcg agaagtacct gacttgggca 840

tcccggcagg agcccagcca gggcaccacc accttcgctg tgaccagcat actgcgcgtg 900

gcagccgagg actggaagaa gggggacacc ttctcctgca tggtgggcca cgaggccctg 960

ccgctggcct tcacacagaa gaccatcgac cgcttggcgg gtaaacccac ccatgtcaat 1020

gtgtctgttg tcatggcgga ggtggacggc acctgctact ga 1062

<210> 385
<211> 1546
<212> DNA
<213> homo sapiens;

<400> 385
gcatccccga ccagccccaa ggtcttcccg ctgagcctct gcagcaccca gccagatggg 60

aacgtggtca tcgcctgcct ggtccagggc ttcttccccc aggagccact cagtgtgacc 120

tggagcgaaa gcggacaggg cgtgaccgcc agaaacttcc cacccagcca ggatgcctcc 180

ggggacctgt acaccacgag cagccagctg accctgccgg ccacacagtg cctagccggc 240

aagtccgtga catgccacgt gaagcactac acgaatccca gccaggatgt gactgtgccc 300

tgcccaggtc agagggcagg ctggggagtg gggcggggcc accccgtcgt gccctgacac 360

tgcgcctgca cccgtgttcc ccacagggag ccgccccttc actcacacca gagtggaccg 420

cgggccgagc cccaggaggt ggtggtggac aggccaggag gggcgaggcg ggggcatggg 480

gaagtatgtg ctgaccagct caggccatct ctccactcca gttccctcaa ctccacctac 540

cccatctccc tcaactccac ctaccccatc tccctcatgc tgccaccccc gactgtcact 600

gcaccgaccg gccctcgagg acctgctctt aggttcagaa gcgaacctca cgtgcacact 660

gaccggcctg agagatgcct caggtgtcac cttcacctgg acgccctcaa gtgggaagag 720

cgctgttcaa ggaccacctg accgtgacct ctgtggctgc tacagcgtgt ccagtgtcct 780

gccgggctgt gccgagccat ggaaccatgg gaagaccttc acttgcactg ctgcctaccc 840

cgagtccaag accccgctaa ccgccaccct ctcaaaatcc ggtgggtcca gaccctgctc 900

ggggccctgc tcagtgctct ggtttgcaaa gcatattcct ggcctgcctc ctccctccca 960

atcctgggct ccagtgctca tgccaagtac agagggaaac tgaggcaggc tgaggggcca 1020

ggacacagcc cagggtgccc accagagcag aggggctctc tcatcccctg cccagccccc 1080

tgacctggct ctctaccctc caggaaacac attccggccc gaggtccacc tgctgccgcc 1140

gccgtcggag gagctggccc tgaacgagct ggtgacgctg acgtgcctgg cacgcggctt 1200

cagccccaag gatgtgctgg ttcgctggct gcaggggtca caggagctgc cccgcgagaa 1260

gtacctgact tgggcatccc ggcaggagcc cagccagggc accaccacct tcgctgtgac 1320

cagcatactg cgcgtggcag ccgaggactg gaagaagggg gacaccttct cctgcatggt 1380

gggccacgag gccctgccgc tggccttcac acagaagacc atcgaccgct tggcgggtaa 1440

acccacccat gtcaatgtgt ctgttgtcat ggcggaggtg gacggcacct gctactgagc 1500

cgcccgcctg tccccacccc tgaataaact ccatgctccc ccaagc 1546

<210> 386
<211> 1062
<212> DNA
<213> homo sapiens;

<400> 386
gcatccccga ccagccccaa ggtcttcccg ctgagcctct gcagcaccca gccagatggg 60

aacgtggtca tcgcctgcct ggtccagggc ttcttccccc aggagccact cagtgtgacc 120

tggagcgaaa gcggacaggg cgtgaccgcc agaaacttcc cacccagcca ggatgcctcc 180

ggggacctgt acaccacgag cagccagctg accctgccgg ccacacagtg cctagccggc 240

aagtccgtga catgccacgt gaagcactac acgaatccca gccaggatgt gactgtgccc 300

tgcccagttc cctcaactcc acctacccca tctccctcaa ctccacctac cccatctccc 360

tcatgctgcc acccccgact gtcactgcac cgaccggccc tcgaggacct gctcttaggt 420

tcagaagcga acctcacgtg cacactgacc ggcctgagag atgcctcagg tgtcaccttc 480

acctggacgc cctcaagtgg gaagagcgct gttcaaggac cacctgaccg tgacctctgt 540

ggctgctaca gcgtgtccag tgtcctgccg ggctgtgccg agccatggaa ccatgggaag 600

accttcactt gcactgctgc ctaccccgag tccaagaccc cgctaaccgc caccctctca 660

aaatccggaa acacattccg gcccgaggtc cacctgctgc cgccgccgtc ggaggagctg 720

gccctgaacg agctggtgac gctgacgtgc ctggcacgcg gcttcagccc caaggatgtg 780

ctggttcgct ggctgcaggg gtcacaggag ctgccccgcg agaagtacct gacttgggca 840

tcccggcagg agcccagcca gggcaccacc accttcgctg tgaccagcat actgcgcgtg 900

gcagccgagg actggaagaa gggggacacc ttctcctgca tggtgggcca cgaggccctg 960

ccgctggcct tcacacagaa gaccatcgac cgcttggcgg gtaaacccac ccatgtcaat 1020

gtgtctgttg tcatggcgga ggtggacggc acctgctact ga 1062

<210> 387
<211> 1507
<212> DNA
<213> homo sapiens;

<400> 387
gcatccccga ccagccccaa ggtcttcccg ctgagcctcg acagcacccc ccaagatggg 60

aacgtggtcg tcgcatgcct ggtccagggc ttcttccccc aggagccact cagtgtgacc 120

tggagcgaaa gcggacagaa cgtgaccgcc agaaacttcc cacctagcca ggatgcctcc 180

ggggacctgt acaccacgag cagccagctg accctgccgg ccacacagtg cccagacggc 240

aagtccgtga catgccacgt gaagcactac acgaattcca gccaggatgt gactgtgccc 300

tgccgaggtc agagggcagg ctggggagtg gggcggggcc accccgtcct gccctgacac 360

tgcgcctgca cccgtgttcc ccacagggag ccgccccttc actcacacca gagtggaccg 420

cgggccgagc cccaggaggt ggtggtggac aggccaggag gggcgaggcg gggcacgggg 480

gaagggcgtt ctgaccagct caggccatct ctccactcca gttcccccac ctcccccatg 540

ctgccacccc cgactgtcgc tgcaccgacc ggccctcgag gacctgctct taggttcaga 600

agcgaacctc acgtgcacac tgaccggcct gagagatgcc tctggtgcca ccttcacctg 660

gacgccctca agtgggaaga gcgctgttca aggaccacct gagcgtgacc tctgtggctg 720

ctacagcgtg tccagtgtcc tgcctggctg tgcccagcca tggaaccatg ggagaccttt 780

cacctgcact gctgcccacc ccgagttgaa gaccccacta accgccaaca tcacaaaatc 840

cggtgggtcc agaccctgct cggggccctg ctcagtgctc tggtttgcaa agcatattcc 900

cggcctgcct cctccctccc aatcctgggc tccagtgctc atgccaagta cagagggaaa 960

ctgaggcagg ctgaggggcc aggacacagc ccagggtgcc caccagagca gaggggctct 1020

ctcatcccct gcccagcccc ctgacctggc tctctaccct ccaggaaaca cattccggcc 1080

cgaggtccac ctgctgccgc cgccgtcgga ggagctggcc ctgaacgagc tggtgacgct 1140

gacgtgcctg gcacgtggct tcagccccaa ggatgtgctg gttcgctggc tgcaggggtc 1200

acaggagctg ccccgcgaga agtacctgac ttgggcatcc cggcaggagc ccagccaggg 1260

caccaccacc tacgctgtaa ccagcatact gcgcgtggca gctgaggact ggaagaaggg 1320

ggagaccttc tcctgcatgg tgggccacga ggccctgccg ctggccttca cacagaagac 1380

catcgaccgc atggcgggta aacccaccca catcaatgtg tctgttgtca tggcggaggc 1440

ggatggcacc tgctactgag ccgcccgcct gtccccaccc ctgaataaac tccatgctcc 1500

cccaagc 1507

<210> 388
<211> 1023
<212> DNA
<213> homo sapiens;

<400> 388
gcatccccga ccagccccaa ggtcttcccg ctgagcctcg acagcacccc ccaagatggg 60

aacgtggtcg tcgcatgcct ggtccagggc ttcttccccc aggagccact cagtgtgacc 120

tggagcgaaa gcggacagaa cgtgaccgcc agaaacttcc cacctagcca ggatgcctcc 180

ggggacctgt acaccacgag cagccagctg accctgccgg ccacacagtg cccagacggc 240

aagtccgtga catgccacgt gaagcactac acgaattcca gccaggatgt gactgtgccc 300

tgccgagttc ccccacctcc cccatgctgc cacccccgac tgtcgctgca ccgaccggcc 360

ctcgaggacc tgctcttagg ttcagaagcg aacctcacgt gcacactgac cggcctgaga 420

gatgcctctg gtgccacctt cacctggacg ccctcaagtg ggaagagcgc tgttcaagga 480

ccacctgagc gtgacctctg tggctgctac agcgtgtcca gtgtcctgcc tggctgtgcc 540

cagccatgga accatgggga gaccttcacc tgcactgctg cccaccccga gttgaagacc 600

ccactaaccg ccaacatcac aaaatccgga aacacattcc ggcccgaggt ccacctgctg 660

ccgccgccgt cggaggagct ggccctgaac gagctggtga cgctgacgtg cctggcacgt 720

ggcttcagcc ccaaggatgt gctggttcgc tggctgcagg ggtcacagga gctgccccgc 780

gagaagtacc tgacttgggc atcccggcag gagcccagcc agggcaccac cacctacgct 840

gtaaccagca tactgcgcgt ggcagctgag gactggaaga agggggagac cttctcctgc 900

atggtgggcc acgaggccct gccgctggcc ttcacacaga agaccatcga ccgcatggcg 960

ggtaaaccca cccacatcaa tgtgtctgtt gtcatggcgg aggcggatgg cacctgctac 1020

tga 1023

<210> 389

<211>  1507
<212>  DNA
<213>  homo sapiens;

<400>  389
gcatccccga ccagccccaa ggtcttcccg ctgagcctcg acagcacccc ccaagatggg      60

aacgtggtcg tcgcatgcct ggtccagggc ttcttccccc aggagccact cagtgtgacc     120

tggagcgaaa gcggacagaa cgtgaccgcc agaaacttcc cacctagcca ggatgcctcc     180

ggggacctgt acaccacgag cagccagctg accctgccgg ccacacagtg cccagacggc     240

aagtccgtga catgccacgt gaagcactac acgaatccca gccaggatgt gactgtgccc     300

tgcccaggtc agagggcagg ctggggagtg gggcggggcc accccgtcct gccctgacac     360

tgcgcctgca cccgtgttcc ccacagggag ccgccccttc actcacacca gagtggaccg     420

cgggccgagc cccaggaggt ggtggtggac aggccaggag gggcgaggcg gggcacgggg     480

gaagggcgtt ctgaccagct caggccatct ctccactcca gttcccccac ctcccccatg     540

ctgccacccc cgactgtcgc tgcaccgacc ggccctcgag gacctgctct taggttcaga     600

agcgaacctc acgtgcacac tgaccggcct gagagatgcc tctggtgcca ccttcacctg     660

gacgccctca agtgggaaga gcgctgttca aggaccacct gagcgtgacc tctgtggctg     720

ctacagcgtg tccagtgtcc tgcctggctg tgcccagcca tggaaccatg gggagacctt     780

cacctgcact gctgcccacc ccgagttgaa gaccccacta accgccaaca tcacaaaatc     840

cggtgggtcc agaccctgct cggggccctg ctcagtgctc tggtttgcaa agcatattcc     900

cggcctgcct cctccctccc aatcctgggc tccagtgctc atgccaagta cagagggaaa     960

ctgaggcagg ctgaggggcc aggacacagc ccagggtgcc caccagagca gaggggctct    1020

ctcatcccct gcccagcccc ctgacctggc tctctaccct ccaggaaaca cattccggcc    1080

cgaggtccac ctgctgccgc cgccgtcgga ggagctggcc ctgaacgagc tggtgacgct    1140

gacgtgcctg gcacgtggct tcagccccaa ggatgtgctg gttcgctggc tgcaggggtc    1200

acaggagctg ccccgcgaga agtacctgac ttgggcatcc cggcaggagc ccagccaggg    1260

caccaccacc ttcgctgtaa ccagcatact gcgcgtggca gctgaggact ggaagaaggg    1320

ggacaccttc tcctgcatgg tgggccacga ggccctgccg ctggccttca cacagaagac    1380

catcgaccgc atggcgggta aacccaccca catcaatgtg tctgttgtca tggcggaggt    1440

ggatggcacc tgctactgag ccgcccgcct gtccccaccc ctgaataaac tccatgctcc    1500

cccaagc                                                              1507


<210>  390
<211>  1022
<212>  DNA
<213>  homo sapiens;

<400>  390
gcatccccga ccagccccaa ggtcttcccg ctgagcctcg acagcacccc ccaagatggg      60

aacgtggtcg tcgcatgcct ggtccagggc ttcttccccc aggagccact cagtgtgacc     120

tggagcgaaa gcggacagaa cgtgaccgcc agaaacttcc cacctagcca ggatgcctcc     180

ggggacctgt acaccacgag cagccagctg accctgccgg ccacacagtg cccagacggc     240

aagtccgtga catgccacgt gaagcactac acgaatccca gccaggatgt gactgtgccc     300

tgccagttcc cccacctccc ccatgctgcc acccccgact gtcgctgcac cgaccggccc     360

tcgaggacct gctcttaggt tcagaagcga acctcacgtg cacactgacc ggcctgagag     420

atgcctctgg tgccaccttc acctggacgc cctcaagtgg gaagagcgct gttcaaggac     480

cacctgagcg tgacctctgt ggctgctaca gcgtgtccag tgtcctgcct ggctgtgccc     540

agccatggaa ccatggggag accttcacct gcactgctgc ccaccccgag ttgaagaccc     600

cactaaccgc caacatcaca aaatccggaa acacattccg gcccgaggtc cacctgctgc     660

cgccgccgtc ggaggagctg gccctgaacg agctggtgac gctgacgtgc ctggcacgtg     720

gcttcagccc caaggatgtg ctggttcgct ggctgcaggg gtcacaggag ctgccccgcg     780

agaagtacct gacttgggca tcccggcagg agcccagcca gggcaccacc accttcgctg     840

taaccagcat actgcgcgtg gcagctgagg actggaagaa gggggacacc ttctcctgca     900

tggtgggcca cgaggccctg ccgctggcct tcacacagaa gaccatcgac cgcatggcgg     960

gtaaacccac ccacatcaat gtgtctgttg tcatggcgga ggtggatggc acctgctact    1020

ga                                                                   1022

<210>  391
<211>  1507
<212>  DNA
<213>  homo sapiens;

<400>  391
gcatccccga ccagccccaa ggtcttcccg ctgagcctcg acagcacccc ccaagatggg      60

aacgtggtcg tcgcatgcct ggtccagggc ttcttccccc aggagccact cagtgtgacc     120

tggagcgaaa gcggacagaa cgtgaccgcc agaaacttcc cacctagcca ggatgcctcc     180

ggggacctgt acaccacgag cagccagctg accctgccgg ccacacagtg cccagacggc     240

aagtccgtga catgccacgt gaagcactac acgaatccca gccaggatgt gactgtgccc     300

tgcccaggtc agagggcagg ctggggagtg gggcggggcc accccgtcct gccctgacac     360

tgcgcctgca cccgtgttcc ccacagggag ccgccccttc actcacacca gagtggaccg     420

cgggccgagc cccaggaggt ggtggtggac aggccaggag gggcgaggcg gggcacgggg     480

gaagggcgtt ctgaccagct caggccatct ctccactcca gttcccccac ctcccccatg     540

ctgccacccc cgactgtcgc tgcaccgacc ggccctcgag gacctgctct taggttcaga     600

agcgaacctc acgtgcacac tgaccggcct gagagatgcc tctggtgcca ccttcacctg 660

gacgccctca agtgggaaga gcgctgttca aggaccacct gagcgtgacc tctgtggctg 720

ctacagcgtg tccagtgtcc tgcctggctg tgcccagcca tggaaccatg gggagacctt 780

cacctgcact gctgcccacc ccgagttgaa gaccccacta accgccaaca tcacaaaatc 840

cggtgggtcc agaccctgct cggggccctg ctcagtgctc tggtttgcaa agcatattcc 900

cggcctgcct cctccctccc aatcctgggc tccagtgctc atgccaagta cagagggaaa 960

ctgaggcagg ctgaggggcc aggacacagc ccagggtgcc caccagagca gaggggctct 1020

ctcatcccct gcccagcccc ctgacctggc tctctaccct ccaggaaaca cattccggcc 1080

cgaggtccac ctgctgccgc cgccgtcgga ggagctggcc ctgaacgagc tggtgacgct 1140

gacgtgcctg gcacgtggct tcagccccaa ggatgtgctg gttcgctggc tgcaggggtc 1200

acaggagctg ccccgcgaga agtacctgac ttgggcatcc cggcaggagc ccagccaggg 1260

caccaccacc tacgctgtaa ccagcatact gcgcgtggca gctgaggact ggaagaaggg 1320

ggagaccttc tcctgcatgg tgggccacga ggccctgccg ctggccttca cacagaagac 1380

catcgaccgc atggcgggta aacccaccca catcaatgtg tctgttgtca tggcggaggc 1440

ggatggcacc tgctactgag ccgcccgcct gtccccaccc ctgaataaac tccatgctcc 1500

cccaagc 1507

<210> 392
<211> 1022
<212> DNA
<213> homo sapiens;

<400> 392
gcatccccga ccagccccaa ggtcttcccg ctgagcctcg acagcacccc ccaagatggg 60

aacgtggtcg tcgcatgcct ggtccagggc ttcttccccc aggagccact cagtgtgacc 120

tggagcgaaa gcggacagaa cgtgaccgcc agaaacttcc cacctagcca ggatgcctcc 180

ggggacctgt acaccacgag cagccagctg accctgccgg ccacacagtg cccagacggc 240

aagtccgtga catgccacgt gaagcactac acgaatccca gccaggatgt gactgtgccc 300

tgccagttcc cccacctccc ccatgctgcc acccccgact gtcgctgcac cgaccggccc 360

tcgaggacct gctcttaggt tcagaagcga acctcacgtg cacactgacc ggcctgagag 420

atgcctctgg tgccaccttc acctggacgc cctcaagtgg gaagagcgct gttcaaggac 480

cacctgagcg tgacctctgt ggctgctaca gcgtgtccag tgtcctgcct ggctgtgccc 540

agccatggaa ccatggggag accttcacct gcactgctgc ccaccccgag ttgaagaccc 600

cactaaccgc caacatcaca aaatccggaa acacattccg gcccgaggtc cacctgctgc 660

cgccgccgtc ggaggagctg gccctgaacg agctggtgac gctgacgtgc ctggcacgtg 720

gcttcagccc caaggatgtg ctggttcgct ggctgcaggg gtcacaggag ctgccccgcg 780

agaagtacct gacttgggca tcccggcagg agcccagcca gggcaccacc acctacgctg 840

taaccagcat actgcgcgtg gcagctgagg actggaagaa gggggagacc ttctcctgca 900

tggtgggcca cgaggccctg ccgctggcct tcacacagaa gaccatcgac cgcatggcgg 960

gtaaacccac ccacatcaat gtgtctgttg tcatggcgga ggcggatggc acctgctact 1020

ga 1022

<210> 393
<211> 8912
<212> DNA
<213> homo sapiens;

<400> 393
cacccaccaa ggctccggat gtgttcccca tcatatcagg gtgcagacac ccaaaggata 60

acagccctgt ggtcctggca tgcttgataa ctgggtacca cccaacgtcc gtgactgtca 120

cctggtacat ggggacacag agccagcccc agagaacctt ccctgagata caaagacggg 180

acagctacta catgacaagc agccagctct ccacccccct ccagcagtgg cgccaaggcg 240

agtacaaatg cgtggtccag cacaccgcca gcaagagtaa gaaggagatc ttccgctggc 300

caggtaggtc gcaccggaga tcacccagaa gggcccccca ggacccccag caccttccac 360

tcagggcctg accacaaaga cagaagcaag ggctgggctg tgaggcaacc cccacctccc 420

cctcagagca cgttcctccc ccttcaccct gtatccaccc ctccggaccc tccccatctc 480

agtccctccg ctccctctct ctgaggccca tctcccaata cccagatcac tttccttcca 540

gacccttccc tcagtgtgca cggaggcagc ttgcccagca aaggtgactg tctagtgggc 600

ttcccacagc caagctccca ccccatgctg cggcccttcc cttcttcctg cttggctgcc 660

tgtgcccccc acctgcctgt ccacaaccca gcctctggta catccatgcc ctctgccctc 720

agcctcacct gcacttttcc ttggatttca gagtctccaa aggcacaggc ctcctcagtg 780

cccactgcac aaccccaagc agagggcagc ctcgccaagg caaccacagc cccagccacc 840

acccgtaaca caggtgagaa gccccttccc tgcacactcc acccccaccc acctgctcat 900

tcctcagccg cctcctccag gcagcccttc ataactcctt gtctgagtct ccaagtcaca 960

ctttggtaag gagagggaca ctgaacggac ctctaacaaa cacctactgc cagccagccc 1020

cagtctgggg gccagcagat gccaaacagc cagcagactc ccagagcaga cctgggccgg 1080

ctccctggcc catggaccca gctctgcctc gctgagctga ggcatgggct ctcagcgcag 1140

cctcacatag agccaccctg ccgaggcagt ccggcttgca gactcacagg tcacttgggc 1200

cgcagcagcc cctccccgtg accctcgcct cccgcccgcc ccagcctggc tctctccaag 1260

tgttggatct tggtggccag cctgcttctc accctcaccc tgcctgccac ctcagaatgg 1320

caggggaaag agggccctca ccaagaactt tatctgagga gtctgaggct tgtgactctg 1380

acctgcctga gatgtccatg tggccggggg gacgggttca gtgttcggga gaactcgggt 1440

acgtgcctga ctttctctga gtagggcagg aagctgttag gagaagcagc agtgaggtgg 1500

gctggaccaa caggcagaat gactgtccct cagccaccct ctgggatgtg ggtcaagctc 1560

tgacaaaggc atggcacagc catggtggcc cctgcttgga tgagtggcca cggtgccctc 1620

accctgggcc agaatctgcc tccactctgc aggtgcagaa acacgacatt cccgtctcta 1680

aacacaccta gctcctaggc ttggggtggg cctatcaaat gcagggagat ggacacagca 1740

caagggccag agcttcccat gagaaaggtg agggcagctg ctccctgacc cgggcatctg 1800

cacttgtccc tctccaccct cctcatgggc agtggagact cagcaacaaa acaagttgag 1860

tgcattagca gccagctctg gagccaagtc actcacccca cggccttggc tgctggtgga 1920

ggggccttcc cctgggcagc ctccaagaag acggccaagt gctcttactc agaccacggc 1980

gctgcttcct ggcacctcga tttcccacaa caacatgggg tgcagacagg ctagggcccc 2040

ctgccctggg gcctggacgg catccagtta aagatgaccc ttcacgggcg gtgcctgagg 2100

tgtgctgacc tcagcagcta agccctcagg tctggtctgc actgccccac ctggaggacc 2160

caactgaccc agacacagcc agggttatgg catgaccccg tggacggtga cccacaggcc 2220

agatgcagcc aggggctgtt ttgtgtggcc tagaaatgtc tttacagttg tagtgggatg 2280

gaggaggaag aggaagagag gaggggagag aaaagcaggg aaggggaaaa agaggagttc 2340

aatgcaaccc caaaagccag aacagttttg agctgaaaga acaaggcagg aaacatccca 2400

gtacctgact tcaaaacata ctataaagca gttgtaatca aaacaggatc ataaaaacag 2460

acacacagac ccatggaaca gaaaagcgag cccagaaata aatctacatg cttgcagtcc 2520

attgattttc aacaaaggca ccaggaaaac acaatgggga gaggacagtt tcctcaataa 2580

atagtgctgg ggaaactgga tatccatgtg cagactaatg aaactacaca aaaatcaatt 2640

gaaaacagtc taggccaggc gcggtggctc atgccggtaa tcccagcact ttgggaggcc 2700

gagacaggcg gatcacctga ggtcaggagt tcgagaccag cttggccaac atggcgaaac 2760

ccggtctcca ctaaaaatac aaaaattagc acatggtggc ctacgtctgt tatcccagct 2820

tttcaggagg ctgaggcagg agaatcgctt gaatccggga ggtgaaggtt gcagggagcc 2880

aagattgcgc cactgcattc cagcctgggc aatggagcga gactgtctca aaaaaaaaaa 2940

aaaaaagaaa agaaaacagt ctaaaggttt aactgaacag ataaagctac tagaagaaaa 3000

cataggggga aaactccatg acattagtct gagcaacgat ttttggatat gatcccaaaa 3060

gctcaggcag cactagtcac aaaagccaag atacagaacc aacctaagca cccctcagca 3120

gatgcacagg taaagaaaat gtggtacgta tggggcacaa tggaatacga ttcagccttt 3180

aaaaacagtg aaattctgtc attggcaaca atgtagatga acctgaagga cacttatgct 3240

aagtgaaata agccaggcac agaaggagca atactgcatg attgcactta catctggcag 3300

gttaaaaagg caaactctta gaggcagaca gtagagaggt ggtgccaggg agcgggcact 3360

ggtggctggg gagatgttgg tcaaagggca caaaactgca gttgggagga attagttcag 3420

gacatccctt gtacatgggg acagtggtta gtaacaacgg attgtatcct tgaaaaccgc 3480

taagaaaata gtttttaagt gttcttgaca caaaaagtga cacgtatgtg agatactgca 3540

tggtcattag ctggatttag ccattccaca atgtacacat atttcaaaca ttgtgttgta 3600

tatgataaac atgtataatt tttgtcaatt aaaaattttt aggaagagga ggagaagaga 3660

agaagaagga gaaggagaaa gaggaacaag aagagagaga gacaaagaca ccaggttttt 3720

tctgacccct gggctatcaa aacacctatt gcccaataac tagttggccg ttggtgccct 3780

aaactattga agcgattgct gttatgtgga tgggccccgg acacttagaa actcgtgacc 3840

cctgaggacc cccacgagga cagtcagggt ccccccgaac tcagggagca ctgaggaagg 3900

agctcttaga ggcgtggggc ccctcaggcc cctcagaggg ctctgccaca tgggtcaggg 3960

gcaggctgag ggggagtccc aggctccatg cccagcctct gtgcctctga ccagggtgtc 4020

ccccacaccg cctcctcccc agtgccctcc actggccaca cctggccaga agctggggag 4080

aggagagcac agtggttaag tcagtccctg cagggagacg gcaccagaaa aacctggcct 4140

gtggatgagt cccggcctgg cagccacaga gcagagagct ctagaagcaa cgaaggcccg 4200

agtctgctca gggaagagcg ggcagcagcc ccagggccgg acagtgacca agagtggcac 4260

cgcccatggc tcaacgggtc tttgcccaca gatcccccag cccctggaga cagggtctgt 4320

gtgcctggcc gtgcaggcag gcaccacact cagggggagg ccactgtgga gctctgtgca 4380

gagccccggg cgggagccta ctgctcccga aggtccggcc acagctgctc tcgtttgctc 4440

tcccctgcag agtgtccgag ccacacccag cctcttggcg tctacctgct aacccctgca 4500

gtgcaggacc tgtggctccg ggacaaagcc accttcacct gcttcgtggt gggcagtgac 4560

ctgaaggatg ctcacctgac ctgggaggtg gccgggaagg tccccacagg gggcgtggag 4620

gaagggctgc tggagcggca cagcaacggc tcccagagcc agcacagccg tctgaccctg 4680

cccaggtcct tgtggaacgc ggggacctcc gtcacctgca cactgaacca tcccagcctc 4740

ccaccccaga ggttgatggc gctgagagaa cccggtgagc ctggctccca ggtggggaga 4800

cgagggtgcc cacagcctgc tgacccctac gcctgcccca gggccatgac cccagctggg 4860

ccccagcagc accggtcatc ctccacagga aaggagaagg gaggcaccag caccctggcc 4920

ggccccactt ctctcccagt gcccccgtgg ccagaggctg acagcctccc ccacctcccc 4980

gcagctgcgc aggcacccgt caagctttcc ctgaacctgc tggcctcgtc tgaccctccc 5040

gaggcggcct cgtggctcct gtgtgaggtg tctggcttct cgccccccaa catcctcctg 5100

atgtggctgg aggaccagcg tgaggtgaac acttctgggt ttgcccccgc acgcccccct 5160

ccacagcccg ggagcaccac gttctgggcc tggagtgtgc tgcgtgtccc agccccgccc 5220

agccctcagc cagccaccta cacgtgtgtg gtcagccacg aggactcccg gactctgctc 5280

aacgccagcc ggagcctaga agtcagctgt gagtcacccc caggcccagg gttgggacgg 5340

ggactctgag gggggccata aggagctgga atccatacta ggcaggggtg ggcactgggc 5400

aggggcgggg ctaggctgtc ctgggcacac aggccccttc tcggtgtccg gcaggagcac 5460

agacttccca gtactcctgg gccatggatg tcccagcgtc catccttgct gtccacacca 5520

cgtgctggcc caggctggct ggcacagtgt aagaggtgga tacaacccct cgccgtgccc 5580

tgaggagtgg cggtttcctc ccaagacatt ccccacggct gggtgctggg cacaggcctt 5640

ccctggtgtg accgtgaatg tggtcaccct gaacagctgc cctctctggg gacatctgac 5700

tgtccaagac cacagtcagc acctctggga gccagagggg tctccagaga cccccagatg 5760

tcaggcttgg gctcagtgcc cagcgaaagg tcagccccac acatgcccat aatgggcgcc 5820

cacccagagt gacagccccc agcctcctgc caggcccacc cttttccgcc cccttgaggc 5880

atggcacaca gaccagtgcg cccactgccc gagcatggcc ccagtgggat gtggtggcca 5940

cgaggggctg tacacacagc aggaggctgt ccgccctgct cagggcctgc tgcctatgcc 6000

ccagctgtcc aaccaaggga ggcatggaag ggcccctggt gtaagctgga gccaggcacc 6060

caggcccccg gccaccctgc agagccaagg aaaggaagac acccaagtca acaaggggca 6120

gggctgaggg ctgtcccagg ctcttttggc ccgaggggct gccagcagcc ctgacccggc 6180

atgggccttc cccaaaagcg accctgtgag gtggcctcac agagaacccc ctctgaggac 6240

agtgtctgac cctgcctgcc tcacacagat gggccccaca gcagtgggca acctgggggg 6300

cagcagccca acctgaccct gcagggactg ccccctgcag cagcagctgc ttctcagtcc 6360

cccaacctcc ctgtccccgc cagagggtct tccccgaagc tgcagcccca acccatggct 6420

gcccacctgg aaccgggact ccctgtccac tgccccctcc ccttcggggc cccatctgtg 6480

ctggggccca ggttcggcct acagattccc atcattgcca tggcctcctg accttgccta 6540

tccaccccca accaccggct ccatgctgac cctcccccag gctcccacgc ccagctggcc 6600

ggccatcccc aggcacagac agtctgggat ctcacaggtt agcctggacc atccacctgg 6660

ccagacctgg gagaggctgg aagctgccct gccaccatgc tccagggccc caggttgcag 6720

tactatgggg tgagggtgtg tgtgcacacc tgtgtgtacc taggatatcc gagtgtaccc 6780

ttgtgccccc aagcacaagt ctccctccca ggcagtgagg cccagatggt gcagtggtta 6840

gagctgaggc ttatcccaca gagaaccctg gcgccttggt caaggaagcc cctatgcctt 6900

tcttgcctcg atttcccctc ttgtctgctg agccagcagg ggccacgtcc tgggctgctg 6960

tgaggaggaa gcaagttggt gctaggaggg gctcctgtgt gtgcatgggc gggaggggtg 7020

caggtatctg agcaccccgg tctccacttg agagagcagg gcaggagctc cctgacccac 7080

ccagactaca cacgctgtgt ccacgtgtct cccattatct gtggcagagg atccggcttc 7140

tttctcaatt tccagttctt cacaaagcaa tgcctttgta aaatgcaata agaaatacta 7200

gaaaaatgat atgaacagaa agacacgccg attttttgtt attagatgta acagaccatg 7260

gccccatgaa atgatcccgg accagatccg tccacacccg ccactcagca gctctggccg 7320

agctcacagt acaaccacaa taaactcttg ttgaatgaac tctaggaagt ctgtgacgtg 7380

gctggttctt gtcaatgctt cctgcctgcc cacaggctct tcctcgtgga tggggctgtg 7440

cttgccacgg aagcgcgttt ttcccggcct aggcttgcct tgggccccac tgccgtctcc 7500

agctggagat gaccttctat acacacattt gctcatgaca gacccttgct tagccccctt 7560

ccatggctcc ctcctgctgc tgggataaaa tcaccttgcc tggatatccc ctcctgggcc 7620

cctttccacc ctccttagtc agcaccccca gttcagggca cctgctttcc ccgctgcgga 7680

gaagccactc tctccttgct gcccggctgt gtcttgcctt ccacaccttg tcacagtggc 7740

cacttcctaa ggaaggcctc cctgtgtgca ggtgtgcaga agtgccccag cctcccgtca 7800

cctttgtcac gggagcccaa tccatgagag tctatggttc tgtctgtctg ccccactcag 7860

ggcagcgaca agtccaggcg gggaggacac agtaggcaga gatttgtcga ggggacatat 7920

gagcaagagg gtgaggctgg gagctccctg gagataacca cgcctcctgg gaagactcgc 7980

cgtcatttca gctccacgct gtgcgggggt gggtggaggg gtagcctggc cctcatgacc 8040

agggagcttc tcactcagcc cctgttcctc cccagacctg gccatgaccc ccctgatccc 8100

tcagagcaag gatgagaaca gcgatgacta cacgaccttt gatgatgtgg gcagcctgtg 8160

gaccgccctg tccacgtttg tggccctctt catcctcacc ctcctctaca gcggcattgt 8220

cactttcatc aaggtcaggg gagcggccag gctctcagtg accctcgggg tgggtgtggg 8280

gcaaggtgcc cttccagggg acatgccaga gctggtccag ggatcctgga ccaggcagag 8340

gcagggctga gggagcctgg aggacatgca ggccctctgt ggcctgtgga cactgtcgaa 8400

ggccctcttg accctgtgga taaaggacaa caccccctcc cctgctcctc tgtctcccct 8460

gcccctccac ccctcaggct tctagccccc tgtctgaccc caggggctgt ctttcaggtg 8520

aagtagcccc agaagagcag gacgccctgt acctgcagag aagggaagca gcctctgtac 8580

ctcatctgtg gctaccagag agcagaaagg acccaccctg gactcttctg tgtgcaggaa 8640

gatgcgccag cccctgcccc cggctcccct ctgtccgcca cagaatccag tcttctagac 8700

cagggggacg ggcacccatc actccgcagg cgaatcagag cccccctgcc ccggccctaa 8760

cccctgtgcc tccttcccgt gcttccccca gagccagcta cacccctgcc ccggccctaa 8820

cccccatgcc tccttcctgt gcttccccca gagccagcta gtcccacctg cagcccgctg 8880

gcctccccat aaacacgctt tggttcattt ca                                    8912

<210>  394
<211>  1293
<212>  DNA
<213>  homo sapiens;

<220>
<221>  misc_feature
<222>  (1)..(1)
<223>  n is a, c, g, or t

<400>  394
ncacccacca aggctccgga tgtgttcccc atcatatcag ggtgcagaca cccaaaggat      60

aacagccctg tggtcctggc atgcttgata actgggtacc acccaacgtc cgtgactgtc     120

acctggtaca tggggacaca gagccagccc cagagaacct tccctgagat acaaagacgg     180

gacagctact acatgacaag cagccagctc tccacccccc tccagcagtg gcgccaaggc     240

gagtacaaat gcgtggtcca gcacaccgcc agcaagagta agaaggagat cttccgctgg     300

ccagagtctc caaaggcaca ggcctcctca gtgcccactg cacaacccca agcagagggc     360

agcctcgcca aggcaaccac agccccagcc accacccgta acacaggaag aggaggagaa     420

gagaagaaga aggagaagga gaaagaggaa caagaagaga gagagacaaa gacaccagag     480

tgtccgagcc acacccagcc tcttggcgtc tacctgctaa cccctgcagt gcaggacctg     540

tggctccggg acaaagccac cttcacctgc ttcgtggtgg gcagtgacct gaaggatgct     600

cacctgacct gggaggtggc cgggaaggtc cccacagggg gcgtggagga agggctgctg     660

gagcggcaca gcaacggctc ccagagccag cacagccgtc tgaccctgcc caggtccttg     720

tggaacgcgg ggacctccgt cacctgcaca ctgaaccatc ccagcctccc accccagagg     780

ttgatggcgc tgagagaacc cgctgcgcag gcacccgtca agctttccct gaacctgctg     840

gcctcgtctg accctcccga ggcggcctcg tggctcctgt gtgaggtgtc tggcttctcg     900

ccccccaaca tcctcctgat gtggctggag gaccagcgtg aggtgaacac ttctgggttt     960

gcccccgcac gcccccctcc acagcccggg agcaccacgt tctgggcctg gagtgtgctg    1020

cgtgtcccag ccccgcccag ccctcagcca gccacctaca cgtgtgtggt cagccacgag    1080

gactcccgga ctctgctcaa cgccagccgg agcctagaag tcagctacct ggccatgacc    1140

cccctgatcc ctcagagcaa ggatgagaac agcgatgact acacgacctt tgatgatgtg    1200

ggcagcctgt ggaccgccct gtccacgttt gtggccctct tcatcctcac cctcctctac    1260

agcggcattg tcactttcat caaggtgaag tag                                 1293

<210>  395
<211>  3842

<212>  DNA
<213>  homo sapiens;

<400>  395
gcctccacac agagcccatc cgtcttcccc ttgacccgct gctgcaaaaa cattccctcc      60

aatgccacct ccgtgactct gggctgcctg gccacgggct acttcccgga gccggtgatg     120

gtgacctggg acacaggctc cctcaacggg acaactatga ccttaccagc caccaccctc     180

acgctctctg gtcactatgc caccatcagc ttgctgaccg tctcgggtgc gtgggccaag     240

cagatgttca cctgccgtgt ggcacacact ccatcgtcca cagactgggt cgacaacaaa     300

accttcagcg gtaagagagg gccaagctca gagaccacag ttcccaggag tgccaggctg     360

agggctggca gagtgggcag gggttgaggg ggtgggtggg ctcaaacgtg ggaacaccca     420

gcatgcctgg ggacccgggc caggacgcgg gggcaagagg agggcacaca gagctcagag     480

aggccaacaa ccctcatgac caccagctct cccccagtct gctccaggga cttcaccccg     540

cccaccgtga agatcttaca gtcgtcctgc gacggcggcg ggcacttccc cccgaccatc     600

cagctcctgt gcctcgtctc tgggtacacc ccagggacta tcaacatcac ctggctggag     660

gacgggcagg tcatggacgt ggacttgtcc accgcctcta ccacgcagga gggtgagctg     720

gcctccacac aaagcgagct caccctcagc cagaagcact ggctgtcaga ccgcacctac     780

acctgccagg tcacctatca aggtcacacc tttgaggaca gcaccaagaa gtgtgcaggt     840

acgttcccac ctgccctggt ggccgccacg gaggccagag aagaggggcg ggtgggcctc     900

acacagccct ccggtgtacc acagattcca acccgagagg ggtgagcgcc tacctaagcc     960

ggcccagccc gttcgacctg ttcatccgca agtcgcccac gatcacctgt ctggtggtgg    1020

acctggcacc cagcaagggg accgtgaacc tgacctggtc ccgggccagt gggaagcctg    1080

tgaaccactc caccagaaag gaggagaagc agcgcaatgg cacgttaacc gtcacgtcca    1140

ccctgccggt gggcacccga gactggatcg agggggagac ctaccagtgc agggtgaccc    1200

acccccacct gcccagggcc ctcatgcggt ccacgaccaa gaccagcggt gagccatggg    1260

caggccgggg tcgtggggga agggagggag cgagtgagcg gggcccgggc tgaccccacg    1320

tctggccaca ggcccgcgtg ctgccccgga agtctatgcg tttgcgacgc cggagtggcc    1380

ggggagccgg gacaagcgca ccctcgcctg cctgatccag aacttcatgc ctgaggacat    1440

ctcggtgcag tggctgcaca acgaggtgca gctcccggac gcccggcaca gcacgacgca    1500

gccccgcaag accaagggct ccggcttctt cgtcttcagc cgcctggagg tgaccagggc    1560

cgaatgggag cagaaagatg agttcatctg ccgtgcagtc catgaggcag caagcccctc    1620

acagaccgtc cagcgagcgg tgtctgtaaa tcccggtaaa tgacgtactc ctgcctccct    1680

ccctcccagg gctccatcca gctgtgcagt ggggaggact ggccagacct tctgtccact    1740

gttgcaatga ccccaggaag ctacccccaa taaactgtgc ctgctcagag ccccaggtac    1800

acccattctt gggagcgggc agggctgtgg gcaggtgcat cttggcacag aggaatgggc 1860

cccccaggag gggcagtggg aggaggtggg cagggctgag tccccccagg agaggcggtg 1920

ggaggaggtg ggcagggctg aggtgccact catccatctg ccttcgtgtc agggttattt 1980

gtcaaacagc atatctgcag ggactcatca cagctacccc gggccctctc tgcccccact 2040

ctgggtctac cccctccaag gagtccaaag acccagggga ggtcctcagg gaaggggcaa 2100

gggagccccc acagccctct ctcttggggg cttggcttct acccccctgg acaggagccc 2160

ctgcaccccc aggtatagat gggcacacag gcccctccag gtggaaaaac agccctaagt 2220

gaaaccccca cacagacaca cacgacccga cagccctcgc ccaagtctgt gccactggcg 2280

ttcgcctctc tgccctgtcc cgccttgccg agtcctggcc ccagcaccgg ggccggtgga 2340

gccgagccca ctcacacccc gcagcctccg ccaccctgcc ctgtgggcac accaggccca 2400

ggtcagcagc caggccccct ctcctactgc cccccaccgc cccttggtcc atcctgaatc 2460

ggcccccagg ggatcgccag cctcacacac ccagtctcgc ccactcacgc ctcactcaag 2520

gcacagctgt gcacacacta ggccccatag caactccaca gcaccctgta ccaccaccag 2580

ggcgccatag acaccccaca cgtggtcaca cgtggcccac actccgcctc tcacgctgcc 2640

tccagcgagg ctactgccaa gcccttcctc tgagccatac ctgggccgct ggatcccaga 2700

gagaaatgga gaggccctca cgtggtgtcc tccagtccaa ccctccctgt caccctgtca 2760

gcagcagcac cccacagcca aacacaggat ggatgcgtgg gctccatccc ccactcaccc 2820

acaccggaac cccagagcag gctacgtgcc cctcacagac ctcaaaccca catgtgcatc 2880

tgacacccca gatccaaacg ctccccccgg tcatgcacac caagggcaca gcacccacca 2940

aatccacacg gaaacacggg caccgggcac cccatgagca caaagcccct ccatgtctga 3000

agacagtccc tgcacaccgt cacagccata cattcagctt cactctcacg tcccagccca 3060

cctgcaccca gctctgggcc tggagcagca gaaagaggtg tgagggcccg aggcgggacc 3120

tgcacctgct gatgacccgg gaccagcagg cagctcacgg tgttggggaa gggagtggag 3180

ggcacccagg gcaggagcca gagggaccag gctggtgggc ggggccgggc cggggtaggg 3240

ccaggaggca gctctggaca cccacaggcc tgggctcata gtccacacca ggacagcccc 3300

tcagagcacc catgcagtga gtcccaggtc ttgggagcca ggccgcagag ctcacgcatc 3360

cttccgaggg ccctgagtga ggcggccact gctgtgccga ggggttgggt ccttctctgg 3420

ggagggcgtg gggtctagag aggcggagtg gaggtaacca gaggtcagga gagaagccgt 3480

aaggaacaga gggaaaatgg ggccagagtc ggggcgcagg gacgagaggt caggagtggt 3540

cggcctggct ctgggccgtt gactgactcg ggacctgggt gcccaccctc agggctggct 3600

ggcggctccg cgcagtccca gagggccccg gatagggtgc tctgccactc cggacagcag 3660

cagggactgc cgagagcagc aggaggctct gtcccccacc cccgctgcca ctgtggagcc 3720

gggagggctg actggccagg tcccccagag ctggacgtgt gcgtggagga ggccgagggc 3780

gaggcgccgt ggacgtggac cggcctctgc atcttcgccg cactcttcct gctcagcgtg 3840

ag 3842

<210> 396
<211> 1287
<212> DNA
<213> homo sapiens;

<400> 396
gcctccacac agagcccatc cgtcttcccc ttgacccgct gctgcaaaaa cattccctcc 60

aatgccacct ccgtgactct gggctgcctg gccacgggct acttcccgga gccggtgatg 120

gtgacctggg acacaggctc cctcaacggg acaactatga ccttaccagc caccaccctc 180

acgctctctg gtcactatgc caccatcagc ttgctgaccg tctcgggtgc gtgggccaag 240

cagatgttca cctgccgtgt ggcacacact ccatcgtcca cagactgggt cgacaacaaa 300

accttcagcg tctgctccag ggacttcacc ccgcccaccg tgaagatctt acagtcgtcc 360

tgcgacggcg gcgggcactt ccccccgacc atccagctcc tgtgcctcgt ctctgggtac 420

accccaggga ctatcaacat cacctggctg gaggacgggc aggtcatgga cgtggacttg 480

tccaccgcct ctaccacgca ggagggtgag ctggcctcca cacaaagcga gctcaccctc 540

agccagaagc actggctgtc agaccgcacc tacacctgcc aggtcaccta tcaaggtcac 600

acctttgagg acagcaccaa gaagtgtgca gattccaacc cgagaggggt gagcgcctac 660

ctaagccggc ccagcccgtt cgacctgttc atccgcaagt cgcccacgat cacctgtctg 720

gtggtggacc tggcacccag caaggggacc gtgaacctga cctggtcccg ggccagtggg 780

aagcctgtga accactccac cagaaaggag gagaagcagc gcaatggcac gttaaccgtc 840

acgtccaccc tgccggtggg cacccgagac tggatcgagg gggagaccta ccagtgcagg 900

gtgacccacc cccacctgcc cagggccctc atgcggtcca cgaccaagac cagcggcccg 960

cgtgctgccc cggaagtcta tgcgtttgcg acgccggagt ggccggggag ccgggacaag 1020

cgcaccctcg cctgcctgat ccagaacttc atgcctgagg acatctcggt gcagtggctg 1080

cacaacgagg tgcagctccc ggacgcccgg cacagcacga cgcagccccg caagaccaag 1140

ggctccggct tcttcgtctt cagccgcctg gaggtgacca gggccgaatg ggagcagaaa 1200

gatgagttca tctgccgtgc agtccatgag gcagcaagcc cctcacagac cgtccagcga 1260

gcggtgtctg taaatcccgg taaatga 1287

<210> 397
<211> 30
<212> DNA

<213>  homo sapiens;

<400>  397
tccggcttct tcgtcttcag ccgcctggag                                    30


<210>  398
<211>  228
<212>  DNA
<213>  homo sapiens;

<400>  398
tccggcttct tcgtcttcag ccgcctggag gtgaccaggg ccgaatggga gcagaaagat      60

gagttcatct gccgtgcagt ccatgaggca gcaagcccct cacagaccgt ccagcgagcg     120

gtgtctgtaa atcccgagct ggacgtgtgc gtggaggagg ccgagggcga ggcgccgtgg     180

acgtggaccg gcctctgcat cttcgccgca ctcttcctgc tcagcgtg                  228


<210>  399
<211>  1975
<212>  DNA
<213>  homo sapiens;

<400>  399
gggagtgcat ccgccccaac ccttttcccc ctcgtctcct gtgagaattc cccgtcggat      60

acgagcagcg tggccgttgg ctgcctcgca caggacttcc ttcccgactc catcactttc     120

tcctggaaat acaagaacaa ctctgacatc agcagcaccc ggggcttccc atcagtcctg     180

agagggggca agtacgcagc cacctcacag gtgctgctgc cttccaagga cgtcatgcag     240

ggcacagacg aacacgtggt gtgcaaagtc cagcacccca acggcaacaa agaaaagaac     300

gtgcctcttc caggtgaggg ccgggcccag ccaccgggac agagagggag ccgaaggggg     360

cgggagtggc gggcaccggg ctgacacgtg tccctcactg cagtgattgc cgagctgcct     420

cccaaagtga gcgtcttcgt cccaccccgc gacggcttct tcggcaaccc ccgcaagtcc     480

aagctcatct gccaggccac gggtttcagt ccccggcaga ttcaggtgtc ctggctgcgc     540

gaggggaagc aggtggggtc tggcgtcacc acggaccagg tgcaggctga ggccaaagag     600

tctgggccca cgacctacaa ggtgaccagc acactgacca tcaaagagag cgactggctc     660

agccagagca tgttcacctg ccgcgtggat cacaggggcc tgaccttcca gcagaatgcg     720

tcctccatgt gtggccccgg tgagtgacct gtccccaggg gcagcaccca ccgacacaca     780

ggggtccact cgggtctggc attcgccacc ccggatgcag ccatctactc cctgagcctt     840

ggcttcccag agcggccaag ggcaggggct cgggcggcag gacccctggg ctcggcagag     900

gcagttgcta ctctttgggt gggaaccatg cctccgccca catccacacc tgccccacct     960

ctgactccct tctcttgact ccagatcaag acacagccat ccgggtcttc gccatccccc    1020

catcctttgc cagcatcttc ctcaccaagt ccaccaagtt gacctgcctg gtcacagacc    1080

tgaccaccta tgacagcgtg accatctcct ggacccgcca gaatggcgaa gctgtgaaaa 1140

cccacaccaa catctccgag agccacccca atgccacttt cagcgccgtg ggtgaggcca 1200

gcatctgcga ggatgactgg aattccgggg agaggttcac gtgcaccgtg acccacacag 1260

acctgccctc gccactgaag cagaccatct cccggcccaa gggtaggccc cactcttgcc 1320

cctcttcctg cactccctgg gacctccctt ggcctctggg gcatggtgga aagcacccct 1380

cactcccccg ttgtctgggc aactggggaa aaggggactc aaccccagcc cacaggctgg 1440

tccccccact gccccgccct caccaccatc tctgttcaca ggggtggccc tgcacaggcc 1500

cgatgtctac ttgctgccac cagcccggga gcagctgaac ctgcgggagt cggccaccat 1560

cacgtgcctg gtgacgggct tctctcccgc ggacgtcttc gtgcagtgga tgcagagggg 1620

gcagcccttg tccccggaga agtatgtgac cagcgcccca atgcctgagc cccaggcccc 1680

aggccggtac ttcgcccaca gcatcctgac cgtgtccgaa gaggaatgga acacggggga 1740

gacctacacc tgcgtggtgg cccatgaggc cctgcccaac agggtcaccg agaggaccgt 1800

ggacaagtcc accggtaaac ccaccctgta caacgtgtcc ctggtcatgt ccgacacagc 1860

tggcacctgc tactgaccct gctggcctgc ccacaggctc ggggcggctg gccgctctgt 1920

gtgtgcatgc aaactaaccg tgtcaacggg gtgagatgtt gcatcttata aaatt 1975

<210> 400
<211> 1362
<212> DNA
<213> homo sapiens;

<400> 400
gggagtgcat ccgccccaac ccttttcccc ctcgtctcct gtgagaattc cccgtcggat 60

acgagcagcg tggccgttgg ctgcctcgca caggacttcc ttcccgactc catcactttc 120

tcctggaaat acaagaacaa ctctgacatc agcagcaccc ggggcttccc atcagtcctg 180

agagggggca agtacgcagc cacctcacag gtgctgctgc cttccaagga cgtcatgcag 240

ggcacagacg aacacgtggt gtgcaaagtc cagcacccca acggcaacaa agaaaagaac 300

gtgcctcttc cagtgattgc cgagctgcct cccaaagtga gcgtcttcgt cccaccccgc 360

gacggcttct tcggcaaccc ccgcaagtcc aagctcatct gccaggccac gggtttcagt 420

ccccggcaga ttcaggtgtc ctggctgcgc gaggggaagc aggtggggtc tggcgtcacc 480

acggaccagg tgcaggctga ggccaaagag tctgggccca cgacctacaa ggtgaccagc 540

acactgacca tcaaagagag cgactggctc agccagagca tgttcacctg ccgcgtggat 600

cacaggggcc tgaccttcca gcagaatgcg tcctccatgt gtggccccga tcaagacaca 660

gccatccggg tcttcgccat ccccccatcc tttgccagca tcttcctcac caagtccacc 720

aagttgacct gcctggtcac agacctgacc acctatgaca gcgtgaccat ctcctggacc 780

cgccagaatg gcgaagctgt gaaaacccac accaacatct ccgagagcca ccccaatgcc 840

actttcagcg ccgtgggtga ggccagcatc tgcgaggatg actggaattc cggggagagg 900

ttcacgtgca ccgtgaccca cacagacctg ccctcgccac tgaagcagac catctcccgg 960

cccaaggggg tggccctgca caggcccgat gtctacttgc tgccaccagc ccgggagcag 1020

ctgaacctgc gggagtcggc caccatcacg tgcctggtga cgggcttctc tcccgcggac 1080

gtcttcgtgc agtggatgca gagggggcag cccttgtccc cggagaagta tgtgaccagc 1140

gccccaatgc ctgagcccca ggccccaggc cggtacttcg cccacagcat cctgaccgtg 1200

tccgaagagg aatggaacac gggggagacc tacacctgcg tggtggccca tgaggccctg 1260

cccaacaggg tcaccgagag gaccgtggac aagtccaccg gtaaacccac cctgtacaac 1320

gtgtccctgg tcatgtccga cacagctggc acctgctact ga 1362

<210> 401
<211> 1975
<212> DNA
<213> homo sapiens;

<400> 401
gggagtgcat ccgccccaac ccttttcccc ctcgtctcct gtgagaattc cccgtcggat 60

acgagcagcg tggccgttgg ctgcctcgca caggacttcc ttcccgactc catcactttc 120

tcctggaaat acaagaacaa ctctgacatc agcagcaccc ggggcttccc atcagtcctg 180

agagggggca agtacgcagc cacctcacag gtgctgctgc cttccaagga cgtcatgcag 240

ggcacagacg aacacgtggt gtgcaaagtc cagcacccca acggcaacaa agaaaagaac 300

gtgcctcttc caggtgaggg ccgggcccag ccaccgggac agagagggag ccgaaggggg 360

cgggagtggc gggcaccggg ctgacacgtg tccctcactg cagtgattgc cgagctgcct 420

cccaaagtga gcgtcttcgt cccaccccgc gacggcttct tcggcaaccc ccgcaagtcc 480

aagctcatct gccaggccac gggtttcagt ccccggcaga ttcaggtgtc ctggctgcgc 540

gaggggaagc aggtggggtc tggcgtcacc acggaccagg tgcaggctga ggccaaagag 600

tctgggccca cgacctacaa ggtgaccagc acactgacca tcaaagagag cgactggctc 660

agccagagca tgttcacctg ccgcgtggat cacaggggcc tgaccttcca gcagaatgcg 720

tcctccatgt gtgtccccgg tgagtgacct gtccccaggg gcagcaccca ccgacacaca 780

ggggtccact cgggtctggc attcgccacc ccggatgcag ccatctactc cctgagcctt 840

ggcttcccag agcggccaag ggcaggggct cgggcggcag gacccctggg ctcggcagag 900

gcagttgcta ctctttgggt gggaaccatg cctccgccca catccacacc tgccccacct 960

ctgactccct tctcttgact ccagatcaag acacagccat ccgggtcttc gccatccccc 1020

catcctttgc cagcatcttc ctcaccaagt ccaccaagtt gacctgcctg gtcacagacc 1080

tgaccaccta tgacagcgtg accatctcct ggacccgcca gaatggcgaa gctgtgaaaa 1140

cccacaccaa catctccgag agccacccca atgccacttt cagcgccgtg ggtgaggcca 1200

gcatctgcga ggatgactgg aattccgggg agaggttcac gtgcaccgtg acccacacag 1260

acctgccctc gccactgaag cagaccatct cccggcccaa gggtaggccc cactcttgcc 1320

cctcttcctg cactccctgg gacctccctt ggcctctggg gcatggtgga aagcacccct 1380

cactcccccg ttgtctgggc aactggggaa aaggggactc aaccccagcc cacaggctgg 1440

tccccccact gccccgccct caccaccatc tctgttcaca ggggtggccc tgcacaggcc 1500

cgatgtctac ttgctgccac cagcccggga gcagctgaac ctgcgggagt cggccaccat 1560

cacgtgcctg gtgacgggct tctctcccgc ggacgtcttc gtgcagtgga tgcagagggg 1620

gcagcccttg tccccggaga agtatgtgac cagcgcccca atgcctgagc cccaggcccc 1680

aggccggtac ttcgcccaca gcatcctgac cgtgtccgaa gaggaatgga acacggggga 1740

gacctacacc tgcgtggtgg cccatgaggc cctgcccaac agggtcaccg agaggaccgt 1800

ggacaagtcc accggtaaac ccaccctgta caacgtgtcc ctggtcatgt ccgacacagc 1860

tggcacctgc tactgaccct gctggcctgc ccacaggctc ggggcggctg gccgctctgt 1920

gtgtgcatgc aaactaaccg tgtcaacggg gtgagatgtt gcatcttata aaatt 1975

<210> 402
<211> 1362
<212> DNA
<213> homo sapiens;

<400> 402
gggagtgcat ccgccccaac ccttttcccc ctcgtctcct gtgagaattc cccgtcggat 60

acgagcagcg tggccgttgg ctgcctcgca caggacttcc ttcccgactc catcactttc 120

tcctggaaat acaagaacaa ctctgacatc agcagcaccc ggggcttccc atcagtcctg 180

agagggggca agtacgcagc cacctcacag gtgctgctgc cttccaagga cgtcatgcag 240

ggcacagacg aacacgtggt gtgcaaagtc cagcacccca acggcaacaa agaaaagaac 300

gtgcctcttc cagtgattgc cgagctgcct cccaaagtga gcgtcttcgt cccaccccgc 360

gacggcttct tcggcaaccc ccgcaagtcc aagctcatct gccaggccac gggtttcagt 420

ccccggcaga ttcaggtgtc ctggctgcgc gaggggaagc aggtggggtc tggcgtcacc 480

acggaccagg tgcaggctga ggccaaagag tctgggccca cgacctacaa ggtgaccagc 540

acactgacca tcaaagagag cgactggctc agccagagca tgttcacctg ccgcgtggat 600

cacaggggcc tgaccttcca gcagaatgcg tcctccatgt gtgtccccga tcaagacaca 660

gccatccggg tcttcgccat ccccccatcc tttgccagca tcttcctcac caagtccacc 720

aagttgacct gcctggtcac agacctgacc acctatgaca gcgtgaccat ctcctggacc 780

cgccagaatg gcgaagctgt gaaaacccac accaacatct ccgagagcca ccccaatgcc 840

actttcagcg ccgtgggtga ggccagcatc tgcgaggatg actggaattc cggggagagg 900

ttcacgtgca ccgtgaccca cacagacctg ccctcgccac tgaagcagac catctcccgg 960

cccaaggggg tggccctgca caggcccgat gtctacttgc tgccaccagc ccgggagcag 1020

ctgaacctgc gggagtcggc caccatcacg tgcctggtga cgggcttctc tcccgcggac 1080

gtcttcgtgc agtggatgca gagggggcag cccttgtccc cggagaagta tgtgaccagc 1140

gccccaatgc ctgagcccca ggccccaggc cggtacttcg cccacagcat cctgaccgtg 1200

tccgaagagg aatggaacac gggggagacc tacacctgcg tggtggccca tgaggccctg 1260

cccaacaggg tcaccgagag gaccgtggac aagtccaccg gtaaacccac cctgtacaac 1320

gtgtccctgg tcatgtccga cacagctggc acctgctact ga 1362

<210> 403
<211> 1975
<212> DNA
<213> homo sapiens;

<400> 403
gggagtgcat ccgccccaac ccttttcccc ctcgtctcct gtgagaattc cccgtcggat 60

acgagcagcg tggccgttgg ctgcctcgca caggacttcc ttcccgactc catcactttc 120

tcctggaaat acaagaacaa ctctgacatc agcagcaccc ggggcttccc atcagtcctg 180

agagggggca agtacgcagc cacctcacag gtgctgctgc cttccaagga cgtcatgcag 240

ggcacagacg aacacgtggt gtgcaaagtc cagcacccca acggcaacaa agaaaagaac 300

gtgcctcttc caggtgaggg ccgggcccag ccaccgggac agagagggag ccgaaggggg 360

cgggagtggc gggcaccggg ctgacacgtg tccctcactg cagtgattgc cgagctgcct 420

cccaaagtga gcgtcttcgt cccaccccgc gacggcttct tcggcaaccc ccgcaagtcc 480

aagctcatct gccaggccac gggtttcagt ccccggcaga ttcaggtgtc ctggctgcgc 540

gaggggaagc aggtggggtc tggcgtcacc acggaccagg tgcaggctga ggccaaagag 600

tctgggccca cgacctacaa ggtgaccagc acactgacca tcaaagagag cgactggctc 660

ggccagagca tgttcacctg ccgcgtggat cacaggggcc tgaccttcca gcagaatgcg 720

tcctccatgt gtgtccccgg tgagtgacct gtccccaggg gcagcaccca ccgacacaca 780

ggggtccact cgggtctggc attcgccacc ccggatgcag ccatctactc cctgagcctt 840

ggcttcccag agcggccaag ggcaggggct cgggcggcag gacccctggg ctcggcagag 900

gcagttgcta ctctttgggt gggaaccatg cctccgccca catccacacc tgccccacct 960

ctgactccct tctcttgact ccagatcaag acacagccat ccgggtcttc gccatccccc 1020

catcctttgc cagcatcttc ctcaccaagt ccaccaagtt gacctgcctg gtcacagacc 1080

tgaccaccta tgacagcgtg accatctcct ggacccgcca gaatggcgaa gctgtgaaaa 1140

cccacaccaa catctccgag agccacccca atgccacttt cagcgccgtg ggtgaggcca 1200

gcatctgcga ggatgactgg aattccgggg agaggttcac gtgcaccgtg acccacacag 1260

acctgccctc gccactgaag cagaccatct cccggcccaa gggtaggccc cactcttgcc 1320

cctcttcctg cactccctgg gacctccctt ggcctctggg gcatggtgga aagcacccct 1380

cactcccccg ttgtctgggc aactggggaa aaggggactc aaccccagcc cacaggctgg 1440

tccccccact gccccgccct caccaccatc tctgttcaca ggggtggccc tgcacaggcc 1500

cgatgtctac ttgctgccac cagcccggga gcagctgaac ctgcgggagt cggccaccat 1560

cacgtgcctg gtgacgggct tctctcccgc ggacgtcttc gtgcagtgga tgcagagggg 1620

gcagcccttg tccccggaga agtatgtgac cagcgcccca atgcctgagc cccaggcccc 1680

aggccggtac ttcgcccaca gcatcctgac cgtgtccgaa gaggaatgga acacggggga 1740

gacctacacc tgcgtggtgg cccatgaggc cctgcccaac agggtcaccg agaggaccgt 1800

ggacaagtcc accggtaaac ccaccctgta caacgtgtcc ctggtcatgt ccgacacagc 1860

tggcacctgc tactgaccct gctggcctgc ccacaggctc ggggcggctg gccgctctgt 1920

gtgtgcatgc aaactaaccg tgtcaacggg gtgagatgtt gcatcttata aaatt 1975

<210> 404
<211> 1362
<212> DNA
<213> homo sapiens;

<400> 404
gggagtgcat ccgccccaac ccttttcccc ctcgtctcct gtgagaattc cccgtcggat 60

acgagcagcg tggccgttgg ctgcctcgca caggacttcc ttcccgactc catcactttc 120

tcctggaaat acaagaacaa ctctgacatc agcagcaccc ggggcttccc atcagtcctg 180

agagggggca agtacgcagc cacctcacag gtgctgctgc cttccaagga cgtcatgcag 240

ggcacagacg aacacgtggt gtgcaaagtc cagcacccca acggcaacaa agaaaagaac 300

gtgcctcttc cagtgattgc cgagctgcct cccaaagtga gcgtcttcgt cccaccccgc 360

gacggcttct tcggcaaccc ccgcaagtcc aagctcatct gccaggccac gggtttcagt 420

ccccggcaga ttcaggtgtc ctggctgcgc gaggggaagc aggtggggtc tggcgtcacc 480

acggaccagg tgcaggctga ggccaaagag tctgggccca cgacctacaa ggtgaccagc 540

acactgacca tcaaagagag cgactggctc ggccagagca tgttcacctg ccgcgtggat 600

cacaggggcc tgaccttcca gcagaatgcg tcctccatgt gtgtccccga tcaagacaca 660

gccatccggg tcttcgccat ccccccatcc tttgccagca tcttcctcac caagtccacc 720

aagttgacct gcctggtcac agacctgacc acctatgaca gcgtgaccat ctcctggacc 780

cgccagaatg gcgaagctgt gaaaacccac accaacatct ccgagagcca ccccaatgcc 840

actttcagcg ccgtgggtga ggccagcatc tgcgaggatg actggaattc cggggagagg 900

ttcacgtgca ccgtgaccca cacagacctg ccctcgccac tgaagcagac catctcccgg 960

cccaaggggg tggccctgca caggcccgat gtctacttgc tgccaccagc ccgggagcag 1020

ctgaacctgc gggagtcggc caccatcacg tgcctggtga cgggcttctc tcccgcggac 1080

gtcttcgtgc agtggatgca gagggggcag cccttgtccc cggagaagta tgtgaccagc 1140

gccccaatgc ctgagcccca ggccccaggc cggtacttcg cccacagcat cctgaccgtg 1200

tccgaagagg aatggaacac gggggagacc tacacctgcg tggtggccca tgaggccctg 1260

cccaacaggg tcaccgagag gaccgtggac aagtccaccg gtaaacccac cctgtacaac 1320

gtgtccctgg tcatgtccga cacagctggc acctgctact ga 1362

<210> 405
<211> 11
<212> DNA
<213> homo sapiens;

<400> 405
ctaactgggg a 11

<210> 406
<211> 31
<212> DNA
<213> homo sapiens;

<400> 406
aggatattgt agtggtggta gctgctactc c 31

<210> 407
<211> 37
<212> DNA
<213> homo sapiens;

<400> 407
gtattatgat tacgtttggg ggagttatcg ttatacc 37

<210> 408
<211> 18
<212> DNA
<213> homo sapiens;

<400> 408
gagtatagca gctcgtcc 18

<210> 409
<211> 20
<212> DNA
<213> homo sapiens;

<400> 409

gtggatacag ctatggttac                                                20

<210>  410
<211>  31
<212>  DNA
<213>  homo sapiens;

<400>  410
aggatattgt agtagtacca gctgctatgc c                                   31

<210>  411
<211>  16
<212>  DNA
<213>  homo sapiens;

<400>  411
tgactacagt aactac                                                    16

<210>  412
<211>  23
<212>  DNA
<213>  homo sapiens;

<400>  412
gtggatatag tggctacgat tac                                            23

<210>  413
<211>  31
<212>  DNA
<213>  homo sapiens;

<400>  413
gtattacgat ttttggagtg gttattatac c                                    31

<210>  414
<211>  31
<212>  DNA
<213>  homo sapiens;

<400>  414
aggatattgt actaatggtg tatgctatac c                                   31

<210>  415
<211>  16
<212>  DNA
<213>  homo sapiens;

<400>  415
tgactacagt aactac                                                    16

<210>  416
<211>  19
<212>  DNA
<213>  homo sapiens;

<400>  416

tgactacggt ggtaactcc                                                    19

<210>  417
<211>  17
<212>  DNA
<213>  homo sapiens;

<400>  417
ggtataaccg gaaccac                                                      17

<210>  418
<211>  31
<212>  DNA
<213>  homo sapiens;

<400>  418
gtattactat ggttcgggga gttattataa c                                      31

<210>  419
<211>  20
<212>  DNA
<213>  homo sapiens;

<400>  419
ggtatagtgg gagctactac                                                   20

<210>  420
<211>  31
<212>  DNA
<213>  homo sapiens;

<400>  420
gtattacgat attttgactg gttattataa c                                      31

<210>  421
<211>  17
<212>  DNA
<213>  homo sapiens;

<400>  421
ggtacaactg gaacgac                                                      17

<210>  422
<211>  18
<212>  DNA
<213>  homo sapiens;

<400>  422
gggtatagca gcggctac                                                     18

<210>  423
<211>  20
<212>  DNA
<213>  homo sapiens;

<400>  423

gtagagatgg ctacaattac                                                    20

<210>  424
<211>  28
<212>  DNA
<213>  homo sapiens;

<400>  424
agcatattgt ggtggtgact gctattcc                                          28

<210>  425
<211>  17
<212>  DNA
<213>  homo sapiens;

<400>  425
ggtataactg gaacgac                                                       17

<210>  426
<211>  21
<212>  DNA
<213>  homo sapiens;

<400>  426
gggtatagca gcagctggta c                                                  21

<210>  427
<211>  16
<212>  DNA
<213>  homo sapiens;

<400>  427
tgactacggt gactac                                                        16

<210>  428
<211>  31
<212>  DNA
<213>  homo sapiens;

<400>  428
gtattactat gatagtagtg gttattacta c                                       31

<210>  429
<211>  20
<212>  DNA
<213>  homo sapiens;

<400>  429
gtggatacag ctatggttac                                                    20

<210>  430
<211>  21
<212>  DNA
<213>  homo sapiens;

<400>  430

gggtatagca gtggctggta c                                                21

<210>  431
<211>  17
<212>  DNA
<213>  homo sapiens;

<400>  431
ggtataactg gaactac                                                    17

<210>  432
<211>  6
<212>  PRT
<213>  Oryctolagus cuniculus;

<400>  432

Tyr Tyr Gly Met Asp Leu
1               5

<210>  433
<211>  20
<212>  DNA
<213>  Oryctolagus cuniculus;

<400>  433
attactacgg catggacctc                                                 20

<210>  434
<211>  6
<212>  PRT
<213>  Ovis aries;

<400>  434

Tyr Tyr Gly Val Asp Val
1               5

<210>  435
<211>  20
<212>  DNA
<213>  Ovis aries;

<400>  435
attactacgg tgtagatgtc                                                 20

<210>  436
<211>  6
<212>  PRT
<213>  Bos taurus;

<400>  436

Tyr Tyr Gly Val Asp Val
1               5

<210>  437
<211>  20
<212>  DNA
<213>  Bos taurus;

<400>  437
attactacgg tgtagatgtc                                                    20


<210>  438
<211>  6
<212>  PRT
<213>  Canis familiaris;

<400>  438

Tyr Tyr Gly Met Asp Tyr
1               5


<210>  439
<211>  20
<212>  DNA
<213>  Canis familiaris;

<400>  439
attactatgg tatggactac                                                    20


<210>  440
<211>  9
<212>  PRT
<213>  homo sapiens;

<400>  440

Tyr Tyr Tyr Tyr Tyr Gly Val Asp Val
1               5


<210>  441
<211>  29
<212>  DNA
<213>  homo sapiens;

<400>  441
attactacta ctactacggt atggacgtc                                          29


<210>  442
<211>  57
<212>  DNA
<213>  Homo sapiens

<400>  442
atgggctggt cctgcatcat cctgtttctg gtggccaccg ccaccggcgt gcacagc          57


<210>  443
<211>  19
<212>  PRT
<213>  Homo sapiens

<400>  443

Met Gly Trp Ser Cys Ile Ile Leu Phe Leu Val Ala Thr Ala Thr Gly
1               5                   10                  15

Val His Ser

<210>  444
<211>  21
<212>  DNA
<213>  Artificial Sequence

<220>
<223>  Primer

<400>  444
aggccagcag agggttccat g                                                21

<210>  445
<211>  22
<212>  DNA
<213>  Artificial Sequence

<220>
<223>  Primer

<400>  445
ggctcccaga tcctcaaggc ac                                               22